# EUROPEAN PATENT SPECIFICATION

(11) **EP 3 150 222 B1**
(45) Date of publication and mention of the grant of the patent: **27.11.2019**
(21) Application number: 16193095.3
(22) Date of filing: 21.12.2011
(51) Int. Cl.: A61K 39/085, C07K 16/12

(54) **STABLE IMMUNOGENIC COMPOSITIONS OF STAPHYLOCOCCUS AUREUS ANTIGENS**
STABILE IMMUNOGENE ZUSAMMENSETZUNGEN AUS STAPHYLOCOCCUS AUREUS-ANTIGENEN
COMPOSITIONS IMMUNOGÈNES STABLES D'ANTIGÈNES DE STAPHYLOCOCCUS AUREUS

(30) Priority: 22.12.2010 US 201061426476 P
(43) Date of publication of application: 05.04.2017
(62) Divisional of application: 11804822.2
(73) Proprietor: Wyeth LLC, New York, NY 10017-5755 (US)
(72) Inventor: KHANDKE, Lakshmi, Pearl River, NY 10965 (US); NONOYAMA, Akihisa, Morrisville, NC 27560 (US); HODGE, Tamara Shafer, Chesterfield, MO 63017 (US); NEMA, Sandeep, Chesterfield, MO 63017 (US)
(74) Representative: Pfizer

(56) References cited:
- WO-A1-2010/151544
- WO-A2-2006/032472
- WO-A2-2007/113222
- WO-A2-2007/113223
- WO-A2-2008/057550
- NANRA J S ET AL: "Heterogeneous in vivo expression of clumping factor A and capsular polysaccharide by Staphylococcus aureus: Implications for vaccine design", VACCINE, ELSEVIER LTD, GB, vol. 27, no. 25-26, 26 May 2009 (2009-05-26), pages 3276-3280, XP026122449, ISSN: 0264-410X, DOI: 10.1016/J.VACCINE.2009.01.062 [retrieved on 2009-02-05]
- PROJAN S J ET AL: "Staphylococcal vaccines and immunotherapy: to dream the impossible dream?", CURRENT OPINION IN PHARMACOLOGY, ELSEVIER SCIENCE PUBLISHERS, NL, vol. 6, no. 5, 1 October 2006 (2006-10-01) , pages 473-479, XP028058459, ISSN: 1471-4892, DOI: 10.1016/J.COPH.2006.04.005 [retrieved on 2006-10-01]
- ZAGURSKY R J ET AL: "Application of genomics in bacterial vaccine discovery: a decade in review", CURRENT OPINION IN PHARMACOLOGY, ELSEVIER SCIENCE PUBLISHERS, NL, vol. 8, no. 5, 1 October 2008 (2008-10-01) , pages 632-638, XP025609344, ISSN: 1471-4892, DOI: 10.1016/J.COPH.2008.06.009 [retrieved on 2008-07-24]
- CARPENTER J F ET AL: "Rational design of stable lyophilized protein formulations: Some practical advice", PHARMACEUTICAL RESEARCH, SPRINGER NEW YORK LLC, US, vol. 14, no. 8, 1 January 1997 (1997-01-01), pages 969-975, XP002303280, ISSN: 0724-8741, DOI: 10.1023/A:1012180707283
- ADAMS GERALD D J: "Lyophilization of vaccines: current trends", METHODS IN MOLECULAR MEDICINE,, vol. 87, 1 January 2003 (2003-01-01), pages 223-244, XP009187713, ISSN: 1543-1894

## Description

### Background of the Invention

Humans are the natural reservoirs for *Staphylococcus aureus* (*S. aureus*). Healthy individuals can be colonized by *S. aureus* on the skin, in the nares and the throat either persistently (10-35%), intermittently (20-75%) or be in a non-carriage state (5-70%) with no associated disease. See Vandenbergh et al., J. Clin. Micro. 37:3133-3140 (1999). Disease subsequently occurs when individuals become immunocompromised due to breaches in immune barriers, such as during surgery, placement of indwelling catheters or other devices, trauma, or wounds. The resulting *S. aureus* infection can cause a wide range of diseases that range from mild skin infections to endocarditis, osteomyelitis, bacteremia, sepsis, and other forms of disease with accompanying high mortality rates. The large human reservoir enhances opportunity for evolution and spread of adapted pathogenic clonal types.

Invasive staphylococcal infections from the Gram positive cocci *S. aureus* and *S. epidermidis* are of particular concern because they are an increasing public health problem worldwide. Specifically, *S. aureus* is responsible for the majority of hospital-acquired (nosocomial) infections, and its prevalence in community-onset infections is increasing. For example, the incidence of invasive methicillin-resistant *S. aureus* (MRSA) was estimated at 31.8 per 100,000 persons, including 18,650 deaths in the United States in 2005. See Klevens R.M. et al., JAMA, 298:1763-71 (2007).

Staphylococcal diseases have seen a dramatic increase in the last 20 years; this increase parallels the use of intravascular devices and invasive procedures. The rise in disease incidence is made more troubling because of the parallel rise of antibiotic resistance; therefore, there is an urgent need for immunogenic compositions for use in vaccines or to elicit polyclonal or monoclonal antibodies to confer passive immunity as a means to prevent or treat staphylococcal infection and associated diseases.

Clumping factor A (ClfA) is an *S. aureus* cell wall-associated adhesin that mediates staphylococcal binding to fibrinogen and platelets. It is expressed on the cell surface of the bacterium, where it is thought to promote pathogenesis by binding to the fibrinogen and fibrin that is deposited at the site of tissue damage. ClfA is well conserved, and even the most diverse form (∼85% identity) exhibits extensive cross-reactivity to both monoclonal and polyclonal antibodies. Thus, ClfA is a reasonable candidate for a component of a vaccine against *S. aureus.* However, given the structural instability of ClfA, a formulation of ClfA is problematic since it can readily degrade over time in storage.

Full-length ClfA comprises several regions and domains: an N-terminal secretory domain ("S" domain); followed by a ligand-binding A region, which contains three domains (N1, N2, which contains an EF-hand motif, and N3); followed by an R region, which contains serine-aspartate dipeptide repeats; followed by a cell wall-binding region ("W" region) containing an LPXTG motif; a hydrophobic membrane-spanning domain ("M" region); and a charged C-terminus ("C" region) containing positively charged amino acids. The N1 region contains a protease-sensitive site. Much of the instability of ClfA is attributed to the clipping of ClfA at N1, which results in fragments containing N1 and N2N3. The structure and function of ClfA is disclosed in U.S. Patent Application Publication No. 20070087014A1 (Pavliak et al., April 19, 2007).

Staphylococcal microorganisms capable of causing invasive disease generally also are capable of producing a capsule polysaccharide (CP) that encapsulates the bacterium and enhances its resistance to clearance by the host innate immune system. The CP serves to cloak the bacterial cell in a protective capsule that renders the bacteria resistant to phagocytosis and intracellular killing. Bacteria lacking a capsule are more susceptible to phagocytosis. Capsular polysaccharides are frequently an important virulence factor for many bacterial pathogens, including *Haemophilus influenzae, Streptococcus pneumoniae* and Group B streptococci.

Most clinical isolates of *S. aureus* are encapsulated with either serotypes 5 or 8. Type 5 (CP5) and type 8 (CP8) capsular polysaccharides have similar trisaccharide repeating units comprised of N-acetyl mannosaminuronic acid, N-acetyl L-fucosamine, and N-acetyl D-fucosamine. See Fournier, J.M. et al., Infect. Immun. 45:97-93 (1984) and Moreau, M., et al., Carbohydrate Res. 201:285-297

(1990). The two CPs, which have the same sugars, but differ in the sugar linkages and in sites of O-acetylation, each produce serologically distinct patterns of immunoreactivity.

The *S. aureus* MntC protein (also known as Protein 305, P305, P305A, and ORF305) is a component of a manganese ABC transporter. This protein is expressed *in vivo. S. aureus* uses manganese as a cofactor for an enzyme that enhances the survival of *S. aureus* in neutraphils. MntC is, therefore, important for the *in vivo* survival of *S. aureus* during infection. Like ClfA, this protein is also unstable in solution. However, unlike ClfA, which can aggregate, or clip via hydrolysis, the primary mechanism of MntC degradation is deamidation when subject to basic pH and/or temperature around room temperature (about 25°C) or higher.
WO2007113222 relates to immunogenic compositions comprising Type 5 and/or 8 capular polysaccharide or oligosaccharide from *S. aureus* having between 30-100% O- acetylation.

Accordingly, there is an urgent need not only for vaccines against *S*. *aureus* infection, but in particular for vaccines with enhanced antigen stability.

### Summary of the Invention

The invention is set out in the appended set of claims. The embodiments and/or examples of the following description which are not covered by the appended claims are considered as not being part of the present invention. The present invention is directed towards a reconstituted multi-antigen immunogenic composition as defined in the appended claims. The antigens, which are polypeptides and polysaccharides, may be obtained, *inter alia,* directly from the bacterium using isolation procedures known to those skilled in the art, or they may be produced using synthetic protocols, or they may be recombinantly produced using genetic engineering procedures also known to those skilled in the art, or through a combination of any of the foregoing. The reconstituted immunogenic composition of the invention comprises an isolated *S. aureus* clumping factor A (ClfA). In certain embodiments, the reconstituted immunogenic composition of the invention comprises an isolated *S. aureus* clumping factor A (ClfA) polypeptide, an isolated *S. aureus* clumping factor B (ClfB) polypeptide, an isolated *S. aureus* capsular polysaccharide type 5 (CP5) conjugated to a carrier protein, an isolated *S. aureus* capsular polysaccharide type 8 (CP8) conjugated to a carrier protein, and an isolated *S. aureus* MntC protein. In addition, the present disclosure provides methods for inducing an immune response against a staphylococcal bacterium; methods for preventing, reducing the severity, or delaying onset of a disease caused by a staphylococcal bacterium; and methods for preventing, reducing the severity, or delaying onset of at least one symptom of a disease caused by infection with a staphylococcal bacterium.

Accordingly, the invention provides a reconstituted immunogenic composition comprising: an isolated *S. aureus* clumping factor A (ClfA) polypeptide, an isolated *S. aureus* capsular polysaccharide type 5 (CP5) conjugated to a carrier protein, and an isolated *S. aureus* capsular polysaccharide type 8 (CP8) conjugated to a carrier protein.

In one embodiment, the invention provides a reconstituted immunogenic composition comprising: an isolated *S. aureus* clumping factor A (ClfA) polypeptide, an isolated *S. aureus* clumping factor B (ClfB) polypeptide, an isolated *S. aureus* MntC protein, an isolated *S. aureus* capsular polysaccharide type 5 (CP5) conjugated to a carrier protein, and an isolated *S. aureus* capsular polysaccharide type 8 (CP8) conjugated to a carrier protein.

In one embodiment, the invention provides a reconstituted immunogenic composition comprising: an isolated *S. aureus* clumping factor A (ClfA) polypeptide, an isolated *S. aureus* MntC protein, an isolated *S. aureus* capsular polysaccharide type 5 (CP5) conjugated to a carrier protein, and an isolated *S. aureus* capsular polysaccharide type 8 (CP8) conjugated to a carrier protein.

The disclosure provides a lyophilized immunogenic composition comprising: (a) at least three components selected from the group consisting of an isolated Staphylococcus aureus clumping factor A ("ClfA") polypeptide, an isolated Staphylococcus aureus clumping factor B ("ClfB") polypeptide, a Capsular Polysaccharide Type 5 (CP5)-protein conjugate, a Capsular Polysaccharide Type 8 (CP8)-protein conjugate, and an isolated Staphylococcus aureus MntC polypeptide; (b) a buffer having a pKa of 6.0 ± 0.6, and (c) a bulking agent. In certain embodiments, the ClfA polypeptide remains substantially undegraded for at least 1 month at 37°C.

The disclosure provides a lyophilized immunogenic composition comprising: (a) an isolated Staphylococcus aureus clumping factor A ("ClfA") polypeptide, (b) a Capsular Polysaccharide Type 5 (CP5)-protein conjugate, (c) a Capsular Polysaccharide Type 8 (CP8)-protein conjugate, (d) a buffer having a pKa of 6.0 ± 0.6, and (e) a bulking agent. In certain embodiments, the ClfA polypeptide remains substantially undegraded for at least 1 month at 37°C.

In one embodiment, the lyophilized immunogenic composition comprises water at less than 3 percent weight of the total weight of the immunogenic composition (% w/w), wherein the ClfA polypeptide is between 0.09% ± 0.027% and 0.85% ± 0.26% w/w, the CP5-protein conjugate is between 0.04% ± 0.013% and 0.42 ± 0.13% w/w, the CP8-protein conjugate is between 0.04% ± 0.013% and 0.42 ± 0.13% w/w, and the buffer is at 2.54% ± 0.76% w/w.

The disclosure further provides a lyophilized immunogenic composition comprising: (a) a ClfA polypeptide; (b) a CP5-protein conjugate; (c) a CP8-protein conjugate; (d) a histidine buffer, pH 6.0 ± 0.5; (e) sucrose; (f) polysorbate 80; and (g) water. In certain embodiments, the ClfA polypeptide remains substantially undegraded for at least 3 months at 37°C.

The disclosure further provides a lyophilized immunogenic composition comprising: (a) a ClfA polypeptide between 0.09% ± 0.027% and 0.85% ± 0.26% w/w; (b) a CP5-protein conjugate between 0.04% ± 0.013% and 0.42 ± 0.13% w/w; (c) a CP8-protein conjugate between 0.04% ± 0.013% and 0.42 ± 0.13% w/w; (d) a histidine buffer, pH 6.0 ± 0.5, at 2.54% ± 0.76% w/w; (e) sucrose at 97% ± 2.0% w/w; (f) polysorbate 80 at 0.21% ± 0.04% w/w; and (g) water at 2% ± 1% w/w.

The disclosure further provides a liquid immunogenic tri-antigen composition manufactured by reconstituting a lyophilized immunogenic composition of the disclosure in an aqueous diluent, said reconstituted composition having a final pH of 6.0 ± 0.5.

The disclosure further provides a liquid immunogenic composition manufactured by reconstituting a lyophilized immunogenic composition of the invention comprising: (a) the ClfA polypeptide at a concentration of between 40 µg/ml ± 4µg/ml and 800µg/ml ± 80µg/ml; (b) the CP5-proteinconjugate at a concentration of between 20µg/ml ± 2µg/ml and 400µg/ml ± 40µg/ml; (c) the CP8-protein conjugate at a concentration of between 20µg/ml ± 2µg/ml and 400µg/ml ± 40µg/ml; (d) the histidine buffer at a concentration of 10mM ± 5mM; (e) polysorbate 80 at a concentration of 0.1% ± 0.05% weight to volume (w/v); and (f) sucrose at a concentration of 9% ± 4.5% w/v. In another embodiment, the polysorbate 80 is at a concentration of 0.01% ± 0.005% weight to volume (w/v) and the sucrose is at a concentration of 4.5% ± 1.5% w/v.

The disclosure further provides a liquid immunogenic composition comprising: (a) a reconstituted lyophilized ClfA polypeptide; (b) a CP5-protein conjugate; (c) a CP8-protein conjugate; (d) a histidine buffer, pH 6.0 ± 0.5; (e) polysorbate 80; (f) sucrose; and (g) an aqueous diluent.

The disclosure further provides a liquid immunogenic composition manufactured by reconstituting a lyophilized immunogenic composition of the invention comprising: (a) the reconstituted lyophilized ClfA polypeptide at a concentration of between 40 µg/ml ± 4µg/ml and 800µg/ml ± 80µg/ml; (b) the CP5-protein conjugate at a concentration of between 20µg/ml ± 2µg/ml and 400µg/ml ± 40µg/ml; (c) the CP8-protein conjugate at a concentration of between 20µg/ml ± 2µg/ml and 400µg/ml ± 40µg/ml; (d) the histidine buffer at a concentration of 10mM ± 5mM; (e) polysorbate 80 at a concentration of 0.1% ± 0.05% weight to volume (w/v); (f) sucrose at a concentration of 9% ± 4.5% w/v; and (g) an aqueous diluent. In another embodiment, the polysorbate 80 is at a concentration of 0.01% ± 0.005% weight to volume (w/v) and the sucrose is at a concentration of 4.5% ± 1.5% w/v.

The disclosure further provides a process of making an immunogenic composition comprising the steps of: (a) combining an aqueous solution comprising: (i) a ClfA polypeptide, (ii) a CP5-protein conjugate, (iii) a CP8-protein conjugate, (iv) a buffer having a pKa of 6.0 ± 0.6, and (v) a bulking agent; and (b) lyophilizing the combination of step (a) to form a cake comprising less than 3 percent water by weight. In certain embodiments, the process further comprises combining (vi) a surfactant, at step (a). In certain embodiments, the process further comprises a step of filter sterilizing the combination of step (a) prior to lyophilization of step (b). In certain embodiments, the aqueous solution comprises 10mM ± 1mM histidine, pH 6.0 ± 0.5, sucrose at 9% ± 4.5% w/v and polysorbate 80 at 0.1% ± 0.05% w/v. In another embodiment, the polysorbate 80 is at a concentration of 0.01% ± 0.005% w/v and the sucrose is at a concentration of 4.5% ± 1.5% w/v.

In certain embodiments, the lyophilizing step (b) comprises the steps of: (i) freezing the sterilized combination of step (a) at a rate of 0.3°C ± 0.03°C per minute until reaching a temperature of -50°C ± 5°C at a pressure of 400 millibars ± 40 millibars; and then holding the combination at -50°C ± 5°C for 60 minutes ± 6 minutes; (ii) annealing the combination by increasing the temperature to -10°C ± 5°C at a rate of 0.3°C ± 0.03°C per minute; subsequently holding the temperature at -10°C ± 5°C for 120 minutes ± 12 minutes, followed by decreasing the temperature at a rate of 0.3°C ± 0.03°C per minute until reaching a temperature of - 50°C ± 5°C, and holding the temperature at -50°C ± 5°C for 180 minutes ± 18 minutes; (iii) drying the combination by decreasing the pressure to 50 milliTorrs (mTorr) and holding for 30 minutes, followed by increasing the temperature to - 30°C ± 5°C at a rate of 0.2°C ± 0.02°C per minute, and subsequently holding the temperature at -30°C ± 5°C for 1,920 minutes ± 192 minutes; (iv) further drying the combination by increasing the temperature to 30°C ± 5°C at a rate of 0.2°C ± 0.02°C per minute and increasing the pressure to 200 mTorr, and subsequently holding the temperature at 30°C ± 5°C for 720 minutes ± 72 minutes; and (v) decreasing the temperature to 5°C ± 5°C at a rate of 0.5°C ± 0.05°C per minute. In some embodiments, lyophilization takes place in a vial. In some embodiments, the vial is stoppered after lyophilization. In certain embodiments, the vial is backfilled with nitrogen gas prior to stoppering the vial. In certain embodiments, the process further comprises the step of: (c) reconstituting the lyophilized combination of step (b) in an aqueous medium. In certain embodiments, the osmolality of the reconstituted combination of step (c) is between 250 mOsM ± 25mOsM and 300 mOsM ± 30 mOsM. In certain embodiments, an immunogenic composition of the disclosure is manufactured according to any process of making an immunogenic composition of the disclosure.

The disclosure further provides a lyophilized immunogenic composition comprising: (a) at least four components selected from the group consisting of an isolated *Staphylococcus aureus* clumping factor A (ClfA) polypeptide, an isolated *Staphylococcus aureus* clumping factor B (ClfB) polypeptide, a CP5-protein conjugate, a CP8-protein conjugate, and an isolated *Staphylococcus aureus* MntC polypeptide; (b) a buffer having a pKa of 6.0 ± 0.6, and (c) a bulking agent. In certain embodiments, the ClfA polypeptide remains substantially undegraded for at least 1 month at 37°C.

The disclosure further provides a lyophilized immunogenic composition comprising: (a) an isolated *Staphylococcus aureus* clumping factor A (ClfA) polypeptide; (b) a Capsular Polysaccharide Type 5 conjugated to CRM₁₉₇ (CP5-CRM₁₉₇); (c) a Capsular Polysaccharide Type 8 conjugated to CRM₁₉₇ (CP8-CRM₁₉₇); (d) an isolated MntC polypeptide; (e) a buffer having a pKa of 6.0 ± 0.6, and (f) a bulking agent. In certain embodiments, the ClfA polypeptide remains substantially undegraded for at least 1 month at 37°C.

The disclosure further provides a lyophilized immunogenic composition comprising: water at less than 3 percent weight of the total weight of the immunogenic composition (% w/w), wherein the ClfA polypeptide is between 0.09% ± 0.027% and 0.84% ± 0.25% w/w, the CP5-CRM₁₉₇ is between 0.04% ± 0.013% and 0.42 ± 0.13% w/w, the CP8-CRM₁₉₇ is between 0.04% ± 0.013% and 0.42 ± 0.13% w/w, the MntC is between 0.09% ± 0.027% and 0.84% ± 0.25% w/w, and the buffer is at 3.3% ± 0.99% w/w.

The disclosure further provides a lyophilized immunogenic composition comprising: (a) a ClfA polypeptide; (b) a CP5-CRM₁₉₇ conjugate; (c) a CP8-CRM₁₉₇ conjugate; (d) an MntC polypeptide; (e) a histidine buffer; (f) sucrose; (g) polysorbate 80; and (h) water. In certain embodiments, the ClfA polypeptide remains substantially undegraded for at least 3 months at 37°C.

The disclosure further provides a lyophilized immunogenic composition comprising: (a) a ClfA polypeptide between 0.09% ± 0.027% and 0.84% ± 0.25% w/w; (b) a CP5-CRM₁₉₇ conjugate between 0.04% ± 0.013% and 0.42 ± 0.13% w/w; (c) a CP8-CRM₁₉₇ conjugate between 0.04% ± 0.013% and 0.42 ± 0.13% w/w; (d) an MntC polypeptide 0.09% ± 0.027% and 0.84% ± 0.25% w/w; (e) a histidine buffer at 3.3% ± 0.99% w/w; (f) sucrose at 95% ± 2% w/w; (g) polysorbate 80 at 0.21% ± 0.063% w/w; and (h) water at 2% ± 1% w/w. In certain embodiments, the ClfA polypeptide remains substantially undegraded for at least 3 months at 37°C.

The disclosure further provides a liquid immunogenic tetra-antigen composition manufactured by reconstituting a lyophilized immunogenic composition of the invention in an aqueous diluent, said reconstituted composition having a final pH of 6.5 ± 0.5.

The disclosure further provides a liquid immunogenic composition comprising: (a) the ClfA polypeptide at a concentration of between 40 µg/ml ± 4µg/ml and 800µg/ml ± 80µg/ml; (b) the CP5-CRM₁₉₇ conjugate at a concentration of between 20µg/ml ± 2µg/ml and 400µg/ml ± 40µg/ml; (c) the CP8-CRM₁₉₇ conjugate at a concentration of between 20µg/ml ± 2µg/ml and 400µg/ml ± 40µg/ml; (d) the isolated MntC polypeptide at a concentration of between 40 µg/ml ± 4µg/ml and 800µg/ml ± 80µg/ml; (e) the histidine buffer at a concentration of 10mM ± 5mM; (f) polysorbate 80 at a concentration of 0.01% ± 0.005% weight to volume (w/v); and (g) sucrose at a concentration of 4.5% ± 1.5% w/v. In another embodiment, the polysorbate 80 is at a concentration of 0.01% ± 0.005% w/v and the sucrose is at a concentration of 4.5% ± 1.5% w/v.

The disclosure provides a liquid immunogenic composition comprising: (a) a reconstituted lyophilized ClfA polypeptide; (b) a CP5-CRM₁₉₇ conjugate; (c) a CP8-CRM₁₉₇ conjugate; (d) a MntC polypeptide; (d) a histidine buffer; (e) polysorbate 80; (f) sucrose; and (g) an aqueous diluent.

In one embodiment, the invention provides a liquid immunogenic composition comprising: (a) the reconstituted lyophilized ClfA polypeptide at a concentration of between 40 µg/ml ± 4µg/ml and 800µg/ml ± 80µg/ml; (b) the CP5-CRM₁₉₇ conjugate at a concentration of between 20µg/ml ± 2µg/ml and 400µg/ml ± 40µg/ml; (c) the CP8-CRM₁₉₇ conjugate at a concentration of between 20µg/ml ± 20µg/ml and 400µg/ml ± 40µg/ml; (d) the isolated MntC polypeptide at a concentration of between 40 µg/ml ± 40µg/ml and 800µg/ml ± 80µg/ml; (e) the histidine buffer at a concentration of 10mM ± 5mM; (f) polysorbate 80 at a concentration of 0.1% ± 0.05% weight to volume (w/v); and (g) sucrose at a concentration of 9% ± 4.5% w/v; and (h) the aqueous diluent. In another embodiment, the polysorbate 80 is at a concentration of 0.01% ± 0.005% w/v and the sucrose is at a concentration of 4.5% ± 1.5% w/v.

The disclosure further provides a process of making an immunogenic composition comprising the steps of: (a) combining in an aqueous solution: (i) a ClfA polypeptide, (ii) a CP5-CRM₁₉₇ conjugate, (iii) a CP8-CRM₁₉₇ conjugate, (iv) a MntC polypeptide, (v) a buffer having a pKa of 6.0 ± 0.6, and (vi) a bulking agent; and (b) lyophilizing the combination of step (a) to form a cake comprising less than 3 percent water by weight. In certain embodiments, the process further comprises combining (vi) a surfactant, at step (a). In certain embodiments, the process further comprises a step of filter sterilizing the combination of step (a) prior to lyophilization of step (b). In certain embodiments, the aqueous solution comprises 10mM ± 5mM histidine, pH 6.0 ± 0.5, sucrose at 9% ± 1% w/v and polysorbate 80 at 0.1% ± 0.02% w/v. In other embodiments, the polysorbate 80 is at a concentration of 0.01% ± 0.001% w/v and the sucrose is at a concentration of 4.5% ± 0.45% w/v. In certain embodiments, the lyophilizing step (b) comprises the steps of: (i) freezing the sterilized combination of step (a) at a rate of 0.3°C ± 0.03°C per minute until reaching a temperature of -50°C ± 5°C at a pressure of 400 millibars ± 40 millibars; and then holding the combination at -50°C ± 5°C for 60 minutes ± 6 minutes; (ii) annealing the combination by increasing the temperature to -10°C ± 5°C at a rate of 0.3°C ± 0.03°C per minute; then holding the temperature at -10°C ± 5°C for 120 minutes ± 12 minutes; then decreasing the temperature at a rate of 0.3°C ± 0.03°C per minute until reaching a temperature of - 50°C ± 5°C; and then holding the temperature at -50°C ± 5°C for 180 minutes ± 18 minutes; (iii) drying the combination by decreasing the pressure to 50 milliTorrs (mTorr) and holding for 30 minutes; then increasing the temperature to -30°C ± 5°C at a rate of 0.2°C ± 0.02°C per minute; and then holding the temperature at - 30°C ± 5°C for 1,920 minutes ± 192 minutes; (iv) drying the combination by increasing the temperature to 30°C ± 5°C at a rate of 0.2°C ± 0.02°C per minute; and then increasing the pressure to 200 mTorr and holding the temperature at 30°C ± 5°C for 720 minutes ± 72 minutes; (v) decreasing the temperature to 5°C ± 5°C at a rate of 0.5°C ± 0.05°C per minute. In some embodiments, lyophilization takes place in a vial. In some embodiments, the vial is stoppered after lyophilization. In certain embodiments, the vial is backfilled with nitrogen gas prior to stoppering the vial. In certain embodiments, the process further comprises the step of: (c) reconstituting the lyophilized combination of step (b) in an aqueous medium. In certain embodiments, the osmolality of the reconstituted combination of step (c) is between 250 mOsM ± 25mOsM and 300 mOsM ± 30 mOsM. In certain embodiments, an immunogenic composition of the disclosure is manufactured according to any process of making an immunogenic composition of the disclosure.

The disclosure further provides a lyophilized immunogenic composition comprising: (a) an isolated *Staphylococcus aureus* clumping factor A (ClfA) polypeptide comprising a fibrinogen domain, (b) a buffer having a pKa of 6.0 ± 0.6, and (c) a bulking agent. In certain embodiments, the ClfA polypeptide remains substantially undegraded for at least 1 month at 37°C. In one embodiment, the invention provides a lyophilized immunogenic composition comprising water at less than 3 percent weight of the total weight of the immunogenic composition (% w/w), wherein the ClfA polypeptide is at 0.52 percent ± 0.46 % w/w, and the buffer is at 0.28% ± 0.065% w/w. In certain embodiments, the bulking agent is sucrose at 97% ± 2.0% w/w.

The disclosure further provides a lyophilized immunogenic composition comprising: (a) a ClfA polypeptide at 0.52% ± 0.46% w/w, (b) a succinate buffer at 0.28% ± 0.065% w/w, (c) sucrose at 97% ± 0.57% w/w, (d) polysorbate 80 at 0.20% ± 0.042% w/w, and (e) water at 2.5% ± 0.5% w/w. In certain embodiments, the ClfA polypeptide remains substantially undegraded for at least 1 month at 37°C.

The disclosure further provides a liquid immunogenic ClfA composition manufactured by reconstituting a lyophilized immunogenic composition of the invention in an aqueous diluent, said reconstituted composition having a final pH of 6.0 ± 0.3.

The disclosure further provides a liquid immunogenic ClfA composition manufactured by reconstituting a lyophilized immunogenic composition of the invention in an aqueous diluent, said reconstituted composition having a final pH of 6.5 ± 0.3.

The disclosure further provides a liquid immunogenic composition comprising: (a) the ClfA polypeptide at a concentration of between 20µg/ml ± 2µg/ml and 600µg/ml ± 60µg/ml; (b) the histidine buffer at 10mM ± 5mM; (c) polysorbate 80 at 0.1% ± 0.05% weight to volume (w/v); and (d) sucrose at a concentration of 9% ± 4.5% w/v. In another embodiment, the polysorbate 80 is at a concentration of 0.01% ± 0.005% w/v and the sucrose is at a concentration of 4.5% ± 1.5% w/v.

The disclosure further provides a process of making an immunogenic composition comprising the steps of: (a) combining in an aqueous solution, (i) a recombinant clumping factor A (rClfA) polypeptide, (ii) a buffer having a pKa of 6.0 ± 0.6, and (iii) a bulking agent, and (b) lyophilizing the combination of step (a) to form a cake comprising less than 3% water by weight. In certain embodiments, the process further comprises combining (vi) a surfactant at step (a). In certain embodiments, the process further comprises a step of filter sterilizing the combination of step (a) prior to lyophilization of step (b). In certain embodiments, the aqueous solution comprises 10mM ± 1mM histidine, pH 6.0 ± 0.5, sucrose at 9% ± 1% w/v and 80 at 0.1% ± 0.02% w/v. In other embodiments, the polysorbate 80 is at a concentration of 0.01% ± 0.001% w/v and the sucrose is at a concentration of 4.5% ± 0.45% w/v. In certain embodiments, the lyophilizing step (b) comprises the steps of: (i) freezing the sterilized combination of step (a) at a rate of 0.3°C ± 0.03°C per minute until reaching a temperature of -50°C ± 5°C at a pressure of 400 millibars ± 40 millibars; and then holding the combination at -50°C ± 5°C for 60 minutes ± 6 minutes; (ii) annealing the combination by increasing the temperature to -10°C ± 5°C at a rate of 0.3°C ± 0.03°C per minute; then holding the temperature at -10°C ± 5°C for 120 minutes ± 12 minutes; then decreasing the temperature at a rate of 0.3°C ± 0.03°C per minute until reaching a temperature of - 50°C ± 5°C; and then holding the temperature at -50°C ± 5°C for 180 minutes ± 18 minutes; (iii) drying the combination by decreasing the pressure to 50 mTorrs (mTorr) and increasing the temperature to -25°C ± 5°C at a rate of 0.2°C ± 0.02°C per minute; and then holding the temperature at 25°C ± 5°C for 1,320 minutes ± 132 minutes; (iv) drying the combination by increasing the temperature to 30°C ± 5°C at a rate of 0.2°C ± 0.02°C per minute; and then increasing the pressure to 200 mTorr and holding the temperature at 30°C ± 5°C for 720 minutes ± 72 minutes; (v) decreasing the temperature to 5°C ± 5°C at a rate of 0.5°C ± 0.05°C per minute. In some embodiments, lyophilization takes place in a vial. In some embodiments, the vial is stoppered after lyophilization. In certain embodiments, the vial is backfilled with nitrogen gas prior to stoppering the vial. In certain embodiments, the process further comprises the step of: (c) reconstituting the lyophilized combination of step (b) in an aqueous medium. In certain embodiments, the osmolality of the reconstituted combination of step (c) reconstituting the dried combination of step (b) in an aqueous medium, wherein the osmolality of the reconstituted combination is 300 mOsm ± 30 mOsm. In certain embodiments, the aqueous medium comprises at least two components selected from the group consisting of: (a) a *Staphylococcus aureus* clumping factor B (ClfB) polypeptide, (b) a Capsular Polysaccharide Type 5 (CP5)-protein conjugate, (c) a Capsular Polysaccharide Type 8 (CP8)-protein conjugate, and (d) a *Staphylococcus aureus* MntC polypeptide. In certain embodiments, the aqueous solution comprises a CP5-protein conjugate and a CP8-protein conjugate. In certain embodiments, the aqueous solution comprises a CP-5 protein conjugate, a CP8-protein conjugate, and an MntC polypeptide. In certain embodiments, an immunogenic composition of the disclosure is manufactured according to any process of making an immunogenic composition of the disclosure.

The ClfA polypeptide comprises an N1, N2 and N3 domain. In certain embodiments, the ClfA polypeptide comprises a fibrinogen binding domain. In certain embodiments, the fibrinogen binding domain has been altered so as to bind to fibrinogen at a reduced level compared to the binding observed to fibrinogen with the native fibrinogen binding domain of ClfA. In certain embodiments, the fibrinogen binding domain displays reduced binding to fibrinogen through having an amino acid substitution at one or more of Tyr 338, Tyr 256, Pro 336, Lys 389, Ala 254 and Ile 387. In certain embodiments, the amino acid substitution at one or more of Tyr 338, Tyr 256, Pro 336, Lys 389, Ala 254 and Ile 387 is to Ala or Ser. In certain embodiments, the Tyr 338 is substituted to Ala.

The CP5-protein conjugate is CP5-CRM₁₉₇. The CP8-protein conjugate is CP8-CRM₁₉₇.

In certain embodiments of the present disclosure, the buffer of the lyophilized immunogenic composition comprises succinate at pH 6.0 ± 0.3. In certain embodiments, the buffer comprises histidine at pH 6.5 ± 0.5. In certain embodiments, the buffer comprises histidine at pH 6.0 ± 0.3. In certain embodiments, the bulking agent is selected from the group consisting of sucrose, trehalose, mannitol, glycine and sorbitol. In certain embodiments, the bulking agent is sucrose. In certain embodiments, the bulking agent is sucrose at 96% ± 0.2% w/w. In certain embodiments, the lyophilized immunogenic composition further comprises a surfactant. In certain embodiments, the surfactant is selected from the group consisting of a poloxamer, a polyoxyethylene alkyl ether and a polyoxyethylene sorbitan fatty acid ester. In certain embodiments, the polyoxyethylene sorbitan fatty acid ester is polysorbate 80. In certain embodiments, the polysorbate 80 is at 0.20% ± 0.041% w/w for ClfA and tri-antigen formulations. In certain embodiments, the polysorbate 80 is at 0.1% ± 0.05% w/w for tetra-antigen formulations. In other embodiments, the polysorbate 80 is at a concentration of 0.01% ± 0.005% w/v.

In certain embodiments of the present disclosure, the lyophilized immunogenic composition further comprises an adjuvant. In certain embodiments, the adjuvant is ISCOMATRIX™.

In certain embodiments, the aqueous diluent of the liquid composition is water. In certain embodiments, the aqueous diluent is a low salt solution. In certain embodiments, the low salt solution comprises 60mM ± 10mM sodium chloride. In certain embodiments, the aqueous diluent comprises polysorbate 80. In certain embodiments, the aqueous diluent comprises an adjuvant. In certain embodiments, the adjuvant is ISCOMATRIX™.

### Brief Description of the Drawings

Figure 1 depicts a schematic diagram of the various forms of recombinant ClfA polypeptide and discloses SEQ ID NOs: 125 and 127-129.
Figure 2 depicts the change in concentration of various lots of lyophilized ClfA protein compared to their liquid counterparts, expressed as ln[C], over time under various conditions. Panel A: lot nos. L36051-37-1 and L36051-44. Panel B: lot no. L36051-72. Panel C: lot no. L36051-81-1. Panel D: lot no. 36051-81-2.
Figure 3 depicts size exclusion chromatograms of lyophilized DS L40184-61 at t = 0 compared to cakes stored at 2-8°C, 25°C, and 37°C for three months.
Figure 4 depicts size exclusion chromatograms comparing lyophilized formulation to pre-lyophilized liquid ClfA lot L36051-81-1.
Figure 5 depicts histograms of pH (panel A), percent moisture (panel B) and optical density (panel C), which is a measure of aggregation, of lyophilized ClfA at various times and temperature conditions.
Figure 6 depicts histograms of percent dimer (panel A), percent monomer (panel B), and percent degradant (cleavage) (panel C) of ClfA, as determined by SE-HPLC after 24 hours of agitation at room temperature. X-axis depicts formulations containing different concentrations of polysorbate 80 (0.000, 0.0100, 0.005 and 0.100% w/w) and sucrose (3, 4.5 and 6% w/w). The formulation containing no PS80 exhibited an increase in percent dimer formation after agitation (panel D).
Figure 7 depicts histograms of percent degradant (cleavage) (panel A), percent dimer (panel B), and percent monomer (panel C) of ClfA as determined by SE-HPLC for various bulking agent formulations over time.
Figure 8 depicts the purity of ClfA (panel A), strength (antigenicity) of CP5 (panel B), and strength (antigenicity) of CP8 at various times after reconstitution of high dose lyophilized cakes.
Figure 9 depicts the purity of ClfA (panel A), strength (antigenicity) of CP5 (panel B), and strength (antigenicity) of CP8 at various times after reconstitution of low dose lyophilized cakes.
Figure 10 depicts the concentration (panel A) and percent purity (panel B) of ClfA and the concentration of CP5 and CP8 (panel C) at 0, 4 and 24 hours at room temperature post-reconstitution.
Figure 11 depicts the concentration (panel A) and percent purity (panel B) of ClfA and the concentration of CP5 and CP8 (panel C) after 1, 2 and 3 freeze-thaw cycles.
Figure 12 depicts circular dichroism (CD) scans of multiple lots of rClfA at pH 6.0 (panel A) and 7.0 (panel B)
Figure 13 depicts CD melts of multiple lots of rClfA as a function of pH.
Figure 14 depicts intrinsic tryptophan fluorescence (panel A) and ANS fluorescence (panel B) melts as a function of pH for rClfA*m* and r*m*ClfA.
Figure 15 depicts differential scanning calorimetry (DSC) melts as a function of pH for rClfA*m* and r*m*ClfA.
Figure 16 depicts OD₃₅₀ melts of r*m*ClfA as a function of pH.
Figure 17 depicts size exclusion-HPLC as a function of pH for r*m*ClfA for six weeks at 25°C (panel A) and pH and OD₃₅₀ stability for liquid r*m*ClfA formulations (panel B).
Figure 18 depicts intrinsic tryptophan fluorescence melts for MntC (panel A) and tracking of Tₘ1 (panel B).
Figure 19 depicts tracking of Tₘ1 from DSC melts as a function of pH for MntC (panel A) and thermograms of two main thermal events (panels B and C).
Figure 20 depicts OD₃₅₀ melts of MntC as a function of pH.
Figure 21 depicts chromatograms of r*m*ClfA (panel A) and MntC (panel B) for monitoring stability.
Figure 22 depicts stability results of rmClfA with RP-HPLC at 5°C (panel A) and 25°C (panel B) and of MntC with IEX-HPLC at 5°C (panel C) and 25°C (panel D).
Figure 23 depicts the stability results of CP5-CRM₁₉₇ at 5°C and 25°C (panels A and C, respectively) and CP8-CRM₁₉₇ at 5°C and 25°C (panels B and D, respectively) at different pHs using nephelometry.
Figure 24 depicts osmolality (panel A) and OD₃₅₀ (panel B) measurements of post-rocked samples with varying bulking agents.
Figure 25 depicts stability testing as a function of bulking agent using RP-HPLC data for high dose (panel A) and low dose (panel B) lyophilized formulations and IEX-HPLC data for MntC quality (panel C).
Figure 26 depicts post-agitation data of all four antigens: antigen concentrations (rmClfA and MntC in panel A; CP5-CRM₁₉₇ and CP8-CRM₁₉₇ in panel B), purity of r*m*ClfA and MntC by RP-HPLC (panel B), purity of MntC by IEX-HPLC (panel C), pH (panel D) and OD₃₅₀ (panel E).
Figure 27 depicts pre-lyophilization and post- lyophilization data of all four antigens: RP-HPLC data for r*m*ClfA and MntC (panel A), nephelometry results for CP5-CRM₁₉₇ (panel B) and CP8-CRM₁₉₇ (panel C), CEX-HPLC data for MntC using 4.5% sucrose (panel D) and 4% mannitol/1% sucrose (panel E), DSC data (panel F) and percent potency of r*m*ClfA (panel G) and conjugates (panel H).
Figure 28 depicts the osmolality of high and low dose formulations.
Figure 29 depicts RP-HPLC purity data for MntC (panel A) and r*m*ClfA (panel B), IEX-HPLC purity data for MntC (panel C), and post-reconstitution kinetics of MntC (panel D) and r*m*ClfA degradation (panel E).
Figure 30 depicts post-reconstitution antigen concentration (panels A-D) and pH stability (panel E).
Figure 31 depicts post-lyophilization process recoveries of antigen concentration (panels A and B) and purity (panels C and D).
Figure 32 depicts post-reconstitution r*m*ClfA (panel A) and MntC purity (panel B), r*m*ClfA (panel C) and MntC concentration (panel D), CP5-CRM₁₉₇ (panel E) and CP8-CRM₁₉₇ (panel F) concentration, and pH stability (panel G).
Figure 33 depicts post-freeze thaw r*m*ClfA and MntC purity by RP-HPLC (panel A), MntC purity by IEX-HPLC (panel B), concentrations of antigens (panels C and D) and pH stability (panel E).
Figure 34 depicts post-agitation r*m*ClfA and MntC purity by RP-HPLC (panel A), MntC purity by IEX-HPLC (panel B), concentrations of antigens (panels C and D), pH (panel E) and OD₃₅₀ (panel F).
Figure 35 depicts the degradation of MntC reconstituted with ISCOMATRIX™ at 2-8°C (panel A) and 25°C (panel B) analyzed by CEX-HPLC after 24 hours, the purity of r*m*ClfA reconstituted with ISCOMATRIX™ analyzed by CEX-HPLC (panels C and D), the purity of MntC reconstituted with ISCOMATRIX™ at 2-8°C (panel E) and 25°C (panel F) analyzed by RP-HPLC, the CP5-CRM₁₉₇ and CP8-CRM₁₉₇ conjugate concentrations with ISCOMATRIX™ at 2-8°C (panel G) and 25°C (panel H), and r*m*ClfA and MntC concentrations with ISCOMATRIX™ at 2-8°C (panel I) and 25°C (panel J).
Figure 36 depicts ISCOMATRIX™ concentrations over 24 hours at 2-8°C (panel A) and 25°C (panel B), particle size of ISCOMATRIX™ (panel C), and pH stability (panel D) of reconstituted formulations.
Figure 37 depicts the purity (panel A) and deamidation of MntC reconstituted with ISCOMATRIX™ analyzed by RP-HPLC after 4 hours and antigen concentrations with ISCOMATRIX™ (panels C and D).
Figure 38 depicts the particle size of ISCOMATRIX™ (panel A) and pH stability (panel B) of reconstituted formulations over 4 hours.

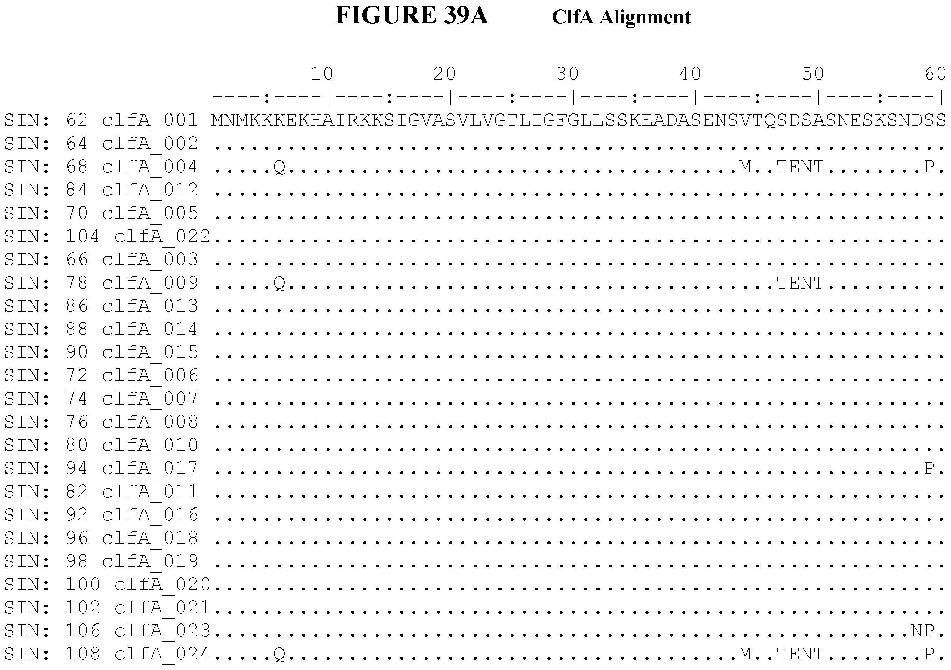

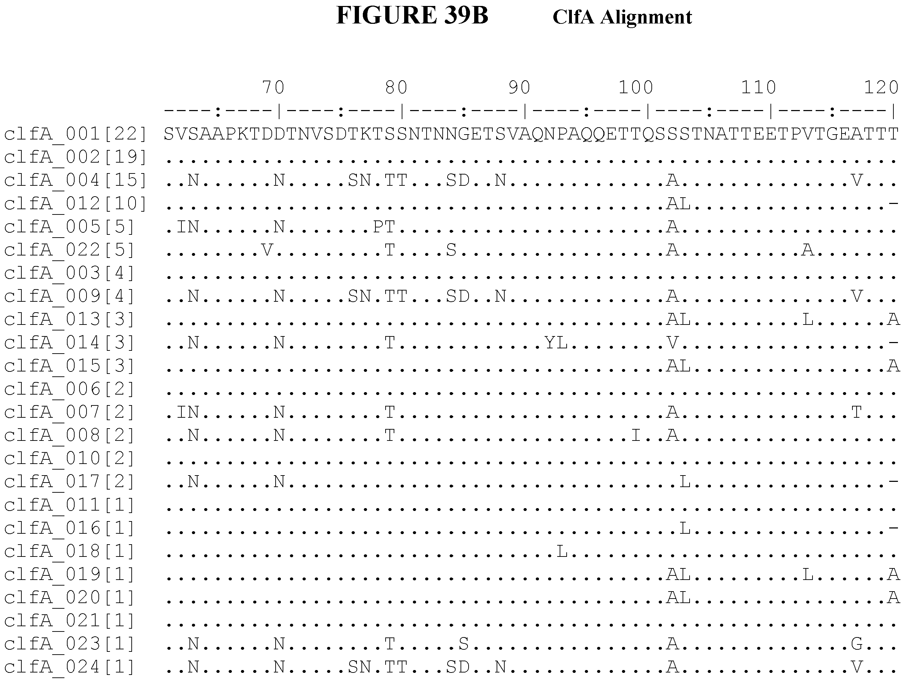

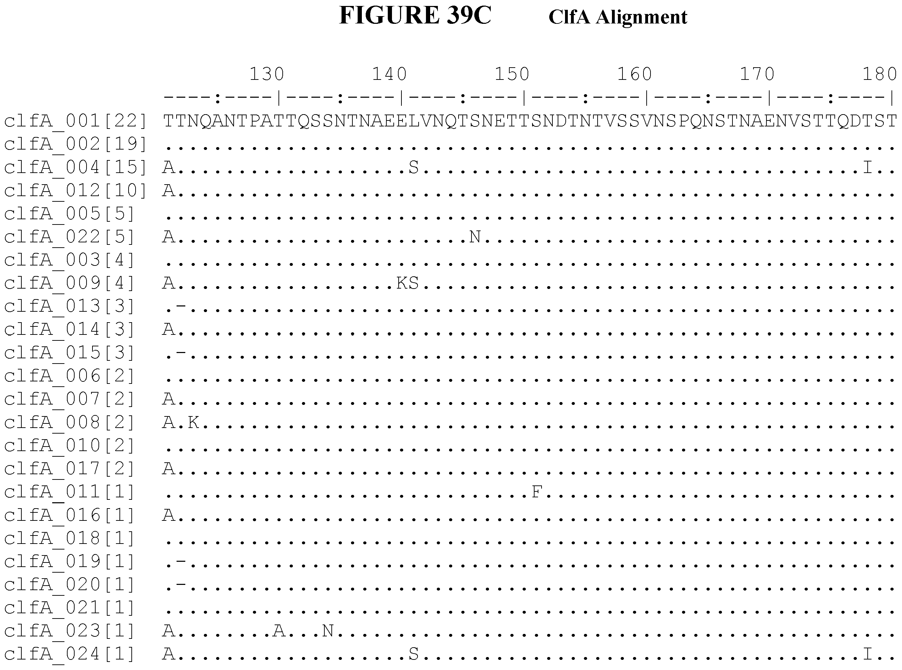

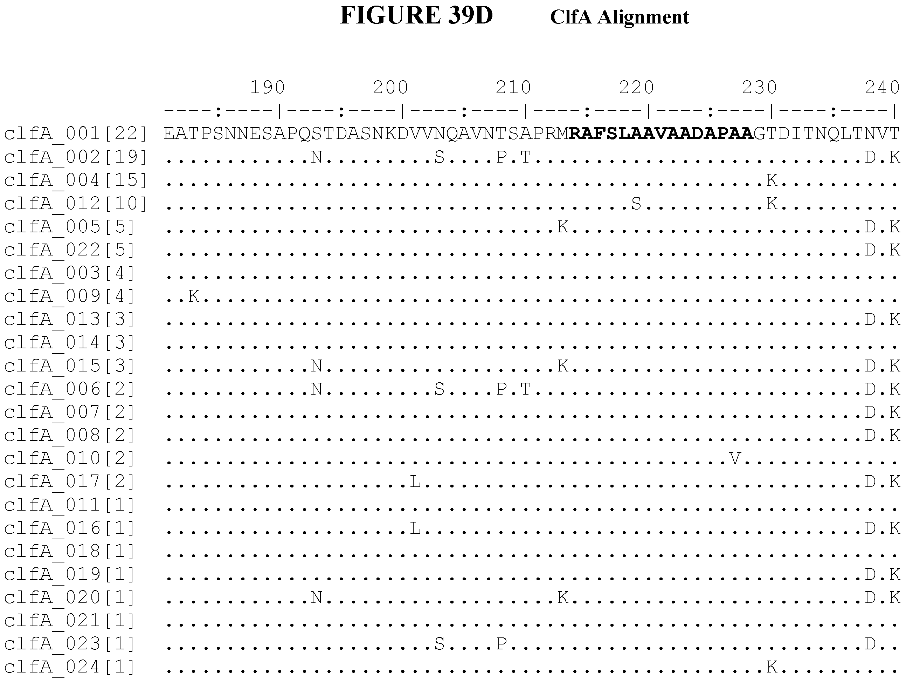

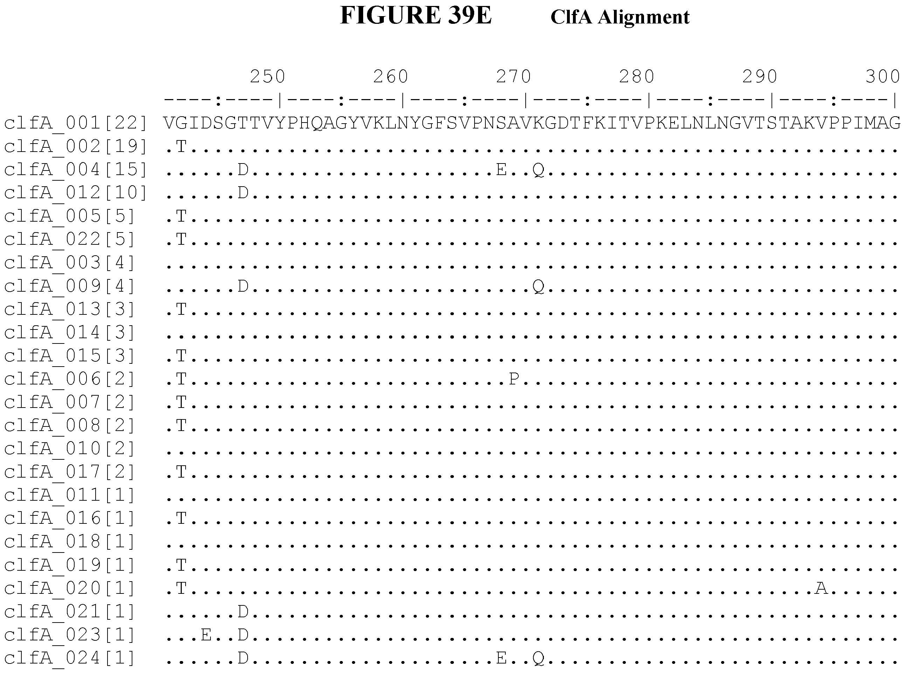

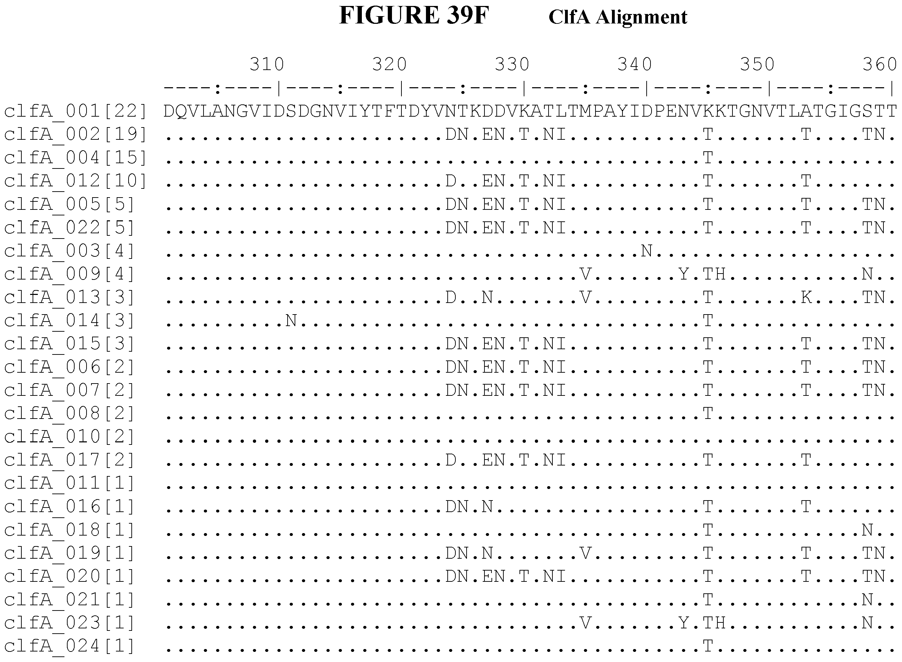

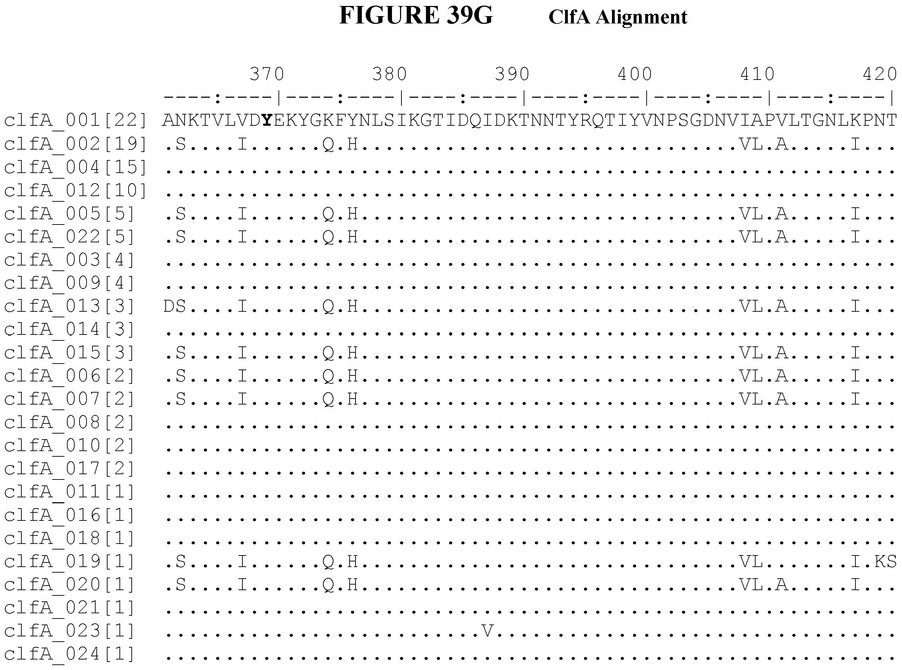

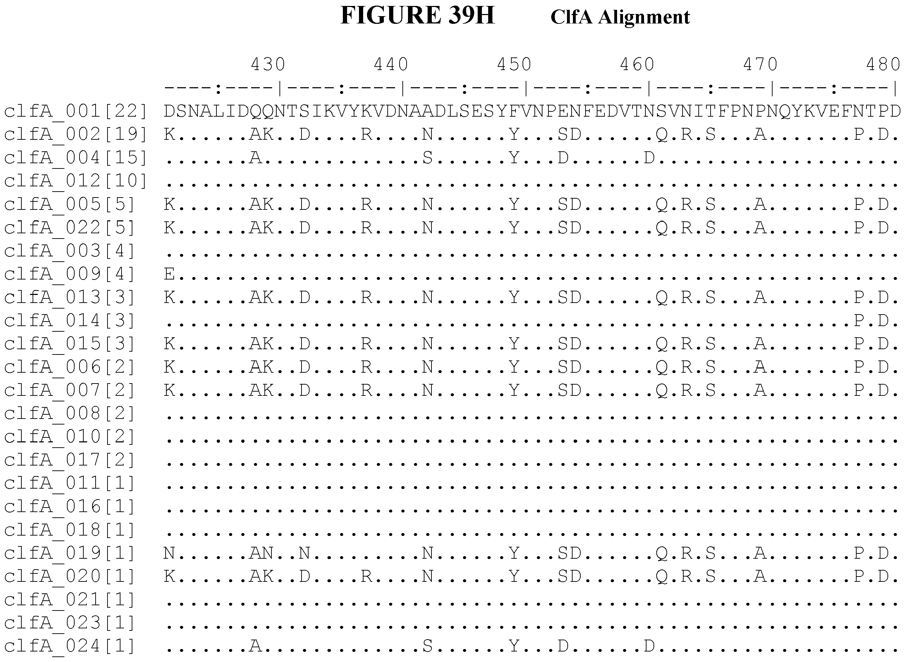

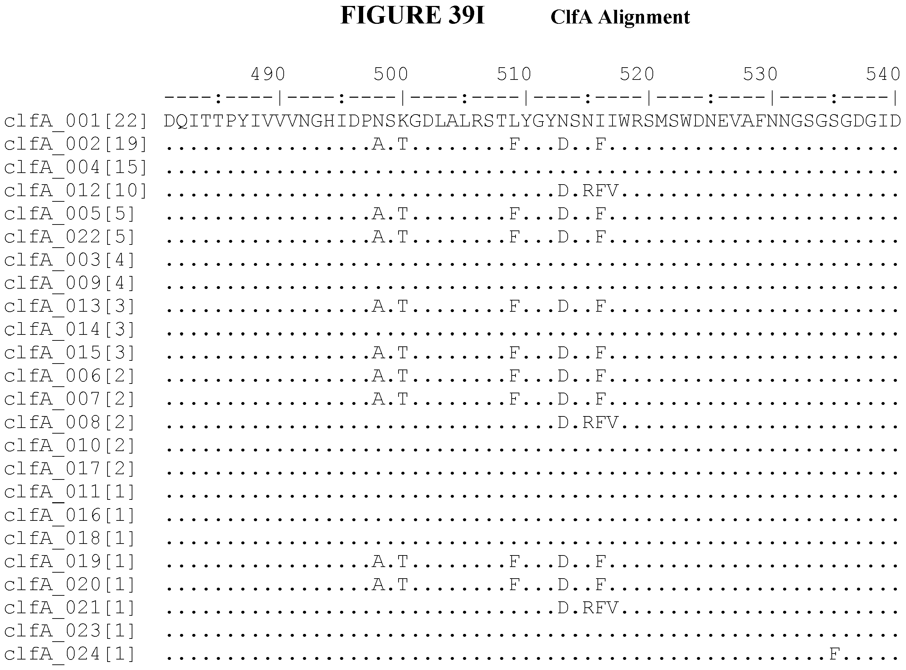

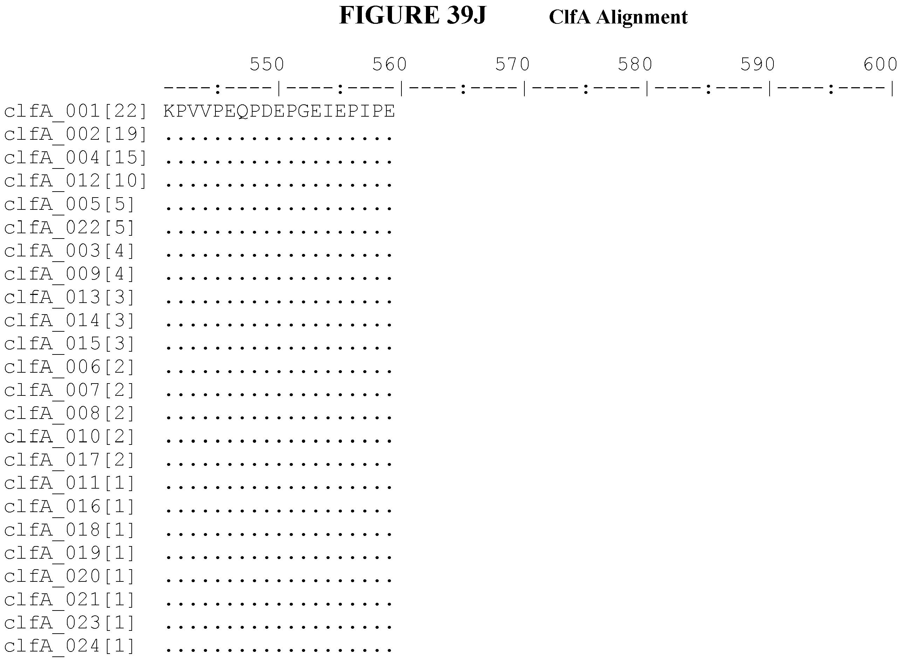

Figures 39A-J depict the alignment of ClfA between various strains of *S. aureus* (SEQ ID NOs: 62, 64, 68, 84, 70, 104, 66, 78, 86, 88, 90, 72, 74, 76, 80, 94, 82, 92, 96, 98, 100, 102, 106, and 108, respectively, in order of appearance).

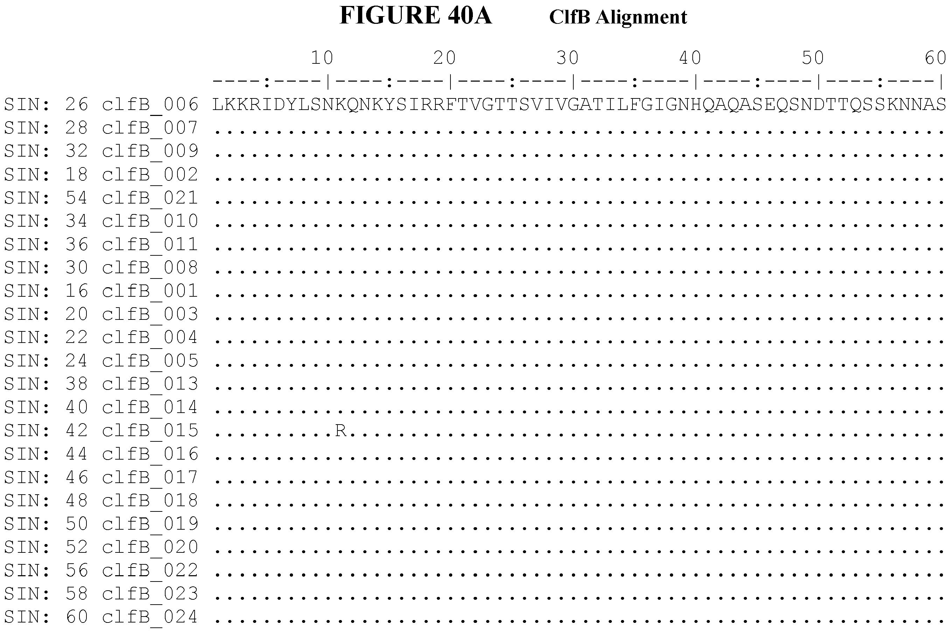

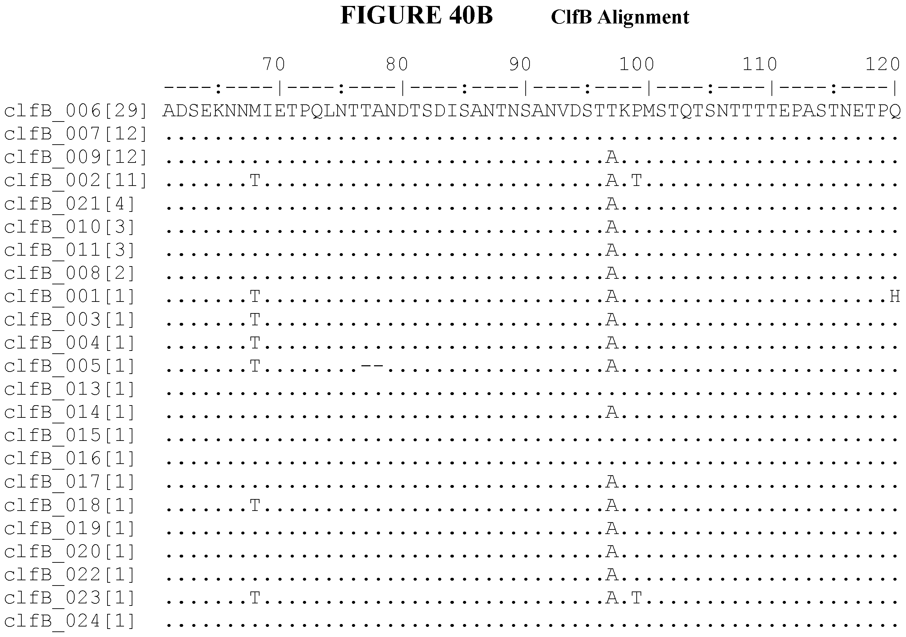

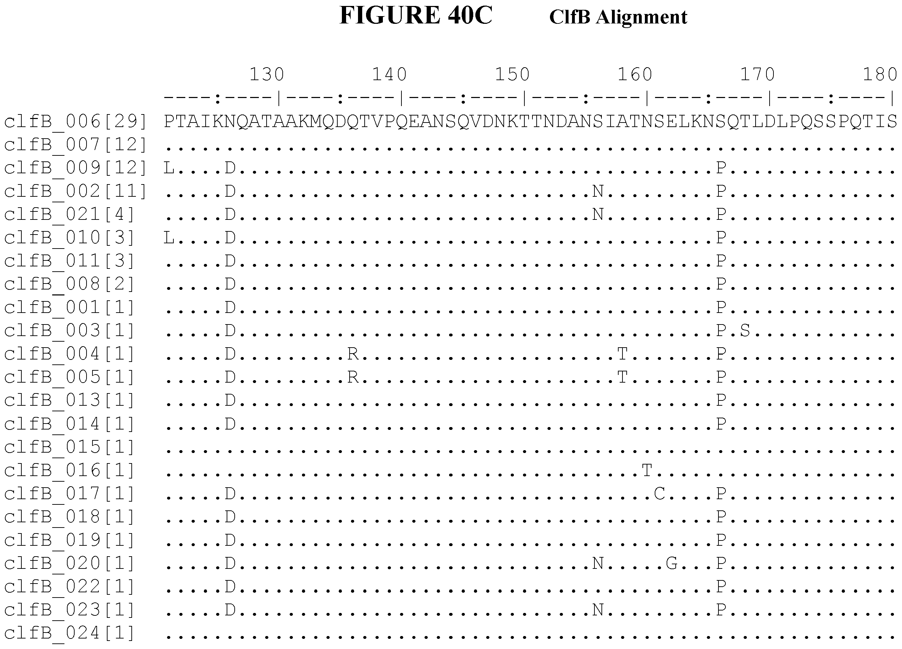

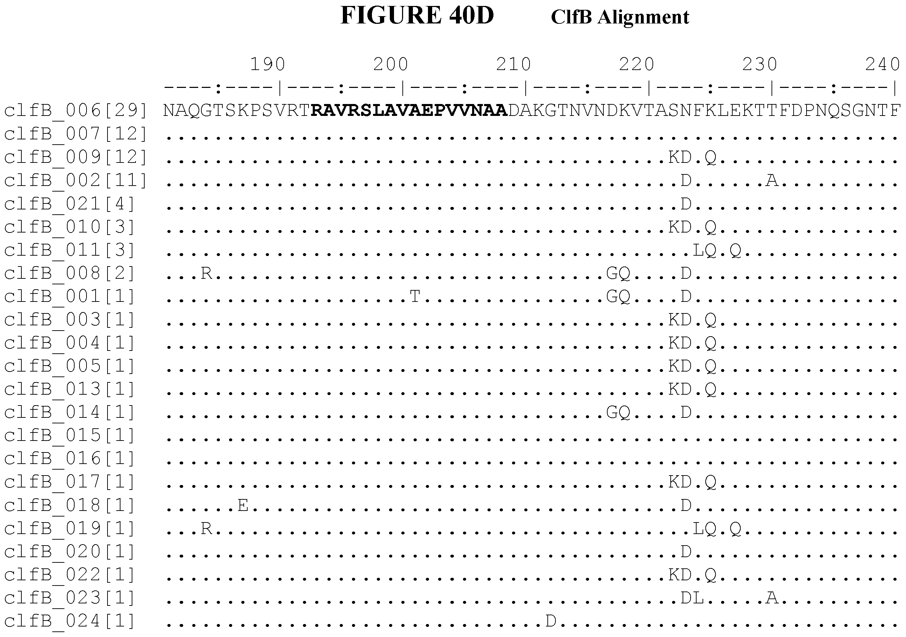

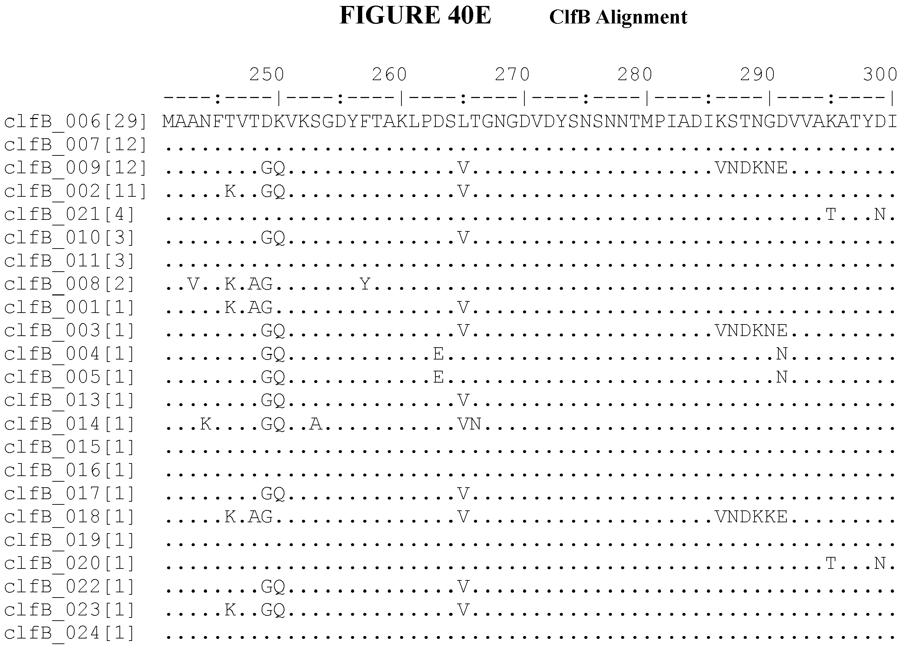

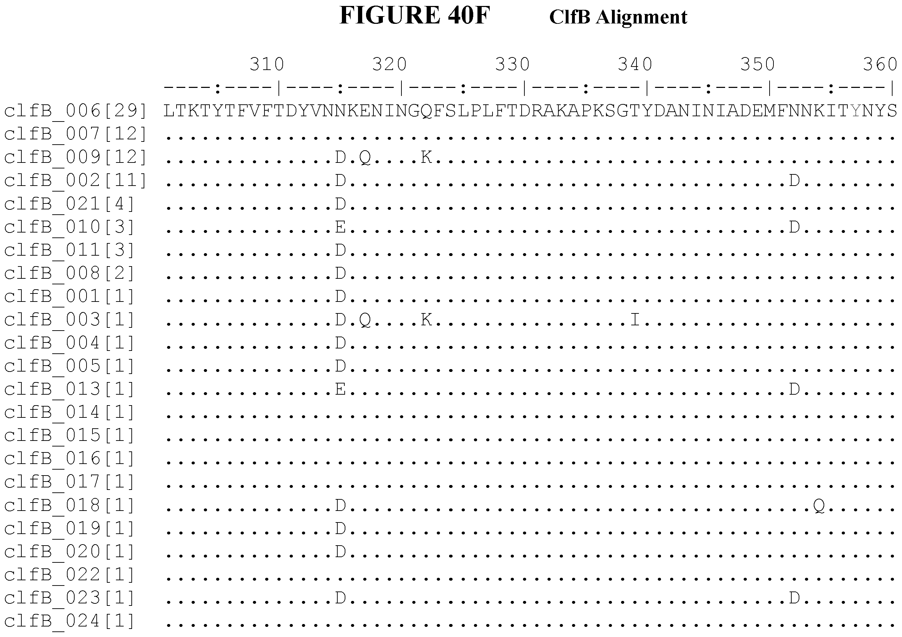

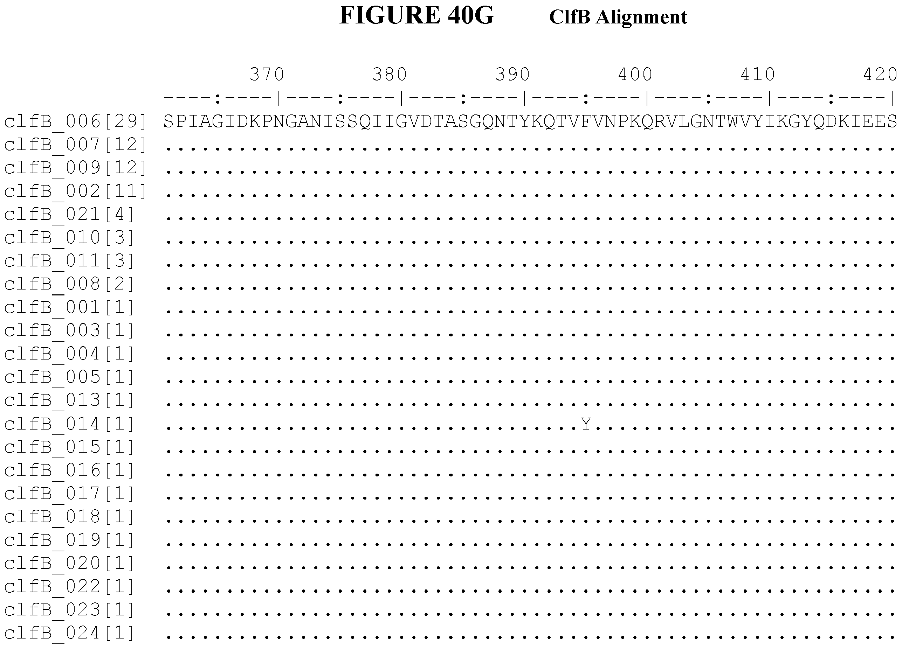

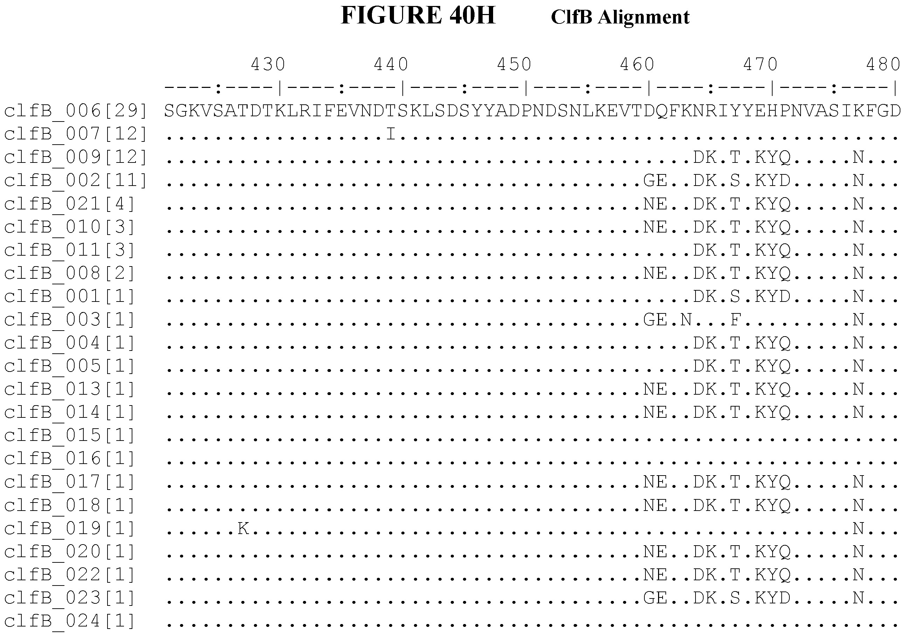

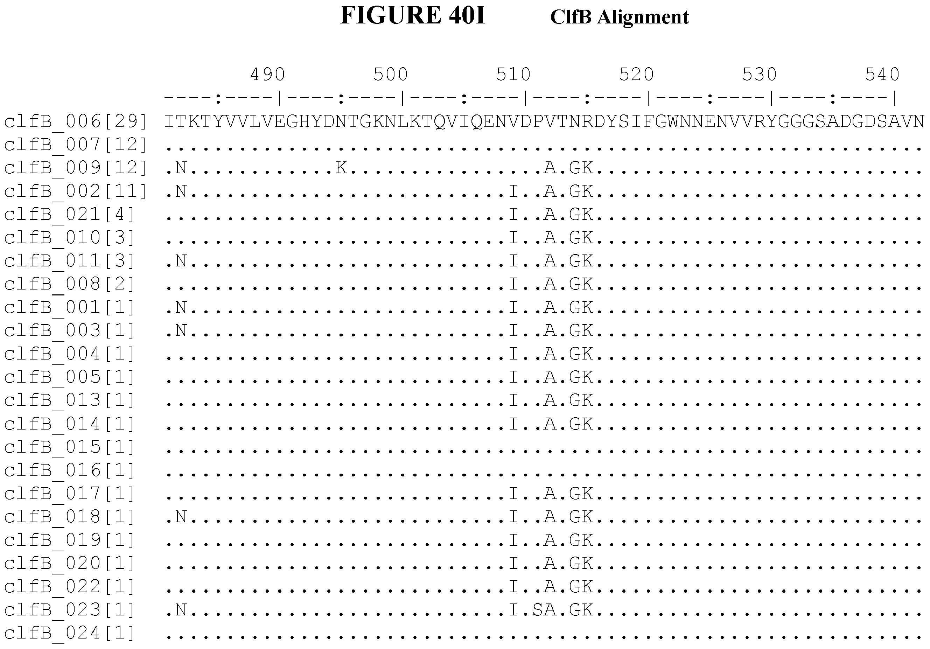

Figures 40A-I depict the alignment of ClfB between various strains of *S. aureus* (SEQ ID NOs: 26, 28, 32, 18, 54, 34, 36, 30, 16, 20, 22, 24, 38, 40, 42, 44, 46, 48, 50, 52, 56, 58, and 60, respectively, in order of appearance).

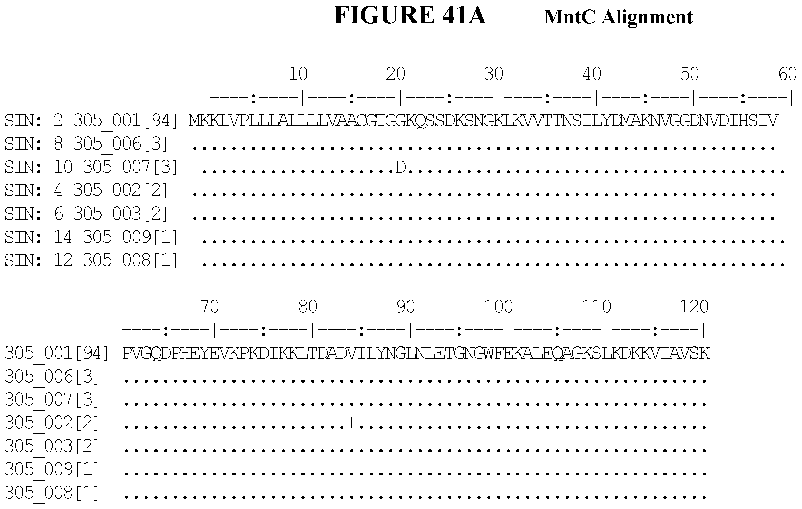

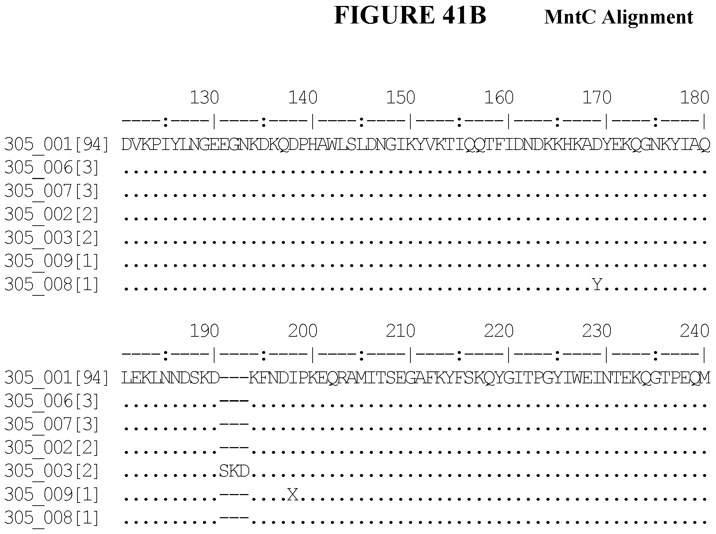

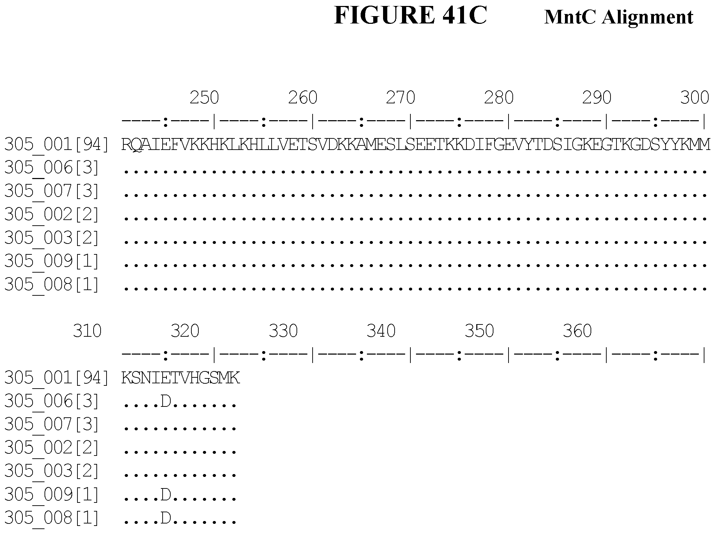

Figure 41A-C depict the alignment of MntC between various strains of *S. aureus* (SEQ ID NOs: 2, 8, 10, 4, 6, 14 and 12, respectively, in order of appearance).
Figure 42 depicts a chart of ClfB sequences from different strains of *S. aureus.*
Figure 43 depicts a chart of MntC sequences from different strains of *S. aureus.*

### Detailed Description of the Invention

Although any methods and materials similar or equivalent to those described herein can be used the preferred methods and materials are now described.

The terms used herein have the meanings recognized and known to those of skill in the art, however, for convenience and completeness, particular terms and their meanings are set forth below.

As used in this specification and the appended claims, the singular forms "a", "an", and "the" include plural references unless the context clearly dictates otherwise. Thus, for example, references to "the method" includes one or more methods, and/or steps of the type described herein and/or which will become apparent to those persons skilled in the art upon reading this disclosure and so forth.

The term "about" or "approximately" means within a statistically meaningful range of a value. Such a range can be within an order of magnitude, typically within 20%, more typically still within 10%, and even more typically within 5% of a given value or range. The allowable variation encompassed by the term "about" or "approximately" depends on the particular system under study, and can be readily appreciated by one of ordinary skill in the art. Whenever a range is recited within this application, every whole number integer within the range is also contemplated as an embodiment of the invention.

An "antibody" is an immunoglobulin molecule capable of specific binding to a target, such as a carbohydrate, polynucleotide, lipid, polypeptide, etc., through at least one antigen recognition site, located in the variable region of the immunoglobulin molecule. As used herein, unless otherwise indicated by context, the term is intended to encompass not only intact polyclonal or monoclonal antibodies, but also engineered antibodies (e.g., chimeric, humanized and/or derivatized to alter effector functions, stability and other biological activities) and fragments thereof (such as Fab, Fab', F(ab')2, Fv), single chain (ScFv) and domain antibodies, including shark and camelid antibodies), and fusion proteins comprising an antibody portion, multivalent antibodies, multispecific antibodies (e.g., bispecific antibodies so long as they exhibit the desired biological activity) and antibody fragments as described herein, and any other modified configuration of the immunoglobulin molecule that comprises an antigen recognition site. An antibody includes an antibody of any class, such as IgG, IgA, or IgM (or sub-class thereof), and the antibody need not be of any particular class. Depending on the antibody amino acid sequence of the constant domain of its heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA1 and IgA2 in humans. The heavy-chain constant domains that correspond to the different classes of immunoglobulins are called alpha, delta, epsilon, gamma, and mu, respectively. The subunit structures and three-dimensional configurations of different classes of immunoglobulins are well known.

"Antibody fragments" comprise only a portion of an intact antibody, wherein the portion preferably retains at least one, preferably most or all, of the functions normally associated with that portion when present in an intact antibody.

The term "antigen" generally refers to a biological molecule, usually a protein, peptide, polysaccharide, lipid or conjugate which contains at least one epitope to which a cognate antibody can selectively bind; or in some instances to an immunogenic substance that can stimulate the production of antibodies or T-cell responses, or both, in an animal, including compositions that are injected or absorbed into an animal. The immune response may be generated to the whole molecule, or to one or more various portions of the molecule (*e.g*., an epitope or hapten). The term may be used to refer to an individual molecule or to a homogeneous or heterogeneous population of antigenic molecules. An antigen is recognized by antibodies, T-cell receptors or other elements of specific humoral and/or cellular immunity. The term "antigen" includes all related antigenic epitopes. Epitopes of a given antigen can be identified using any number of epitope mapping techniques, well known in the art. See, *e.g.,* Epitope Mapping Protocols in Methods in Molecular Biology, Vol. 66 (Glenn E. Morris, Ed., 1996) Humana Press, Totowa, N. J. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, *e.g.,* U.S. Pat. No. 4,708,871; Geysen et al. (1984) Proc. Natl. Acad. Sci. USA 81:3998-4002; Geysen et al. (1986) Molec. Immunol. 23:709-715. Similarly, conformational epitopes maybe identified by determining spatial conformation of amino acids such as by, *e.g.,* x-ray crystallography and 2-dimensional nuclear magnetic resonance. See, *e.g.,* Epitope Mapping Protocols, *supra.* Furthermore, for purposes of the present invention, an "antigen" may also be used to refer to a protein that includes modifications, such as deletions, additions and substitutions (generally conservative in nature, but they may be non-conservative), to the native sequence, so long as the protein maintains the ability to elicit an immunological response. These modifications may be deliberate, as through site-directed mutagenesis, or through particular synthetic procedures, or through a genetic engineering approach, or may be accidental, such as through mutations of hosts, which produce the antigens. Furthermore, the antigen can be derived, obtained, or isolated from a microbe, e.g. a bacterium, or can be a whole organism. Similarly, an oligonucleotide or polynucleotide, which expresses an antigen, such as in nucleic acid immunization applications, is also included in the definition. Synthetic antigens are also included, for example, polyepitopes, flanking epitopes, and other recombinant or synthetically derived antigens (Bergmann et al. (1993) Eur. J. Immunol. 23:2777 2781; Bergmann et al. (1996) J. Immunol. 157:3242 3249; Suhrbier, A. (1997) Immunol. and Cell Biol. 75:402 408; Gardner et al. (1998) 12th World AIDS Conference, Geneva, Switzerland, Jun. 28 - Jul. 3, 1998).

The term "adjuvant" refers to a compound or mixture that enhances the immune response to an antigen as further described and exemplified herein.

"Bacteremia" is a transient presence of bacteria in the blood. A bacteremia can progress to septicemia, or sepsis, which would be considered an infection and is the persistent presence of bacteria in the blood with associated clinical signs/symptoms. Not all bacteria are capable of surviving in the blood. Those that do have special genetic traits that provide for that ability. Also, the host factors play an important role as well.

"Capsular polysaccharide" or "capsule polysaccharide" refers to the polysaccharide capsule that is external to the cell wall of most isolates of Staphylococci. For example, *S. aureus* includes a cell wall component composed of a peptidoglycan complex, which enables the organism to survive under unfavorable osmotic conditions and also includes a unique teichoic acid linked to the peptidoglycan. External to the cell wall a thin polysaccharide capsule coats most isolates of *S. aureus.* This serologically distinct capsule can be used to serotype various isolates of *S. aureus.* Many of the clinically significant isolates have been shown to include two capsular types: serotype 5 (CP5) and serotype 8 (CP8).

As used herein, "conjugates" comprise a capsule polysaccharide usually of a desired range of molecular weight and a carrier protein, wherein the capsule polysaccharide is conjugated to the carrier protein. Conjugates may or may not contain some amount of free capsule polysaccharide. As used herein, "free capsule polysaccharide" refers to capsule polysaccharide that is non-covalently associated with (i.e., non-covalently bound to, adsorbed to or entrapped in or with) the conjugated capsular polysaccharide-carrier protein. The terms "free capsule polysaccharide," "free polysaccharide" and "free saccharide" may be used interchangeably and are intended to convey the same meaning. Regardless of the nature of the carrier molecule, it can be conjugated to the capsular polysaccharide either directly or through a linker. As used herein, "to conjugate", "conjugated" and "conjugating" refers to a process whereby a bacterial capsular polysaccharide is covalently attached to the carrier molecule. Conjugation enhances the immunogenicity of the bacterial capsular polysaccharide. The conjugation can be performed according to the methods described below or by processes known in the art.

As described above, the present disclosure relates to conjugates comprising *S. aureus* serotype 5 capsular polysaccharides (CP5) conjugated to carrier proteins and conjugates comprising *S. aureus* serotype 8 capsular polysaccharides (CP8) conjugated to carrier proteins. One embodiment of the disclosure provides conjugates comprising a *S. aureus* serotype 5 capsular polysaccharide conjugated to a carrier protein and a *S. aureus* serotype 8 capsular polysaccharide conjugated to a carrier protein wherein: the type 5 capsular polysaccharide has a molecular weight of between 50 kDa and 800 kDa; the type 8 capsular polysaccharide has a molecular weight of between 50 and 700 kDa; the immunogenic conjugates have molecular weights of between about 1000 kDa and about 5000 kDa; and the conjugates comprise less than about 30% free polysaccharide relative to total polysaccharide. In one embodiment, the conjugates comprise less than about 25%, about 20%, about 15%, about 10%, or about 5% free polysaccharide relative to total polysaccharide. In one embodiment, the type 5 or 8 polysaccharide has a molecular weight between 20 about kDa and about 1000 kDa; between about 50 kDa and about 500 kDa; between about 50 kDa and about 200 kDa; and between about 75 kDa and about 150 kDa.

In one embodiment, the conjugate has a molecular weight of between about 50 kDa and about 5000 kDa. In one embodiment, the conjugate has a molecular weight of between about 200 kDa and about 5000 kDa. In one embodiment, the immunogenic conjugate has a molecular weight of between about 400 kDa and about 2500 kDa. In one embodiment, the immunogenic conjugate has a molecular weight of between about 500 kDa and about 2500 kDa. In one embodiment, the immunogenic conjugate has a molecular weight of between about 600 kDa and about 2800 kDa. In one embodiment, the immunogenic conjugate has a molecular weight of between about 700 kDa and about 2700 kDa. In one embodiment, the immunogenic conjugate has a molecular weight of between about 1000 kDa and about 2000 kDa; between about 1800 kDa and about 2500 kDa; between about 1100 kDa and about 2200 kDa; between about 1900 kDa and about 2700 kDa; between about 1200 kDa and about 2400 kDa; between about 1700 kDa and about 2600 kDa; between about 1300 kDa and about 2600 kDa; between about 1600 kDa and about 3000 kDa.

The carrier protein within the immunogenic conjugate is CRM₁₉₇, and the CRM₁₉₇ is covalently linked to the capsular polysaccharide via a carbamate linkage, an amide linkage, or both. The number of lysine residues in the carrier protein that become conjugated to a capsular polysaccharide can be characterized as a range of conjugated lysines. For example, in a given immunogenic composition, the CRM₁₉₇ may comprise 5 to 15 lysines out of 39 covalently linked to the capsular polysaccharide. Another way to express this parameter is that 12% to 40% of CRM₁₉₇ lysines are covalently linked to the capsular polysaccharide. In some embodiments, the CRM₁₉₇ portion of the polysaccharide covalently bound to the CRM₁₉₇ comprises 5 to 25 lysines covalently linked to the polysaccharide. In some embodiments, the CRM₁₉₇ portion of the polysaccharide covalently bound to the CRM₁₉₇ comprises 5 to 20 lysines covalently linked to the polysaccharide. In some embodiments, the CRM₁₉₇ portion of the polysaccharide covalently bound to carrier protein of comprises 10 to 25 lysines covalently linked to the polysaccharide. In some embodiments, the CRM₁₉₇ portion of the polysaccharide covalently bound to carrier protein of comprises 8 to 15 lysines covalently linked to the polysaccharide. For example, in a given immunogenic composition, the CRM₁₉₇ may comprise 18 to 22 lysines out of 39 covalently linked to the capsular polysaccharide. Another way to express this parameter is that 40% to 60% of CRM₁₉₇ lysines are covalently linked to the capsular polysaccharide. In some embodiments, the CRM₁₉₇ comprises 5 to 15 lysines out of 39 covalently linked to CP8. Another way to express this parameter is that 12% to 40% of CRM₁₉₇ lysines are covalently linked to CP8. In some embodiments, the CRM₁₉₇ comprises 18 to 22 lysines out of 39 covalently linked to CP5. Another way to express this parameter is that 40% to 60% of CRM₁₉₇ lysines are covalently linked to CP5.

As discussed above, the number of lysine residues in the carrier protein conjugated to the capsular polysaccharide can be characterized as a range of conjugated lysines, which may be expressed as a molar ratio. For example, the molar ratio of conjugated lysines to CRM₁₉₇ in the CP8 immunogenic conjugate can be between about 18:1 to about 22:1. In one embodiment, the range of molar ratios of conjugated lysines to CRM₁₉₇ in the CP8 immunogenic conjugate can be between about 15:1 to about 25:1. In some embodiments, the range of molar ratios of conjugated lysines to CRM₁₉₇ in the CP8 immunogenic conjugate can be between about 14:1 to about 20:1; about 12:1 to about 18:1; about 10:1 to about 16:1; about 8:1 to about 14:1; about 6:1 to about 12:1; about 4:1 to about 10:1; about 20:1 to about 26:1; about 22:1 to about 28:1; about 24:1 to about 30:1; about 26:1 to about 32:1; about 28:1 to about 34:1; about 30:1 to about 36:1; about 5:1 to about 10:1; about 5:1 to about 20:1; about 10:1 to about 20:1; or about 10:1 to about 30:1. Also, the molar ratio of conjugated lysines to CRM₁₉₇ in the CP5 immunogenic conjugate can be between about 3:1 and 25:1. In one embodiment, the range of molar ratio of conjugated lysines to CRM₁₉₇ in the CP5 immunogenic conjugate can be between about 5:1 to about 20:1. In one embodiment, the range of molar ratio of conjugated lysines to CRM₁₉₇ in the CP5 immunogenic conjugate can be between about 4:1 to about 20:1; about 6:1 to about 20:1; about 7:1 to about 20:1; about 8:1 to about 20:1; about 10:1 to about 20:1; about 11:1 to about 20:1; about 12:1 to about 20:1; about 13:1 to about 20:1; about 14:1 to about 20:1; about 15:1 to about 20:1; about 16:1 to about 20:1; about 17:1 to about 20:1; about 18:1 to about 20:1; about 5:1 to about 18:1; about 7:1 to about 16:1; or about 9:1 to about 14:1.

Another way to express the number of lysine residues in the carrier protein conjugated to the capsular polysaccharide can be as a range of conjugated lysines. For example, in a given CP8 immunogenic conjugate, the CRM₁₉₇ may comprise 5 to 15 lysines out of 39 covalently linked to the capsular polysaccharide. Alternatively, this parameter can be expressed as a percentage. For example, in a given CP8 immunogenic conjugate, the percentage of conjugated lysines can be between 10% and 50%. In some embodiments, 20% to 50% of lysines can be covalently linked to CP8. Alternatively still, 30% to 50% of CRM₁₉₇ lysines can be covalently linked to the CP8; 10% to 40% of CRM₁₉₇ lysines; 10% to 30% of CRM₁₉₇ lysines; 20% to 40% of CRM₁₉₇ lysines; 25% to 40% of CRM₁₉₇ lysines; 30% to 40% of CRM₁₉₇ lysines; 10% to 30% of CRM₁₉₇ lysines; 15% to 30% of CRM₁₉₇ lysines; 20% to 30% of CRM₁₉₇ lysines; 25% to 30% of CRM₁₉₇ lysines; 10% to 15% of CRM₁₉₇ lysines; or 10% to 12% of CRM₁₉₇ lysines are covalently linked to CP8. Also, in a given CP5 immunogenic conjugate, the CRM₁₉₇ may comprise 18 to 22 lysines out of 39 covalently linked to the capsular polysaccharide. Alternatively, this parameter can be expressed as a percentage. For example, in a given CP5 immunogenic conjugate, the percentage of conjugated lysines can be between 40% and 60%. In some embodiments, 40% to 60% of lysines can be covalently linked to CP5. Alternatively still, 30% to 50% of CRM₁₉₇ lysines can be covalently linked to CP5; 20% to 40% of CRM₁₉₇ lysines; 10% to 30% of CRM₁₉₇ lysines; 50% to 70% of CRM₁₉₇ lysines; 35% to 65% of CRM₁₉₇ lysines; 30% to 60% of CRM₁₉₇ lysines; 25% to 55% of CRM₁₉₇ lysines; 20% to 50% of CRM₁₉₇ lysines; 15% to 45% of CRM₁₉₇ lysines; 10% to 40% of CRM₁₉₇ lysines; 40% to 70% of CRM₁₉₇ lysines; or 45% to 75% of CRM₁₉₇ lysines are covalently linked to CP5.

The frequency of attachment of the capsular polysaccharide chain to a lysine on the carrier molecule is another parameter for characterizing conjugates of capsule polysaccharides. For example, in one embodiment, at least one covalent linkage between CRM₁₉₇ and polysaccharide occurs for at least every 5 to 10 saccharide repeat units of the capsular polysaccharide. In another embodiment, there is at least one covalent linkage between CRM₁₉₇ and capsular polysaccharide for every 5 to 10 saccharide repeat units; every 2 to 7 saccharide repeat units, every 3 to 8 saccharide repeat units; every 4 to 9 saccharide repeat units; every 6 to 11 saccharide repeat units; every 7 to 12 saccharide repeat units; every 8 to 13 saccharide repeat units; every 9 to 14 saccharide repeat units; every 10 to 15 saccharide repeat units; every 2 to 6 saccharide repeat units, every 3 to 7 saccharide repeat units; every 4 to 8 saccharide repeat units; every 6 to 10 saccharide repeat units; every 7 to 11 saccharide repeat units; every 8 to 12 saccharide repeat units; every 9 to 13 saccharide repeat units; every 10 to 14 saccharide repeat units; every 10 to 20 saccharide repeat units; every 5 to 10 saccharide repeat units of the capsular polysaccharide. In another embodiment, at least one linkage between CRM₁₉₇ and capsular polysaccharide occurs for every 2, 3, 4, 5, 6, 7, 8, 9, 10, 11, 12, 13, 14, 15, 16, 17, 18, 19, or 20 saccharide repeat units of the capsular polysaccharide.

The chemical activation of the polysaccharides and subsequent conjugation to the carrier protein may be achieved by conventional means. See, for example, U.S. Pat. Nos. 4,673,574 and 4,902,506. Other activation and conjugation methods may alternatively be used.

"Carrier protein" or "protein carrier" as used herein, refers to any protein molecule that may be conjugated to an antigen (such as the capsular polysaccharides) against which an immune response is desired. Conjugation of an antigen such as a polysaccharide to a carrier protein can render the antigen immunogenic. Carrier proteins are preferably proteins that are non-toxic and non-reactogenic and obtainable in sufficient amount and purity. Examples of carrier proteins are toxins, toxoids or any mutant cross-reactive material (CRM₁₉₇) of the toxin from tetanus, diphtheria, pertussis, *Pseudomonas* species, *E. coli, Staphylococcus* species, and *Streptococcus* species. Carrier proteins should be amenable to standard conjugation procedures. In a particular embodiment of the present invention, CRM₁₉₇ is used as the carrier protein.

CRM₁₉₇ (Wyeth/Pfizer, Sanford, NC) is a non-toxic variant (i.e., toxoid) of diphtheria toxin isolated from cultures of *Corynebacterium diphtheria* strain C7 (β₁₉₇) grown in casamino acids and yeast extract-based medium. CRM₁₉₇ is purified through ultra-filtration, ammonium sulfate precipitation, and ion-exchange chromatography. A culture of *Corynebacterium diphtheriae* strain C7 (₁₉₇), which produces CRM₁₉₇ protein, has been deposited with the American Type Culture Collection, Rockville, Maryland and has been assigned accession number ATCC 53281. Other diphtheria toxoids are also suitable for use as carrier proteins.

Other suitable carrier proteins include inactivated bacterial toxins such as tetanus toxoid, pertussis toxoid, cholera toxoid (e.g., as described in International Patent Application WO2004/083251), *E. coli* LT, *E. coli* ST, and exotoxin A from *Pseudomonas aeruginosa.* Bacterial outer membrane proteins such as outer membrane protein complex c (OMPC), porins, transferrin binding proteins, pneumolysin, pneumococcal surface protein A (PspA), pneumococcal adhesin protein (PsaA), C. *difficile* enterotoxin (toxin A) and cytotoxin (toxin B) or *Haemophilus influenzae* protein D, can also be used. Other proteins, such as streptococcal C5a peptidase (SCP), ovalbumin, keyhole limpet hemocyanin (KLH), bovine serum albumin (BSA) or purified protein derivative of tuberculin (PPD) can also be used as carrier proteins.

After conjugation of the capsular polysaccharide to the carrier protein, the polysaccharide-protein conjugates are purified (enriched with respect to the amount of polysaccharide-protein conjugate) by a variety of techniques. These techniques include, e.g., concentration/diafiltration operations, precipitation/elution, column chromatography, and depth filtration. See examples below.

After the individual conjugates are purified, they may be combined to formulate an immunogenic composition of the present invention, which may be used, for example, in a vaccine. Formulation of the immunogenic composition of the present invention can be accomplished using art-recognized methods.

It is noted that in this disclosure, terms such as "comprises", "comprised", "comprising", "contains", "containing" and the like can have the meaning attributed to them in U.S. patent law; *e.g.,* they can mean "includes", "included", "including" and the like. Such terms refer to the inclusion of a particular ingredients or set of ingredients without excluding any other ingredients. Terms such as "consisting essentially of" and "consists essentially of" have the meaning attributed to them in U.S. patent law, *e.g.,* they allow for the inclusion of additional ingredients or steps that do not detract from the novel or basic characteristics of the invention, *i.e*., they exclude additional unrecited ingredients or steps that detract from novel or basic characteristics of the invention, and they exclude ingredients or steps of the prior art, such as documents in the art that are cited herein, especially as it is a goal of this document to define embodiments that are patentable, *e.g.,* novel, non-obvious, inventive, over the prior art, *e.g.,* over documents cited herein. And, the terms "consists of" and "consisting of" have the meaning ascribed to them in U.S. patent law; namely, that these terms are close-ended. Accordingly, these terms refer to the inclusion of a particular ingredient or set of ingredients and the exclusion of all other ingredients.

A "conservative amino acid substitution" refers to the substitution of one or more of the amino acid residues of a protein with other amino acid residues having similar physical and/or chemical properties. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. Amino acids containing aromatic ring structures are phenylalanine, tryptophan, and tyrosine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Such alterations will not be expected to affect apparent molecular weight as determined by polyacrylamide gel electrophoresis, or isoelectric point. Particularly preferred substitutions are: Lys for Arg and vice versa such that a positive charge may be maintained; Glu for Asp and vice versa such that a negative charge may be maintained; Ser for Thr such that a free --OH can be maintained; and Gln for Asn such that a free NH₂ can be maintained.

"Fragment" refers to proteins where only specific domains of a larger protein are included. For example, ClfA and ClfB proteins contain as many as 8 domains each if the signal sequences are included. A polypeptide corresponding to the N1N2N3, N2N3, N1N2, N1, N2, or N3 domains are each considered to be fragments of ClfA or ClfB. "Fragment" also refers to either a protein or polypeptide comprising an amino acid sequence of at least 4 amino acid residues (preferably, at least 10 amino acid residues, at least 15 amino acid residues, at least 20 amino acid residues, at least 25 amino acid residues, at least 40 amino acid residues, at least 50 amino acid residues, at least 60 amino residues, at least 70 amino acid residues, at least 80 amino acid residues, at least 90 amino acid residues, at least 100 amino acid residues, at least 125 amino acid residues, or at least 150 amino acid residues) of the amino acid sequence of a parent protein or polypeptide or a nucleic acid comprising a nucleotide sequence of at least 10 base pairs (preferably at least 20 base pairs, at least 30 base pairs, at least 40 base pairs, at least 50 base pairs, at least 50 base pairs, at least 100 base pairs, at least 200 base pairs) of the nucleotide sequence of the parent nucleic acid.

"Functional activity" of an antibody or "functional antibody" as used herein refers to an antibody that, at a minimum, can bind specifically to an antigen. Additional functions are known in the art and may include additional components of the immune system that effect clearance or killing of the pathogen such as through opsonization, ADCC or complement-mediated cytotoxicity. After antigen binding, any subsequent antibody functions can be mediated through the Fc region of the antibody. The antibody opsonophagocytic assay (OPA) is an *in vitro* assay designed to measure *in vitro* Ig complement-assisted killing of bacteria with effector cells (white blood cells), thus mimicking a biological process. Antibody binding may also directly inhibit the biological function of the antigen it binds, e.g., antibodies that bind ClfA can neutralize its enzymatic function. In some embodiments, a "functional antibody" refers to an antibody that is functional as measured by the killing of bacteria in an animal efficacy model or an opsonophagocytic killing assay that demonstrates that the antibodies kill the bacteria.

The molecular weight of the *S. aureus* capsule polysaccharides is a consideration for use in immunogenic compositions. For example, high molecular weight capsule polysaccharides may be able to induce certain antibody immune responses due to a higher valency of the epitopes present on the antigenic surface. The isolation of "high molecular weight capsular polysaccharides" is contemplated for use in the compositions and methods of the present invention. For example, in one embodiment of the invention, the isolation of type 5 high molecular weight polysaccharides ranging in size from about 50 to about 800 kDa in molecular weight is contemplated. In one embodiment of the invention, the isolation of type 5 high molecular weight polysaccharides ranging in size from about 20 to about 1000 kDa in molecular weight is contemplated. In one embodiment of the invention, the isolation and purification of type 5 high molecular weight capsular polysaccharides ranging in size from about 50 to about 300 kDa in molecular weight is contemplated. In one embodiment, the isolation and purification of type 5 high molecular weight capsular polysaccharide ranging from 70 kDa to 300 kDa in molecular weight is contemplated. In one embodiment, the isolation and purification of type 5 high molecular weight capsular polysaccharide ranging from 90 kDa to 250 kDa in molecular weight is contemplated. In one embodiment, the isolation and purification of type 5 high molecular weight capsular polysaccharide ranging from 90 kDa to 150 kDa in molecular weight is contemplated. In one embodiment, the isolation and purification of type 5 high molecular weight capsular polysaccharide ranging from 90 kDa to 140 kDa in molecular weight is contemplated. In one embodiment, the isolation and purification of type 5 high molecular weight capsular polysaccharide ranging from 80 kDa to 120 kDa in molecular weight is contemplated. Other ranges of high molecular weight serotype 5 capsular polysaccharide that can be isolated and purified by the methods of this invention include size ranges of about 70 kDa to about 100 kDa in molecular weight; 70 kDa to 110 kDa in molecular weight; 70 kDa to 120 kDa in molecular weight; 70 kDa to 130 kDa in molecular weight; 70 kDa to 140 kDa in molecular weight; 70 kDa to 150 kDa in molecular weight; 70 kDa to 160 kDa in molecular weight; 80 kDa to 110 kDa in molecular weight; 80 kDa to 120 kDa in molecular weight; 80 kDa to 130 kDa in molecular weight; 80 kDa to 140 kDa in molecular weight; 80 kDa to 150 kDa in molecular weight; 80 kDa to 160 kDa in molecular weight; 90 kDa to 110 kDa in molecular weight; 90 kDa to 120 kDa in molecular weight; 90 kDa to 130 kDa in molecular weight; 90 kDa to 140 kDa in molecular weight; 90 kDa to 150 kDa in molecular weight; 90 kDa to 160 kDa in molecular weight; 100 kDa to 120 kDa in molecular weight; 100 kDa to 130 kDa in molecular weight; 100 kDa to 140 kDa in molecular weight; 100 kDa to 150 kDa in molecular weight; 100 kDa to 160 kDa in molecular weight; and similar desired molecular weight ranges.

As discussed above, the molecular weight of the *S. aureus* capsule polysaccharides is a consideration for use in immunogenic compositions. For example, high molecular weight capsule polysaccharides may be able to induce certain antibody immune responses due to a higher valency of the epitopes present on the antigenic surface. In one embodiment of the invention, the isolation and purification of type 8 high molecular weight capsular polysaccharides ranging from about 20 kDa to about 1000 kDa in molecular weight is contemplated. In one embodiment of the invention, the isolation and purification of type 8 high molecular weight capsular polysaccharides ranging from about 50 kDa to about 700 kDa in molecular weight is contemplated. In one embodiment of the invention, the isolation and purification of type 8 high molecular weight capsular polysaccharides ranging from 50 kDa to 300 kDa in molecular weight is contemplated. In one embodiment, the isolation and purification of type 8 high molecular weight capsular polysaccharide ranging from 70 kDa to 300 kDa in molecular weight is contemplated. In one embodiment, the isolation and purification of type 8 high molecular weight capsular polysaccharides ranging from 90 kDa to 250 kDa in molecular weight is contemplated. In one embodiment, the isolation and purification of type 8 high molecular weight capsular polysaccharides ranging from 90 kDa to 150 kDa in molecular weight is contemplated. In one embodiment, the isolation and purification of type 8 high molecular weight capsular polysaccharides ranging from 90 kDa to 120 kDa in molecular weight is contemplated. In one embodiment, the isolation and purification of type 8 high molecular weight capsular polysaccharides ranging from 80 kDa to 120 kDa in molecular weight is contemplated. Other ranges of high molecular weight serotype 8 capsular polysaccharides that can be isolated and purified by the methods of this invention include size ranges of about 70 kDa to about 100 kDa in molecular weight; 70 kDa to 110 kDa in molecular weight; 70 kDa to 120 kDa in molecular weight; 70 kDa to 130 kDa in molecular weight; 70 kDa to 140 kDa in molecular weight; 70 kDa to 150 kDa in molecular weight; 70 kDa to 160 kDa in molecular weight; 80 kDa to 110 kDa in molecular weight; 80 kDa to 120 kDa in molecular weight; 80 kDa to 130 kDa in molecular weight; 80 kDa to 140 kDa in molecular weight; 80 kDa to 150 kDa in molecular weight; 80 kDa to 160 kDa in molecular weight; 90 kDa to 110 kDa in molecular weight; 90 kDa to 120 kDa in molecular weight; 90 kDa to 130 kDa in molecular weight; 90 kDa to 140 kDa in molecular weight; 90 kDa to 150 kDa in molecular weight; 90 kDa to 160 kDa in molecular weight; 100 kDa to 120 kDa in molecular weight; 100 kDa to 130 kDa in molecular weight; 100 kDa to 140 kDa in molecular weight; 100 kDa to 150 kDa in molecular weight; 100 kDa to 160 kDa in molecular weight; and similar desired molecular weight ranges.

An "immune response" to an immunogenic composition is the development in a subject of a humoral and/or a cell-mediated immune response to molecules present in the composition of interest (for example, an antigen, such as a protein or polysaccharide). For purposes of the present invention, a "humoral immune response" is an antibody-mediated immune response and involves the generation of antibodies with affinity for the antigens present in the immunogenic compositions of the invention, while a "cell-mediated immune response" is one mediated by T-lymphocytes and/or other white blood cells. A "cell-mediated immune response" is elicited by the presentation of antigenic epitopes in association with Class I or Class II molecules of the major histocompatibility complex (MHC). This activates antigen-specific CD4+ T helper cells or CD8+ cytotoxic T lymphocyte cells ("CTLs"). CTLs have specificity for peptide or lipid antigens that are presented in association with proteins encoded by the major histocompatibility complex (MHC) or CD1 and expressed on the surfaces of cells. CTLs help induce and promote the intracellular destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with classical or nonclassical MHC molecules on their surface. A "cell-mediated immune response" also refers to the production of cytokines, chemokines and other such molecules produced by activated T-cells and/or other white blood cells, including those derived from CD4+ and CD8+ T-cells. The ability of a particular antigen or composition to stimulate a cell-mediated immunological response may be determined by a number of assays, such as by lymphoproliferation (lymphocyte activation) assays, CTL cytotoxic cell assays, by assaying for T-lymphocytes specific for the antigen in a sensitized subject, or by measurement of cytokine production by T cells in response to restimulation with antigen. Such assays are well known in the art. See, *e.g.,* Erickson et al., J. Immunol. (1993) 151:4189-4199; Doe et al., Eur. J. Immunol. (1994) 24:2369-2376.

The term "immunogenic" refers to the ability of an antigen or a vaccine to elicit an immune response, either humoral or cell-mediated, or both.

An "immunogenic amount", or an "immunologically effective amount" or "dose", each of which is used interchangeably herein, generally refers to the amount of antigen or immunogenic composition sufficient to elicit an immune response, either a cellular (T cell) or humoral (B cell or antibody) response, or both, as measured by standard assays known to one skilled in the art.

The amount of a particular conjugate in a composition is generally calculated based on total polysaccharide, conjugated and non-conjugated for that conjugate. For example, a CP5 conjugate with 20% free polysaccharide will have about 80 mcg of conjugated CP5 polysaccharide and about 20 mcg of non-conjugated CP5 polysaccharide in a 100 mcg CP5 polysaccharide dose. The protein contribution to the conjugate is usually not considered when calculating the dose of a conjugate. The amount of conjugate can vary depending upon the staphylococcal serotype. Generally, each dose will comprise 0.01 to 100 mcg of polysaccharide, particularly 0.1 to 10 mcg, and more particularly 1 to 10 mcg. The "immunogenic amount" of the different polysaccharide components in the immunogenic composition, may diverge and each may comprise 0.01 mcg, 0.1 mcg, 0.25 mcg, 0.5 mcg, 1 mcg, 2 mcg, 3 mcg, 4 mcg, 5 mcg, 6 mcg, 7 mcg, 8 mcg, 9 mcg, 10 mcg, 15 mcg, 20 mcg, 30 mcg, 40 mcg,50 mcg, 60 mcg, 70 mcg, 80 mcg, 90 mcg, or about 100 mcg of any particular polysaccharide antigen.

In another embodiment, the "immunogenic amount" of the protein components in the immunogenic composition, may range from about 10 mcg to about 300 mcg of each protein antigen. In a particular embodiment, the "immunogenic amount" of the protein components in the immunogenic composition, may range from about 20 mcg to about 200 mcg of each protein antigen. The "immunogenic amount" of the different protein components in the immunogenic composition may diverge, and each comprise 10 mcg, 20 mcg, 30 mcg, 40 mcg, 50 mcg, 60 mcg, 70 mcg, 80 mcg, 90 mcg, 100 mcg, 125 mcg, 150 mcg, 175 mcg or about 200 mcg of any particular protein antigen.

The effectiveness of an antigen as an immunogen can be measured by measuring the levels of B cell activity by measuring the levels of circulating antibodies specific for the antigen in serum using immunoassays, immunoprecipitation assays, functional antibody assays, such as *in vitro* opsonic assay and many other assays known in the art. Another measure of effectiveness of an antigen as an T-cell immunogen can be measured by either by proliferation assays, by cytolytic assays, such as chromium release assays to measure the ability of a T cell to lyse its specific target cell. Furthermore, in the present invention, an "immunogenic amount" may also be defined by measuring the serum levels of antigen specific antibody induced following administration of the antigen, or, by measuring the ability of the antibodies so induced to enhance the opsonophagocytic ability of particular white blood cells, as described herein. The level of protection of the immune response may be measured by challenging the immunized host with the antigen that has been injected. For example, if the antigen to which an immune response is desired is a bacterium, the level of protection induced by the "immunogenic amount" of the antigen can be measured by detecting the percent survival or the percent mortality after challenge of the animals with the bacterial cells. In one embodiment, the amount of protection may be measured by measuring at least one symptom associated with the bacterial infection, for example, a fever associated with the infection. The amount of each of the antigens in the multi-antigen or multi-component vaccine or immunogenic compositions will vary with respect to each of the other components and can be determined by methods known to the skilled artisan. Such methods would include, for example, procedures for measuring immunogenicity and/or *in vivo* efficacy.

The term "immunogenic composition" relates to any pharmaceutical composition containing an antigen, *e.g.* a microorganism, or a component thereof, which composition can be used to elicit an immune response in a subject. The immunogenic compositions of the present invention can be used to treat a human susceptible to *S. aureus* infection, by means of administering the immunogenic compositions via a systemic transdermal or mucosal route. These administrations can include injection via the intramuscular (i.m.), intraperitoneal (i.p.), intradermal (i.d.) or subcutaneous routes; application by a patch or other transdermal delivery device; or via mucosal administration to the oral/alimentary, respiratory or genitourinary tracts. In one embodiment, intranasal administration is used for the treatment or prevention of nasopharyngeal carriage of *S. aureus,* thus attenuating infection at its earliest stage. In one embodiment, the immunogenic composition may be used in the manufacture of a vaccine or in the elicitation of a polyclonal or monoclonal antibodies that could be used to passively protect or treat an animal.

Optimal amounts of components for a particular immunogenic composition can be ascertained by standard studies involving observation of appropriate immune responses in subjects. Following an initial vaccination, subjects can receive one or several booster immunizations adequately spaced.

In one embodiment of the present disclosure, the *S. aureus* lyophilized immunogenic composition comprises a recombinant *S. aureus* clumping factor A (ClfA) fragment (N1N2N3, or combinations thereof). In a further embodiment of the present disclosure, the *S. aureus* lyophilized immunogenic composition comprises a recombinant *S. aureus* clumping factor A (ClfA) fragment, an isolated capsular polysaccharides type 5 conjugated to CRM₁₉₇ and an isolated capsular polysaccharides type 8 conjugated to CRM₁₉₇.

The immunogenic compositions of the present invention can further comprise one or more additional "immunomodulators", which are agents that perturb or alter the immune system, such that either up-regulation or down-regulation of humoral and/or cell-mediated immunity is observed. In one particular embodiment, up-regulation of the humoral and/or cell-mediated arms of the immune system is preferred. Examples of certain immunomodulators include, for example, an adjuvant or cytokine, or ISCOMATRIX™ (CSL Limited, Parkville, Australia), described in U.S. Patent No. 5,254,339 among others. Non-limiting examples of adjuvants that can be used in the vaccine of the present invention include the RIBI adjuvant system (Ribi Inc., Hamilton, Mont.), alum, mineral gels such as aluminum hydroxide gel, oil-in-water emulsions, water-in-oil emulsions such as, *e.g.,* Freund's complete and incomplete adjuvants, Block copolymer (CytRx, Atlanta Ga.), QS-21 (Cambridge Biotech Inc., Cambridge Mass.), SAF-M (Chiron, Emeryville Calif.), AMPHIGEN® adjuvant, saponin, Quil A or other saponin fraction, monophosphoryl lipid A, and Avridine lipid-amine adjuvant. Non-limiting examples of oil-in-water emulsions useful in the vaccine of the invention include modified SEAM62 and SEAM 1/2 formulations. Modified SEAM62 is an oil-in-water emulsion containing 5% (v/v) squalene (Sigma), 1% (v/v) SPAN® 85 detergent (ICI Surfactants), 0.7% (v/v) polysorbate 80 detergent (ICI Surfactants), 2.5% (v/v) ethanol, 200 µg/ml Quil A, 100 µg/ml cholesterol, and 0.5% (v/v) lecithin. Modified SEAM 1/2 is an oil-in-water emulsion comprising 5% (v/v) squalene, 1% (v/v) SPAN® 85 detergent, 0.7% (v/v) polysorbate 80 detergent, 2.5% (v/v) ethanol, 100 µg/ml Quil A, and 50 µg/ml cholesterol. Other "immunomodulators" that can be included in the compositions of the invention include, *e.g.,* one or more interleukins, interferons, or other known cytokines or chemokines. In one embodiment, the adjuvant may be a cyclodextrin derivative or a polyanionic polymer, such as those described in U.S. patent numbers 6,165,995 and 6,610,310, respectively. It is to be understood that the immunomodulator and/or adjuvant to be used will depend on the subject to which the vaccine or immunogenic composition will be administered, the route of injection and the number of injections to be given.

In a still further embodiment of the present disclosure, the *S. aureus* lyophilized immunogenic composition comprises a recombinant *S. aureus* clumping factor A (ClfA) fragment, an isolated capsular polysaccharides type 5 conjugated to CRM₁₉₇, an isolated capsular polysaccharides type 8 conjugated to CRM₁₉₇, and a recombinant MntC protein (also known as rP305A). In some embodiments, the MntC protein is lipidated. In other embodiments, the MntC protein is not lipidated. In another embodiment, the *S. aureus* immunogenic composition is a sterile formulation (liquid, lyophilized, DNA vaccine, intradermal preparation) of recombinant *S. aureus* clumping factor (ClfA) fragment (N1N2N3, or combinations thereof), recombinant *S. aureus* clumping factor B (ClfB) fragment (N1N2N3, or combinations thereof), an isolated capsular polysaccharides type 5 conjugated to CRM₁₉₇ and an isolated capsular polysaccharides type 8 conjugated to CRM₁₉₇.

In one embodiment of the present invention, the *S. aureus* immunogenic composition comprises a recombinant *S. aureus* clumping factor A (ClfA) fragment (N1N2N3), *S. aureus* iron binding protein MntC, an isolated capsular polysaccharides type 5 conjugated to CRM₁₉₇ and an isolated capsular polysaccharides type 8 conjugated to CRM₁₉₇. In one embodiment, the *S. aureus* immunogenic composition is a sterile formulation (liquid) of recombinant *S. aureus* clumping factor (ClfA) fragment (N1N2N3), recombinant *S. aureus* clumping factor B (ClfB) fragment (N1N2N3, or combinations thereof), *S. aureus* iron binding protein MntC, an isolated capsular polysaccharides type 5 conjugated to CRM₁₉₇ and an isolated capsular polysaccharides type 8 conjugated to CRM₁₉₇.

In one embodiment of the present invention, the *S. aureus* immunogenic composition comprises a recombinant *S. aureus* clumping factor B (ClfB) fragment (N1N2N3, or combinations thereof), an isolated capsular polysaccharides type 5 conjugated to CRM₁₉₇ and an isolated capsular polysaccharides type 8 conjugated to CRM₁₉₇. In one embodiment of the present invention, the *S. aureus* immunogenic composition comprises a recombinant *S. aureus* clumping factor B (ClfB) fragment (N1N2N3, or combinations thereof), *S. aureus* iron binding protein MntC, an isolated capsular polysaccharides type 5 conjugated to CRM₁₉₇ and an isolated capsular polysaccharides type 8 conjugated to CRM₁₉₇. In one embodiment of the present invention, the *S. aureus* immunogenic composition comprises a *S. aureus* iron binding protein MntC, an isolated capsular polysaccharides type 5 conjugated to CRM₁₉₇ and an isolated capsular polysaccharides type 8 conjugated to CRM₁₉₇.

*S. aureus* "invasive disease" is the isolation of bacteria from a normally sterile site, where there is associated clinical signs/symptoms of disease. Normally sterile body sites include blood, CSF, pleural fluid, pericardial fluid, peritoneal fluid, joint/synovial fluid, bone, internal body site (lymph node, brain, heart, liver, spleen, vitreous fluid, kidney, pancreas, ovary), or other normally sterile sites. Clinical conditions characterizing invasive diseases include bacteremia, pneumonia, cellulitis, osteomyelitis, endocarditis, septic shock and more.

The term "isolated" means that the material is removed from its original environment (*e.g*., the natural environment if it is naturally occurring or from its host organism if it is a recombinant entity, or taken from one environment to a different environment). For example, an "isolated" capsule polysaccharide, protein or peptide is substantially free of cellular material or other contaminating proteins from the cell or tissue source from which the protein is derived, or substantially free of chemical precursors or other chemicals when chemically synthesized, or otherwise present in a mixture as part of a chemical reaction. In the present invention, the proteins or polysaccharides may be isolated from the bacterial cell or from cellular debris, so that they are provided in a form useful in the manufacture of an immunogenic composition. The term "isolated" or "isolating" may include purifying, or purification, including for example, the methods of purification of the proteins or capsular polysaccharides, as described herein. The language "substantially free of cellular material" includes preparations of a polypeptide/protein in which the polypeptide/protein is separated from cellular components of the cells from which it is isolated or recombinantly produced. Thus, a capsule polysaccharide, protein or peptide that is substantially free of cellular material includes preparations of the capsule polysaccharide, protein or peptide having less than about 30%, 20%, 10%, 5%, 2.5%, or 1%, (by dry weight) of contaminating protein or polysaccharide or other cellular material. When the polypeptide/protein is recombinantly produced, it is also preferably substantially free of culture medium, i.e., culture medium represents less than about 20%, 10%, or 5% of the volume of the protein preparation. When polypeptide/protein or polysaccharide is produced by chemical synthesis, it is preferably substantially free of chemical precursors or other chemicals, i.e., it is separated from chemical precursors or other chemicals which are involved in the synthesis of the protein or polysaccharide. Accordingly, such preparations of the polypeptide/protein or polysaccharide have less than about 30%, 20%, 10%, 5% (by dry weight) of chemical precursors or compounds other than polypeptide/protein or polysaccharide fragment of interest.

A "non-conservative amino acid substitution" refers to the substitution of one or more of the amino acid residues of a protein with other amino acid residues having dissimilar physical and/or chemical properties, using the characteristics defined above.

The term "pharmaceutically acceptable carrier" means a carrier approved by a regulatory agency of a Federal, a state government, or other regulatory agency, or listed in the U.S. Pharmacopeia or other generally recognized pharmacopeia for use in animals, including humans as well as non-human mammals. The term "carrier" refers to a diluent, adjuvant, excipient, or vehicle with which the pharmaceutical composition is administered. Such pharmaceutical carriers can be sterile liquids, such as water and oils, including those of petroleum, animal, vegetable or synthetic origin. Water, saline solutions and aqueous dextrose and glycerol solutions can be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting, bulking, emulsifying agents, or pH buffering agents. These compositions can take the form of solutions, suspensions, emulsion, sustained release formulations and the like. Examples of suitable pharmaceutical carriers are described in "Remington's Pharmaceutical Sciences" by E. W. Martin. The formulation should suit the mode of administration.

The terms "protein", "polypeptide" and "peptide" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include modifications, such as deletions, additions and substitutions (generally conservative in nature, but which may be non-conservative), to a native sequence, preferably such that the protein maintains the ability to elicit an immunological response within an animal to which the protein is administered. Also included are post-expression modifications, e.g. glycosylation, acetylation, lipidation, phosphorylation and the like.

A "protective" immune response refers to the ability of an immunogenic composition to elicit an immune response, either humoral or cell mediated, which serves to protect the subject from an infection. The protection provided need not be absolute, i.e., the infection need not be totally prevented or eradicated, if there is a statistically significant improvement compared with a control population of subjects, e.g. infected animals not administered the vaccine or immunogenic composition. Protection may be limited to mitigating the severity or rapidity of onset of symptoms of the infection. In general, a "protective immune response" would include the induction of an increase in antibody levels specific for a particular antigen in at least 50% of subjects, including some level of measurable functional antibody responses to each antigen. In particular situations, a "protective immune response" could include the induction of a two fold increase in antibody levels or a four fold increase in antibody levels specific for a particular antigen in at least 50% of subjects, including some level of measurable functional antibody responses to each antigen. In certain embodiments, opsonising antibodies correlate with a protective immune response. Thus, protective immune response may be assayed by measuring the percent decrease in the bacterial count in an opsonophagocytosis assay, for instance those described below. Preferably, there is a decrease in bacterial count of at least 10%, 25%, 50%, 65%, 75%, 80%, 85%, 90%, 95% or more.

The term "recombinant" as used herein simply refers to any protein, polypeptide, or cell expressing a gene of interest that is produced by genetic engineering methods. The term "recombinant" as used with respect to a protein or polypeptide, means a polypeptide produced by expression of a recombinant polynucleotide. The proteins used in the immunogenic compositions of the invention may be isolated from a natural source or produced by genetic engineering methods, such as, for example recombinant ClfA, recombinant ClfB or recombinant MntC. "Recombinant," as used herein, further describes a nucleic acid molecule, which, by virtue of its origin or manipulation, is not associated with all or a portion of the polynucleotide with which it is associated in nature. The term "recombinant" as used with respect to a host cell means a host cell into which a recombinant polynucleotide has been introduced.

Recombinant ClfA (rClfA) and recombinant ClfB (rClfB) as used herein refers to forms of ClfA or ClfB for use in the immunogenic compositions of the disclosure. In certain embodiments, rClfA is a fragment of ClfA comprising one or more of the N domains, for example, N1N2N3, N2N3, N2 or N3 and is referred to herein as "recombinant ClfA" or "rClfA". In one embodiment, rClfB is a fragment of ClfB comprising one or more of the N domains of ClfB, for example, N1N2N3, N2N3, N2 or N3 and is referred to herein as "recombinant ClfB" or "rClfB".

The term "subject" refers to a mammal, bird, fish, reptile, or any other animal. The term "subject" also includes humans. The term "subject" also includes household pets. Non-limiting examples of household pets include: dogs, cats, pigs, rabbits, rats, mice, gerbils, hamsters, guinea pigs, ferrets, birds, snakes, lizards, fish, turtles, and frogs. The term "subject" also includes livestock animals. Non-limiting examples of livestock animals include: alpaca, bison, camel, cattle, deer, pigs, horses, llamas, mules, donkeys, sheep, goats, rabbits, reindeer, yak, chickens, geese, and turkeys.

As used herein, "treatment" (including variations thereof, for example, "treat" or "treated") refers to any one or more of the following: (i) the prevention of infection or reinfection, as in a traditional vaccine, (ii) the reduction in the severity of, or, in the elimination of symptoms, and (iii) the substantial or complete elimination of the pathogen or disorder in question. Hence, treatment may be effected prophylactically (prior to infection) or therapeutically (following infection). In the present invention, prophylactic or therapeutic treatments can be used. According to a particular embodiment of the present disclosure, compositions and methods are provided which treat, including prophylactically and/or therapeutically immunize, a host animal against a microbial infection (e.g. a bacterium such as *Staphylococcus* species). The methods of the present disclosure are useful for conferring prophylactic and/or therapeutic immunity to a subject. The methods of the present disclosure can also be practiced on subjects for biomedical research applications.

The terms "vaccine" or "vaccine composition", which are used interchangeably, refer to pharmaceutical compositions comprising at least one immunogenic composition that induces an immune response in an animal.

### General Description

The present disclosure relates to lyophilized immunogenic compositions comprising antigens from a staphylococcal organism, for example *S. aureus.* In some embodiments, the lyophilized immunogenic composition comprises ClfA. In some embodiments, the lyophilized immunogenic composition comprises at least three antigens. In some embodiments, the lyophilized immunogenic composition comprises at least four antigens. The antigens may be isolated from the organism using biochemical isolation procedures, or they may be produced synthetically or by recombinant means. The antigens may be polypeptides, or polysaccharides, or a combination thereof. These immunogenic compositions may be used in the manufacture of a vaccine to immunize subjects against infections caused by a staphylococcal organism. The components suitable for use in these compositions are described in greater detail below.

### Staphylococcal Immunogenic Compositions

*S. aureus* is the causative agent of a wide variety of human diseases ranging from superficial skin infections to life threatening conditions such as pneumonia, sepsis and endocarditis. See Lowy N. Eng. J. Med. 339:580-532(1998). In cases of invasive disease, *S. aureus* can be isolated from normally sterile body sites including blood, cerebral spinal fluid CSF, pleural fluid, pericardial fluid, peritoneal fluid, joint/synovial fluid, bone, internal body site (lymph node, brain, heart, liver, spleen, vitreous fluid, kidney, pancreas, ovary), or other normally sterile sites. This can lead to life threatening clinical conditions such as bacteremia, pneumonia, cellulitis, osteomyelitis, endocarditis, and septic shock. Adults, elderly and pediatric patients are most at risk for *S. aureus* infections.

Embodiments of the present disclosure describe a selected antigen or antigens in lyophilized immunogenic compositions including an isolated *S. aureus* clumping factor A (ClfA) polypeptide, an isolated *S. aureus* capsular polysaccharide type 5 conjugated to a carrier protein, an isolated *S. aureus* capsular polysaccharide type 8 conjugated to a carrier protein, an isolated *S. aureus* clumping factor B (ClfB), and isolated *S. aureus* MntC protein. Some formulations of the lyophilized immunogenic compositions were tested to demonstrate increased stability of the ClfA protein. Some formulations of the lyophilized immunogenic compositions were tested to demonstrate stability of the MntC protein. Some formulations of the lyophilized compositions were also tested to ensure that CP5-protein conjugates and CP8-protein conjugates were stable after lyophilization.

Accordingly, one lyophilized immunogenic composition comprises: an isolated *S. aureus* clumping factor A (ClfA) polypeptide. One lyophilized immunogenic composition comprises: an isolated *S. aureus* clumping factor A (ClfA) polypeptide, an isolated *S. aureus* capsular polysaccharide type 5 conjugated to a carrier protein, and an isolated *S. aureus* capsular polysaccharide type 8 conjugated to a carrier protein. One lyophilized immunogenic composition comprises: an isolated *S. aureus* clumping factor A (ClfA) polypeptide, an isolated *S. aureus* clumping factor B (ClfB), isolated *S. aureus* capsular polysaccharide type 5 conjugated to a carrier protein, and an isolated *S. aureus* capsular polysaccharide type 8 conjugated to a carrier protein. One lyophilized immunogenic composition comprises: an isolated *S. aureus* clumping factor A (ClfA) polypeptide, an isolated *S. aureus* clumping factor B (ClfB) polypeptide, an isolated *S. aureus* MntC protein, an isolated *S. aureus* capsular polysaccharide type 5 conjugated to a carrier protein, and an isolated *S. aureus* capsular polysaccharide type 8 conjugated to a carrier protein. One lyophilized immunogenic composition comprises: an isolated *S. aureus* clumping factor A (ClfA) polypeptide, an isolated *S. aureus* MntC protein, an isolated *S. aureus* capsular polysaccharide type 5 conjugated to a carrier protein, and an isolated *S. aureus* capsular polysaccharide type 8 conjugated to a carrier protein. One lyophilized immunogenic composition comprises: an isolated *S. aureus* clumping factor B (ClfB) polypeptide, an isolated *S. aureus* capsular polysaccharide type 5 conjugated to a carrier protein, and an isolated *S. aureus* capsular polysaccharide type 8 conjugated to a carrier protein. One lyophilized immunogenic composition comprises: an isolated *S. aureus* clumping factor B (ClfB) polypeptide, an isolated *S. aureus* MntC protein, an isolated *S. aureus* capsular polysaccharide type 5 conjugated to a carrier protein, and an isolated *S. aureus* capsular polysaccharide type 8 conjugated to a carrier protein. One lyophilized immunogenic composition comprises: an isolated *S. aureus* MntC protein, an isolated *S. aureus* capsular polysaccharide type 5 conjugated to a carrier protein, and an isolated *S. aureus* capsular polysaccharide type 8 conjugated to a carrier protein. One lyophilized immunogenic composition comprises: an isolated *S. aureus* clumping factor A (ClfA) polypeptide, an isolated *S. aureus* clumping factor B (ClfB) polypeptide, and an isolated *S. aureus* MntC protein.

In some embodiments, the above combinations further comprise at least one of the following antigens: EkeS, DsqA, KesK, KrkN, KrkN2, RkaS, RrkN, KnkA, SdrC, SdrD, SdrE, Opp3a, DltD, HtsA, LtaS, IsdA, IsdB, IsdC, SdrF, SdrG, SdrH, SrtA, SpA, Sbi, alpha-hemolysin (hla), beta-hemolysin, fibronectin-binding protein A (fnbA), fibronectin-binding protein B (fnbB), coagulase, Fig, map, Panton-Valentine leukocidin (pvl), alpha-toxin and its variants, gamma-toxin (hlg) and variants, ica, immunodominant ABC transporter, Mg2+ transporter, Ni ABC transporter, RAP, autolysin, laminin receptors, IsaA/PisA, IsaB/PisB , SPOIIIE, SsaA, EbpS, SasA, SasF, SasH, EFB (FIB), SBI, Npase, EBP, bone sialo binding protein II, aureolysin precursor (AUR)/Sepp1, Cna, and fragments thereof such as M55, TSST-1, mecA, poly-N-acetylglucosamine (PNAG/dPNAG) exopolysaccharide, GehD, EbhA, EbhB, SSP-1, SSP-2, HBP, vitronectin binding protein, HarA, EsxA, EsxB, Enterotoxin A, Enterotoxin B, Enterotoxin C1, and novel autolysin.

### Adjuvants

Lyophilized immunogenic compositions as described herein also comprise, in certain embodiments, one or more adjuvants. In some embodiments, the adjuvant is a component of the dried lyophilized composition. In other embodiments, the adjuvant can be added to the reconstituted lyophilized composition prior to administration of the composition to patient. An adjuvant is a substance that enhances the immune response when administered together with an immunogen or antigen. A number of cytokines or lymphokines have been shown to have immune modulating activity, and thus are useful as adjuvants, including, but not limited to, the interleukins 1-α, 1-β, 2, 4, 5, 6, 7, 8, 10, 12 (see, *e.g.,* U.S. Patent No. 5,723,127), 13, 14, 15, 16, 17 and 18 (and its mutant forms); the interferons-α, β and γ; granulocyte-macrophage colony stimulating factor (GM-CSF) (see, *e.g.,* U.S. Patent No. 5,078,996 and ATCC Accession Number 39900); macrophage colony stimulating factor (M-CSF); granulocyte colony stimulating factor (G-CSF); and the tumor necrosis factors α and β. Still other adjuvants that are useful with the immunogenic compositions described herein include chemokines, including without limitation, MCP-1, MIP-1α, MIP-1β, and RANTES; adhesion molecules, such as a selectin, *e.g.,* L-selectin, P-selectin and E-selectin; mucin-like molecules, *e.g.,* CD34, GlyCAM-1 and MadCAM-1; a member of the integrin family such as LFA-1, VLA-1, Mac-1 and p150.95; a member of the immunoglobulin superfamily such as PECAM, ICAMs, *e.g.,* ICAM-1, ICAM-2 and ICAM-3, CD2 and LFA-3; co-stimulatory molecules such as B7-1, B7-2,CD40 and CD40L; growth factors including vascular growth factor, nerve growth factor, fibroblast growth factor, epidermal growth factor, PDGF, BL-1, and vascular endothelial growth factor; receptor molecules including Fas, TNF receptor, Flt, Apo-1, p55, WSL-1, DR3, TRAMP, Apo-3, AIR, LARD, NGRF, DR4, DR5, KILLER, TRAIL-R2, TRICK2, and DR6; and Caspase (ICE).

Suitable adjuvants used to enhance an immune response further include, without limitation, MPL™ (3-O-deacylated monophosphoryl lipid A, Corixa, Hamilton, MT), which is described in U.S. Patent No. 4,912,094. Also suitable for use as adjuvants are synthetic lipid A analogs or aminoalkyl glucosamine phosphate compounds (AGP), or derivatives or analogs thereof, which are available from Corixa (Hamilton, MT), and which are described in United States Patent No. 6,113,918. One such AGP is 2-[(R)-3-Tetradecanoyloxytetradecanoyl-amino] ethyl 2-Deoxy-4-O-phosphono-3-O-[(R)-3-tetradecanoyoxytetradecanoyl]-2-[(R)-3-tetradecanoyloxytetradecanoyl-amino]-b-D-glucopyranoside, which is also known as 529 (formerly known as RC529). This 529 adjuvant is formulated as an aqueous form (AF) or as a stable emulsion (SE).

Still other adjuvants include muramyl peptides, such as N-acetyl-muramyl-L-threonyl-D-isoglutamine (thr-MDP), N-acetyl-normuramyl-L-alanine-2-(1'-2'dipalmitoyl-*sn*-glycero-3-hydroxyphosphoryloxy)-ethylamine (MTP-PE); oil-in-water emulsions, such as MF59 (U.S. Patent No. 6,299,884) (containing 5% Squalene, 0.5% polysorbate 80, and 0.5% SPAN™ 85 (optionally containing various amounts of MTP-PE) formulated into submicron particles using a microfluidizer such as Model 110Y microfluidizer (Microfluidics, Newton, MA)), and SAF (containing 10% Squalene, 0.4% polysorbate 80, 5% pluronic-blocked polymer L121, and thr-MDP, either microfluidized into a submicron emulsion or vortexed to generate a larger particle size emulsion); incomplete Freund's adjuvant (IFA); aluminum salts (alum), such as aluminum hydroxide, aluminum phosphate, aluminum sulfate; Amphigen; Avridine; L121/squalene; D-lactide-polylactide/glycoside; pluronic polyols; killed *Bordetella;* saponins, such as STIMULON™ QS-21 (Antigenics, Framingham, MA.), described in U.S. Patent No. 5,057,540, ISCOMATRIX™ (CSL Limited, Parkville, Australia), described in U.S. Patent No. 5,254,339, and immunostimulating complexes (ISCOMATRIX™); *Mycobacterium tuberculosis*; bacterial lipopolysaccharides; synthetic polynucleotides such as oligonucleotides containing a CpG motif (*e.g*., U.S. Patent No. 6,207,646); IC-31 (Intercell AG, Vienna, Austria), described in European Patent Nos. 1,296,713 and 1,326,634; a pertussis toxin (PT) or mutant thereof, a cholera toxin or mutant thereof (*e.g*., U.S. Patent Nos. 7,285,281, 7,332,174, 7,361,355 and 7,384,640); or an *E. coli* heat-labile toxin (LT) or mutant thereof, particularly LT-K63, LT-R72 (*e.g*., U.S. Patent Nos. 6,149,919, 7,115,730 and 7,291,588).

In some embodiments, the adjuvant is ISCOMATRIX™ (see, e.g., Davis et al., Proc. Nat'l. Acad. Sci., 2004, 101(29):10697-10702).

### Candidate Antigens:

### ClfA: Domain organization

Clumping factor A (ClfA) is a *S. aureus* surface protein associated with binding to host matrix proteins via a fibrinogen binding site. ClfA is a member of a family of proteins containing the carboxyl terminal LPXTG (SEQ ID NO: 125) motif that enables the protein to become covalently linked to the cell surface. ClfA also belongs to another family of proteins (Microbial Surface Components Recognizing Adhesive Matrix Molecule, or MSCRAMMs) that are associated with binding host proteins such as fibrinogen (bound by ClfA), the fibronectin binding proteins (FnbA and FnbB), the collagen binding protein (Cna) and others. These proteins all share the amino terminal signal sequence that mediates transport to the cell surface. The MSCRAMMs also include an A-domain that is the functional region containing the active site for ligand binding (*e.g*., fibrinogen, fibronectin, elastin, keratin). The A-domain is followed by a region composed of serine aspartate repeats (SD repeat), which is thought to span the peptidoglycan layer. The SD repeat is followed by a membrane-spanning region that includes the LPXTG (SEQ ID NO: 125) motif for covalent linkage of the protein to peptidoglycan. ClfA is described in U.S. Pat. No. 6,008,341.

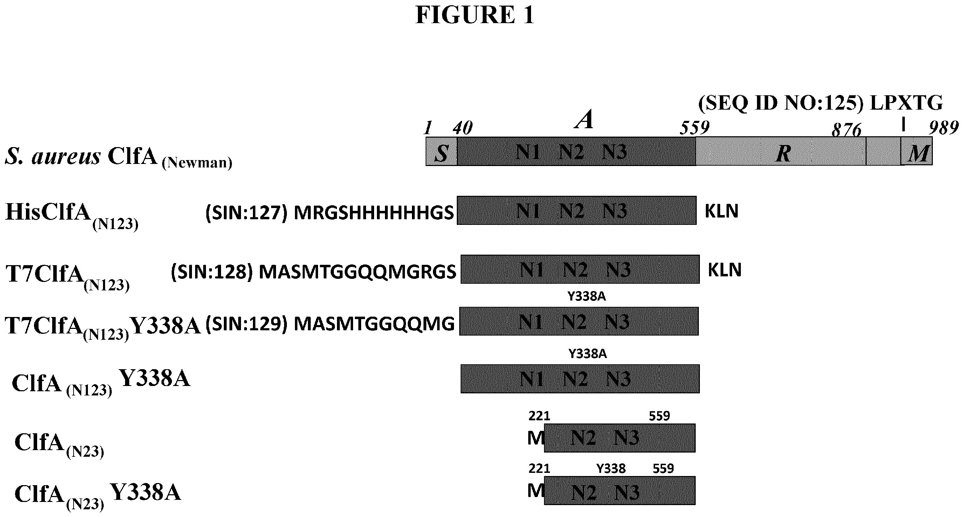

The ligand binding region of ClfA comprising N1N2N3 of the A domain (Figure 1) spans amino acids 40-559. The N domains of ClfA have been assigned as follows: N1 encompasses residues 45-220; N2 encompasses residues 229-369; and N3 encompasses residues 370-559. See Deivanayagam et al. EMBO J. 21:6660-6672 (2002). For ease of reference the N1N2N3 domains may be referred to as N123, likewise N2N3 may be referred to as N23. In preparations of recombinant N1N2N3, the N1 domain has been found to be protease sensitive and is easily cleaved or hydrolyzed to leave the N2N3 as a stable ligand binding recombinant fragment. See Deivanayagam et al. EMBO J. 21:6660-6672 (2002). The crystal structure of the fibrinogen binding N2N3 fragment of ClfA A domain, revealed that both N2 and N3 are dominated by anti-parallel beta strands. In addition to the anti-parallel beta strands, the N2 domain contains a single turn alpha helix and two 3₁₀ helices and the N3 domain contains three 3₁₀ helices. See Deivanayagam et al. EMBO J. 21:6660-6672 (2002). Sequence alignment of N2 and N3 reveals only 13% sequence identity and 36% sequence similarity over their lengths. See Deivanayagam et al. EMBO J. 21:6660-6672 (2002). The topology of the N2 and N3 domains are similar to the classic IgG fold and have been proposed to be novel variants of the IgG fold. See Deivanayagam et al. EMBO J. 21:6660-6672 (2002).

Disclosed herein is a composition comprising a stable Staphylococcus aureus clumping factor A ("ClfA") polypeptide, and methods for making same, which is useful as an immunogenic composition or vaccine. Also disclosed herein is a composition comprising a stable ClfA polypeptide, a CP5 conjugate, and a CP8 conjugate ("tri-antigen"), and methods for making same, which is useful as an immunogenic composition or vaccine. Further disclosed herein is a composition comprising a stable ClfA polypeptide, a CP5 conjugate, a CP8 conjugate, and an MntC polypeptide ("tetra-antigen"), and methods for making same, which is useful as an immunogenic composition or vaccine. ClfA is known to be an unstable protein, which readily undergoes clipping between the N1 and N2 domains. This application is directed to a ClfA formulation which enables ClfA N1N2N3 to remain substantially undegraded under accelerated stress conditions for at least three months. In some embodiments, the formulation is a lyophilized composition that comprises less than three percent water. The term "lyophilized composition" refers to the formulation having less than 3% water and is not meant to limit the composition to the method of which the composition was made, e.g., lyophilization or any other method of drying a mixture, or compounding dry ingredients. By accelerated stress conditions, what is meant are conditions of extreme temperature, pH and/or ionic strength, generally used to test the stability of a formulation, such as, e.g., 37°C for several weeks or more.

Thus, in one embodiment, the disclosure is directed to a stable composition comprising a substantially undegraded ClfA protein and water at less than three percent. In one embodiment, the disclosure is directed to a stable composition comprising a substantially undegraded ClfA protein, a capsular polysaccharide Type 5 (CP5) conjugate, a CP8 conjugate, and water at less than three percent. In one embodiment, the disclosure is directed to a stable composition comprising a substantially undegraded ClfA protein, a capsular polysaccharide Type 5 (CP5) conjugate, a CP8 conjugate, an MntC protein, and water at less than three percent. The ClfA protein can be isolated from any Staphylococcus strain or it can be a recombinant polypeptide. A recombinant ClfA polypeptide may have a wildtype sequence, may comprise one or more mutations, or may be at least 90% identical in amino acid sequence to a wildtype sequence. Table 1 depicts several *S. aureus* strains and their respective ClfA DNA and protein sequences.

**Table 1: ClfA strains and Sequence Listings**

| Example Strain | DNA-ClfA | NT SEQ ID NO: | Protein-ClfA | AA SEQ ID NO: | % Identity to Antigen |
|---|---|---|---|---|---|
| PFESA0131 | clfA_001-1 | 61 | clfA_001 | 62 | 99 |
| PFESA0074 | clfA_002-1 | 63 | clfA_002 | 64 | 92 |
| PFESA0072 | clfA_003-1 | 65 | clfA_003 | 66 | 99 |
| PFESA0159 | clfA_004-1 | 67 | clfA 004 | 68 | 94 |
| PFESA0154 | clfA_005-1 | 69 | clfA_005 | 70 | 91 |
| PFESA0096 | clfA_006-1 | 71 | clfA_006 | 72 | 91 |
| PFESA0269 | clfA_007-1 | 73 | clfA_007 | 74 | 91 |
| PFESA0081 | clfA_008-1 | 75 | clfA_008 | 76 | 97 |
| PFESA0005 | clfA_009-1 | 77 | clfA_009 | 78 | 95 |
| PFESA0139 | clfA_010-1 | 79 | clfA_010 | 80 | 99 |
| PFESA0237 | clfA_011-1 | 81 | clfA_011 | 82 | 100 |
| PFESA0157 | clfA_012-1 | 83 | clfA_012 | 84 | 96 |
| PFESA0069 | clfA_013-1 | 85 | clfA_013 | 86 | 92 |
| PFESA0002 | clfA_014-1 | 87 | clfA_014 | 88 | 98 |
| PFESA0147 | clfA_015-1 | 89 | clfA_015 | 90 | 91 |
| PFESA0094 | clfA_016-1 | 91 | clfA_016 | 92 | 98 |
| PFESA0143 | clfA_017-1 | 93 | clfA_017 | 94 | 97 |
| PFESA0129 | clfA_018-1 | 95 | clfA_018 | 96 | 99 |
| PFESA0128 | clfA_019-1 | 97 | clfA_019 | 98 | 92 |
| PFESA0148 | clfA_020-1 | 99 | clfA_020 | 100 | 91 |
| PFESA0140 | clfA_021-1 | 101 | clfA_021 | 102 | 98 |
| PFESA0152 | clfA_022-1 | 103 | clfA_022 | 104 | 91 |
| PFESA0141 | clfA_023-1 | 105 | clfA_023 | 106 | 96 |
| PFESA0 160 | clfA_024-1 | 107 | clfA_024 | 108 | 94 |

### ClfA Sequence

The gene for clumping factor protein A, designated ClfA, has been cloned, sequenced and analyzed in detail at the molecular level (McDevitt et al., Mol. Microbiol. 11: 237-248 (1994); McDevitt et al., Mol. Microbiol. 16:895-907 (1995)). The sequence identifiers for the amino acid sequences of ClfA from 111 *S. aureus* disease-causing isolates are shown in Table 1. The amino acid sequence of the full length (including the signal sequence) wild type ClfA from *S. aureus* strain PFESA0237, is shown in SEQ ID NO: 130. This sequence shows a tyrosine at position 338, which is changed to an alanine in the mutated form of ClfA. The full length gene encoding the wild type ClfA from *S. aureus* strain PFESA0237, comprising the N123 region, the repeat region and the anchor region is shown in SEQ ID NO: 131. The amino acid sequence of the Y338A mutated forms of ClfA is shown in SEQ ID NO: 123. However, it should be noted that the change from a tyrosine to an alanine, which occurs in the wild type ClfA at position 338 of SEQ ID NO: 130, and which is designated as Y338A, is shown in the mutated form of ClfA, in SEQ ID NO: 123 at position 310. Furthermore, the mutated form of ClfA shown in the amino acid sequence of SEQ ID NO: 123 is the mature form of ClfA without the signal sequence, thus accounting for the difference in position of this mutation between SEQ ID NO: 130 and SEQ ID NO: 123.

In one embodiment, the ClfA protein comprises the sequence of SEQ ID NO: 123, which comprises a Y388A mutation abolishing fibrinogen binding.

In some embodiments, the ClfA protein need not be full-length and may consist of amino acids 50-597 (as numbered according to SEQ ID NO: 130 but which can be based on any ClfA protein).

In some embodiments, the ClfA protein comprises or consists essentially of an N1 domain, an N2 domain, and an N3 domain. In some embodiments, the ClfA protein comprises an N domain. By N domain, what is meant is an N1 domain, an N2 domain, or an N3 domain.

ClfA proteins, their methods of making and using are described in copending patent application U.S. Patent Application serial number 61/219,134, filed June 22, 2009. ClfA proteins are also described in International Patent Application No. PCT/US2010/039510.

By stable, what is meant is that the ClfA remains substantially undegraded under accelerated or other stress conditions and over extended periods of time, such as in storage. By substantially undegraded, what is meant is that at least 70%, 80%, 90%, 95% or 99% of the total cumulative population of ClfA polypeptides contains polypeptides comprising N1 sequences and additional sequences of N2 and/or N3. By intact, what is meant is that the ClfA protein contains at least the N1, N2, and N3 domains. In some embodiments, the ClfA remains stable for at least two weeks, at least one month, or at least three months at 37°C.

In some embodiments, a lyophilized composition of the disclosure contains an intact ClfA in an amount that is less than one percent by weight of the total weight of the lyophilized immunogenic composition (1% [w/w]) In some embodiments, the ClfA is an amount between 0.09% ± 0.027% and 0.85% ± 0.26%.

### ClfB: Domain Organization

ClfB is a *S. aureus* protein having fibrinogen binding activity and triggers *S. aureus* to form clumps in the presence of plasma. ClfB is an MSCRAMM protein and displays the characteristic MSCRAMM domain organization including an A-domain that is the functional region containing the active site for ligand binding (*e.g*., fibrinogen, fibronectin, elastin, keratin). The A-domain is followed by a region composed of serine aspartate repeats (SD repeat), which is thought to span the peptidoglycan layer. The SD repeat is followed by a membrane-spanning region that includes the LPXTG (SEQ ID NO: 125) motif for covalent linkage of the protein to peptidoglycan. ClfB is described in WO 99/27109 and in US Patent 6,680,195.

The internal organization of ClfB N-terminal A domain is very similar to the organization as found in ClfA. The A domain is composed of three subdomains N1, N2, and N3. The ligand binding region of ClfB comprising N1N2N3 of the A domain spans amino acids 44-585. For ease of reference the N1N2N3 domains may be referred to as N123, likewise N2N3 may be referred to as N23. The N domains of ClfB have been assigned as follows: N1 encompasses residues 44-197; N2 encompasses residues 198-375; and N3 encompasses residues 375-585. In ClfA, the crystal structure of the A domain was found to have a unique version of the immunoglobulin fold and by analogy the same may be speculated to be the case for ClfB. See Deivanayagam et al., EMBO J. 21:6660-6672 (2002). Even though organization of the A domains of ClfB and ClfA are similar, sequence identity is only 26%. See Ni Eidhin et al., Mol. Microbiol. 30:245-257 (2002).

### ClfB Sequence

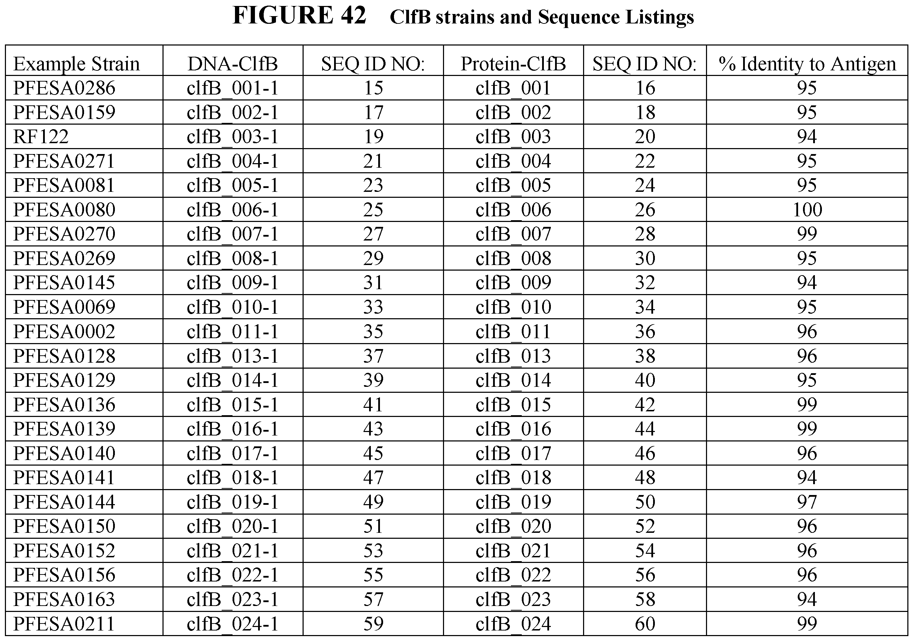

The gene encoding ClfB is classified as a core adhesion gene. ClfB sequences from 92 strains of *S. aureus* associated with multiple disease states are summarized in Figure 42. Additional sequences were obtained from GenBank.

### Other MSCRAMMS

Other MSCRAMMS may be considered for use in an immunogenic composition of the present invention. For example, the serine-aspartate repeat (Sdr) proteins, SdrC, SdrD, and SdrE are related in primary sequence and structural organization to the ClfA and ClfB proteins and are localized on the cell surface. The SdrC, SdrD and SdrE proteins are cell wall-associated proteins, having a signal sequence at the N-terminus and an LPXTG (SEQ ID NO: 125) motif, hydrophobic domain and positively charged residues at the C-terminus. Each also has an SD repeat containing region R of sufficient length to allow, along with the B motifs, efficient expression of the ligand binding domain region A on the cell surface. With the A region of the SdrC, SdrD and SdrE proteins located on the cell surface, the proteins can interact with proteins in plasma, the extracellular matrix or with molecules on the surface of host cells. The Sdr proteins share some limited amino acid sequence similarity with ClfA and ClfB. Like ClfA and ClfB, SdrC, SdrD and SdrE also exhibit cation-dependent ligand binding of extracellular matrix proteins.

The sdr genes are closely linked and tandemly arrayed. The Sdr proteins (of SdrC, SdrD, SdrE, ClfA, and ClfB) characteristically comprise an A region where there is highly conserved amino acid sequence that can be used to derive a consensus TYTFTDYVD (SEQ ID NO: 126) motif. The motif exhibits slight variation between the different proteins. This variation, along with the consensus sequence of the motif is described in US Patent 6,680,195. In the Clf-Sdr proteins, this motif is highly conserved. The motif can be used in immunogenic compositions to impart broad spectrum immunity to bacterial infections, and also can be used as an antigen in the production of monoclonal or polyclonal antibodies. Such an antibody can be used to impart broad spectrum passive immunity.

The Sdr proteins differ from ClfA and ClfB by having two to five additional 110-113 residue repeated sequences (B-motifs) located between region A and the R-region. Each B-motif contains a consensus Ca²⁺-binding EF-hand loop normally found in eukaryotic proteins. The structural integrity of a recombinant protein comprising the five B-repeats of SdrD was shown by bisANS fluorescence analysis to be Ca²⁺ -dependent, suggesting that the EF-hands are functional. When Ca²⁺ was removed the structure collapsed to an unfolded conformation. The original structure was restored by addition of Ca²⁺. The C-terminal R-domains of the Sdr proteins contain 132-170 SD residues. These are followed by conserved wall-anchoring regions characteristic of many surface proteins of Gram positive bacteria.

In the Sdr and Clf proteins this B motif is highly conserved while a degenerate version occurs in fibronectin binding MSCRAMMS, as well as the collagen binding protein Cna. The B motifs, in conjunction with the R regions, are necessary for displaying the ligand-binding domain at some distance from the cell surface. The repeated B motifs are one common denominator of the sub-group of SD repeat proteins described herein. These motifs are found in different numbers in the three Sdr proteins from strain PFESA0237. There are clear distinctions between the individual B motifs. The most conserved units are those located adjacent to the R regions (SdrC B2, SdrD B5 and SdrE B3). They differ from the rest at several sites, especially in the C-terminal half. A noteworthy structural detail is that adjacent B repeats are always separated by a proline residue present in the C-terminal region, but a proline never occurs between the last B repeats and the R region. Instead this linker is characterized by a short acidic stretch. These differences are evidence that the end units have a different structural or functional role compared to the other B motifs. The N-terminal B motifs of SdrD and SdrE have drifted apart from the others, and there are numerous amino acid alterations, including small insertions and deletions whereas the remaining internal B motifs are more highly conserved. Note that each of the three Sdr proteins has at least one B motif of each kind.

The C-terminal R-domains of the Sdr proteins contain 132-170 SD residues. These are followed by conserved wall-anchoring regions characteristic of many surface proteins of Gram positive bacteria.

Other candidate SdrD molecules may be derived from various species of organisms for use in an immunogenic composition of the invention, some of which include the following SdrD from *S. aureus:* strain USA300 FPR3757 (protein accession number SAUSA300 0547); strain NCTC8325 (protein accession number SAOUHSC 00545); strain MW2 (protein accession number MW0517); strain MSSA476 (protein accession number SAS0520; and strain Mu50 (protein accession number SAV0562).

Further MSCRAMMS which may be considered for use in an immunogenic composition of the present invention include EkeS, DsqA, KesK, KrkN, KrkN2, RkaS, RrkN, and KnkA. These MSCRAMMS are described in WO 02/102829. Additional MSCRAMMS, identified by GenBank Accession No., include NP_373261.1, NP_373371.1, NP_374246.1, NP_374248.1, NP_374841.1, NP_374866.1, NP_375140.1, NP_375614.1, NP_375615.1, NP_375707.1, NP_375765.1, and NP_375773.1.

### Capsule Polysaccharides Type 5 and Type 8

Staphylococcal microorganisms capable of causing invasive disease generally also are capable of producing a capsule polysaccharide (CP) that encapsulates the bacterium and enhances its resistance to clearance by host innate immune system. The CP serves to cloak the bacterial cell in a protective capsule that renders the bacteria resistant to phagocytosis and intracellular killing. Bacteria lacking a capsule are more susceptible to phagocytosis. Capsular polysaccharides are frequently an important virulence factor for many bacterial pathogens, including *Haemophilus influenzae, Streptococcus pneumoniae* and Group B streptococci.

The capsule polysaccharide can be used to serotype a particular species of bacteria. Typing is usually accomplished by reaction with a specific antiserum or monoclonal antibody generated to a specific structure or unique epitope characteristic of the capsule polysaccharide. Encapsulated bacteria tend to grow in smooth colonies whereas colonies of bacteria that have lost their capsules appear rough. Colonies producing a mucoid appearance are known as Heavily Encapsulated. Types 1 and 2 of *S. aureus* are heavily encapsulated and are rarely associated with disease.

Most clinical isolates *of S. aureus* are encapsulated with either serotypes 5 or 8. The type 5 (CP5) and type 8 (CP8) capsular polysaccharides have similar trisaccharide repeating units comprised of N-acetyl mannosaminuronic acid, N-acetyl L-fucosamine, and N-acetyl D-fucosamine. See Fournier, J.M. et al., Infect. Immun. 45:97-93 (1984) and Moreau, M., et al., Carbohydrate Res. 201:285-297 (1990). The two CPs have the same sugars, but differ in the sugar linkages and in sites of O acetylation to produce serologically distinct patterns of immunoreactivity.

In some embodiments, the serotype 5 and/or 8 capsular polysaccharides of the invention are O-acetylated. In some embodiments, the degree of O-acetylation of type 5 capsular polysaccharide or oligosaccharide is 10-100%, 20-100%, 30-100%, 40-100%, 50-100%. 60-100%, 70-100%, 80-100%, 90-100%, 50- 90%, 60-90%, 70-90% or 80-90%. In some embodiments, the degree of O-acetylation of type 8 capsular polysaccharide or oligosaccharide is 10-100%, 20-100%, 30-100%, 40-100%, 50-100%. 60-100%, 70-100%, 80-100%, 90-100%, 50-90%, 60-90%, 70-90% or 80-90%. In some embodiments, the degree of O-acetylation of type 5 and type 8 capsular polysaccharides or oligosaccharides is 10-100%, 20-100%, 30-100%, 40-100%, 50-100%. 60-100%, 70-100%, 80-100%, 90-100%, 50-90%, 60-90%, 70-90% or 80-90%.

The degree of O-acetylation of the polysaccharide or oligosaccharide can be determined by any method known in the art, for example, by proton NMR (Lemercinier and Jones 1996, Carbohydrate Research 296; 83-96, Jones and Lemercinier 2002, J Pharmaceutical and Biomedical Analysis 30; 1233-1247, WO 05/033148 or WO 00/56357). Another commonly used method is described by Hestrin (1949) J. Biol. Chem. 180; 249-261.

In some embodiments, the serotype 5 and/or 8 capsular polysaccharides of the invention are used to generate antibodies that are functional as measured by the killing of bacteria in an animal efficacy model or an opsonophagocytic killing assay that demonstrates that the antibodies kill the bacteria. Such functionality may not be observed using an assay that monitors the generation of antibodies alone, which is not indicative of the importance of O-acetylation in efficacy.

### Capsule Epidemiology

The association of particular capsule serotypes with disease is possible through monitoring of clinical isolates. Of the eight different serotypes of *S. aureus* identified (Karakawa and Vann (1982)) only serotypes 1 and 2 are heavily encapsulated, and these are rarely isolated. See Capsular Polysaccharides of Staphylococcus aureus, p. 285-293, In J.B. Robbins, J.C. Hill and J.C. Sadoff (ed.), Seminars in infectious disease, vol. 4, Bacterial Vaccines. Thieme Stratton, Inc. New York. Surveys have shown that approximately 85-90% of *S. aureus* clinical isolates express CP5 or CP8 (Arbeit RD, et al., Diagn. Microbiol. Infect. Dis. (1984) Apr;2(2):85-91; Karakawa WW, et al., J. Clin. Microbiol. (1985) Sep;22(3):445-7; Essawi T, et al., Trop. Med. Int. Health. (1998) Jul;3(7):576-83; Na'was T, et al., J. Clin. Microbiol. (1998) 36(2):414-20). Most of CP5 and CP8 non-typeable strains are genetically type 5 or type 8 containing mutations in *cap5*/*8* locus (Cocchiaro, Gomez et al., (2006), Mol. Microbiol. Feb. 59(3):948-960). Capsulation for some strains is lost rapidly within few passages *in vitro* which is due to a repressive effect of high phosphate concentration in media used in clinical diagnosis on capsule production. It was also reported that non-capsulated isolates recover capsule expression after passing through cows. See Opdebeck, J.P. et al., J. Med. Microbiol. 19:275-278 (1985). Some non-typeable strains become capsule positive under appropriate growth conditions.

### CP5 and CP8 Structure

The repeat unit of both CP5 and CP8 is composed of 2-acetamido-2-deoxy-D-mannuronic acid, 2-acetamido-2-deoxy-L-fucose and 2-acetamido-2-deoxy-D-fucose. See C. Jones et al., Carbohydr. Res. 340:1097-1106 (2005). Although CP5 and CP8 have the same sugar composition, they have been demonstrated to be immunologically distinct. They differ in glycosidic linkages and site of O-acetylation of uronic acid. Strain dependent incomplete N-acetylation of one of the FucNAc residues was observed. See Tzianabos et al., PNAS V98: 9365(2001).

### S. aureus Capsule Polysaccharide in an Immunogenic Composition

The molecular weight of the *S. aureus* capsule polysaccharides is an important consideration for use in immunogenic compositions. High molecular weight capsule polysaccharides are able to induce certain antibody immune responses due to a higher valency of the epitopes present on the antigenic surface. The methods described herein provide for isolation and purification of much higher molecular weight capsule polysaccharide type 5 and type 8 than was previously available.

### MntC/SitC /Saliva Binding Protein

MntC/SitC /Saliva Binding Protein is an ABC transporter protein and has homologues in *S. epidermidis* and *S. aureus.* It is referred to in the present invention as MntC. This protein is a 32 kDa lipoprotein and is located in the bacterial cell wall. See Sellman et al., and Cockayne et al., Infect. Immun. 66: 3767(1998). In *S. epidermidis,* it is a component of an iron-regulated operon. It shows considerable homology to both adhesins including FimA of *S. parasanguis,* and with lipoproteins of a family of ABC transporters with proven or putative metal iron transport functions. (See Table 2 for strains *of S. aureus* and sequences.)

### S. aureus MntC protein

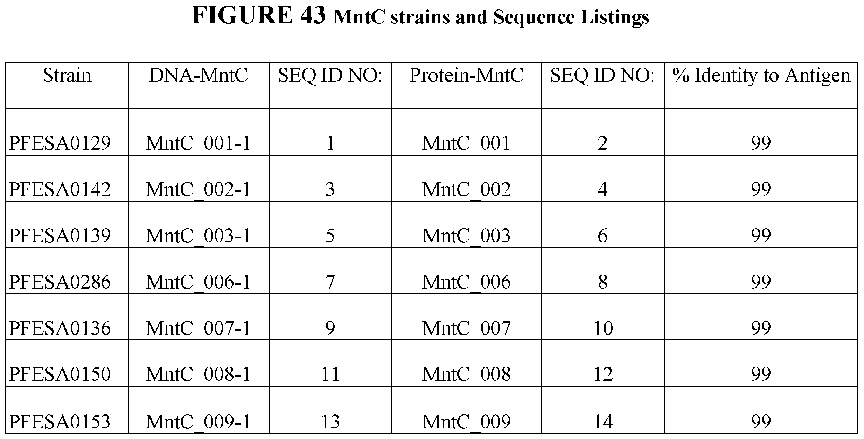

The *S. aureus* homologue of MntC is known as saliva binding protein and was disclosed in U.S. Pat. No. 5,801,234 and can be included in an immunogenic composition of the invention. The protein sequence for the *S. aureus* homologue of MntC/SitC/Saliva Binding Protein is found in GenBank accession number NP_371155 for strain Mu50 (also known as SAV0631). The sequence identifier is SEQ ID NO: 134. The accession number for the nucleotide sequence for the complete genome of strain Mu50 is NC_002758.2. The coordinates for the coding DNA sequence for the protein sequence found in GenBank accession number NP_371155 is 704988-705917 (SEQ ID NO: 135). In certain embodiments, the N-terminus of MntC is cleaved as shown in SEQ ID NO: 119 (polypeptide sequence) and SEQ ID NO: 136 (nucleotide sequence encoding the polypeptide). MntC sequences from different strains of *S. aureus* are summarized in Figure 43.

### S. epidermidis SitC protein

The *S. epidermidis* homologue of MntC/SitC/Saliva Binding Protein is known as SitC and was disclosed in Sellman et al., (Sellman et al., Infect. Immun. 2005 October; 73(10): 6591-6600). The protein sequence for the *S. epidermidis* homologue of MntC/SitC/Saliva Binding Protein is found in GenBank accession number YP_187886.1 (also known as SERP0290). The sequence identifier is SEQ ID NO: 132.

The accession number for the nucleotide sequence for the complete genome of strain RP62A, is NC_002976. The coordinates for the coding DNA sequence of the protein sequence found in GenBank accession number YP_187886.1 is 293030-293959 (SEQ ID NO:133). The corresponding N-terminus truncated version of SitC is shown in SEQ ID NO: 121 (polypeptide sequence) and SEQ ID NO: 137 (nucleotide sequence encoding the polypeptide). Other candidate SitC molecules may be derived from various species of organisms for use in an immunogenic composition of the invention, some of which are listed in Table 2 below.

**Table 2**

| Protein | Species | Example strain | Protein Accession |
|---|---|---|---|
| SitC | S. haemolyticus | JCSC1435 | BAE03450.1 |
| SitC | S. epidermidis | ATCC 12228 | AAO04002.1 |
| SitC | S. saprophyticus | ATCC 15305 | BAE19233.1 |
| SitC | S. xylosus | DSM20267 | ABR57162.1 |
| SitC | S. carnosus | TM300 | CAL27186.1 |

### S. aureus Iron Binding Proteins

Another potential candidate antigen to be considered for use in the immunogenic compositions of the invention include the *S. aureus* surface protein iron surface determinant B (IsdB). This MSCRAMM was described by Mazmanian et al. (Mazmanian, SK et al. Proc. Natl. Acad. Sci., USA 99:2293-2298 (2002)) and it has subsequently been tested and shown to be effective as a vaccine candidate in a murine model of infection and a rhesus macaque immunogenicity study by Kuklin, et al. (Kuklin, NA, et al. Infection and Immunity, Vol. 74, No. 4, 2215-2223, (2006)). This IsdB molecule is present in various strains of *S. aureus,* including strain MRSA252 (protein accession number CAG40104.1); strain Newman (protein accession number BAF67312.1); strain MSSA476 (protein accession number CAG42837.1); strain Mu3 (protein accession number BAF78003.1); strain RF122 (protein accession number CAI80681.1).

### Candidate Antigens:

The immunogenic compositions of the present invention may also include one or more of the following antigens: Opp3a, DltD, HtsA, LtaS, IsdA, IsdC, SdrF, SdrG, SdrH, SrtA, SpA, Sbi, alpha-hemolysin (hla), beta-hemolysin, fibronectin-binding protein A (fnbA), fibronectin-binding protein B (fnbB), coagulase, Fig, map, Panton-Valentine leukocidin (pvl), alpha-toxin and its variants, gamma-toxin (hlg) and variants, ica, immunodominant ABC transporter, Mg2+ transporter, Ni ABC transporter, RAP, autolysin, laminin receptors, IsaA/PisA, IsaB/PisB , SPOIIIE, SsaA, EbpS, Sas A, SasF, SasH, EFB (FIB), SBI, Npase, EBP, bone sialo binding protein II, aureolysin precursor (AUR)/Seppl, Cna, and fragments thereof such as M55, TSST-1, mecA, poly-N-acetylglucosamine (PNAG/dPNAG) exopolysaccharide, GehD, EbhA, EbhB, SSP-1, SSP-2, HBP, vitronectin binding protein, HarA, EsxA, EsxB, Enterotoxin A, Enterotoxin B, Enterotoxin Cl, and novel autolysin. In certain embodiments of the invention, when the immunogenic composition comprises certain forms of CP5 and/or CP8, it may not further comprise PNAG.

### Immunogenic Composition Formulations

In one embodiment, the lyophilized immunogenic compositions of the disclosure further comprise at least one of an adjuvant, a buffer, a cryoprotectant, a salt, a divalent cation, a non-ionic detergent, an inhibitor of free radical oxidation, a bulking agent, or a carrier.

The immunogenic compositions of the invention may further comprise one or more preservatives in addition to a plurality of staphylococcal protein antigens and capsular polysaccharide-protein conjugates. The FDA requires that biological products in multiple-dose (multi-dose) vials contain a preservative, with only a few exceptions. Vaccine products containing preservatives include vaccines containing benzethonium chloride (anthrax), 2-phenoxyethanol (DTaP, HepA, Lyme, Polio (parenteral)), phenol (Pneumo, Typhoid (parenteral), Vaccinia) and thimerosal (DTaP, DT, Td, HepB, Hib, Influenza, JE, Mening, Pneumo, Rabies). Preservatives approved for use in injectable drugs include, e.g., chlorobutanol, m-cresol, methylparaben, propylparaben, 2-phenoxyethanol, benzethonium chloride, benzalkonium chloride, benzoic acid, benzyl alcohol, phenol, thimerosal and phenylmercuric nitrate.

Formulations of the invention may further comprise one or more of a buffer, a salt, a divalent cation, a non-ionic detergent, a cryoprotectant such as a sugar, a bulking agent, and an anti-oxidant such as a free radical scavenger or chelating agent, or any multiple combination thereof. The choice of any one component, e.g., a chelator, may determine whether or not another component (e.g., a scavenger) is desirable. The final composition formulated for administration should be sterile and/or pyrogen free. The skilled artisan may empirically determine which combinations of these and other components will be optimal for inclusion in the preservative containing immunogenic compositions of the invention depending on a variety of factors such as the particular storage and administration conditions required.

In some embodiments, the lyophilized composition further contains a buffer that has a pKa of 6.0 ± 0.6 in an amount that is less than 3% (w/w). In certain embodiments, the formulation is buffered to within a pH range of about 6.0 to about 9.0, preferably from about 7.5 to about 7.5. In some embodiments, the buffer is at a concentration of 2.54% ± 0.76% (w/w). In some embodiments, the buffer comprises succinate at pH 6.0 ± 0.6. In some embodiments, the buffer comprises histidine at pH 6.0 ± 0.6. In some embodiments, the buffer comprises histidine at pH 6.5 ± 0.6. Table 3 lists some non-limiting examples of buffers which may be used in the practice of this invention.

**Table 3: Buffers**

| buffer | pH range | pKa |
|---|---|---|
| maleate | 4.0-6.0 | 5.13 |
| pyridine | 4.9-5.9 | 5.23 |
| piperazine (pK1) | 5.0-6.0 | 5.33 |
| cacodylate | 5.0-7.4 | 6.27 |
| succinate | 5.5-6.5 | 5.64 |
| MES | 5.5-6.7 | 6.10 |
| citrate | 5.5-7.2 | 6.40 |
| maleate | 5.5-7.2 | 6.24 |
| histidine | 5.5-7.4 | 1.70, 6.04, 9.09 |
| bis-tris | 5.8-7.2 | 6.46 |
| phosphate | 5.8-8.0 | 7.20 |
| ADA | 6.0-7.2 | 6.59 |
| carbonate | 6.0-8.0 | 6.35 |

In certain embodiments, it may be desirable to adjust the pH of the lyophilized immunogenic composition or formulation of the disclosure. The pH of a formulation of the invention may be adjusted using standard techniques in the art. The pH of the formulation may be adjusted to be between 3.0 and 8.0. In certain embodiments, the pH of the formulation may be, or may be adjusted to be, between 3.0 and 6.0, 4.0 and 6.0, 5.0 and 7.0, or 5.0 and 8.0. In other embodiments, the pH of the formulation may be, or maybe adjusted to be, about 3.0, about 3.5, about 4.0, about 4.5, about 5.0, about 5.5, about 5.8, about 6.0, about 6.5, about 7.0, about 7.5, or about 8.0. In certain embodiments, the pH may be, or may be adjusted to be, in a range from 4.5 to 7.5, or from 4.5 to 6.5, from 5.0 to 5.4, from 5.4 to 5.5, from 5.5 to 5.6, from 5.6 to 5.7, from 5.7 to 5.8, from 5.8 to 5.9, from 5.9 to 6.0, from 6.0 to 6.1, from 6.1 to 6.2, from 6.2 to 6.3, from 6.3 to 6.4, from 6.4 to 6.5, from 6.5 to 6.6, from 6.6 to 6.7, from 6.7 to 6.8, from 6.8 to 6.9, from 6.9 to 7.0, from 6.5 to 7.0, from 7.0 to 7.5 or from 7.5 to 8.0. In a specific embodiment, the pH of the formulation is about 6.0. In a specific embodiment, the pH of the formulation is about 6.5.

In some embodiments, the lyophilized immunogenic composition comprises a bulking agent. The bulking agent generally serves to provide bulk to the composition and facilitate visualization of the drug, which is generally in such low amounts per dose that the dry pellet of a drug is invisible or barely visible, and/or prevents blowout of active ingredients from vial, such as during lyophilization. The bulking agent also can serve to facilitate precipitating the drug agent. Bulking agents can also serve as cryoprotectants. A variety of cryoprotectants are described in conventional texts, such as Remington: The Science and Practice of Pharmacy, Vol. 2, 19th edition (1995).

Non-limiting examples of bulking agents include sugar alcohols, such as alditol, mannitol, sorbitol, inositol, and polyethylene glycol, sugar acids, such as aldonic acid, uronic acid, and aldaric acid, and carbohydrates, such as aldoses, ketoses, amino sugars, alditols, inositols, aldonic acids, uronic acids, aldaric acids, mono-, a di-, or poly-, carbohydrates, glyceraldehyde, arabinose, lyxose, pentose, ribose, xylose, galactose, glucose, hexose, idose, mannose, talose, heptose, glucose, fructose, gluconic acid, sorbitol, lactose, mannitol, methyl a-glucopyranoside, maltose, isoascorbic acid, ascorbic acid, lactone, sorbose, glucaric acid, erythrose, threose, arabinose, allose, altrose, gulose, idose, talose, erythrulose, ribulose, xylulose, psicose, tagatose, glucuronic acid, gluconic acid, glucaric acid, galacturonic acid, mannuronic acid, glucosamine, galactosamine, sucrose, trehalose, neuraminic acid, arabinans, fructans, fucans, galactans, galacturonans, glucans, mannans, xylans (such as, for example, inulin), levan, fucoidan, carrageenan, galactocarolose, pectins, pectic acids, amylose, pullulan, glycogen, amylopectin, cellulose, dextran, pustulan, chitin, agarose, keratin, chondroitin, dermatan, hyaluronic acid, alginic acid, xanthin gum, or starch.

In some embodiments, the bulking agent is any one or more of sucrose, mannitol, glycine and sorbitol. In some embodiments, the bulking agent is sucrose. In some embodiments, the sucrose is at greater than 91% (w/w). In some embodiments, the sucrose is at 96% ± 2.0% (w/w).

In certain embodiments, a formulation of the lyophilized or reconstituted lyophilized composition which is compatible with parenteral administration comprises one or more divalent cations, including but not limited to MgCl2, CaCl₂ and MnCl₂, at a concentration ranging from about 0.1 mM to about 10 mM, with up to about 5 mM being preferred.

In certain embodiments, a formulation of the lyophilized or reconstituted lyophilized composition which is compatible with parenteral administration comprises one or more salts, including but not limited to sodium chloride, potassium chloride, sodium sulfate, and potassium sulfate, present at an ionic strength which is physiologically acceptable to the subject upon parenteral administration and included at a final concentration to produce a selected ionic strength or osmolarity in the final formulation. The final ionic strength or osmolality of the formulation will be determined by multiple components (e.g., ions from buffering compound(s) and other non-buffering salts. A preferred salt, NaCl, is present from a range of up to about 250 mM, with salt concentrations being selected to complement other components (e.g., sugars) so that the final total osmolarity of the formulation is compatible with parenteral administration (e.g., intramuscular or subcutaneous injection) and will promote long term stability of the immunogenic components of the immunogenic composition formulation over various temperature ranges. Salt-free formulations will tolerate increased ranges of the one or more selected cryoprotectants to maintain desired final osmolarity levels.

In certain embodiments, a formulation of the lyophilized or reconstituted lyophilized composition which is compatible with parenteral administration comprises one or more cryoprotectants selected from but not limited to disaccharides (e.g., lactose, maltose, sucrose or trehalose) and polyhydroxy hydrocarbons (e.g., dulcitol, glycerol, mannitol and sorbitol).

In certain embodiments, the osmolarity of a reconstituted lyophilized formulation is in a range of from about 200 mOs/L to about 800 mOs/L, with a preferred range of from about 250 mOs/L to about 500 mOs/L, or about 300 mOs/L to about 400 mOs/L. A reconstituted lyophilized formulation may contain, for example, from about 0% to about 25% sucrose, from about 3% to about 15%, and from about 5 to about 10% sucrose. Alternatively, a reconstituted lyophilized formulation may contain, for example, from about 3% to about 12% sorbitol. If salt such as sodium chloride is added, then the effective range of sucrose or sorbitol may or may not be relatively decreased. These and other such osmolality and osmolarity considerations are well within the skill of the art.

In certain embodiments, a lyophilized or reconstituted lyophilized formulation of the disclosure which is compatible with parenteral administration comprises one or more free radical oxidation inhibitors and/or chelating agents. A variety of free radical scavengers and chelators are known in the art and apply to the formulations and methods of use described herein. Examples include but are not limited to ethanol, EDTA, a EDTA/ethanol combination, triethanolamine, mannitol, histidine, glycerol, sodium citrate, inositol hexaphosphate, tripolyphosphate, ascorbic acid/ascorbate, succinic acid/succinate, malic acid/maleate, desferal, EDDHA and DTPA, and various combinations of two or more of the above. In certain embodiments, at least one non-reducing free radical scavenger may be added at a concentration that effectively enhances long term stability of the formulation. One or more free radical oxidation inhibitors/chelators may also be added in various combinations, such as a scavenger and a divalent cation. The choice of chelator will determine whether or not the addition of a scavenger is needed.

In some embodiments, the lyophilized immunogenic composition comprises a surfactant at less than 0.5% (w/w). In some embodiments, the surfactant is at 0.21% ± 0.04% (w/w). Surfactants have multiple non-limiting roles in formulations, including, e.g., as an adjuvant, as an emulsifier and as a solubilizer. For example, in some embodiments, the surfactant acts as a solubilizer to prevent the drug substance, i.e., ClfA protein, from sticking to the walls of a container or syringe and hence not being recovered or delivered. Surfactants are also used to prevent possible aggregation from occurring such as , *e.g.,* when and if the ClfA protein comes in contact with a silicone lubrication from stoppers. Surfactants for use in immunogenic compositions and vaccines are described in Ascarateil and Dupuis, Vaccine, 24(2006): S83-S85. Briefly, non-limiting surfactants include lipopolysaccharides (LPS), saponins, dimethyl dioctadecyl ammonium bromide (DDAB), block polymers, or poloxamers, which are random polymers of ethylene oxide (EO) and propylene oxide (PO), sorbitan esters, which can be sorbitan mono or tri-oleate and which are made of sorbitol and an oleic acid linked by ester bonds and ethoxylated ones (i.e., polysorbates, such as, e.g., polysorbate 80), phospholipids such as lecithin, and mannide oleates, which are esters of oleic acid and mannitol.

In some embodiments, the surfactant is any one or more of a poloxamer, a polyoxyethylene alkyl ether including but not limited to Brij 58, Brij 35, as well as others such as Triton X-100; Triton X-114, NP40, Span 85 and the Pluronic series of non-ionic surfactants (e.g., Pluronic 121), and a polyoxyethylene sorbitan fatty acid ester, which includes the polysorbates. In some embodiments, the surfactant is Polysorbate-80 (Tween 80), Polysorbate-60 (Tween 60), Polysorbate-40 (Tween 40), or Polysorbate-20 (Tween 20). In some embodiments, the polysorbate 80 (Tween 80) is at 0.20% ± 0.042% (w/w).

In some embodiments, especially those in which the lyophilized composition does not contain an adjuvant, the aqueous diluent comprises an adjuvant. In some embodiments, the adjuvant is ISCOMATRIX™.

In some embodiments, especially those in which the lyophilized composition does not contain a surfactant, the diluent comprises a surfactant, such as, e.g., polysorbate 80.

In some embodiments, the liquid immunogenic composition comprises an intact rClfA polypeptide, as described herein, which is at a concentration of between 20µg/ml ± 2µg/ml and 800µg/ml ± 80µg/ml, including e.g. 20µg/ml ± 2µg/ml, 40µg/ml ± 4µg/ml, 200µg/ml ± 20µg/ml, 400µg/ml ± 40µg/ml, 600µg/ml ± 60µg/ml, and 800µg/ml ± 80µg/ml. In some embodiments, the liquid immunogenic composition comprises an intact ClfA polypeptide which is at a concentration of between 40µg/ml ± 4µg/ml and 800µg/ml ± 80µg/ml. In some embodiments, the liquid immunogenic composition comprises (a) an intact rClfA polypeptide, as described herein, which is at a concentration of between 20µg/ml ± 2µg/ml and 800µg/ml ± 80µg/ml, including *e.g.* 20µg/ml ± 2µg/ml, 40µg/ml ± 4µg/ml, 200µg/ml ± 20µg/ml, 400µg/ml ± 40µg/ml, 600µg/ml ± 60µg/ml, and 800µg/ml ± 80µg/ml, (b) a CP5-CRM₁₉₇ conjugate at between 10µg/ml ± 1µg/ml and 400µg/ml ± 40µg/ml, including, e.g., 10µg/ml ± 1µg/ml, 20µg/ml ± 2µg/ml, 100µg/ml ± 10µg/ml, 200µg/ml ± 20µg/ml, and 400µg/ml ± 40µg/ml (c) a CP8-CRM₁₉₇ conjugate at between 10µg/ml ± 1µg/ml and 400µg/ml ± 40µg/ml, including, *e.g.,* 10µg/ml ± 1µg/ml, 20µg/ml ± 2µg/ml, 100µg/ml ± 10µg/ml, 200µg/ml ± 20µg/ml, and 400µg/ml ± 40µg/ml, (d) histidine buffer at from about 5 mM to about 50 mM, from about 5 mM to about 25 mM, and from about 5 mM to about 15 mM, (e) polysorbate 80 at from about 0.05% to about 0.5%, from about 0.075% to about 0.25%, and from about 0.1% to about 0.2% weight to volume (w/v); and (f) sucrose at a concentration of from about 0% to about 25% sucrose, from about 3% to about 15%, and from about 5 to about 10%w/v. In other embodiments, the polysorbate 80 is at a concentration of 0.01% ± 0.005% weight to volume (w/v) and the sucrose is at a concentration of 4.5% ± 1.5% w/v. In some embodiments, the liquid immunogenic composition comprises (a) an intact rClfA polypeptide, as described herein, which is at a concentration of between 20µg/ml ± 2µg/ml and 800µg/ml ± 80µg/ml, including e.g. 20µg/ml ± 2µg/ml, 40µg/ml ± 4µg/ml, 200µg/ml ± 20µg/ml, 400µg/ml ± 40µg/ml, 600µg/ml ± 60µg/ml, and 800µg/ml ± 80µg/ml, (b) a CP5-CRM₁₉₇ conjugate at between 10µg/ml ± 1µg/ml and 400µg/ml ± 40µg/ml, including, *e.g.,* 10µg/ml ± 1µg/ml, 20µg/ml ± 2µg/ml, 100µg/ml ± 10µg/ml, 200µg/ml ± 20µg/ml, and 400µg/ml ± 40µg/ml, (c) a CP8-CRM₁₉₇ conjugate at between 10µg/ml ± 1µg/ml and 400µg/ml ± 40µg/ml, including, e.g., 10µg/ml ± 1µg/ml, 20µg/ml ± 2µg/ml, 100µg/ml ± 10µg/ml, 200µg/ml ± 20µg/ml, and 400µg/ml ± 40µg/ml, (d) a MntC polypeptide at between 20µg/ml ± 2µg/ml and 800µg/ml ± 80µg/ml, including *e.g.* 20µg/ml ± 2µg/ml, 40µg/ml ± 4µg/ml, 200µg/ml ± 20µg/ml, 400µg/ml ± 40µg/ml, 600µg/ml ± 60µg/ml, and 800µg/ml ± 80µg/ml, (e) histidine buffer at from about 5 mM to about 50 mM, from about 5 mM to about 25 mM, and from about 5 mM to about 15 mM, (f) polysorbate 80 at from about 0.05% to about 0.5%, from about 0.075% to about 0.25%, and from about 0.1% to about 0.2% weight to volume (w/v); and (g) sucrose at a concentration of from about 0% to about 25% sucrose, from about 3% to about 15%, and from about 5 to about 10%w/v. In another embodiment, the polysorbate 80 is at a concentration of 0.01% ± 0.005% weight to volume (w/v) and the sucrose is at a concentration of 4.5% ± 1.5% w/v.

In some embodiments, especially in cases in which neither the lyophilized composition nor the diluent contains an adjuvant, the liquid immunogenic is combined with an adjuvant.

In one embodiment, the disclosure provides a process of making an immunogenic composition comprising the steps of: (a) combining, in an aqueous medium, (i) a clumping factor A polypeptide ("rClfA") polypeptide, (ii) a buffer having a pKa of about 6.0 ± 0.6, and (iii) a bulking agent; and (b) lyophilizing the combination of step (a) to form a cake, which contains less than 3% water by weight. In one embodiment, the disclosure provides a process of making an immunogenic composition comprising the steps of: (a) combining, in an aqueous medium, (i) a clumping factor A polypeptide ("rClfA") polypeptide, (ii) a CP5-CRM₁₉₇ conjugate, (iii) a CP8-CRM₁₉₇ conjugate, (iv) a buffer having a pKa of about 6.0 ± 0.6, and (v) a bulking agent; and (b) lyophilizing the combination of step (a) to form a cake, which contains less than 3% water by weight. In one embodiment, the disclosure provides a process of making an immunogenic composition comprising the steps of: (a) combining an aqueous solution comprising (i) a clumping factor A polypeptide ("rClfA") polypeptide, (ii) a CP5-CRM₁₉₇ conjugate, (iii) a CP8-CRM₁₉₇ conjugate, (iv) an isolated MntC polypeptide, (v) a buffer having a pKa of about 6.0 ± 0.6, and (vi) a bulking agent; and (b) lyophilizing the combination of step (a) to form a cake, which contains less than 3% water by weight. Immunogenic compositions of the invention are useful in the prevention or treatment of a *Staphylococcus aureus* infection. In some embodiments, the cake is white in color and has a spongy texture.

By "lyophilization", what is meant is a process for freeze-drying (cryodessication) material. In general, the lyophilization process involves freezing a material, then reducing the surrounding pressure and providing heat sufficient to allow the frozen water in the material to sublime. In some cases, lyophilization includes an initial freezing step, a primary drying (sublimation) step, a secondary drying aimed at eliminating the final traces of water which remain. In some cases, an annealing step is included to improve cake characteristics and to improve sublimation. See Lyophilization of Biopharmaceuticals, Henry Costatino and Michael Pikal, eds., AAPS Press, Arlington VA, 2004 for a more detailed discussion of lyophilization.

By "cake", what is generally meant is the dried material remaining after the lyophilization process. Cake appearance depends upon the solid matrix structure formed by freezing and is influenced by various parameters during the lyophilization procedure. The addition of an annealing step to the lyophilization procedure can often result in a more uniform cake appearance.

In some embodiments, a surfactant, such as, e.g., polysorbate 80, is combined with the rClfA, buffer and bulking agent at step (a). In some embodiments, the surfactant has a concentration of about 0.1% ± 0.05% (w/v). In other embodiments, the surfactant has at a concentration of 0.01% ± 0.005% w/v.

In some embodiments, the aqueous combination that is formed at step (a) contains an intact rClfA polypeptide at a concentration of between 20µg/ml ± 2µg/ml and 800µg/ml ± 80µg/ml, including *e.g.* 20µg/ml ± 2µg/ml, 40µg/ml ± 4µg/ml, 200µg/ml ± 20µg/ml, 400µg/ml ± 40µg/ml, 600µg/ml ± 60µg/ml, and 800µg/ml ± 80µg/ml. In some embodiments, the aqueous combination that is formed at step (a) contains an intact rClfA polypeptide at a concentration of between 20µg/ml ± 2µg/ml and 800µg/ml ± 80µg/ml, including *e.g.* 20µg/ml ± 2µg/ml, 40µg/ml ± 4µg/ml, 200µg/ml ± 20µg/ml, 400µg/ml ± 40µg/ml, 600µg/ml ± 60µg/ml, and 800µg/ml ± 80µg/ml, a CP5-CRM₁₉₇ conjugate at between 10µg/ml ± 1µg/ml and 400µg/ml ± 40µg/ml, including, *e.g.,* 10µg/ml ± 1µg/ml, 20µg/ml ± 2µg/ml, 100µg/ml ± 10µg/ml, 200µg/ml ± 20µg/ml, and 400µg/ml ± 40µg/ml, a CP8-CRM₁₉₇ conjugate at between 10µg/ml ± 1µg/ml and 400µg/ml ± 40µg/ml, including, *e.g.,* 10µg/ml ± 1µg/ml, 20µg/ml ± 2µg/ml, 100µg/ml ± 10µg/ml, 200µg/ml ± 20µg/ml, and 400µg/ml ± 40µg/ml, histidine buffer at 10 mM ± 5 mM, polysorbate 80 at 0.1% ± 0.05% weight to volume (w/v), and sucrose at a concentration of 9% ± 4.5% w/v. In another embodiment, the polysorbate 80 is at a concentration of 0.01% ± 0.005% w/v and the sucrose is at a concentration of 4.5% ± 1.5% w/v.

In some embodiments, the aqueous combination that is formed at step (a) contains an intact rClfA polypeptide at a concentration of between 40µg/ml ± 4µg/ml and 800µg/ml ± 80µg/ml, including *e.g.* 40µg/ml ± 4µg/ml, 200µg/ml ± 20µg/ml, 600µg/ml ± 60µg/ml, and 800µg/ml ± 80µg/ml, a CP5-CRM₁₉₇ conjugate at between 10µg/ml ± 1µg/ml and 400µg/ml ± 40µg/ml, including, e.g., 10µg/ml ± 1µg/ml, 20µg/ml ± 2µg/ml, 100µg/ml ± 10µg/ml, 200µg/ml ± 20µg/ml, and 400µg/ml ± 40µg/ml, a CP8-CRM₁₉₇ conjugate at between 10µg/ml ± 1µg/ml and 400µg/ml ± 40µg/ml, including, *e.g.,* 10µg/ml ± 1µg/ml, 20µg/ml ± 2µg/ml, 100µg/ml ± 10µg/ml, 200µg/ml ± 20µg/ml, and 400µg/ml ± 40µg/ml, a MntC polypeptide at between 40µg/ml ± 4µg/ml and 800µg/ml ± 80µg/ml, including e.g. 40µg/ml ± 4µg/ml, 200µg/ml ± 20µg/ml, 600µg/ml ± 60µg/ml, and 800µg/ml ± 80µg/ml, histidine buffer at 10 mM ± 5 mM, polysorbate 80 at 0.1% ± 0.05% weight to volume (w/v), and sucrose at a concentration of 9% ± 4.5% w/v. In another embodiment, the polysorbate 80 is at a concentration of 0.01% ± 0.005% weight to volume (w/v) and the sucrose is at a concentration of 4.5% ± 1.5% w/v.

In some embodiments, the lyophilization step comprises the steps of (i) freezing the aqueous combination, (ii) annealing the aqueous combination, and (iii) drying the combination in two phases. In one embodiment, (i) freezing is performed by reducing the temperature of the aqueous combination in steps of 0.3°C ± 0.03°C per minute until a temperature of -50°C ± 5°C is reached at a pressure of 400 millibars ± 40 millibars, then holding for 60 minutes ± 6 minutes; (ii) annealing is performed by increasing the temperature to -10°C ± 5°C at a rate of 0.3°C ± 0.03°C per minute, followed by holding the temperature at -10°C ± 5°C for 120 minutes ± 12 minutes, followed by decreasing the temperature at a rate of 0.3°C ± 0.03°C per minute until reaching a temperature of -50°C ± 5°C, and then holding the temperature at -50°C ± 5°C for 180 minutes ± 18 minutes; (iii) the first phase of drying is performed by decreasing the pressure to 50 mTorrs (mTorr) and holding for 30 minutes ± 3 minutes, followed by increasing the temperature to - 30°C ± 5°C at a rate of 0.2°C ± 0.02°C per minute and then holding at that temperature for 1,920 minutes ± 192 minutes; and (iv) the second phase of drying is performed by increasing the temperature to 30°C ± 5°C at a rate of 0.2°C ± 0.02°C per minute, followed by increasing the pressure to 200 mTorr and holding the temperature at 30°C ± 5°C for 720 minutes ± 72 minutes. In certain embodiments, freezing is performed at about 1013 mbar (1 atm). In certain embodiments, freezing is performed at up to 1013 mbar (1 atm). In certain embodiments, the drying time can be about 2,600 minutes. In certain embodiments, the drying time can be up to 2,600 minutes. In certain embodiments, the drying temperature is 40°C ± 5°C, 50°C ± 5°C, or 60°C ± 5°C. After the final drying step, the temperature of lyophilized composition is decreased to 5°C ± 5°C at a rate of 0.5°C ± 0.05°C per minute. In certain embodiments, lyophilization takes place in a vial. In some embodiments, the vial is stoppered after lyophilization. In some embodiments, the vial is backfilled with nitrogen prior to stoppering, and is stoppered under a partial vacuum, such as 60% atmospheric pressure.

In one embodiment, the process also comprises the step of reconstituting the lyophilized composition in an aqueous diluent, wherein the osmolality of the reconstituted combination is 300 mOsm ± 30 mOsm. In some embodiments, the aqueous diluent is water. In some embodiments, the aqueous diluent is 60mM NaCl.

In one embodiment, the application provides an immunogenic composition manufactured according to the process described above.

In certain embodiments, a formulation of the invention comprises one or more additional stabilizing agents suitable for parenteral administration, e.g., a reducing agent comprising at least one thiol (-SH) group (e.g., cysteine, N-acetyl cysteine, reduced glutathione, sodium thioglycolate, thiosulfate, monothioglycerol, or mixtures thereof). Alternatively or optionally, preservative-containing immunogenic composition formulations of the invention may be further stabilized by removing oxygen from storage containers, protecting the formulation from light (e.g., by using amber glass containers).

Preservative-containing immunogenic composition formulations of the invention may comprise one or more pharmaceutically acceptable carriers or excipients, which includes any excipient that does not itself induce an immune response. Suitable excipients include but are not limited to macromolecules such as proteins, saccharides, polylactic acids, polyglycolic acids, polymeric amino acids, amino acid copolymers, sucrose (Paoletti et al, 2001, Vaccine, 19:2118), trehalose, lactose and lipid aggregates (such as oil droplets or liposomes). Such carriers are well known to the skilled artisan. Pharmaceutically acceptable excipients are discussed, e.g., in Gennaro, 2000, Remington: The Science and Practice of Pharmacy, 20th edition, ISBN:0683306472.

In one embodiment, the invention provides a liquid immunogenic composition manufactured by reconstituting a lyophilized immunogenic composition, as herein described, in an aqueous diluent. In some embodiments, the aqueous diluent is water. In other embodiments, the aqueous diluent is a low salt solution. What is meant by a low salt solution is an aqueous solution with a salt concentration between about 1 mM and about 200 mM, preferably between about 1 mM and about 100 mM. In some embodiments, the salt is sodium chloride. In some embodiments, the low salt solution comprises sodium chloride at 60 mM ± 6 mM. In some embodiments, the liquid immunogenic composition has a pH of 6.0 ± 0.6.

By use of the term "diluent", what is meant is a liquid capable of suspending, diluting or solubilizing any substance. In some embodiments, the substance is a lyophilized composition that contains a ClfA polypeptide. In some embodiments, the diluent is aqueous and solubilizes the lyophilized composition.

Direct delivery of reconstituted lyophilized immunogenic compositions of the present invention to a subject may be accomplished by parenteral administration (intramuscularly, intraperitoneally, intradermally, subcutaneously, intravenously, or to the interstitial space of a tissue); or by rectal, oral, vaginal, topical, transdermal, intranasal, ocular, aural, pulmonary or other mucosal administration. In a preferred embodiment, parenteral administration is by intramuscular injection, e.g., to the thigh or upper arm of the subject. Injection may be via a needle (e.g., a hypodermic needle), but needle free injection may alternatively be used. A typical intramuscular dose is 0.5mL. Compositions of the invention may be prepared in various forms, e.g., for injection either as liquid solutions or suspensions.

Optimal amounts of components for a particular immunogenic composition may be ascertained by standard studies involving observation of appropriate immune responses in subjects. Following an initial vaccination, subjects can receive one or several booster immunizations adequately spaced.

### Packaging and Dosage Forms

Immunogenic compositions of the disclosure may be packaged in unit dose or multi-dose form (e.g. 2 doses, 4 doses, or more). In certain embodiments, the dose is a 0.5 mL dose after reconstitution of a lyophilized immunogenic composition. See, e.g., International Patent Application WO2007/127668.

Compositions may be presented in vials or other suitable storage containers, or may be presented in pre-filled delivery devices, e.g., single or multiple component syringes, which may be supplied with or without needles. A syringe typically but need not necessarily contains a single dose of the preservative-containing immunogenic composition of the invention, although multi-dose, pre-filled syringes are also envisioned. Likewise, a vial may include a single dose but may alternatively include multiple doses.

Effective dosage volumes can be routinely established, but a typical dose of a reconstituted lyophilized composition for injection has a volume of 0.5 mL. In certain embodiments, the dose is formulated for administration to a human subject. In certain embodiments, the dose is formulated for administration to an adult, teen, adolescent, toddler or infant (i.e., no more than one year old) human subject and may in preferred embodiments be administered by injection.

Immunogenic compositions of the present disclosure may be lyophilized and reconstituted, e.g., using one of a multitude of methods for freeze drying well known in the art to form dry, regular shaped (e.g., spherical) particles, such as micropellets or microspheres, having particle characteristics such as mean diameter sizes that may be selected and controlled by varying the exact methods used to prepare them. The immunogenic compositions may further comprise an adjuvant which may optionally be prepared with or contained in separate dry, regular shaped (e.g., spherical) particles such as micropellets or microspheres. In one embodiment, the present disclosure further provides an immunogenic composition kit comprising a first component that includes a lyophilized immunogenic composition, optionally further comprising one or more preservatives of the invention, and a second component comprising a sterile, aqueous solution for reconstitution of the first component. In certain embodiments, the aqueous solution comprises one or more preservatives, and may optionally comprise at least one adjuvant (see, e.g., WO2009/109550).

In yet another embodiment, a container of the multi-dose format is selected from one or more of the group consisting of, but not limited to, general laboratory glassware, flasks, beakers, graduated cylinders, fermentors, bioreactors, tubings, pipes, bags, jars, vials, vial closures (e.g., a rubber stopper, a screw on cap), ampoules, syringes, dual or multi-chamber syringes, syringe stoppers, syringe plungers, rubber closures, plastic closures, glass closures, cartridges and disposable pens and the like. The container of the present invention is not limited by material of manufacture, and includes materials such as glass, metals (e.g., steel, stainless steel, aluminum, etc.) and polymers (e.g., thermoplastics, elastomers, thermoplastic-elastomers). In a particular embodiment, the container of the format is a 5 mL Schott Type 1 glass vial with a butyl stopper. The skilled artisan will appreciate that the format set forth above is by no means an exhaustive list, but merely serve as guidance to the artisan with respect to the variety of formats available for the present invention. Additional formats contemplated for use in the present invention may be found in published catalogues from laboratory equipment vendors and manufacturers such as United States Plastic Corp. (Lima, OH), VWR.

### Evaluation of Immunogenic Compositions

In one embodiment, the present disclosure provides immunogenic compositions comprising at least one antigen from a *S. aureus* organism. In one embodiment, the present disclosure provides immunogenic compositions comprising at least three antigens from a *S. aureus* organism. The present invention provides immunogenic compositions comprising at least four antigens from a *S. aureus* organism.

Various *in vitro* tests can be used to assess the immunogenicity of the immunogenic compositions of the invention. For example, an *in vitro* opsonic assay can be conducted by incubating together a mixture of staphylococcal cells, heat inactivated serum containing specific antibodies to the antigens in question, and an exogenous complement source. Opsonophagocytosis proceeds during incubation of freshly isolated polymorphonuclear cells (PMN's) or differentiated effector cells such as HL60s and the antibody/complement/staphylococcal cell mixture. Bacterial cells that are coated with antibody and complement are killed upon opsonophagocytosis. Colony forming units (cfu) of surviving bacteria that are recovered from opsonophagocytosis can be determined by plating the assay mixture. Titers are reported as the reciprocal of the highest dilution that gives 50% bacterial killing, as determined by comparison to assay controls.

A whole cell ELISA assay can also be used to assess *in vitro* immunogenicity and surface exposure of the antigen, wherein the bacterial strain of interest (*S. aureus*) is coated onto a plate, such as a 96 well plate, and test sera from an immunized animal is reacted with the bacterial cells. If any antibody, specific for the test antigen, is reactive with a surface exposed epitope of the antigen, it can be detected by standard methods known to one skilled in the art.

Any antigen demonstrating the desired *in vitro* activity can then be tested in an *in vivo* animal challenge model. In certain embodiments, immunogenic compositions can be used in the immunization of an animal (*e.g*., a mouse) by methods and routes of immunization known to those of skill in the art (*e.g.,* intranasal, parenteral, oral, rectal, vaginal, transdermal, intraperitoneal, intravenous, subcutaneous, etc.). Following immunization of the animal with a particular *Staphylococcus sp.* immunogenic composition, the animal is challenged with a *Staphylococcus sp.* and assayed for resistance to the staphylococcal infection.

In one embodiment, pathogen-free mice can be immunized and challenged with *S. aureus.* For example, mice are immunized with one or more doses of the desired antigen in an immunogenic composition. Subsequently, the mice are challenged with *S. aureus* and survival is monitored over time post challenge.

### Methods of Immunizing

Provided also are methods for immunizing a host to prevent staphylococcal infection. In a preferred embodiment, the host is human. Thus, a host or subject is administered an immunogenic amount of an immunogenic composition as described herein. An immunogenic amount of an immunogenic composition can be determined by doing a dose response study in which subjects are immunized with gradually increasing amounts of the immunogenic composition and the immune response analyzed to determine the optimal dosage. Starting points for the study can be inferred from immunization data in animal models. The dosage amount can vary depending upon specific conditions of the individual. The amount can be determined in routine trials by means known to those skilled in the art. In some embodiments, the method of immunizing a host to prevent staphylococcal infection, disease or condition comprises human, veterinary, animal, or agricultural treatment. Another embodiment provides a method of immunizing a host to prevent staphylococcal infection, disease or condition associated with a *Staphylococcus sp.* in a subject, the method comprising generating a polyclonal or monoclonal antibody preparation from the immunogenic composition described herein, and using said antibody preparation to confer passive immunity to the subject.

An immunologically effective amount of the immunogenic composition in an appropriate number of doses is administered to the subject to elicit an immune response. The treated individual should not exhibit the more serious clinical manifestations of the staphylococcal infection. The dosage amount can vary depending upon specific conditions of the individual, such as age and weight. This amount can be determined in routine trials by means known to those skilled in the art.

In one embodiment, patients being administered immunogenic compositions of the invention show a reduction in *S. aureus* carriage rates. Such reduction in carriage or a prolonged interval of time spent as a non-carrier following administration of an immunogenic composition is significant from a medical need perspective. For example, reduction in overall *S. aureus* carriage in carriers may be assessed following one dose of *S. aureus* multi-antigen vaccine. For example, 1 day prior to administration of an immunogenic composition, a group of adults aged 18-50 years may be screened for carriage by nasal and throat swabs followed by cultivation to determine their carriage state. Next, the group can be administered an immunogenic composition of the invention with a group receiving a control. Nasal and throat swabs performed weekly over a 12 week period, and monthly up to 6 months post administration of the immunogenic composition are performed and compared to placebo. One primary endpoint is to compare carriage rates in patients after administration of an immunogenic composition versus placebo at 3 month intervals post immunization.

### Animal Models of Staphylococcal Infection

Several animal models are described below for use in assessing the efficacy of any one of the immunogenic compositions described herein.

### Murine Sepsis Model (Passive or Active)

### Passive Immunization Model

Mice are passively immunized intraperitoneally (i.p.) with immune IgG or monoclonal antibody. The mice are subsequently challenged 24 hours later with a lethal dose of *S. aureus.* The bacterial challenge is administered intravenously (i.v.) or i.p. ensuring that any survival could be attributed to the specific *in vivo* interaction of the antibody with the bacteria. The bacterial challenge dose is determined to be the dose required to achieve lethal sepsis of approximately 20% of the unimmunized control mice. Statistical evaluation of survival studies can be carried out by Kaplan-Meier analysis.

### Active Immunization Model

In this model, mice (e.g. Swiss Webster mice) are actively immunized intraperitoneally (i.p.) or subcutaneously (s.c.) with a target antigen at 0, 3 and 6 weeks (or other similar appropriately spaced vaccination schedule) and subsequently challenged with *S. aureus* at week 8 by the intravenous route. The bacterial challenge dose is calibrated to achieve approximately 20% survival in the control group over a 10-14 day period. Statistical evaluation of survival studies can be carried out by Kaplan-Meier analysis.

### Infectious Endocarditis Model (Passive or Active)

A passive immunization model for infectious endocarditis (IE) caused by *S. aureus* has previously been used to show that ClfA can induce protective immunity. See Vernachio et al., Antmicro. Agents & Chemo. 50:511-518 (2006). In this model of IE, rabbits or rats are used to simulate clinical infections that include a central venous catheter, bacteremia, and hematogenous seeding to distal organs. Catheterized rabbits or rats with sterile aortic valve vegetations are administered a single or multiple intravenous injection of a monoclonal or polyclonal antibody specific for the target antigen. Subsequently, the animals are challenged i.v. with a *S. aureus* or *S. epidermidis* strain. Then after challenge, heart, cardiac vegetations, and additional tissues, including kidneys, and blood are harvested and cultured. The frequency of staphylococcal infection in cardiac tissue, kidneys, and blood is then measured. In one study, when animals were challenged with either MRSE ATCC 35984 or MRSA 67-0, significant reductions in infection rate were shown using either the polyclonal antibody preparation or the monoclonal antibody to ClfA. See Vernachio et al., Antmicro. Agents & Chemo. 50:511-518 (2006).

The infectious endocarditis model has also been adapted for active immunization studies in both rabbits and rats. Rabbits or rats are immunized intramuscularly or subcutaneously with target antigen and challenged with *S. aureus* two weeks later via the intravenous route.

### Pyelonephritis Model

In the pyelonephritis model, mice are immunized on wks 0, 3 and 6 (or other appropriately spaced immunization schedule) with the target antigens. Subsequently, the animals are challenged i.p. or i.v. with *S. aureus* PFESA0266. After 48 hrs, the kidneys are harvested and bacterial CFU are enumerated.

### Antibodies and Antibody Compositions

The disclosure further provides antibodies and antibody compositions which bind specifically and selectively to one or more antigens of an immunogenic composition of the present disclosure. In some embodiments, antibodies are generated upon administration to a subject of an immunogenic composition of the present disclosure. In some embodiments, the disclosure provides purified or isolated antibodies directed against one or more of the antigens of an immunogenic composition of the present disclosure. In some embodiments, the antibodies of the present disclosure are functional as measured by killing bacteria in either an animal efficacy model or via an opsonophagocytic killing assay. In some embodiments, the antibodies of the disclosure confer passive immunity to a subject. The present disclosure further provides polynucleotide molecules encoding an antibody or antibody fragment of the disclosure, and a cell or cell line (such as hybridoma cells or other engineered cell lines for recombinant production of antibodies) and a transgenic animal that produces an antibody or antibody composition of the disclosure, using techniques well-known to those of skill in the art.

Antibodies or antibody compositions of the disclosure may be used in a method of treating or preventing a Staphylococcal infection, disease or condition associated with a *Staphylococcus sp.* in a subject, the method comprising generating a polyclonal or monoclonal antibody preparation, and using said antibody or antibody composition to confer passive immunity to the subject. Antibodies of the invention may also be useful for diagnostic methods, e.g., detecting the presence of or quantifying the levels of one or more antigens of the immunogenic compositions of the present disclosure.

### Examples

The following examples demonstrate some embodiments of the present invention. It should be appreciated that when typical reaction conditions (*e.g*., temperature, reaction times, etc.) have been given, the conditions both above and below the specified ranges can also be used, though generally less conveniently. All parts and percents referred to herein are on a weight basis and all temperatures are expressed in degrees centigrade unless otherwise specified.

Furthermore, the following examples were carried out using standard techniques, which are well known and routine to those of skill in the art, except where otherwise described in detail.

Disclosed is a stable, lyophilized tri-antigen formulation comprising recombinant Clumping Factor A (rClfA), capsular polysaccharide Type-5 conjugate (CP5-CRM₁₉₇) and capsular polysaccharide Type-8 conjugate (CP8-CRM₁₉₇), which has been developed as a *Staphylococcus aureus* vaccine or immunogenic composition. In one particular embodiment, the rClfA is a mutated form of a surface-expressed virulence factor engineered to diminish its binding affinity to fibrinogen (Y388A) (rClfA*m*). CP5 and CP8 are common polysaccharides found in clinical isolates and, in this vaccine, are conjugated to a carrier protein CRM₁₉₇.

The lyophilized cakes that were made were visually suitable and the active components were stable upon reconstitution of the lyophilized cakes. Moreover, one-month storage of multiple lots (bracketing high and low doses) of the lyophilized formulation at real time (2-8°C), and accelerated (25°C and 37°C) temperatures showed that the lyophilized tri-antigen formulation remained stable. In addition, three freeze-thaw cycles and 24-hour stability test conducted at room temperature after reconstitution each showed stability of the active ingredients under those conditions.

Also disclosed is a stable, lyophilized tetra-antigen formulation comprising recombinant ClfA, CP5-CRM₁₉₇, CP8-CRM₁₉₇, and MntC, which has been developed as a *Staphylococcus aureus* vaccine or immunogenic composition. In one particular embodiment, ClfA is a mutated form of a surface-expressed virulence factor engineered to diminish its binding affinity to fibrinogen (Y388A). In one particular embodiment, MntC is not lipidated. In one particular embodiment, MntC is recombinant. As with the tri-antigen formulation, the lyophilized tetra-antigen formulation had greatly increased stability in comparison to liquid formulations comprising the same antigens.

### EXAMPLE 1: PREFORMULATION METHODOLOGY

A variety of biophysical tools (derivative UV absorption spectroscopy, fluorescence spectroscopy, circular dichroism (CD) and differential scanning calorimetry (DSC) were used to examine the intrinsic characteristics of *Staphylococcus aureus* clumping factor A (ClfA) protein in the context of structural stability. Temperature and pH were the variable parameters used to exert stress on the protein to help characterize the intrinsic structural behavior of ClfA protein. This data was then used to screen for excipients that may provide additional stability to the protein. Furthermore, binding studies were performed to assess the dose- and pH-dependent affinity of ClfA to AlPO₄ and Al(OH)₃. The data disclosed herein were used to arrive at a formulation for ClfA for use in biomedical applications, such as, e.g., as a vaccine or immunogenic composition.

While the invention is directed to any and all clumping factor A (ClfA) proteins and formulations containing ClfA, the following examples used a ClfA variant, which comprises the N1N2N3 domains of ClfA and has a Y338A substitution, which comprises an alanine substituted for tyrosine corresponding to position 338 of a full length ClfA and which lacks the ability to bind fibrinogen.

The samples prepared for the initial biophysical characterizations were composed of 10 mM buffer (acetate, succinate or phosphate) in 150 mM NaCl. Approximate target concentrations of the ClfA were 0.1 mg/mL for fluorescence, 0.2 mg/mL for circular dichroism (CD), and 0.5 mg/mL for UV absorption spectroscopy.

The protein concentrations were determined using a commercially available Modified Lowry protein assay (Pierce; Lowry et al. J. Biol. Chem.; 193:265-275 [1951]) or UV absorbance spectroscopy.

Circular Dichoism (CD) was performed using a Jasco J-810 spectropolarimeter to assess the secondary structure of the ClfA protein in the immunogenic composition or vaccine. The effect of pH on the structure of ClfA was examined by observing qualitative differences in the CD spectra when the protein was formulated at pH intervals from 5.0 to 8.0. Moreover, temperature perturbation experiments were performed from 10°C to 85°C at 218 nm to observe the conformational stability of the protein from a secondary structure perspective. The wavelength of 218 nm was chosen because the known N2N3 structure of ClfA comprises large amounts of β-sheet structure. Experimental parameters for CD scans included a wavelength range of 190 - 260 nm, scan rate of 20 nm/min, response time of two seconds, bandwidth of 1 nm, and accumulation of three (3). The temperature perturbation experiments were performed with the Variable Temperature mode in the Jasco software, using a temperature ramp rate of 15°C/hour, a response of one second, and a bandwidth of 1 nm.

Intrinsic tryptophan fluorescence emission spectroscopy was used to monitor changes in the tertiary structure of ClfA protein as a function of temperature. The method was also used to observe changes in protein structure in real-time and during accelerated studies. Fluorescence spectra were recorded on a PTI QM-1 (Brunswick, NJ) fluorometer with 295 nm excitation. The concentration of protein used was ~0.1 mg/mL. Emission spectra characteristic of tryptophan was monitored between 320 ~ 350 nm using a 1-cm pathlength quartz cuvette. Fluorescence data were acquired from 10°C - 85°C (2.5°C intervals) for a series of pH values between 4.0 and 8.0. Control experiments were performed using buffers as blanks for background correction. Instrumental settings included an excitation and emission bandwidth of 4 nm and 3 nm respectively, an integration time of one second and a data collection wavelength range of 290 nm - 400 nm. For formulations containing AlPO₄, a front-face (triangular) cuvette geometry was used to bypass the turbidity limitations of the sample. Analysis of the data was performed with Origin® 7.0 using a second-order polynomial 11-point Savitsky-Golay function for smoothing.

Temperature perturbation experiments with second-derivative UV absorption spectroscopy were used as another method for examining ClfA tertiary structure to assess the intrinsic protein stability. Multi-wavelength UV-visible absorption spectra were obtained from 200 nm-400 nm with an Agilent 8453 UV-visible diode array spectrometer equipped with a Peltier temperature controller. A 1-cm path length quartz cuvette with a 100 µL volume was used for all experiments. For the melting experiments, four minutes were allowed after each temperature change, which was deemed to be sufficient for equilibrium to be reached. Optical density data at 350 nm was also collected as a function of temperature to monitor potential aggregation of the ClfA protein.

Analyses of spectra were performed with CHEMSTATION™ software (Agilent). Second-derivative spectra were acquired using a nine-point data filter and fitted to a third-degree Savitzky-Golay polynomial. The derivative spectra were interpolated with 99 data points between each one-nanometer interval, providing an effective resolution of approximately 0.01 nm under non-aggregating conditions. MICROCAL ORIGIN™ 6.0 was used to select peak positions of the derivative spectra. Peak positions corresponding to the amino acid residues phenylalanine, tyrosine, and tryptophan were identified (based on the method of Kueltzo et al., J Pharm Sci. 2003; 92(9): 1805 - 1820), and plotted as a function of temperature.

Size Exclusion Chromatography was conducted using TOSOH TSK-GEL G3000SWxl Column, 7.8mm x 30cm, 5µm, Part number 08541, using the following conditions: Flow Rate, 0.5 mL/min; Max Pressure, 60 Bar; Run time, 30 min; Mobile Phase, Cellgro PBS, pH 7.4; Injection volume, 100 µL; Detector, UV 280, 260, 214 nm, 4 nm bandwidth for all; Reference, 340 nm, 16 nm bandwidth; Temperature, 25°C.

The melting temperature (Tₘ) of the ClfA protein was determined by differential scanning calorimetry (DSC). The DSC profile of the ClfA sample was measured against a matching buffer prepared at the same time as the ClfA sample, except the volume that would have been occupied by the drug substance (ClfA) was replaced with buffer. The instrument settings were as follows: Scan rate, 200°C / Hr; Scan range, 10-90°C; Filter periods, 8 second; Feedback mode/Gain, Medium. After each scan, the DSC cells and syringe were rinsed 3-5 times with water for injection (WFI). In the case of any rescan experiments, the cell cleaning cycle included the use of 10% Contrad (Decon Laboratories Ltd., East Sussex, UK) to ensure a clean cell for the next run.

Microcalorimetry capillary DSC was used as a high throughput platform to screen for excipients. In each excipient screening experiment, ClfA in 10 mM succinate buffer, 150 mM NaCl, pH 6.0 was used as the base for calculation of a change in Tₘ. The apparent Tₘ was acquired for duplicate DSC samples, and the average Tₘ was then compared to the average Tₘ of formulation without excipient in the same DSC run. The effect of each excipient/concentration was compared by the effect of change in Tₘ. A positive change in Tₘ in the presence of an excipient was considered a positive effect on thermal stability of ClfA. For ΔG calculation, ΔC_{P} was calculated from the pre- and post-transition baselines manually selected, ΔH and Tₘ was then calculated by integration of the area between the curve and the baseline. ΔG curve was calculated using Microcal DSC software.

### EXAMPLE 2: PREFORMULATION OF A CLFA DRUG PRODUCT: RESULTS

Pharmaceutically relevant stresses of temperature (10 - 85°C) and pH (4.0 - 8.0) were exerted on the drug product (liquid formulated ClfA) and drug substance (unformulated ClfA protein in liquid phase) to identify their susceptibilities.

Intrinsic tryptophan fluorescence thermal melt studies were conducted on ClfA samples at various pHs from pH 4.0 to pH 8.0 and fluorescence emission curves were generated. For these experiments, the ClfA was generally at a concentration of 0.1 mg/ml - 0.2 mg/ml. The inflection points of each curve were identified to be the melting temperature (Tₘ) and were tabulated in Table 4. At 10°C, the samples at higher pH values showed peak positions at higher wavelengths, suggesting that the tryptophan residues were more exposed to the solvent. Moreover, at higher pH values, ClfA exhibited lower Tₘ values indicating less stability compared to lower pH values such as pH 5.5 and 6.0. A pH panel at finer increments of 0.2 (Table 5) from pH 5.5 to 6.5 showed a gradual increase in Tₘ with a decrease in pH.

**TABLE 4**

| pH | BUFFER | Tₘ |
|---|---|---|
| 4 | acetate | 50.1 |
| 5 | acetate | 56.1 |
| 5.5 | succinate | 55.7 |
| 6 | succinate | 53.7 |
| 6.5 | succinate | 49.7 |
| 7 | phosphate | 48.2 |
| 8 | phosphate | 43.7 |

**TABLE 5**

| pH | Tₘ(°C) |
|---|---|
| 5.5 | 55.7 |
| 5.8 | 53.9 |
| 6.0 | 53.2 |
| 6.3 | 50.4 |
| 6.5 | 49.7 |

Circular dichroism (CD) spectroscopy was employed to examine the secondary structure of ClfA as a function of pH. The spectra obtained at pH 7.0 and pH 8.0, in particular, showed well-defined minima at ~200 nm and 220 nm. One likely interpretation of this observation is that the minimum at ~220 nm could be due the β-sheet component of the known N2N3 structure, and that the minimum at ∼200 nm is approaching the random coil minimum usually seen at around 195 nm. These observations support the fluorescence data that the ClfA protein is slightly more unfolded at pH 7.0 and pH 8.0.

CD melts of ClfA from pH 4.0 to 8.0 were performed from 10°C-85°C and curves were generated depicting the molar ellipticity as a function of absorbance for each pH point. The shape of the curves across the different pH values was observed. Data at pH 6.0 and higher all seemed to have similar transitions that can be visually estimated at around 55°C. The curves at pH 5.0 and 5.5 have higher transitions. Moreover, pH 5.0 exhibited the largest extent of change (in ellipticity values) suggesting the largest increase in β-sheet content. Because increased β-sheet is typical in aggregated samples, pH 5.0 maybe showing that ClfA protein is most prone to self-association at elevated temperatures.

Optical density at 350 nm (OD₃₅₀) was measured using the Agilent 8453 UV-visible spectrophotometer and right angle light scattering measured using the Photon Technology International spectrofluorometer as a function of temperature to observe increases in particle size which would correspond to increases in signal intensities and possibly correlate to aggregation of the ClfA protein. Notable transitions were observed in the temperature traces at pH 4.0, 5.0 and 5.5, with pH 4.0 having the largest extent of change (most aggregation). On the contrary, pH 6.0 and higher only exhibited small, gradual upward changes indicative of minor self-associating events.

Right angle light scattering is a technique that is more sensitive than OD₃₅₀ in detecting oligomerizations (aggregation). According to the data obtained from this technique, the earliest onsets of aggregation of ClfA were seen at pH 5.0 and 5.5 while the higher pH values showed as much as a 15°C delay in particle size increase.

The structural stability of ClfA was characterized by differential scanning calorimetry (DSC), which measures the change in enthalpy as measured in kcal/mole/°C. According to the DSC analysis, the unfolding of ClfA was observed to be reversible with a single transition noted at about 55.5°C. The enthalpy of this transition was calculated to be approximately 10 kCal/mol.

The Tₘ of ClfA was also measured as a function of concentration, which can be consistently determined from 0.1 mg/mL to 1.0 mg/mL. The Tₘ of ClfA was observed not to change with increasing protein concentration, which indicates that ClfA exists mostly as a monomer. This observation is consistent with the size exclusion chromatography data herein disclosed below.

The Tₘ of ClfA was also determined by capillary DSC as a function of pH (Table 6). In these experiments, the Tₘ of ClfA was observed to reach a plateau between pH 5.0 and 8.0 at about 55-56°C, although the Tₘ trends downward as the pH increases. The Tₘ of ClfA at pH 4.0 was only 50.8°C, indicating the structural instability of ClfA at this pH. For these DSC experiments, the concentration of ClfA was 1.0 mg/ml. The Tₘ of ClfA determined by DSC was also compared to the Tₘ determined by tryptophan (Trp) fluorescence peak shifting (*supra*). Tₘ determined by Trp fluorescence was consistent with the Tₘ determined by DSC between pH 4.0 and pH 5.5. However, the Tₘ determined by Trp fluorescence sharply decreased when pH increased from 6.0 to 8.0. A possible explanation is that the N3 domain unfolds as pH increases from 6.0 to 8.0. There are only two tryptophans in ClfA, both of which are located on the N3 domain. The sharp decrease in Tₘ by Trp fluorescence as pH increased from 6.0 to 8.0 indicated that these residues were exposed to a more polar environment, which is usually indicative of protein unfolding.

**TABLE 6**

| pH | Buffer | Average Tₘ |
|---|---|---|
| 4.0 | Acetate | 50.8 |
| 5.5 | Acetate | 56.2 |
| 5.5 | Succinate | 55.3 |
| 6.0 | Succinate | 55.7 |
| 6.5 | Succinate | 55.6 |
| 7.0 | Phosphate | 55.0 |
| 8.0 | Phosphate | 54.8 |

The ClfA drug product can be delivered in a pre-filled syringe that is pre-siliconized. To prevent protein aggregation caused by the silicone oil on the syringe barrel and plunger, as well as, *inter alia,* to prevent protein adsorption to a glass surface, polysorbate 80 (PS80) is often included in the final drug product formulation. Since PS80 is a detergent, which could potentially destabilize a protein, the Tₘ of ClfA was evaluated in the presence of PS80. Table 7 summarizes the results of capillary DSC experiments with 1.0 mg/ml ClfA in the presence or absence of polysorbate 80, which showed that the presence of PS80 decreased the Tₘ of ClfA by about 1°C, which is statistically significantly different from the Tₘ of ClfA formulation with no PS80.

**TABLE 7**

| pH | [PS80] | PS80:clfA Molar ratio | Average Tₘ | SD |
|---|---|---|---|---|
| 6.0 | 0 | 0.00 | 55.68 | 0.28 |
| 6.0 | 0.10% | 71.58 | 54.82 | 0.00 |
| 6.0 | 0.02% | 14.32 | 54.37 | 0.00 |
| 6.0 | 0.01% | 7.16 | 54.67 | 0.26 |

The effect of AlPO₄ on the Tₘ of ClfA was also evaluated. To measure Tₘ of ClfA bound to AlPO₄, 0.1 mg/mL ClfA was formulated with succinate-saline at pH 5.0, in which most of the ClfA was bound to aluminum. The Tₘ of bound ClfA was measured against the matching buffer containing the same concentration of aluminum phosphate. The result of that experiment showed that the Tₘ of ClfA did not notably change when bound to aluminum phosphate (i.e., 56.16°C ± 0.12°C) compared to unbound ClfA (i.e., 56.34°C ± 0.15°C).

To support the selection of buffer type, concentration and ionic strength of the drug product, the change in Tₘ was determined in high/low salt formulations and formulations of different concentrations of histidine and succinate buffer. Table 8 showed that the Tₘ of ClfA increased as NaCl concentration or succinate buffer concentration increased. However, the Tₘ of ClfA did not increase with any of the tested histidine-saline buffer formulations.

**TABLE 8**

| **Buffer type/strength** | **Change in Tₘ (°C)** |
|---|---|
| 0 mM NaCl, 10mM succinate, pH 6.0 | -0.94 |
| 300 mM NaCl, 10mM succinate, pH 6.0 | 1.38 |
| 600 mM NaCl, 10mM succinate, pH 6.0 | 3.43 |
| 5 mM succinate-saline, pH 6.0 | -0.16 |
| 50 mM succinate-saline, pH 6.0 | 0.70 |
| 5 mM histidine-saline, pH 6.0 | -0.41 |
| 10 mM histidine-saline, pH 6.0 | -0.20 |
| 50 mM histidine-saline, pH 6.0 | -0.25 |

Excipients of known pharmaceutical stabilizers, such as sugars, poly-ols, and amino acids, were screened for their impact on the Tₘ of ClfA compared to the base formulation (0.6 mg/mL ClfA in 10 mM succinate buffer, 150 mM NaCl, pH 6.0). Table 9 shows that increases in the Tₘ of ClfA were dependent on the concentration of CaCl₂, trehalose, sucrose, sorbitol, mannitol, and glutamic acid. Arginine however decreased the Tₘ of ClfA. Proline did not have a significant effect on Tₘ of ClfA. The effect of excipients on proteins is protein-dependent, thus one of ordinary skill in the art will not know what effect an excipient will have *a priori.* Sucrose, sorbitol, CaCl₂ and high concentration of NaCl not only increased Tₘ but also increased the ΔG at 4°C. Table 10 shows the additive effect of excipients in elevating ClfA Tₘ 2°C - 3°C, even in the presence of PS80.

**TABLE 9**

| **Excipient** | **Δ Tₘ (°C)** |
|---|---|
| 5 mM CaCl₂ | 0.22 |
| 20 mM CaCl₂ | 1.43 |
| 2 % Trehalose | 0.23 |
| 5 % Trehalose | 0.81 |
| 10 % Trehalose | 1.88 |
| 2 % Sucrose | 0.37 |
| 5 % Sucrose | 0.73 |
| 10 % Sucrose | 1.84 |
| 2 % Sorbitol | 0.57 |
| 5 % Sorbitol | 1.12 |
| 10 % Sorbitol | 2.36 |
| 2 % Mannitol | 0.66 |
| 5 % Mannitol | 1.11 |
| 10 % Mannitol | 1.33 |
| 10 % Glycerol | 0.89 |
| 10 mM Glutamic acid | 0.12 |
| 100 mM Glutamic acid | 1.39 |
| 10 mM Arginine | -0.02 |
| 100 mM Arginine | -0.85 |
| 100 mM Proline | 0.04 |

**TABLE 10**

| **Excipient** | **Δ Tₘ (°C)** |
|---|---|
| 5 % sucrose | 0.89 |
| 5 % mannitol | 1.19 |
| 10 mM CaCl₂ | 1.04 |
| 5 % sucrose + 5 % mannitol | 2.39 |
| 5 % mannitol + 10 mM CaCl₂ | 2.23 |
| 5 % sucrose+ 10 mM CaCl₂ | 2.08 |
| 5 % sucrose + 5 % mannitol + 10 mM CaCl₂ | 3.85 |
| 0.02 % NOF PS80 | -0.34 |
| 5 % sucrose + 5 % mannitol + 5 mM CaCl₂ + 0.02 % NOF PS80 | 2.09 |
| 300 mM NaCl + 5 % sucrose + 5 % mannitol + 5 mM CaCl₂ + 0.02 % NOF PS80 | 2.59 |
| 500 mM NaCl + 5 % sucrose + 5 % mannitol + 5 mM CaCl₂ + 0.02 % NOF PS80 | 3.74 |
| 150 mM NaCl + 5 % sucrose + 5 % mannitol + 5 mM CaCl₂ | 1.96 |
| 5 % sucrose + 5 % mannitol + 0.02 % NOF PS80 | 1.42 |
| 300 mM NaCl + 5 % sucrose + 5 % mannitol + 0.02 % NOF PS80 | 2.38 |
| 500 mM NaCl + 5 % sucrose + 5 % mannitol + 0.02 % NOF PS80 | 3.64 |
| 150 mM NaCl + 5% sucrose + 5% mannitol | 1.70 |
| 150 mM NaCl + 3 %sucrose+ 10m M CaCl₂ + 0.02 % NOF 0.02 % NOF PS80 | 1.55 |
| 300 mM NaCl + 3 %sucrose +10 mM CaCl₂ + 0.02 % NOF PS80 | 2.28 |
| 300 mM NaCl + 3 %sucrose + 10 mM CaCl₂ | 2.39 |
| 150 mM NaCl + 3 %Mannitol + 10 mM CaCl₂ + 0.02 % NOF PS80 | 1.69 |
| 300 mM NaCl + 3 %Mannitol + 10 mM CaCl₂ + 0.02 % NOF PS80 | 2.47 |

The net surface charges of ClfA and AlPO₄ were obtained as a function of pH between 4.0 and 8.0 to be used as predictors of binding. Buffer systems used for the study were acetate (for pH 4.0 - 5.0), succinate (for pH 5.5 - 6.5) and phosphate (for pH 7.0 - 8.0). The zeta potential profile of ClfA crosses the point of zero charge somewhere between pH 4.0 and 5.0. (Zeta potential is the measure of the electrokinetic potential of agents in a colloidal system. See Lyklema, J. "Fundamentals of Interface and Colloid Science", vol.2, page.3-208 1995.) This is consistent with the fact that ClfA possesses a pI value in the low 4s. Furthermore, the zeta potential profile of AlPO₄ was also found to be consistent with its pI of ~5.5 - 5.8. Thus, the best binding would occur where the two entities have opposite net charges, roughly between pH 4.5 - 5.5. Lowry assays indicated that the best binding occurred at pH 5.0 at both 0.020 and 0.100 mg/mL ClfA concentrations. The opposite trend was true for the Al(OH)₃ curve where pH 5.0 exhibited the lowest binding. Al(OH)₃ never achieved 100% binding at any pH value tested.

AlPO₄ concentration titration (0.25, 0.50, 0.75 mg/mL) binding experiments were conducted with ClfA at pH 5.0, 5.5, and 6.0. According to those experiments, ClfA at pH 6.0 seemed insensitive to protein concentration consistently achieving ∼50% binding, whereas the protein at pH 5.0 showed an increase in binding concomitant to an increase in AlPO₄. ClfA was observed to achieve the best binding at pH 5.0 and at lower protein concentrations, with binding decreasing as concentrations were increased. Furthermore, pH 5.5 and 6.0 both showed the same trend, but binding was not adequate for either of these pH values, even at the lowest dose.

In an attempt to improve binding at pH 6.0, a few excipients, which included 150 mM NaCl, 500 mM NaCl, 300 mM glycine, 300 mM lysine, 20 mM MgCl₂ and 1 mM EDTA, were selected to test along with varying NaCl concentrations. Many of the excipients were chosen due to their cationic characteristics, since at pH 6.0, both ClfA and AlPO₄ are both negatively charged. A modified Lowry assay was used to test for percent bound protein. According to the assay, none of the excipients were observed to show a marked improvement compared to the 150 mM NaCl control.

### EXAMPLE 3: FORMULATION OF A CLFA LYOPHILIZED DRUG PRODUCT: SUMMARY

ClfA is generally considered to be an unstable protein, which means that it readily undergoes hydrolysis or "clipping" between the N1 domain and the N2 domain to generate at least two fragments, one of which contains the N1 domain and another which contains the N2N3 domains. By stability, what is meant is the relative amount of unhydrolyzed ClfA that contains substantially undegraded ClfA compared to degradation products, which include, for example, N1 and N2N3 peptide fragments. The greater the relative amount of substantially undegraded ClfA, the more stable the protein.

It was observed that the ClfA protein in a liquid formulation (*supra*) suffered from clipping between the N1 and N2N3 domains, as seen with size exclusion HPLC. After screening with carefully chosen excipients, lyophilization was investigated as an alternative to the liquid formulation based on liquid instability. Four lots of ClfA drug substance were examined using a modified lyophilization formulation of 10 mM succinate, pH 6.0, 0.01% polysorbate 80, and 4.5% sucrose (bulking agent) in water for injection (WFI) (little or no NaCl). The results for all four lots indicated that a freeze-dried formulation of ClfA successfully stabilized the protein from clipping for up to three months at real-time and accelerated (37°C) temperatures. Analysis of the formulation using reversed phase HPLC demonstrated that lyophilization prevented other modifications such as deamidation and oxidation throughout the observed time points. Also, *in vitro* potency tests (*i.e.,* BIOVERIS tests) revealed that ClfA maintained its potency over three months.

The lyophilized drug products were also characterized using biophysical (pH, moisture by Karl Fisher coulometry [see Scholz, Eugen, Fresenius' J. of Anal. Chem., 348 (4): 269-271, Apr. 1994], OD₃₅₀, osmolality, circular dichroism, UV absorbance spectroscopy, differential scanning calorimetry) methods, separation methods (HPLC), and potency (BIOVERIS) methods to monitor the effects of freeze-drying on the formulation (drug product) and on the protein itself (drug substance). Analyses using multiple methods suggested that the protein characteristics were similar pre- and post-lyophilization. Additionally, agitation, freeze-thaw, and excipient screening experiments were performed as preliminary optimization for the lyophilization formulation.

### EXAMPLE 4: FORMULATION OF A CLFA LYOPHILIZED DRUG PRODUCT: LYOPHILIZATION

Prior to lyophilization, vials were filled with 650 µl of liquid formulations. Post-lyophilization, it was necessary to determine a reconstitution volume with a syringe such that the dispensing volume would take into account the dead space within the syringe. In this experiment, syringes were filled with 0.55, 0.60, 0.65, and 0.70 mL of diluent. The diluent was extruded into the lyophilized vials and the reconstituted liquid was drawn back in to the syringe. The liquid was then dispensed and weighed on a scale. Assuming the density of the liquid to be approximately 1 g/mL, it was determined that the vial must contain approximately 0.65 mL of vaccine in order to deliver approximately 0.5 mL of the vaccine to the patient. Volumes in excess of 0.5 mL are acceptable, but volumes of less than approximately 0.5 mL are not preferred.

Filtration recovery of pre-lyophilized liquid formulations of two lots L36051-81-1 and L36051-81-2 were monitored by SE-HPLC. The filter used was a Millipore 0.22 µm PVDF membrane. The recovery of ClfA for both lots was >99%.

A Virtis Genesis EL35 Lyophilizer was used for all formulations reported herein. The parameters of an exemplary and non-limiting lyophilization run cycle are shown in Table 11. Lot L36051-44 (DS L35812-59) did not include an annealing step whereas the remaining three lots were prepared with an annealing step.

**TABLE 11**

| | Shelf Temp. (°C) | Rate / Hold | Time (min) | Pressure |
|---|---|---|---|---|
| Freezing | -50 | 0.3 C/min | 183.3 | 400 mbar |
| | -50 | Hold | 60 | 400 mbar |
| | -10 | 0.3 C/min | 133.3 | 400 mbar |
| Annealing | -10 | Hold | 120 | 400 mbar |
| | -50 | 0.3 C/min | 133.3 | 400 mbar |
| | -50 | Hold | 180 | 400 mbar |
| Primary drying | -25 | 0.2 C/min | 125 | 50 mTorr |
| | -25 | Hold | 1320 | 50 mTorr |
| Secondary drying | 25 | 0.2 C/min | 275 | 50 mTorr |
| | 30 | Hold | 720 | 200 mTorr |
| | 5 | 0.5 C/min | 50 | 200 mTorr |
| | 5 | Hold | Until Stoppered | 200 mTorr |

| | | | | |
|---|---|---|---|---|
| mTorr = milliTorrs | | | | |

Moisture was measured by Karl Fisher chemistry using a Brinkmann coulometer connected to a sample oven. Dry nitrogen gas was used to carry moisture from the sample into the Karl Fisher cell. A 5.5% dry moisture standard is used to monitor system performance. Using the oven, the water standard was measured at 230°C and freeze-dried samples are measured at 115°C. In general, a successful freeze-drying cycle contains the least amount of water while retaining all other desirable product characteristics.

Ten random vials of post-lyophilized formulations were inspected from each lot and evaluated for visual characteristics. The cake quality from lot to lot were comparable, however, lot L36051-44, which was prepared with no annealing step, showed greater shrinkage than the other lots that included an annealing step. Overall, the cakes were white and spongy, exhibiting good structural integrity. Visual characteristics of 10 individual lyophilization products of one particular formulation lot (with annealing) are depicted in Table 12.

**TABLE 12**

| **Vial #** | **1** | **2** | **3** | **4** | **5** | **6** | **7** | **8** | **9** | **10** |
|---|---|---|---|---|---|---|---|---|---|---|
| **Vial Integrity** | | | | | | | | | | |
| Intact | yes | yes | yes | yes | yes | yes | yes | yes | yes | yes |
| Chipped | no | no | no | no | no | no | no | no | no | no |
| Fractured, hairline | no | no | no | no | no | no | no | no | no | no |
| Fractured, open | no | no | no | no | no | no | no | no | no | no |

| **Cake Integrity** | | | | | | | | | | |
|---|---|---|---|---|---|---|---|---|---|---|
| Intact | yes | yes | yes | yes | yes | yes | yes | yes | yes | yes |
| Adhesion to glass | no | no | no | no | no | no | no | no | no | no |
| Shrinkage | yes | yes | yes | yes | yes | yes | yes | yes | yes | yes |
| Collapse | no | no | no | no | no | no | no | no | no | no |
| Meltback | no | no | no | no | no | no | no | no | no | no |
| Chipped | no | no | no | no | yes | yes | yes | no | yes | no |
| Cracked, hairline | no | no | no | no | yes | no | no | no | no | no |
| Cracked, open | no | no | no | no | no | no | no | no | no | no |

| **Cake Cosmetics** | | | | | | | | | | |
|---|---|---|---|---|---|---|---|---|---|---|
| Color | white | white | white | white | white | white | white | white | white | white |
| Texture | Thick sponge | Thick sponge | Thick sponge | Thick sponge | Thick sponge | Thick sponge | Thick sponge | Thick sponge | Thick sponge | Thick sponge |
| Crust | small | small | small | small | small | small | Small | small | small | Small |
| Glaze | yes | yes | yes | yes | yes | yes | yes | yes | yes | yes |
| Pores | no | no | no | no | no | no | no | no | no | no |
| Bubbling | no | no | no | no | no | no | no | no | no | no |
| Puffing | no | no | no | no | no | no | no | no | no | no |
| Froth or Foaming | no | no | no | no | no | no | no | no | no | no |

Physical attributes of the formulations were characterized before and after lyophilization. Samples were reconstituted after lyophilization with approximately 630 µl saline (Lot 44 was reconstituted with 150 mM NaCl, whereas the other lots were reconstituted with 50 mM NaCl). The osmolality after reconstitution was close to a physiological target of ∼290 mOsm when using 50 mM NaCl. It was observed that pH values remained 6.0 ± 0.3 when lyophilized cakes were reconstituted with diluent. Furthermore, the reconstitution times of all lots were less than 30 seconds. Transient micro-bubbles were generated upon addition of diluent to the cakes, as a result of nitrogen back-filling during stoppering.

A TA Instruments Modulated DSC instrument was used to characterize the glass transition temperature (T_{g}') of the pre-lyophilized liquid. T_{g}' is defined as the temperature at which an amorphous solid achieves a brittle, glassy state with a high level of hydrogen-bonding upon cooling. Above the T_{g}', more molecular fluidity is enabled. Experimental acquisition of T_{g}' is important for the optimization of the lyophilization cycle in order to achieve a high quality cake and a stable product. A modulated DSC instrument was used for T_{g}' measurements because of its ability to scan at sub-zero temperatures.

The glass transition (T_{g}') and moisture content of the cakes were considered during the optimization of the lyophilization procedure. It is important that the sample be above its glass transition during the annealing phase of lyophilization. The annealing step for lyophilization was at -10°C, which is above the T_{g}' of -34.6°C ± 0.8°C for all formulations. It is also important that the lyophilization procedure adequately dries the cake to prevent reactions between the proteins and water, thereby reducing the product's stability and shelf life. The lyophilization procedure resulted in less than 1% (*i.e.,* 0.42% ± 0.19%) water for all formulations.

### EXAMPLE 5: FORMULATION OF A CLFA LYOPHILIZED DRUG PRODUCT: THERMOSTABILITY

A MICROCAL VP-Differential Scanning Calorimeter (DSC) was used to characterize the intrinsic thermostability of ClfA. A thermal ramp from 10°C to 100°C revealed the temperature at which the protein melts (Tₘ) and the change in enthalpy (ΔH) required for the unfolding (melting) event. This provides information about the folding characteristics of the protein (i.e. secondary and tertiary structures) and whether or not the conformation had changed post-lyophilization.

Differential Scanning Calorimetry (DSC) using the MicroCal Capillary DSC was performed on pre- and post-lyophilized lots of ClfA to compare the melting temperatures (Tₘ) of the formulations. All tested lots revealed similar Tₘ values and unfolding enthalpies (ΔH) pre- and post-lyophilization, suggesting that the overall folding of the protein does not change from pre- to post-lyophilization. The average Tₘ for pre-lyophilization was 55.2°C ± 0.14°C compared to 55.2°C ± 0.16°C post-lyophilization. The average ΔH for pre-lyophilization was 1.90x10⁶ ± 0.12x10⁶ cal/mole/°C compared to 1.90x10⁶ ± 0.11x10⁶ cal/mole/°C post-lyophilization.

### EXAMPLE 6: FORMULATION OF A CLFA LYOPHILIZED DRUG PRODUCT: ANTIGEN SECONDARY STRUCTURE

Circular Dichroism (CD) was used to assess the secondary structure of the protein in each formulation pre- and post-lyophilization. All experiments were performed on a Jasco J-810 spectropolarimeter. Experimental parameters for CD scans included a wavelength range of 190 nm - 260 nm, scan rate of 20 nm/min, response time of 2 seconds, bandwidth of 1 nm, and accumulation of 3. An identical buffer matrix containing succinate, sucrose and NaCl was used for background subtraction.

Circular Dichroism (CD) provides spectral fingerprints of protein secondary structures such as α-helix and β-sheet. A comparison of the pre- and post-lyophilized lots of ClfA showed that the overall shapes of the spectra before and after lyophilization did not change, suggesting that all lots maintained their secondary structures throughout the lyophilization and reconstitution procedure.

### EXAMPLE 7: FORMULATION OF A CLFA LYOPHILIZED DRUG PRODUCT: ANTIGEN TERTIARY STRUCTURE

Second-derivative UV absorption spectroscopy were used as another method for examining antigen tertiary structure to assess the intrinsic protein stability before and after lyophilization. Multi-wavelength UV-visible absorption spectra were obtained from 200 nm - 400 nm with an Agilent 8453 UV-visible diode array spectrometer. A 1-cm path length quartz cuvette with a 100 µL volume was used for all experiments.

Analysis of spectra was performed with CHEMSTATION™ software (Agilent). Second-derivative spectra were acquired using a nine-point data filter and fitted to a third-degree Savitzky-Golay polynomial. The derivative spectra were interpolated with 99 data points between each one-nanometer interval, providing an effective resolution of approximately 0.01 nm under non-aggregating conditions. Microcal Origin™ 6.0 was used to select peak positions of the derivative spectra. Peak positions corresponding to the amino acid residues phenylalanine, tyrosine, and tryptophan were identified, and plotted as a function of temperature.

In three of the four lots (Lots -44, -81-1 and -81-2), some differences in the phenylalanine/tyrosine regions (260 nm ∼ 270 nm) were seen after lyophilization. However, lot -72 showed good overlay. This lot contained the highest residual NaCl (carried over from the bulk matrix).

### EXAMPLE 8: FORMULATION OF A CLFA LYOPHILIZED DRUG PRODUCT: STRUCTURAL STABILITY: N1 CLIPPING

Size exclusion HPLC was used to assess the initial quality of the pre-lyophilized drug product, reconstituted post-lyophilized drug product, and reconstituted drug product held at different temperatures over time for stability studies. This method is capable of detecting ClfA species of different sizes including monomer, dimer, oligomers (larger than dimer) and degradants (due to cleavage between the N1 and N2N3 domains) hence it was used as one of the primary techniques to compare the stabilities of pre-lyophilized liquid formulations to their lyophilized counterparts.

Stability of the liquid and lyophilized formulations of ClfA at 2-8°C, 25°C, and 37°C were monitored by two HPLC methods. Size exclusion HPLC was used to detect cleavage of the N1 and N2N3 domains whereas reverse phase-HPLC was used to monitor any chemical degradation (i.e. deamidation, oxidation) of the protein over time.

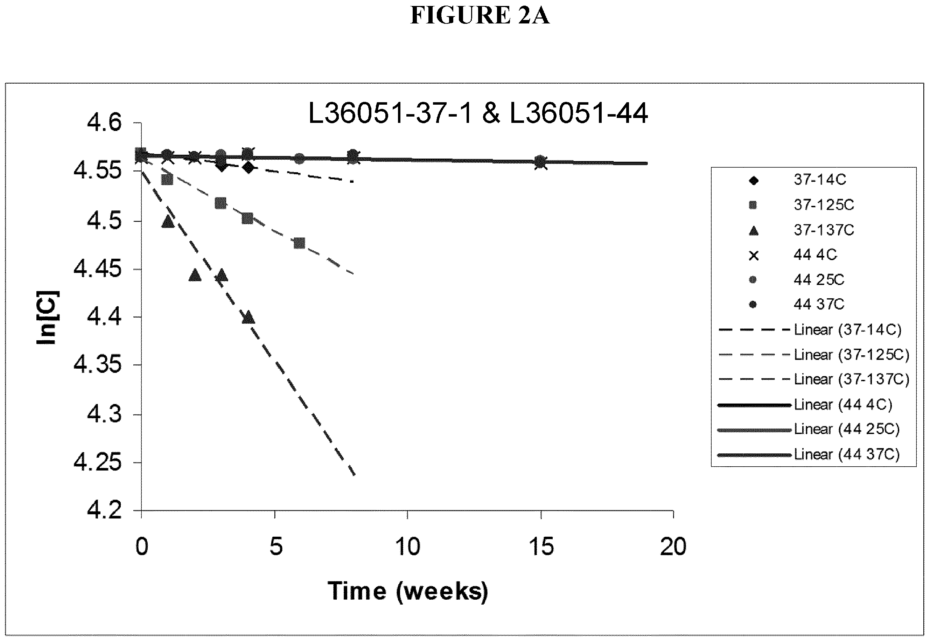

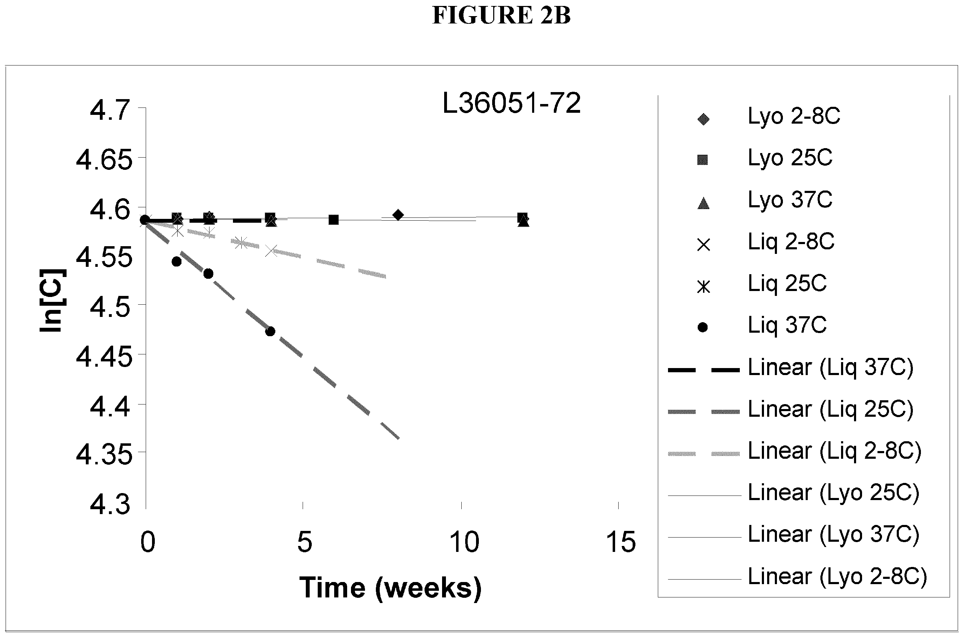

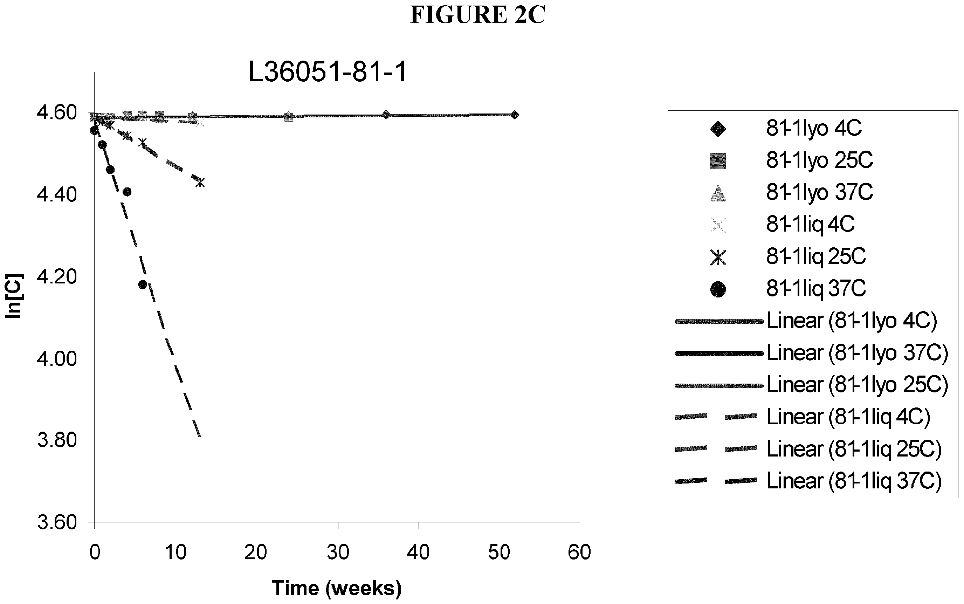

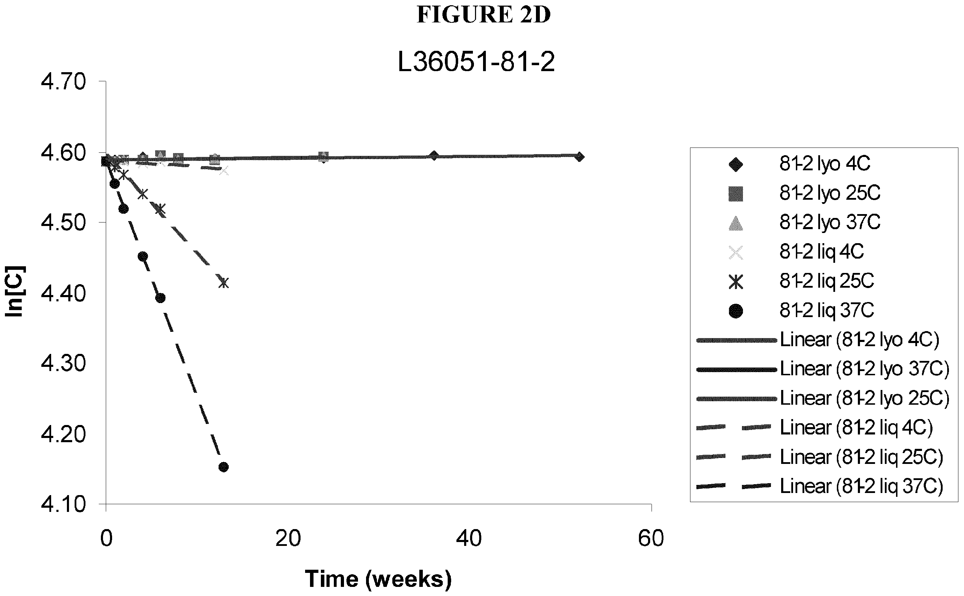

Figure 2, panels A, B, C and D illustrate the first-order kinetic plots of four lyophilized lots of ClfA compared to their liquid counterparts, all incubated at 2-8°C, 25°C and 37°C and monitored over time by SE-HPLC. The results were plotted as ln[C] vs. time where C is the concentration of the intact antigen (dimer + monomer) while excluding the cleaved degradants. All four lots show consistent stability of the lyophilized product at all three incubation temperatures as indicated by the minimal change in C for up to three months. In contrast, the pre-lyophilized liquid samples show a proportionate rate increase of degradation with increasing temperatures. It should be noted that Lot L36051-44 liquid counterpart (L36051-37-1) is the liquid formulation (as opposed to a pre-lyo formulation) containing 10 mM succinate, pH 6.0, 0.01% Polysorbate 80 and 150 mM NaCl (Figure 2A). However, the overall trends of Lot -37-1 as a function of temperature are similar to those of the pre-lyophilized liquids.

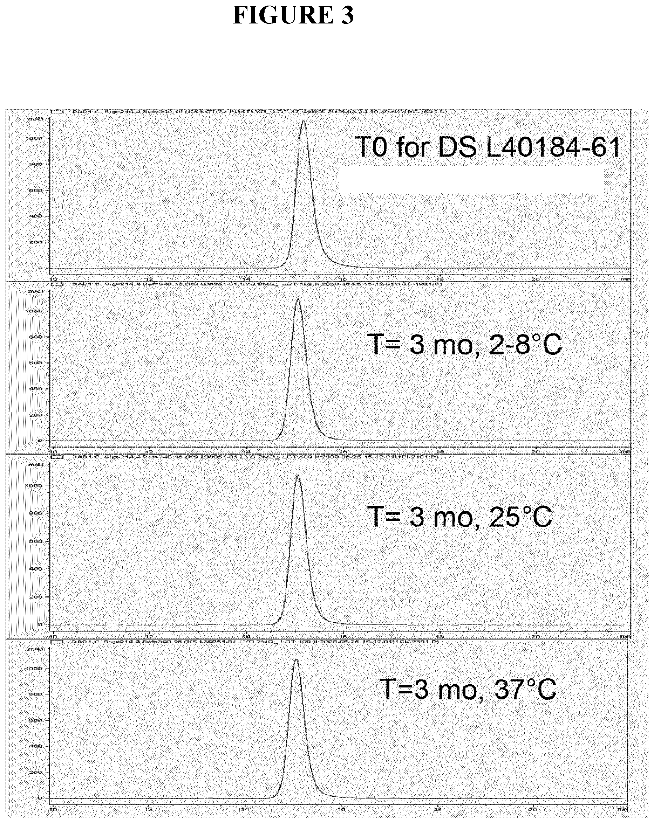

Figure 3 illustrates representative SE-HPLC chromatograms of lyophilized DS L40184-61 at t = 0 compared to cakes stored at 2-8°C, 25°C, and 37°C for three months. The profiles of the stacked chromatograms look identical, with no apparent increase in dimer or degradant peaks. The other three lyophilized lots showed similar results.

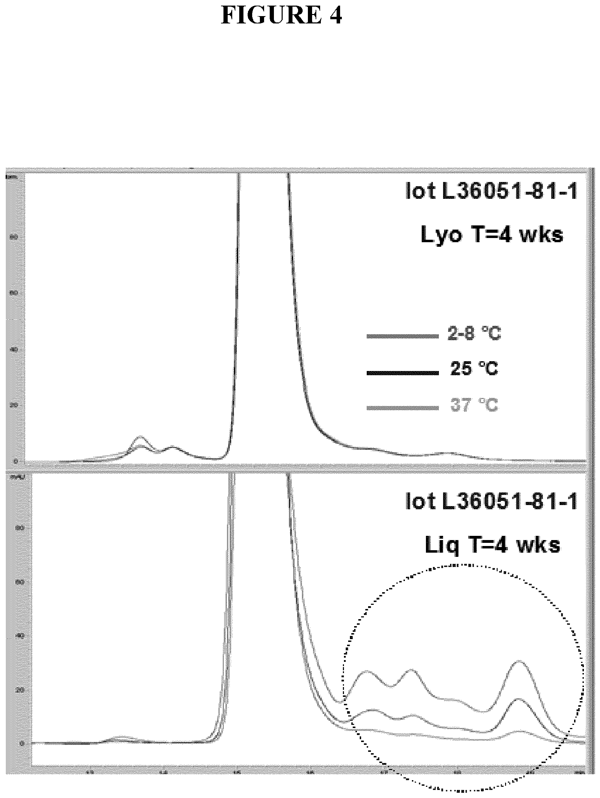

A comparison of a liquid (pre-lyophilized) and a lyophilized ClfA formulation lot was conducted. Representative SE-HPLC chromatograms are shown in Figure 4 comparing the lyophilized formulation to the pre-lyophilized liquids after being stored at 2-8°C, 25°C, and 37°C for four weeks. The lyophilized samples exhibited good overlay across all three storage temperatures, in particular around the degradant region to immediate right of the major monomer peak. In contrast, the liquid formulations showed marked increase in the degradant peaks (Fig. 4, circled). Additionally, the lyophilized peaks showed a minor presence of oligomer peaks (to the left of the major monomer peak, Fig. 4), an attribute that was common to all of the lyophilized lots.

### EXAMPLE 9: FORMULATION OF A CLFA LYOPHILIZED DRUG PRODUCT: STRUCTURAL STABILITY: PROTEIN MODIFICATIONS

Reversed phase HPLC is an analytical method often used to provide characterization of protein quality and integrity. Separation of analytes is based on the difference in hydrophobicity of different species, which may vary significantly from protein to protein. As a result, a reversed phase HPLC assay can typically provide a good separation between the protein of interest and, for example, protein degradants, the protein with sequence modifications (such as deamidation and oxidation), and residual host cell protein impurities, in addition to cleavage. This assay was used to provide data about protein quality over time in the stability studies.

As with the SE-HPLC data, the reversed phase chromatograms show similar profiles for the lyophilized product after three months at 37°C, 25°C and 2-8°C, compared to freshly made formula.

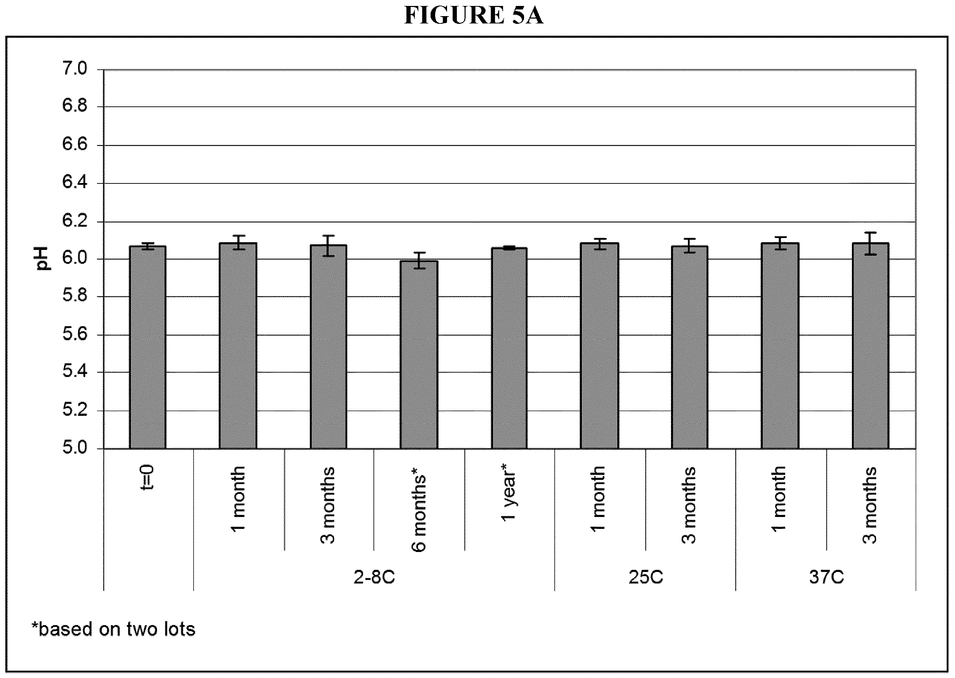

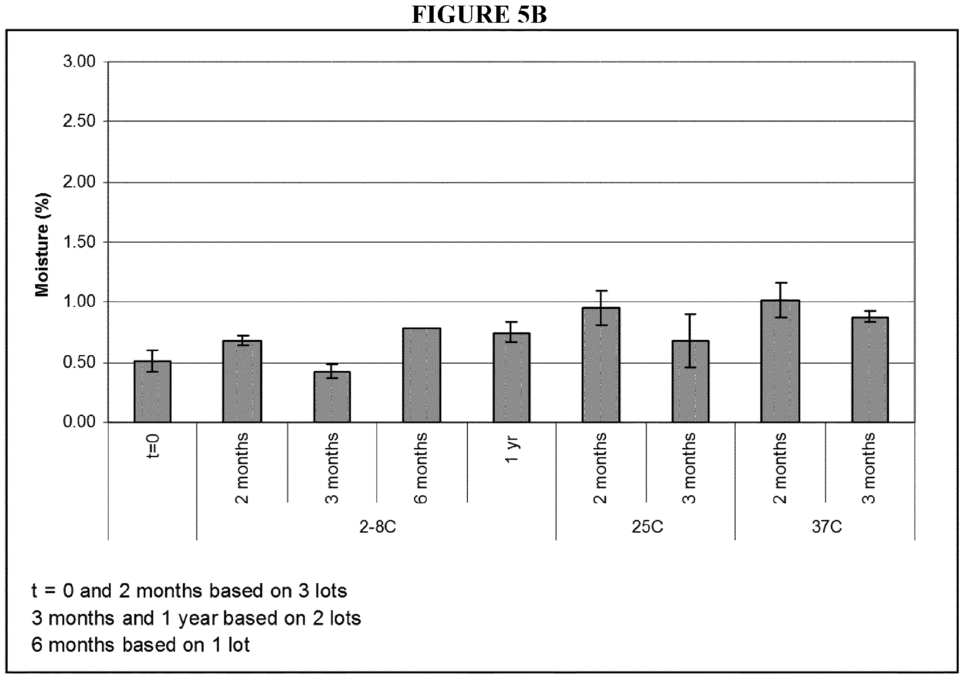

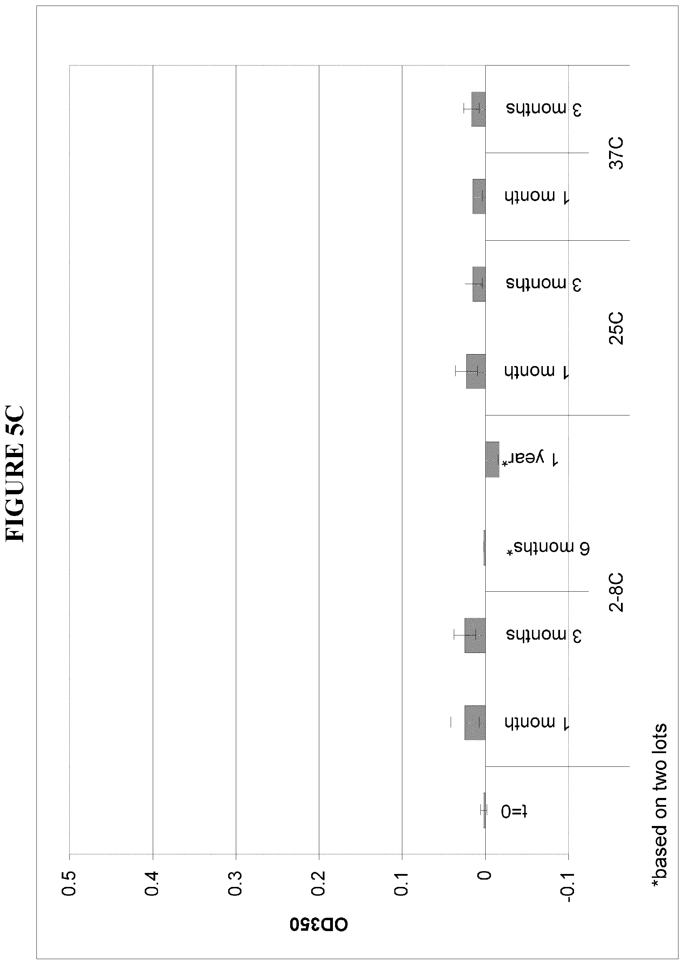

During the course of the lyophilized stability study, pH, moisture, and OD₃₅₀ were monitored. pH values should remain within ± 0.03 units of the target pH, and moisture should be < 3%. OD₃₅₀ is a measure of scattered light by aggregated particles. Typically, the absence of detectable aggregates will return OD₃₅₀ numbers of <0.03. Figure 5, panels A, B and C are data combined from multiple lyophilized lots, which demonstrate that all three parameters show good stability over three months.

### EXAMPLE 10: FORMULATION OF A CLFA LYOPHILIZED DRUG PRODUCT: POTENCY AS DETERMINED BY BIOVERIS

An *in vitro* potency assay was used to assess the persistence of a functional epitope of the ClfA protein at selected time points in the stability study. The BIOVERIS platform was used for this purpose. In this assay, a sandwich format was employed in which one monoclonal antibody (mAb 12-9) was used to capture the antigen and another monoclonal antibody (mAb 15EC6) raised against a different epitope was used to detect the presence of antigen with ECL detection. The monoclonal antibody 12-9 is an antibody that recognizes and binds the N3 domain of ClfA, while mAb 15EC6, recognizes and binds the N2 domain of ClfA.

The BIOVERIS antibody competition assay system was employed to monitor the potency of the ClfA in Lots L36051-81-1 (DS L40184-61) and L36051-81-2 (DS L40184-62) for up to three months. Both the lyophilized and pre-lyophilized liquid formulations showed no decrease in potency over the 3-month period at 2-8°C, 25°C, and 37°C.

### EXAMPLE 11: FORMULATION OF A CLFA LYOPHILIZED DRUG PRODUCT: THE EFFECT OF EXCIPIENTS

After the stability of ClfA had been established, further studies were planned (1) to set specifications for formulation components, (2) to screen for excipients to optimize cake cosmetics, (3) to understand the formulation procedure in the context of process design, and (4) to examine the freeze-thaw tolerance of drug substance and drug product.

A polysorbate 80 (PS80) titration was performed in a lyophilization buffer matrix containing 0.2 mg/ml ClfA and 10 mM succinate, pH 6.0, while varying the sucrose content from 3% to 4.5% to 6% w/v. PS80 concentrations ranged from zero (0) to 0.005% to 0.01% v/v. This experiment was performed to (1) determine the need for polysorbate in a pre-lyophilized liquid formulation, (2) set a specification concentration for polysorbate in a pre-lyophilized liquid formulation, and (3) to also set a specification for sucrose concentration in a pre-lyophilized liquid formulation.

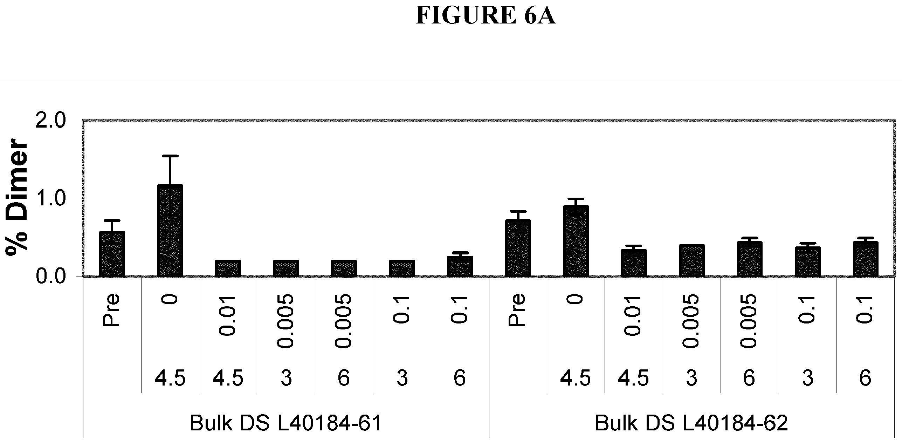

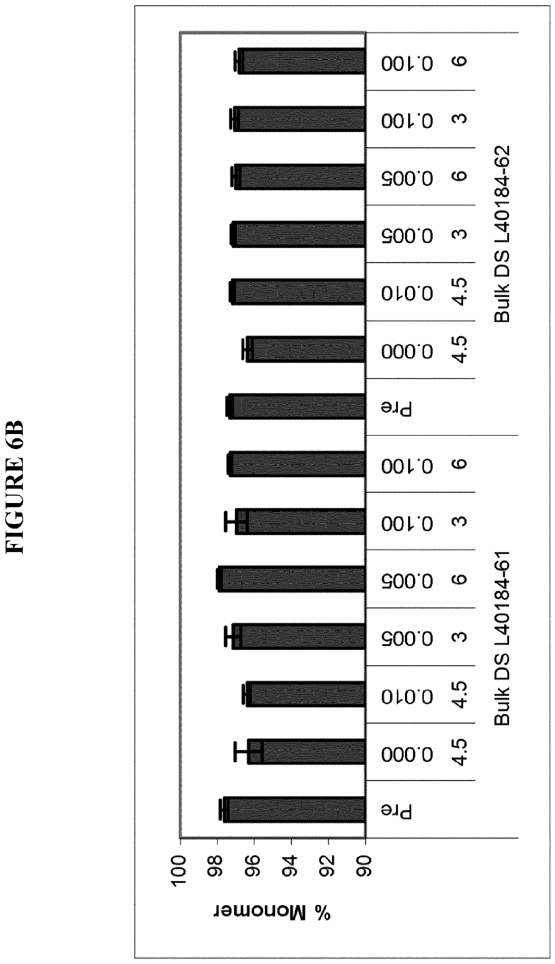

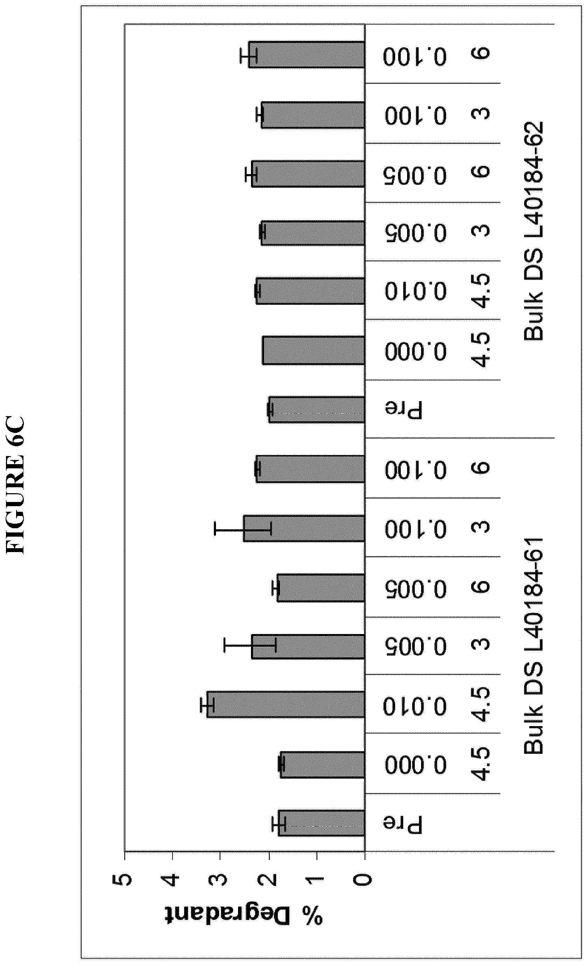

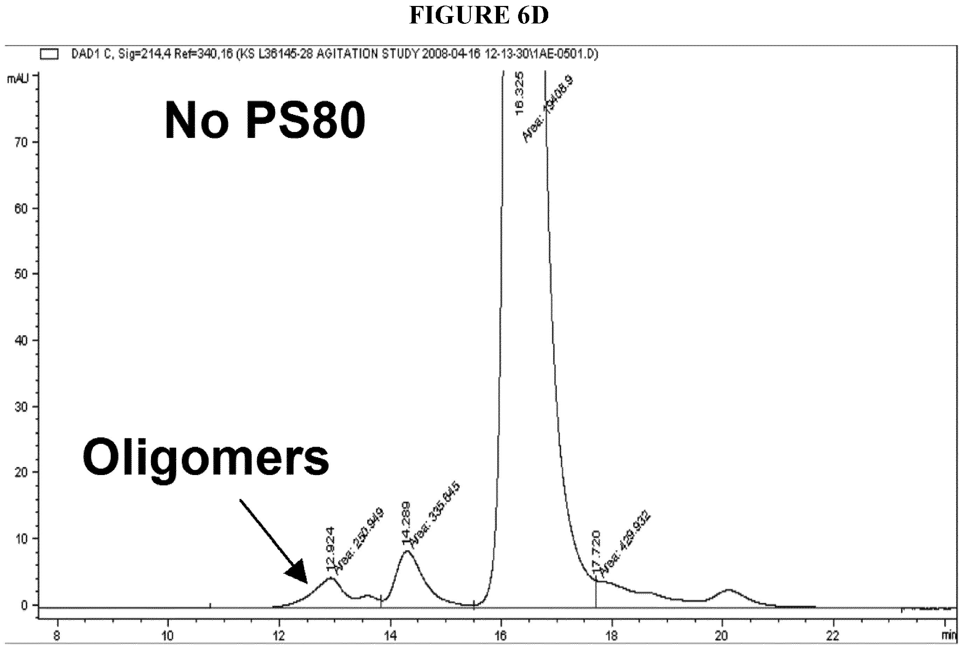

An agitation experiment was performed with varying PS80 and sucrose concentrations. Figure 6, panels A, B and C show percent dimer, percent monomer, and percent degradant (cleavage) results by SE-HPLC after 24 hours of agitation at room temperature for the L40184-61 (200 µg/ml ClfA, 6% sucrose, 10 mM succinate pH 6.0, 0.005% PS80) and L401840-62 (200 µg/ml ClfA, 4.5% sucrose, 10 mM succinate pH 6.0, 0.01% PS80) formulations. The formulations containing no PS80 exhibited an increase in percent dimer formation after agitation relative to the t = 0 sample (Figure 6D). In the presence of even the lowest amount of PS80 (0.005%), the amount of dimerization is less than that of t = 0, suggesting that PS80 was beneficial to the formulation. Furthermore, SE-HPLC analysis of the "no PS80" control showed the presence of higher order oligomers (larger than dimers) after agitation, whereas the samples containing PS80 did not. The recommendation for Polysorbate 80 was therefore established at 0.01 ± 0.005%. The varying amounts of sucrose (3, 4.5, and 6%) did not show any differences in the SEC data. Also, percent monomer and degradants did not seem to be largely affected by the presence or absence of PS80, or with varying amounts of sucrose.

Mixing studies were conducted to determine whether various excipients would have an adverse effect on ClfA during the formulation/mixing stage of the manufacturing process. The various excipients included sucrose at 0% and 5%, trehalose at 0% and 5%, and a combination of sucrose and mannitol at 4% sucrose and 1% mannitol. The excipients were chosen based on their common use in lyophilization. For these experiments, the formulation base included ClfA at 0.3 mg/ml, 10 mM succinate, pH 6.0, and polysorbate 80 at 0.01%. The samples were formulated, stored overnight at 2-8°C and the studies were initiated the following morning. The formulations were placed in 30 mL Shott type 1 glass vials with West 4432 grey butyl stoppers and mounted on a VWR Microplate Shaker. The vials were mixed at a continuous 500 RPM at room temperature, and were sampled at t = 0, 2, 4, 8, and 24 hours with SE-HPLC being used as the primary assay.

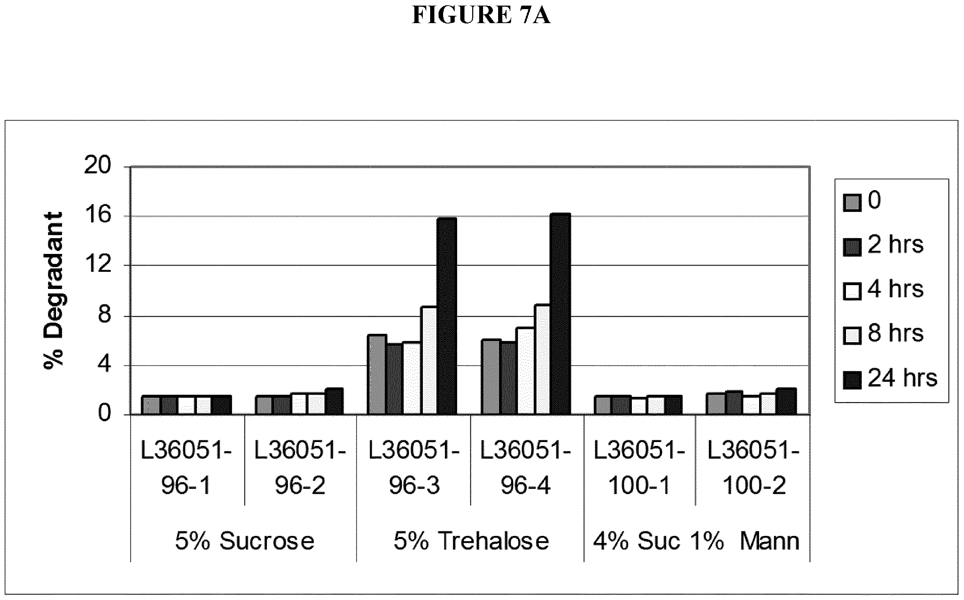

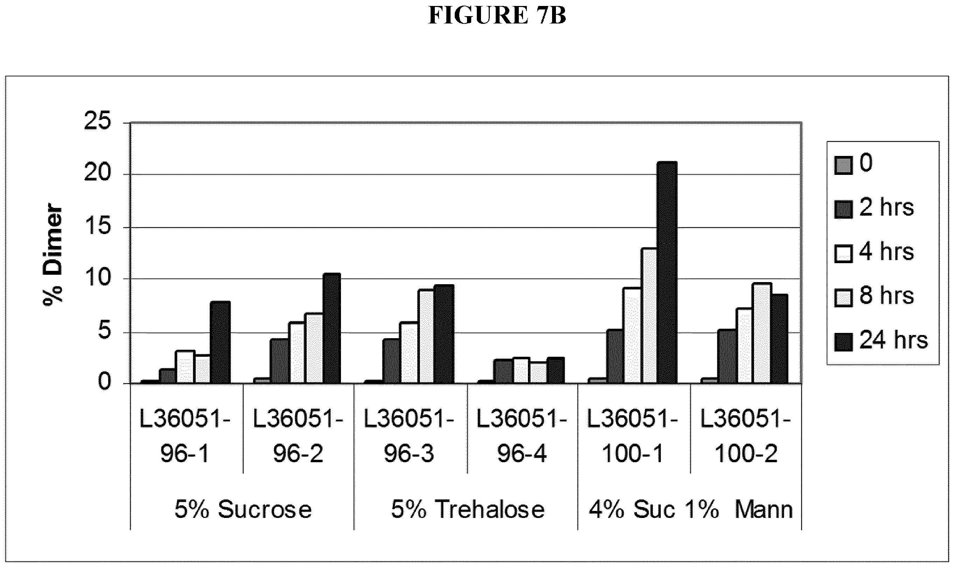

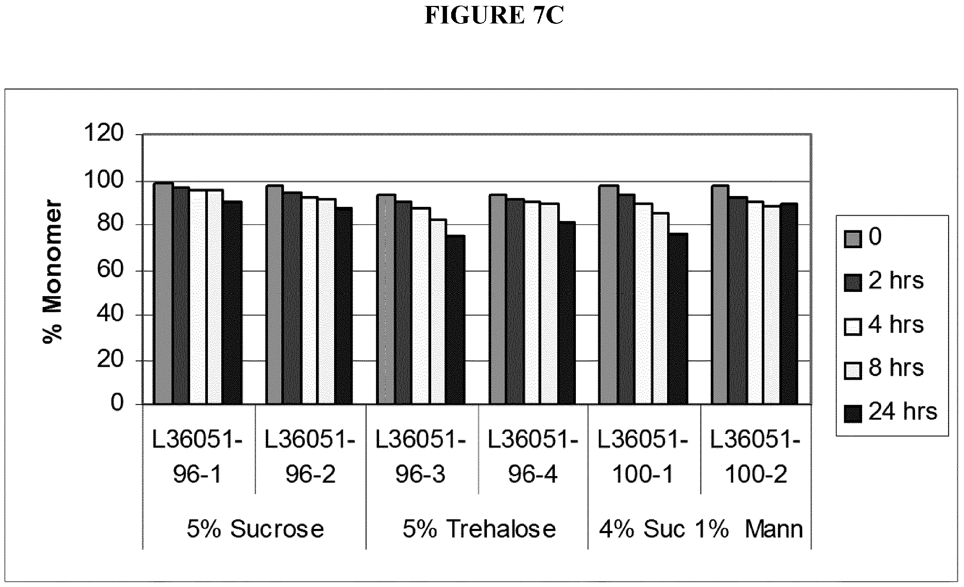

Several lots (L36051-96-1, L36501-96-2, L36051-96-3, L36051-96-4, L36051-100-1, and L36051-100-2) were examined in mixing studies to determine if sucrose, trehalose and sucrose/mannitol have an effect on the stability of ClfA when mixed for 24 hours at room temperature. Figure 7, panels A, B and C represent percent degradant (cleavage), percent dimer, and percent monomer reported by SE-HPLC for each formulation over the time course. No major degradation was seen with the sucrose alone and sucrose/mannitol samples; however, the trehalose formulation showed a significant enhancement of degradation. The dimerization rate was comparable for both DS lots of ClfA with sucrose alone, but a lot-dependent increase in dimers was seen with trehalose and sucrose/mannitol. The changes in monomer reflected the changes in degradants and dimers.

Various combinations of sucrose, methionine, glycine, and mannitol were formulated with ClfA and subsequently tested for ClfA stability by way of accelerated stability tests. Accelerated stability tests involve storing a formulation (liquid or lyophilized) at a temperature that is higher than the planned storage temperature, and monitoring the ClfA protein over time for signs of degradation. In the case of lyophilized ClfA, the recommended storage temperature is 2-8°C, thus 25°C and 37°C would be considered "accelerated" conditions. Eight formulations (see Table 13), each of which contained ClfA at 0.300 mg/ml, 10 mM succinate pH 6.0, and polysorbate 80, and containing varying combinations of sucrose, methionine, glycine, and mannitol, were made to examine their effect on (1) pre-lyophilized liquid stability of ClfA, (2) stability of lyophilized cakes, and (3) lyophilized cake cosmetics. Methionine was chosen for its ability to prevent oxidation of protein, glycine for its general stabilization attributes via excluded volume, and mannitol for its propensity to improve cake cosmetics. Liquid formulations were placed on accelerated stability and monitored over one week by SE-HPLC, and RP-HPLC at two weeks. Lyophilized cakes were stored at 2-8°C and 37°C and monitored at two months. Cake cosmetics were evaluated visually relative to the sucrose-only formulation.

**TABLE 13**

| **Sample #** | **ClfA Protein** | **Buffer Succinate mM** | **Sucrose %** | **PS 80 %** | **Methionine mM** | **Glycine %** | **Mannitol %** | **pH** |
|---|---|---|---|---|---|---|---|---|
| 1 | 200 | 10 | 4.5 | 0.01 | 0 | 0 | 0 | 6.0 |
| 2 | 200 | 10 | 4.5 | 0.01 | 0 | 2 | 0 | 6.0 |
| 3 | 200 | 10 | 4.5 | 0.01 | 25mM | 0 | 0 | 6.0 |
| 4 | 200 | 10 | 4.5 | 0.01 | 25mM | 2 | 0 | 6.0 |
| 5 | 200 | 10 | 4.5 | 0.01 | 0 | 0 | 1 | 6.0 |
| 6 | 200 | 10 | 4.5 | 0.01 | 0 | 2 | 1 | 6.0 |
| 7 | 200 | 10 | 4.5 | 0.01 | 25mM | 0 | 1 | 6.0 |
| 8 | 200 | 10 | 4.5 | 0.01 | 25mM | 2 | 1 | 6.0 |

Size exclusion data for the liquid samples at 2-8°C, 25°C, and 37°C after one week showed that none of the examined excipients improved the stability of ClfA (in terms of cleavage) compared to the sucrose-alone control (formulation - 109-1). At t = 0, all formulations had very similar profiles. All formulations showed a slight loss of the main peak height as a function of temperature coupled with a concurrent increase in smaller peaks flanking the main peak, caused by degradation products. It was observed that the presence of the various excipients did not show any marked improvements over the sucrose-alone formulation.

Additionally, the formulations were lyophilized and inspected for cake cosmetics. Compared to the sucrose-only control, none of the excipients at the tested proportions offered any improvements in the visual quality of the cakes. The lyophilized cakes were also subjected to stability testing and examined for degradation (cleavage) with SE-HPLC after two months at 2-8°C and 37°C. Results indicated that all formulations were stable.

### EXAMPLE 12: FORMULATION OF A CLFA LYOPHILIZED DRUG PRODUCT: FREEZE-THAW

Freeze-thaw studies were performed on several lots of ClfA protein (*i.e.,* L40184-63, -64 and -65). The formulation comprised 0.2 mg/ml of ClfA, 10 mM succinate, pH 6.0, 4.5% sucrose w/v and 0.01% polysorbate 80 v/v. The study was divided into three arms: (1) 3x freeze-thaw of unformulated ClfA (drug substance); (2) 3x freeze thaw of formulated ClfA (drug product); and (3) drug substance frozen and thawed three times and formulated to drug product after each thaw. Analysis was performed using SE-HPLC.

The purpose of the study was to assess the stability of the protein after multiple freeze-thaws as a drug substance and drug product. The samples were analyzed after each thaw using SE-HPLC to detect any changes in degradation (cleavage) of ClfA. It was observed that three freeze-thaw events showed no increase in degradation by the third thaw for two of the three lots examined. One lot in particular showed larger changes in degradation than the others.

### EXAMPLE 13: FORMULATION OF A CLFA/CP5/CP8 (TRI-ANTIGEN) LYOPHILIZED DRUG PRODUCT

Based on the success of stabilizing ClfA by lyophilization, further drug products were formulated by combining ClfA with CP5 and CP8 Staphylococcus antigens and CRM conjugates. Some embodiments of the liquid formulation (pre-lyophilization or post-reconstitution) contain 200µg of rClfA*m* and 100µg each of CP5-CRM₁₉₇ and CP8-CRM₁₉₇ conjugates per 0.5 ml of product. In some embodiments, the liquid formulation contains 100µg of rClfA*m* and 50µg each of CP5-CRM₁₉₇ and CP8-CRM₁₉₇ conjugates per 0.5 ml of product.

Various bulking agents were evaluated, including sucrose, mannitol and trehalose. Sucrose was observed to offer the best stability based on mixing studies, cake cosmetics and short-term liquid stability.

Short term accelerated stability of liquid formulations based on three lots at varying pH suggested the pH 6.0 is optimal. CP5- and CP8-CRM₁₉₇ conjugates were stable within a pH range of 5.0 to 7.0.

Various buffer systems, which buffer around pH 6, including succinate and histidine, were tested. No difference in the melting temperature (Tₘ) of rClfA*m* was observed using histidine or succinate buffers. Both buffers were compatible with both CP5 and CP8 CRM₁₉₇ conjugates. 10mM succinate buffer (pH 6.0) was chosen going forward.

Polysorbate 80 (PS80) was tested as a potential stabilizer in agitation studies. Agitation studies mimic shear and stress of transportation, which generally can lead to an increase in dimers or higher order oligomers. The result of these experiments suggests that PS80 prevents aggregation of ClfA. 0.01% (0.005 to 0.15%) polysorbate 80 was chosen going forward.

For reconstitution of the lyophilized product, a diluent that achieves physiological osmolality (*i.e.,* 250 to 300 mOsM) was chosen. In one embodiment, 60 mM NaCl was chosen as the diluent. Based on ability to deliver a 0.5mL dose after reconstitution, a fill volume of 0.64 to 0.66mL *per* vial was chosen. To ensure a reconstituted volume of 0.64 to 0.66mL to deliver a dose of 0.5mL, a fill volume of 0.66 to 0.68 mL *per* syringe was chosen.

The diluent formulation was determined to be optimal at 60mM for reconstituting a lyophilized tri-antigen cake to achieve a near-physiological osmolality of ∼300 mOsM. The 4.5% sucrose in the formulation makes a significant contribution to the osmolality of the liquid drug product, rendering an osmolality of about 200 mOsM without any additional salt.

Lyophilization parameters were chosen to generate a cake that is white and fluffy, with no melt back or shrinkage, and which yields a clear solution having a pH of 6.0 ± 0.3 upon reconstitution with 60 mM NaCl.

### EXAMPLE 14: PROCESS FOR FORMULATING TRI-ANTIGEN DRUG PRODUCT

An exemplary liquid formulation comprising 10 mM succinate, pH 6.0, 4.5% sucrose, and 0.01% Polysorbate 80 was made, filtered with a Millipore 0.22 µm PVDF membrane, aliquoted at 650 µL *per* 2 mL vial, and lyophilized using the cycle described below. The lyophilized cakes were reconstituted using syringes pre-filled with 60 mM NaCl as the diluent.

In one embodiment, the liquid formulation was assembled as follows: (a) 25% sucrose, 100mM succinate pH 6.0, 1% PS80, and WFI were combined to obtain 10mM succinate, 4.5% sucrose and 0.01% PS80. (b) The appropriate amount of rClfA*m*, CP5 conjugate and CP8 conjugate were added to the mixture of (a), which was then (c) sterilized through a 0.22µm filter. (d) Lyophilization vials were each filled with 0.65ml ± 0.1ml of the filtered solution, (e) lyophilized according to the parameters of Table 14, (f) stoppered, (g) sealed and (h) labeled. The lyophilized drug product was stored at 2-8°C.

**Table 14: Lyophilization Cycle**

| | **Shelf Temp. (°C)** | **Rate / Hold** | **Time (min)** | **Pressure (mTorr)** |
|---|---|---|---|---|
| Freezing | -50 | 0.3°C/min | 183.3 | Default^{a} |
| | -50 | Hold | 60 | |
| | -10 | 0.3°C/min | 133.3 | |
| Annealing | -10 | Hold | 120 | |
| | -50 | 0.3°C/min | 133.3 | |
| | -50 | Hold | 180 | |
| Primary drying | -50 | Hold | 30 | 50 |
| | -30 | 0.2° C/min | 100 | 50 |
| | -30 | Hold | 1920 | 50 |
| Secondary drying | 30 | 0.2°C/min | 300 | 50 |
| | 30 | Hold | 720 | 200 |
| | 5 | 0.5°C/min | 50 | 200 |
| | 5 | Hold | Until Stoppered | 200 |

| | | | | |
|---|---|---|---|---|
| a. Default means 400 mbar (∼40% of atmospheric pressure). | | | | |

Two experimental designs were conducted to determine the influence of active and inactive ingredients on the formulation process, recovery following filtration, and lyophilization. The key variables were the concentration of rClfAm and the CP5 and CP8 conjugates (±30% target). The overall recovery of these components from formulation to lyophilization was shown to be greater than 95% for all formulations. Data analysis (using DESIGN EXPERT software) suggested the following: (a) rClfA*m* purity is not affected by concentration of rClfA*m* within assay limitations, wherein the low dose (70µg/ml) exhibited a maximum measured purity of ∼93%, and the high dose (520µg,ml) exhibited a maximum measured purity of ∼97%; (b) the concentration of CP5 and CP8 conjugates can influence the recovery of the individual conjugates, but nonetheless remain within assay capabilities; and (c) all three components are not influenced by the PS 80 concentration. The results are depicted in Table 15.

**Table 15: Analysis of Tri-Antigen Formulation Following Lyophilization**

| **Lot Number** | **rClfA*m* Conc. (µg/mL)** | **rClfA*m* Purity (%)** | **CP5 (µg/mL)** | **CP8 (µg/mL)** | **pH** | **Appearance** | **Moisture (%)** | **Recon Time** |
|---|---|---|---|---|---|---|---|---|
| 1 | 74 | 93.5 | 36.4 | 38.5 | 6.08 | CC^{a} | 0.50 | < 30s |
| 2 | 573 | 97.2 | 35.8 | 39.3 | 6.05 | CC | 0.62 | < 30s |
| 3 | 79 | 93.8 | 299.6 | 28.5 | 6.08 | CC | 0.81 | < 30s |
| 4 | 543 | 97.2 | 292.5 | 26.0 | 6.06 | CC | 1.02 | < 30s |
| 5 | 76 | 93.6 | 29.8 | 311.8 | 6.07 | CC | 0.67 | < 30s |
| 6 | 550 | 97.5 | 26.8 | 321.7 | 6.06 | CC | 1.03 | < 30s |
| 7 | 75 | 93.5 | 251.4 | 243.3 | 6.08 | CC | 0.87 | < 30s |
| 8 | 544 | 97.4 | 263.4 | 250.7 | 6.05 | CC | 0.93 | < 30s |
| 9 | 395 | 97.1 | 201.2 | 195.8 | 6.05 | CC | 0.95 | < 30s |
| 10 | 106 | 95.4 | 52.9 | 57.9 | 6.05 | CC | 0.56 | < 30s |
| 11 | 396 | 97.7 | 201.4 | 201.5 | 6.06 | CC | 1.11 | < 30s |
| 12 | 101 | 95.1 | 49.8 | 53.8 | 6.07 | CC | 0.62 | < 30s |
| Average Recovery | 105.3 | 95.7 | 100.0 | 102.8 | 6.1 | NA | 0.81 | NA |
| SD | 4.2 | 1.8 | 10.6 | 15.0 | 0.0 | NA | 0.21 | NA |

| | | | | | | | | |
|---|---|---|---|---|---|---|---|---|
| ^{a}Clear and colorless. Based on the analysis of the % purity data, the concentration of ClfA in the formulation influences the assay capability. The specification will be > 90% for the low dose. | | | | | | | | |

In other experiments, the pH (ranging from 5.7 to 6.3, with the target being 6.0), the succinate (5mM to 15mM, with the target being lOmM), the Polysorbate 80 (0.005% to 0.15%, with the target being 0.01%), and the sucrose (3.0% to 6.0%, with the target being 4.5%) were varied in the formulation. The results, shown in Table 16, indicate that the acceptance criteria for the active drug products (rClfA*m* (397±6.7µg/mL), CP5-CRM₁₉₇ conjugate (179.8±64.0µg/mL), and CP8-CRM₁₉₇ conjugate (188.2±10.5µg/mL)) are within the control range.

**Table 16: Analysis of Tri-Antigen for Inactive Ingredients**

| | **Formulations** | | | | **Results** | | | | |
|---|---|---|---|---|---|---|---|---|---|
| | **Target pH** | **Succinate (mM)** | **PS 80 (%)** | **Sucrose (%)** | **pH** | **rClfA*m* % Purity** | **rClfA*m* strength (µg/ML)** | **CP5 strength (µg/mL)** | **CP8 strength (µg/mL)** |
| 1 | 6.0 | 10 | 0.01 | 4.5 | 6.07 | 97.9 | 390 | 181.6 | 187.2 |
| 2 | 5.7 | 15 | 0.015 | 6.0 | 5.75 | 97.9 | 410 | 175.6 | 184.8 |
| 3 | 5.7 | 5.0 | 0.005 | 3.0 | 5.83 | 98.1 | 400 | 183.2 | 200.0 |
| 4 | 5.7 | 5.0 | 0.015 | 6.0 | 5.83 | 98.0 | 400 | 178.0 | 183.7 |
| 5 | 5.7 | 15.0 | 0.005 | 3.0 | 5.75 | 97.9 | 400 | 174.2 | 166.7 |
| 6 | 6.3 | 15.0 | 0.005 | 6.0 | 6.32 | 98.0 | 390 | 186.8 | 199.7 |
| 7 | 6.3 | 15.0 | 0.015 | 3.0 | 6.31 | 98.3 | 400 | 179.6 | 183.3 |
| 8 | 6.3 | 5.0 | 0.015 | 3.0 | 6.27 | 98.2 | 400 | 181.9 | 196.9 |
| 9 | 6.3 | 5.0 | 0.005 | 6.0 | 6.28 | 98.1 | 390 | 177.4 | 191.7 |
| Average | 6.0 | 10.0 | 0.01 | 4.5 | 6.0 | 98.0 | 397.8 | 179.8 | 188.2 |
| SD | 0.3 | 5.0 | 0.005 | 1.5 | 0.3 | 0.1 | 6.7 | 4.0 | 10.5 |
| Min | 5.7 | 5.0 | 0.005 | 3.0 | 5.75 | 97.9 | 390 | 174.2 | 166.7 |
| Max | 6.3 | 15.0 | 0.015 | 6.0 | 6.32 | 98.3 | 410 | 186.8 | 200 |

| | | | | | | | | | |
|---|---|---|---|---|---|---|---|---|---|
| Active ingredients kept constant at 400 µg/mL rClfA*m*, 200 µg/mL CP5 and 200 µg/mL CP8. | | | | | | | | | |

Data of representative formulation lots are shown in Tables 17 and 18. Three representative lots were formulated, filled and lyophilized at the high dose (400µg/mL rClfA*m*, 200µg/mL each of CP5- and CP8-CRM₁₉₇ conjugates), and two at the low dose (200µg/mL rClfA*m*, 50µg/mL each of CP5- and CP8-CRM₁₉₇ conjugates). The formulations are well within the target acceptance criteria for purity, strength, moisture, appearance and pH. These data were used to bracket mid level doses.

**Table 17: Analysis of t = 0 Data from High Dose Formulations**

| **Tests** | **1** | **2** | **3** | **Average ± SD** |
|---|---|---|---|---|
| Appearance | wfc^{a} | wfc | wfc | NA |
| Reconstitution time | <30 sec | <30 sec | <30 sec | NA |
| pH | 5.97 | 6.10 | 6.07 | 6.05 ± 0.07 |
| Moisture | 0.64 | 0.73 | 0.69 | 0.69 ± 0.05 |
| rClfA*m* purity (%) | 97.5 | 98.5 | 97.9 | 98.0 ± 0.5 |
| rClfA*m* strength (µg/mL) | 410 | 430 | 390 | 410 ± 20 |
| CP5 strength (µg/mL) | 187 | 183 | 182 | 184 ± 3 |
| CP8 strength (µg/mL) | 165 | 179 | 187 | 177 ± 11 |
| Identity rClfA*m* | Positive | Positive | ND | NA |
| Identity CP5 and CP8 polysaccharide | Positive | Positive | ND | NA |
| Identity CRM₁₉₇ | Positive | Positive | ND | NA |

| | | | | |
|---|---|---|---|---|
| ^{a.}wfc: white fluffy cake | | | | |

**Table 18: Analysis of t=0 Data from Low Dose Formulations**

| **Tests** | **1** | **2** | **3** | **Average** ± **SD** |
|---|---|---|---|---|
| Appearance | wfc^{aa} | wfc | wfc | NA |
| Reconstitution time | < 30 sec | <30 sec | < 30 sec | NA |
| pH | 5.96 | 6.05 | 6.08 | 6.03 ± 0.06 |
| Moisture | 0.37 | 0.56 | 1.49 | 0.81 ± 0.60 |
| rClfA*m* purity (%) | 95.5 | 95.4 | 95.4 | 95.4 ± 0.1 |
| rClfA*m* strength (µg/mL) | 110.0 | 106.0 | 108.0 | 108 ± 2 |
| CP5 strength (µg/mL) | 49.0 | 53.0 | 47.0 | 50 ± 3 |
| CP8 strength (µg/mL) | 47.0 | 58.0 | 51.0 | 52 ± 6 |
| Identity rClfAm | Positive | ND | ND | NA |
| Identity CP5 and CP8 polysaccharide | Positive | ND | ND | NA |
| Identity CRM₁₉₇ | Positive | ND | ND | NA |

| | | | | |
|---|---|---|---|---|
| ^{a.} wfc: white fluffy cake | | | | |

### EXAMPLE 15: TRI-ANTIGEN DRUG PRODUCT STABILITY

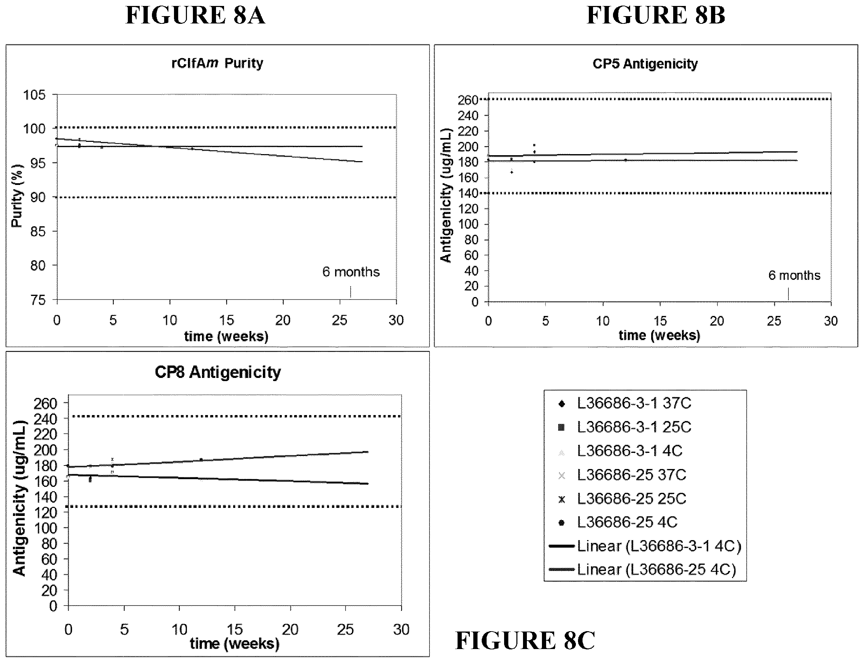

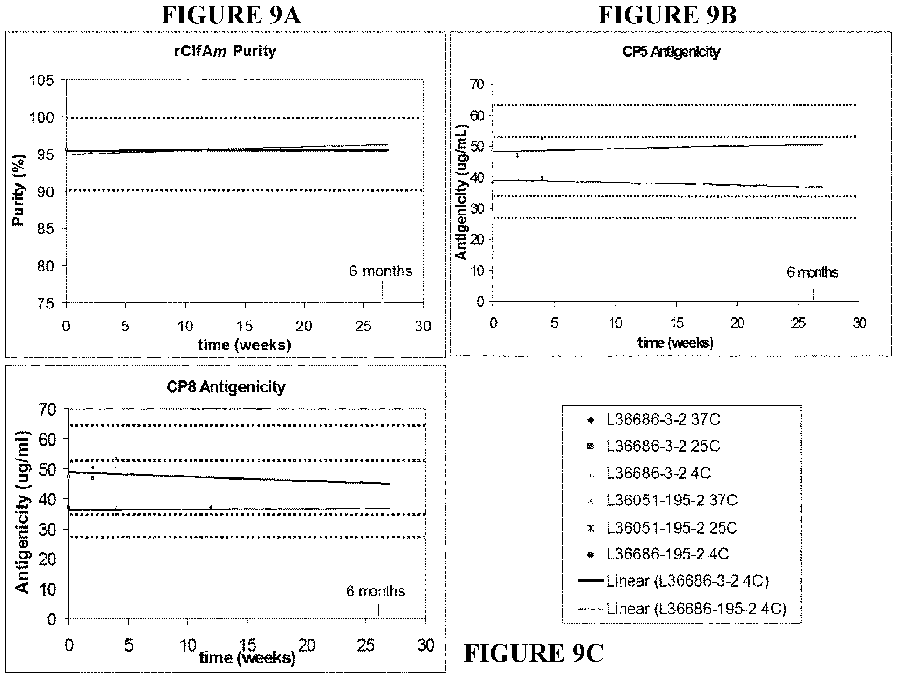

Stability assays were performed on six lots of lyophilized tri-antigen formulations. Some of the key assays were reverse phase chromatography (*e.g*., RP-HPLC) for rClfA*m* strength and purity, and nephelometry for CP5-CRM₁₉₇ and CP8-CRM₁₉₇ strength. The lyophilized cakes were placed at 2°C-8°C, 25°C and 37°C and analyzed at selected time points. Cakes were reconstituted immediately prior to testing. The test results show no change in strength (concentration) or purity of rClfA*m* and strength (antigenicity) of the conjugates after four weeks in the dried form (Figures 8 and 9). rClfA*m* concentration and purity, and CP5-CRM₁₉₇/CP8-CRM₁₉₇ concentration (antigenicity), were also determined by RP-HPLC and nephelometry, respectively, for periods as long as three months. Linear regression analysis performed on the samples stored at 2°C-8°C indicates that the samples are stable for a minimum of six months (Figures 8 and 9). Samples were tested at both high (L36686-3-1 and L36686-25) and low (L36686-3-2 and L360510195-2) dosages (Tables 19-22).

**Table 19: Stability of Tri-Antigen Formulated at 400 µg/ML rClfAm, 200 µg/mL CP5- and CP8- Conjugates (L36686-3-1)**

| **Tests** | **2-8°C** | | | **RT** | | **37°C** | |
|---|---|---|---|---|---|---|---|
| | **t=0** | **4w** | **3 mo** | **2w** | **4w** | **2w** | **4w** |
| Appearance | wfc | wfc | wfc | wfc | wfc | wfc | wfc |
| pH | 5.97 | 5.95 | 5.97 | 5.96 | 5.96 | 5.96 | 5.96 |
| rClfA*m* Concentration (µg/mL) | 410 | 400 | 430 | 410 | 410 | 410 | 410 |
| rClfA*m* Purity (%) | 97.5 | 97.1 | 97.4 | 97.3 | 97.2 | 97.6 | 97.3 |
| CP5-CRM₁₉₇ Antigenicity (µg/mL) | 187 | 188 | 190 | 184 | 202 | 167 | 193 |
| CP8-CRM₁₉₇ Antigenicity (µg/mL) | 165 | 171 | 161 | 160 | 171 | 164 | 180 |
| Moisture (%) | (1.64 ± 0.16 | 1.02 ± 0.06 | 0.68 ± 0.03 | ND | 1.15 ± 0.07 | ND | 1.30 ± 0.20 |

| | | | | | | | |
|---|---|---|---|---|---|---|---|
| wfc: white fluffy cake | | | | | | | |

**Table 20: Stability of Tri-Antigen Formulated at 400 µg/ML rClfAm, 200 µg/mL CP5- and CP8- Conjugates (L36686-25)**

| **Tests** | **2-8°C** | | | **RT** | | **37°C** | |
|---|---|---|---|---|---|---|---|
| | **t=0** | **4w** | **3 mo** | **2w** | **4w** | **2w** | **4w** |
| Appearance | wfc | wfc | wfc | wfc | wfc | wfc | wfc |
| pH | 6.10 | 6.09 | 6.10 | 6.10 | 6.12 | 6.11 | 6.11 |
| rClfA*m* Concentration (µg/mL) | 440 | NA | 420 | 400 | 394 | 410 | 396 |
| rClfA*m* Purity (%) | 98.5 | NA | 97.0 | 98.4 | 97.2 | 98.4 | 97.1 |
| CP5-CRM₁₉₇ Antigenicity (µg/mL) | 183 | 180 | 182 | 183 | 181 | 171 | 187 |
| CP8-CRM₁₉₇ Antigenicity (µg/mL) | 179 | 178 | 187 | 179 | 188.2 | 178 | 185 |
| Moisture (%) | 0.73 ± 0.13 | 1.25 ± 0.23 | 0.80 ± 0.07 | ND | 0.57 ± 0.07 | ND | 1.40 ± 0.25 |

| | | | | | | | |
|---|---|---|---|---|---|---|---|
| wfc: white fluffy cake | | | | | | | |

**Table 21: Stability of Tri-Antigen Formulated at 100 µg/ML rClfAm, 50 µg/ML CP5- and CP8- Conjugates (L36686-3-2)**

| **Tests** | **2-8°C** | | | **RT** | | **37°C** | |
|---|---|---|---|---|---|---|---|
| | **t=0** | **4w** | **3 mo** | **2w** | **4w** | **2w** | **4w** |
| Appearance | wfc | wfc | wfc | wfc | wfc | wfc | wfc |
| pH | 5.96 | 5.93 | 5.97 | 5.93 | 5.93 | 5.93 | 5.92 |
| rClfA*m* Concentration (µg/mL) | 110 | 100 | 110 | 110 | 100 | 110 | 110 |
| rClfA*m* Purity (%) | 95.5 | 95.2 | 95.5 | 95.3 | 95.2 | 95.3 | 95.2 |
| CP5-CRM₁₉₇ Antigenicity (µg/mL) | 49 | 48 | 50 | 48 | 52 | 47 | 53 |
| CP8-CRM₁₉₇ Antigenicity (µg/mL) | 47 | 51 | 46 | 47 | 53 | 50 | 53 |
| Moisture (%) | 0.37 + 0.17 | 0.73 ± 0.15 | | NA | 0.80 ± 0.07 | NA | 0.83 ± 0.01 |

| | | | | | | | |
|---|---|---|---|---|---|---|---|
| wfc: white fluffy cake | | | | | | | |

**Table 22: Stability of Tri-Antigen Formulated at 100 µg/ML rClfAm, 50 µg/mL CP5- and CP8- Conjugates (L36051-195-2)**

| **Tests** | **2-8°C** | | | **RT** | | **37°C** | |
|---|---|---|---|---|---|---|---|
| | **t=0** | **4w** | **3 mo** | **2w** | **4w** | **2w** | **4w** |
| Appearance | wfc | wfc | wfc | Wfc | wfc | wfc | wfc |
| pH | 5.93 | 5.91 | 5.93 | 5.98 | 5.92 | 5.94 | 5.92 |
| rClfA*m* Concentration (µg/mL) | 100 | 100 | 110 | 110 | 100 | 110 | 100 |
| rClfA*m* Purity (%) | NA | 95.1 | 95.5 | 95.3 | 95.2 | 95.3 | 94.9 |
| CP5-CRM₁₉₇ Antigenicity (µg/mL) | 38.1 | 39.8 | 37.6 | NA | 39.5 | 39.8 | 40 |
| CP8-CRM₁₉₇ Antigenicity (µg/mL) | 37.2 | 34.9 | 37 | NA | 37.3 | 36 | 36.9 |
| Moisture (%) | 0.48 ± 0.06 | 0.32 ± 0.04 | 0.98 ± 0.12 | NA | 0.66 ± 0.09 | NA | 0.82 ± 0.12 |

| | | | | | | | |
|---|---|---|---|---|---|---|---|
| wfc: white fluffy cake | | | | | | | |

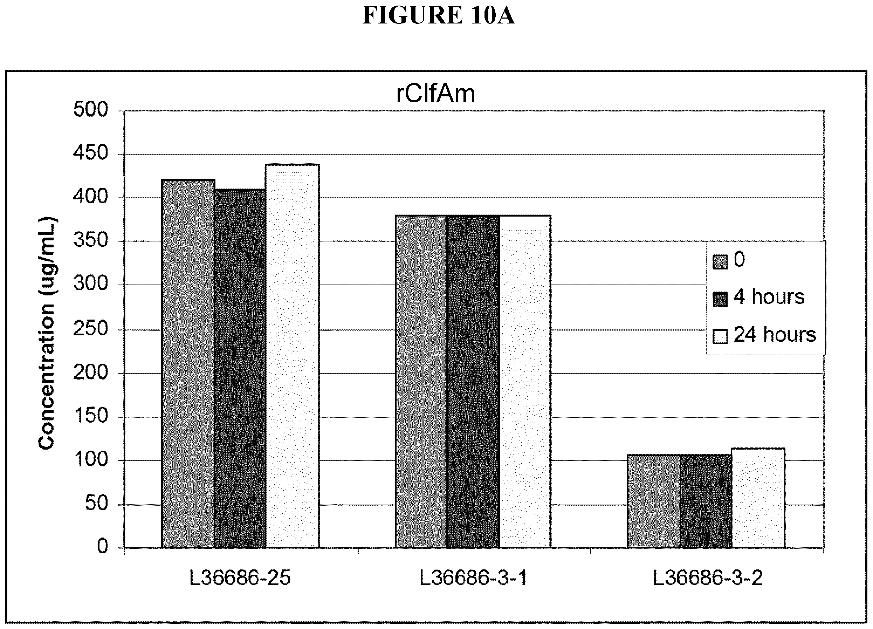

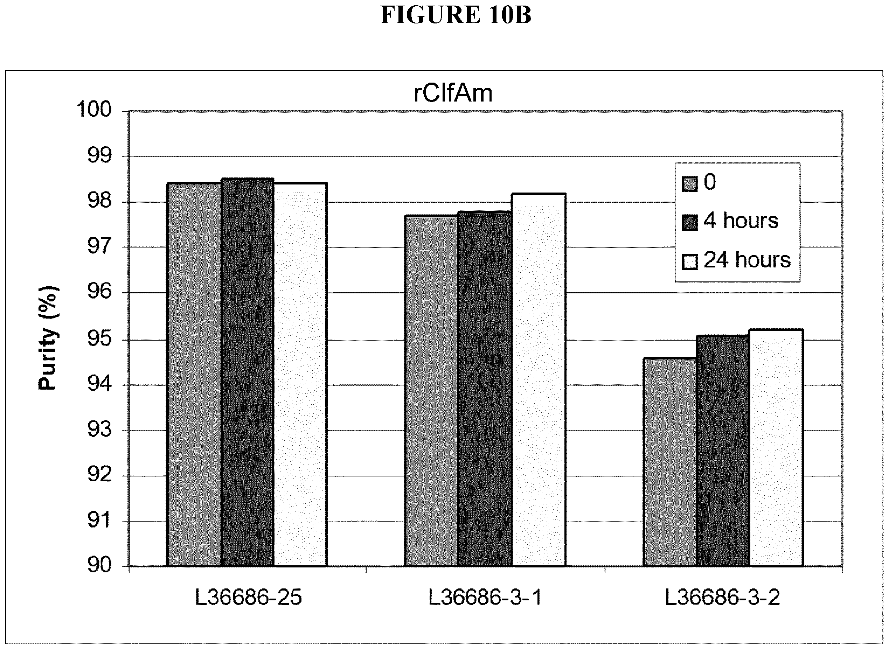

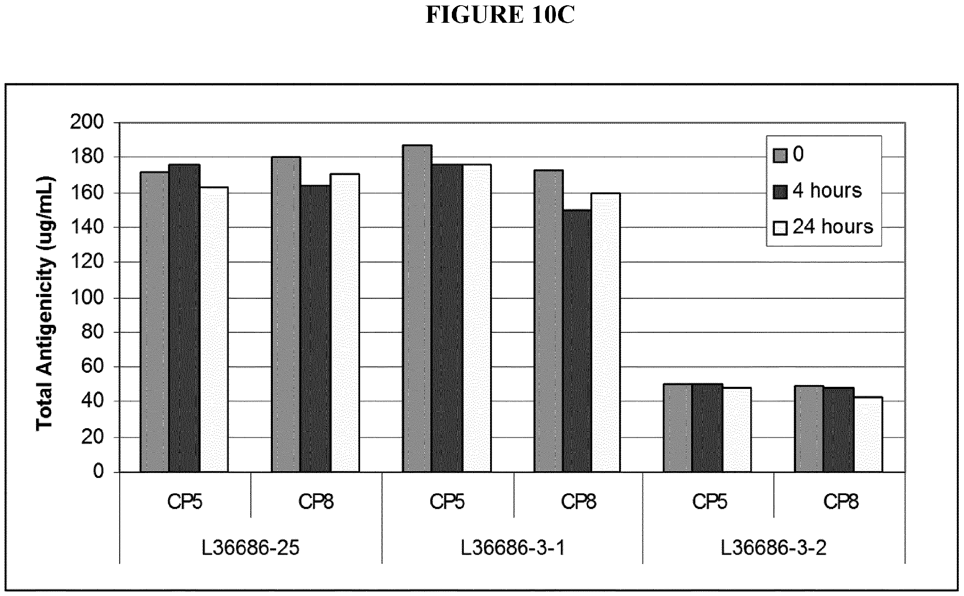

Short term stability assays were also performed. Lyophilized tri-antigen formulations were reconstituted with 60mM NaCl diluent and incubated at room temperature for various time, with t = 0, 4 hours and 24 hour time points being analyzed for rClfA*m* strength and purity, and CP5-CRM₁₉₇/CP8-CRM₁₉₇ strength (Tables 23-25). High dose (400 µg/mL rClfA*m*, 200 µg/mL CP5-CRM₁₉₇ and 200 µg/mL CP8-CRM₁₉₇) and low dose (200µg/mL rClfA*m*, 50µg/mL CP5- CRM₁₉₇ and CP8-CRM₁₉₇) formulations were examined. Both doses show stability of the components at 24 hours at room temperature (post-reconstitution) as seen in Figure 10.

**Table 23: Short Term Stability of Tri-Antigen Formulated at 400 µg/ML rClfAm, 200 µg/ML CP5- and CP8- Conjugates (L36686-25)**

| | **T = 0** | **T= 4 hours** | **T = 24 hours** |
|---|---|---|---|
| rClfAm concentration (µg/mL) | 420 | 410 | 440 |
| rClfA purity (%) | 98.4 | 98.5 | 98.4 |
| CP5-CRM₁₉₇ concentration (µg/mL) | 171 | 176 | 163 |
| CP8-CRM₁₉₇ concentration (µg/mL) | 180 | 163 | 170 |

**Table 24: Short Term Stability of Tri-Antigen Formulated at 400 µg/ML rClfAm, 200 µg/mL CP5- and CP8- Conjugates (L36686-3-1)**

| | **T=0** | **T= 4 hours** | **T = 24 hours** |
|---|---|---|---|
| rClfAm concentration (µg/mL) | 380 | 380 | 380 |
| rClfA purity (%) | 97.7 | 97.8 | 98.2 |
| CP5-CRM₁₉₇ concentration (µg/mL) | 186 | 176 | 176 |
| CP8-CRM₁₉₇ concentration (µg/mL) | 172 | 150 | 160 |

**Table 25: Short Term Stability of Tri-Antigen Formulated at 100 µg/ML rClfAm, 50 µg/mL CP5- and CP8- Conjugates (L36686-3-2)**

| | **T=0** | **T= 4 hours** | **T = 24 hours** |
|---|---|---|---|
| rClfAm concentration (µg/mL) | 106 | 107 | 113 |
| rClfA purity (%) | 94.6 | 95.1 | 95.2 |
| CP5- CRM₁₉₇ concentration (µg/mL) | 51 | 51 | 48 |
| CP8- CRM₁₉₇ concentration (µg/mL) | 49 | 49 | 43 |

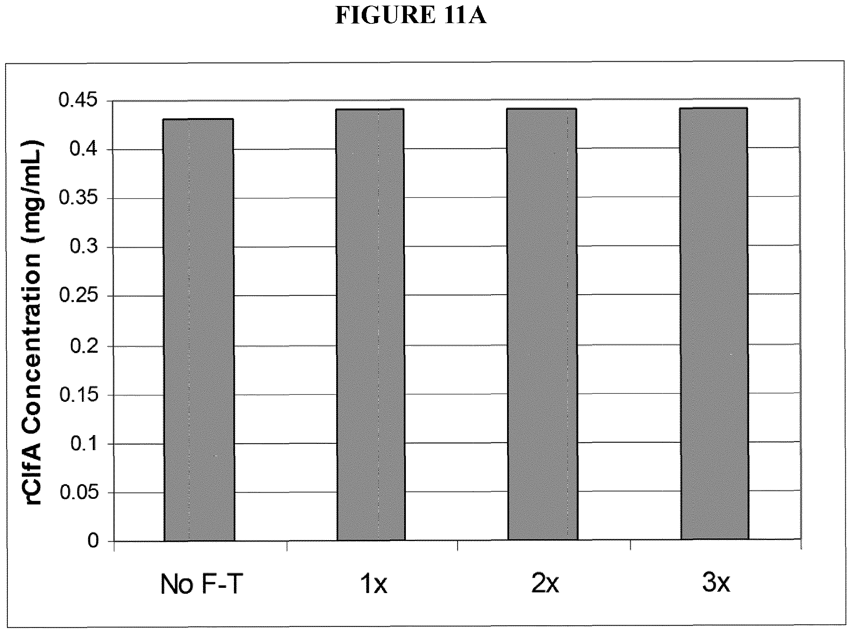

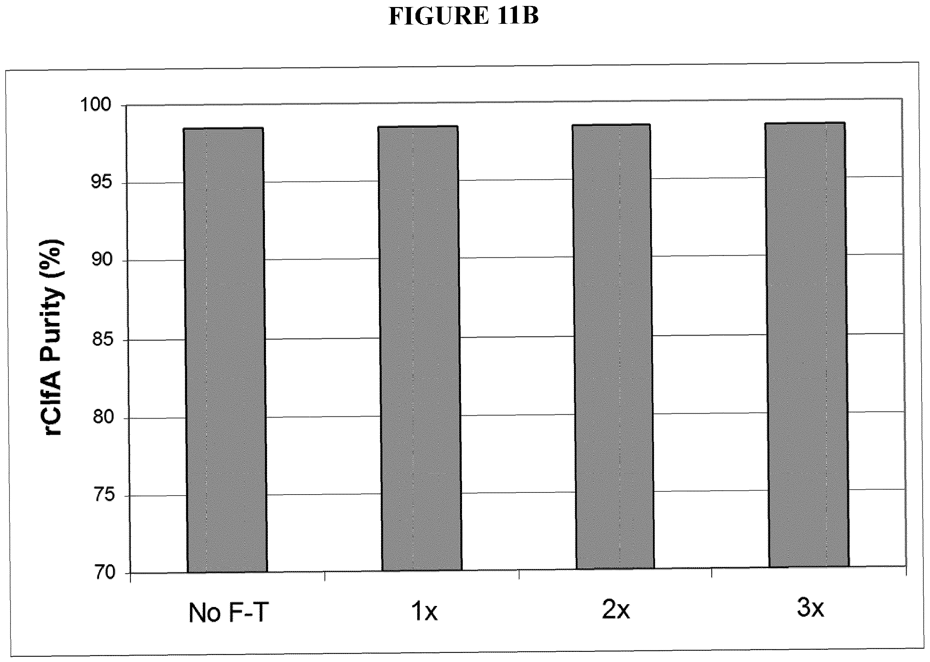

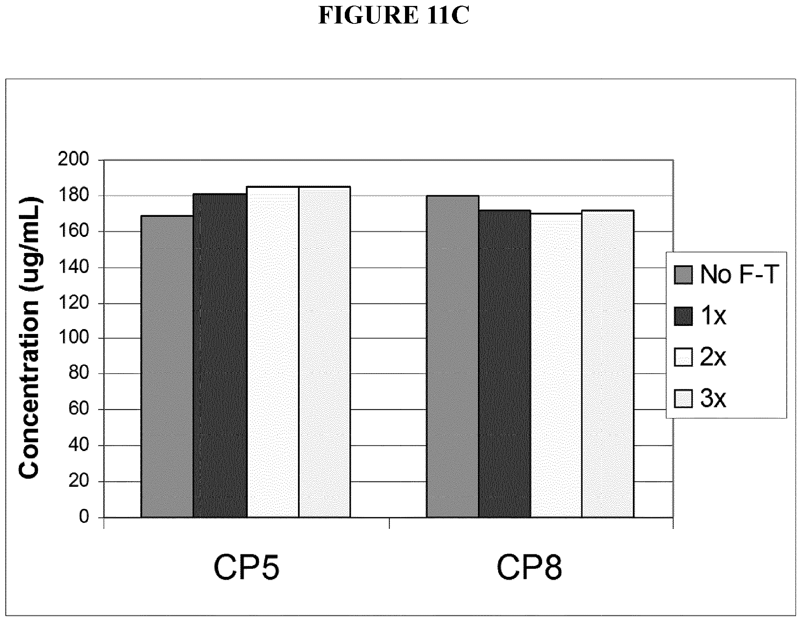

Freeze-thaw studies were also performed. The high dose tri-antigen drug product (400 µg/mL rClfA*m*, 200 µg/mL CP5-CRM₁₉₇ and 200 µg/mL CP8-CRM₁₉₇) was subjected to three freeze-thaw cycles and analyzed for rClfA*m* strength and purity, and CP5-CRM₁₉₇/CP8-CRM₁₉₇ strength. Virtually no change in concentration was observed for each component, suggesting that the active components of the drug product are stable over the three cycles (Figure 11, Table 26).

**Table 26: Summary of Freeze-Thaw Data of L36686-25**

| | **1x Freeze-Thaw** | **2x Freeze-Thaw** | **3x Freeze-Thaw** |
|---|---|---|---|
| rClfA*m* concentration (µg/mL) | 440 | 440 | 440 |
| rClfA*m* purity (%) | 98.5 | 98.5 | 98.5 |
| CP5-CRM₁₉₇ concentration (µg/mL) | 181 | 185 | 185 |
| CP8-CRM₁₉₇ concentration (µg/mL) | 172 | 170 | 172 |

### EXAMPLE 16: CLFA/CP5/CP8/MNTC (TETRA-ANTIGEN) DRUG PRODUCT: CHARACTERIZATION OF CLFA

The successful stabilization of ClfA in the tri-antigen formulation led to development of formulations further comprising a *S*. *aureus* MntC protein. In addition, Clumping factor A was updated from a T7 tagged version (rClfAm) to one without the tag (r*m*ClfA). CP5- and CP8-CRM₁₉₇ remained the same in terms of carrier protein and saccharide repeat unit.

Because of the various changes to the components of the drug product, a systematic and rational approach was taken to formulate a new drug product that was compatible with all four active components. Activities included pre-formulation characterization of antigens, development of rationales for formulation components, demonstration of stability and robustness of the formulated drug product, and the development of a formulation process. Studies were designed around the following doses:
1. 400/400/200/200 µg/mL of r*m*ClfA/MntC/CP5-CRM₁₉₇/CP8-CRM₁₉₇, and
2. 200/200/100/100 µg/mL of r*m*ClEA/MntC/CP5-CRM₁₉₇/CP8-CRM₁₉₇.

Biophysical characterization using multiple orthogonal methods (Circular Dichroism, fluorescence, capillary differential scanning calorimetry and OD₃₅₀) were performed on two lots of rClfA*m* (L40184-77 and L40256-26; matrix: 10 mM succinate pH 6.0, 20 mM NaCl) and two lots of r*m*ClfA (L40227-36 and L40256-28; matrix: 10 mM histidine pH 6.5). The resulting data showed 1) comparability between rClfA*m* and r*m*ClfA, 2) lot-to-lot consistency within each rClfA construct, and 3) provided a framework for a pH working range moving forward.

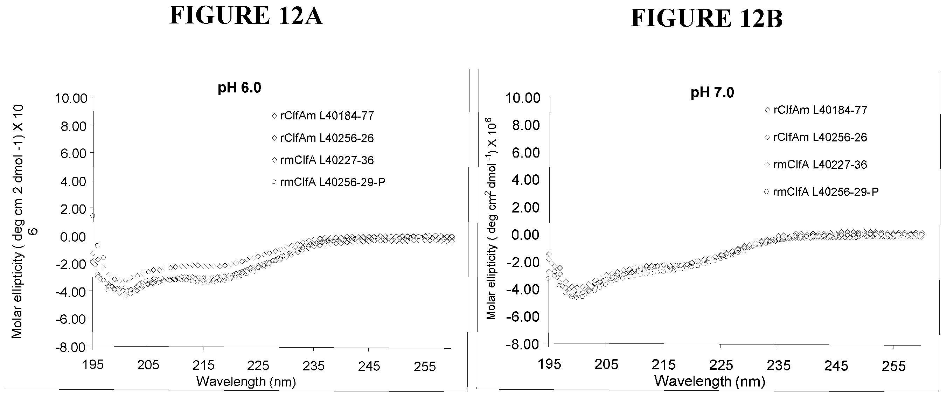

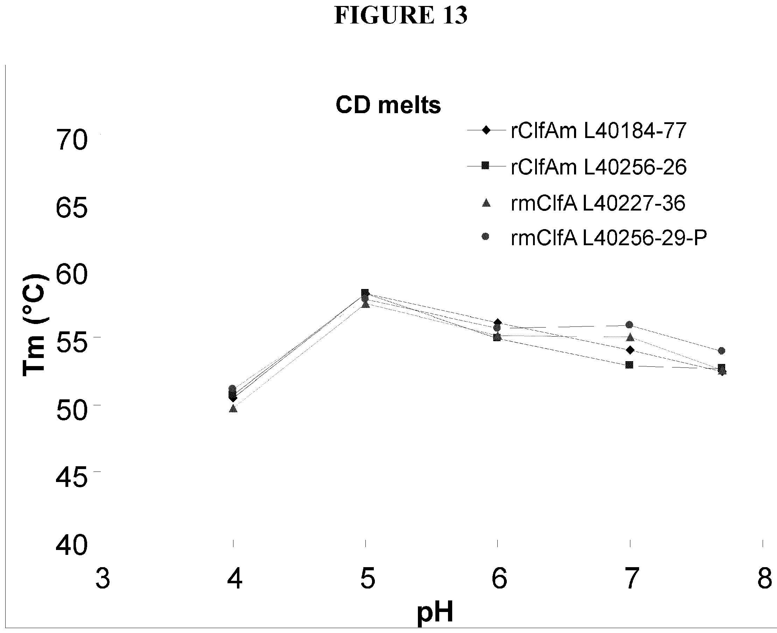

Circular dichroism (CD) was used as a tool to compare the various lots of rClfA at pH 6.0 and 7.0 (Figure 12). The CD scans at both pH showed that all tested lots of rClfA*m* and r*m*ClfA have similar secondary structures based on the overlays of the spectra. In addition, a CD melt of the proteins was performed to compare the thermostability of the lots as a function of pH. The results seen in Figure 13 indicate that all lots are comparable in the context of secondary structure. The results also suggested that pH 5.0 - 7.0 is the optimal formulation range based on this method.

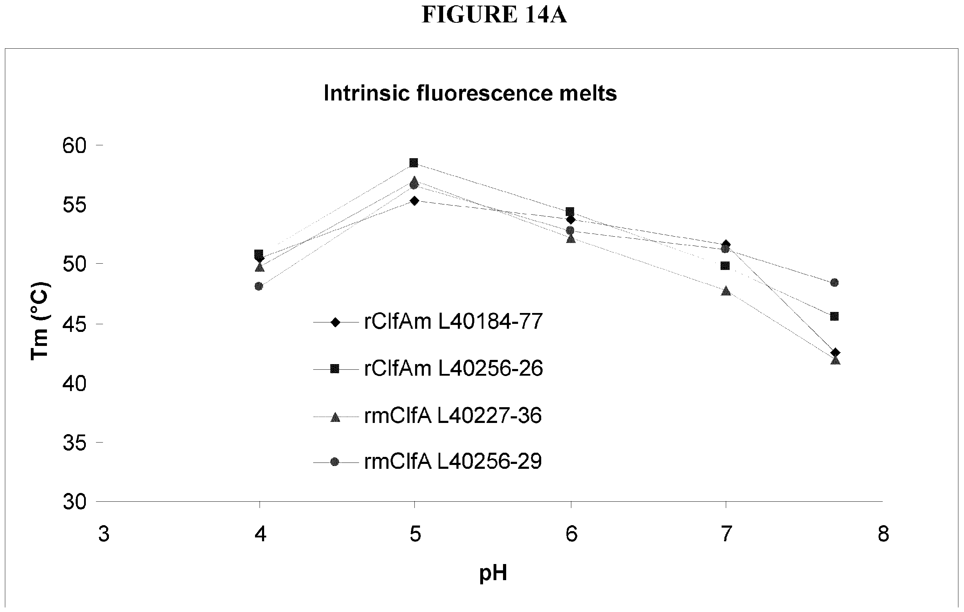

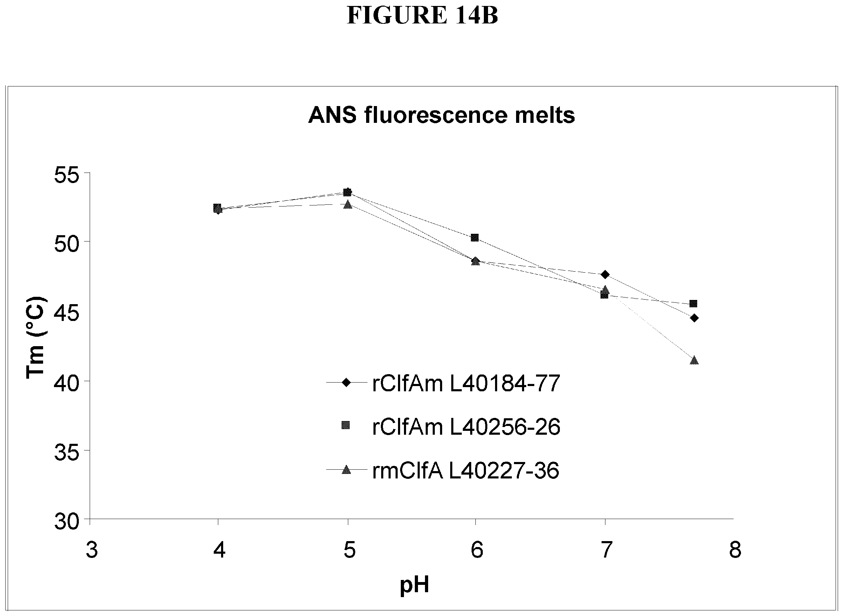

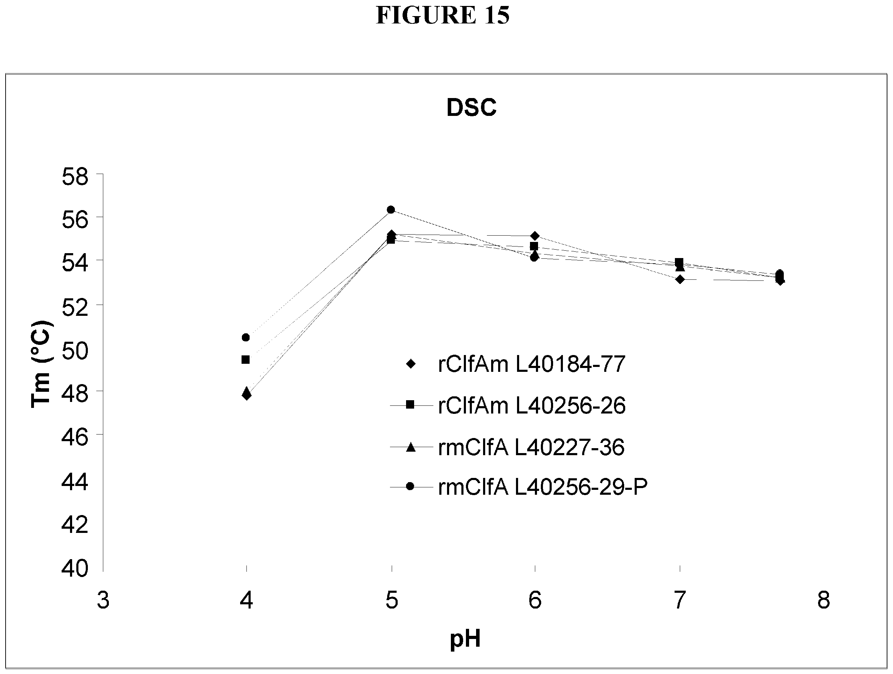

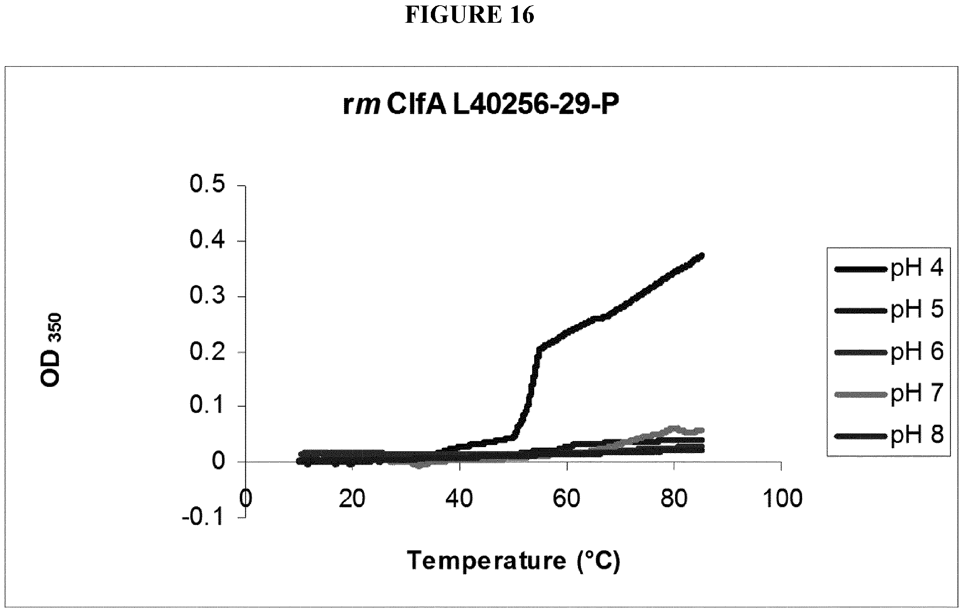

Intrinsic tryptophan fluorescence was used to obtain information about the tertiary structure of rClfA*m* and r*m*ClfA. Lots of both proteins were subjected to fluorescence melts as a function of pH to assess thermostability. It is important to note that rClfA possesses two tryptophans that are located on the same N3 domain of the protein; hence the data supplied by this method is localized to this region. Figure 14A shows that all tested lots of rClfA possessed comparable thermostabilities, inferring that the structures were also similar. Extrinsic fluorescence melts were also performed on multiple rClfA lots using a hydrophobic dye, 8-anilino-1-naphthalene sulfonate (ANS). As the protein structure unfolds with increasing temperature, ANS binds to the newly exposed hydrophobic patches of the protein, increasing in emission intensity. In Figure 14B, the melting temperatures based on ANS signal are similar as a function of pH for all tested protein lots. DSC data confirmed that the melting temperatures of all tested lots of rClfA were similar as a function of pH (Figure 15), and that pH 5-7 is a good range moving forward. OD₃₅₀ was also used to monitor aggregation as functions of temperature and pH (Figure 16). Melts of r*m*ClfA lot L40256-29 (matrix: 10 mM histidine pH 6.5) as function of pH showed that the only aggregation event detected by this method was at pH 4.0.

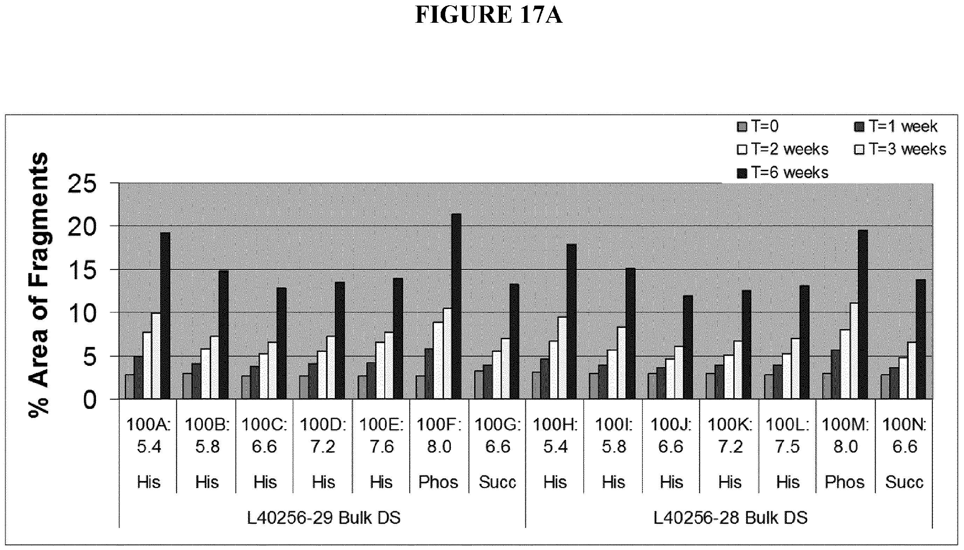

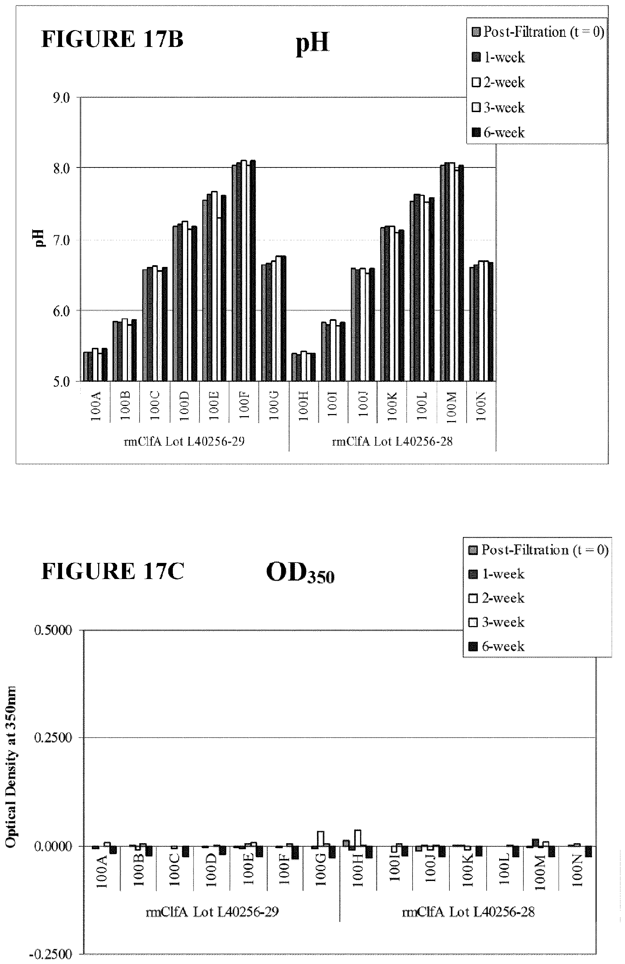

Two drug substance lots (L40256-28, L40256-29) were used to assess the liquid stability of r*m*ClfA in histidine, rather than succinate, as a function of pH. The formulations consisted of 400 µg/mL r*m*ClfA, 10 mM histidine (pH range of 5.5 - 7.5), 4.5% sucrose and 0.01% Polysorbate 80. Two additional formulations consisted of 10 mM succinate, pH 6.5 as a comparison to histidine pH 6.5, and 10 mM phosphate, pH 8.0 as an examination of high pH (both formulations also contained 4.5% sucrose and 0.01% PS80). Size exclusion-HPLC was used to monitor the cleavage of r*m*ClfA for six weeks at 25°C. The results indicated that histidine pH ∼6.5 exhibited the slowest rate of degradation in both the -28 and -29 lots (Figure 17A). This rate coincided with that of the succinate pH 6.6 formulations, indicating that histidine was as compatible with r*m*ClfA as succinate. Moreover, the pH for all formulations remained stable and no aggregation was detectable by OD₃₅₀ throughout the study (Figure 17B).

### EXAMPLE 17: TETRA-ANTIGEN DRUG PRODUCT: CHARACTERIZATION OF MNTC

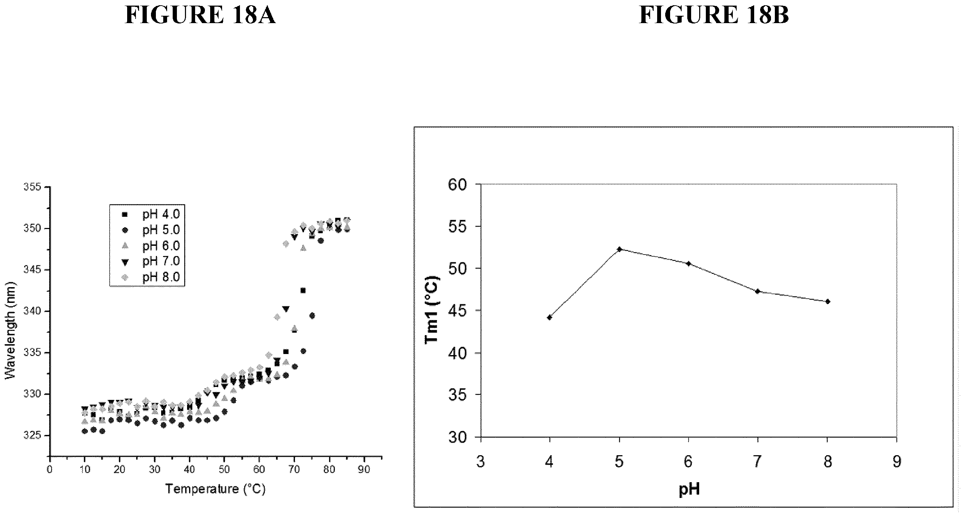

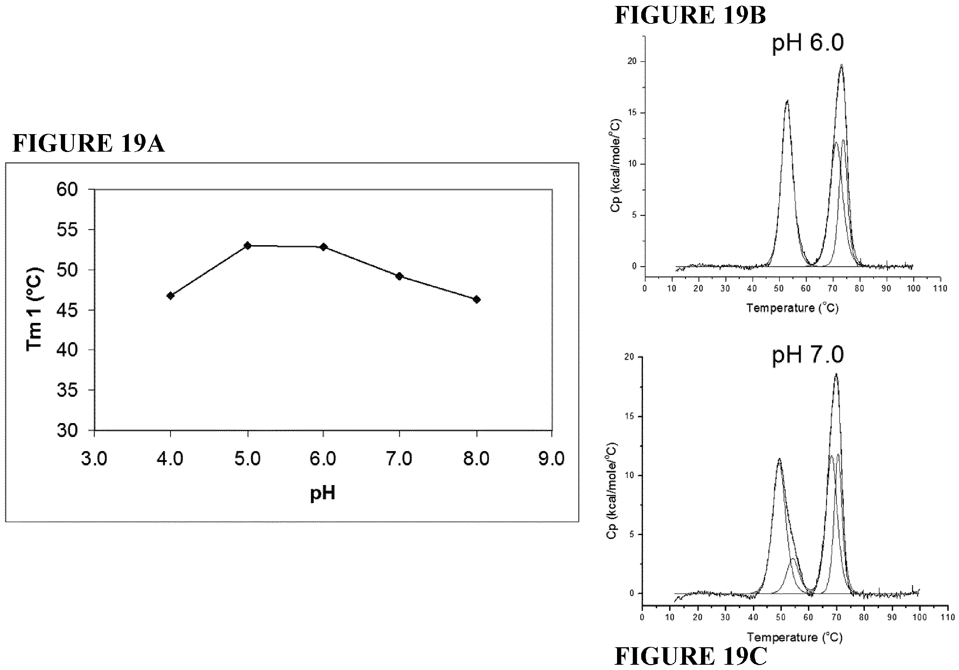

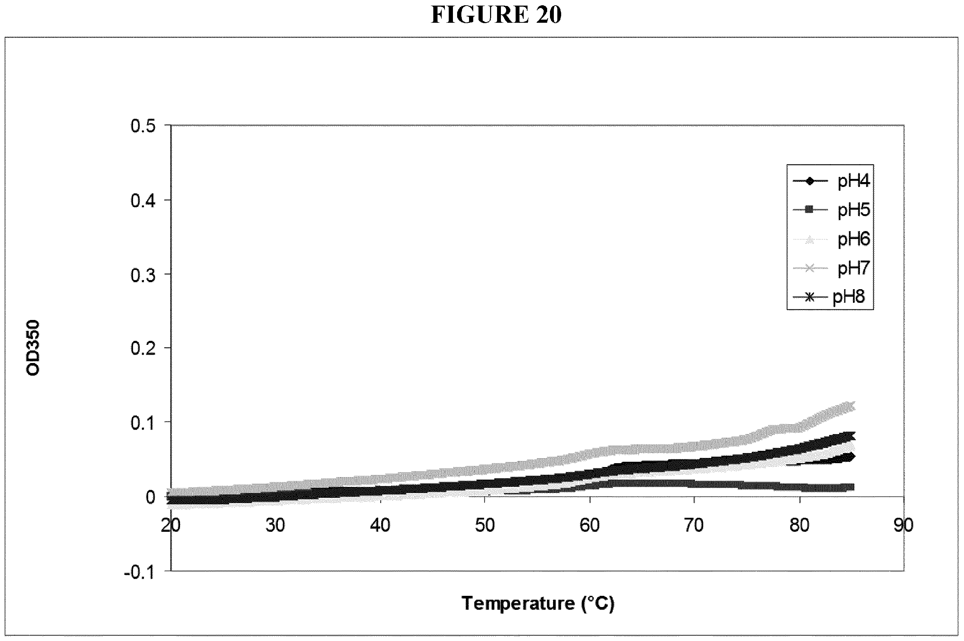

Unlike ClfA, which is prone to degradation via hydrolysis, MntC is degraded by deamidation. Thus, it was important to determine the stability of MntC over a range of pHs. Intrinsic tryptophan fluorescence experiments performed with MntC indicated that two major events were occurring, with Tₘ1 at 40 - 55°C and Tₘ2 at 65 - 75°C (Figure 18). Tracking Tₘ1, pH 5.0 and 6.0 were highest followed by pH 7.0. The data indicated a working pH range of 5.0 - 7.0. DSC with a representative lot of MntC was also performed. Representative thermograms illustrate two main thermal events, which can be further deconvoluted (Figure 19). Tracking the first melting temperature (Tₘ1), it is evident that pH 5.0 and 6.0 have the highest thermostability followed by pH 7.0 (Figure 19). In agreement with the fluorescence results, these data suggest a working pH range of 5.0 - 7.0. OD₃₅₀ data support these observations as well (Figure 20). Analysis of deamidation by IEX-HPLC confirmed that high pH and high temperature increased the rate of deamidation of MntC.

### EXAMPLE 18: TETRA-ANTIGEN DRUG PRODUCT: FORMULATION DEVELOPMENT (BUFFER)

In tri-antigen formulations described above, the CP5 and CP8 conjugate drug substances were provided in succinate at pH 7.0. The succinate buffer system, however, did not offer good buffering capacity at lower pHs. Experiments were therefore performed with CP5 and CP8 conjugates reformulated in 10 mM histidine at pH 6.7. Because conjugate drug substances do not filter well at pH<6.5, resulting in slowed filtration and a loss of the conjugated protein, a higher pH than 6.5 was preferred. This became the driving force to evaluate r*m*ClfA, MntC, and drug product formulations in histidine for uniformity.

From the pre-formulation characterization studies, it was determined that a working range of pH for both r*m*ClfA and MntC was 5-7. The change of the conjugate DS matrix to histidine necessitated the evaluation of this buffer system in the drug product. The degradation rates of the antigens were evaluated as a tetra-antigen formulation as a function of time to establish optimal formulation pH. Agitation experiments were conducted to determine the need for a surfactant in the formulation. Looking forward to lyophilization, stabilizers and bulking agents were evaluated for compatibility with the antigens.

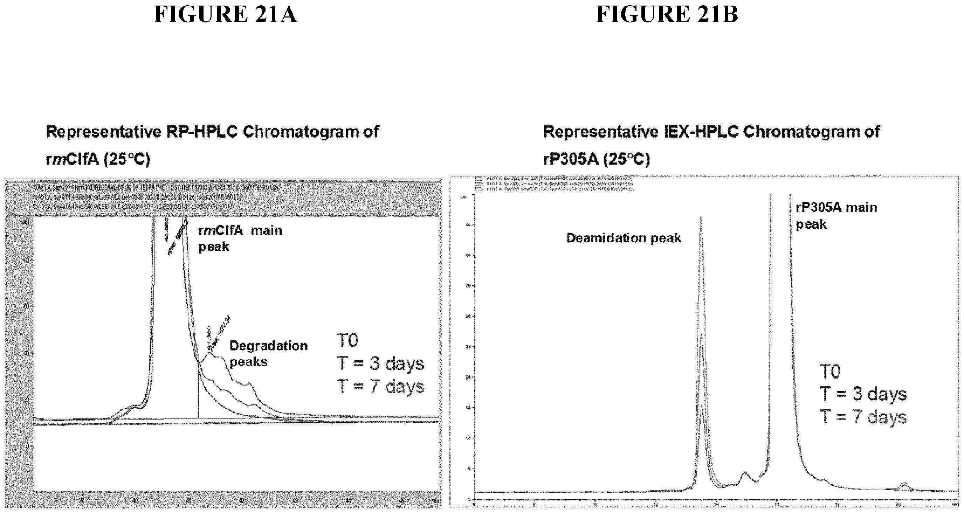

High dose tetra-antigen formulations consisting of 400/400/200/200 µg/mL of r*m*ClfA/MntC/CP5-CRM₁₉₇/CP8-CRM₁₉₇ were prepared as a function of pH in histidine buffer. RP-HPLC was used to detect degradation of the r*m*ClfA protein, and IEX-HPLC was used to detect deamidation of MntC. Figure 21 illustrates representative chromatograms of the two proteins and how they were monitored for the degraded species. For r*m*ClfA, the cleaved protein fragments were manifested to right of the main protein peak, whereas for MntC, the deamidated peak can be found to the left of the main peak. Both proteins are shown in Figure 21 as degrading over time at 25°C.

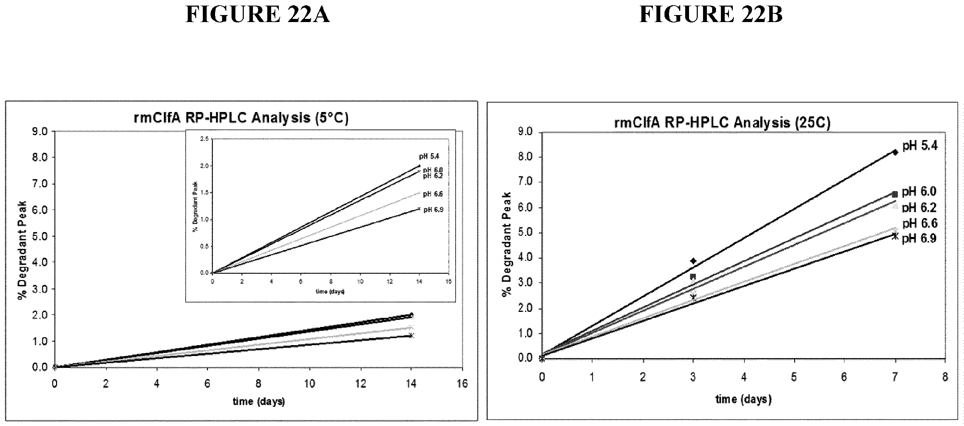

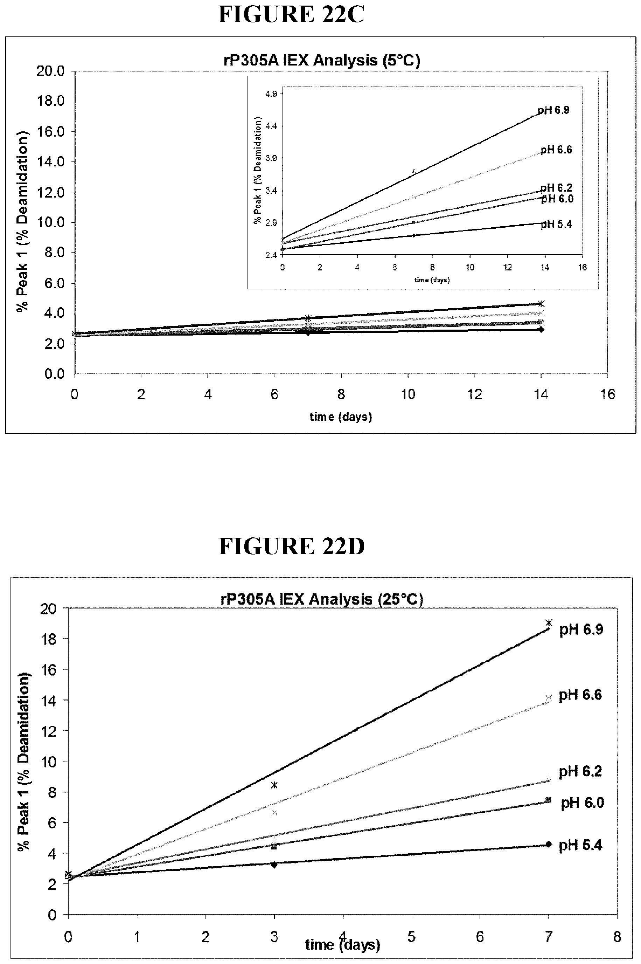

The stabilities of the formulations were monitored for 0, 1 and 2 weeks 2-8°C and for 0, 3 and 7 days at 25°C. At 25°C, r*m*ClfA showed that increasing pH decreased the rate of cleavage of the protein (Figures 22A and B). Conversely, increasing pH decreased the rate of MntC deamidation (Figures 22C and D). Both mechanisms are significantly suppressed at 2-8°C, though the pH-dependent trend remained consistent.

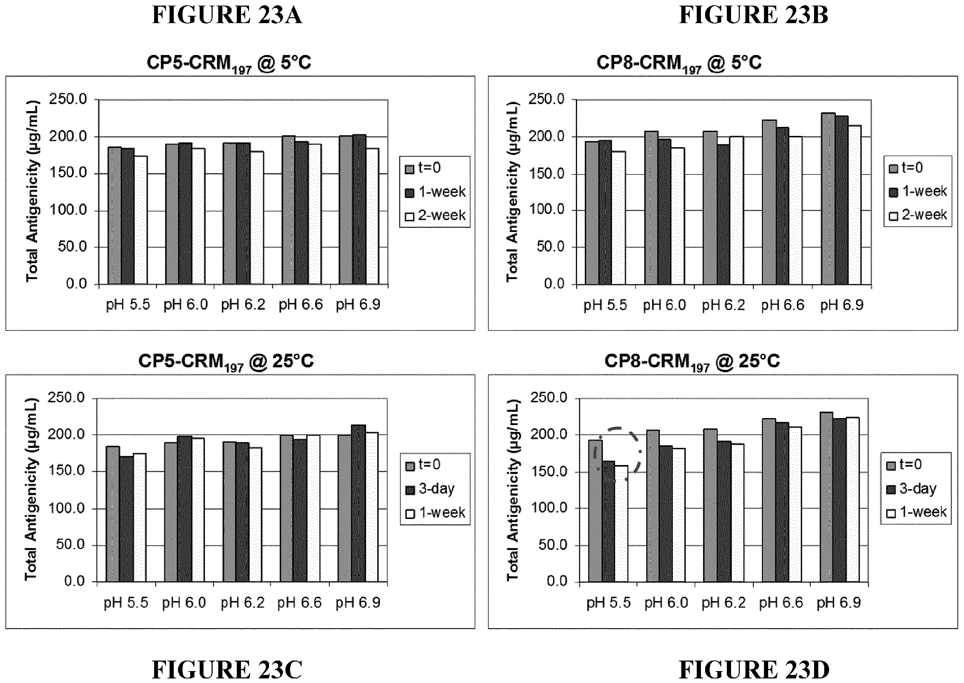

The antigenicities of CP5- and CP8-CRM₁₉₇ conjugates were monitored at different pHs at 2-8°C and 25°C using nephelometry. At 2-8°C, both conjugates maintained antigenicity for the 2-week duration for the experiment (Figures 23A and B). At 25°C, CP5-CRM₁₉₇ showed no change after one week, however, CP8-CRM₁₉₇ showed approximately an 18% decrease in antigenicity at pH 5.5 (Figures 23C and D).

### EXAMPLE 19: TETRA-ANTIGEN DRUG PRODUCT: FORMULATION DEVELOPMENT (BULKING AGENT)

Formulations comprising 400/400/200/200 µg/mL of r*m*ClfA/MntC/CP5-CRM₁₉₇/CP8-CRM₁₉₇ and containing 1) 4.5% sucrose, 2) 4.5% trehalose, 3) 3% mannitol/1% sucrose, 4) 3% mannitol/1% trehalose, 5) 3% glycine/1% trehalose, 6) 3% glycine/1% trehalose and 7) 4.5% sucrose, no PS80, were tested for stability. All formulations contained 10 mM histidine, and 0.01% Polysorbate 80 (with the exception of formulation 7) with the measured pH of the formulations at 6.3. Aliquots of the formulations were transferred to Schott type 1 2 mL vials with Flurotec-coated 4432/50 stoppers and the vials were gently rocked for 6 days at room temperature.

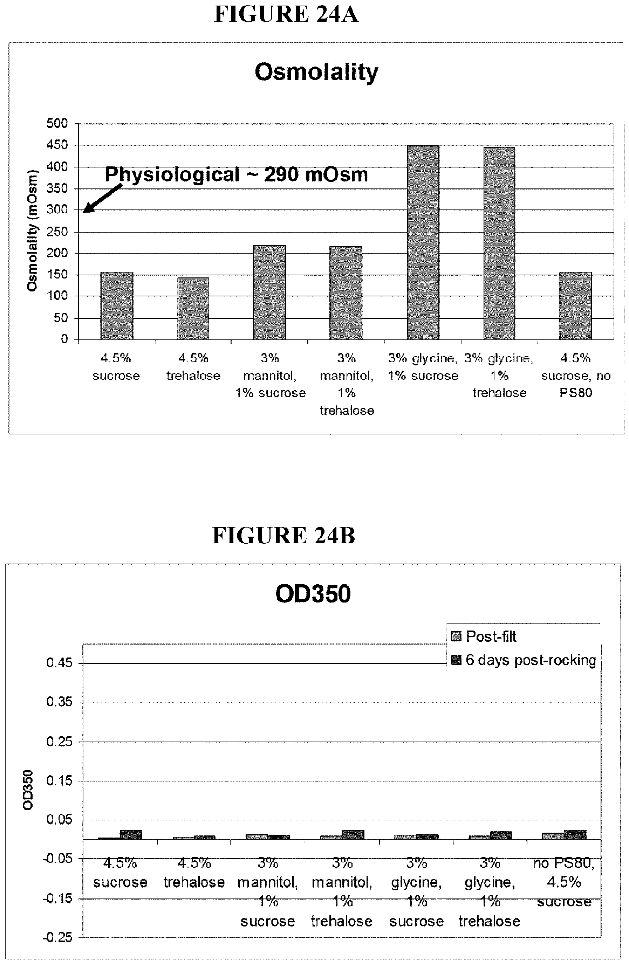

OD₃₅₀ measurements of all post-rocked samples showed no increase in aggregated species (Figure 24B). Osmolality measurements revealed that glycine-containing formulations had values that exceeded a physiological osmolality of 290 mOsm (Figure 24A). Recoveries were >98% for ClfA and MntC, >95% for CP5-CRM₁₉₇, and >93% for CP8-CRM₁₉₇, which were all well within assay variabilities. IEX-HPLC analysis to monitor deamidation of MntC showed increase after 6 days of rocking, but all formulations showed similar rates, indicating that no particular excipient or excipient combination improved nor accelerated the process. Finally, RP-HPLC analysis of MntC and r*m*ClfA showed no significant changes in the chromatogram profiles.

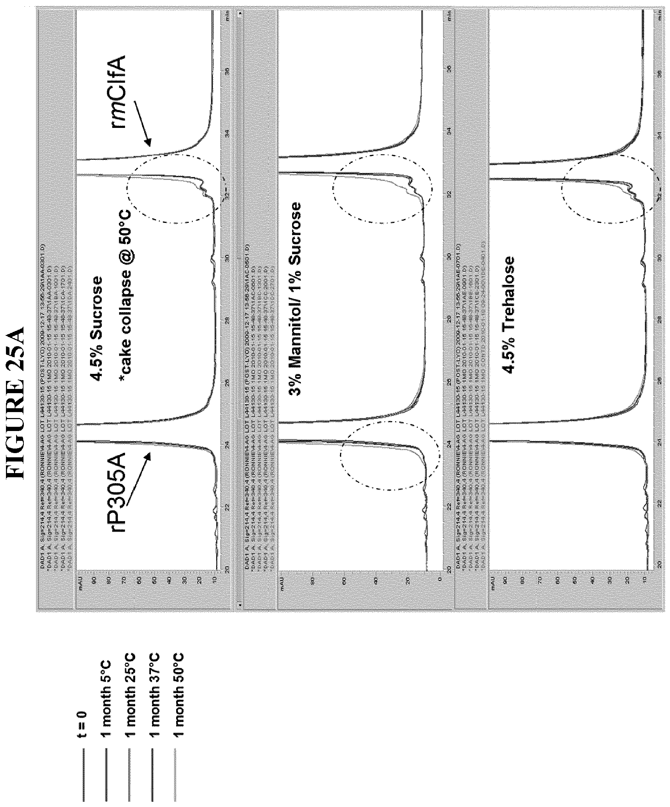

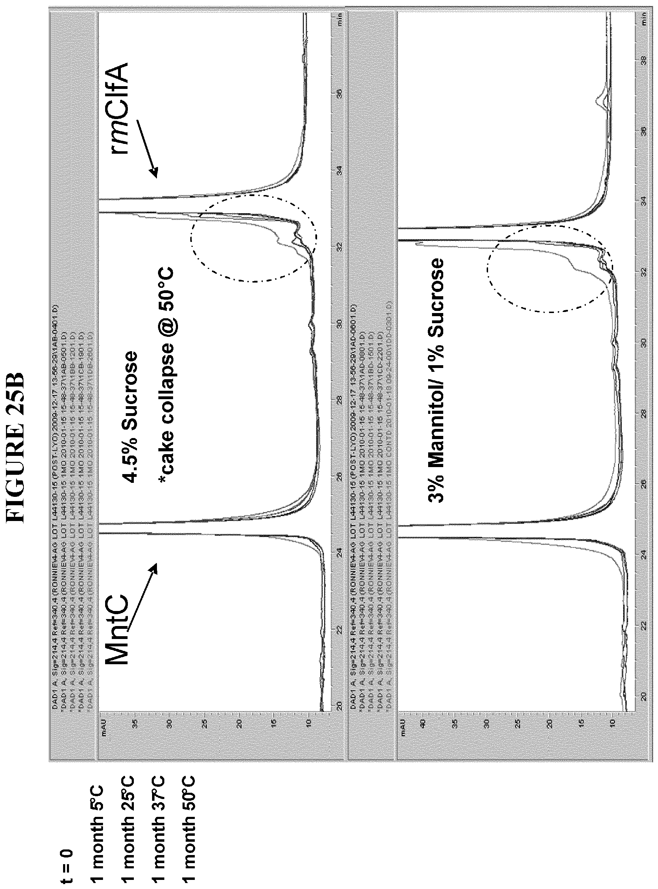

The glycine-containing samples were eliminated from the list of excipient candidates due to high osmolality. Sucrose, trehalose, mannitol and glycine were evaluated as lyophilized products as described by the formulations in Table 27. The formulations were lyophilized and each was tested for stability at 2-8°C, 25°C, 37°C and 50°C. RP-HPLC data for high doses in Figure 25A showed no change after one month at 2-8°C, 25°C and 37°C. At 50°C, r*m*ClfA showed an increase in the front shoulder peak, with the mannitol/sucrose formation exhibiting the largest change. In addition, the front shoulder peak of MntC increased in the mannitol/sucrose formulation as well. For low dose, a change was seen in both r*m*ClfA front shoulder peaks, but with the mannitol/sucrose formulation again demonstrating a larger increase (Figure 25B).

**Table 27: Formulations**

| | **r*m*ClfA (µg/mL)** | **MntC (µg/mL)** | **CP5-CRM₁₉₇ (µg/mL)** | **CP8-CRM₁₉₇ (µg/mL)** | **Excipient** |
|---|---|---|---|---|---|
| L44130-15-1 (High) | 400 | 400 | 200 | 200 | 4.5% sucrose |
| L44130-15-2 (Low) | 40 | 40 | 20 | 20 | 4.5% sucrose |
| L44130-15-3 (High) | 400 | 400 | 200 | 200 | 3% mannitol/1% sucrose |
| L44130-15-4 (Low) | 40 | 40 | 20 | 20 | 3% mannitol/1% sucrose |
| L44130-15-5 (High) | 400 | 400 | 200 | 200 | 4.5% trehalose |

| | | | | | |
|---|---|---|---|---|---|
| Constant parameters: 10 mM histidine, 0.01% PS80, measure pH ∼ 6.3 | | | | | |

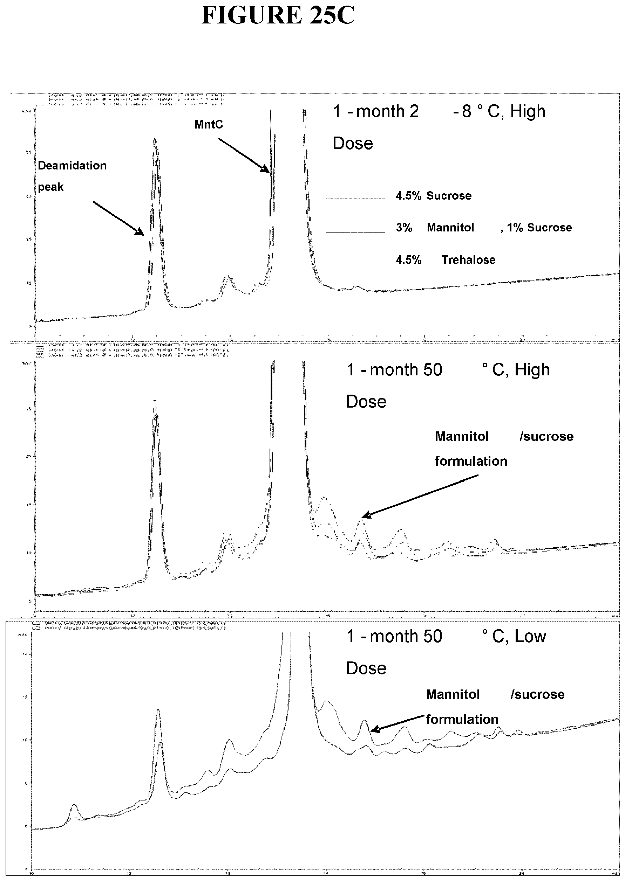

IEX-HPLC was also used to monitor the quality of MntC. In Figure 25C, the 2-8°C stability samples for high dose show no change in any of the formulations after one month. Samples held at 25 and 37°C produced similar results (not pictured). At 50°C, however, both high and low doses showed the largest changes in profile for the mannitol/sucrose combination. Nephelometry results for CP5 and CP8 conjugates did not show any changes in antigenicities at any temperature after one month.

Because sucrose was the lead excipient candidate are these experiments, drug product matrices were formulated with 10 mM histidine, pH 6.5 and 0.01% PS80 held constant, and varying sucrose concentrations over a range of 2.0 - 7.0%, and lyophilized. Key readouts were drying time in the primary drying step, appearance, and moisture. Test results indicated that the sucrose concentration range of 3 - 6% used in tri-antigen formulations was also acceptable for a tetra-antigen formulation.

### EXAMPLE 20: TETRA-ANTIGEN DRUG PRODUCT: FORMULATION DEVELOPMENT (POLYSORBATE 80)

An agitation experiment was designed 1) to determine the need for Polysorbate 80, and 2) to verify the robustness of the formulation. As described in Table 28, monovalent and SA4ag formulations were evaluated with 0, 0.01, and 0.03% PS80. The samples were aliquoted into Schott type 1 2 mL vials with flurotec-coated 4432/50 stoppers, and were agitated for 24 hours at RT with a VWR vortexer (Model DVX-2500, 500 RPM pulse mode). Non-agitated controls were placed at room temperature for 24 hours in parallel with the agitated samples. Multiple vials were pooled for each formulation and analyzed.

**Table 28: Formulations for Agitation Experiment**

| | **Polysorbate 80** | **Buffer matrix** | **Concentrations of Actives** |
|---|---|---|---|
| r*m*ClfA monovalent | 0, 0.01, 0.03% | 10 mM histidine, pH 6.5, 4.5% sucrose | 400 µg/mL |
| MntC monovalent | 0, 0.01, 0.03% | 10 mM histidine, pH 6.5, 4.5% sucrose | 400 µg/mL |
| CP5-CRM197 monovalent | 0, 0.01, 0.03% | 10 mM histidine, pH 6.5, 4.5% sucrose | 400 µg/mL |
| CP8-CRM monovalent | 0, 0.01, 0.03% | 10 mM histidine, pH 6.5, 4.5% sucrose | 400 µg/mL |
| Tetra-antigen | 0, 0.01, 0.03% | 10 mM histidine, pH 6.5, 4.5% sucrose | 400/400/200/200 µg/mL of r*m*ClfA/MntC/CP5-CRM₁₉₇/CP8-CRM₁₉₇ |

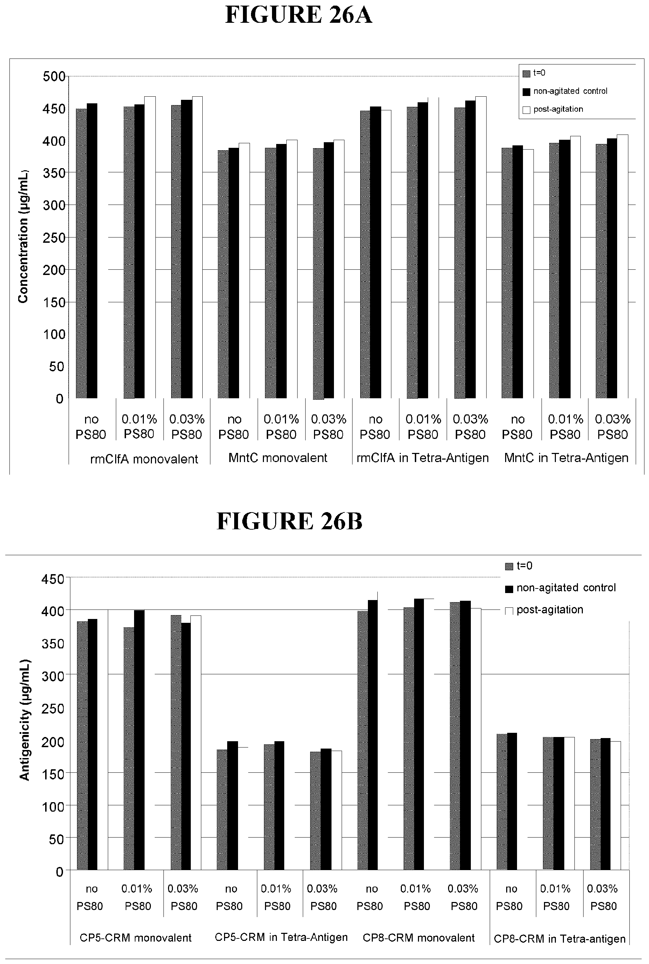

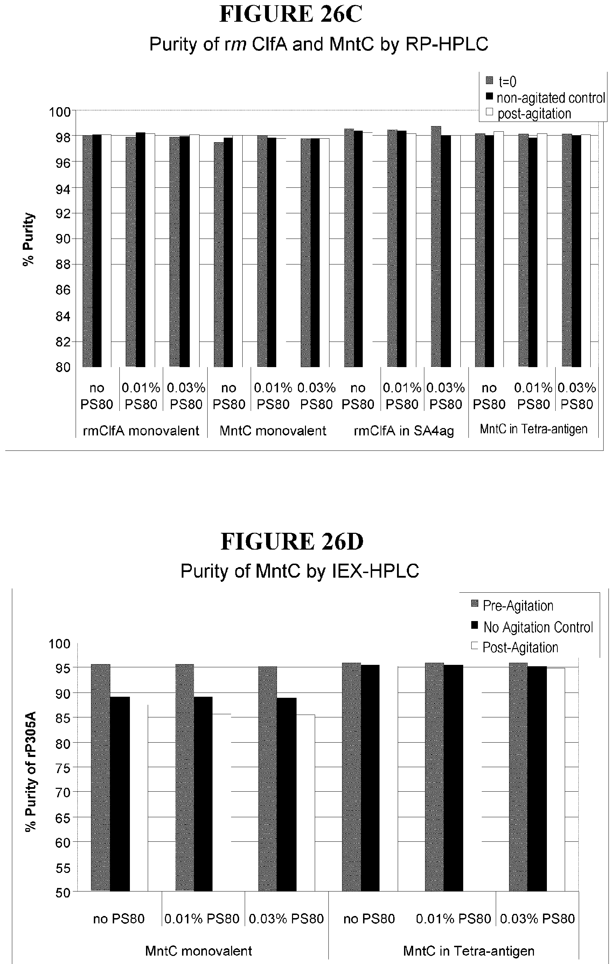

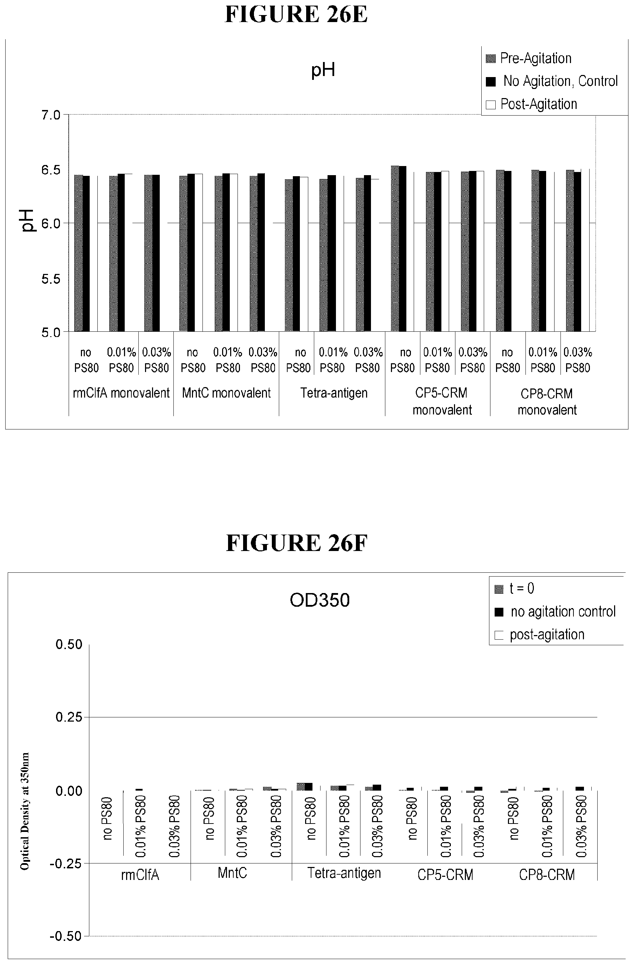

Concentrations of the antigens were examined for recovery after agitation. Post-agitation data of all four antigens indicated no change in concentrations, demonstrating no loss in any of the samples, including the formulations containing no PS80 (Figures 26A and B). Purity analysis of r*m*ClfA and MntC using RP-HPLC revealed no change in either protein after agitation (Figure 26C). Purity of MntC using IEX-HPLC showed similar decrease (due to deamidation) in the monovalent formulations when comparing the levels of PS80 (Figure 26D). One interesting observation from the IEX-HPLC purity data of MntC is that, as a monovalent formulation, the rate of degradation (corresponding to deamidation) is significant compared the MntC in the tetra-antigen formulation. Given that all formulations shared the same buffer matrix, it is possible that MntC interacts with another antigen which slows the deamidation rate of the protein. pH values of the formulations also remained constant (Figure 26E), and OD₃₅₀ measurements revealed no increases in aggregation after agitation (Figure 26F).

### EXAMPLE 21: TETRA-ANTIGEN DRUG PRODUCT: LYOPHILIZATION

A TA Instruments Modulated Differential Scanning Calorimeter (mDSC) was used to obtain glass transitions for the pre-lyophilized liquid formulations and the T_{g}' values are reported in Table 29. Lots L44130-39-1 and 39-2 represent high and low dose target formulations containing 4.5% sucrose. Lot L44130-45 samples bracket the target sucrose concentration and contain 3% or 6% of the excipient. These samples contain ClfA/CP5-CRM₁₉₇/CP8-CRM₁₉₇/MntC concentrations bracketing the L44130-39-1 High and Low doses (+30% and - 30%). All formulations gave T_{g}' values of -33 to -35°C which is typical of sucrose as reported in literature.

**Table 29: T_{g}' Measurements of Formulations**

| **Formulation** | **T_{g}' (°C)** |
|---|---|
| L44130-39-1 High Dose | -34.5 |
| L44130-39-1 Low Dose | -34.5 |
| L44130-45-1 | -33.8 |
| L44130-45-2 | -35.5 |
| L44130-45-3 | -34.7 |
| L44130-45-4 | -34.4 |

The T_{g} values of lyophilized samples are reported in Table 30. The samples were opened, transferred, and sealed in aluminum hermetic DSC pans in a dry glove box to prevent the lyophilized cakes from accruing moisture from the atmosphere. The measured values were in the range of 65 - 70 °C which is typical of a sucrose formulation.

**Table 30: T_{g} Measurements of Lyophilized Formulations**

| | **T_{g} (°C)** |
|---|---|
| L44130-39-1 High Dose | 65.6 |
| L44130-39-1 Low Dose | 67.1 |
| L44130-45-1 | 69.0 |
| L44130-45-2 | 65.6 |
| L44130-45-3 | 69.2 |
| L44130-45-4 | 69.5 |

The lyophilization cycle was based on the cycle used for the SA3ag drug product, as the two formulations were similar, with both using sucrose as the bulking agent. Table 31 defines ranges for lyophilization cycles used for production of tetra-antigen formulations.

**Table 31: Lyophilization Cycle Parameters**

| | **Shelf Temp. (°C)** | **Rate / Hold** | **Time (min)** | **Pressure (mTorr)** |
|---|---|---|---|---|
| Freezing | -50 | 0.3°C/min | ∼200 | atmospheric |
| | -50 | Hold | 60 - 120 | |
| | -10 | 0.3° C/min | ∼140 | |
| Annealing | -10 | Hold | 120 - 300 | |
| | -50 | 0.3 °C/min | ∼140 | |
| | -50 | Hold | ∼180 | |
| Primary drying | -50 | Hold | ∼30 | 50 |
| | -30 | 0.2°C/mm | ∼100 | 50 |
| | -30 | Hold | 1900 - 2600 | 50 |
| Secondary drying | 30 | 0.2°C/mm | ∼300 | 50 |
| | 30 | Hold | 700 - 900 | 200 |
| | 5 | 0.5°C/mm | ∼50 | 200 |
| | 5 | Hold | Until Stoppered | 200 |

Lyophilization studies were performed comparing the following parameters:
1. 4.5% sucrose vs. 4% mannitol/1% sucrose, and
2. conservative lyophilization cycle vs. faster, aggressive cycle.
Samples were evaluated both as monovalent drug product formulations as well as a tetra-antigen formulation. All formulations included 10 mM histidine, pH 6.5, and 0.01% PS80. Doses were 400 µg/mL for r*m*ClfA and MntC, and 200 µg/mL for CP5- and CP8-CRM₁₉₇ conjugates. Pre- and post-lyophilized formulations were compared using the following assays: appearance, pH, CEX-HPLC for MntC deamidation, RP-HPLC for MntC and r*m*ClfA purity, nephelometry for conjugate recovery, moisture, OD₃₅₀ for detection of precipitates, and differential scanning calorimetry (DSC) for structural evaluation. The cycles used are described in Table 32 and Table 33.

**Table 32: Lyophilization cycle parameters (Slow Cycle)**

| | **Shelf Temp. (°C)** | **Rate / Hold** | **Time (min)** | **Pressure (mTorr)** |
|---|---|---|---|---|
| Freezing | -50 | 0.3° C/min | 183.3 | atmospheric |
| | -50 | Hold | 60 | |
| | -10 | 0.3°C/min | 133.3 | |
| Annealing | -10 | Hold | 120 | |
| | -50 | 0.3° C/min | 133.3 | |
| | -50 | Hold | 180 | |
| Primary drying | -50 | Hold | 30 | 50 |
| | -30 | 0.2°C/min | 100 | 50 |
| | -30 | Hold | 1920 | 50 |
| Secondary drying | 30 | 0.2° C/min | 300 | 50 |
| | 30 | Hold | 720 | 200 |
| | 5 | 0.5°C/min | 50 | 200 |
| | 5 | Hold | Until Stoppered | 200 |

| | | | | |
|---|---|---|---|---|
| Total run time: 66 hours | | | | |

**Table 33: Lyophilization cycle parameters (Fast Cycle)**

| | **Shelf Temp. (°C)** | **Rate / Hold** | **Time (min)** | **Pressure (mTorr)** |
|---|---|---|---|---|
| Freezing | -50 | 1°C/min | 55 | atmospheric |
| | -50 | Hold | 60 | |
| | -10 | 1°C/min | 40 | |
| Annealing | -10 | Hold | 120 | |
| | -50 | 1°C/min | 40 | |
| | -50 | Hold | 180 | |
| Primary drying | -50 | Hold | 30 | 50 |
| | 0 | 1°C/min | 50 | 50 |
| | 0 | Hold | 1800 | 50 |
| Secondary drying | 40 | 0.5°C/min | 80 | 50 |
| | 40 | Hold | 360 | 200 |
| | 5 | 1°C/min | 25 | 200 |
| | 5 | Hold | Until Stoppered | 200 |

| | | | | |
|---|---|---|---|---|
| Total run time: 47 hours | | | | |

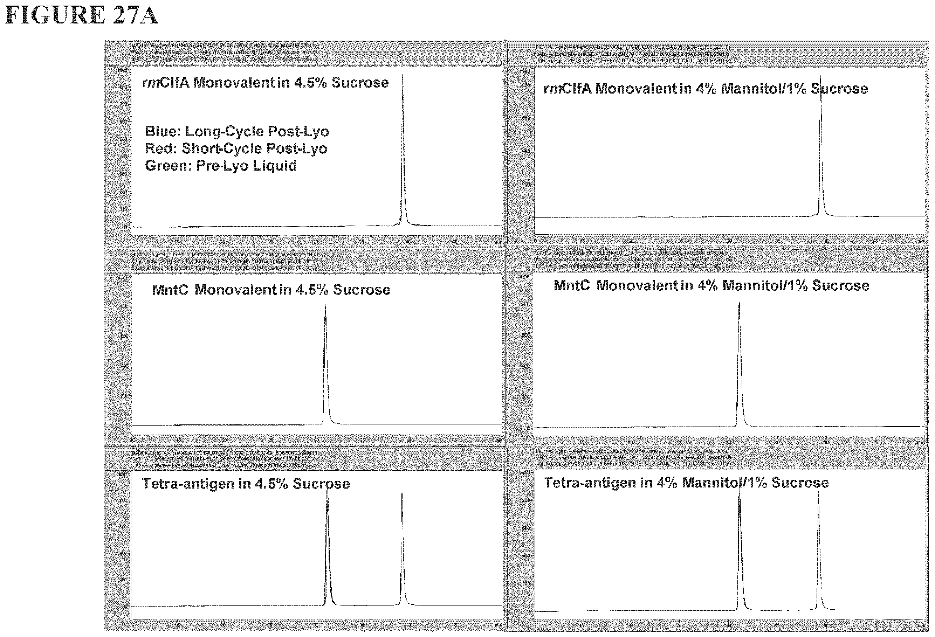

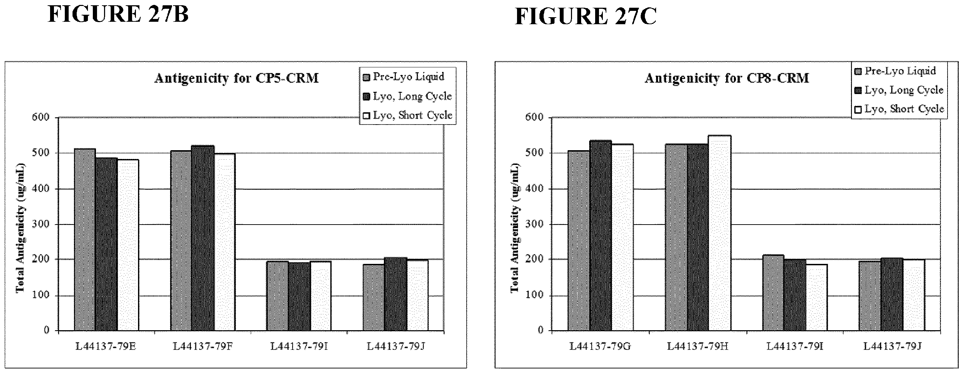

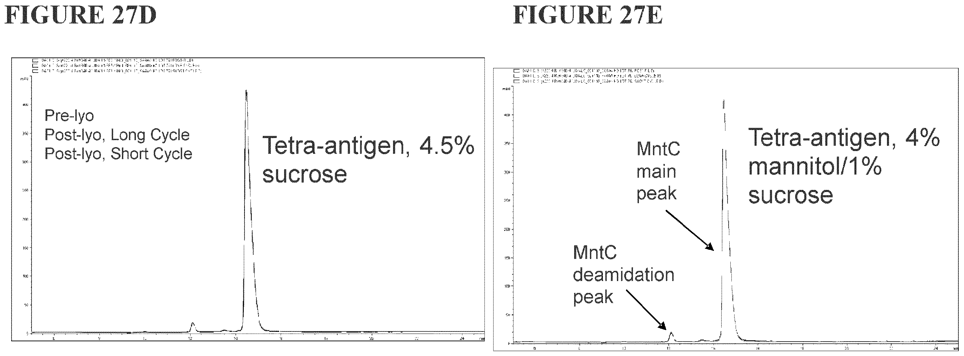

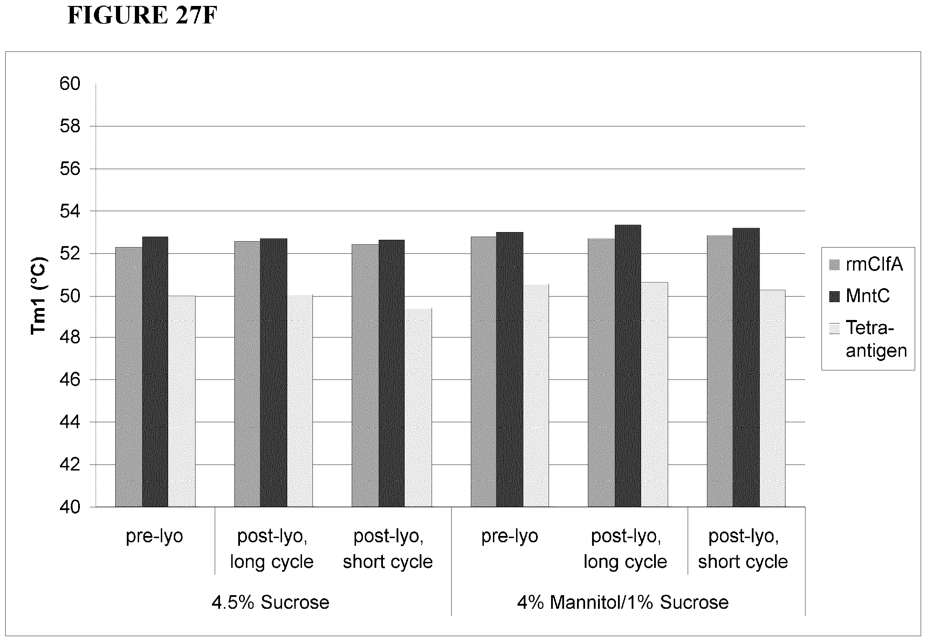

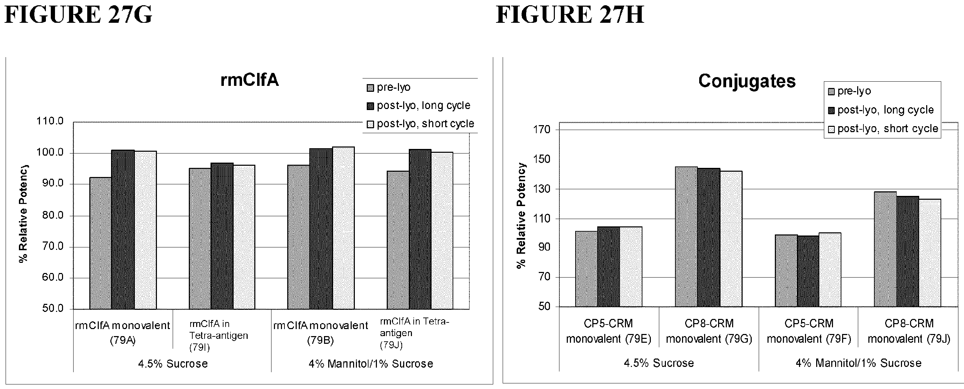

Appearance of the cakes are described in Table 34. All mannitol-containing cakes yielded visually elegant cakes with both cycles. Sucrose formulations showed acceptable cakes as expected with the long cycle, but with the short aggressive cycle, produced partially collapsed cakes. Cake moisture content data revealed that all formulations were < 1%, however, mannitol-containing samples generally had lower moisture than sucrose alone. RP-HPLC overlays in Figure 27A showed no change in r*m*ClfA and MntC purities pre- and post-lyophilization, and nephelometry results also revealed no change in antigenicities for CP5-CRM197 (Figure 27B) and CP8-CRM197 (Figure 27C) after lyophilization regardless of cycle or excipient. Figures 27D and E demonstrate that the deamidated species of MntC is maintained at ∼2.5% post-lyophilization for the tetra-antigen formulation. pH remained the same after lyophilization, and no notable increase in OD₃₅₀ was detected in any post-lyophilization samples, indicating the absence of precipitates. It is important to note, however, that OD₃₅₀ will not detect the generation of small soluble aggregates. DSC data indicated that no structural perturbations in the antigens (r*m*ClfA and MntC) were promoted as a result of either cycle, as evidenced by the lack of change in melting temperatures (Figure 27F). Conjugates were not monitored as they do not produce a strong melting signal. Finally, % *in vitro* relative potencies of the antigens were compared pre- and post-lyophilization using a Luminex assay. r*m*ClfA was assayed both monovalently and in a tetra-antigen formulation, but the conjugates were only assayed as monovalent formulations because the tetra-antigen combination produced interference. Results showed that all analyzed samples (mannitol and sucrose) were well within assay error when comparing pre- and post-lyophilization (both slow and fast cycles), and that r*m*ClfA (Figure 27G) and conjugates (Figure 27H) were stable under those conditions.

**Table 34: Appearance of Cakes**

| Lot Number | Antigens | Long Cycle | Short Cycle |
|---|---|---|---|
| **Lyophilized Samples containing 4.5% Sucrose** | | | |
| L44137-79A L44137-79C L44137-79E L44137-79G | rmClfA MntC CP5-CRM | All samples were observed to be white freeze dried powder with evidence of slight shrinkage | Partial cake collapse observed (cake was sparse or not dense in the middle). All samples were observed to be white freeze-dried powder with evidence of slight shrinkage. |
| | CP8-CRM Tetra-antigen | | |
| L44137-79I | | | |

| **Lyophilized Samples containing 4% Mannitol, 1% Sucrose** | | | |
|---|---|---|---|
| L44137-79B L44137-79D L44137-79F L44137-79H | rmClfA | All samples were observed to be white freeze dried powder with an elegant smooth cake. | |
| | MntC CP5-CRM | | |
| | CP8-CRM Tetra-antigen | | |
| L44137-79J | | | |

### EXAMPLE 22: TETRA-ANTIGEN DRUG PRODUCT: RECONSTITUTION

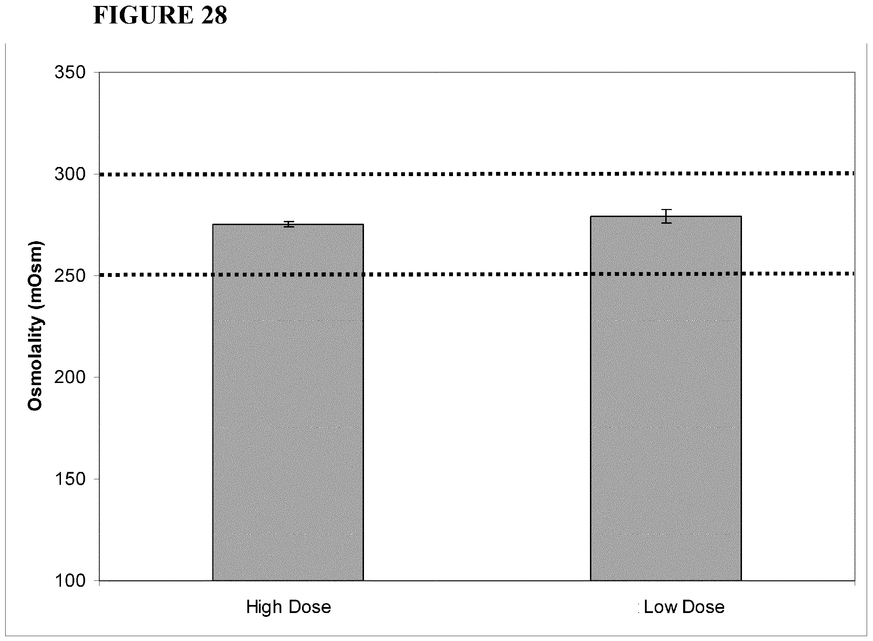

The reconstitution diluent was chosen to be 60 mM NaCl in water for injection (WFI). Figure 28 demonstrates that when reconstituted with this diluent, both high (400/400/200/200 µg/mL r*m*ClfA/MntC/CP5-CRM₁₉₇/CP8-CRM₁₉₇) and low dose (200/200/100/100 µg/mL r*m*ClfA/MntC/CP5-CRM₁₉₇/CP8-CRM₁₉₇) show osmolality in the physiological range. The reconstitution volume chosen was 0.7 mL, resulting in a final volume of 0.73 mL.

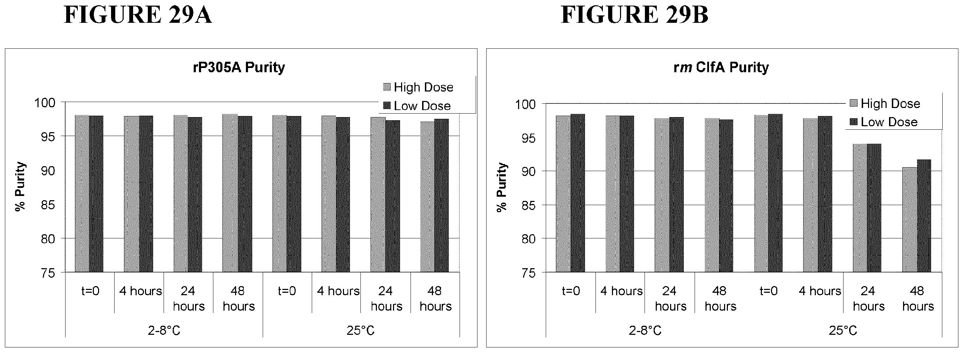

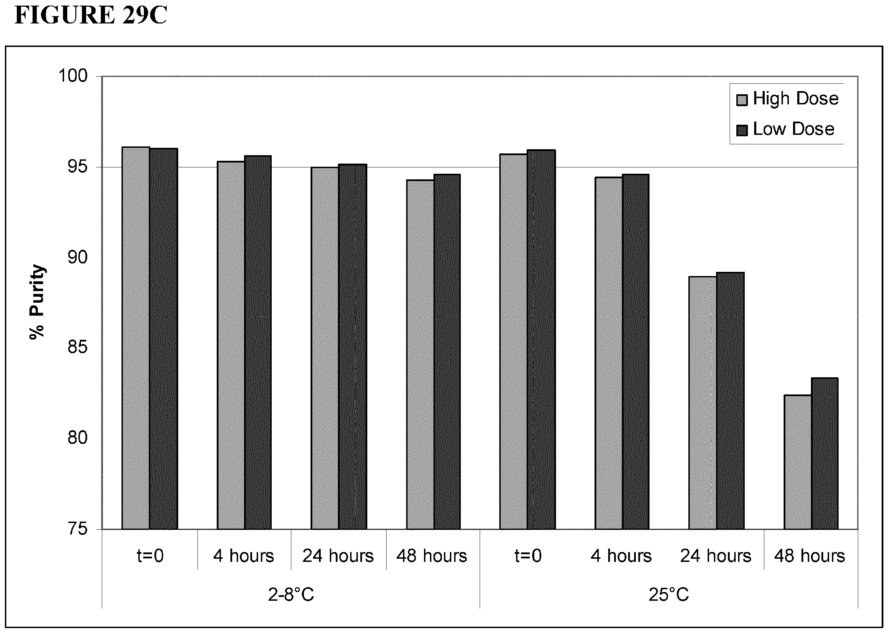

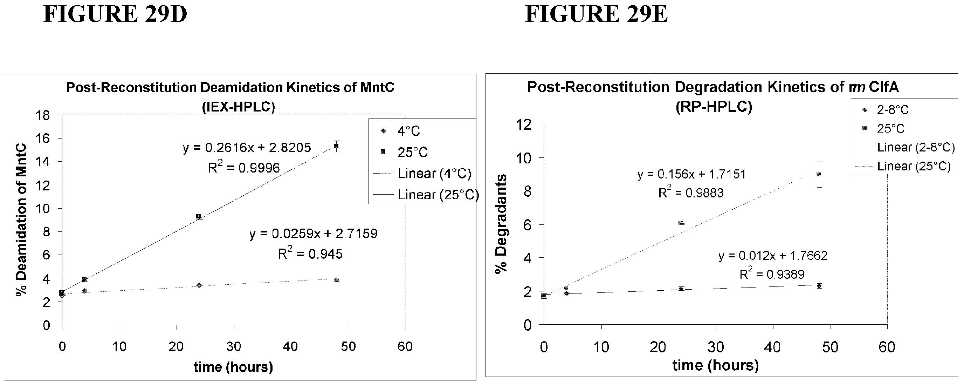

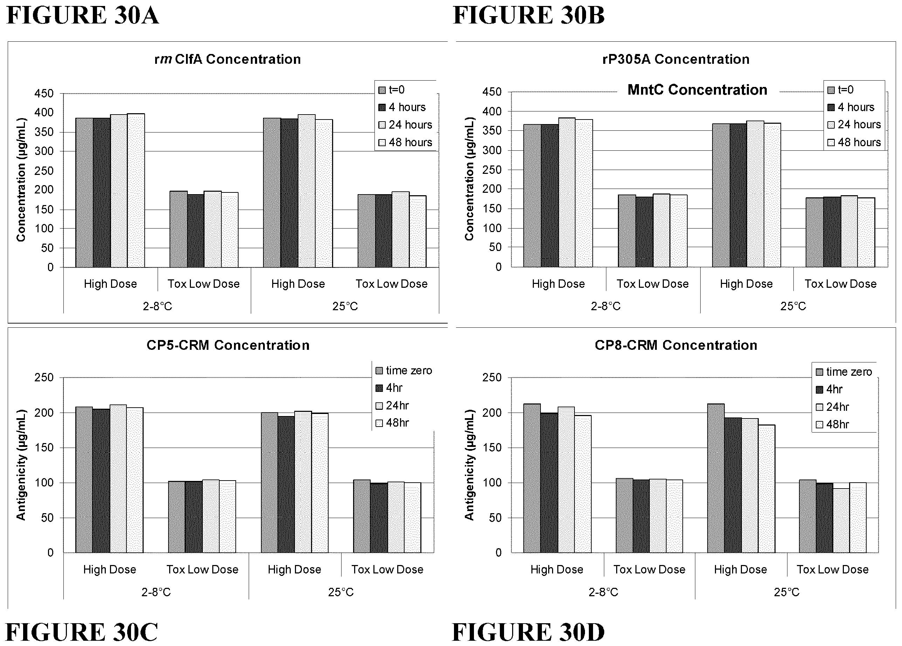

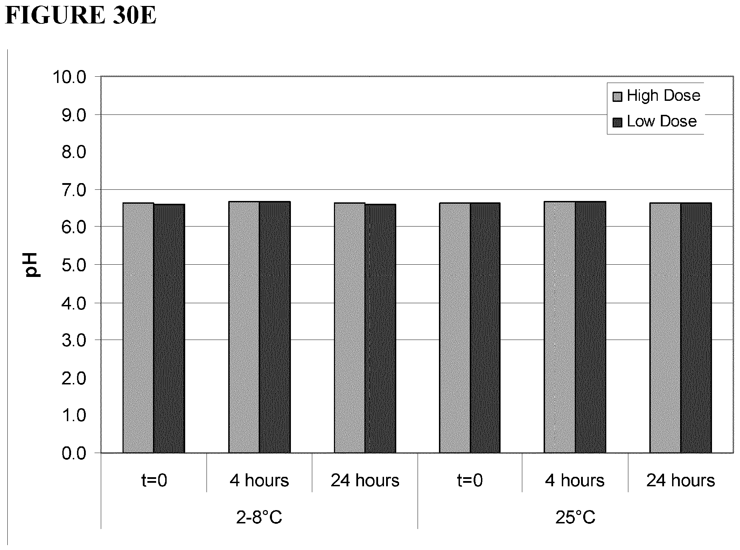

After reconstituting with 60 mM NaCl, high and low dose tetra-antigen vaccines were held for stability studies at 2-8°C and 25°C for 48 hours. Data in Figure 29A show that purity of MntC as detected by RP-HPLC remained unchanged for 48 hours at 2-8°C and 25°C. r*m*ClfA was stable for 48 hours at 2-8°C, but at 25°C, purity was decreased at the 24-hour time point (Figure 29B). IEX-HPLC was used to monitor purity of MntC, for which a decrease signified an increase in deamidation (Figure 29C). The protein remained stable at 2-8°C with a nominal purity decrease of <2%. At 25°C, MntC remained stable for 4 hours, but significantly decreased in purity (below 90%) after 24 hours. Kinetic analysis of the data showed the following: 0.03 % and 0.26 % degradation per hour at 2-8°C and 25°C respectively for MntC (Figure 29D), and 0.01 % and 0.16 % degradation per hour at 2-8°C and 25°C, respectively, for r*m*ClfA (Figure 29E). Additional data showed that the concentrations of the antigens did not change at either temperature after 48 hours (Figures 30A-D). pH also remained the same for 48 hours (Figure 30E).

The vaccine following reconstitution was stable for four hours at room temperature and up to twenty four hours at 2-8°C based on analysis of the post-reconstituted vaccine for all four antigens.

### EXAMPLE 23: TETRA-ANTIGEN DRUG PRODUCT: POST-LYOPHILIZATION RECOVERY

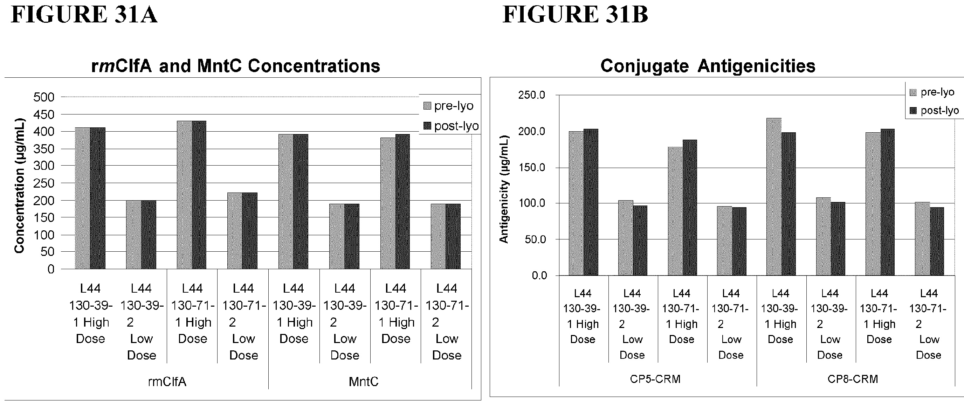

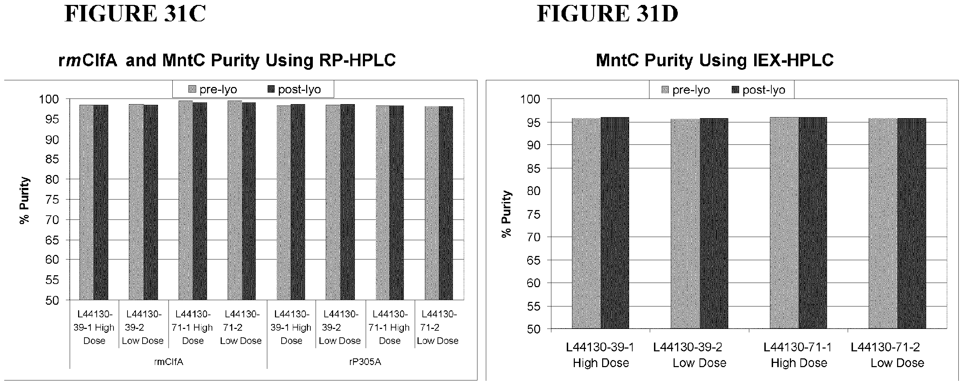

Post-lyophilization process recovery of tetra-antigen formulations were evaluated with two sets of studies (two high and two low doses), where the process consisted of formulation, filtration, filling, and lyophilization. Figures 31A and B shows that there was no loss of any antigen after lyophilization. Likewise, Figures 31C and D shows that there was no change in the purity of r*m*ClfA or MntC in the post-lyophilized samples.

### EXAMPLE 24: TETRA-ANTIGEN DRUG PRODUCT: FORMULATION STABILITY

Tetra-antigen formulations with:
1) 400/400/200/200 µg/mL of r*m*ClfA/MntC/CP5-CRM₁₉₇/CP8-CRM₁₉₇,
2) 200/200/100/100 µg/mL of r*m*ClfA/MntC/CP5-CRM₁₉₇/CP8-CRM₁₉₇, or
3) 40/40/20/20 µg/mL of r*m*ClfA/MntC/CP5-CRM₁₉₇/CP8-CRM₁₉₇
were tested for stability at 2-8°C, 25°C, and 37°C. Formulations (1) and (2) were stable at the three temperatures for six months. Formulation (3) demonstrated 5 months of stability at 2-8°C (Table 35). The drug substances used for this study were L40256-30 for r*m*ClfA (tetra-antigen representative lot), L44124-64 for MntC (tetra-antigen representative lot), 7CP5C-1002 for CP5-CRM₁₉₇ (tri-antigen representative lot) and G-C8-3-9 for CP8-CRM₁₉₇ (tri-antigen representative lot).

**Table 35: 5 Month Stability of Tetra-antigen Formulation (3)**

| | **t=0** | **2-8°C, 5 months** |
|---|---|---|
| **Appearance** | White fluffy cake | White fluffy cake |
| **Reconstitution time** | < 30 seconds | < 30 seconds |
| **pH** | 6.5 | 6.5 |
| **Moisture (%)** | 0.64 | 0.77 |
| **r*m*ClfA strength (µg/mL) by IEX** | 38 | 36 |
| **MntC strength (µg/mL) by IEX** | 35 | 40 |
| **CP5 strength (µg/mL) by nephelometry** | 24 | 24 |
| **CP8 strength (µg/mL) by nephelometry** | 25 | 25 |
| **Purity (%) by IEX** | 96.1 | 96.3 |
| **MntC deamidation (%) by IEX** | 1.7 | 2.2 |
| **r*m*ClfA purity (%) by RP-HPLC** | 97.1 | 97.5 |
| **MntC purity (%) by RP-HPLC** | 97.2 | 97.4 |

Table 36 describes the lots of tetra-antigen lyophilized formulations used to study stability.

**Table 36: Description of Formulations Used for Stability Studies**

| | **Concentrations of Actives (µg/mL)** | | | | **Bulk DS Lot No.** | | | |
|---|---|---|---|---|---|---|---|---|
| | r*m*Clf A | MntC | CP5-CRM_{1 97} | CP8-CRM_{1 97} | r*m*ClfA | MntC | CP5-CRM₁₉₇ | CP8-CRM₁₉₇ |
| L44130-39-1 | 400 | 400 | 200 | 200 | L40256-30 | L44124-64 | L40241-84 | L43364-13 |
| L44130-39-2 | 200 | 200 | 100 | 100 | L40256-30 | L44124-64 | L40241-84 | L43364-13 |
| L44130-71-1 | 400 | 400 | 200 | 200 | L40256-53 | L44124-64 | L43365-75 | L43342-177 |
| L44130-71-2 | 200 | 200 | 100 | 100 | L40256-53 | L44124-64 | L43365-75 | L43342-177 |
| L44130-88-1 | 400 | 400 | 200 | 200 | L40256-53 | L44124-64 | L43365-75 | L43342-177 |
| L44130-88-2 | 200 | 200 | 100 | 100 | L40256-53 | L44124-64 | L43365-75 | L43342-177 |
| L44130-100 | 400 | 400 | 200 | 200 | L40256-53 | L44124-64 | L43365-75 | L43342-177 |

| | | | | | | | | |
|---|---|---|---|---|---|---|---|---|
| All formulations contained 10 mM histidine, pH 6.5, 4.5% sucrose, 0.01% Polysorbate 80 Drug substances were provided in the following matrices: r*m*ClfA: 10 mM histidine, pH 6.5 MntC: 10 mM histidine, pH 6.0 CP5- and CP8-CRM₁₉₇ conjugates: 10 mM histidine, pH 6.7 | | | | | | | | |

Table 37 and Table 38 show summaries of 6-month stability data for the high and low doses, respectively. After six months, both doses maintained the same cake appearance, reconstituted rapidly with diluent, pH remained 6.5 ± 0.3, and moisture was retained at < 1.5%. Strengths of r*m*ClfA, MntC, CP5-CRM₁₉₇ and CP8-CRM₁₉₇ did not change. In addition, the purity values of r*m*ClfA and MntC were maintained with respect to t=0. Tetra-antigen high and low dose formulations are shown to be stable at long-term and accelerated storage conditions for three months. Similarly, a second set of formulations at the two dose levels showed stability at 2-8°C, 25°C, and 37°C for six months (Table 39 and Table 40). Based on data, the proposed shelf life for these samples is 12 months.

| **Table 37: 6-Months Stability Summary of Tetra-antigen High Dose Lyophilized Formulation (L44130-39-1)** | | | | | | | | | | |
|---|---|---|---|---|---|---|---|---|---|---|
| | **t=0** | **2-8°, 1 month** | **25°, 1 month** | **37°, 1 month** | **2-8°, 3 months** | **25°, 3 months** | **37°, 3 months** | **2-8°, 6 months** | **25°,6 months** | **37°, 6 months** |
| Appearance | white fluffy cake | white fluffy cake | white fluffy cake | white fluffy cake | white fluffy cake | white fluffy cake | white fluffy cake | white fluffy cake | white fluffy cake | white fluffy cake |
| Reconstitution time | < 30 seconds | < 30 seconds | < 30 seconds | < 30 seconds | <30 seconds | < 30 seconds | < 30 seconds | < 30 seconds | < 30 seconds | < 30 seconds |
| pH | 6.7 | 6.6 | 6.6 | 6.6 | 6.6 | 6.6 | 6.5 | 6.6 | 6.6 | 6.6 |
| Moisture | 0.79 | 0.84 | 1.1 | 1.26 | 0.99 | 1.41 | 1.45 | 1.02 | 1.33 | 1.38 |
| r*m*ClfA strength (µg/mL) by IEX | 360 | 370 | 370 | 370 | 350 | 350 | 350 | 370 | 370 | 370 |
| MntC strength (µg/mL) by IEX | 370 | 360 | 360 | 360 | 360 | 360 | 360 | 380 | 380 | 380 |
| CP5 strength (µg/mL) by nephelometry | 204 | 199 | 195 | 194 | 199 | 197 | 199 | 212 | 209 | 207 |
| CP8 strength (µg/mL) by nephelometry | 198 | 197 | 209 | 211 | 213 | 211 | 206 | 198 | 201 | 202 |
| Purity (%) by IEX | 95.9 | 96.3 | 96.3 | 96.2 | 96.4 | 96.5 | 96.4 | 96.1 | 95.9 | 96.0 |
| MntC deamidation (%) by IEX | 2.9 | 2.6 | 2.6 | 2.6 | 2.6 | 2.5 | 2.5 | 2.7 | 2.8 | 2.7 |
| r*m*ClfA purity (%) by RP-HPLC | 98.5 | 98.7 | 98.8 | 98.7 | 98.2 | 98.0 | 97.9 | 98.3 | 98.2 | 97.8 |
| MntC purity (%) by RP-HPLC | 98.7 | 98.4 | 98.4 | 98.3 | 98.5 | 98.2 | 98.3 | 98.1 | 98.1 | 97.9 |

| **Table 38: 6-Months Stability Summary of Tetra-antigen Low Dose Lyophilized Formulation (L44130-39-2)** | | | | | | | | | | |
|---|---|---|---|---|---|---|---|---|---|---|
| | **t=0** | **2-8°, 1 mo.** | **25°, 1 mo.** | **37°, 1 mo.** | **2-8°, 3 mos.** | **25°, 3mos** | **37°, 3 mos.** | **2-8°, 6 mos.** | **25°,6 mos.** | **37°, 6 mos.** |
| Appearance | white fluffy cake | white fluffy cake | white fluffy cake | white fluffy cake | white fluffy cake | white fluffy cake | white fluffy cake | white fluffy cake | white fluffy cake | white fluffy cake |
| Reconstitution time (seconds) | <30 | <30 | <30 | <30 | <30 | <30 | <30 | <30 | <30 | <30 |
| pH | 6.6 | 6.6 | 6.7 | 6.7 | 6.5 | 6.5 | 6.5 | 6.6 | 6.6 | 6.6 |
| Moisture | 0.75 | 0.74 | 0.92 | 0.98 | 0.80 | 1.06 | 1.07 | 0.96 | 1.15 | 1.06 |
| rmClfA strength (µg/mL) by IEX | 180 | 190 | 190 | 190 | 170 | 170 | 170 | 190 | 190 | 190 |
| MntC strength (µg/mL) by IEX | 180 | 180 | 180 | 180 | 180 | 180 | 180 | 190 | 190 | 190 |
| CP5 strength (µg/mL) by nephelometry | 97 | 97 | 95 | 95 | 99 | 100 | 99 | 106 | 104 | 102 |
| CP8 strength (µg/mL) by nephelometry | 101 | 100 | 100 | 98 | 107 | 109 | 101 | 104 | 104 | 102 |
| Purity (%) by IEX | 95.7 | 96 | 96 | 96.1 | 96.3 | 96.4 | 96.3 | 96.2 | 96.1 | 96.0 |
| MntC deamidation (%) by IEX | 3 | 2.8 | 2.8 | 2.7 | 2.6 | 2.5 | 2.5 | 2.6 | 2.6 | 2.7 |
| r*m*ClfA purity (%) by RP-HPLC | 98.5 | 98.6 | 98.7 | 98.5 | 97.9 | 97.7 | 97.8 | 98.0 | 97.6 | 98.1 |
| MntC purity (%) by RP-HPLC | 98.7 | 98.4 | 98.4 | 98.4 | 98.2 | 97.8 | 98.0 | 98.1 | 98.0 | 97.7 |

| **Table 39: 6-Months Stability Summary of Tetra-antigen High Dose Lyophilized Formulation (L44130-71-1)** | | | | | | | | | | |
|---|---|---|---|---|---|---|---|---|---|---|
| | **t=0** | **2-8°, 1 month** | **25°, 1 month** | **37°, 1 month** | **2-8°, 3 months** | **25°, 3 months** | **37°, 3 months** | **2-8°, 6 months** | **25°, 6 months** | **37°, 6 months** |
| Appearance | white fluffy cake | white fluffy cake | white fluffy cake | white fluffy cake | white fluffy cake | white fluffy cake | white fluffy cake | white fluffy cake | white fluffy cake | white fluffy cake |
| Reconstitution time (seconds) | <30 | <30 | <30 | <30 | <30 | <30 | <30 | <30 | <30 | <30 |
| pH | NA | 6.4 | 6.4 | 6.2 | 6.3 | 6.2 | 6.2 | 6.4 | 6.4 | 6.4 |
| Moisture | 0.57 | 0.56 | 0.60 | 0.58 | 0.57 | 0.56 | 0.58 | 0.59 | 0.62 | 0.61 |
| r*m*ClfA strength (µg/mL) by IEX | 440 | 440 | 440 | 450 | 450 | 450 | 450 | 420 | 430 | 430 |
| MntC strength (µg/mL) by IEX | 400 | 390 | 390 | 390 | 400 | 410 | 400 | 400 | 410 | 400 |
| CP5 strength (µg/mL) by nephelometry | 189 | 187 | 196 | 193 | 181 | 192 | 187 | 179 | 197 | 190 |
| CP8 strength (µg/mL) by nephelometry | 203 | 200 | 189 | 195 | 188 | 201 | 193 | 220 | 216 | 215 |
| MntC Purity (%) by IEX | 95.9 | 96.1 | 96.2 | 96.2 | 95.9 | 96.0 | 96.2 | 95.8 | 96.0 | 96.0 |
| MntC deamidation (%) by IEX | 2.9 | 2.7 | 2.6 | 2.7 | 2.9 | 2.9 | 2.7 | 3.0 | 2.8 | 2.7 |
| r*m*ClfA purity (%) by RP-HPLC | 99.0 | 98.9 | 98.8 | 98.8 | 99.0 | 98.9 | 99.0 | 99.0 | 99.0 | 98.9 |
| MntC purity (%) by RP-HPLC | 98.2 | 98.1 | 98.2 | 98.2 | 97.9 | 97.9 | 98.3 | 98.5 | 98.4 | 98.2 |

| **Table 40: 6-Month Stability Summary of Tetra-antigen Low Dose Lyophilized Formulation (L44130-71-2)** | | | | | | | | | | |
|---|---|---|---|---|---|---|---|---|---|---|
| | **t=0** | **2-8°, 1 month** | **25°, 1 month** | **37°, 1 month** | **2-8°, 3 months** | **25°, 3 months** | **37°, 3 months** | **2-8°, 6 months** | **25°, 6 months** | **37°, 6 months** |
| Appearance | white fluffy cake | white fluffy cake | white fluffy cake | white fluffy cake | white fluffy cake | white fluffy cake | white fluffy cake | white fluffy cake | white fluffy cake | white fluffy cake |
| Reconstitution time (seconds) | <30 | <30 | <30 | <30 | <30 | <30 | <30 | <30 | <30 | < 30 |
| pH | NA | 6.3 | 6.3 | 6.2 | 6.3 | 6.3 | 6.3 | 6.4 | 6.2 | 6.2 |
| Moisture | 0.61 | 0.62 | 0.61 | 0.60 | 0.64 | 0.56 | 0.64 | 0.65 | 0.64 | 0.68 |
| rmClfA strength (µg/mL) by IEX | 220 | 220 | 220 | 220 | 230 | 230 | 230 | 220 | 210 | 210 |
| MntC strength (µg/mL) by IEX | 190 | 190 | 190 | 190 | 200 | 200 | 200 | 190 | 200 | 200 |
| CP5 strength (µg/mL) by nephelometry | 94 | 99 | 94 | 95 | 96 | 96 | 88 | 102 | 103 | 97 |
| CP8 strength (µg/mL) by nephelometry | 94 | 100 | 98 | 98 | 99 | 99 | 93 | 114 | 114 | 114 |
| MntC Purity (%) by IEX | 95.8 | 96.3 | 96.3 | 96.2 | 95.9 | 96.2 | 96.2 | 96.1 | 96.3 | 96.2 |
| MntC deamidation (%) by IEX | 3.1 | 2.7 | 2.6 | 2.7 | 3.0 | 2.7 | 2.7 | 2.8 | 2.6 | 2.6 |
| r*m*ClfA purity (%) by RP-HPLC | 99.0 | 98.7 | 98.7 | 98.6 | 99.0 | 99.0 | 99.0 | 99.0 | 98.8 | 99.0 |
| MntC purity (%) by RP-HPLC | 98.1 | 98.2 | 98.4 | 98.2 | 98 | 98.1 | 97.9 | 98.4 | 98.3 | 98.4 |

Six months of stability testing of a lyophilized drug product matrix at 2-8°C showed that all tested parameters remained the same over the duration of the study (Table 41). The proposed shelf life of the drug product matrix is 12 months.

**Table 41: 6-Month Stability Summary of Tetra-antigen Drug Product Matrix Lyophilized Formulation (L44130-57-2)**

| | **t=0** | **2-8°, 3 months** | **25°, 3 months** |
|---|---|---|---|
| Appearance | white fluffy cake | white fluffy cake | white fluffy cake |
| Reconstitution time | < 30 seconds | < 30 seconds | < 30 seconds |
| pH | 6.5 | 6.4 | 6.5 |
| Moisture | 0.66 | 0.78 | 0.78 |

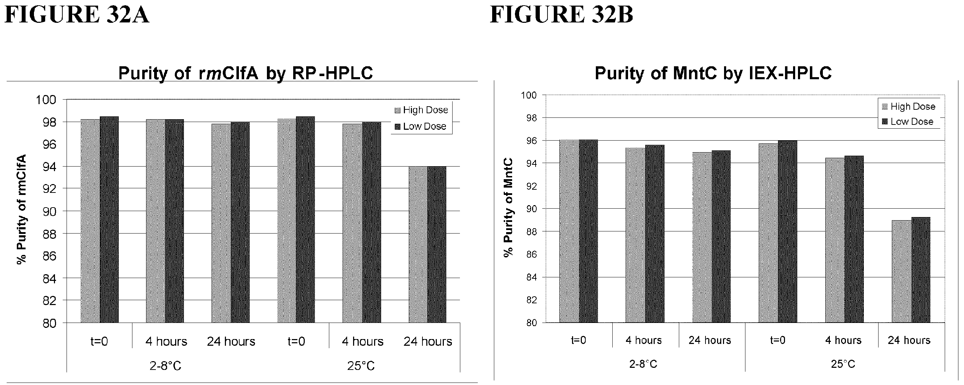

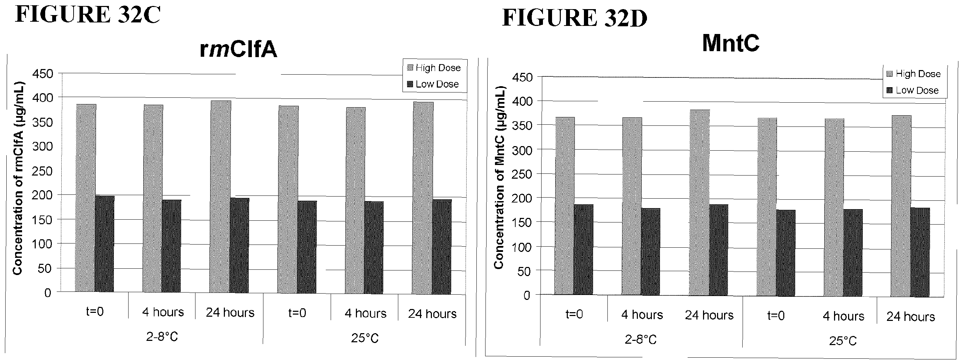

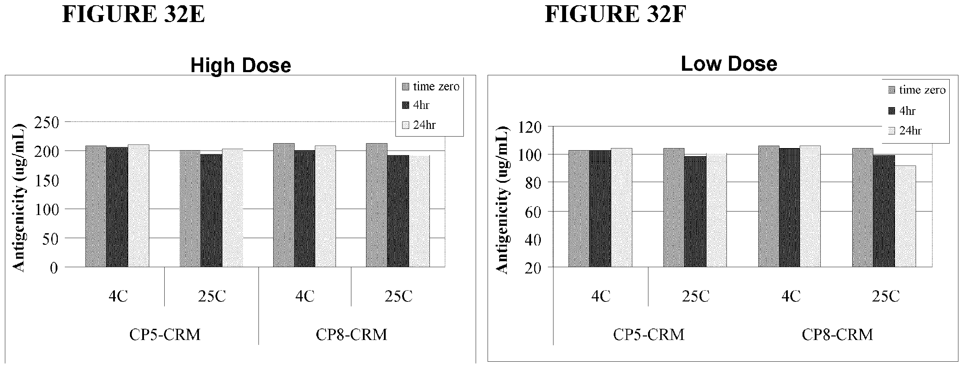

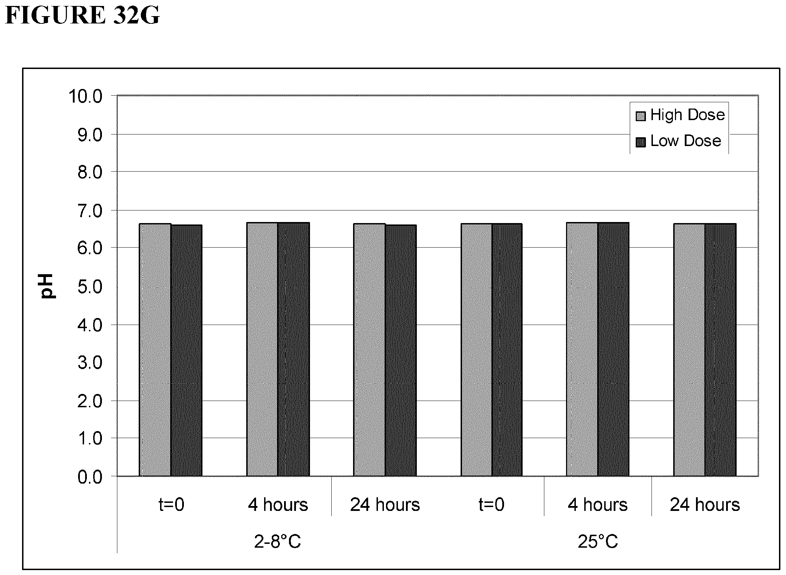

Lyophilized tetra-antigen toxicology high dose (L44130-39-1) and low dose (L44130-39-2) formulations were reconstituted with 0.7 mL of 60 mM NaCl diluent and tested for stability at 2-8°C and 25°C. The samples were analyzed at t=0, 4 and 24 hours. Critical assays were pH, r*m*ClfA purity by RP-HPLC, MntC purity by IEX-HPLC, r*m*ClfA and MntC strengths by IEX-HPLC, and CP5/CP8 conjugate antigenicity by nephelometry. The major mechanisms of degradation being monitored were protein cleavage of r*m*ClfA and deamidation of MntC. In Figures 32A and B, the purities of both recombinant proteins are maintained at 2-8°C for 24 hours for both doses. At 25°C incubation, the purities of both proteins remained unchanged at 4 hours but decreased at 24 hours. The strengths of the proteins (Figures 32C and D) and conjugates (Figures 32E and F), and the pH (Figure 32G) also were constant after 24 hours at 2-8 and 25°C. In short, the formulations for all four antigens were stable following reconstitution for four hours at room temperature and up to twenty four hours at 2-8°C based on analysis of the post-reconstituted formulations.

### EXAMPLE 25: TETRA-ANTIGEN DRUG PRODUCT: EDGE STUDIES

An edge study was designed to bracket the components of high and low formulations to demonstrate the robustness of the formulation. Table 42details the formulations. The active components were varied as 30% higher than the high dose (400/400/200/200 µg/mL of r*m*ClfA/MntC/CP5-CRM₁₉₇/CP8-CRM₁₉₇) and 30% lower than the low dose (200/200/100/100 µg/mL of r*m*ClfA/MntC/CP5-CRM₁₉₇/CP8-CRM₁₉₇). The inactives were varied in the ranges of 6.0 - 7.0 for pH, 5 - 15 mM for histidine, 3 - 6% for sucrose, and 0.005 - 0.015% for PS80.

**Table 42: Edge Study Design**

| **Description** | **r*m*ClfA (µg/mL)** | **MntC (µg/mL)** | **CP5-CRM (µg/mL)** | **CP8-CRM (µg/mL)** | **pH** | **Histidine (mM)** | **sucrose (%)** | **PS80 (%)** |
|---|---|---|---|---|---|---|---|---|
| **+30% of High dose -30% of** | 520 | 520 | 260 | 260 | 7.0 | 15 | 6 | 0.015 |
| **Low dose** | 140 | 140 | 70 | 70 | 6.0 | 5 | 3 | 0.005 |
| **High/Low** | 520 | 520 | 260 | 260 | 6.0 | 5 | 3 | 0.005 |
| **Low/High** | 140 | 140 | 70 | 70 | 7.0 | 15 | 6 | 0.015 |

The edge formulations were lyophilized, and placed on stability at 2-8°C, 25°C and 37°C. The results shown in Tables 43-46 indicate that all edge formulations were stable for 3 months at the three temperatures, confirming robustness of the tetra-antigen formulation.

**Table 43: Edge Stability: +30% High Dose**

| | **t=0** | **2-8°, 1 month** | **25°, 1 month** | **37°, 1 month** | **2-8°, 3 months** | **25°, 3 months** | **37°, 3 months** |
|---|---|---|---|---|---|---|---|
| Appearance | white fluffy cake | white fluffy cake | white fluffy cake | white fluffy cake | white fluffy cake | white fluffy cake | white fluffy cake |
| Reconstitution time | < 30 seconds | < 30 seconds | < 30 seconds | < 30 seconds | < 30 seconds | < 30 seconds | < 30 seconds |
| pH | 7.0 | 7.0 | 7.0 | 7.0 | 7.0 | 7.0 | 7.0 |
| Moisture | 0.58 | 0.62 | 0.83 | 0.89 | 0.72 | 0.93 | 1.02 |
| r*m*ClfA strength (µg/mL) by IEX | 500 | 460 | 460 | 470 | 460 | 460 | 460 |
| MntC strength (µg/mL) by IEX | 480 | 510 | 510 | 510 | 490 | 490 | 490 |
| CP5 strength (µg/mL) by nephelometry | 259 | 263 | 265 | 271 | 252 | 272 | 278 |
| CP8 strength (µg/mL) by nephelometry | 277 | 280 | 293 | 280 | 264 | 278 | 275 |
| Purity (%) by IEX | 95.7 | 95.8 | 95.9 | 95.9 | 96.1 | 96.2 | 96.1 |
| MntC deamidation (%) by IEX | 3.1 | 3.0 | 3.0 | 3.0 | 2.8 | 2.8 | 2.7 |
| r*m*ClfA purity (%) by RP-HPLC | 97.9 | 98.1 | 97.9 | 98 | 97.7 | 97.5 | 97.8 |
| MntC purity (%) by RP-HPLC | 98.1 | 98.2 | 97.8 | 97.9 | 97.7 | 97.6 | 97.9 |

**Table 44: Edge Stability: -30% Low Dose**

| | **t=0** | **2-8°, 1 month** | **25°, 1 month** | **37°, 1 month** | **2-8°, 3 months** | **25°, 3 months** | **37°, 3 months** |
|---|---|---|---|---|---|---|---|
| Appearance | white fluffy cake | white fluffy cake | white fluffy cake | white fluffy cake | white fluffy cake | white fluffy cake | white fluffy cake |
| Reconstitution time | < 30 seconds | < 30 seconds | < 30 seconds | < 30 seconds | < 30 seconds | < 30 seconds | < 30 seconds |
| pH | 5.8 | 5.9 | 5.9 | 5.9 | 5.8 | 5.7 | 5.8 |
| Moisture | 0.83 | 0.96 | 1.27 | 1.33 | 1.03 | 1.46 | 1.47 |
| r*m*ClfA strength (µg/mL) by IEX | 140 | 130 | 130 | 130 | 130 | 130 | 120 |
| MntC strength (µg/mL) by IEX | 140 | 130 | 130 | 130 | 140 | 140 | 130 |
| CP5 strength (µg/mL) by nephelometry | 67 | 66 | 65 | 64 | 67 | 71 | 72 |
| CP8 strength (µg/mL) by nephelometry | 71 | 74 | 69 | 70 | 73 | 74 | 72 |
| Purity (%) by IEX | 95.9 | 95.9 | 96.1 | 96.1 | 96.4 | 96.5 | 96.5 |
| MntC deamidation (%) by IEX | 2.8 | 2.8 | 2.7 | 2.6 | 2.5 | 2.4 | 2.4 |
| r*m*ClfA purity (%) by RP-HPLC | 97.9 | 97.8 | 97.9 | 98 | 97.7 | 97.2 | 97.9 |
| MntC purity (%) by RP-HPLC | 98 | 97.5 | 97.6 | 97.6 | 97.7 | 97.2 | 97.8 |

**Table 45: Edge Stability: High/Low**

| | **t=0** | **2-8°, 1 month** | **25°, 1 month** | **37°, 1 month** | **2-8°, 3 months** | **25°, 3 months** | **37°, 3 months** |
|---|---|---|---|---|---|---|---|
| Appearance | white fluffy cake | white fluffy cake | white fluffy cake | white fluffy cake | white fluffy cake | white fluffy cake | white fluffy cake |
| Reconstitution time | < 30 seconds | < 30 seconds | < 30 seconds | < 30 seconds | < 30 seconds | < 30 seconds | < 30 seconds |
| pH | 5.9 | 5.9 | 5.9 | 6.0 | 5.9 | 6.0 | 6.0 |
| Moisture | 0.5 | 0.57 | 0.86 | 1.05 | 0.58 | 1.14 | 1.20 |
| r*m*ClfA strength (µg/mL) by IEX | 510 | 470 | 470 | 470 | 460 | 460 | 460 |
| MntC strength (µg/mL) by IEX | 490 | 520 | 510 | 510 | 500 | 500 | 500 |
| CP5 strength (µg/mL) by nephelometry | 251 | 259 | 252 | 262 | 256 | 259 | 260 |
| CP8 strength (µg/mL) by nephelometry | 263 | 269 | 278 | 272 | 270 | 273 | 265 |
| Purity (%) by IEX | 95.9 | 96.1 | 96.1 | 96.1 | 96.2 | 96.4 | 96.3 |
| MntC deamidation (%) by IEX | 2.9 | 2.8 | 2.7 | 2.8 | 2.7 | 2.6 | 2.6 |
| r*m*ClfA purity (%) by RP-HPLC | 97.7 | 98 | 98.1 | 97.9 | 97.8 | 97.5 | 97.5 |
| MntC purity (%) by RP-HPLC | 98.2 | 98.2 | 97.9 | 97.9 | 98.2 | 98.0 | 97.6 |

**Table 46: Edge Stability: Low/High**

| | **t=0** | **2-8°, 1 month** | **25°, 1 month** | **37°, 1 month** | **2-8°, 3 months** | **25°, 3 months** | **37°, 3 months** |
|---|---|---|---|---|---|---|---|
| Appearance | white fluffy cake | white fluffy cake | white fluffy cake | white fluffy cake | white fluffy cake | white fluffy cake | white fluffy cake |
| Reconstitution time | <30 seconds | <30 seconds | <30 seconds | <30 seconds | <30 seconds | <30 seconds | <30 seconds |
| pH | 7.0 | 7.1 | 7.1 | 7.1 | 7.1 | 7.0 | 7.0 |
| Moisture | 0.58 | 0.68 | 0.82 | 0.87 | 0.68 | 0.92 | 0.97 |
| r*m*ClfA strength (µg/mL) by IEX | 140 | 120 | 130 | 130 | 120 | 120 | 120 |
| MntC strength (µg/mL) by IEX | 140 | 130 | 130 | 130 | 130 | 130 | 140 |
| CP5 strength (µg/mL) by nephelometry | 67 | 66 | 67 | 65 | 69 | 72 | 69 |
| CP8 strength (µg/mL) by nephelometry | 74 | 70 | 71 | 71 | 75 | 73 | 75 |
| Purity (%) by IEX | 95.9 | 96.2 | 96.3 | 96 | 96.6 | 96.7 | 96.6 |
| MntC deamidation (%) by IEX | 3.0 | 2.6 | 2.6 | 2.9 | 2.4 | 2.4 | 2.3 |
| r*m*ClfA purity (%) by RP-HPLC | 97.8 | 97.7 | 97.9 | 97.7 | 97.4 | 97.3 | 97.3 |
| MntC purity (%) by RP-HPLC | 98 | 97.9 | 97.9 | 97.7 | 97.5 | 97.2 | 97.3 |

### EXAMPLE 26: TETRA-ANTIGEN DRUG PRODUCT: FREEZE-THAW STABILITY

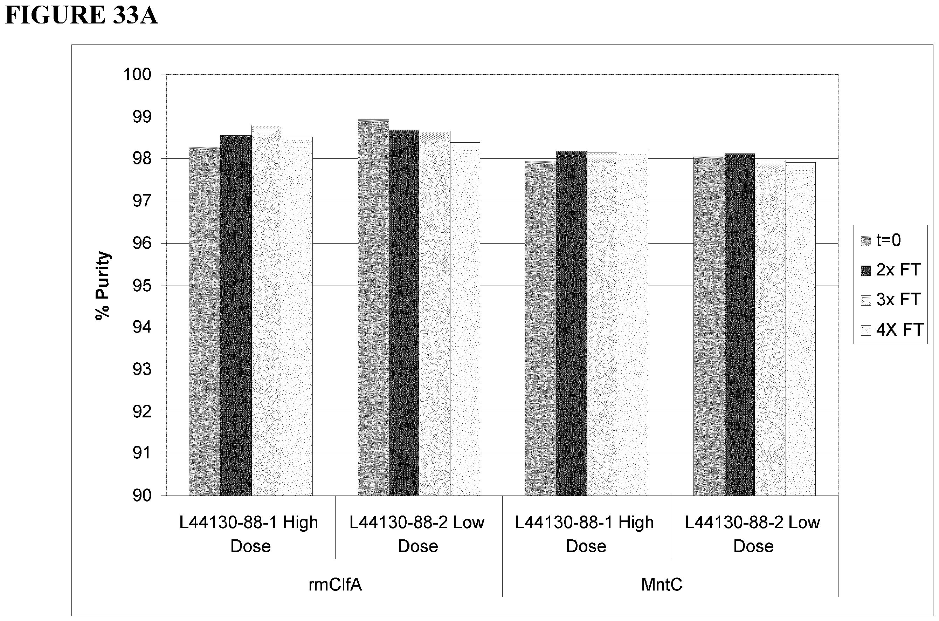

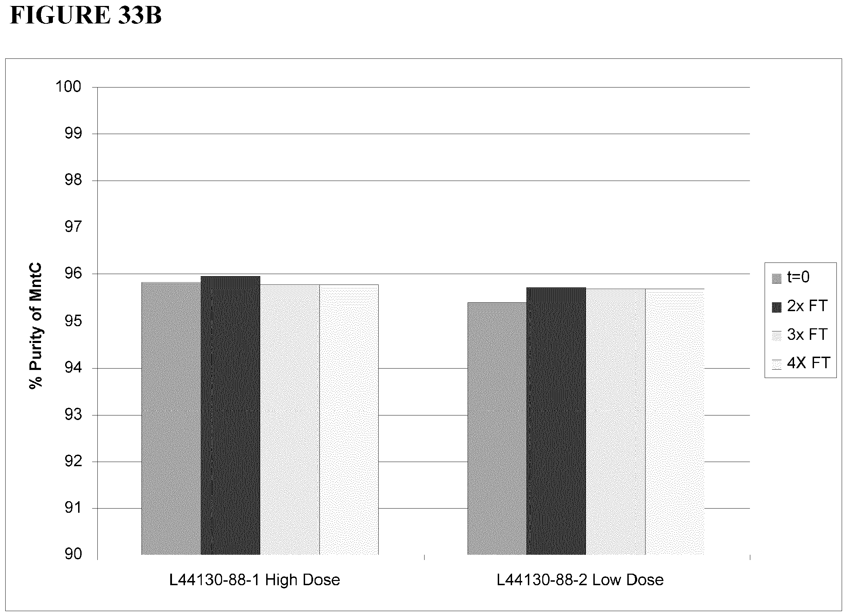

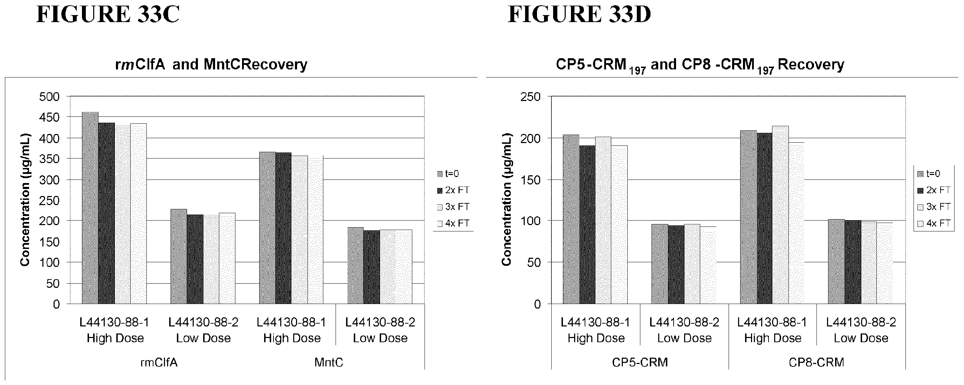

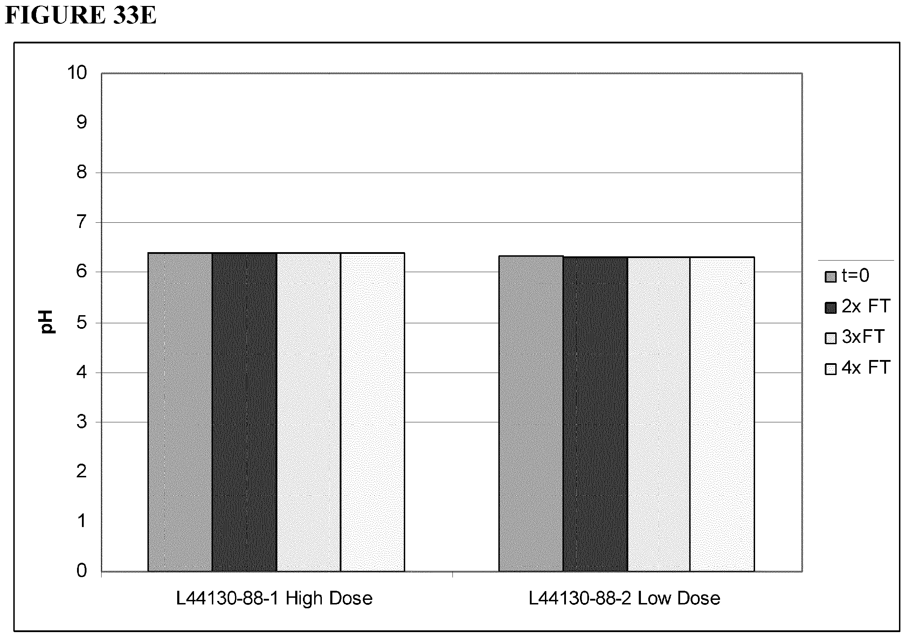

Pre-lyophilized liquid formulations of tetra-antigen high dose (L44130-88-1) and low dose (L44130-88-2) were frozen and thawed in Schott type 1 vials for four cycles. The samples were frozen each time for at least 24 hours at -70°C and then thawed at room temperature. During the thaw cycle, the vials were monitored closely so that the defrosted liquid formulations were not left at room temperature for an extended period. This was done to ensure that the data did not reflect any clipping of r*m*ClfA and deamidation of MntC as a result of the formulations incubating at room temperature. Each freeze-thaw set (done in triplicates) was pooled and analyzed by RP-HPLC for r*m*ClfA and MntC purity, IEX-HPLC for r*m*ClfA and MntC concentration, IEX-HPLC for MntC purity, and nephelometry for CP5 and CP8 conjugate concentrations. After four freeze-thaw cycles, the purities of r*m*ClfA and MntC by RP-HPLC (Figure 33A) and the purity of MntC by IEX-HPLC (Figure 33B) did not change. Likewise, the concentrations of the antigens (Figures 33C and D) and pH (Figure 33E) remained consistent after four freeze-thaw cycles.

### EXAMPLE 27: TETRA-ANTIGEN DRUG PRODUCT: STABILITY AFTER AGITATION

The objective of studies measuring stability after agitation was to ensure that the tetra-antigen formulation containing 0.01% polysorbate 80 did not show any adsorption issues with the container closure system. High dose pre-lyophilized tetra-antigen formulations (L44130-100) were prepared with 0, 0.01%, and 0.03% polysorbate 80, and the samples were agitated for 24 hours at room temperature in a lyophilization vial/stopper container closure. Control samples were placed at the same ambient temperature but without agitation. For agitating, a VWR DVX-2500 multi-tube vortexer was set to 500 RPM in pulse mode. The polysorbate 80 concentration was bracketed over a range of 0 to 0.03%.

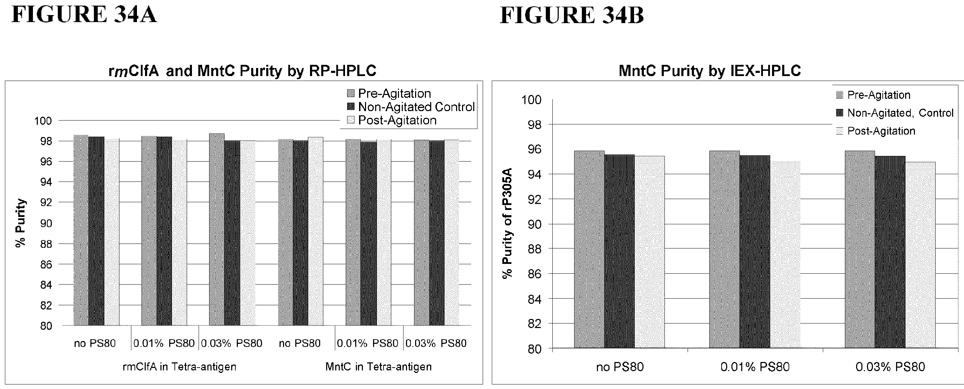

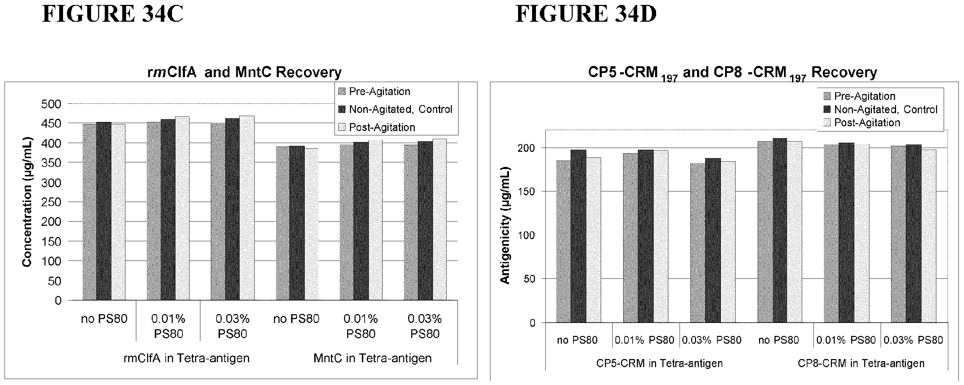

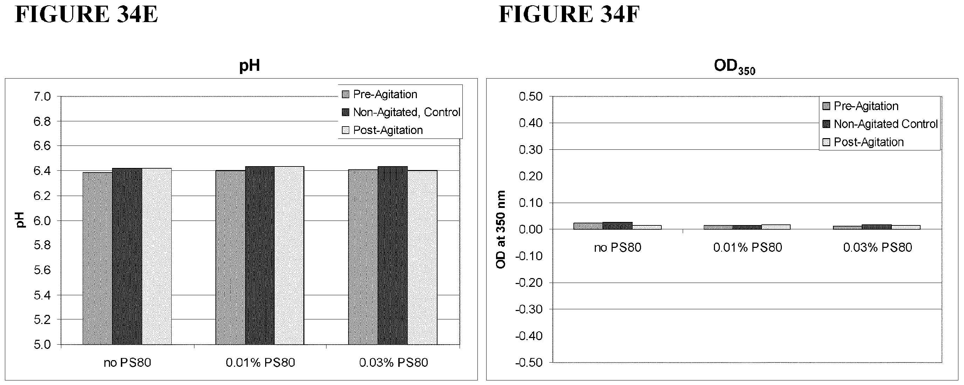

Key assays included the following: RP-HPLC for detection of r*m*ClfA cleavage and MntC degradation (excluding deamidation), IEX-HPLC to monitor MntC deamidation, concentration of the four antigens, and pH and OD₃₅₀. Figures 34A and B show that purity of r*m*ClfA and MntC remained similar after agitation. It is also shown in Figures 34C and D that all four antigens are recovered post-agitation. The pH is maintained (Figure 34E), and agitation does not promote aggregation of the antigens as monitored by OD₃₅₀ (Figure 34E). All data indicate that the formulation is compatible with the lyophilization container closure system.

Though the lyophilization container closure does not contain silicone, the formulation will come in contact with silicone on the stoppers of glass syringes that will be used to reconstitute and deliver the vaccine.

### EXAMPLE 28: TETRA-ANTIGEN DRUG PRODUCT: ISCOMATRIX™ STUDIES

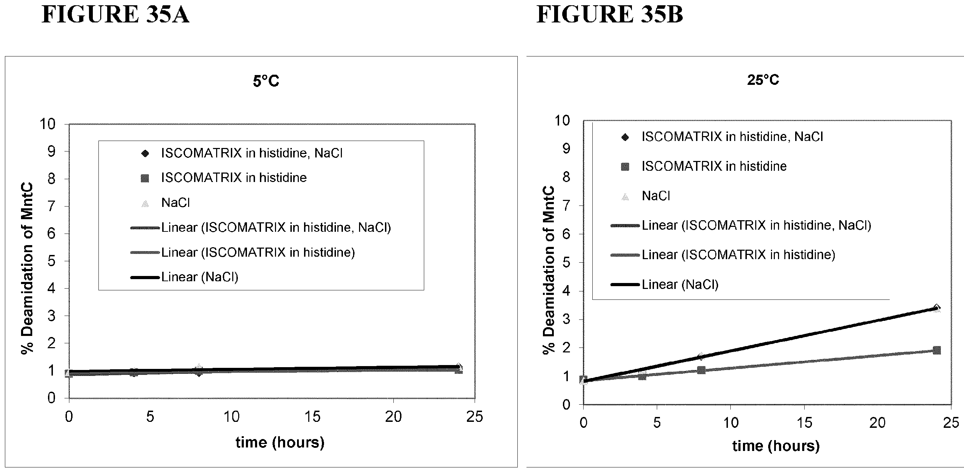

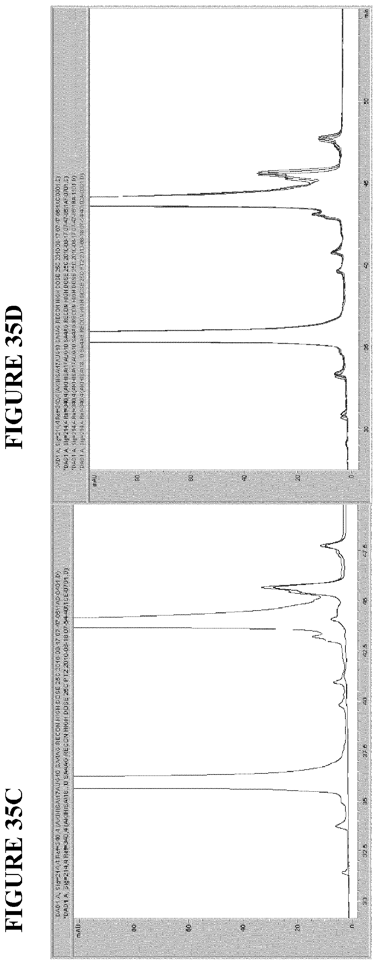

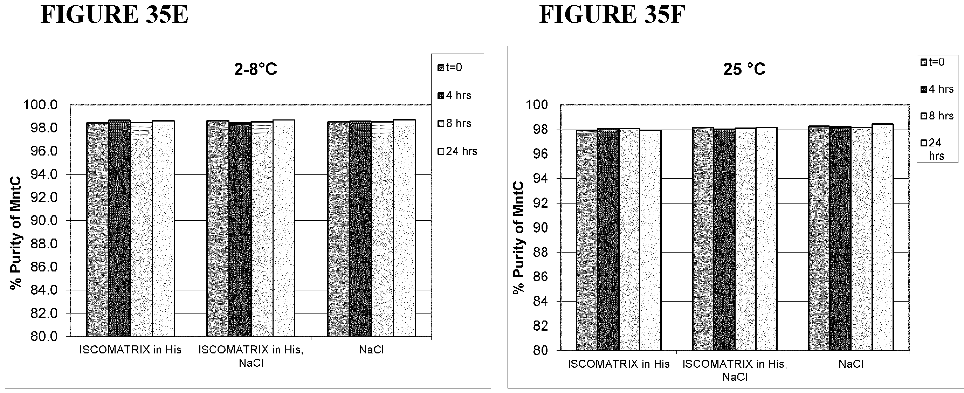

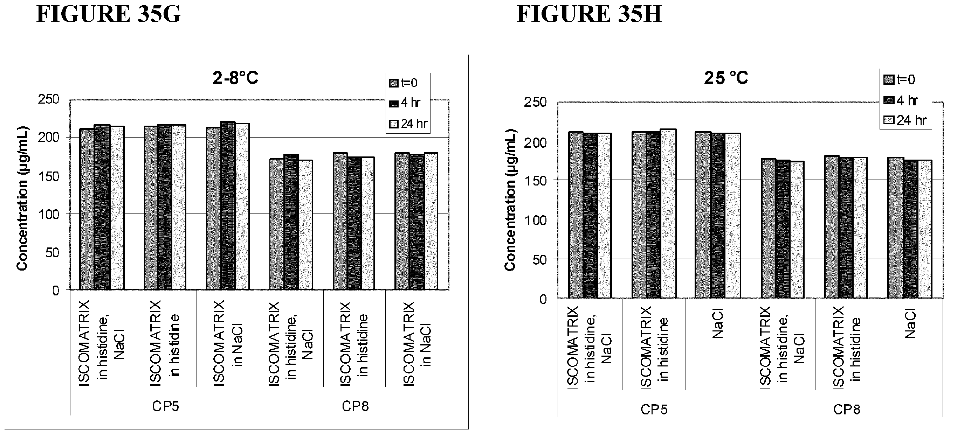

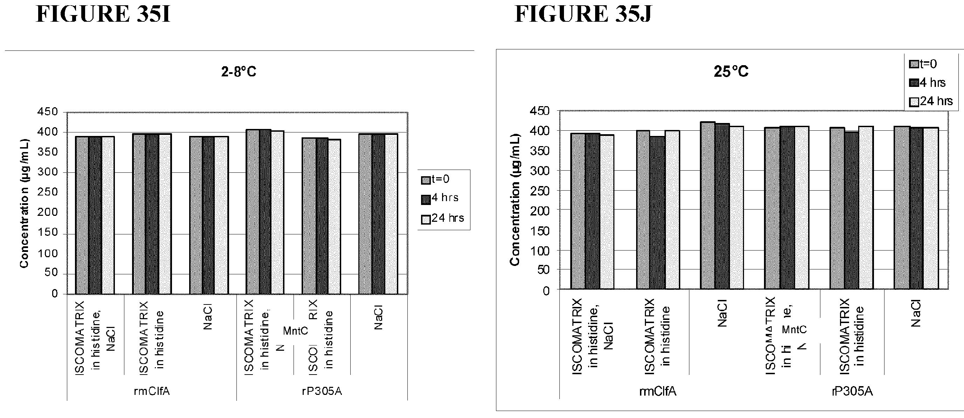

The stability of the high dose tetra-antigen formulation (400/400/200/200 µg/mL r*m*ClfA/rP305A/CP5-CRM₁₉₇/CP8-CRM₁₉₇) was analyzed after reconstitution in 10 mM histidine, pH 6.5 with 20 units/mL of ISCOMATRIX™ with and without 60 mM NaCl. Stability was monitored at 2-8°C and 25°C over 24 hrs. The purity of MntC was analyzed by CEX-HPLC. At 25°C ISCOMATRIX™ with NaCl and control NaCl with salt show similar deamidation rates (Figure 35B). ISCOMATRIX™ with no salt showed slower rate of deamidation. At 5°C the rate of deamidation was drastically reduced (Figure 35A). It was not possible to quantify r*m*ClfA purity directly due to interference from ISCOMATRIX™ (Figures 35C). An analysis of representative time course overlays indicated that the r*m*ClfA main peak was not changed indicating stability over 24 hours at 25°C (Figure 35D). Similar results were seen for all samples at 2-8°C. No change in purity of MntC after 24 hours at 2-8°C (Figure 35E) and 25°C (Figure F) was detected by RP-HPLC (Figure 35C). No changes in CP5-CRM₁₉₇ and CP8-CRM₁₉₇ conjugate concentrations were observed after 24 hours at 2-8°C (Figure 35G) and 25°C (Figure 35H). No changes in r*m*ClfA or MntC concentrations were observed after 24 hours at 2-8°C (Figure 35I) and 25°C (Figure 35J).

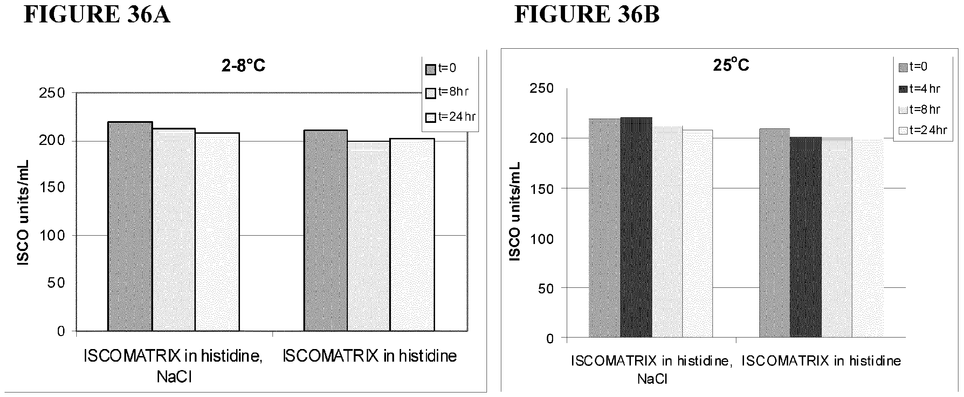

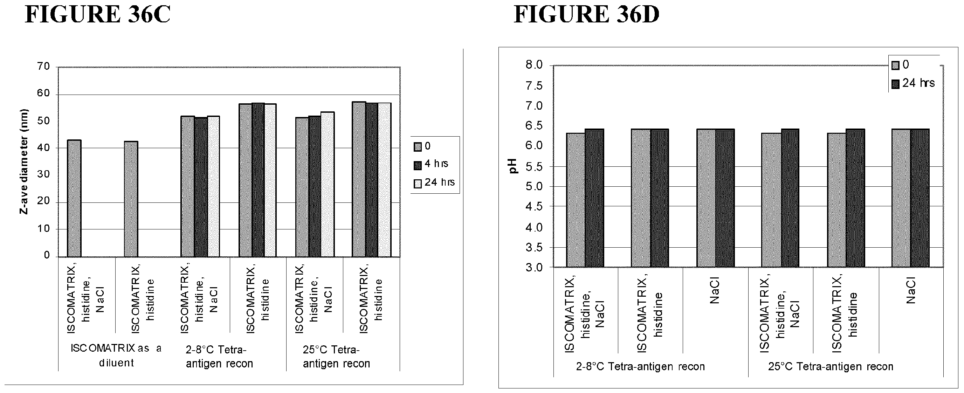

ISCOMATRIX™ concentrations did not change over 24 hours at 2-8°C (Figure 36A) and 25°C (Figure 36B). The particle size of ISCOMATRIX™ with NaCl increased in size to ∼50 nm and without NaCl increased in size to ∼60 nm (Figure 36C). All populations were monodispersed (< 0.2). The pH of reconstituted formulations remained constant through the stability study (Figure 36D).

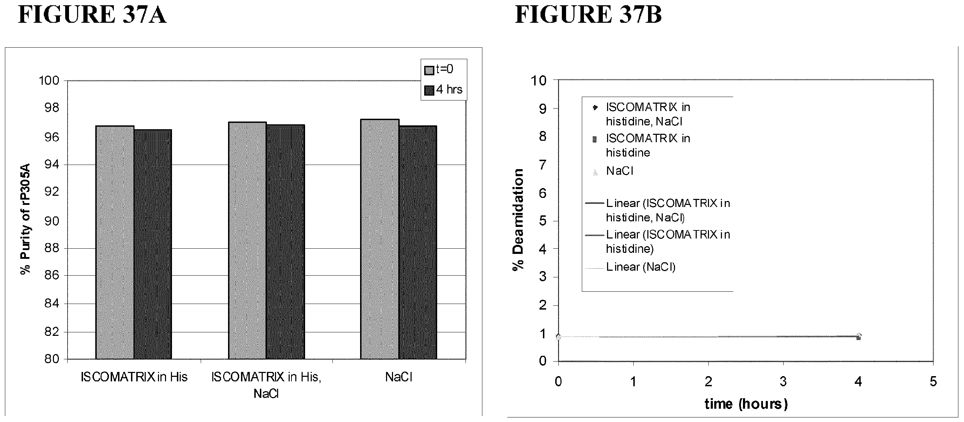

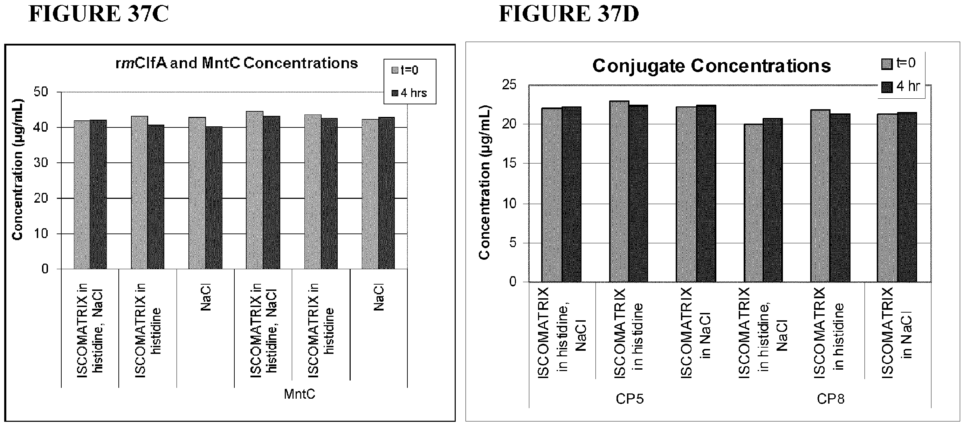

The stability of the low dose tetra-antigen formulation low dose (40/40/20/20 µg/mL r*m*ClfA/rP305A/CP5-CRM₁₉₇/CP8-CRM₁₉₇) was analyzed after reconstitution in 10 mM histidine, pH 6.5 with 20 units/mL of ISCOMATRIX™ with and without 60 mM NaCl. No change in MntC purity was detected by RP-HPLC after 4 hours at 2-8°C (Figure 37A). No increase in MntC deamidation was detected by CEX-HPLC after 4 hours at 2-8°C (Figures 37B). All four antigen concentrations remained constant throughout the study (Figures 37C and D).

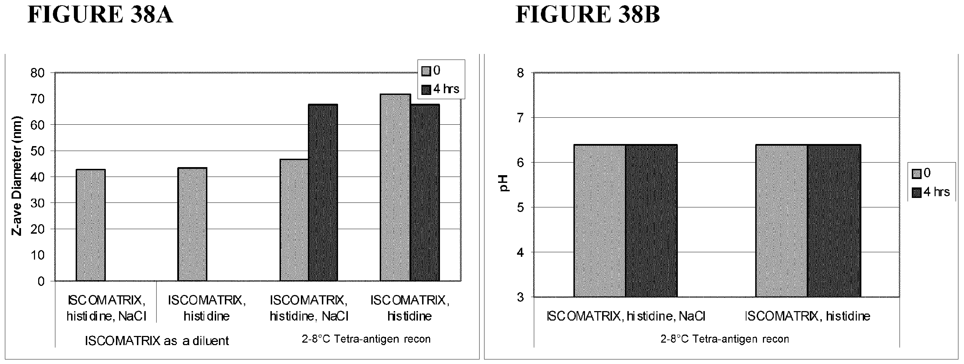

The particle size of ISCOMATRIX™ with NaCl increased after 4 hours and without NaCl remained constant, but in both cases was larger than ISCOMATRIX™ alone (Figure 38A). Reconstituted samples were polydispersed (∼0.3). The pH of reconstituted formulations remained constant after 4 hours (Figure 38B).

### EXAMPLE 29: TETRA-ANTIGEN DRUG PRODUCT: DILUENT COMPARISON

A short-term stability study was conducted using low-, mid-, and high-doses of MntC in the tetra-antigen formulation to compare 60mM NaCl, 4.5% sucrose in water, and water as diluents. Each formulation contained 4.5% sucrose, 0.01% PS80, and the concentration of antigen provided in Table 47 at a pH of 6.5.

**Table 47: Tetra-Antigen Drug Product Formulations**

| **Dose** | **Recon Lot #** | **Concentration of rP305A** | **Concentration of CP5-CRM197, CP8-CRM197, rmClfA, respectively** |
|---|---|---|---|
| Low | 125426-036-A | 40 ug/mL | 60ug/mL, 60 ug/mL, 120 ug/mL |
| Mid | 125426-036-B | 120 ug/mL | 60ug/mL, 60 ug/mL, 120 ug/mL |
| High | 125426-036-C | 400 ug/mL | 60ug/mL, 60 ug/mL, 120 ug/mL |

Vials were stored at 2-8°C prior to the start of the experiment and tested for stability at 5°C and 25°C after 2 and 7 days. The formulations were compared using the following assays: CEX-HPL for MntC deamidation and purity, RP-HPLC for MntC and ClfA purity, IEX for MntC and ClfA concentration, and nephelometry for conjugate concentration. Details of the results are provided in Tables 48-56. The deamidation rate of MntC was higher in all three formulations reconstituted with 60 mM NaCl, and the high-dose MntC had the highest rate of deamidation among the three dose levels. All other results were comparable.

**Table 48: Short-Term Stability Summary of Low-Dose MntC (125426-036-A) Using 60 mM NaCl as a Diluent**

| | **5°C, t=0** | **5°C, 2days** | **5°C,7 days** | **25°C, 2 days** | **25°C, 7 days** |
|---|---|---|---|---|---|
| MntC Deamidation (%) by CEX-HPLC | 1.3 | 1.7 | 2.7 | 5.7 | 18.9 |
| MntC Purity (%) by CEX-HPLC | 98.3 | 98.0 | 97.0 | 93.9 | 80.2 |
| MntC Purity (%) by RP-HPLC | 99.4 | 98.5 | 99.2 | 99.3 | 99.2 |
| ClfA Purity (%) by RP-HPLC | 97.6 | 98.2 | 97.8 | 97.5 | 98.0 |
| MntC Concentration (µg/mL) by IEX | 41 | 42 | 41 | 41 | 41 |
| ClfA Concentration (µg/mL) by IEX | 120 | 118 | 116 | 117 | 115 |
| CP5 Concentration (µg/mL) by nephelometry | 61 | 63 | 64 | 61 | 64 |
| CP8 Concentration (µg/mL) by nephelometry | 56 | 55 | 57 | 53 | 53 |

**Table 49: Short-Term Stability Summary of Low-Dose MntC (125426-036-A) Using 4.5% Sucrose in Water as a Diluent**

| | **5°C, t=0** | **5°C, 2days** | **5°C,7 days** | **25°C, 2 days** | **25°C, 7 days** |
|---|---|---|---|---|---|
| MntC Deamidation (%) by CEX-HPLC | 1.2 | 1.4 | 1.5 | 1.9 | 3.9 |
| MntC Purity (%) by CEX-HPLC | 98.4 | 98.3 | 98.2 | 97.7 | 95.7 |
| MntC Purity (%) by RP-HPLC | 99.4 | NT | 99.2 | 99.3 | 99.3 |
| ClfA Purity (%) by RP-HPLC | 97.6 | 98.0 | 97.6 | 97.5 | 97.8 |
| MntC Concentration (µg/mL) by IEX | 41 | 41 | 39 | 39 | 39 |
| ClfA Concentration (µg/mL) by IEX | 119 | 118 | 114 | 117 | 115 |
| CP5 Concentration (µg/mL) by nephelometry | 63 | 61 | 60 | 62 | 66 |
| CP8 Concentration (µg/mL) by nephelometry | 59 | 58 | 59 | 58 | 58 |

| | | | | | |
|---|---|---|---|---|---|
| NT - Not Tested | | | | | |

**Table 50: Short-Term Stability Summary of Low-Dose MntC 125426-036-A Using Water as a Diluent**

| | **5°C, t=0** | **5°C, 2days** | **5°C,7 days** | **25°C, 2 days** | **25°C, 7 days** |
|---|---|---|---|---|---|
| MntC Deamidation (%) by CEX-HPLC | 1.2 | 1.3 | 1.5 | 1.9 | 3.9 |
| MntC Purity (%) by CEX-HPLC | 98.4 | 98.4 | 98.2 | 97.8 | 95.8 |
| MntC Purity (%) by RP-HPLC | 99.3 | NT | 99.4 | 99.3 | 99.4 |
| ClfA Purity (%) by RP-HPLC | 97.8 | NT | 98.2 | 97.4 | 98.8 |
| MntC Concentration (µg/mL) by IEX | 40 | 40 | 39 | 39 | 40 |
| ClfA Concentration (µg/mL) by IEX | 118 | 118 | 117 | 117 | 115 |
| CP5 Concentration (µg/mL) by nephelometry | 59 | 62 | 63 | 61 | 66 |
| CP8 Concentration (µg/mL) by nephelometry | 54 | 58 | 60 | 59 | 61 |
| NT- Not Tested | | | | | |

**Table 51: Short-Term Stability Summary of Mid-Dose MntC (125426-036-B) Using 60 mM NaCl as a Diluent**

| | **5°C, t=0** | **5°C, 2days** | **5°C, 7 days** | **25°C, 2 days** | **25°C, 7 days** |
|---|---|---|---|---|---|
| MntC Deamidation (%) by CEX-HPLC | 1.1 | 1.7 | 2.8 | 7.2 | 24.4 |
| MntC Purity (%) by CEX-HPLC | 98.6 | 98.1 | 96.9 | 92.6 | 74.2 |
| MntC Purity (%) by RP-HPLC | 99.4 | NT | 99.2 | 99.4 | 99.4 |
| ClfA Purity (%) by RP-HPLC | 97.9 | NT | 98.1 | 97.8 | 98.3 |
| MntC Concentration (µg/mL) by IEX | 131 | 135 | 132 | 130 | 127 |
| ClfA Concentration (µg/mL) by IEX | 125 | 126 | 124 | 124 | 121 |
| CP5 Concentration (µg/mL) by nephelometry | 65 | 64 | 67 | 62 | 68 |
| CP8 Concentration (µg/mL) by nephelometry | 54 | 53 | 54 | 52 | 54 |

| | | | | | |
|---|---|---|---|---|---|
| **NT - Not Tested** | | | | | |

**Table 52: Short-Term Stability Summary of Mid-Dose MntC (125426-036-B) Using 4.5% Sucrose in Water as a Diluent**

| | **5°C, t=0** | **5°C, 2days** | **5°C,7 days** | **25°C, 2 days** | **25°C, 7 days** |
|---|---|---|---|---|---|
| MntC Deamidation (%) by CEX-HPLC | 1.0 | 1.1 | 1.4 | 2.0 | 5.1 |
| MntC Purity (%) by CEX-HPLC | 98.7 | 98.6 | 98.4 | 97.7 | 94.6 |
| MntC Purity (%) by RP-HPLC | 99.4 | NT | 99.3 | 99.3 | 99.4 |
| ClfA Purity (%) by RP-HPLC | 97.7 | NT | 97.8 | 97.5 | 97.9 |
| MntC Concentration (µg/mL) by IEX | 132 | 133 | 129 | 132 | 124 |
| ClfA Concentration (µg/mL) by IEX | 128 | 128 | 126 | 129 | 122 |
| CP5 Concentration (µg/mL) by nephelometry | 60 | 70 | 68 | 68 | 73 |
| CP8 Concentration (µg/mL) by nephelometry | 51 | 60 | 59 | 56 | 59 |

| | | | | | |
|---|---|---|---|---|---|
| **NT - Not Tested** | | | | | |

**Table 53: Short-Term Stability Summary of Mid-Dose MntC 125426-036-B Using Water as a Diluent**

| | **5°C, t=0** | **5°C, 2days** | **5°C,7 days** | **25°C, 2 days** | **25°C, 7 days** |
|---|---|---|---|---|---|
| MntC Deamidation (%) by CEX-HPLC | 1.0 | 1.1 | 1.4 | 2.0 | 5.3 |
| MntC Purity (%) by CEX-HPLC | 98.7 | 98.6 | 98.4 | 97.7 | 94.4 |
| MntC Purity (%) by RP-HPLC | 99.4 | NT | 99.5 | 99.4 | 99.0 |
| ClfA Purity (%) by RP-HPLC | 97.6 | NT | 98.9 | 97.9 | 99.2 |
| MntC Concentration (µg/mL) by IEX | 135 | 130 | 126 | 128 | 124 |
| ClfA Concentration (µg/mL) by IEX | 131 | 125 | 123 | 125 | 119 |
| CP5 Concentration (µg/mL) by nephelometry | 66 | 68 | 69 | 68 | 70 |
| CP8 Concentration (µg/mL) by nephelometry | 56 | 57 | 62 | 56 | 58 |

| | | | | | |
|---|---|---|---|---|---|
| **NT - Not Tested** | | | | | |

**Table 54: Short-Term Stability Summary of High-Dose MntC (125426-036-C) Using 60 mM NaCl as a Diluent**

| | **5°C, t=0** | **5°C, 2days** | **5°C,7 days** | **25°C, 2 days** | **25°C, 7 days** |
|---|---|---|---|---|---|
| MntC Deamidation (%) by CEX-HPLC | 1.2 | 1.9 | 3.4 | 8.1 | 27.3 |
| MntC Purity (%) by CEX-HPLC | 98.6 | 97.9 | 96.3 | 91.6 | 71.4 |
| MntC Purity (%) by RP-HPLC | 99.3 | 99.3 | NT | 99.2 | NT |
| ClfA Purity (%) by RP-HPLC | 97.9 | 97.8 | NT | 98.0 | NT |
| MntC Concentration (µg/mL) by IEX | 434 | 444 | 446 | 448 | 423 |
| ClfA Concentration (µg/mL) by IEX | 121 | 122 | 123 | 121 | 119 |
| CP5 Concentration (µg/mL) by nephelometry | 65 | 62 | 68 | 65 | 68 |
| CP8 Concentration (µg/mL) by nephelometry | 54 | 55 | 55 | 52 | 56 |

| | | | | | |
|---|---|---|---|---|---|
| **NT - Not Tested** | | | | | |

**Table 55: Short-Term Stability Summary of High-Dose MntC (125426-036-C) Using 4.5% Sucrose in Water as a Diluent**

| | **5°C, t=0** | **5°C, 2days** | **5°C,7 days** | **25°C, 2 days** | **25°C, 7 days** |
|---|---|---|---|---|---|
| MntC Deamidation (%) by CEX-HPLC | 1.2 | 1.4 | 2.0 | 3.2 | 10.6 |
| MntC Purity (%) by CEX-HPLC | 98.6 | 98.3 | 97.7 | 96.6 | 88.8 |
| MntC Purity (%) by RP-HPLC | 99.4 | 99.3 | NT | 99.3 | NT |
| ClfA Purity (%) by RP-HPLC | 97.9 | 97.8 | NT | 98.0 | NT |
| MntC Concentration (µg/mL) by IEX | 434 | 444 | 446 | 448 | 423 |
| ClfA Concentration (µg/mL) by IEX | 121 | 122 | 123 | 121 | 119 |
| CP5 Concentration (µg/mL) by nephelometry | 65 | 62 | 68 | 65 | 68 |
| CP8 Concentration (µg/mL) by nephelometry | 54 | 55 | 55 | 52 | 56 |

| | | | | | |
|---|---|---|---|---|---|
| **NT - Not Tested** | | | | | |

**Table 56: Short-Term Stability Summary of High-Dose MntC (125426-036-C) Using Water as a Diluent**

| | **5°C, t=0** | **5°C, 2days** | **5°C, 7 days** | **25°C, 2 days** | **25°C, 7 days** |
|---|---|---|---|---|---|
| MntC Deamidation (%) by CEX-HPLC | 1.1 | 1.4 | 2.0 | 3.3 | 11.1 |
| MntC Purity (%) by CEX-HPLC | 98.6 | 98.3 | 97.7 | 96.6 | 88.2 |
| MntC Purity (%) by RP-HPLC | 99.3 | 99.2 | 99.3 | 99.3 | 99.2 |
| ClfA Purity (%) by RP-HPLC | 98.1 | 98.2 | 98.6 | 97.8 | 99.0 |
| MntC Concentration (µg/mL) by IEX | 447 | 452 | 429 | 426 | 436 |
| ClfA Concentration (µg/mL) by IEX | 127 | 126 | 122 | 119 | 121 |
| CP5 Concentration (µg/mL) by nephelometry | 67 | 68 | 67 | 66 | 69 |
| CP8 Concentration (µg/mL) by nephelometry | 57 | 57 | 56 | 53 | 57 |

### EXAMPLE 30: TETRA-ANTIGEN DRUG PRODUCT: ALTERNATIVE FORMULATIONS

Stability assays were performed on several different formulations of a tetra-antigen drug product as shown in Table 57.

**Table 57: Tetra-Antigen Drug Product Formulations**

| **Formulation ID** | **30S/mClfA/CP5/CP8 (mg/ml)** | **pH** | **Buffer (mM)** | **Sucrose (mg/ml)** | **Surfactant (mg/ml)** | **Excipient (mg/ml)** |
|---|---|---|---|---|---|---|
| 124369-8-A | .40/.40/.20/.20 | 6.5 | 10 Histidine | 45 | 0.10 mg/ml PS80 | 0 |
| 124369-8-B | .40/.40/.20/.20 | 6.5 | 10 Histidine | 90 | 0.10 mg/ml PS80 | 0 |
| 124369-8-C | .40/.40/.20/.20 | 6.2 | 10 Histidine | 90 | 0.10 mg/ml PS80 | 0 |
| 124369-8-D | .40/.40/.20/.20 | 6.0 | 10 Histidine | 90 | 0.1 mg/ml PS80 | 0 |
| 124369-8-E | .40/.40/.20/.20 | 5.8 | 10 Histidine | 90 | 0.10 mg/ml PS80 | 0 |
| 124369-8-F | .40/.40/.20/.20 | 6.0 | 10 Succinate | 90 | 0.10 mg/ml PS80 | 0 |
| 124369-8-G | .40/.40/.20/.20 | 6.0 | 10 Histidine | 90 | 0.10 mg/ml PS80 | 0.05 mg/mL EDTA |
| 124369-8-H | .40/.40/.20/.20 | 6.0 | 10 Histidine | 90 | 0.10 mg/ml PS80 | 0.1 mg/mL methionine |
| 124369-8-L | .04/.40/.06/.06 | 6.0 | 10 histidine | 90 | 0.10 mg/ml PS80 | 0 |

The lyophilized formulations were reconstituted with 0.7 mL of water and tested for stability at 5°C and 25°C after 1, 3, and 6 months and 40°C after 1 and 3 months. The formulations were compared using the following assays: Karl Fischer moisture, clarity, color, detection of particulates, CEX-HPL for MntC deamidation and purity, RP-HPLC for MntC and ClfA purity, IEX for MntC and ClfA concentration, and nephelometry for conjugate concentration. The percent moisture was higher in the formulation with 45 mg/ml of sucrose. The amount of water in the cake is a percentage of the total mass of the cake. Thus, the 90 mg/ml sucrose cakes were more robust in the percent of moisture due to twice the amount of mass of sucrose. Details of the results are provided in Tables 58-66.

**Table 58: Stability Summary of Tetra-Antigen Formulation 124369-8-A**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 1.6 | NT | 1.9 | 2.0 | NT | 2.3 | 2.4 | NT | 2.8 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | 1 short white fiberlike particle | 1 short white fiberlike particle | 1 short white fiberlike particle, 1 small white round particle, 1 white flakelike particle | Essentially Free of Visible Particles | 1 short white fiberlike particle | Essentially Free of Visible Particles | Essentially Free of Visible Particles | Essentially Free of Visible Particles | 1 white flakelike particle |
| MntC Deamidation (%) by CEX-HPLC | 1.2 | 1.2 | 1.4 | 1.3 | 1.2 | 1.5 | 1.4 | 1.3 | 1.6 |
| MntC Purity (%) by CEX-HPLC | 98.3 | 98.3 | 98.0 | 98.1 | 98.3 | 98.0 | 98.0 | 98.2 | 97.7 |
| MntC Purity (%) by RP-HPLC | 99.7 | 99.2 | 99.2 | 99.3 | 99.1 | 99.2 | 98.9 | 99.3 | 99.4 |
| ClfA Purity (%) by RP-HPLC | 97.2 | 95.3 | 95.0 | 93.2 | 96.4 | 95.0 | 92.9 | 96.2 | 95.0 |
| MntC Concentration (µg/mL) by IEX | 493 | 498 | 476 | 477 | 396 | 488 | 484 | 513 | 484 |
| ClfA Concentration (µg/mL) by IEX | 532 | 541 | 513 | 521 | 422 | 529 | 530 | 545 | 522 |
| CP5 Concentration (µg/mL) by nephelometry | 297 | NT | 279 | 251 | NT | 308 | 243 | NT | 275 |
| CP8 Concentration (µg/mL) by nephelometry | 337 | NT | 381 | 420 | NT | 420 | 383 | NT | 380 |

| | | | | | | | | | |
|---|---|---|---|---|---|---|---|---|---|
| NT - Not Tested NMO RSI - Not More Than EP Reference Standard I | | | | | | | | | |

**Table 59: Stability Summary of Tetra-Antigen Formulation 124369-8-B**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 1.2 | NT | 1.2 | 1.2 | NT | 1.4 | 1.5 | NT | 1.6 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | Essentially Free of Visible Particles | 1 short white fiberlike particle | Essentially Free of Visible Particles | 1 long white fiberlike particle | Essentiall y Free of Visible Particles | 1 short white fiberlike particle | Essentially Free of Visible Particles | Essentially Free of Visible Particles | 1 short white fiberlike particle |
| MntC Deamidation (%) by CEX-HPLC | 1.2 | 1.2 | 1.5 | 1.3 | 1.2 | 1.5 | 1.3 | 1.3 | 1.6 |
| MntC Purity (%) by CEX-HPLC | 98.3 | 98.4 | 98.0 | 98.1 | 98.3 | 98.0 | 98.2 | 98.2 | 97.8 |
| MntC Purity (%) by RP-HPLC | 99.7 | 99.2 | 99.4 | 99.1 | 99.0 | 99.2 | 98.3 | 99.2 | 99.1 |
| ClfA Purity (%) by RP-HPLC | 97.1 | 95.4 | 94.6 | 92.6 | 96.0 | 94.7 | 91.9 | 96.1 | 94.3 |
| MntC Concentration (µg/mL) by IEX | 362 | 361 | 353 | 347 | 405 | 353 | 344 | 350 | 351 |
| ClfA Concentration (µg/mL) by IEX | 396 | 397 | 386 | 383 | 442 | 386 | 380 | 362 | 383 |
| CP5 Concentration (µg/mL) by nephelometry | 220 | NT | 187 | 211 | NT | 197 | 189 | NT | 180 |
| CP8 Concentration (µg/mL) by nephelometry | 246 | NT | 279 | 314 | NT | 298 | 303 | NT | 272 |

| | | | | | | | | | |
|---|---|---|---|---|---|---|---|---|---|
| NT - Not Tested NMO RSI - Not More Than EP Reference Standard I | | | | | | | | | |

**Table 60: Stability Summary of Tetra-Antigen Formulation 124369-8-C**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 0.9 | NT | 1.1 | 1.2 | NT | 1.3 | 1.5 | NT | 1.5 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | Essen tially Free of Visible Particles | 1 short white fiberlike particle | Essentially Free of Visible Particles | Essentially Free of Visible Particles | Essentially Free of Visible Particles | Essentially Free of Visible Particles | Essentially Free of Visible Particles | Essentially Free of Visible Particles | Essentially Free of Visible Particles |
| MntC Deamidation (%) by CEX-HPLC | 1.2 | 1.2 | 1.4 | 1.3 | 1.2 | 1.5 | 1.3 | 1.2 | 1.6 |
| MntC Purity (%) by CEX-HPLC | 98.3 | 98.3 | 98.1 | 98.2 | 98.4 | 98.0 | 98.1 | 98.3 | 97.9 |
| MntC Purity (%) by RP-HPLC | 99.7 | 99.1 | 99.1 | 98.4 | 99.2 | 99.2 | 98.4 | 99.1 | 99.2 |
| ClfA Purity (%) by RP-HPLC | 97.4 | 95.6 | 94.7 | 93.8 | 96.4 | 95.0 | 91.4 | 96.4 | 94.1 |
| MntC Concentration (µg/mL) by IEX | 365 | 371 | 358 | 351 | 367 | 356 | 347 | 353 | 354 |
| ClfA Concentration (µg/mL) by IEX | 394 | 403 | 386 | 384 | 392 | 385 | 376 | 376 | 382 |
| CP5 Concentration (µg/mL) by nephelometry | 218 | NT | 198 | 189 | NT | 198 | 206 | NT | 190 |
| CP8 Concentration (µg/mL) by nephelometry | 236 | NT | 299 | 287 | NT | 301 | 313 | NT | 281 |

| | | | | | | | | | |
|---|---|---|---|---|---|---|---|---|---|
| NT - Not Tested NMO RSI - Not More Than EP Reference Standard I | | | | | | | | | |

**Table 61: Stability Summary of Tetra-Antigen Formulation 124369-8-D**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 1.1 | NT | 1.1 | 1.4 | NT | 1.4 | 1.5 | NT | 1.6 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | Essentially Free of Visible Particles | 2 short white fiberlike particle | 1 short white fiberlike particle | 1 short white fiberlike particle, 1 small white round particle | Essentially Free of Visible Particles | Essentially Free of Visible Particles | 1 short white fiberlike particle 1 small white round particle | 1 short white fiberlike particle | Essentially Free of Visible Particles |
| MntC Deamidation (%) by CEX-HPLC | 1.2 | 1.2 | 1.4 | 1.3 | 1.2 | 1.4 | 1.3 | 1.2 | 1.5 |
| MntC Purity (%) by CEX-HPLC | 98.3 | 98.4 | 98.1 | 98.2 | 98.3 | 98.0 | 98.2 | 98.3 | 97.9 |
| MntC Purity (%) by RP-HPLC | 99.7 | 99.2 | 99.4 | 99.3 | 99.1 | 99.1 | 98.3 | 99.1 | 99.1 |
| ClfA Purity (%) by RP-HPLC | 97.4 | 95.8 | 95.0 | 94.0 | 96.3 | 94.7 | 92.9 | 95.9 | 94.3 |
| MntC Concentration (µg/mL) by IEX | 369 | 357 | 355 | 350 | 360 | 353 | 348 | 349 | 353 |
| ClfA Concentration (µg/mL) by IEX | 396 | 389 | 384 | 386 | 384 | 383 | 383 | 371 | 382 |
| CP5 Concentration (µg/mL) by nephelometry | 215 | NT | 196 | 205 | NT | 210 | 191 | NT | 178 |
| CP8 Concentration (µg/mL) by nephelometry | 247 | NT | 293 | 320 | NT | 300 | 293 | NT | 262 |

| | | | | | | | | | |
|---|---|---|---|---|---|---|---|---|---|
| NT - Not Tested NMO RSI - Not More Than EP Reference Standard I | | | | | | | | | |

**Table 62: Stability Summary of Tetra-Antigen Formulation 124369-8-E**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 1.1 | NT | 1.2 | 1.2 | NT | 1.4 | 1.5 | NT | 1.6 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | Essentially Free of Visible Particles | Essentially Free of Visible Particles | Essentially Free of Visible Particles | Essentially Free of Visible Particles | 1 short white fiberlike particle | Essentially Free of Visible Particles | 1 medium dark fiberlike particle | Essentially Free of Visible Particles | Essentially Free of Visible Particles |
| MntC Deamidation (%) by CEX-HPLC | 1.2 | 1.2 | 1.4 | 1.3 | 1.2 | 1.4 | 1.3 | 1.2 | 1.5 |
| MntC Purity (%) by CEX-HPLC | 98.4 | 98.3 | 98.1 | 98.2 | 98.3 | 98.1 | 98.1 | 98.3 | 97.9 |
| MntC Purity (%) by RP-HPLC | 99.6 | 99.4 | 99.4 | 98.3 | 99.2 | 99.2 | 98.2 | 99.1 | 99.1 |
| ClfA Purity (%) by RP-HPLC | 97.1 | 96.0 | 94.8 | 93.6 | 96.3 | 94.7 | 91.6 | 96.5 | 94.8 |
| MntC Concentration (µg/mL) by IEX | 362 | 356 | 353 | 338 | 350 | 349 | 344 | 363 | 351 |
| ClfA Concentration (µg/mL) by IEX | 420 | 413 | 409 | 400 | 399 | 403 | 401 | 412 | 405 |
| CP5 Concentration (µg/mL) by nephelometry | 215 | NT | 185 | 187 | NT | 197 | 187 | NT | 168 |
| CP8 Concentration (µg/mL) by nephelometry | 226 | NT | 288 | 286 | NT | 298 | 292 | NT | 261 |

| | | | | | | | | | |
|---|---|---|---|---|---|---|---|---|---|
| NT - Not Tested NMO RSI - Not More Than EP Reference Standard I | | | | | | | | | |

**Table 63: Stability Summary of Tetra-Antigen Formulation 124369-8-F**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 1.2 | NT | 1.4 | 1.3 | NT | 1.5 | 1.6 | NT | 1.7 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | Essentially Free of Visible Particles | 1 short white fiberlike particle | 1 small white round particle | 1 short dark fiberlike particle | 1 short white fiberlike particle | Essentially Free of Visible Particles | 1 short white fiberlike particle | 1 short white fiberlike particle | Essentially Free of Visible Particles |
| MntC Deamidation (%) by CEX-HPLC | 1.3 | 1.2 | 1.4 | 1.3 | 1.3 | 1.4 | 1.4 | 1.3 | 1.6 |
| MntC Purity (%) by CEX-HPLC | 98.2 | 98.3 | 98.1 | 98.1 | 98.3 | 98.0 | 98.1 | 98.2 | 97.8 |
| MntC Purity (%) by RP-HPLC | 99.7 | 99.1 | 97.2 | 98.4 | 99.1 | 99.2 | 98.4 | 99.1 | 99.3 |
| ClfA Purity (%) by RP-HPLC | 97.4 | 95.8 | 94.9 | 93.7 | 96.4 | 95.2 | 92.5 | 96.4 | 95.0 |
| MntC Concentration (µg/mL) by IEX | 364 | 358 | 353 | 345 | 359 | 351 | 344 | 354 | 359 |
| ClfA Concentration (µg/mL) by IEX | 392 | 382 | 384 | 378 | 378 | 378 | 375 | 362 | 384 |
| CP5 Concentration (µg/mL) by nephelometry | NT | NT | 188 | 184 | NT | 189 | 198 | NT | 180 |
| CP8 Concentration (µg/mL) by nephelometry | NT | NT | 283 | 280 | NT | 275 | 301 | NT | 283 |

| | | | | | | | | | |
|---|---|---|---|---|---|---|---|---|---|
| NT - Not Tested NMO RSI - Not More Than EP Reference Standard I | | | | | | | | | |

**Table 64: Stability Summary of Tetra-Antigen Formulation 124369-8-G**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 1.2 | NT | 1.3 | 1.4 | NT | 1.5 | 1.7 | NT | 1.7 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | 2 small white round and 1 white flakelike particles | Essentially Free of Visible Particles | Essentially Free of Visible Particles | Essentially Free of Visible Particles | Essentially Free of Visible Particles | 1 long white fiberlike particle, 1 short red fiberlike particle | 1 short white fiberlike particle ,2 small white round particles | 1 short white fiberlike particle | 1 short white fiberlike particle, 1 small white round particle |
| MntC Deamidation (%) by CEX-HPLC | 1.2 | 1.2 | 1.4 | 1.3 | 1.2 | 1.4 | 1.3 | 1.2 | 1.5 |
| MntC Purity (%) by CEX-HPLC | 98.3 | 98.3 | 98.1 | 98.2 | 98.4 | 98.1 | 98.1 | 98.4 | 98.0 |
| MntC Purity (%) by RP-HPLC | 99.7 | 99.1 | 99.0 | 98.4 | 99.2 | 99.2 | 98.4 | 99.5 | 99.1 |
| ClfA Purity (%) by RP-HPLC | 96.9 | 95.9 | 94.6 | 93.4 | 96.4 | 95.0 | 92.0 | 96.0 | 95.5 |
| MntC Concentration (µg/mL) by IEX | 363 | 357 | 355 | 346 | 363 | 352 | 339 | 361 | 354 |
| ClfA Concentration (µg/mL) by IEX | 405 | 397 | 396 | 393 | 395 | 373 | 384 | 383 | 394 |
| CP5 Concentration (µg/mL) by nephelometry | 223 | NT | 191 | 192 | NT | 203 | 194 | NT | 187 |
| CP8 Concentration (µg/mL) by nephelometry | 238 | NT | 295 | 293 | NT | 308 | 305 | NT | 277 |

| | | | | | | | | | |
|---|---|---|---|---|---|---|---|---|---|
| NT - Not Tested NMO RSI - Not More Than EP Reference Standard I | | | | | | | | | |

**Table 65: Stability Summary of Tetra-Antigen Formulation 124369-8-H**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 1.1 | NT | 1.3 | 1.3 | NT | 1.5 | 1.8 | NT | 1.7 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | Essentially Free of Visible Particles | 1 short white sliverlike particle | Essentially Free of Visible Particles | Essentially Free of Visible Particles | Essentially Free of Visible Particles | Essentially Free of Visible Particles | 1 short white fiberlike particle , 1 white flakelike particle | 1 white flakelike particle | Essentially Free of Visible Particles |
| MntC Deamidation (%) by CEX-HPLC | 1.2 | 1.2 | 1.4 | 1.3 | 1.2 | 1.4 | 1.4 | 1.2 | 1.5 |
| MntC Purity (%) by CEX-HPLC | 98.3 | 98.3 | 98.1 | 98.2 | 98.3 | 98.1 | 98.1 | 98.3 | 98.0 |
| MntC Purity (%) by RP-HPLC | 99.7 | 99.2 | 99.2 | 99.0 | 99.2 | 99.2 | 98.4 | 99.4 | NT |
| ClfA Purity (%) by RP-HPLC | 97.1 | 96.3 | 95.3 | 93.4 | 96.6 | 95.3 | 92.1 | 96.6 | NT |
| MntC Concentration (µg/mL) by IEX | 366 | 357 | 345 | 342 | 355 | 355 | 346 | 351 | 352 |
| ClfA Concentration (µg/mL) by IEX | 409 | 398 | 387 | 393 | 390 | 398 | 389 | 375 | 395 |
| CP5 Concentration (µg/mL) by nephelometry | 213 | NT | 182 | 188 | NT | 208 | 188 | NT | 206 |
| CP8 Concentration (µg/mL) by nephelometry | 233 | NT | 262 | 286 | NT | 310 | 283 | NT | 291 |

| | | | | | | | | | |
|---|---|---|---|---|---|---|---|---|---|
| NT - Not Tested NMO RSI - Not More Than EP Reference Standard I | | | | | | | | | |

**Table 66: Stability Summary of Tetra-Antigen Formulation 124369-8-L**

| | **5°C, t=0** | **5°C, 1 mo.** | **5°C, 3 mos.** | **5°C, 6 mos.** | **25°C, 1 mo.** | **25°C, 3 mos.** | **25°C, 6 mos.** | **40°C, 1 mo.** | **40°C, 3 mos.** |
|---|---|---|---|---|---|---|---|---|---|
| Karl Fischer Moisture (% H₂0) | 1.0 | NT | 0.9 | 1.0 | NT | 1.1 | 1.4 | NT | 1.4 |
| Clarity | NMO RSI | NMO RSI | NMO RSI | NMO RSI | NMO RSI | NMO RSI | NMO RSI | NMO RSI | NMO RSI |
| Color | Colorless | Colorless | Colorless | Colorless | Colorless | Colorless | Colorless | Colorless | Colorless |
| Particulates | Essentially Free of Visible Particles | Essentially Free of Visible Particles | 1 small white round particle | Essentially Free of Visible Particles | Essentially Free of Visible Particles | Essentially Free of Visible Particles | Essentially Free of Visible Particles | 1 short white fiberlike particle, 1 white flakelike particle | Essentially Free of Visible Particles |
| MntC Deamidation (%) by CEX-HPLC | 1.7 | 1.7 | 1.9 | 1.7 | 1.7 | 2.0 | 1.7 | 1.7 | 2.0 |
| MntC Purity (%) by CEX-HPLC | 97.7 | 97.9 | 97.5 | 97.8 | 97.9 | 97.5 | 97.9 | 97.9 | 97.5 |
| MntC Purity (%) by RP-HPLC | 98.5 | 98.5 | 99.0 | 98.9 | 98.4 | 98.9 | 98.9 | 98.4 | 99.0 |
| ClfA Purity (%) by RP-HPLC | 97.0 | 96.2 | 94.8 | 94.7 | 96.2 | 95.2 | 93.6 | 96.1 | 95.1 |
| MntC Concentration (µg/mL) by IEX | 32 | 32 | 31 | 30 | 31 | 31 | 30 | 31 | 31 |
| ClfA Concentration (µg/mL) by IEX | 422 | 435 | 417 | 416 | 419 | 418 | 414 | 410 | 419 |
| CP5 Concentration (µg/mL) by nephelometry | 57 | NT | 53 | 56 | NT | 53 | 51 | NT | 55 |
| CP8 Concentration (µg/mL) by nephelometry | 64 | NT | 80 | 86 | NT | 79 | 77 | NT | 80 |

| | | | | | | | | | |
|---|---|---|---|---|---|---|---|---|---|
| NT - Not Tested NMO RSI - Not More Than EP Reference Standard I | | | | | | | | | |

## Claims

1. A liquid immunogenic composition prepared by reconstituting a lyophilized immunogenic composition comprising: (a) an isolated *Staphylococcus aureus* clumping factor A (ClfA) polypeptide, wherein the ClfA polypeptide comprises an N1, N2 and N3 domain, (b) an isolated *Staphylococcus aureus* MntC polypeptide, (c) a Capsular Polysaccharide Type 5 (CP5)-CRM₁₉₇ conjugate, (d) a Capsular Polysaccharide Type 8 (CP8)-CRM₁₉₇ conjugate, (e) a buffer having a pKa of 6.0 ± 0.6, and (f) a bulking agent, with an aqueous diluent, wherein said liquid immunogenic composition has a final pH of 6.0 ± 0.5.

2. The liquid immunogenic composition of claim 1, wherein the liquid immunogenic composition comprises:
(a) the ClfA polypeptide at a concentration of between 40 µg/ml ± 4µg/ml and 800µg/ml ± 80µg/ml;
(b) the CP5- CRM₁₉₇ conjugate at a concentration of between 20µg/ml ± 2µg/ml and 400µg/ml ± 40µg/ml;
(c) the CP8- CRM₁₉₇ conjugate at a concentration of between 20µg/ml ± 2µg/ml and 400µg/ml ± 40µg/ml;
(d) histidine buffer at a concentration of 10mM ± 5mM;
(e) polysorbate 80 at a concentration of 0.01% ± 0.005% weight to volume (w/v); and
(f) sucrose at a concentration of 9% ± 4.5% w/v.

3. The liquid immunogenic composition of any one of claims 1-2, wherein the liquid immunogenic composition further comprises an isolated *Staphylococcus aureus* clumping factor B (ClfB) polypeptide at a concentration of between 40 µg/ml ± 4µg/ml and 800µg/ml ± 80µg/ml.

4. The liquid immunogenic composition of any one of claims 1-3, wherein the fibrinogen binding domain of the ClfA polypeptide displays reduced binding to fibrinogen through having an amino acid substitution at one or more of Tyr 338, Tyr 256, Pro 336, Lys 389, Ala 254 and Ile 387.

5. The liquid immunogenic composition of claim 4, wherein the amino acid substitution at one or more of Tyr 338, Tyr 256, Pro 336, Lys 389, Ala 254 and Ile 387 is to Ala or Ser.

6. The liquid immunogenic composition of claim 5, wherein the Tyr 338 is substituted to Ala.

## Patentansprüche

1. Flüssige immunogene Zusammensetzung, die durch Rekonstituieren einer lyophilisierten immunogenen Zusammensetzung, welche umfasst: (a) ein isoliertes *Staphylococcus-aureus-Ver*klumpungsfaktor-A (ClfA)-Polypeptid, wobei das ClfA-Polypeptid eine N1-, N2- und N3-Domäne umfasst, (b) ein isoliertes *Staphylococcus-aureus-MntC-Polypeptid,* (c) ein Kapselpolysaccharid-Typ-5 (CP5)-CRM₁₉₇-Konjugat, (d) ein Kapselpolysaccharid-Typ-8 (CP8)-CRM₁₉₇-Konjugat, (e) einen Puffer, der einen pKa von 6,0 ± 0,6 hat, und (f) einen Füllstoff, mit einem wässrigen Verdünnungsmittel hergestellt wird, wobei die flüssige immunogene Zusammensetzung einen End-pH von 6,0 ± 0,5 hat.

2. Flüssige immunogene Zusammensetzung gemäß Anspruch 1, wobei die flüssige immunogene Zusammensetzung umfasst:
(a) das ClfA-Polypeptid mit einer Konzentration von zwischen 40 µg/ml ± 4 µg/ml und 800 µg/ml ± 80 µg/ml;
(b) das CP5-CRM₁₉₇-Konjugat mit einer Konzentration von zwischen 20 µg/ml ± 2 µg/ml und 400 µg/ml ± 40 µg/ml;
(c) das CP8-CRM₁₉₇-Konjugat mit einer Konzentration von zwischen 20 µg/ml ± 2 µg/ml und 400 µg/ml ± 40 µg/ml;
(d) Histidinpuffer mit einer Konzentration von 10 mM ± 5 mM;
(e) Polysorbat 80 mit einer Konzentration von 0,01% ± 0,005% Gewicht zu Volumen (G/V) und
(f) Saccharose mit einer Konzentration von 9% ± 4,5% G/V.

3. Flüssige immunogene Zusammensetzung gemäß einem der Ansprüche 1-2, wobei die flüssige immunogene Zusammensetzung außerdem ein isoliertes *Staphylococcus-aureus*-Verklumpungsfaktor-B (ClfB)-Polypeptid mit einer Konzentration von zwischen 40 µg/ml ± 4 µg/ml und 800 µg/ml ± 80 µg/ml umfasst.

4. Flüssige immunogene Zusammensetzung gemäß einem der Ansprüche 1-3, wobei die Fibrinogen-bindende Domäne des ClfA-Polypeptids dadurch eine verringerte Bindung an Fibrinogen zeigt, dass sie eine Aminosäuresubstitution an einer oder mehreren von Tyr 338, Tyr 256, Pro 336, Lys 389, Ala 254 und Ile 387 hat.

5. Flüssige immunogene Zusammensetzung gemäß Anspruch 4, wobei die Aminosäuresubstitution an einer oder mehreren von Tyr 338, Tyr 256, Pro 336, Lys 389, Ala 254 und Ile 387 zu Ala oder Ser ist.

6. Flüssige immunogene Zusammensetzung gemäß Anspruch 5, wobei das Tyr 338 zu Ala substituiert ist.

## Revendications

1. Composition immunogène liquide préparée par la reconstitution d'une composition immunogène lyophilisée comprenant : (a) un polypeptide isolé du facteur d'agglutination A (ClfA) de *Staphylococcus aureus,* dans laquelle le polypeptide ClfA comprend un domaine N1, N2 et N3, (b) un polypeptide MntC isolé de *Staphylococcus aureus,* (c) un conjugué polysaccharide capsulaire de type 5 (CP5)-CRM₁₉₇, (d) un conjugué polysaccharide capsulaire de type 8 (CP8)-CRM₁₉₇, (e) un tampon ayant un pKa de 6,0 ± 0,6, et (f) un agent de remplissage, avec un diluant aqueux, dans laquelle ladite composition immunogène liquide présente un pH final de 6,0 ± 0,5.

2. Composition immunogène liquide selon la revendication 1, dans laquelle la composition immunogène liquide comprend :
(a) le polypeptide ClfA à une concentration située entre 40 µg/ml ± 4 µg/ml et 800 µg/ml ± 80 µg/ml ;
(b) le conjugué CP5-CRM₁₉₇ à une concentration située entre 20 µg/ml ± 2 µg/ml et 400 µg/ml ± 40 µg/ml ;
(c) le conjugué CP8-CRM₁₉₇ à une concentration située entre 20 µg/ml ± 2 µg/ml et 400 µg/ml ± 40 µg/ml ;
(d) un tampon à l'histidine à une concentration de 10 mM ± 5 mM ;
(e) du polysorbate 80 à une concentration de 0,01 % ± 0,005 % en poids sur volume (poids/volume) ; et
(f) du saccharose à une concentration de 9 % ± 4,5 % en poids/volume.

3. Composition immunogène liquide selon l'une quelconque des revendications 1 et 2, dans laquelle la composition immunogène liquide comprend en outre un polypeptide isolé du facteur d'agglutination B (ClfB) de *Staphylococcus aureus* à une concentration située entre 40 µg/ml ± 4 µg/ml et 800 µg/ml ± 80 µg/ml.

4. Composition immunogène liquide selon l'une quelconque des revendications 1 à 3, dans laquelle le domaine de liaison au fibrinogène du polypeptide ClfA présente une liaison réduite au fibrinogène à cause d'une substitution d'acide aminé au niveau d'un ou de plusieurs parmi Tyr 338, Tyr 256, Pro 336, Lys 389, Ala 254 et Ile 387.

5. Composition immunogène liquide selon la revendication 4, dans laquelle la substitution d'acide aminé au niveau d'un ou de plusieurs parmi Tyr 338, Tyr 256, Pro 336, Lys 389, Ala 254 et Ile 387 est pour Ala ou Ser.

6. Composition immunogène liquide selon la revendication 5, dans laquelle le Tyr 338 est substitué pour Ala.


### SEQUENCE LISTING

<110> Wyeth LLC
<120> STABLE IMMUNOGENIC COMPOSITIONS OF STAPHYLOCOCCUS AUREUS
<130> PC057927A
<150> 61/426,476
   <151> 2010-12-22
<160> 137
<170> PatentIn version 3.5
<210> 1
   <211> 927
   <212> DNA
   <213> Staphylococcus aureus
<400> 1
   atgaaaaaat tagtaccttt attattagcc ttattacttc tagttgctgc atgtggtact        60
   ggtggtaaac aaagcagtga taagtcaaat ggcaaattaa aagtagtaac gacgaattca       120
   attttatatg atatggctaa aaatgttggt ggagacaacg tcgatattca tagtattgta       180
   cctgttggtc aagatcctca tgaatatgaa gttaaaccta aagatattaa aaagttaact       240
   gacgctgacg ttattttata caacggatta aatttagaga ctggtaacgg ttggtttgaa       300
   aaagccttag aacaggctgg taaatcatta aaagataaaa aagttatcgc agtatcaaaa       360
   gatgttaaac ctatctattt aaacggtgaa gaaggcaaca aagataaaca agatccacac       420
   gcatggttaa gtttagataa cggtattaaa tacgtaaaaa caattcaaca aacatttatc       480
   gataacgaca aaaaacataa agcagattat gaaaagcaag gtaacaaata cattgctcaa       540
   ttggaaaaat taaataatga cagtaaagac aaatttaatg acattccaaa agaacaacgt       600
   gccatgatta caagtgaagg tgccttcaag tacttctcaa aacaatacgg tattacacca       660
   ggttatattt gggaaattaa cactgaaaaa caaggtacac cagaacaaat gagacaagct       720
   attgagtttg ttaaaaagca caaattaaaa cacttattag tagaaacaag tgttgataag       780
   aaagcaatgg aaagtttatc tgaagaaacg aagaaagata tctttggtga agtgtacaca       840
   gattcaatcg gtaaagaagg cactaaaggt gactcttact acaaaatgat gaaatcaaat       900
   attgaaactg tacacggaag catgaaa                                           927
<210> 2
   <211> 309
   <212> PRT
   <213> Staphylococcus aureus
<400> 2
   Met Lys Lys Leu Val Pro Leu Leu Leu Ala Leu Leu Leu Leu Val Ala
   1               5                   10                  15
   Ala Cys Gly Thr Gly Gly Lys Gln Ser Ser Asp Lys Ser Asn Gly Lys
   20 25 30
   Leu Lys Val Val Thr Thr Asn Ser Ile Leu Tyr Asp Met Ala Lys Asn
   35 40 45
   Val Gly Gly Asp Asn Val Asp Ile His Ser Ile Val Pro Val Gly Gln
   50 55 60
   Asp Pro His Glu Tyr Glu Val Lys Pro Lys Asp Ile Lys Lys Leu Thr
   65 70 75 80
   Asp Ala Asp Val Ile Leu Tyr Asn Gly Leu Asn Leu Glu Thr Gly Asn
   85 90 95
   Gly Trp Phe Glu Lys Ala Leu Glu Gln Ala Gly Lys Ser Leu Lys Asp
   100 105 110
   Lys Lys Val Ile Ala Val Ser Lys Asp Val Lys Pro Ile Tyr Leu Asn
   115 120 125
   Gly Glu Glu Gly Asn Lys Asp Lys Gln Asp Pro His Ala Trp Leu Ser
   130 135 140
   Leu Asp Asn Gly Ile Lys Tyr Val Lys Thr Ile Gln Gln Thr Phe Ile
   145 150 155 160
   Asp Asn Asp Lys Lys His Lys Ala Asp Tyr Glu Lys Gln Gly Asn Lys
   165 170 175
   Tyr Ile Ala Gln Leu Glu Lys Leu Asn Asn Asp Ser Lys Asp Lys Phe
   180 185 190
   Asn Asp Ile Pro Lys Glu Gln Arg Ala Met Ile Thr Ser Glu Gly Ala
   195 200 205
   Phe Lys Tyr Phe Ser Lys Gln Tyr Gly Ile Thr Pro Gly Tyr Ile Trp
   210 215 220
   Glu Ile Asn Thr Glu Lys Gln Gly Thr Pro Glu Gln Met Arg Gln Ala
   225 230 235 240
   Ile Glu Phe Val Lys Lys His Lys Leu Lys His Leu Leu Val Glu Thr
   245 250 255
   Ser Val Asp Lys Lys Ala Met Glu Ser Leu Ser Glu Glu Thr Lys Lys
   260 265 270
   Asp Ile Phe Gly Glu Val Tyr Thr Asp Ser Ile Gly Lys Glu Gly Thr
   275 280 285
   Lys Gly Asp Ser Tyr Tyr Lys Met Met Lys Ser Asn Ile Glu Thr Val
   290 295 300
   His Gly Ser Met Lys
   305
<210> 3
   <211> 927
   <212> DNA
   <213> Staphylococcus aureus
<400> 3
   atgaaaaaat tagtaccttt attattagcc ttattacttc tagttgctgc atgtggtact 60
   ggtggtaaac aaagcagtga taagtcaaat ggcaaattaa aagtagtaac gacgaattca 120
   attttatatg atatggctaa aaatgttggt ggagacaacg tcgatattca tagtattgta 180
   cctgttggtc aagatcctca tgaatatgaa gttaaaccta aagatattaa aaagttaact 240
   gacgctgaca ttattttata caacggatta aatttagaga ctggtaacgg ttggtttgaa 300
   aaagccttag aacaggctgg taaatcatta aaagataaaa aagttatcgc agtatcaaaa 360
   gatgttaaac ctatctattt aaacggtgaa gaaggcaaca aagataaaca agatccacac 420
   gcatggttaa gtttagataa tggtattaaa tacgtaaaaa caattcaaca aacatttatc 480
   gataacgaca aaaaacataa agcagattat gaaaagcaag gtaacaaata cattgctcaa 540
   ttggaaaaat taaataatga cagtaaagac aaatttaatg acattccaaa agaacaacgt 600
   gccatgatta caagtgaagg tgccttcaag tacttctcaa aacaatacgg tattacacca 660
   ggttatattt gggaaattaa cactgaaaaa caaggtacac ctgaacaaat gagacaagct 720
   attgagtttg ttaaaaagca caaattaaaa cacttattag tagaaacaag tgttgataag 780
   aaagcaatgg aaagtttatc tgaagaaacg aagaaagata tctttggtga agtgtacaca 840
   gattcaatcg gtaaagaagg cactaaaggt gactcttatt acaaaatgat gaaatcaaat 900
   attgaaactg tacacggaag catgaaa 927
<210> 4
   <211> 309
   <212> PRT
   <213> Staphylococcus aureus
<400> 4
   Met Lys Lys Leu Val Pro Leu Leu Leu Ala Leu Leu Leu Leu Val Ala
   1               5                   10                  15
   Ala Cys Gly Thr Gly Gly Lys Gln Ser Ser Asp Lys Ser Asn Gly Lys
               20                  25                  30
   Leu Lys Val Val Thr Thr Asn Ser Ile Leu Tyr Asp Met Ala Lys Asn
           35                  40                  45
   Val Gly Gly Asp Asn Val Asp Ile His Ser Ile Val Pro Val Gly Gln
       50                  55                  60
   Asp Pro His Glu Tyr Glu Val Lys Pro Lys Asp Ile Lys Lys Leu Thr
   65                  70                  75                  80
   Asp Ala Asp Ile Ile Leu Tyr Asn Gly Leu Asn Leu Glu Thr Gly Asn
                   85                  90                  95
   Gly Trp Phe Glu Lys Ala Leu Glu Gln Ala Gly Lys Ser Leu Lys Asp
               100                 105                 110
   Lys Lys Val Ile Ala Val Ser Lys Asp Val Lys Pro Ile Tyr Leu Asn
           115                 120                 125
   Gly Glu Glu Gly Asn Lys Asp Lys Gln Asp Pro His Ala Trp Leu Ser
       130                 135                 140
   Leu Asp Asn Gly Ile Lys Tyr Val Lys Thr Ile Gln Gln Thr Phe Ile
   145                 150                 155                 160
   Asp Asn Asp Lys Lys His Lys Ala Asp Tyr Glu Lys Gln Gly Asn Lys
                   165                 170                 175
   Tyr Ile Ala Gln Leu Glu Lys Leu Asn Asn Asp Ser Lys Asp Lys Phe
               180                 185                 190
   Asn Asp Ile Pro Lys Glu Gln Arg Ala Met Ile Thr Ser Glu Gly Ala
           195                 200                 205
   Phe Lys Tyr Phe Ser Lys Gln Tyr Gly Ile Thr Pro Gly Tyr Ile Trp
       210                 215                 220
   Glu Ile Asn Thr Glu Lys Gln Gly Thr Pro Glu Gln Met Arg Gln Ala
   225                 230                 235                 240
   Ile Glu Phe Val Lys Lys His Lys Leu Lys His Leu Leu Val Glu Thr
                   245                 250                 255
   Ser Val Asp Lys Lys Ala Met Glu Ser Leu Ser Glu Glu Thr Lys Lys
   260 265 270
   Asp Ile Phe Gly Glu Val Tyr Thr Asp Ser Ile Gly Lys Glu Gly Thr
   275 280 285
   Lys Gly Asp Ser Tyr Tyr Lys Met Met Lys Ser Asn Ile Glu Thr Val
   290 295 300
   His Gly Ser Met Lys
   305
<210> 5
   <211> 936
   <212> DNA
   <213> Staphylococcus aureus
<400> 5
   atgaaaaaat tagtaccttt attattagcc ttattacttc tagttgctgc atgtggtact      60
   ggtggtaaac aaagcagtga taagtcaaat ggcaaattaa aagtagtaac gacgaattca     120
   attttatatg atatggctaa aaatgttggt ggagacaacg tcgatattca tagtattgta     180
   cctgttggtc aagatcctca tgaatatgaa gttaaaccta aagatattaa aaagttaact     240
   gacgctgacg ttattttata caacggatta aatttagaga ctggtaacgg ttggtttgaa     300
   aaagccttag aacaggctgg taaatcatta aaagataaaa aagttatcgc agtatcaaaa     360
   gatgttaaac ctatctattt aaacggtgaa gaaggcaaca aagataaaca agatccacac     420
   gcatggttaa gtttagataa cggtattaaa tacgtaaaaa caattcaaca aacatttatc     480
   gataacgaca aaaaacataa agcagattat gaaaagcaag gtaacaaata cattgctcaa     540
   ttggaaaaat taaataatga cagtaaagac agtaaagaca aatttaatga cattccaaaa     600
   gaacaacgtg ccatgattac aagtgaaggt gccttcaagt acttctcaaa acaatacggt     660
   attacaccag gttatatttg ggaaattaac actgaaaaac aaggtacacc tgaacaaatg     720
   agacaagcta ttgagtttgt taaaaagcac aaattaaaac acttattagt agaaacaagt     780
   gttgataaga aagcaatgga aagtttatct gaagaaacga agaaagatat ctttggtgaa     840
   gtgtacacag attcaatcgg taaagaaggc actaaaggtg actcttacta caaaatgatg     900
   aaatcaaata ttgaaactgt acacggaagc atgaaa                               936
<210> 6
   <211> 312
   <212> PRT
   <213> Staphylococcus aureus
<400> 6
   Met Lys Lys Leu Val Pro Leu Leu Leu Ala Leu Leu Leu Leu Val Ala
   1               5                   10                  15
   Ala Cys Gly Thr Gly Gly Lys Gln Ser Ser Asp Lys Ser Asn Gly Lys
   20 25 30
   Leu Lys Val Val Thr Thr Asn Ser Ile Leu Tyr Asp Met Ala Lys Asn
   35 40 45
   Val Gly Gly Asp Asn Val Asp Ile His Ser Ile Val Pro Val Gly Gln
   50 55 60
   Asp Pro His Glu Tyr Glu Val Lys Pro Lys Asp Ile Lys Lys Leu Thr
   65 70 75 80
   Asp Ala Asp Val Ile Leu Tyr Asn Gly Leu Asn Leu Glu Thr Gly Asn
   85 90 95
   Gly Trp Phe Glu Lys Ala Leu Glu Gln Ala Gly Lys Ser Leu Lys Asp
   100 105 110
   Lys Lys Val Ile Ala Val Ser Lys Asp Val Lys Pro Ile Tyr Leu Asn
   115 120 125
   Gly Glu Glu Gly Asn Lys Asp Lys Gln Asp Pro His Ala Trp Leu Ser
   130 135 140
   Leu Asp Asn Gly Ile Lys Tyr Val Lys Thr Ile Gln Gln Thr Phe Ile
   145 150 155 160
   Asp Asn Asp Lys Lys His Lys Ala Asp Tyr Glu Lys Gln Gly Asn Lys
   165 170 175
   Tyr Ile Ala Gln Leu Glu Lys Leu Asn Asn Asp Ser Lys Asp Ser Lys
   180 185 190
   Asp Lys Phe Asn Asp Ile Pro Lys Glu Gln Arg Ala Met Ile Thr Ser
   195 200 205
   Glu Gly Ala Phe Lys Tyr Phe Ser Lys Gln Tyr Gly Ile Thr Pro Gly
   210 215 220
   Tyr Ile Trp Glu Ile Asn Thr Glu Lys Gln Gly Thr Pro Glu Gln Met
   225 230 235 240
   Arg Gln Ala Ile Glu Phe Val Lys Lys His Lys Leu Lys His Leu Leu
   245 250 255
   Val Glu Thr Ser Val Asp Lys Lys Ala Met Glu Ser Leu Ser Glu Glu
   260 265 270
   Thr Lys Lys Asp Ile Phe Gly Glu Val Tyr Thr Asp Ser Ile Gly Lys
   275 280 285
   Glu Gly Thr Lys Gly Asp Ser Tyr Tyr Lys Met Met Lys Ser Asn Ile
   290 295 300
   Glu Thr Val His Gly Ser Met Lys
   305 310
<210> 7
   <211> 927
   <212> DNA
   <213> Staphylococcus aureus
<400> 7
   atgaaaaaat tagtaccttt attattagcc ttattacttt tagttgctgc atgtggtact 60
   ggtggtaaac aaagcagtga taagtcaaat ggcaaactaa aagtagtaac gacgaattca 120
   attttatatg atatggctaa aaatgttggt ggagacaacg tcgatattca tagtattgta 180
   cctgttggtc aagatcctca tgaatatgaa gttaaaccta aagatattaa aaagttaact 240
   gacgctgacg ttattttata caacggatta aatttagaga ctggtaacgg ttggtttgaa 300
   aaagccttag aacaggctgg taaatcatta aaagataaaa aagttatcgc agtatcaaaa 360
   gatgttaaac ctatctattt aaacggtgaa gaaggcaaca aagataaaca agatccacac 420
   gcatggttaa gtttagataa cggtattaaa tacgtaaaaa caattcaaca aacatttatc 480
   gataacgaca aaaaacataa agcagattat gaaaagcaag gtaacaaata cattgctcaa 540
   ttggaaaaat taaataatga cagtaaagac aaatttaatg acattccaaa agaacaacgt 600
   gccatgatta caagtgaagg tgccttcaaa tacttctcaa aacaatacgg tattacacca 660
   ggttatattt gggaaattaa cactgaaaaa caaggtacac cagaacaaat gagacaagct 720
   attgagtttg ttaaaaaaca caaattaaaa cacttattag tagaaacaag tgttgataag 780
   aaagcaatgg aaagtttatc tgaagaaacg aagaaagata tctttggtga agtgtacaca 840
   gattcaatcg gtaaagaagg cactaaaggt gactcttact acaaaatgat gaaatcaaat 900
   attgatactg tacacggaag catgaaa 927
<210> 8
   <211> 309
   <212> PRT
   <213> Staphylococcus aureus
<400> 8
   Met Lys Lys Leu Val Pro Leu Leu Leu Ala Leu Leu Leu Leu Val Ala
   1               5                   10                  15
   Ala Cys Gly Thr Gly Gly Lys Gln Ser Ser Asp Lys Ser Asn Gly Lys
               20                  25                  30
   Leu Lys Val Val Thr Thr Asn Ser Ile Leu Tyr Asp Met Ala Lys Asn
           35                  40                  45
   Val Gly Gly Asp Asn Val Asp Ile His Ser Ile Val Pro Val Gly Gln
       50                  55                  60
   Asp Pro His Glu Tyr Glu Val Lys Pro Lys Asp Ile Lys Lys Leu Thr
   65                  70                  75                  80
   Asp Ala Asp Val Ile Leu Tyr Asn Gly Leu Asn Leu Glu Thr Gly Asn
                   85                  90                  95
   Gly Trp Phe Glu Lys Ala Leu Glu Gln Ala Gly Lys Ser Leu Lys Asp
               100                 105                 110
   Lys Lys Val Ile Ala Val Ser Lys Asp Val Lys Pro Ile Tyr Leu Asn
           115                 120                 125
   Gly Glu Glu Gly Asn Lys Asp Lys Gln Asp Pro His Ala Trp Leu Ser
       130                 135                 140
   Leu Asp Asn Gly Ile Lys Tyr Val Lys Thr Ile Gln Gln Thr Phe Ile
   145                 150                 155                 160
   Asp Asn Asp Lys Lys His Lys Ala Asp Tyr Glu Lys Gln Gly Asn Lys
                   165                 170                 175
   Tyr Ile Ala Gln Leu Glu Lys Leu Asn Asn Asp Ser Lys Asp Lys Phe
               180                 185                 190
   Asn Asp Ile Pro Lys Glu Gln Arg Ala Met Ile Thr Ser Glu Gly Ala
           195                 200                 205
   Phe Lys Tyr Phe Ser Lys Gln Tyr Gly Ile Thr Pro Gly Tyr Ile Trp
       210                 215                 220
   Glu Ile Asn Thr Glu Lys Gln Gly Thr Pro Glu Gln Met Arg Gln Ala
   225                 230                 235                 240
   Ile Glu Phe Val Lys Lys His Lys Leu Lys His Leu Leu Val Glu Thr
                   245                 250                 255
   Ser Val Asp Lys Lys Ala Met Glu Ser Leu Ser Glu Glu Thr Lys Lys
   260 265 270
   Asp Ile Phe Gly Glu Val Tyr Thr Asp Ser Ile Gly Lys Glu Gly Thr
   275 280 285
   Lys Gly Asp Ser Tyr Tyr Lys Met Met Lys Ser Asn Ile Asp Thr Val
   290 295 300
   His Gly Ser Met Lys
   305
<210> 9
   <211> 927
   <212> DNA
   <213> Staphylococcus aureus
<400> 9
   atgaaaaaat tagtaccttt attattagcc ttattacttc tagttgctgc atgtggtact       60
   gatggtaaac aaagcagtga taagtcaaat ggcaaattaa aagtagtaac gacgaattca      120
   attttatatg atatggctaa aaatgttggt ggagacaacg tcgatattca tagtattgta      180
   cctgttggtc aagatcctca tgaatatgaa gttaaaccta aagatattaa aaagttaact      240
   gacgctgacg ttattttata caacggatta aatttagaga ctggtaacgg ttggtttgaa      300
   aaagccttag aacaggctgg taaatcatta aaagataaaa aagttatcgc agtatcaaaa      360
   gatgttaaac ctatctattt aaacggtgaa gaaggcaaca aagataaaca agatccacac      420
   gcatggttaa gtttagataa cggtattaaa tacgtaaaaa caattcaaca aacatttatc      480
   gataacgaca aaaaacataa agcagattat gaaaagcaag gtaacaaata cattgctcaa      540
   ttggaaaaat taaataatga cagtaaagac aaatttaatg acattccaaa agaacaacgt      600
   gccatgatta caagtgaagg tgccttcaag tacttctcaa aacaatacgg tattacacca      660
   ggttatattt gggaaattaa cactgaaaaa caaggtacac ctgaacaaat gagacaagct      720
   attgagtttg ttaaaaagca caaattaaaa cacttattag tagaaacaag tgttgataag      780
   aaagcaatgg aaagtttatc tgaagaaacg aagaaagata tctttggtga agtgtacaca      840
   gattcaatcg gtaaagaagg cactaaaggt gactcttact acaaaatgat gaaatcaaat      900
   attgaaactg tacacggaag catgaaa                                           927
<210> 10
   <211> 309
   <212> PRT
   <213> Staphylococcus aureus
<400> 10
   Met Lys Lys Leu Val Pro Leu Leu Leu Ala Leu Leu Leu Leu Val Ala
   1               5                   10                  15
   Ala Cys Gly Thr Asp Gly Lys Gln Ser Ser Asp Lys Ser Asn Gly Lys
               20                  25                  30
   Leu Lys Val Val Thr Thr Asn Ser Ile Leu Tyr Asp Met Ala Lys Asn
           35                  40                  45
   Val Gly Gly Asp Asn Val Asp Ile His Ser Ile Val Pro Val Gly Gln
       50                  55                  60
   Asp Pro His Glu Tyr Glu Val Lys Pro Lys Asp Ile Lys Lys Leu Thr
   65                  70                  75                  80
   Asp Ala Asp Val Ile Leu Tyr Asn Gly Leu Asn Leu Glu Thr Gly Asn
                   85                  90                  95
   Gly Trp Phe Glu Lys Ala Leu Glu Gln Ala Gly Lys Ser Leu Lys Asp
               100                 105                 110
   Lys Lys Val Ile Ala Val Ser Lys Asp Val Lys Pro Ile Tyr Leu Asn
           115                 120                 125
   Gly Glu Glu Gly Asn Lys Asp Lys Gln Asp Pro His Ala Trp Leu Ser
       130                 135                 140
   Leu Asp Asn Gly Ile Lys Tyr Val Lys Thr Ile Gln Gln Thr Phe Ile
   145                 150                 155                 160
   Asp Asn Asp Lys Lys His Lys Ala Asp Tyr Glu Lys Gln Gly Asn Lys
                   165                 170                 175
   Tyr Ile Ala Gln Leu Glu Lys Leu Asn Asn Asp Ser Lys Asp Lys Phe
               180                 185                 190
   Asn Asp Ile Pro Lys Glu Gln Arg Ala Met Ile Thr Ser Glu Gly Ala
           195                 200                 205
   Phe Lys Tyr Phe Ser Lys Gln Tyr Gly Ile Thr Pro Gly Tyr Ile Trp
       210                 215                 220
   Glu Ile Asn Thr Glu Lys Gln Gly Thr Pro Glu Gln Met Arg Gln Ala
   225                 230                 235                 240
   Ile Glu Phe Val Lys Lys His Lys Leu Lys His Leu Leu Val Glu Thr
                   245                 250                 255
   Ser Val Asp Lys Lys Ala Met Glu Ser Leu Ser Glu Glu Thr Lys Lys
               260                 265                 270
   Asp Ile Phe Gly Glu Val Tyr Thr Asp Ser Ile Gly Lys Glu Gly Thr
           275                 280                 285
   Lys Gly Asp Ser Tyr Tyr Lys Met Met Lys Ser Asn Ile Glu Thr Val
       290                 295                 300
   His Gly Ser Met Lys
   305
<210> 11
   <211> 927
   <212> DNA
   <213> Staphylococcus aureus
<400> 11
   atgaaaaaat tagtaccttt attattagcc ttattacttc tagttgctgc atgtggtact      60
   ggtggtaaac aaagcagtga taagtcaaat ggcaaattaa aagtagtaac gacgaattca     120
   attttatatg atatggctaa aaatgttggt ggagacaacg tcgatattca tagtattgta     180
   cctgttggtc aagatcctca tgaatatgaa gttaaaccta aagatattaa aaagttaact     240
   gacgctgacg ttattttata caacggatta aatttagaga ctggtaacgg ttggtttgaa     300
   aaagccttag aacaggctgg taaatcatta aaagataaaa aagttatcgc agtatcaaaa     360
   gatgttaaac ctatctattt aaacggtgaa gaaggcaaca aagataaaca agatccacac     420
   gcatggttaa gtttagataa cggtattaaa tacgtaaaaa caattcaaca aacatttatc     480
   gataacgaca aaaaacataa agcatattat gaaaagcaag gtaacaaata cattgctcaa     540
   ttggaaaaat taaataatga cagtaaagac aaatttaatg acattccaaa agaacaacgt     600
   gccatgatta caagtgaagg tgccttcaag tacttctcaa aacaatacgg tattacacca     660
   ggttatattt gggaaattaa cactgaaaaa caaggtacac ctgaacaaat gagacaagct     720
   attgagtttg ttaaaaagca caaattaaaa cacttattag tagaaacaag tgttgataag     780
   aaagcaatgg aaagtttatc tgaagaaacg aagaaagata tctttggtga agtgtacaca     840
   gattcaatcg gtaaagaagg cactaaaggt gactcttact acaaaatgat gaaatcaaat     900
   attgatactg tacacggaag catgaaa                                         927
<210> 12
   <211> 309
   <212> PRT
   <213> Staphylococcus aureus
<400> 12
   Met Lys Lys Leu Val Pro Leu Leu Leu Ala Leu Leu Leu Leu Val Ala
   1               5                   10                  15
   Ala Cys Gly Thr Gly Gly Lys Gln Ser Ser Asp Lys Ser Asn Gly Lys
   20 25 30
   Leu Lys Val Val Thr Thr Asn Ser Ile Leu Tyr Asp Met Ala Lys Asn
   35 40 45
   Val Gly Gly Asp Asn Val Asp Ile His Ser Ile Val Pro Val Gly Gln
   50 55 60
   Asp Pro His Glu Tyr Glu Val Lys Pro Lys Asp Ile Lys Lys Leu Thr
   65 70 75 80
   Asp Ala Asp Val Ile Leu Tyr Asn Gly Leu Asn Leu Glu Thr Gly Asn
   85 90 95
   Gly Trp Phe Glu Lys Ala Leu Glu Gln Ala Gly Lys Ser Leu Lys Asp
   100 105 110
   Lys Lys Val Ile Ala Val Ser Lys Asp Val Lys Pro Ile Tyr Leu Asn
   115 120 125
   Gly Glu Glu Gly Asn Lys Asp Lys Gln Asp Pro His Ala Trp Leu Ser
   130 135 140
   Leu Asp Asn Gly Ile Lys Tyr Val Lys Thr Ile Gln Gln Thr Phe Ile
   145 150 155 160
   Asp Asn Asp Lys Lys His Lys Ala Tyr Tyr Glu Lys Gln Gly Asn Lys
   165 170 175
   Tyr Ile Ala Gln Leu Glu Lys Leu Asn Asn Asp Ser Lys Asp Lys Phe
   180 185 190
   Asn Asp Ile Pro Lys Glu Gln Arg Ala Met Ile Thr Ser Glu Gly Ala
   195 200 205
   Phe Lys Tyr Phe Ser Lys Gln Tyr Gly Ile Thr Pro Gly Tyr Ile Trp
   210 215 220
   Glu Ile Asn Thr Glu Lys Gln Gly Thr Pro Glu Gln Met Arg Gln Ala
   225 230 235 240
   Ile Glu Phe Val Lys Lys His Lys Leu Lys His Leu Leu Val Glu Thr
   245 250 255
   Ser Val Asp Lys Lys Ala Met Glu Ser Leu Ser Glu Glu Thr Lys Lys
   260 265 270
   Asp Ile Phe Gly Glu Val Tyr Thr Asp Ser Ile Gly Lys Glu Gly Thr
   275 280 285
   Lys Gly Asp Ser Tyr Tyr Lys Met Met Lys Ser Asn Ile Asp Thr Val
   290 295 300
   His Gly Ser Met Lys
   305
<210> 13
   <211> 927
   <212> DNA
   <213> Staphylococcus aureus
<400> 13
   atgaaaaaat tagtaccttt attattagcc ttattacttc tagttgctgc atgtggtact       60
   ggtggtaaac aaagcagtga taagtcaaat ggcaaattaa aagtagtaac gacgaattca      120
   attttatatg atatggctaa aaatgttggt ggagacaacg tcgatattca tagtattgta      180
   cctgttggtc aagatcctca tgaatatgaa gttaaaccta aagatattaa aaagttaact      240
   gacgctgacg ttattttata caacggatta aatttagaga ctggtaacgg ttggtttgaa      300
   aaagccttag aacaggctgg taaatcatta aaagataaaa aagttatcgc agtatcaaaa      360
   gatgttaaac ctatctattt aaacggtgaa gaaggcaaca aagataaaca agatccacac      420
   gcatggttaa gtttagataa cggtattaaa tacgtaaaaa caattcaaca aacatttatc      480
   gataacgaca aaaaacataa agcagattat gaaaagcaag gtaacaaata cattgctcaa      540
   ttggaaaaat taaataatga cagtaaagac aaatttaatg acrttccaaa agaacaacgt      600
   gccatgatta caagtgaagg tgccttcaag tacttctcaa aacaatacgg tattacacca      660
   ggttatattt gggaaattaa cactgaaaaa caaggtacac ctgaacaaat gagacaagct      720
   attgagtttg ttaaaaagca caaattaaaa cacttattag tagaaacaag tgttgataag      780
   aaagcaatgg aaagtttatc tgaagaaacg aagaaagata tctttggtga agtgtacaca      840
   gattcaatcg gtaaagaagg cactaaaggt gactcttact acaaaatgat gaaatcaaat      900
   attgatactg tacacggaag catgaaa                                          927
<210> 14
   <211> 309
   <212> PRT
   <213> Staphylococcus aureus
<220>
   <221> MOD_RES
   <222> (195)..(195)
   <223> Any amino acid
<400> 14
   Met Lys Lys Leu Val Pro Leu Leu Leu Ala Leu Leu Leu Leu Val Ala
   1               5                   10                  15
   Ala Cys Gly Thr Gly Gly Lys Gln Ser Ser Asp Lys Ser Asn Gly Lys
               20                  25                  30
   Leu Lys Val Val Thr Thr Asn Ser Ile Leu Tyr Asp Met Ala Lys Asn
           35                  40                  45
   Val Gly Gly Asp Asn Val Asp Ile His Ser Ile Val Pro Val Gly Gln
       50                  55                  60
   Asp Pro His Glu Tyr Glu Val Lys Pro Lys Asp Ile Lys Lys Leu Thr
   65                  70                  75                  80
   Asp Ala Asp Val Ile Leu Tyr Asn Gly Leu Asn Leu Glu Thr Gly Asn
                   85                  90                  95
   Gly Trp Phe Glu Lys Ala Leu Glu Gln Ala Gly Lys Ser Leu Lys Asp
               100                 105                 110
   Lys Lys Val Ile Ala Val Ser Lys Asp Val Lys Pro Ile Tyr Leu Asn
           115                 120                 125
   Gly Glu Glu Gly Asn Lys Asp Lys Gln Asp Pro His Ala Trp Leu Ser
       130                 135                 140
   Leu Asp Asn Gly Ile Lys Tyr Val Lys Thr Ile Gln Gln Thr Phe Ile
   145                 150                 155                 160
   Asp Asn Asp Lys Lys His Lys Ala Asp Tyr Glu Lys Gln Gly Asn Lys
                   165                 170                 175
   Tyr Ile Ala Gln Leu Glu Lys Leu Asn Asn Asp Ser Lys Asp Lys Phe
               180                 185                 190
   Asn Asp Xaa Pro Lys Glu Gln Arg Ala Met Ile Thr Ser Glu Gly Ala
           195                 200                 205
   Phe Lys Tyr Phe Ser Lys Gln Tyr Gly Ile Thr Pro Gly Tyr Ile Trp
       210                 215                 220
   Glu Ile Asn Thr Glu Lys Gln Gly Thr Pro Glu Gln Met Arg Gln Ala
   225                 230                 235                 240
   Ile Glu Phe Val Lys Lys His Lys Leu Lys His Leu Leu Val Glu Thr
   245 250 255
   Ser Val Asp Lys Lys Ala Met Glu Ser Leu Ser Glu Glu Thr Lys Lys
   260 265 270
   Asp Ile Phe Gly Glu Val Tyr Thr Asp Ser Ile Gly Lys Glu Gly Thr
   275 280 285
   Lys Gly Asp Ser Tyr Tyr Lys Met Met Lys Ser Asn Ile Asp Thr Val
   290 295 300
   His Gly Ser Met Lys
   305
<210> 15
   <211> 1626
   <212> DNA
   <213> Staphylococcus aureus
<400> 15
   ttgaaaaaaa gaattgatta tttgtcgaat aagcagaata agtattcgat tagacgtttt 60
   acagtaggta ccacatcagt aatagtaggg gcaacgatac tatttgggat aggcaatcat 120
   caagcacaag cttcagaaca atcgaacgat acaacgcaat cttcgaaaaa taatgcaagt 180
   gcagattccg aaaaaaacaa tacgatagaa acacctcaat taaatacaac ggctaatgat 240
   acatctgata ttagtgcaaa cacaaacagt gcgaatgtag atagcacagc aaaaccaatg 300
   tctacacaaa cgagcaatac cactacaaca gagccagctt caacaaatga aacacctcac 360
   ccgacggcaa ttaaagatca agcaactgct gcaaaaatgc aagatcaaac tgttcctcaa 420
   gaagcaaatt ctcaagtaga taataaaaca acgaatgatg ctaatagcat agcaacaaac 480
   agtgagctta aaaatcctca aacattagat ttaccacaat catcaccaca aacgatttcc 540
   aatgcgcaag gaactagtaa accaagtgtt agaacgagag ctgtacgtag tcttgcagtt 600
   actgaacctg tagtaaatgc tgctgatgct aaaggtacaa atgtaaatgg tcaagttacg 660
   gcaagtgatt tcaagttaga aaagactaca tttgacccta accaaagtgg taacacattt 720
   atggcggcaa attttaaagt ggcagggaaa gtgaaatcag gggattattt tacagcgaag 780
   ttaccagata gtgtaactgg taatggagat gtggattact ctaactcgaa taatacgatg 840
   ccaattgcag atattaaaag tacgaatggc gatgttgtag ctaaagcaac atatgatatc 900
   ttgactaaga cgtatacatt tgtctttaca gattatgtaa atgataaaga aaatattaac 960
   ggacaatttt cattaccatt atttacagac agagcaaagg cacctaaatc aggaacatat 1020
   gatgcaaata ttaatattgc ggatgaaatg tttaataata aaattactta taactatagt 1080
   tcaccaattg caggaattga taagccaaat ggcgcgaaca tttcttctca aattattggt     1140
   gtagatacag cttcaggtca aaacacatac aagcaaacgg tatttgttaa ccctaagcaa     1200
   cgagttttag gtaatacgtg ggtgtatatt aaaggttacc aagataaaat cgaagaaagt     1260
   agcggtaaag taagtgctac agatacaaaa ctgagaattt ttgaagtgaa tgatacatct     1320
   aaattatcag atagctacta tgcagaccca aatgactcta accttaaaga agtaactgat     1380
   caatttaaag ataaaatttc atacaaatac gataacgtag caagtattaa ttttggtgat     1440
   ataaataaaa cgtatgtcgt tttagtcgaa ggtcattatg ataatactgg taaaaacttg     1500
   aaaacacagg ttattcaaga aaatattgac ccagcgacag gtaaagatta cagtattttc     1560
   ggttggaata atgagaatgt tgtacgttat ggaggcggaa gtgctgatgg tgattcagca     1620
   gtaaat                                                                1626
<210> 16
   <211> 542
   <212> PRT
   <213> Staphylococcus aureus
<400> 16
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Thr Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro His Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Thr Glu Pro Val Val Asn Ala Ala
           195                 200                 205
   Asp Ala Lys Gly Thr Asn Val Asn Gly Gln Val Thr Ala Ser Asp Phe
       210                 215                 220
   Lys Leu Glu Lys Thr Thr Phe Asp Pro Asn Gln Ser Gly Asn Thr Phe
   225                 230                 235                 240
   Met Ala Ala Asn Phe Lys Val Ala Gly Lys Val Lys Ser Gly Asp Tyr
                   245                 250                 255
   Phe Thr Ala Lys Leu Pro Asp Ser Val Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asp Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asp Gln Phe Lys Asp
   450 455 460
   Lys Ile Ser Tyr Lys Tyr Asp Asn Val Ala Ser Ile Asn Phe Gly Asp
   465 470 475 480
   Ile Asn Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 17
   <211> 1626
   <212> DNA
   <213> Staphylococcus aureus
<400> 17
   ttgaaaaaaa gaattgatta tttgtcgaat aagcagaata agtattcgat tagacgtttt 60
   acagtaggta ccacatcagt aatagtaggg gcaactatac tatttgggat aggcaatcat 120
   caagcacaag cttcagaaca atcgaacgat acaacgcaat cttcgaaaaa taatgcaagt 180
   gcagattccg aaaaaaacaa tacgatagaa acacctcaat taaatacaac ggctaatgat 240
   acatctgata ttagtgcaaa cacaaacagt gcgaatgtag atagcacagc aaaaacaatg 300
   tctacacaaa cgagcaatac cactacaaca gagccagctt caacaaatga aacacctcaa 360
   ccgacggcaa ttaaagatca agcaactgct gcaaaaatgc aagatcaaac tgttcctcaa 420
   gaagcaaatt ctcaagtaga taataaaaca acgaatgatg ctaataacat agcaacaaac 480
   agtgagctta aaaatcctca aacattagat ttaccacaat catcaccaca aacgatttcc 540
   aatgcgcaag gaactagtaa accaagtgtt agaacgagag ctgtacgtag tcttgcagtt 600
   gctgaacctg tagtaaatgc tgctgatgct aaaggtacaa atgtaaatga taaagttacg 660
   gcaagtgatt tcaagttaga aaagactgca tttgacccta accaaagtgg taacacattt 720
   atggcggcaa attttaaagt gactggacaa gtgaaatcag gggattattt tacagcgaag 780
   ttaccagata gtgtaactgg taatggagac gtggattact ctaattcaaa taatacgatg 840
   ccaattgcag acattaaaag tacgaatggc gatgttgtag ctaaagcaac atatgatatc 900
   ttgactaaga cgtatacatt tgtctttaca gattatgtaa atgataaaga aaatattaac 960
   ggacaatttt cattaccttt atttacagac cgagcaaagg cacctaaatc aggaacatat 1020
   gatgcaaata ttaatattgc ggatgaaatg tttgataata aaattactta taactatagt 1080
   tcgccaattg caggaattga taagccaaat ggcgcgaaca tttcttctca aattattggt 1140
   gtagatacag cttcaggtca aaacacatac aagcaaacgg tatttgttaa ccctaagcaa 1200
   cgagttttag gtaatacgtg ggtgtatatt aaaggttacc aagataaaat cgaagaaagt 1260
   agcggtaaag taagtgctac agatacaaaa ctgagaattt ttgaagtgaa tgatacatct 1320
   aaattatcag atagctacta tgcagaccca aatgactcta accttaaaga agtgactggt 1380
   gagtttaaag ataaaatttc atacaaatac gataacgtag caagtattaa ttttggtgat 1440
   ataaataaaa cgtatgttgt attagtggaa ggtcactatg ataatactgg taaaaacttg 1500
   aaaacacagg ttattcaaga aaatattgac ccagcgacag gtaaagacta cagtattttc 1560
   ggttggaata atgagaatgt tgtacgttat ggaggcggaa gtgctgatgg tgattcagca 1620
   gtaaat 1626
<210> 18
   <211> 542
   <212> PRT
   <213> Staphylococcus aureus
<400> 18
   Leu Lys Lys Arg Ile Asp Tyr Leu Ser Asn Lys Gln Asn Lys Tyr Ser
   1 5 10 15
   Ile Arg Arg Phe Thr Val Gly Thr Thr Ser Val Ile Val Gly Ala Thr
   20 25 30
   Ile Leu Phe Gly Ile Gly Asn His Gln Ala Gln Ala Ser Glu Gln Ser
   35 40 45
   Asn Asp Thr Thr Gln Ser Ser Lys Asn Asn Ala Ser Ala Asp Ser Glu
   50 55 60
   Lys Asn Asn Thr Ile Glu Thr Pro Gln Leu Asn Thr Thr Ala Asn Asp
   65 70 75 80
   Thr Ser Asp Ile Ser Ala Asn Thr Asn Ser Ala Asn Val Asp Ser Thr
   85 90 95
   Ala Lys Thr Met Ser Thr Gln Thr Ser Asn Thr Thr Thr Thr Glu Pro
   100 105 110
   Ala Ser Thr Asn Glu Thr Pro Gln Pro Thr Ala Ile Lys Asp Gln Ala
   115 120 125
   Thr Ala Ala Lys Met Gln Asp Gln Thr Val Pro Gln Glu Ala Asn Ser
   130 135 140
   Gln Val Asp Asn Lys Thr Thr Asn Asp Ala Asn Asn Ile Ala Thr Asn
   145 150 155 160
   Ser Glu Leu Lys Asn Pro Gln Thr Leu Asp Leu Pro Gln Ser Ser Pro
   165 170 175
   Gln Thr Ile Ser Asn Ala Gln Gly Thr Ser Lys Pro Ser Val Arg Thr
   180 185 190
   Arg Ala Val Arg Ser Leu Ala Val Ala Glu Pro Val Val Asn Ala Ala
   195 200 205
   Asp Ala Lys Gly Thr Asn Val Asn Asp Lys Val Thr Ala Ser Asp Phe
   210 215 220
   Lys Leu Glu Lys Thr Ala Phe Asp Pro Asn Gln Ser Gly Asn Thr Phe
   225 230 235 240
   Met Ala Ala Asn Phe Lys Val Thr Gly Gln Val Lys Ser Gly Asp Tyr
   245 250 255
   Phe Thr Ala Lys Leu Pro Asp Ser Val Thr Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asp Val Val Ala Lys Ala Thr Tyr Asp Ile Leu Thr Lys Thr
   290 295 300
   Tyr Thr Phe Val Phe Thr Asp Tyr Val Asn Asp Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asp
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Gly Glu Phe Lys Asp
   450 455 460
   Lys Ile Ser Tyr Lys Tyr Asp Asn Val Ala Ser Ile Asn Phe Gly Asp
   465 470 475 480
   Ile Asn Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 19
   <211> 1626
   <212> DNA
   <213> Staphylococcus aureus
<400> 19
   ttgaaaaaaa gaattgatta tttgtcgaat aagcagaata agtattcgat tagacgtttt        60
   acagtaggta ccacatcagt aatagtaggg gcaactatac tatttgggat aggcaatcat       120
   caagcacaag cttcagaaca atcgaacgat acaacgcaat cttcgaaaaa taatgcaagt       180
   gcagattccg aaaaaaacaa tacgatagaa acacctcaat taaatacaac ggctaatgat       240
   acatctgata ttagtgcaaa cacaaacagt gcgaatgtag atagcacagc aaaaccaatg       300
   tctacacaaa cgagcaatac cactacaaca gagccagctt caacaaatga aacacctcaa       360
   ccgacggcaa ttaaagatca agcaactgct gcaaaaatgc aagatcaaac tgttcctcaa       420
   gaagcaaatt ctcaagtaga taataaaaca acgaatgatg ctaatagcat agcgacaaac       480
   agcgagctta aaaatcctca atcattagat ttaccacaat catcaccaca aacgatttcc       540
   aatgcgcaag gaactagtaa accaagtgtt agaacgagag ctgtacgtag tcttgcagtt       600
   gctgaacctg tagtaaatgc tgctgatgct aaaggtacaa atgtaaatga taaagttacg       660
   gcaaaagatt ttcaattaga aaagactaca tttgacccta accaaagtgg taatactttt       720
   atggcggcaa actttacagt gactggacaa gtgaaatcag gggattattt tacagcgaag       780
   ttaccagata gtgtaactgg taatggagac gtggattact ctaattcaaa taatacgatg       840
   ccaattgcag atatagtaaa cgataaaaat gaagttgtag caaaagcgac atatgatatt       900
   ttgactaaga catatacatt tgtctttaca gattatgtaa atgataagca aaatattaat       960
   gggaaatttt cattaccatt atttacagac cgagcaaagg cacctaaatc aggaatatat      1020
   gatgcgaata ttaatattgc ggatgaaatg tttaataata aaattactta taactatagt      1080
   tcgccaattg caggaattga taagccaaat ggcgcgaaca tttcttctca aattattggt      1140
   gtagatacag cttcaggtca aaatacatac aagcaaacgg tatttgttaa ccctaagcaa      1200
   cgagttttag gtaatacgtg ggtgtatatt aaaggttacc aagataaaat cgaagaaagt      1260
   agcggtaaag taagtgctac agatacaaaa ctgagaattt ttgaagtgaa tgatacatct      1320
   aaattatcag atagctacta tgcggaccca aatgactcta accttaaaga agtgactggt      1380
   gagtttaata atagaatttt ttatgaacat ccaaacgtag caagtattaa ttttggtgat      1440
   ataaataaaa cgtatgtcgt tttagttgaa ggtcactatg ataatactgg taaaaacttg      1500
   aaaacacagg ttattcaaga aaatattgac ccagcgacag gtaaagacta cagtattttc      1560
   ggatggaata atgagaatgt tgtacgttat ggtggtggaa gtgctgatgg tgattcagca      1620
   gtaaat                                                                 1626
<210> 20
   <211> 542
   <212> PRT
   <213> Staphylococcus aureus
<400> 20
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Thr Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Pro Gln Ser Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Lys Asp Phe
       210                 215                 220
   Gln Leu Glu Lys Thr Thr Phe Asp Pro Asn Gln Ser Gly Asn Thr Phe
   225                 230                 235                 240
   Met Ala Ala Asn Phe Thr Val Thr Gly Gln Val Lys Ser Gly Asp Tyr
                   245                 250                 255
   Phe Thr Ala Lys Leu Pro Asp Ser Val Thr Gly Asn Gly Asp Val Asp
               260                 265                 270
   Tyr Ser Asn Ser Asn Asn Thr Met Pro Ile Ala Asp Ile Val Asn Asp
           275                 280                 285
   Lys Asn Glu Val Val Ala Lys Ala Thr Tyr Asp Ile Leu Thr Lys Thr
       290                 295                 300
   Tyr Thr Phe Val Phe Thr Asp Tyr Val Asn Asp Lys Gln Asn Ile Asn
   305                 310                 315                 320
   Gly Lys Phe Ser Leu Pro Leu Phe Thr Asp Arg Ala Lys Ala Pro Lys
                   325                 330                 335
   Ser Gly Ile Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Gly Glu Phe Asn Asn
       450                 455                 460
   Arg Ile Phe Tyr Glu His Pro Asn Val Ala Ser Ile Asn Phe Gly Asp
   465                 470                 475                 480
   Ile Asn Lys Thr Tyr Val Val Leu Val Glu Gly His Tyr Asp Asn Thr
                   485                 490                 495
   Gly Lys Asn Leu Lys Thr Gln Val Ile Gln Glu Asn Ile Asp Pro Ala
               500                 505                 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 21
   <211> 1626
   <212> DNA
   <213> Staphylococcus aureus
<400> 21
   ttgaaaaaaa gaattgatta tttgtcgaat aagcagaata agtattcgat tagacgtttt 60
   acagtaggta ccacatcagt aatagtaggg gcaactatac tatttgggat aggcaatcat 120
   caagcacaag cttcagaaca atcgaacgat acaacgcaat cttcgaaaaa taatgcaagt 180
   gcagattccg aaaaaaacaa tacgatagaa acacctcaat taaatacaac ggctaatgat 240
   acatctgata ttagtgcaaa cacaaacagt gcgaatgtag atagcacagc aaaaccaatg 300
   tctacacaaa cgagcaatac cactacaaca gagccagctt caacaaatga aacacctcaa 360
   ccgacggcaa ttaaagatca agcaactgct gcaaaaatgc aagatcgaac tgttcctcaa 420
   gaagcaaatt ctcaagtaga taataaaaca acgaatgatg ctaatagcat aacaacaaac 480
   agtgagctta aaaatcctca aacattagat ttaccacaat catcaccaca aacgatttcc 540
   aatgcgcaag gaactagtaa accaagtgtt agaacgagag ctgtacgtag tcttgcagtt 600
   gctgaacctg tagtaaatgc tgctgatgct aaaggcacaa atgtaaatga taaagttacg 660
   gcaaaagatt ttcaattaga aaagactaca tttgacccta accaaagtgg taatactttt 720
   atggcggcaa actttacagt gactggacaa gtgaaatcag gggattattt tacagcgaag 780
   ttaccagaaa gtttaactgg taatggagac gtggattact ctaactcgaa taatacgatg 840
   ccaattgcag atattaaaag tacgaatggc aatgttgtag ctaaagcaac atatgatatc 900
   ttgactaaga cgtatacatt tgtctttaca gattatgtaa atgataaaga aaatattaac 960
   ggacaatttt cattaccttt atttacagac cgagcaaagg cacctaaatc aggaacatat 1020
   gatgcaaata ttaatattgc ggatgaaatg tttaacaata aaattactta taactatagt 1080
   tcaccaattg caggaattga taagccaaat ggcgcgaaca tttcttctca aattattggt 1140
   gtagatacag cttcaggtca aaacacatac aagcaaacgg tatttgttaa ccctaagcaa 1200
   cgagttttag gtaatacgtg ggtgtatatt aaaggttacc aagataaaat cgaagaaagt 1260
   agcggtaaag taagtgctac agatacaaaa ctgagaattt ttgaagtgaa tgatacatct 1320
   aaattatcag atagctacta tgcggaccca aatgactcta atcttaaaga agtaactgat 1380
   caatttaagg ataaaatcac ttataaatac caaaatgtag caagtattaa ttttggtgat 1440
   attactaaaa cgtatgttgt attagtggaa ggtcactatg ataatactgg taaaaacttg 1500
   aaaacacagg ttattcaaga aaatattgac ccagcgacag gtaaagacta cagtattttc 1560
   ggatggaata atgagaatgt tgtacgttat ggtggtggaa gtgctgatgg tgattcagca 1620
   gtaaat 1626
<210> 22
   <211> 542
   <212> PRT
   <213> Staphylococcus aureus
<400> 22
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Thr Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Arg Thr Val Pro Gln Glu Ala Asn Ser
       130                 135                 140
   Gln Val Asp Asn Lys Thr Thr Asn Asp Ala Asn Ser Ile Thr Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
   195 200 205
   Asp Ala Lys Gly Thr Asn Val Asn Asp Lys Val Thr Ala Lys Asp Phe
   210 215 220
   Gln Leu Glu Lys Thr Thr Phe Asp Pro Asn Gln Ser Gly Asn Thr Phe
   225 230 235 240
   Met Ala Ala Asn Phe Thr Val Thr Gly Gln Val Lys Ser Gly Asp Tyr
   245 250 255
   Phe Thr Ala Lys Leu Pro Glu Ser Leu Thr Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asn Val Val Ala Lys Ala Thr Tyr Asp Ile Leu Thr Lys Thr
   290 295 300
   Tyr Thr Phe Val Phe Thr Asp Tyr Val Asn Asp Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asp Gln Phe Lys Asp
       450                 455                 460
   Lys Ile Thr Tyr Lys Tyr Gln Asn Val Ala Ser Ile Asn Phe Gly Asp
   465                 470                 475                 480
   Ile Thr Lys Thr Tyr Val Val Leu Val Glu Gly His Tyr Asp Asn Thr
                   485                 490                 495
   Gly Lys Asn Leu Lys Thr Gln Val Ile Gln Glu Asn Ile Asp Pro Ala
               500                 505                 510
   Thr Gly Lys Asp Tyr Ser Ile Phe Gly Trp Asn Asn Glu Asn Val Val
           515                 520                 525
   Arg Tyr Gly Gly Gly Ser Ala Asp Gly Asp Ser Ala Val Asn
       530                 535                 540
<210> 23
   <211> 1620
   <212> DNA
   <213> Staphylococcus aureus
<400> 23
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tacgatagaa acacctcaat taaatactaa tgatacatct     240
   gatattagtg caaacacaaa cagtgcgaat gtagatagca cagcaaaacc aatgtctaca     300
   caaacgagca ataccactac aacagagcca gcttcaacaa atgaaacacc tcaaccgacg     360
   gcaattaaag atcaagcaac tgctgcaaaa atgcaagatc gaactgttcc tcaagaagca     420
   aattctcaag tagataataa aacaacgaat gatgctaata gcataacaac aaacagtgag     480
   cttaaaaatc ctcaaacatt agatttacca caatcatcac cacaaacgat ttccaatgcg     540
   caaggaacta gtaaaccaag tgttagaacg agagctgtac gtagtcttgc agttgctgaa     600
   cctgtagtaa atgctgctga tgctaaaggc acaaatgtaa atgataaagt tacggcaaaa     660
   gattttcaat tagaaaagac tacatttgac cctaaccaaa gtggtaatac ttttatggcg     720
   gcaaacttta cagtgactgg acaagtgaaa tcaggggatt attttacagc gaagttacca     780
   gaaagtttaa ctggtaatgg agacgtggat tactctaact cgaataatac gatgccaatt     840
   gcagatatta aaagtacgaa tggcaatgtt gtagctaaag caacatatga tatcttgact     900
   aagacgtata catttgtctt tacagattat gtaaatgata aagaaaatat taacggacaa     960
   ttttcattac ctttatttac agaccgagca aaggcaccta aatcaggaac atatgatgca    1020
   aatattaata ttgcggatga aatgtttaac aataaaatta cttataacta tagttcacca    1080
   attgcaggaa ttgataagcc aaatggcgcg aacatttctt ctcaaattat tggtgtagat    1140
   acagcttcag gtcaaaacac atacaagcaa acggtatttg ttaaccctaa gcaacgagtt    1200
   ttaggtaata cgtgggtgta tattaaaggt taccaagata aaatcgaaga aagtagcggt    1260
   aaagtaagtg ctacagatac aaaactgaga atttttgaag tgaatgatac atctaaatta    1320
   tcagatagct actatgcgga cccaaatgac tctaatctta aagaagtaac tgatcaattt    1380
   aaggataaaa tcacttataa ataccaaaat gtagcaagta ttaattttgg tgatattact    1440
   aaaacgtatg ttgtattagt ggaaggtcac tatgataata ctggtaaaaa cttgaaaaca    1500
   caggttattc aagaaaatat tgacccagcg acaggtaaag actacagtat tttcggatgg    1560
   aataatgaga atgttgtacg ttatggtggt ggaagtgctg atggtgattc agcagtaaat    1620
<210> 24
   <211> 540
   <212> PRT
   <213> Staphylococcus aureus
<400> 24
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Thr Ile Glu Thr Pro Gln Leu Asn Thr Asn Asp Thr Ser
   65                  70                  75                  80
   Asp Ile Ser Ala Asn Thr Asn Ser Ala Asn Val Asp Ser Thr Ala Lys
                   85                  90                  95
   Pro Met Ser Thr Gln Thr Ser Asn Thr Thr Thr Thr Glu Pro Ala Ser
               100                 105                 110
   Thr Asn Glu Thr Pro Gln Pro Thr Ala Ile Lys Asp Gln Ala Thr Ala
           115                 120                 125
   Ala Lys Met Gln Asp Arg Thr Val Pro Gln Glu Ala Asn Ser Gln Val
       130                 135                 140
   Asp Asn Lys Thr Thr Asn Asp Ala Asn Ser Ile Thr Thr Asn Ser Glu
   145                 150                 155                 160
   Leu Lys Asn Pro Gln Thr Leu Asp Leu Pro Gln Ser Ser Pro Gln Thr
                   165                 170                 175
   Ile Ser Asn Ala Gln Gly Thr Ser Lys Pro Ser Val Arg Thr Arg Ala
               180                 185                 190
   Val Arg Ser Leu Ala Val Ala Glu Pro Val Val Asn Ala Ala Asp Ala
           195                 200                 205
   Lys Gly Thr Asn Val Asn Asp Lys Val Thr Ala Lys Asp Phe Gln Leu
       210                 215                 220
   Glu Lys Thr Thr Phe Asp Pro Asn Gln Ser Gly Asn Thr Phe Met Ala
   225                 230                 235                 240
   Ala Asn Phe Thr Val Thr Gly Gln Val Lys Ser Gly Asp Tyr Phe Thr
                   245                 250                 255
   Ala Lys Leu Pro Glu Ser Leu Thr Gly Asn Gly Asp Val Asp Tyr Ser
               260                 265                 270
   Asn Ser Asn Asn Thr Met Pro Ile Ala Asp Ile Lys Ser Thr Asn Gly
           275                 280                 285
   Asn Val Val Ala Lys Ala Thr Tyr Asp Ile Leu Thr Lys Thr Tyr Thr
       290                 295                 300
   Phe Val Phe Thr Asp Tyr Val Asn Asp Lys Glu Asn Ile Asn Gly Gln
   305                 310                 315                 320
   Phe Ser Leu Pro Leu Phe Thr Asp Arg Ala Lys Ala Pro Lys Ser Gly
                   325                 330                 335
   Thr Tyr Asp Ala Asn Ile Asn Ile Ala Asp Glu Met Phe Asn Asn Lys
               340                 345                 350
   Ile Thr Tyr Asn Tyr Ser Ser Pro Ile Ala Gly Ile Asp Lys Pro Asn
           355                 360                 365
   Gly Ala Asn Ile Ser Ser Gln Ile Ile Gly Val Asp Thr Ala Ser Gly
       370                 375                 380
   Gln Asn Thr Tyr Lys Gln Thr Val Phe Val Asn Pro Lys Gln Arg Val
   385                 390                 395                 400
   Leu Gly Asn Thr Trp Val Tyr Ile Lys Gly Tyr Gln Asp Lys Ile Glu
                   405                 410                 415
   Glu Ser Ser Gly Lys Val Ser Ala Thr Asp Thr Lys Leu Arg Ile Phe
               420                 425                 430
   Glu Val Asn Asp Thr Ser Lys Leu Ser Asp Ser Tyr Tyr Ala Asp Pro
           435                 440                 445
   Asn Asp Ser Asn Leu Lys Glu Val Thr Asp Gln Phe Lys Asp Lys Ile
       450                 455                 460
   Thr Tyr Lys Tyr Gln Asn Val Ala Ser Ile Asn Phe Gly Asp Ile Thr
   465                 470                 475                 480
   Lys Thr Tyr Val Val Leu Val Glu Gly His Tyr Asp Asn Thr Gly Lys
                   485                 490                 495
   Asn Leu Lys Thr Gln Val Ile Gln Glu Asn Ile Asp Pro Ala Thr Gly
               500                 505                 510
   Lys Asp Tyr Ser Ile Phe Gly Trp Asn Asn Glu Asn Val Val Arg Tyr
           515                 520                 525
   Gly Gly Gly Ser Ala Asp Gly Asp Ser Ala Val Asn
       530                 535                 540
<210> 25
   <211> 1626
   <212> DNA
   <213> Staphylococcus aureus
<400> 25
   ttgaaaaaaa gaattgatta tttgtcgaat aagcagaata agtattcgat tagacgtttt 60
   acagtaggta ccacatcagt aatagtaggg gcaactatac tatttgggat aggcaatcat 120
   caagcacaag cttcagaaca atcgaacgat acaacgcaat cttcgaaaaa taatgcaagt 180
   gcagattccg aaaaaaacaa tatgatagaa acacctcaat taaatacaac ggctaatgat 240
   acatctgata ttagtgcaaa cacaaacagt gcgaatgtag atagcacaac aaaaccaatg 300
   tctacacaaa cgagcaatac cactacaaca gagccagctt caacaaatga aacacctcaa 360
   ccgacggcaa ttaaaaatca agcaactgct gcaaaaatgc aagatcaaac tgttcctcaa 420
   gaagcaaatt ctcaagtaga taataaaaca acgaatgatg ctaatagcat agcaacaaac 480
   agtgagctta aaaattctca aacattagat ttaccacaat catcaccaca aacgatttcc 540
   aatgcgcaag gaactagtaa accaagtgtt agaacgagag ctgtacgtag tttagctgtt 600
   gctgaaccgg tagtaaatgc tgctgatgct aaaggtacaa atgtaaatga taaagttacg 660
   gcaagtaatt tcaagttaga aaagactaca tttgacccta atcaaagtgg taacacattt 720
   atggcggcaa attttacagt gacagataaa gtgaaatcag gggattattt tacagcgaag 780
   ttaccagata gtttaactgg taatggagac gtggattatt ctaattcaaa taatacgatg 840
   ccaattgcag acattaaaag tacgaatggc gatgttgtag ctaaagcaac atatgatatc 900
   ttgactaaga cgtatacatt tgtctttaca gattatgtaa ataataaaga aaatattaac 960
   ggacaatttt cattaccttt atttacagac cgagcaaagg cacctaaatc aggaacatat 1020
   gatgcgaata ttaatattgc ggatgaaatg tttaataata aaattactta taactatagt 1080
   tcgccaattg caggaattga taaaccaaat ggcgcgaaca tttcttctca aattattggt 1140
   gtagatacag cttcaggtca aaacacatac aagcaaacag tatttgttaa ccctaagcaa 1200
   cgagttttag gtaatacgtg ggtgtatatt aaaggctacc aagataaaat cgaagaaagt 1260
   agcggtaaag taagtgctac agatacaaaa ctgagaattt ttgaagtgaa tgatacatct 1320
   aaattatcag atagctacta tgcagatcca aatgactcta accttaaaga agtaacagac 1380
   caatttaaaa atagaatcta ttatgagcat ccaaatgtag ctagtattaa atttggtgat 1440
   attactaaaa catatgtagt attagtagaa gggcattacg acaatacagg taagaactta 1500
   aaaactcagg ttattcaaga aaatgttgat cctgtaacaa atagagacta cagtattttc 1560
   ggttggaata atgagaatgt tgtacgttat ggtggtggaa gtgctgatgg tgattcagca 1620
   gtaaat 1626
<210> 26
   <211> 542
   <212> PRT
   <213> Staphylococcus aureus
<400> 26
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Thr Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asn Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Ser Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Ser Asn Phe
       210                 215                 220
   Lys Leu Glu Lys Thr Thr Phe Asp Pro Asn Gln Ser Gly Asn Thr Phe
   225                 230                 235                 240
   Met Ala Ala Asn Phe Thr Val Thr Asp Lys Val Lys Ser Gly Asp Tyr
                   245                 250                 255
   Phe Thr Ala Lys Leu Pro Asp Ser Leu Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asn Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Asp Gln Phe Lys Asn
       450                 455                 460
   Arg Ile Tyr Tyr Glu His Pro Asn Val Ala Ser Ile Lys Phe Gly Asp
   465                 470                 475                 480
   Ile Thr Lys Thr Tyr Val Val Leu Val Glu Gly His Tyr Asp Asn Thr
                   485                 490                 495
   Gly Lys Asn Leu Lys Thr Gln Val Ile Gln Glu Asn Val Asp Pro Val
               500                 505                 510
   Thr Asn Arg Asp Tyr Ser Ile Phe Gly Trp Asn Asn Glu Asn Val Val
           515                 520                 525
   Arg Tyr Gly Gly Gly Ser Ala Asp Gly Asp Ser Ala Val Asn
       530                 535                 540
<210> 27
   <211> 1626
   <212> DNA
   <213> Staphylococcus aureus
<400> 27
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tatgatagaa acacctcaat taaatacaac ggctaatgat     240
   acatctgata ttagtgcaaa cacaaacagt gcgaatgtag atagcacaac aaaaccaatg     300
   tctacacaaa cgagcaatac cactacaaca gagccagctt caacaaatga aacacctcaa     360
   ccgacggcaa ttaaaaatca agcaactgct gcaaaaatgc aagatcaaac tgttcctcaa     420
   gaagcaaatt ctcaagtaga taataaaaca acgaatgatg ctaatagcat agcaacaaac     480
   agtgagctta aaaattctca aacattagat ttaccacaat catcaccaca aacgatttcc     540
   aatgcgcaag gaactagtaa accaagtgtt agaacgagag ctgtacgtag tttagctgtt     600
   gctgaaccgg tagtaaatgc tgctgatgct aaaggtacaa atgtaaatga taaagttacg     660
   gcaagtaatt tcaagttaga aaagactaca tttgacccta atcaaagtgg taacacattt     720
   atggcggcaa attttacagt gacagataaa gtgaaatcag gggattattt tacagcgaag    780
   ttaccagata gtttaactgg taatggagac gtggattatt ctaattcaaa taatacgatg     840
   ccaattgcag acattaaaag tacgaatggc gatgttgtag ctaaagcaac atatgatatc     900
   ttgactaaga cgtatacatt tgtctttaca gattatgtaa ataataaaga aaatattaac     960
   ggacaatttt cattaccttt atttacagac cgagcaaagg cacctaaatc aggaacatat    1020
   gatgcgaata ttaatattgc ggatgaaatg tttaataata aaattactta taactatagt    1080
   tcgccaattg caggaattga taaaccaaat ggcgcgaaca tttcttctca aattattggt    1140
   gtagatacag cttcaggtca aaacacatac aagcaaacag tatttgttaa ccctaagcaa    1200
   cgagttttag gtaatacgtg ggtgtatatt aaaggctacc aagataaaat cgaagaaagt    1260
   agcggtaaag taagtgctac agatacaaaa ctgagaattt ttgaagtgaa tgatatatct    1320
   aaattatcag atagctacta tgcagatcca aatgactcta accttaaaga agtaacagac    1380
   caatttaaaa atagaatcta ttatgagcat ccaaatgtag ctagtattaa atttggtgat    1440
   attactaaaa catatgtagt attagtagaa gggcattacg acaatacagg taagaactta    1500
   aaaactcagg ttattcaaga aaatgttgat cctgtaacaa atagagacta cagtattttc    1560
   ggttggaata atgagaatgt tgtacgttat ggtggtggaa gtgctgatgg tgattcagca    1620
   gtaaat                                                               1626
<210> 28
   <211> 542
   <212> PRT
   <213> Staphylococcus aureus
<400> 28
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Thr Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asn Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Ser Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Ser Asn Phe
       210                 215                 220
   Lys Leu Glu Lys Thr Thr Phe Asp Pro Asn Gln Ser Gly Asn Thr Phe
   225                 230                 235                 240
   Met Ala Ala Asn Phe Thr Val Thr Asp Lys Val Lys Ser Gly Asp Tyr
   245 250 255
   Phe Thr Ala Lys Leu Pro Asp Ser Leu Thr Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asp Val Val Ala Lys Ala Thr Tyr Asp Ile Leu Thr Lys Thr
   290 295 300
   Tyr Thr Phe Val Phe Thr Asp Tyr Val Asn Asn Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Ile Ser Lys Leu Ser Asp Ser Tyr Tyr Ala
   435 440 445
   Asp Pro Asn Asp Ser Asn Leu Lys Glu Val Thr Asp Gln Phe Lys Asn
   450 455 460
   Arg Ile Tyr Tyr Glu His Pro Asn Val Ala Ser Ile Lys Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Val Asp Pro Val
   500 505 510
   Thr Asn Arg Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 29
   <211> 1626
   <212> DNA
   <213> Staphylococcus aureus
<400> 29
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tatgatagaa acacctcaat taaatacaac ggctaatgat     240
   acatctgata ttagtgcaaa cacaaacagt gcgaatgtag atagcacagc aaaaccaatg     300
   tctacacaaa cgagcaatac cactacaaca gagccagctt caacaaatga aacacctcaa     360
   ccgacggcaa ttaaagatca agcaactgct gcaaaaatgc aagatcaaac tgttcctcaa     420
   gaagcaaatt ctcaagtaga taataaaaca acgaatgatg ctaatagcat agcgacaaac     480
   agtgagctta aaaatcctca aacattagat ttaccacaat catcaccaca aacgatttcc     540
   aatgcgcaaa gaactagtaa accaagtgtt agaacgagag ctgtacgtag tttagctgtt     600
   gctgaaccgg tagtaaatgc tgctgatgct aaaggtacaa atgtaaatgg tcaagttacg     660
   gcaagtgatt tcaagttaga aaagactaca tttgacccta accaaagtgg taacacattt     720
   atggcggtaa attttaaagt ggcagggaaa gtgaaatcag gggattatta tacagcgaag     780
   ttaccagata gtttaactgg taatggagac gtggattact ctaattcaaa taatacgatg     840
   ccaattgcag atattaaaag tacgaatggc gatgttgtag ctaaagcaac atatgatatc     900
   ttgactaaga cgtatacatt tgtctttaca gattatgtaa atgataaaga aaatattaac     960
   ggacaatttt cattaccttt atttacagac cgagcaaagg cacctaaatc aggaacatac    1020
   gatgcaaata ttaatattgc ggatgaaatg tttaataata aaattactta taactatagt    1080
   tcgccaattg caggaattga taagccaaat ggcgcgaaca tttcttctca aattattggt    1140
   gtagatacag cttcaggtca aaacacatac aagcaaacgg tatttgttaa ccctaagcaa    1200
   cgagttttag gtaatacgtg ggtgtatatt aaaggttacc aagataaaat cgaagaaagt    1260
   agcggtaaag taagtgctac agatacaaaa ctgagaattt ttgaagtgaa tgatacatct    1320
   aaattatcag atagctacta tgcagatcca aatgactcta accttaaaga agtaacgaat    1380
   gagtttaagg ataaaatcac ttataaatac caaaatgtag caagtattaa ttttggcgat    1440
   attactaaaa cgtatgttgt attagtggaa ggtcactatg ataatactgg taaaaacttg    1500
   aaaacacagg ttattcaaga aaatattgac ccagcgacag gtaaagacta cagtattttc    1560
   ggttggaata atgagaatgt tgtacgttat ggtggtggaa gtgctgatgg tgattcagca    1620
   gtaaat                                                               1626
<210> 30
   <211> 542
   <212> PRT
   <213> Staphylococcus aureus
<400> 30
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Arg Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Gly Gln Val Thr Ala Ser Asp Phe
       210                 215                 220
   Lys Leu Glu Lys Thr Thr Phe Asp Pro Asn Gln Ser Gly Asn Thr Phe
   225                 230                 235                 240
   Met Ala Val Asn Phe Lys Val Ala Gly Lys Val Lys Ser Gly Asp Tyr
                   245                 250                 255
   Tyr Thr Ala Lys Leu Pro Asp Ser Leu Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asp Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Asn Glu Phe Lys Asp
       450                 455                 460
   Lys Ile Thr Tyr Lys Tyr Gln Asn Val Ala Ser Ile Asn Phe Gly Asp
   465                 470                 475                 480
   Ile Thr Lys Thr Tyr Val Val Leu Val Glu Gly His Tyr Asp Asn Thr
                   485                 490                 495
   Gly Lys Asn Leu Lys Thr Gln Val Ile Gln Glu Asn Ile Asp Pro Ala
               500                 505                 510
   Thr Gly Lys Asp Tyr Ser Ile Phe Gly Trp Asn Asn Glu Asn Val Val
           515                 520                 525
   Arg Tyr Gly Gly Gly Ser Ala Asp Gly Asp Ser Ala Val Asn
       530                 535                 540
<210> 31
   <211> 1626
   <212> DNA
   <213> Staphylococcus aureus
<400> 31
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tatgatagaa acacctcaat taaatacaac ggctaatgat     240
   acatctgata ttagtgcaaa cacaaacagt gcgaatgtag atagcacagc aaaaccaatg     300
   tctacacaaa cgagcaatac cactacaaca gagccagctt caacaaatga aacacctcaa     360
   ctgacggcaa ttaaagatca agcaactgct gcaaaaatgc aagatcaaac tgttcctcaa     420
   gaagcaaatt ctcaagtaga taataaaaca acgaatgatg ctaatagcat agcgacaaac     480
   agtgagctta aaaatcctca aacattagat ttaccacaat catcaccaca aacaatttcc     540
   aatgcgcaag gaactagtaa accaagtgtt agaacgagag ctgtacgtag tcttgcagtt     600
   gctgaacctg tagtaaatgc tgctgatgct aaaggtacaa atgtaaatga taaagttacg     660
   gcaaaagatt ttcaattaga aaagactaca tttgacccta accaaagtgg taatactttt     720
   atggcggcaa actttacagt gactggacaa gtgaaatcag ggattatttt tacagcgaag     780
   ttaccagata gtgtaactgg taatggagac gtggattact ctaattcgaa taatacgatg     840
   ccaattgcag atatagtaaa cgataaaaat gaagttgtag caaaagcgac atatgatatt     900
   ttgactaaga catatacatt tgtctttaca gattatgtaa atgataagca aaatattaat     960
   gggaaatttt cattaccact atttacagac agagcaaagg cacctaaatc aggaacatat    1020
   gatgcaaata ttaatattgc ggatgaaatg tttaataata aaattactta taactatagt    1080
   tcgccaattg caggaattga taagccaaat ggcgcgaaca tttcttctca aattattggt    1140
   gtagatacag cttcaggtca aaatacatac aagcaaacgg tatttgttaa ccctaagcaa    1200
   cgagttttag gtaatacgtg ggtgtatatt aaaggttacc aagataaaat cgaagaaagt    1260
   agcggtaaag taagtgctac agatacaaaa ctgagaattt ttgaagtgaa tgatacatct    1320
   aaattatcag atagctacta tgcagaccca aatgactcta atcttaaaga agtaactgat    1380
   caatttaagg ataaaatcac ttataaatac caaaatgtag caagtattaa ttttggtgat    1440
   ataaataaaa cgtatgtcgt tttagtcgaa ggtcattatg ataaaacagg taaaaacttg    1500
   aaaacgcaag tcattcaaga aaatgttgac ccagcgacag gtaaagacta cagtattttc    1560
   ggttggaata atgagaatgt tgtacgttat ggtggtggaa gtgctgatgg tgattcagca    1620
   gtaaat                                                               1626
<210> 32
   <211> 542
   <212> PRT
   <213> Staphylococcus aureus
<400> 32
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Leu Thr Ala Ile Lys Asp Gln Ala
   115 120 125
   Thr Ala Ala Lys Met Gln Asp Gln Thr Val Pro Gln Glu Ala Asn Ser
   130 135 140
   Gln Val Asp Asn Lys Thr Thr Asn Asp Ala Asn Ser Ile Ala Thr Asn
   145 150 155 160
   Ser Glu Leu Lys Asn Pro Gln Thr Leu Asp Leu Pro Gln Ser Ser Pro
   165 170 175
   Gln Thr Ile Ser Asn Ala Gln Gly Thr Ser Lys Pro Ser Val Arg Thr
   180 185 190
   Arg Ala Val Arg Ser Leu Ala Val Ala Glu Pro Val Val Asn Ala Ala
   195 200 205
   Asp Ala Lys Gly Thr Asn Val Asn Asp Lys Val Thr Ala Lys Asp Phe
   210 215 220
   Gln Leu Glu Lys Thr Thr Phe Asp Pro Asn Gln Ser Gly Asn Thr Phe
   225 230 235 240
   Met Ala Ala Asn Phe Thr Val Thr Gly Gln Val Lys Ser Gly Asp Tyr
   245 250 255
   Phe Thr Ala Lys Leu Pro Asp Ser Val Thr Gly Asn Gly Asp Val Asp
   260 265 270
   Tyr Ser Asn Ser Asn Asn Thr Met Pro Ile Ala Asp Ile Val Asn Asp
   275 280 285
   Lys Asn Glu Val Val Ala Lys Ala Thr Tyr Asp Ile Leu Thr Lys Thr
   290 295 300
   Tyr Thr Phe Val Phe Thr Asp Tyr Val Asn Asp Lys Gln Asn Ile Asn
   305 310 315 320
   Gly Lys Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asp Gln Phe Lys Asp
   450 455 460
   Lys Ile Thr Tyr Lys Tyr Gln Asn Val Ala Ser Ile Asn Phe Gly Asp
   465 470 475 480
   Ile Asn Lys Thr Tyr Val Val Leu Val Glu Gly His Tyr Asp Lys Thr
   485 490 495
   Gly Lys Asn Leu Lys Thr Gln Val Ile Gln Glu Asn Val Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 33
   <211> 1626
   <212> DNA
   <213> Staphylococcus aureus
<400> 33
   ttgaaaaaaa gaattgatta tttgtcgaat aagcagaata agtattcgat tagacgtttt     60
   acagtaggta ccacatcagt aatagtaggg gcaactatac tatttgggat aggcaatcat    120
   caagcacaag cttcagaaca atcgaacgat acaacgcaat cttcgaaaaa taatgcaagt    180
   gcagattccg aaaaaaacaa tatgatagaa acacctcaat taaatacaac ggctaatgat    240
   acatctgata ttagtgcaaa cacaaacagt gcgaatgtag atagcacagc aaaaccaatg    300
   tctacacaaa cgagcaatac cactacaaca gagccagctt caacaaatga aacacctcaa    360
   ctgacggcaa ttaaagatca agcaactgct gcaaaaatgc aagatcaaac tgttcctcaa    420
   gaagcaaatt ctcaagtaga taataaaaca acgaatgatg ctaatagcat agcgacaaac    480
   agtgagctta aaaatcctca aacattagat ttaccacaat catcaccaca aacaatttcc    540
   aatgcgcaag gaactagtaa accaagtgtt agaacgagag ctgtacgtag tcttgcagtt    600
   gctgaacctg tagtaaatgc tgctgatgct aaaggtacaa atgtaaatga taaagttacg    660
   gcaaaagatt ttcaattaga aaagactaca tttgacccta accaaagtgg taatactttt    720
   atggcggcaa actttacagt gactggacaa gtgaaatcag gggattattt tacagcgaag    780
   ttaccagata gtgtaactgg taatggagac gtggattact ctaattcgaa taatacgatg    840
   ccaattgcag atattaaaag cacgaatggt gatgttgtag ctaaagcaac atatgatatc    900
   ttgactaaga catatacatt tgtctttaca gattatgtaa atgaaaaaga aaatattaac    960
   ggacaatttt cattaccttt atttacagac agagcaaagg cacctaaatc aggaacatac   1020
   gatgcaaata ttaatattgc ggatgaaatg tttgataata aaattactta taactatagt   1080
   tcgcctattg caggaattga taaaccaaat ggcgcaaata tttcttctca aattattggt   1140
   gtagatacag cttcaggtca aaacacatac aagcaaacag tatttgttaa ccctaagcaa   1200
   cgagttttag gtaatacgtg ggtgtatatt aaaggttacc aagataaaat cgaagaaagt   1260
   agcggtaaag taagtgctac agatacaaaa ctgagaattt ttgaagtgaa tgatacatct   1320
   aaattatcag atagctacta tgcggaccca aatgactcta accttaaaga agtaacgaat   1380
   gaatttaagg ataaaatcac ttataaatac caaaatgtag caagtattaa ttttggcgat   1440
   attactaaaa cgtatgttgt attagtggaa ggtcactatg ataatactgg taaaaacttg   1500
   aaaacacagg ttattcaaga aaatattgac ccagcgacag gtaaagacta cagtattttc   1560
   ggttggaata atgagaatgt tgtacgttat ggtggtggaa gtgctgatgg tgattcagca   1620
   gtaaat                                                              1626
<210> 34
   <211> 542
   <212> PRT
   <213> Staphylococcus aureus
<400> 34
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Leu Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Lys Asp Phe
       210                 215                 220
   Gln Leu Glu Lys Thr Thr Phe Asp Pro Asn Gln Ser Gly Asn Thr Phe
   225                 230                 235                 240
   Met Ala Ala Asn Phe Thr Val Thr Gly Gln Val Lys Ser Gly Asp Tyr
                   245                 250                 255
   Phe Thr Ala Lys Leu Pro Asp Ser Val Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
   290 295 300
   Tyr Thr Phe Val Phe Thr Asp Tyr Val Asn Glu Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asp
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asn Glu Phe Lys Asp
   450 455 460
   Lys Ile Thr Tyr Lys Tyr Gln Asn Val Ala Ser Ile Asn Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 35
   <211> 1626
   <212> DNA
   <213> Staphylococcus aureus
<400> 35
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaataa tatgatagaa acacctcaat taaatacaac ggctaatgat     240
   acatctgata ttagtgcaaa cacaaacagt gcgaatgtag atagcacagc aaaaccaatg     300
   tctacacaaa cgagcaatac cactacaaca gagccagctt caacaaatga aacacctcaa     360
   ccgacggcaa ttaaagatca agcaactgct gcaaaaatgc aagatcaaac tgttcctcaa     420
   gaagcaaatt ctcaagtaga taataaaaca acgaatgatg ctaatagcat agcaacaaac     480
   agtgaactta aaaatcctca aacattagat ttaccacaat catcaccaca aacgatttcc     540
   aatgcgcaag gaactagtaa accaagtgtt agaacgagag ctgtacgtag tcttgcagtt     600
   gctgaacctg tagtaaatgc tgctgatgct aaaggtacaa atgtaaatga taaagttacg     660
   gcaagtaatc tacagttgca aaagactaca tttgacccta accaaagtgg aaatacattt     720
   atggcggcaa attttacagt gacagataaa gtgaaatcag gggattattt tacagcgaag  780
   ttaccagata gtttaactgg taatggagac gtggattact ctaattcaaa taatacgatg     840
   ccaattgcag atattaaaag tacgaatggc gatgttgtag ctaaagcaac atatgatatc     900
   ttgactaaga cgtatacatt tgtctttaca gattatgtaa atgataaaga aaatattaac     960
   gggcaatttt cattaccttt atttacagac cgagcaaagg cacctaaatc aggaacatat    1020
   gatgcaaata ttaatattgc ggatgaaatg tttaataata aaattactta taactatagt    1080
   tcgccaattg caggaattga taagccaaat ggcgcgaaca tttcttctca aattattggt    1140
   gtagatacag cttcaggtca aaacacatac aagcaaacgg tatttgttaa ccctaagcaa    1200
   cgagttttag gtaatacgtg ggtgtatatt aaaggttacc aagataaaat cgaagaaagt    1260
   agcggtaaag taagtgctac agatacaaaa ctgagaattt ttgaagtgaa tgatacatct    1320
   aaattatcag atagctacta tgcagaccca aatgactcta accttaaaga agtaactgat    1380
   caatttaagg ataaaatcac ttataaatac caaaacgtag caagtattaa ttttggtgat    1440
   ataaataaaa cgtatgtcgt tttagttgaa ggtcactatg ataatactgg aaaaaacttg    1500
   aaaacacagg ttattcaaga aaatattgac ccagcgacag gtaaagacta cagtattttc    1560
   ggatggaata atgagaatgt tgtacgttat ggtggtggaa gtgctgatgg tgattcagca    1620
   gtaaat                                                               1626
<210> 36
   <211> 542
   <212> PRT
   <213> Staphylococcus aureus
<400> 36
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Ser Asn Leu
       210                 215                 220
   Gln Leu Gln Lys Thr Thr Phe Asp Pro Asn Gln Ser Gly Asn Thr Phe
   225 230 235 240
   Met Ala Ala Asn Phe Thr Val Thr Asp Lys Val Lys Ser Gly Asp Tyr
   245 250 255
   Phe Thr Ala Lys Leu Pro Asp Ser Leu Thr Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asp Val Val Ala Lys Ala Thr Tyr Asp Ile Leu Thr Lys Thr
   290 295 300
   Tyr Thr Phe Val Phe Thr Asp Tyr Val Asn Asp Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asp Gln Phe Lys Asp
   450 455 460
   Lys Ile Thr Tyr Lys Tyr Gln Asn Val Ala Ser Ile Asn Phe Gly Asp
   465 470 475 480
   Ile Asn Lys Thr Tyr Val Val Leu Val Glu Gly His Tyr Asp Asn Thr
                   485                 490                 495
   Gly Lys Asn Leu Lys Thr Gln Val Ile Gln Glu Asn Ile Asp Pro Ala
               500                 505                 510
   Thr Gly Lys Asp Tyr Ser Ile Phe Gly Trp Asn Asn Glu Asn Val Val
           515                 520                 525
   Arg Tyr Gly Gly Gly Ser Ala Asp Gly Asp Ser Ala Val Asn
       530                 535                 540
<210> 37
   <211> 1626
   <212> DNA
   <213> Staphylococcus aureus
<400> 37
   ttgaaaaaaa gaattgatta tttgtcgaat aagcagaata agtattcgat tagacgtttt 60
   acagtaggta ccacatcagt aatagtaggg gcaactatac tatttgggat aggcaatcat 120
   caagcacaag cttcagaaca atcgaacgat acaacgcaat cttcgaaaaa taatgcaagt 180
   gcagattccg aaaaaaacaa tatgatagaa acacctcaat taaatacaac ggctaatgat 240
   acatctgata ttagtgcaaa cacaaacagt gcgaatgtag atagcacaac aaaaccaatg 300
   tctacacaaa cgagcaatac cactacaaca gagccagctt caacaaatga aacacctcaa 360
   ccgacggcaa ttaaagatca agcaactgct gcaaaaatgc aagatcaaac tgttcctcaa 420
   gaagcaaatt ctcaagtaga taataaaaca acgaatgatg ctaatagcat agcgacaaac 480
   agtgagctta aaaatcctca aacattagat ttaccacaat catcaccaca aacaatttcc 540
   aatgcgcaag gaactagtaa accaagtgtt agaacgagag ctgtacgtag tcttgcagtt 600
   gctgaacctg tagtaaatgc tgctgatgct aaaggtacaa atgtaaatga taaagttacg 660
   gcaaaagatt ttcaattaga aaagactaca tttgacccta accaaagtgg taatactttt 720
   atggcggcaa actttacagt gactggacaa gtgaaatcag gggattattt tacagcgaag 780
   ttaccagata gtgtaactgg taatggagac gtggattact ctaattcgaa taatacgatg 840
   ccaattgcag atattaaaag cacgaatggt gatgttgtag ctaaagcaac atatgatatc 900
   ttgactaaga catatacatt tgtctttaca gattatgtaa atgaaaaaga aaatattaac 960
   ggacaatttt cattaccttt atttacagac agagcaaagg cacctaaatc aggaacatac 1020
   gatgcaaata ttaatattgc ggatgaaatg tttgataata aaattactta taactatagt 1080
   tcgcctattg caggaattga taaaccaaat ggcgcaaata tttcttctca aattattggt 1140
   gtagatacag cttcaggtca aaacacatac aagcaaacag tatttgttaa ccctaagcaa 1200
   cgagttttag gtaatacgtg ggtgtatatt aaaggttacc aagataaaat cgaagaaagt 1260
   agcggtaaag taagtgctac agatacaaaa ctgagaattt ttgaagtgaa tgatacatct 1320
   aaattatcag atagctacta tgcggaccca aatgactcta accttaaaga agtaacgaat 1380
   gaatttaagg ataaaatcac ttataaatac caaaatgtag caagtattaa ttttggcgat 1440
   attactaaaa cgtatgttgt attagtggaa ggtcactatg ataatactgg taaaaacttg 1500
   aaaacacagg ttattcaaga aaatattgac ccagcgacag gtaaagacta cagtattttc 1560
   ggttggaata atgagaatgt tgtacgttat ggtggtggaa gtgctgatgg tgattcagca 1620
   gtaaat 1626
<210> 38
   <211> 542
   <212> PRT
   <213> Staphylococcus aureus
<400> 38
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Thr Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Lys Asp Phe
       210                 215                 220
   Gln Leu Glu Lys Thr Thr Phe Asp Pro Asn Gln Ser Gly Asn Thr Phe
   225                 230                 235                 240
   Met Ala Ala Asn Phe Thr Val Thr Gly Gln Val Lys Ser Gly Asp Tyr
                   245                 250                 255
   Phe Thr Ala Lys Leu Pro Asp Ser Val Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Glu Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asp
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asn Glu Phe Lys Asp
   450 455 460
   Lys Ile Thr Tyr Lys Tyr Gln Asn Val Ala Ser Ile Asn Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 39
   <211> 1626
   <212> DNA
   <213> Staphylococcus aureus
<400> 39
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacacaat cttcgaaaaa taatgcaagt     180
   gcagattccg aaaaaaacaa tatgatagaa acacctcaat taaatacaac ggctaatgat     240
   acatctgata ttagtgcaaa cacaaacagt gcgaatgtag atagcacagc aaaaccaatg     300
   tctacacaaa cgagcaatac cactacaaca gagccagctt caacaaatga aacacctcaa     360
   ccgacggcaa ttaaagatca agcaactgct gcaaaaatgc aagatcaaac tgttcctcaa     420
   gaagcaaatt ctcaagtaga taataaaaca acgaatgatg ctaatagcat agcaacaaac     480
   agtgagctta aaaatcctca aacattagat ttaccacaat catcaccaca aacgatttcc     540
   aatgcgcaag gaactagtaa accgagtgtt agaacgagag ctgtacgtag tcttgcagtt     600
   gctgaacctg tagtaaatgc tgctgatgct aaaggtacaa atgtaaatgg tcaagttacg     660
   gcaagtgatt tcaagttaga aaagactaca tttgacccta accaaagtgg taacacattt     720
   atggcggcaa aatttacagt gactggacaa gtgaaagcag gggattattt tacagcgaag     780
   ttaccagata gtgtaaatgg taatggagat gtggattact ctaattcaaa taatacgatg     840
   ccaattgcag atattaaaag tacgaatggc gatgttgtag ctaaagcaac atatgatatc     900
   ttgactaaga cgtatacatt tgtctttaca gattatgtaa ataataaaga aaatattaac     960
   ggacaatttt cattaccttt atttacagac cgagcaaagg cacctaaatc aggaacatat    1020
   gatgcgaata ttaatattgc ggatgaaatg tttaataata aaattactta taactatagt    1080
   tcgccaattg caggaattga taaaccaaat ggcgcgaaca tttcttctca aattattggt    1140
   gtagatacag cttcaggtca aaacacatac aagcaaacag tatatgttaa ccctaagcaa    1200
   cgagttttag gtaatacgtg ggtgtatatt aaaggttacc aagataaaat cgaagaaagt    1260
   agcggtaaag taagtgctac agatacaaaa ctgagaattt ttgaagtgaa tgatacatct    1320
   aaattatcag atagctacta tgcagaccca aatgattcga atcttaaaga agtaacgaat    1380
   gagtttaagg ataaaatcac ttataaatac caaaatgtag caagtattaa ttttggcgat    1440
   attactaaaa cgtatgttgt attagtggaa ggtcactatg ataatactgg taaaaacttg    1500
   aaaacacagg ttattcaaga aaatattgac ccagcgacag gtaaagacta cagtattttc    1560
   ggttggaata atgagaatgt tgtacgttat ggtggtggaa gtgctgatgg tgattcagca    1620
   gtaaat                                                               1626
<210> 40
   <211> 542
   <212> PRT
   <213> Staphylococcus aureus
<400> 40
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
   100 105 110
   Ala Ser Thr Asn Glu Thr Pro Gln Pro Thr Ala Ile Lys Asp Gln Ala
   115 120 125
   Thr Ala Ala Lys Met Gln Asp Gln Thr Val Pro Gln Glu Ala Asn Ser
   130 135 140
   Gln Val Asp Asn Lys Thr Thr Asn Asp Ala Asn Ser Ile Ala Thr Asn
   145 150 155 160
   Ser Glu Leu Lys Asn Pro Gln Thr Leu Asp Leu Pro Gln Ser Ser Pro
   165 170 175
   Gln Thr Ile Ser Asn Ala Gln Gly Thr Ser Lys Pro Ser Val Arg Thr
   180 185 190
   Arg Ala Val Arg Ser Leu Ala Val Ala Glu Pro Val Val Asn Ala Ala
   195 200 205
   Asp Ala Lys Gly Thr Asn Val Asn Gly Gln Val Thr Ala Ser Asp Phe
   210 215 220
   Lys Leu Glu Lys Thr Thr Phe Asp Pro Asn Gln Ser Gly Asn Thr Phe
   225 230 235 240
   Met Ala Ala Lys Phe Thr Val Thr Gly Gln Val Lys Ala Gly Asp Tyr
   245 250 255
   Phe Thr Ala Lys Leu Pro Asp Ser Val Asn Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asp Val Val Ala Lys Ala Thr Tyr Asp Ile Leu Thr Lys Thr
   290 295 300
   Tyr Thr Phe Val Phe Thr Asp Tyr Val Asn Asn Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Tyr Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asn Glu Phe Lys Asp
   450 455 460
   Lys Ile Thr Tyr Lys Tyr Gln Asn Val Ala Ser Ile Asn Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 41
   <211> 1626
   <212> DNA
   <213> Staphylococcus aureus
<400> 41
   ttgaaaaaaa gaattgatta tttgtcgaat aggcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tatgatagaa acacctcaat taaatacaac ggctaatgat     240
   acatctgata ttagtgcaaa cacaaacagt gcgaatgtag atagcacaac aaaaccaatg     300
   tctacacaaa cgagcaatac cactacaaca gagccagctt caacaaatga aacacctcaa     360
   ccgacggcaa ttaaaaatca agcaactgct gcaaaaatgc aagatcaaac tgttcctcaa     420
   gaagcaaatt ctcaagtaga taataaaaca acgaatgatg ctaatagcat agcaacaaac     480
   agtgagctta aaaattctca aacattagat ttaccacaat catcaccaca aacgatttcc     540
   aatgcgcaag gaactagtaa accaagtgtt agaacgagag ctgtacgtag tttagctgtt     600
   gctgaaccgg tagtaaatgc tgctgatgct aaaggtacaa atgtaaatga taaagttacg     660
   gcaagtaatt tcaagttaga aaagactaca tttgacccta atcaaagtgg taacacattt     720
   atggcggcaa attttacagt gacagataaa gtgaaatcag gggattattt tacagcgaag      780
   ttaccagata gtttaactgg taatggagac gtggattatt ctaattcaaa taatacgatg     840
   ccaattgcag acattaaaag tacgaatggc gatgttgtag ctaaagcaac atatgatatc     900
   ttgactaaga cgtatacatt tgtctttaca gattatgtaa ataataaaga aaatattaac     960
   ggacaatttt cattaccttt atttacagac cgagcaaagg cacctaaatc aggaacatat    1020
   gatgcgaata ttaatattgc ggatgaaatg tttaataata aaattactta taactatagt    1080
   tcgccaattg caggaattga taaaccaaat ggcgcgaaca tttcttctca aattattggt    1140
   gtagatacag cttcaggtca aaacacatac aagcaaacag tatttgttaa ccctaagcaa    1200
   cgagttttag gtaatacgtg ggtgtatatt aaaggctacc aagataaaat cgaagaaagt    1260
   agcggtaaag taagtgctac agatacaaaa ctgagaattt ttgaagtgaa tgatacatct    1320
   aaattatcag atagctacta tgcagatcca aatgactcta accttaaaga agtaacagac    1380
   caatttaaaa atagaatcta ttatgagcat ccaaatgtag ctagtattaa atttggtgat    1440
   attactaaaa catatgtagt attagtagaa gggcattacg acaatacagg taagaactta    1500
   aaaactcagg ttattcaaga aaatgttgat cctgtaacaa atagagacta cagtattttc    1560
   ggttggaata atgagaatgt tgtacgttat ggtggtggaa gtgctgatgg tgattcagca    1620
   gtaaat                                                               1626
<210> 42
   <211> 542
   <212> PRT
   <213> Staphylococcus aureus
<400> 42
   Leu Lys Lys Arg Ile Asp Tyr Leu Ser Asn Arg Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Thr Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asn Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Ser Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Ser Asn Phe
       210                 215                 220
   Lys Leu Glu Lys Thr Thr Phe Asp Pro Asn Gln Ser Gly Asn Thr Phe
   225                 230                 235                 240
   Met Ala Ala Asn Phe Thr Val Thr Asp Lys Val Lys Ser Gly Asp Tyr
                   245                 250                 255
   Phe Thr Ala Lys Leu Pro Asp Ser Leu Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asn Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Asp Gln Phe Lys Asn
       450                 455                 460
   Arg Ile Tyr Tyr Glu His Pro Asn Val Ala Ser Ile Lys Phe Gly Asp
   465                 470                 475                 480
   Ile Thr Lys Thr Tyr Val Val Leu Val Glu Gly His Tyr Asp Asn Thr
                   485                 490                 495
   Gly Lys Asn Leu Lys Thr Gln Val Ile Gln Glu Asn Val Asp Pro Val
               500                 505                 510
   Thr Asn Arg Asp Tyr Ser Ile Phe Gly Trp Asn Asn Glu Asn Val Val
           515                 520                 525
   Arg Tyr Gly Gly Gly Ser Ala Asp Gly Asp Ser Ala Val Asn
           530                 535                 540
<210> 43
   <211> 1626
   <212> DNA
   <213> Staphylococcus aureus
<400> 43
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tatgatagaa acacctcaat taaatacaac ggctaatgat     240
   acatctgata ttagtgcaaa cacaaacagt gcgaatgtag atagcacaac aaaaccaatg     300
   tctacacaaa cgagcaatac cactacaaca gagccagctt caacaaatga aacacctcaa     360
   ccgacggcaa ttaaaaatca agcaactgct gcaaaaatgc aagatcaaac tgttcctcaa     420
   gaagcaaatt ctcaagtaga taataaaaca acgaatgatg ctaatagcat agcaacaacc     480
   agtgagctta aaaattctca aacattagat ttaccacaat catcaccaca aacgatttcc     540
   aatgcgcaag gaactagtaa accaagtgtt agaacgagag ctgtacgtag tttagctgtt     600
   gctgaaccgg tagtaaatgc tgctgatgct aaaggtacaa atgtaaatga taaagttacg     660
   gcaagtaatt tcaagttaga aaagactaca tttgacccta atcaaagtgg taacacattt     720
   atggcggcaa attttacagt gacagataaa gtgaaatcag ggdattattt tacagcgaag     780
   ttaccagata gtttaactgg taatggagac gtggattatt ctaattcaaa taatacgatg     840
   ccaattgcag acattaaaag tacgaatggc gatgttgtag ctaaagcaac atatgatatc     900
   ttgactaaga cgtatacatt tgtctttaca gattatgtaa ataataaaga aaatattaac     960
   ggacaatttt cattaccttt atttacagac cgagcaaagg cacctaaatc aggaacatat    1020
   gatgcgaata ttaatattgc ggatgaaatg tttaataata aaattactta taactatagt    1080
   tcgccaattg caggaattga taaaccaaat ggcgcgaaca tttcttctca aattattggt    1140
   gtagatacag cttcaggtca aaacacatac aagcaaacag tatttgttaa ccctaagcaa    1200
   cgagttttag gtaatacgtg ggtgtatatt aaaggctacc aagataaaat cgaagaaagt    1260
   agcggtaaag taagtgctac agatacaaaa ctgagaattt ttgaagtgaa tgatacatct    1320
   aaattatcag atagctacta tgcagatcca aatgactcta accttaaaga agtaacagac    1380
   caatttaaaa atagaatcta ttatgagcat ccaaatgtag ctagtattaa atttggtgat    1440
   attactaaaa catatgtagt attagtagaa gggcattacg acaatacagg taagaactta    1500
   aaaactcagg ttattcaaga aaatgttgat cctgtaacaa atagagacta cagtattttc    1560
   ggttggaata atgagaatgt tgtacgttat ggtggtggaa gtgctgatgg tgattcagca 1620
   gtaaat 1626
<210> 44
   <211> 542
   <212> PRT
   <213> Staphylococcus aureus
<400> 44
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Thr Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asn Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Thr
   145                 150                 155                 160
   Ser Glu Leu Lys Asn Ser Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Ser Asn Phe
       210                 215                 220
   Lys Leu Glu Lys Thr Thr Phe Asp Pro Asn Gln Ser Gly Asn Thr Phe
   225                 230                 235                 240
   Met Ala Ala Asn Phe Thr Val Thr Asp Lys Val Lys Ser Gly Asp Tyr
                   245                 250                 255
   Phe Thr Ala Lys Leu Pro Asp Ser Leu Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asn Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Asp Gln Phe Lys Asn
       450                 455                 460
   Arg Ile Tyr Tyr Glu His Pro Asn Val Ala Ser Ile Lys Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Val Asp Pro Val
   500 505 510
   Thr Asn Arg Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 45
   <211> 1626
   <212> DNA
   <213> Staphylococcus aureus
<400> 45
   ttgaaaaaaa gaattgatta tttgtcgaat aagcagaata agtattcgat tagacgtttt 60
   acagtaggta ccacatcagt aatagtaggg gcaactatac tatttgggat aggcaatcat 120
   caagcacaag cttcagaaca atcgaacgat acaacgcaat cttcgaaaaa taatgcaagt 180
   gcagattccg aaaaaaacaa tatgatagaa acacctcaat taaatacaac ggctaatgat 240
   acatctgata ttagtgcaaa cacaaacagt gcgaatgtag atagcacagc aaaaccaatg 300
   tctacacaaa cgagcaatac cactacaaca gagccagctt caacaaatga aacacctcaa 360
   ccgacggcaa ttaaagatca agcaactgct gcaaaaatgc aagatcaaac tgttcctcaa 420
   gaagcaaatt ctcaagtaga taataaaaca acgaatgatg ctaatagcat agcgacaaac 480
   tgtgagctta aaaatcctca aacattagat ttaccacaat catcaccaca aacaatttcc 540
   aatgcgcaag gaactagtaa accaagtgtt agaacgagag ctgtacgtag tcttgcagtt 600
   gctgaacctg tagtaaatgc tgctgatgct aaaggtacaa atgtaaatga taaagttacg 660
   gcaaaagatt ttcaattaga aaagaccaca tttgacccta accaaagtgg taatactttt 720
   atggcggcaa actttacagt gactggacaa gtgaaatcag gggattattt tacagcgaag 780
   ttaccagata gtgtaactgg taatggagac gtggattact ctaattcgaa taatacgatg 840
   ccaattgcag atattaaaag tacgaatggc gatgttgtag ctaaagcaac atatgatatc 900
   ttgactaaga cgtatacatt tgtctttaca gattatgtaa ataataaaga aaatattaac 960
   ggacaatttt cattaccttt atttacagac cgagcaaagg cacctaaatc aggaacatat 1020
   gatgcgaata ttaatattgc ggatgaaatg tttaataata aaattactta taactatagt 1080
   tcgccaattg caggaattga taaaccaaat ggcgcgaaca tttcttctca aattattggt 1140
   gtagatacag cttcaggtca aaacacatac aagcaaacag tatttgttaa ccctaagcaa 1200
   cgagttttag gtaatacgtg ggtgtatatt aaaggttacc aagataaaat cgaagaaagt 1260
   agcggtaaag taagtgctac agatacaaaa ctgagaattt ttgaagtgaa tgatacatct 1320
   aaattatcag atagctacta tgcggaccca aatgactcta accttaaaga agtaacgaat 1380
   gagtttaagg ataaaatcac ttataaatac caaaatgtag caagtattaa ttttggcgat 1440
   attactaaaa cgtatgttgt attagtggaa ggtcactatg ataatactgg taaaaacttg 1500
   aaaacacagg ttattcaaga aaatattgac ccagcgacag gtaaagacta cagtattttc 1560
   ggttggaata atgagaatgt tgtacgttat ggtggtggaa gtgctgatgg tgattcagca 1620
   gtaaat 1626
<210> 46
   <211> 542
   <212> PRT
   <213> Staphylococcus aureus
<400> 46
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Cys Glu Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Lys Asp Phe
       210                 215                 220
   Gln Leu Glu Lys Thr Thr Phe Asp Pro Asn Gln Ser Gly Asn Thr Phe
   225                 230                 235                 240
   Met Ala Ala Asn Phe Thr Val Thr Gly Gln Val Lys Ser Gly Asp Tyr
                   245                 250                 255
   Phe Thr Ala Lys Leu Pro Asp Ser Val Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asn Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asn Glu Phe Lys Asp
   450 455 460
   Lys Ile Thr Tyr Lys Tyr Gln Asn Val Ala Ser Ile Asn Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 47
   <211> 1626
   <212> DNA
   <213> Staphylococcus aureus
<400> 47
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tacgatagaa acacctcaat taaatacaac ggctaatgat     240
   acatctgata ttagtgcaaa cacaaacagt gcgaatgtag atagcacagc aaaaccaatg     300
   tctacacaaa cgagcaatac cactacaaca gagccagctt caacaaatga aacacctcaa     360
   ccgacggcaa ttaaagatca agcaactgct gcaaaaatgc aagatcaaac tgttcctcaa     420
   gaagcaaatt ctcaagtaga taataaaaca acgaatgatg ctaatagcat agcgacaaac     480
   agcgagctta aaaatcctca gacattagat ttaccacaat catcaccaca aacgatttcc     540
   aatgcgcaag gaactagtga accaagtgtt agaacgagag ctgtacgtag tcttgcagtt     600
   gctgaacctg tagtaaatgc tgctgatgct aaaggtacaa atgtaaatga taaagttacg     660
   gcaagtgatt tcaagttaga aaagactaca tttgacccta accaaagtgg taacacattt     720
   atggcggcaa attttaaagt ggcagggaaa gtgaaatcag gggattattt tacagcgaag     780
   ttaccagata gtgtaactgg taatggagac gtggattact ctaattcaaa taatacgatg     840
   ccaattgcag atatagtgaa tgataaaaaa gaagttgtag ctaaagcaac atatgatatc     900
   ttgactaaga cgtatacatt tgtctttaca gattatgtaa atgataaaga aaatattaac     960
   ggacaatttt cattaccttt atttacagac cgagcaaagg cacctaaatc aggaacatat    1020
   gatgcaaata ttaatattgc ggatgaaatg tttaacaatc aaattactta taactatagt    1080
   tcaccaattg caggaattga taagccaaat ggcgcgaaca tttcttctca aattattggt    1140
   gtagatacag cttcaggtca aaacacatac aagcaaacgg tatttgttaa ccctaagcaa    1200
   cgagttttag gtaatacgtg ggtgtatatt aaaggttacc aagataaaat cgaagaaagt    1260
   agcggtaaag taagtgctac agatacaaaa ctgagaattt ttgaagtgaa tgatacatct    1320
   aaattatcag atagctacta tgcagaccca aatgactcta accttaaaga agtaactaat    1380
   gagtttaagg ataaaatcac ttataaatac caaaacgtag caagtattaa ttttggtgat    1440
   ataaataaaa cgtatgtcgt tttagtcgaa ggtcattatg ataatactgg taaaaacttg    1500
   aaaacacagg ttattcaaga aaatattgac ccagcgacag gtaaagatta cagtattttc    1560
   ggttggaata atgagaatgt tgtacgttat ggaggcggaa gtgctgatgg tgattcagca    1620
   gtaaat                                                               1626
<210> 48
   <211> 542
   <212> PRT
   <213> Staphylococcus aureus
<400> 48
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Thr Ile Glu Thr Pro Gln Leu Asn Thr Thr Ala Asn Asp
   65 70 75 80
   Thr Ser Asp Ile Ser Ala Asn Thr Asn Ser Ala Asn Val Asp Ser Thr
   85 90 95
   Ala Lys Pro Met Ser Thr Gln Thr Ser Asn Thr Thr Thr Thr Glu Pro
   100 105 110
   Ala Ser Thr Asn Glu Thr Pro Gln Pro Thr Ala Ile Lys Asp Gln Ala
   115 120 125
   Thr Ala Ala Lys Met Gln Asp Gln Thr Val Pro Gln Glu Ala Asn Ser
   130 135 140
   Gln Val Asp Asn Lys Thr Thr Asn Asp Ala Asn Ser Ile Ala Thr Asn
   145 150 155 160
   Ser Glu Leu Lys Asn Pro Gln Thr Leu Asp Leu Pro Gln Ser Ser Pro
   165 170 175
   Gln Thr Ile Ser Asn Ala Gln Gly Thr Ser Glu Pro Ser Val Arg Thr
   180 185 190
   Arg Ala Val Arg Ser Leu Ala Val Ala Glu Pro Val Val Asn Ala Ala
   195 200 205
   Asp Ala Lys Gly Thr Asn Val Asn Asp Lys Val Thr Ala Ser Asp Phe
   210 215 220
   Lys Leu Glu Lys Thr Thr Phe Asp Pro Asn Gln Ser Gly Asn Thr Phe
   225 230 235 240
   Met Ala Ala Asn Phe Lys Val Ala Gly Lys Val Lys Ser Gly Asp Tyr
   245 250 255
   Phe Thr Ala Lys Leu Pro Asp Ser Val Thr Gly Asn Gly Asp Val Asp
   260 265 270
   Tyr Ser Asn Ser Asn Asn Thr Met Pro Ile Ala Asp Ile Val Asn Asp
   275 280 285
   Lys Lys Glu Val Val Ala Lys Ala Thr Tyr Asp Ile Leu Thr Lys Thr
   290 295 300
   Tyr Thr Phe Val Phe Thr Asp Tyr Val Asn Asp Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Gln Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asn Glu Phe Lys Asp
   450 455 460
   Lys Ile Thr Tyr Lys Tyr Gln Asn Val Ala Ser Ile Asn Phe Gly Asp
   465 470 475 480
   Ile Asn Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 49
   <211> 1626
   <212> DNA
   <213> Staphylococcus aureus
<400> 49
   ttgaaaaaaa gaattgatta tttgtcgaat aagcagaata agtattcgat tagacgtttt        60
   acagtaggta ccacatcagt aatagtaggg gcaactatac tatttgggat aggcaatcat       120
   caagcacaag cttcagaaca atcgaacgat acaacgcaat cttcgaaaaa taatgcaagt       180
   gcagattccg aaaaaaacaa tatgatagaa acacctcaat taaatacaac ggctaatgat       240
   acatctgata ttagtgcaaa cacaaacagt gcgaatgtag atagcacagc aaaaccaatg       300
   tctacacaaa cgagcaatac cactacaaca gagccagctt caacaaatga aacacctcaa       360
   ccgacggcaa ttaaagatca agcaactgct gcaaaaatgc aagatcaaac tgttcctcaa       420
   gaagcaaatt ctcaagtaga taataaaaca acgaatgatg ctaatagcat agcgacaaac       480
   agtgagctta aaaatcctca aacattagat ttaccacaat catcaccaca aacgatttcc       540
   aatgcgcaaa gaactagtaa accaagtgtt agaaccagag ctgtacgtag tttagctgtt       600
   gctgaaccgg tagtaaatgc tgctgatgct aaaggtacaa atgtaaatga taaagttacg       660
   gcaagtaatc tacagttgca aaagactacg tttgacccta accaaagtgg taacacattt       720
   atggcggcaa attttacagt gacagataaa gtgaaatcag gagattattt tacagcgaag       780
   ttaccagata gtttaactgg taatggagac gtggattact ctaattcaaa taatacgatg       840
   ccaattgcag atattaaaag tacgaatggc gatgttgtag ctaaagcaac atatgatatc       900
   ttgactaaga cgtatacatt tgtctttaca gattatgtaa atgataaaga aaatattaac       960
   ggacaatttt cattaccttt atttacagac cgagcaaagg cacctaaatc aggaacatac      1020
   gatgcaaata ttaatattgc ggatgaaatg tttaataata aaattactta taactatagt      1080
   tcgccaattg caggaattga taagccaaat ggcgcgaaca tttcttctca gattattggt      1140
   gtagatacag cttcaggtca aaacacatac aagcaaacgg tatttgttaa ccctaagcaa      1200
   cgagttttag gtaatacgtg ggtgtatatt aaaggctacc aagataaaat cgaagaaagt      1260
   agcggtaaag taagtgctaa agatacaaaa ctgagaattt ttgaagtgaa tgatacatct      1320
   aaattatcag atagctacta tgcagaccca aatgattcga atcttaaaga agtaacagac      1380
   caatttaaaa atagaatcta ttatgagcat ccaaatgtag ctagtattaa ttttggcgat      1440
   attactaaaa cgtatgttgt attagtggaa ggtcactatg ataatactgg taaaaacttg      1500
   aaaacacagg ttattcaaga aaatattgac ccagcgacag gtaaagacta cagtattttc      1560
   ggttggaata atgagaatgt tgtacgttat ggtggtggaa gtgctgatgg tgattcagca      1620
   gtaaat                                                                 1626
<210> 50
   <211> 542
   <212> PRT
   <213> Staphylococcus aureus
<400> 50
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Arg Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Ser Asn Leu
       210                 215                 220
   Gln Leu Gln Lys Thr Thr Phe Asp Pro Asn Gln Ser Gly Asn Thr Phe
   225                 230                 235                 240
   Met Ala Ala Asn Phe Thr Val Thr Asp Lys Val Lys Ser Gly Asp Tyr
                   245                 250                 255
   Phe Thr Ala Lys Leu Pro Asp Ser Leu Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asp Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asn
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Lys Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Asp Gln Phe Lys Asn
       450                 455                 460
   Arg Ile Tyr Tyr Glu His Pro Asn Val Ala Ser Ile Asn Phe Gly Asp
   465                 470                 475                 480
   Ile Thr Lys Thr Tyr Val Val Leu Val Glu Gly His Tyr Asp Asn Thr
                   485                 490                 495
   Gly Lys Asn Leu Lys Thr Gln Val Ile Gln Glu Asn Ile Asp Pro Ala
               500                 505                 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 51
   <211> 1626
   <212> DNA
   <213> Staphylococcus aureus
<400> 51
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tatgatagaa acacctcaat taaatacaac ggctaatgat     240
   acatctgata ttagtgcaaa cacaaacagt gcgaatgtag atagcacagc aaaaccaatg     300
   tctacacaaa cgagcaatac cactacaaca gagccagctt caacaaatga aacacctcaa     360
   ccgacggcaa ttaaagatca agcaactgct gcaaaaatgc aagatcaaac tgttcctcaa     420
   gaagcaaatt ctcaagtaga taataaaaca acgaatgatg ctaataacat agcaacaaac     480
   agtgggctta aaaatcctca aacattagat ttaccacaat catcaccaca aacgatttcc     540
   aatgcgcaag gaactagtaa accaagtgtt agaacgagag ctgtacgtag tcttgcagtt     600
   gctgaacctg tagtaaatgc tgctgatgct aaaggtacaa atgtaaatga taaagttacg     660
   gcaagtgatt tcaagttaga aaagactaca ttcgacccta accaaagtgg taacacattt     720
   atggcggcaa attttacagt gacagataaa gtgaaatcag gggattattt tacagcgaag      780
   ttaccagata gtttaactgg taatggagac gtggattact ctaattcaaa taatacgatg     840
   ccaattgcag acattaaaag tacgaatggt gatgttgtag cgacagcgac ttataatatc     900
   ttgactaaga cgtatacatt tgtctttaca gattatgtaa atgataaaga aaatattaac     960
   ggacaatttt cattaccttt atttacagac cgagcaaagg cacctaaatc aggaacatat    1020
   gatgcaaata ttaatattgc ggatgaaatg tttaataata aaattactta taactatagt    1080
   tcgccaattg caggaattga taagccaaat ggcgcgaaca tttcttctca aattattggt    1140
   gtagatacag cttcaggcca aaacacatac aagcaaacgg tatttgttaa ccctaagcaa    1200
   cgagttttag gtaatacgtg ggtgtatatt aaaggctacc aagataaaat cgaagaaagt    1260
   agcggtaaag taagtgctac agatacaaaa ctgagaattt ttgaagtgaa tgatacatct    1320
   aaattatcag atagctacta tgcggaccca aatgactcta atcttaaaga agtaacgaat    1380
   gagtttaagg ataaaatcac ttataaatac caaaatgtag caagtattaa ttttggcgat    1440
   attactaaaa cgtatgttgt attagtggaa ggtcactatg ataatactgg taaaaacttg    1500
   aaaacacagg ttattcaaga aaatattgac ccagcgacag gtaaagatta cagtattttc    1560
   ggttggaata atgagaatgt tgtacgttat ggaggcggaa gtgctgatgg tgattcagca    1620
   gtaaat                                                               1626
<210> 52
   <211> 542
   <212> PRT
   <213> Staphylococcus aureus
<400> 52
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Asn Ile Ala Thr Asn
   145                 150                 155                 160
   Ser Gly Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
   195 200 205
   Asp Ala Lys Gly Thr Asn Val Asn Asp Lys Val Thr Ala Ser Asp Phe
   210 215 220
   Lys Leu Glu Lys Thr Thr Phe Asp Pro Asn Gln Ser Gly Asn Thr Phe
   225 230 235 240
   Met Ala Ala Asn Phe Thr Val Thr Asp Lys Val Lys Ser Gly Asp Tyr
   245 250 255
   Phe Thr Ala Lys Leu Pro Asp Ser Leu Thr Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asp Val Val Ala Thr Ala Thr Tyr Asn Ile Leu Thr Lys Thr
   290 295 300
   Tyr Thr Phe Val Phe Thr Asp Tyr Val Asn Asp Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asn Glu Phe Lys Asp
   450 455 460
   Lys Ile Thr Tyr Lys Tyr Gln Asn Val Ala Ser Ile Asn Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 53
   <211> 1626
   <212> DNA
   <213> Staphylococcus aureus
<400> 53
   ttgaaaaaaa gaattgatta tttgtcgaat aagcagaata agtattcgat tagacgtttt 60
   acagtaggta ccacatcagt aatagtaggg gcaactatac tatttgggat aggcaatcat 120
   caagcacaag cttcagaaca atcgaacgat acaacgcaat cttcgaaaaa taatgcaagt 180
   gcagattccg aaaaaaacaa tatgatagaa acacctcaat taaatacaac ggctaatgat 240
   acatctgata ttagtgcaaa cacaaacagt gcgaatgtag atagcacagc aaaaccaatg 300
   tctacacaaa cgagcaatac cactacaaca gagccagctt caacaaatga aacacctcaa 360
   ccgacggcaa ttaaagatca agcaactgct gcaaaaatgc aagatcaaac tgttcctcaa 420
   gaagcaaatt ctcaagtaga taataaaaca acgaatgatg ctaataacat agcaacaaac 480
   agtgagctta aaaatcctca aacattagat ttaccacaat catcaccaca aacgatttcc 540
   aatgcgcaag gaactagtaa accaagtgtt agaacgagag ctgtacgtag tcttgcagtt 600
   gctgaacctg tagtaaatgc tgctgatgct aaaggtacaa atgtaaatga taaagttacg 660
   gcaagtgatt tcaagttaga aaagactaca ttcgacccta accaaagtgg taacacattt 720
   atggcggcaa attttacagt gacagataaa gtgaaatcag gggattattt tacagcgaag 780
   ttaccagata gtttaactgg taatggagac gtggattact ctaattcaaa taatacgatg 840
   ccaattgcag acattaaaag tacgaatggt gatgttgtag cgacagcgac ttataatatc 900
   ttgactaaga cgtatacatt tgtctttaca gattatgtaa atgataaaga aaatattaac 960
   ggacaatttt cattaccttt atttacagac cgagcaaagg cacctaaatc aggaacatat 1020
   gatgcaaata ttaatattgc ggatgaaatg tttaataata aaattactta taactatagt 1080
   tcgccaattg caggaattga taagccaaat ggcgcgaaca tttcttctca aattattggt 1140
   gtagatacag cttcaggcca aaacacatac aagcaaacgg tatttgttaa ccctaagcaa 1200
   cgagttttag gtaatacgtg ggtgtatatt aaaggctacc aagataaaat cgaagaaagt 1260
   agcggtaaag taagtgctac agatacaaaa ctgagaattt ttgaagtgaa tgatacatct 1320
   aaattatcag atagctacta tgcggaccca aatgactcta atcttaaaga agtaacgaat 1380
   gagtttaagg ataaaatcac ttataaatac caaaatgtag caagtattaa ttttggcgat 1440
   attactaaaa cgtatgttgt attagtggaa ggtcactatg ataatactgg taaaaacttg 1500
   aaaacacagg ttattcaaga aaatattgac ccagcgacag gtaaagatta cagtattttc 1560
   ggttggaata atgagaatgt tgtacgttat ggaggcggaa gtgctgatgg tgattcagca 1620
   gtaaat 1626
<210> 54
   <211> 542
   <212> PRT
   <213> Staphylococcus aureus
<400> 54
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
   115 120 125
   Thr Ala Ala Lys Met Gln Asp Gln Thr Val Pro Gln Glu Ala Asn Ser
   130 135 140
   Gln Val Asp Asn Lys Thr Thr Asn Asp Ala Asn Asn Ile Ala Thr Asn
   145 150 155 160
   Ser Glu Leu Lys Asn Pro Gln Thr Leu Asp Leu Pro Gln Ser Ser Pro
   165 170 175
   Gln Thr Ile Ser Asn Ala Gln Gly Thr Ser Lys Pro Ser Val Arg Thr
   180 185 190
   Arg Ala Val Arg Ser Leu Ala Val Ala Glu Pro Val Val Asn Ala Ala
   195 200 205
   Asp Ala Lys Gly Thr Asn Val Asn Asp Lys Val Thr Ala Ser Asp Phe
   210 215 220
   Lys Leu Glu Lys Thr Thr Phe Asp Pro Asn Gln Ser Gly Asn Thr Phe
   225 230 235 240
   Met Ala Ala Asn Phe Thr Val Thr Asp Lys Val Lys Ser Gly Asp Tyr
   245 250 255
   Phe Thr Ala Lys Leu Pro Asp Ser Leu Thr Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asp Val Val Ala Thr Ala Thr Tyr Asn Ile Leu Thr Lys Thr
   290 295 300
   Tyr Thr Phe Val Phe Thr Asp Tyr Val Asn Asp Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Asn Glu Phe Lys Asp
       450                 455                 460
   Lys Ile Thr Tyr Lys Tyr Gln Asn Val Ala Ser Ile Asn Phe Gly Asp
   465                 470                 475                 480
   Ile Thr Lys Thr Tyr Val Val Leu Val Glu Gly His Tyr Asp Asn Thr
                   485                 490                 495
   Gly Lys Asn Leu Lys Thr Gln Val Ile Gln Glu Asn Ile Asp Pro Ala
               500                 505                 510
   Thr Gly Lys Asp Tyr Ser Ile Phe Gly Trp Asn Asn Glu Asn Val Val
           515                 520                 525
   Arg Tyr Gly Gly Gly Ser Ala Asp Gly Asp Ser Ala Val Asn
       530                 535                 540
<210> 55
   <211> 1626
   <212> DNA
   <213> Staphylococcus aureus
<400> 55
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tatgatagaa acacctcaat taaatacaac ggctaatgat     240
   acatctgata ttagtgcaaa cacaaacagt gcgaatgtag atagcacagc aaaaccaatg     300
   tctacacaaa cgagcaatac cactacaaca gagccagctt caacaaatga aacacctcaa     360
   ccgacggcaa ttaaagatca agcaactgct gcaaaaatgc aagatcaaac tgttcctcaa     420
   gaagcaaatt ctcaagtaga taataaaaca acgaatgatg ctaatagcat agcgacaaac     480
   agtgagctta aaaatcctca aacattagat ttaccacaat catcaccaca aacaatttcc     540
   aatgcgcaag gaactagtaa accaagtgtt agaacgagag ctgtacgtag tcttgcagtt     600
   gctgaacctg tagtaaatgc tgctgatgct aaaggtacaa atgtaaatga taaagttacg     660
   gcaaaagatt ttcaattaga aaagaccaca tttgacccta accaaagtgg taatactttt     720
   atggcggcaa actttacagt gactggacaa gtgaaatcag gggattattt tacagcgaag     780
   ttaccagata gtgtaactgg taatggagac gtggattact ctaattcgaa taatacgatg     840
   ccaattgcag atattaaaag tacgaatggc gatgttgtag ctaaagcaac atatgatatc     900
   ttgactaaga cgtatacatt tgtctttaca gattatgtaa ataataaaga aaatattaac     960
   ggacaatttt cattaccttt atttacagac cgagcaaagg cacctaaatc aggaacatat    1020
   gatgcgaata ttaatattgc ggatgaaatg tttaataata aaattactta taactatagt    1080
   tcgccaattg caggaattga taaaccaaat ggcgcgaaca tttcttctca aattattggt    1140
   gtagatacag cttcaggtca aaacacatac aagcaaacag tatttgttaa ccctaagcaa    1200
   cgagttttag gtaatacgtg ggtgtatatt aaaggttacc aagataaaat cgaagaaagt    1260
   agcggtaaag taagtgctac agatacaaaa ctgagaattt ttgaagtgaa tgatacatct    1320
   aaattatcag atagctacta tgcggaccca aatgactcta accttaaaga agtaacgaat    1380
   gagtttaagg ataaaatcac ttataaatac caaaatgtag caagtattaa ttttggcgat    1440
   attactaaaa cgtatgttgt attagtggaa ggtcactatg ataatactgg taaaaacttg    1500
   aaaacacagg ttattcaaga aaatattgac ccagcgacag gtaaagacta cagtattttc    1560
   ggttggaata atgagaatgt tgtacgttat ggtggtggaa gtgctgatgg tgattcagca    1620
   gtaaat                                                               1626
<210> 56
   <211> 542
   <212> PRT
   <213> Staphylococcus aureus
<400> 56
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Lys Asp Phe
       210                 215                 220
   Gln Leu Glu Lys Thr Thr Phe Asp Pro Asn Gln Ser Gly Asn Thr Phe
   225                 230                 235                 240
   Met Ala Ala Asn Phe Thr Val Thr Gly Gln Val Lys Ser Gly Asp Tyr
                   245                 250                 255
   Phe Thr Ala Lys Leu Pro Asp Ser Val Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asn Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asn Glu Phe Lys Asp
   450 455 460
   Lys Ile Thr Tyr Lys Tyr Gln Asn Val Ala Ser Ile Asn Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Pro Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 57
   <211> 1626
   <212> DNA
   <213> Staphylococcus aureus
<400> 57
   ttgaaaaaaa gaattgatta tttgtcgaat aagcagaata agtattcgat tagacgtttt      60
   acagtaggta ccacatcagt aatagtaggg gcaactatac tatttgggat aggcaatcat     120
   caagcacaag cttcagaaca atcgaacgat acaacgcaat cttcgaaaaa taatgcaagt     180
   gcagattccg aaaaaaacaa tacgatagaa acacctcaat taaatacaac ggctaatgat     240
   acatctgata ttagtgcaaa cacaaacagt gcgaatgtag atagcacagc aaaaacaatg     300
   tctacacaaa cgagcaatac cactacaaca gagccagctt caacaaatga aacacctcaa     360
   ccgacggcaa ttaaagatca agcaactgct gcaaaaatgc aagatcaaac tgttcctcaa     420
   gaagcaaatt ctcaagtaga taataaaaca acgaatgatg ctaataacat agcaacaaac     480
   agtgagctta aaaatcctca aacattagat ttaccacaat catcaccaca aacgatttcc     540
   aatgcgcaag gaactagtaa accaagtgtt agaacgagag ctgtacgtag tcttgcagtt     600
   gctgaacctg tagtaaatgc tgctgatgct aaaggtacaa atgtaaatga taaagttacg     660
   gcaagtgatc tcaagttaga aaagactgca tttgacccta accaaagtgg taacacattt     720
   atggcggcaa attttaaagt gactggacaa gtgaaatcag gggattattt tacagcgaag     780
   ttaccagata gtgtaactgg taatggagac gtggattact ctaattcaaa taatacgatg     840
   ccaattgcag acattaaaag tacgaatggc gatgttgtag ctaaagcaac atatgatatc     900
   ttgactaaga cgtatacatt tgtctttaca gattatgtaa atgataaaga aaatattaac     960
   ggacaatttt cattaccttt atttacagac cgagcaaagg cacctaaatc aggaacatat    1020
   gatgcaaata ttaatattgc ggatgaaatg tttgataata aaattactta taactatagt    1080
   tcgccaattg caggaattga taagccaaat ggcgcgaaca tttcttctca aattattggt    1140
   gtagatacag cttcaggtca aaacacatac aagcaaacgg tatttgttaa ccctaagcaa    1200
   cgagttttag gtaatacgtg ggtgtatatt aaaggttacc aagataaaat cgaagaaagt    1260
   agcggtaaag taagtgctac agatacaaaa ctgagaattt ttgaagtgaa tgatacatct    1320
   aaattatcag atagctacta tgcagaccca aatgactcta accttaaaga agtgactggt    1380
   gagtttaaag ataaaatttc atacaaatac gataacgtag caagtattaa ttttggtgat    1440
   ataaataaaa cgtatgttgt attagtggaa ggtcactatg ataatactgg taaaaacttg    1500
   aaaacacagg ttattcaaga aaatattgac tcagcgacag gtaaagacta cagtattttc    1560
   ggttggaata atgagaatgt tgtacgttat ggaggcggaa gtgctgatgg tgattcagca    1620
   gtaaat                                                               1626
<210> 58
   <211> 542
   <212> PRT
   <213> Staphylococcus aureus
<400> 58
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Thr Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Ala Lys Thr Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asp Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Asn Ile Ala Thr Asn
   145                 150                 155                 160
   Ser Glu Leu Lys Asn Pro Gln Thr Leu Asp Leu Pro Gln Ser Ser Pro
                   165                 170                 175
   Gln Thr Ile Ser Asn Ala Gln Gly Thr Ser Lys Pro Ser Val Arg Thr
               180                 185                 190
   Arg Ala Val Arg Ser Leu Ala Val Ala Glu Pro Val Val Asn Ala Ala
           195                 200                 205
   Asp Ala Lys Gly Thr Asn Val Asn Asp Lys Val Thr Ala Ser Asp Leu
       210                 215                 220
   Lys Leu Glu Lys Thr Ala Phe Asp Pro Asn Gln Ser Gly Asn Thr Phe
   225                 230                 235                 240
   Met Ala Ala Asn Phe Lys Val Thr Gly Gln Val Lys Ser Gly Asp Tyr
                   245                 250                 255
   Phe Thr Ala Lys Leu Pro Asp Ser Val Thr Gly Asn Gly Asp Val Asp
               260                 265                 270
   Tyr Ser Asn Ser Asn Asn Thr Met Pro Ile Ala Asp Ile Lys Ser Thr
           275                 280                 285
   Asn Gly Asp Val Val Ala Lys Ala Thr Tyr Asp Ile Leu Thr Lys Thr
       290                 295                 300
   Tyr Thr Phe Val Phe Thr Asp Tyr Val Asn Asp Lys Glu Asn Ile Asn
   305                 310                 315                 320
   Gly Gln Phe Ser Leu Pro Leu Phe Thr Asp Arg Ala Lys Ala Pro Lys
                   325                 330                 335
   Ser Gly Thr Tyr Asp Ala Asn Ile Asn Ile Ala Asp Glu Met Phe Asp
               340                 345                 350
   Asn Lys Ile Thr Tyr Asn Tyr Ser Ser Pro Ile Ala Gly Ile Asp Lys
           355                 360                 365
   Pro Asn Gly Ala Asn Ile Ser Ser Gln Ile Ile Gly Val Asp Thr Ala
       370                 375                 380
   Ser Gly Gln Asn Thr Tyr Lys Gln Thr Val Phe Val Asn Pro Lys Gln
   385                 390                 395                 400
   Arg Val Leu Gly Asn Thr Trp Val Tyr Ile Lys Gly Tyr Gln Asp Lys
                   405                 410                 415
   Ile Glu Glu Ser Ser Gly Lys Val Ser Ala Thr Asp Thr Lys Leu Arg
               420                 425                 430
   Ile Phe Glu Val Asn Asp Thr Ser Lys Leu Ser Asp Ser Tyr Tyr Ala
           435                 440                 445
   Asp Pro Asn Asp Ser Asn Leu Lys Glu Val Thr Gly Glu Phe Lys Asp
       450                 455                 460
   Lys Ile Ser Tyr Lys Tyr Asp Asn Val Ala Ser Ile Asn Phe Gly Asp
   465                 470                 475                 480
   Ile Asn Lys Thr Tyr Val Val Leu Val Glu Gly His Tyr Asp Asn Thr
   485 490 495
   Gly Lys Asn Leu Lys Thr Gln Val Ile Gln Glu Asn Ile Asp Ser Ala
   500 505 510
   Thr Gly Lys Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 59
   <211> 1626
   <212> DNA
   <213> Staphylococcus aureus
<400> 59
   ttgaaaaaaa gaattgatta tttgtcgaat aagcagaata agtattcgat tagacgtttt 60
   acagtaggta ccacatcagt aatagtaggg gcaactatac tatttgggat aggcaatcat 120
   caagcacaag cttcagaaca atcgaacgat acaacgcaat cttcgaaaaa taatgcaagt 180
   gcagattccg aaaaaaacaa tatgatagaa acacctcaat taaatacaac ggctaatgat 240
   acatctgata ttagtgcaaa cacaaacagt gcgaatgtag atagcacaac aaaaccaatg 300
   tctacacaaa cgagcaatac cactacaaca gagccagctt caacaaatga aacacctcaa 360
   ccgacggcaa ttaaaaatca agcaactgct gcaaaaatgc aagatcaaac tgttcctcaa 420
   gaagcaaatt ctcaagtaga taataaaaca acgaatgatg ctaatagcat agcaacaaac 480
   agtgagctta aaaattctca aacattagat ttaccacaat catcaccaca aacgatttcc 540
   aatgcgcaag gaactagtaa accaagtgtt agaacgagag ctgtacgtag tttagctgtt 600
   gctgaaccgg tagtaaatgc tgctgatgct aaagatacaa atgtaaatga taaagttacg 660
   gcaagtaatt tcaagttaga aaagactaca tttgacccta atcaaagtgg taacacattt 720
   atggcggcaa attttacagt gacagataaa gtgaaatcag gggattattt tacagcgaag 780
   ttaccagata gtttaactgg taatggagac gtggattatt ctaattcaaa taatacgatg 840
   ccaattgcag acattaaaag tacgaatggc gatgttgtag ctaaagcaac atatgatatc 900
   ttgactaaga cgtatacatt tgtctttaca gattatgtaa ataataaaga aaatattaac 960
   ggacaatttt cattaccttt atttacagac cgagcaaagg cacctaaatc aggaacatat 1020
   gatgcgaata ttaatattgc ggatgaaatg tttaataata aaattactta taactatagt 1080
   tcgccaattg caggaattga taaaccaaat ggcgcgaaca tttcttctca aattattggt 1140
   gtagatacag cttcaggtca aaacacatac aagcaaacag tatttgttaa ccctaagcaa 1200
   cgagttttag gtaatacgtg ggtgtatatt aaaggctacc aagataaaat cgaagaaagt 1260
   agcggtaaag taagtgctac agatacaaaa ctgagaattt ttgaagtgaa tgatacatct 1320
   aaattatcag atagctacta tgcagatcca aatgactcta accttaaaga agtaacagac 1380
   caatttaaaa atagaatcta ttatgagcat ccaaatgtag ctagtattaa atttggtgat 1440
   attactaaaa catatgtagt attagtagaa gggcattacg acaatacagg taagaactta 1500
   aaaactcagg ttattcaaga aaatgttgat cctgtaacaa atagagacta cagtattttc 1560
   ggttggaata atgagaatgt tgtacgttat ggtggtggaa gtgctgatgg tgattcagca 1620
   gtaaat 1626
<210> 60
   <211> 542
   <212> PRT
   <213> Staphylococcus aureus
<400> 60
   Leu Lys Lys Arg Ile Asp Tyr Leu Ser Asn Lys Gln Asn Lys Tyr Ser
   1               5                   10                  15
   Ile Arg Arg Phe Thr Val Gly Thr Thr Ser Val Ile Val Gly Ala Thr
               20                  25                  30
   Ile Leu Phe Gly Ile Gly Asn His Gln Ala Gln Ala Ser Glu Gln Ser
           35                  40                  45
   Asn Asp Thr Thr Gln Ser Ser Lys Asn Asn Ala Ser Ala Asp Ser Glu
       50                  55                  60
   Lys Asn Asn Met Ile Glu Thr Pro Gln Leu Asn Thr Thr Ala Asn Asp
   65                  70                  75                  80
   Thr Ser Asp Ile Ser Ala Asn Thr Asn Ser Ala Asn Val Asp Ser Thr
                   85                  90                  95
   Thr Lys Pro Met Ser Thr Gln Thr Ser Asn Thr Thr Thr Thr Glu Pro
               100                 105                 110
   Ala Ser Thr Asn Glu Thr Pro Gln Pro Thr Ala Ile Lys Asn Gln Ala
           115                 120                 125
   Thr Ala Ala Lys Met Gln Asp Gln Thr Val Pro Gln Glu Ala Asn Ser
       130                 135                 140
   Gln Val Asp Asn Lys Thr Thr Asn Asp Ala Asn Ser Ile Ala Thr Asn
   145                 150                 155                 160
   Ser Glu Leu Lys Asn Ser Gln Thr Leu Asp Leu Pro Gln Ser Ser Pro
   165 170 175
   Gln Thr Ile Ser Asn Ala Gln Gly Thr Ser Lys Pro Ser Val Arg Thr
   180 185 190
   Arg Ala Val Arg Ser Leu Ala Val Ala Glu Pro Val Val Asn Ala Ala
   195 200 205
   Asp Ala Lys Asp Thr Asn Val Asn Asp Lys Val Thr Ala Ser Asn Phe
   210 215 220
   Lys Leu Glu Lys Thr Thr Phe Asp Pro Asn Gln Ser Gly Asn Thr Phe
   225 230 235 240
   Met Ala Ala Asn Phe Thr Val Thr Asp Lys Val Lys Ser Gly Asp Tyr
   245 250 255
   Phe Thr Ala Lys Leu Pro Asp Ser Leu Thr Gly Asn Gly Asp Val Asp
   260 265 270
   Tyr Ser Asn Ser Asn Asn Thr Met Pro Ile Ala Asp Ile Lys Ser Thr
   275 280 285
   Asn Gly Asp Val Val Ala Lys Ala Thr Tyr Asp Ile Leu Thr Lys Thr
   290 295 300
   Tyr Thr Phe Val Phe Thr Asp Tyr Val Asn Asn Lys Glu Asn Ile Asn
   305 310 315 320
   Gly Gln Phe Ser Leu Pro Leu Phe Thr Asp Arg Ala Lys Ala Pro Lys
   325 330 335
   Ser Gly Thr Tyr Asp Ala Asn Ile Asn Ile Ala Asp Glu Met Phe Asn
   340 345 350
   Asn Lys Ile Thr Tyr Asn Tyr Ser Ser Pro Ile Ala Gly Ile Asp Lys
   355 360 365
   Pro Asn Gly Ala Asn Ile Ser Ser Gln Ile Ile Gly Val Asp Thr Ala
   370 375 380
   Ser Gly Gln Asn Thr Tyr Lys Gln Thr Val Phe Val Asn Pro Lys Gln
   385 390 395 400
   Arg Val Leu Gly Asn Thr Trp Val Tyr Ile Lys Gly Tyr Gln Asp Lys
   405 410 415
   Ile Glu Glu Ser Ser Gly Lys Val Ser Ala Thr Asp Thr Lys Leu Arg
   420 425 430
   Ile Phe Glu Val Asn Asp Thr Ser Lys Leu Ser Asp Ser Tyr Tyr Ala
   435 440 445
   Asp Pro Asn Asp Ser Asn Leu Lys Glu Val Thr Asp Gln Phe Lys Asn
   450 455 460
   Arg Ile Tyr Tyr Glu His Pro Asn Val Ala Ser Ile Lys Phe Gly Asp
   465 470 475 480
   Ile Thr Lys Thr Tyr Val Val Leu Val Glu Gly His Tyr Asp Asn Thr
   485 490 495
   Gly Lys Asn Leu Lys Thr Gln Val Ile Gln Glu Asn Val Asp Pro Val
   500 505 510
   Thr Asn Arg Asp Tyr Ser Ile Phe Gly Trp Asn Asn Glu Asn Val Val
   515 520 525
   Arg Tyr Gly Gly Gly Ser Ala Asp Gly Asp Ser Ala Val Asn
   530 535 540
<210> 61
   <211> 1677
   <212> DNA
   <213> Staphylococcus aureus
<400> 61
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc tgatagcgca agtaacgaaa gcaaaagtaa tgattcaagt 180
   agcgttagtg ctgcacctaa aacagacgac acaaacgtga gtgatactaa aacatcgtca 240
   aacactaata atggcgaaac gagtgtggcg caaaatccag cacaacagga aacgacacaa 300
   tcatcatcaa caaatgcaac tacggaagaa acgccggtaa ctggtgaagc tactactacg 360
   acaacgaatc aagctaatac accggcaaca actcaatcaa gcaatacaaa tgcggaggaa 420
   ttagtgaatc aaacaagtaa tgaaacgact tctaatgata ctaatacagt atcatctgta 480
   aattcacctc aaaattctac aaatgcggaa aatgtttcaa caacgcaaga tacttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggca 660
   gtagctgcag atgcaccggc agctggcaca gatattacga atcagttgac gaatgtgaca 720
   gttggtattg actctggtac gactgtgtat ccgcaccaag caggttatgt caaactgaat 780
   tatggttttt cagtgcctaa ttctgctgtt aaaggtgaca cattcaaaat aactgtacct 840
   aaagaattaa acttaaatgg tgtaacttca actgctaaag tgccaccaat tatggctgga 900
   gatcaagtat tggcaaatgg tgtaatcgat agtgatggta atgttattta tacatttaca 960
   gactatgtaa atactaaaga tgatgtaaaa gcaactttga ccatgcccgc ttatattgac 1020
   cctgaaaatg ttaaaaagac aggtaatgtg acattggcta ctggcatagg tagtacaaca 1080
   gcaaacaaaa cagtattagt agattatgaa aaatatggta agttttataa cttatctatt 1140
   aaaggtacaa ttgaccaaat cgataaaaca aataatacgt atcgtcagac aatttatgtc 1200
   aatccaagtg gagataacgt tattgcgccg gttttaacag gtaatttaaa accaaatacg 1260
   gatagtaatg cattaataga tcagcaaaat acaagtatta aagtatataa agtagataat 1320
   gcagctgatt tatctgaaag ttactttgtg aatccagaaa actttgagga tgtcactaat 1380
   agtgtgaata ttacattccc aaatccaaat caatataaag tagagtttaa tacgcctgat 1440
   gatcaaatta caacaccgta tatagtagtt gttaatggtc atattgatcc gaatagcaaa 1500
   ggtgatttag ctttacgttc aactttatat gggtataact cgaatataat ttggcgctct 1560
   atgtcatggg acaacgaagt agcatttaat aacggatcag gttctggtga cggtatcgat 1620
   aaaccagttg ttcctgaaca acctgatgag cctggtgaaa ttgaaccaat tccagag 1677
<210> 62
   <211> 559
   <212> PRT
   <213> Staphylococcus aureus
<400> 62
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Ser Thr Asp Ala Ser Asn Lys Asp Val Val Asn Gln Ala Val Asn Thr
           195                 200                 205
   Ser Ala Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asn Val Thr
   225                 230                 235                 240
   Val Gly Ile Asp Ser Gly Thr Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
           275                 280                 285
   Thr Ser Thr Ala Lys Val Pro Pro Ile Met Ala Gly Asp Gln Val Leu
       290                 295                 300
   Ala Asn Gly Val Ile Asp Ser Asp Gly Asn Val Ile Tyr Thr Phe Thr
   305                 310                 315                 320
   Asp Tyr Val Asn Thr Lys Asp Asp Val Lys Ala Thr Leu Thr Met Pro
                   325                 330                 335
   Ala Tyr Ile Asp Pro Glu Asn Val Lys Lys Thr Gly Asn Val Thr Leu
               340                 345                 350
   Ala Thr Gly Ile Gly Ser Thr Thr Ala Asn Lys Thr Val Leu Val Asp
           355                 360                 365
   Tyr Glu Lys Tyr Gly Lys Phe Tyr Asn Leu Ser Ile Lys Gly Thr Ile
       370                 375                 380
   Asp Gln Ile Asp Lys Thr Asn Asn Thr Tyr Arg Gln Thr Ile Tyr Val
   385                 390                 395                 400
   Asn Pro Ser Gly Asp Asn Val Ile Ala Pro Val Leu Thr Gly Asn Leu
                   405                 410                 415
   Lys Pro Asn Thr Asp Ser Asn Ala Leu Ile Asp Gln Gln Asn Thr Ser
               420                 425                 430
   Ile Lys Val Tyr Lys Val Asp Asn Ala Ala Asp Leu Ser Glu Ser Tyr
           435                 440                 445
   Phe Val Asn Pro Glu Asn Phe Glu Asp Val Thr Asn Ser Val Asn Ile
       450                 455                 460
   Thr Phe Pro Asn Pro Asn Gln Tyr Lys Val Glu Phe Asn Thr Pro Asp
   465                 470                 475                 480
   Asp Gln Ile Thr Thr Pro Tyr Ile Val Val Val Asn Gly His Ile Asp
                   485                 490                 495
   Pro Asn Ser Lys Gly Asp Leu Ala Leu Arg Ser Thr Leu Tyr Gly Tyr
               500                 505                 510
   Asn Ser Asn Ile Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 63
   <211> 1677
   <212> DNA
   <213> Staphylococcus aureus
<400> 63
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacagga aacgacacaa     300
   tcatcatcaa caaatgcaac tacggaagaa acgccggtaa ctggtgaagc tactactacg     360
   acaacgaatc aagctaatac accggcaaca actcaatcaa gcaatacaaa tgcggaggaa     420
   ttagtgaatc aaacaagtaa tgaaacgact tctaatgata ctaatacagt atcatctgta     480
   aattcacctc aaaattctac aaatgcggaa aatgtttcaa caacgcaaga tacttcaact     540
   gaagcaacac cttcaaacaa tgaatcagct ccacagaata cagatgcaag taataaagat     600
   gtagttagtc aagcggttaa tccaagtacg cctagaatga gagcatttag tttagcggca     660
   gtagctgcag atgcaccggc agctggcaca gatattacga atcagttgac agatgtgaaa     720
   gttactattg actctggtac gactgtgtat ccgcaccaag caggttatgt caaactgaat     780
   tatggttttt cagtgcctaa ttctgctgtt aaaggtgaca cattcaaaat aactgtacct     840
   aaagaattaa acttaaatgg tgtaacttca actgctaaag tgccaccaat tatggctgga     900
   gatcaagtat tggcaaatgg tgtaatcgat agtgatggta atgttattta tacatttaca     960
   gactatgttg ataataaaga aaatgtaaca gctaatatta ctatgccagc ttatattgac    1020
   cctgaaaatg ttacaaagac aggtaatgtg acattgacaa ctggcatagg aaccaatact    1080
   gctagtaaga cagtattaat cgactatgag aaatatggac aattccataa tttatcaatt    1140
   aaaggtacga ttgatcaaat cgataaaaca aataatacgt atcgccaaac aatttatgtc    1200
   aatccaagcg gagataacgt tgtgttacct gccttaacag gtaatttaat tcctaataca    1260
   aagagtaatg cgttaataga tgcaaaaaac actgatatta aagtttatag agtcgataat    1320
   gctaatgatt tatctgaaag ttattatgtg aatcctagcg attttgaaga tgtaactaat    1380
   caagttagaa tttcatttcc aaatgctaat caatacaaag tagaatttcc tacggacgat    1440
   gaccaaatta caacaccgta tattgtagtt gttaatggcc atattgatcc tgctagtaca    1500
   ggtgatttag cactacgttc gacattttat ggttatgatt ctaattttat atggagatct    1560
   atgtcatggg acaacgaagt agcatttaat aacggatcag gttctggtga cggtatcgat    1620
   aaaccagttg ttcctgaaca acctgatgag cctggtgaaa ttgaaccaat tccagag       1677
<210> 64
   <211> 559
   <212> PRT
   <213> Staphylococcus aureus
<400> 64
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Asn Thr Asp Ala Ser Asn Lys Asp Val Val Ser Gln Ala Val Asn Pro
           195                 200                 205
   Ser Thr Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asp Val Lys
   225                 230                 235                 240
   Val Thr Ile Asp Ser Gly Thr Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
           275                 280                 285
   Thr Ser Thr Ala Lys Val Pro Pro Ile Met Ala Gly Asp Gln Val Leu
       290                 295                 300
   Ala Asn Gly Val Ile Asp Ser Asp Gly Asn Val Ile Tyr Thr Phe Thr
   305                 310                 315                 320
   Asp Tyr Val Asp Asn Lys Glu Asn Val Thr Ala Asn Ile Thr Met Pro
                   325                 330                 335
   Ala Tyr Ile Asp Pro Glu Asn Val Thr Lys Thr Gly Asn Val Thr Leu
               340                 345                 350
   Thr Thr Gly Ile Gly Thr Asn Thr Ala Ser Lys Thr Val Leu Ile Asp
           355                 360                 365
   Tyr Glu Lys Tyr Gly Gln Phe His Asn Leu Ser Ile Lys Gly Thr Ile
       370                 375                 380
   Asp Gln Ile Asp Lys Thr Asn Asn Thr Tyr Arg Gln Thr Ile Tyr Val
   385                 390                 395                 400
   Asn Pro Ser Gly Asp Asn Val Val Leu Pro Ala Leu Thr Gly Asn Leu
                   405                 410                 415
   Ile Pro Asn Thr Lys Ser Asn Ala Leu Ile Asp Ala Lys Asn Thr Asp
               420                 425                 430
   Ile Lys Val Tyr Arg Val Asp Asn Ala Asn Asp Leu Ser Glu Ser Tyr
           435                 440                 445
   Tyr Val Asn Pro Ser Asp Phe Glu Asp Val Thr Asn Gln Val Arg Ile
       450                 455                 460
   Ser Phe Pro Asn Ala Asn Gln Tyr Lys Val Glu Phe Pro Thr Asp Asp
   465                 470                 475                 480
   Asp Gln Ile Thr Thr Pro Tyr Ile Val Val Val Asn Gly His Ile Asp
                   485                 490                 495
   Pro Ala Ser Thr Gly Asp Leu Ala Leu Arg Ser Thr Phe Tyr Gly Tyr
               500                 505                 510
   Asp Ser Asn Phe Ile Trp Arg Ser Met Ser Trp Asp Asn Glu Val Ala
   515 520 525
   Phe Asn Asn Gly Ser Gly Ser Gly Asp Gly Ile Asp Lys Pro Val Val
   530 535 540
   Pro Glu Gln Pro Asp Glu Pro Gly Glu Ile Glu Pro Ile Pro Glu
   545 550 555
<210> 65
   <211> 1677
   <212> DNA
   <213> Staphylococcus aureus
<400> 65
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc tgatagcgca agtaacgaaa gcaaaagtaa tgattcaagt 180
   agcgttagtg ctgcacctaa aacagacgac acaaacgtga gtgatactaa aacatcgtca 240
   aacactaata atggcgaaac gagtgtggcg caaaatccag cacaacagga aacgacacaa 300
   tcatcatcaa caaatgcaac tacggaagaa acgccggtaa ctggtgaagc tactactacg 360
   acaacgaatc aagctaatac accggcaaca actcaatcaa gcaatacaaa tgcggaggaa 420
   ttagtgaatc aaacaagtaa tgaaacgact tctaatgata ctaatacagt atcatctgta 480
   aattcacctc aaaattctac aaatgcggaa aatgtttcaa caacgcaaga tacttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggca 660
   gtagctgcag atgcaccggc agctggcaca gatattacga atcagttgac gaatgtgaca 720
   gttggtattg actctggtac gactgtgtat ccgcaccaag caggttatgt caaactgaat 780
   tatggttttt cagtgcctaa ttctgctgtt aaaggtgaca cattcaaaat aactgtacct 840
   aaagaattaa acttaaatgg tgtaacttca actgctaaag tgccaccaat tatggctgga 900
   gatcaagtat tggcaaatgg tgtaatcgat agtgatggta atgttattta tacatttaca 960
   gactatgtaa atactaaaga tgatgtaaaa gcaactttga ccatgcccgc ttatattaac 1020
   cctgaaaatg ttaaaaagac aggtaatgtg acattggcta ctggcatagg tagtacaaca 1080
   gcaaacaaaa cagtattagt agattatgaa aaatatggta agttttataa cttatctatt 1140
   aaaggtacaa ttgaccaaat cgataaaaca aataatacgt atcgtcagac aatttatgtc 1200
   aatccaagtg gagataacgt tattgcgccg gttttaacag gtaatttaaa accaaatacg 1260
   gatagtaatg cattaataga tcagcaaaat acaagtatta aagtatataa agtagataat 1320
   gcagctgatt tatctgaaag ttactttgtg aatccagaaa actttgagga tgtcactaat 1380
   agtgtgaata ttacattccc aaatccaaat caatataaag tagagtttaa tacgcctgat 1440
   gatcaaatta caacaccgta tatagtagtt gttaatggtc atattgatcc gaatagcaaa 1500
   ggtgatttag ctttacgttc aactttatat gggtataact cgaatataat ttggcgctct 1560
   atgtcatggg acaacgaagt agcatttaat aacggatcag gttctggtga cggtatcgat 1620
   aaaccagttg ttcctgaaca acctgatgag cctggtgaaa ttgaaccaat tccagag 1677
<210> 66
   <211> 559
   <212> PRT
   <213> Staphylococcus aureus
<400> 66
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
   180 185 190
   Ser Thr Asp Ala Ser Asn Lys Asp Val Val Asn Gln Ala Val Asn Thr
   195 200 205
   Ser Ala Pro Arg Met Arg Ala Phe Ser Leu Ala Ala Val Ala Ala Asp
   210 215 220
   Ala Pro Ala Ala Gly Thr Asp Ile Thr Asn Gln Leu Thr Asn Val Thr
   225 230 235 240
   Val Gly Ile Asp Ser Gly Thr Thr Val Tyr Pro His Gln Ala Gly Tyr
   245 250 255
   Val Lys Leu Asn Tyr Gly Phe Ser Val Pro Asn Ser Ala Val Lys Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asn Pro Glu Asn Val Lys Lys Thr Gly Asn Val Thr Leu
   340 345 350
   Ala Thr Gly Ile Gly Ser Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Asp Ser Asn Ala Leu Ile Asp Gln Gln Asn Thr Ser
   420 425 430
   Ile Lys Val Tyr Lys Val Asp Asn Ala Ala Asp Leu Ser Glu Ser Tyr
           435                 440                 445
   Phe Val Asn Pro Glu Asn Phe Glu Asp Val Thr Asn Ser Val Asn Ile
       450                 455                 460
   Thr Phe Pro Asn Pro Asn Gln Tyr Lys Val Glu Phe Asn Thr Pro Asp
   465                 470                 475                 480
   Asp Gln Ile Thr Thr Pro Tyr Ile Val Val Val Asn Gly His Ile Asp
                   485                 490                 495
   Pro Asn Ser Lys Gly Asp Leu Ala Leu Arg Ser Thr Leu Tyr Gly Tyr
               500                 505                 510
   Asn Ser Asn Ile Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 67
   <211> 1677
   <212> DNA
   <213> Staphylococcus aureus
<400> 67
   atgaatatga agaaacaaga aaaacacgca attcgtaaaa aatcgattgg cgtggcttca 60
   gtgcttgtag gtacgttaat cggttttgga ctactcagca gtaaagaagc agatgcaagt 120
   gaaaatagta tgacacaaac ggaaaatacg agtaatgaga gcaaaagtaa tgatccaagt 180
   agcgttaatg ctgcacctaa aacagacaac acaaacgtga gtgattctaa tacaacgaca 240
   aacactaata gtgacgaaac gaatgtagcg caaaatccag cacaacagga aacgacacaa 300
   tcagcatcaa caaatgcaac tacagaagaa acaccggtaa ctggtgaagt tactactacg 360
   gcaacgaatc aagctaatac accggcaaca actcaatcaa gcaatacaaa tgcggaagaa 420
   tcagtgaatc aaacaagtaa tgaaacgact tctaatgata ctaatacagt atcatctgta 480
   aattcacctc aaaattctac aaatgcggaa aatgtttcaa caacgcaaga catttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggct 660
   gtagctgcag atgcaccggc tgctggcaaa gatattacga atcagttgac gaatgtgaca 720
   gttggtattg actctggaga tacagtttat ccgcaccaag caggctatgt caaactgaat 780
   tatgggttct cagtaccaaa tgaggctgtt caaggtgaca cattcaaaat aactgtgccc 840
   aaagaattaa acttaaatgg tgtaacttca actgctaaag tgccaccaat tatggccgga 900
   gatcaagtat tggcaaatgg tgtaatcgat agtgatggta atgttattta tacatttaca 960
   gactatgtaa atactaaaga tgatgttaaa gcaactttga ccatgcccgc ttatattgac 1020
   cctgaaaatg ttacaaagac aggtaatgtg acattggcta ctggcatagg tagtacaaca 1080
   gcaaacaaaa cagtattagt agattatgaa aaatatggta agttttataa cttatctatt 1140
   aaaggtacaa ttgaccaaat cgataaaaca aataatacgt atcgtcagac aatttatgtc 1200
   aatccaagtg gagataacgt tattgcgccg gttttaacag gtaatttaaa accaaatacg 1260
   gatagtaatg cattaataga tgcacaaaat actagtatta aagtatataa agttgataat 1320
   gcatcagact tgtctgaaag ttattatgtg aatccagata actttgaaga tgtcactgat 1380
   agtgtgaata ttacattccc aaatccaaat caatataaag tagagttcaa tacgcctgat 1440
   gatcaaataa caacaccata tattgtagtt gttaatgggc atattgatcc taatagtaaa 1500
   ggtgatttag ctttacgttc aactttatat ggatataatt cgaatataat ttggcgatca 1560
   atgtcatggg ataatgaagt agcatttaat aacggatcag gttctggtga cggtatcgat 1620
   aaacctgttg ttcctgaaca acctgatgag ccgggtgaaa ttgaaccaat tccagag 1677
<210> 68
   <211> 559
   <212> PRT
   <213> Staphylococcus aureus
<400> 68
   Met Asn Met Lys Lys Gln Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Met Thr Gln Thr Glu
           35                  40                  45
   Asn Thr Ser Asn Glu Ser Lys Ser Asn Asp Pro Ser Ser Val Asn Ala
       50                  55                  60
   Ala Pro Lys Thr Asp Asn Thr Asn Val Ser Asp Ser Asn Thr Thr Thr
   65                  70                  75                  80
   Asn Thr Asn Ser Asp Glu Thr Asn Val Ala Gln Asn Pro Ala Gln Gln
                   85                  90                  95
   Glu Thr Thr Gln Ser Ala Ser Thr Asn Ala Thr Thr Glu Glu Thr Pro
   100 105 110
   Val Thr Gly Glu Val Thr Thr Thr Ala Thr Asn Gln Ala Asn Thr Pro
   115 120 125
   Ala Thr Thr Gln Ser Ser Asn Thr Asn Ala Glu Glu Ser Val Asn Gln
   130 135 140
   Thr Ser Asn Glu Thr Thr Ser Asn Asp Thr Asn Thr Val Ser Ser Val
   145 150 155 160
   Asn Ser Pro Gln Asn Ser Thr Asn Ala Glu Asn Val Ser Thr Thr Gln
   165 170 175
   Asp Ile Ser Thr Glu Ala Thr Pro Ser Asn Asn Glu Ser Ala Pro Gln
   180 185 190
   Ser Thr Asp Ala Ser Asn Lys Asp Val Val Asn Gln Ala Val Asn Thr
   195 200 205
   Ser Ala Pro Arg Met Arg Ala Phe Ser Leu Ala Ala Val Ala Ala Asp
   210 215 220
   Ala Pro Ala Ala Gly Lys Asp Ile Thr Asn Gln Leu Thr Asn Val Thr
   225 230 235 240
   Val Gly Ile Asp Ser Gly Asp Thr Val Tyr Pro His Gln Ala Gly Tyr
   245 250 255
   Val Lys Leu Asn Tyr Gly Phe Ser Val Pro Asn Glu Ala Val Gln Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asp Pro Glu Asn Val Thr Lys Thr Gly Asn Val Thr Leu
   340 345 350
   Ala Thr Gly Ile Gly Ser Thr Thr Ala Asn Lys Thr Val Leu Val Asp
           355                 360                 365
   Tyr Glu Lys Tyr Gly Lys Phe Tyr Asn Leu Ser Ile Lys Gly Thr Ile
       370                 375                 380
   Asp Gln Ile Asp Lys Thr Asn Asn Thr Tyr Arg Gln Thr Ile Tyr Val
   385                 390                 395                 400
   Asn Pro Ser Gly Asp Asn Val Ile Ala Pro Val Leu Thr Gly Asn Leu
                   405                 410                 415
   Lys Pro Asn Thr Asp Ser Asn Ala Leu Ile Asp Ala Gln Asn Thr Ser
               420                 425                 430
   Ile Lys Val Tyr Lys Val Asp Asn Ala Ser Asp Leu Ser Glu Ser Tyr
           435                 440                 445
   Tyr Val Asn Pro Asp Asn Phe Glu Asp Val Thr Asp Ser Val Asn Ile
       450                 455                 460
   Thr Phe Pro Asn Pro Asn Gln Tyr Lys Val Glu Phe Asn Thr Pro Asp
   465                 470                 475                 480
   Asp Gln Ile Thr Thr Pro Tyr Ile Val Val Val Asn Gly His Ile Asp
                   485                 490                 495
   Pro Asn Ser Lys Gly Asp Leu Ala Leu Arg Ser Thr Leu Tyr Gly Tyr
               500                 505                 510
   Asn Ser Asn Ile Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 69
   <211> 1677
   <212> DNA
   <213> Staphylococcus aureus
<400> 69
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcattaatg ctgcacctaa aacagacaac acaaacgtga gtgatactaa accaacgtca     240
   aacactaata atggcgaaac gagtgtggcg caaaatccag cacaacagga aacgacacaa     300
   tcagcatcaa caaatgcaac tacggaagaa acgccggtaa ctggtgaagc tactactacg     360
   acaacgaatc aagctaatac accggcaaca actcaatcaa gcaatacaaa tgcggaggaa     420
   ttagtgaatc aaacaagtaa tgaaacgact tctaatgata ctaatacagt atcatctgta     480
   aattcacctc aaaattctac aaatgcggaa aatgtttcaa caacgcaaga tacttcaact     540
   gaagcaacac cttcaaacaa tgaatcagct ccacagagta cagatgcaag taataaagat     600
   gtagttaatc aagcggttaa tacaagtgcg cctagaaaga gagcatttag tttagcggct     660
   gtagctgcag atgcaccggc agctggcaca gatattacga atcagttgac agatgtgaaa     720
   gttactattg actctggtac gactgtgtat ccgcaccaag caggttatgt caaactgaat     780
   tatggttttt cagtgcctaa ttctgctgtt aaaggtgaca cattcaaaat aactgtacct     840
   aaagaattaa acttaaatgg tgtaacttca actgctaaag tgccaccaat tatggctgga     900
   gatcaagtat tggcaaatgg tgtaatcgat agtgatggta atgttattta tacatttaca     960
   gactatgttg ataataaaga aaatgtaaca gctaatatta ctatgccagc ttatattgac    1020
   cctgaaaatg ttacaaagac aggtaatgtg acattgacaa ctggcatagg aaccaatact    1080
   gctagtaaga cagtattaat cgactatgag aaatatggac aattccataa tttatcaatt    1140
   aaaggtacga ttgatcaaat cgataaaaca aataatacgt atcgccaaac aatttatgtc    1200
   aatccaagcg gagataacgt tgtgttacct gccttaacag gtaatttaat tcctaataca    1260
   aagagtaatg cgttaataga tgcaaaaaac actgatatta aagtttatag agtcgataat    1320
   gctaatgatt tatctgaaag ttattatgtg aatcctagcg attttgaaga tgtaactaat    1380
   caagttagaa tttcatttcc aaatgctaat caatacaaag tagaatttcc tacggacgat    1440
   gaccaaatta caacaccgta tattgtagtt gttaatggcc atattgatcc tgctagtaca    1500
   ggtgatttag cactacgttc gacattttat ggttatgatt ctaattttat atggagatct    1560
   atgtcatggg acaacgaagt agcatttaat aacggatcag gttctggtga cggtatcgat    1620
   aaaccagttg ttcctgaaca acctgatgag cctggtgaaa ttgaaccaat tccagag       1677
<210> 70
   <211> 559
   <212> PRT
   <213> Staphylococcus aureus
<400> 70
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Ile Asn Ala
       50                  55                  60
   Ala Pro Lys Thr Asp Asn Thr Asn Val Ser Asp Thr Lys Pro Thr Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ala Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Ser Thr Asp Ala Ser Asn Lys Asp Val Val Asn Gln Ala Val Asn Thr
           195                 200                 205
   Ser Ala Pro Arg Lys Arg Ala Phe Ser Leu Ala Ala Val Ala Ala Asp
       210                 215                 220
   Ala Pro Ala Ala Gly Thr Asp Ile Thr Asn Gln Leu Thr Asp Val Lys
   225                 230                 235                 240
   Val Thr Ile Asp Ser Gly Thr Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
           275                 280                 285
   Thr Ser Thr Ala Lys Val Pro Pro Ile Met Ala Gly Asp Gln Val Leu
       290                 295                 300
   Ala Asn Gly Val Ile Asp Ser Asp Gly Asn Val Ile Tyr Thr Phe Thr
   305                 310                 315                 320
   Asp Tyr Val Asp Asn Lys Glu Asn Val Thr Ala Asn Ile Thr Met Pro
                   325                 330                 335
   Ala Tyr Ile Asp Pro Glu Asn Val Thr Lys Thr Gly Asn Val Thr Leu
               340                 345                 350
   Thr Thr Gly Ile Gly Thr Asn Thr Ala Ser Lys Thr Val Leu Ile Asp
           355                 360                 365
   Tyr Glu Lys Tyr Gly Gln Phe His Asn Leu Ser Ile Lys Gly Thr Ile
       370                 375                 380
   Asp Gln Ile Asp Lys Thr Asn Asn Thr Tyr Arg Gln Thr Ile Tyr Val
   385                 390                 395                 400
   Asn Pro Ser Gly Asp Asn Val Val Leu Pro Ala Leu Thr Gly Asn Leu
                   405                 410                 415
   Ile Pro Asn Thr Lys Ser Asn Ala Leu Ile Asp Ala Lys Asn Thr Asp
               420                 425                 430
   Ile Lys Val Tyr Arg Val Asp Asn Ala Asn Asp Leu Ser Glu Ser Tyr
           435                 440                 445
   Tyr Val Asn Pro Ser Asp Phe Glu Asp Val Thr Asn Gln Val Arg Ile
       450                 455                 460
   Ser Phe Pro Asn Ala Asn Gln Tyr Lys Val Glu Phe Pro Thr Asp Asp
   465                 470                 475                 480
   Asp Gln Ile Thr Thr Pro Tyr Ile Val Val Val Asn Gly His Ile Asp
                   485                 490                 495
   Pro Ala Ser Thr Gly Asp Leu Ala Leu Arg Ser Thr Phe Tyr Gly Tyr
               500                 505                 510
   Asp Ser Asn Phe Ile Trp Arg Ser Met Ser Trp Asp Asn Glu Val Ala
   515 520 525
   Phe Asn Asn Gly Ser Gly Ser Gly Asp Gly Ile Asp Lys Pro Val Val
   530 535 540
   Pro Glu Gln Pro Asp Glu Pro Gly Glu Ile Glu Pro Ile Pro Glu
   545 550 555
<210> 71
   <211> 1677
   <212> DNA
   <213> Staphylococcus aureus
<400> 71
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacagga aacgacacaa     300
   tcatcatcaa caaatgcaac tacggaagaa acgccggtaa ctggtgaagc tactactacg     360
   acaacgaatc aagctaatac accggcaaca actcaatcaa gcaatacaaa tgcggaggaa     420
   ttagtgaatc aaacaagtaa tgaaacgact tctaatgata ctaatacagt atcatctgta     480
   aattcacctc aaaattctac aaatgcggaa aatgtttcaa caacgcaaga tacttcaact     540
   gaagcaacac cttcaaacaa tgaatcagct ccacagaata cagatgcaag taataaagat     600
   gtagttagtc aagcggttaa tccaagtacg cctagaatga gagcatttag tttagcggca     660
   gtagctgcag atgcaccggc agctggcaca gatattacga atcagttgac agatgtgaaa     720
   gttactattg actctggtac gactgtgtat ccgcaccaag caggttatgt caaactgaat     780
   tatggttttt cagtgcctaa ttctcctgtt aaaggtgaca cattcaaaat aactgtacct     840
   aaagaattaa acttaaatgg tgtaacttca actgctaaag tgccaccaat tatggctgga     900
   gatcaagtat tggcaaatgg tgtaatcgat agtgatggta atgttattta tacatttaca     960
   gactatgttg ataataaaga aaatgtaaca gctaatatta ctatgccagc ttatattgac    1020
   cctgaaaatg ttacaaagac aggtaatgtg acattgacaa ctggcatagg aaccaatact    1080
   gctagtaaga cagtattaat cgactatgag aaatatggac aattccataa tttatcaatt    1140
   aaaggtacga ttgatcaaat cgataaaaca aataatacgt atcgccaaac aatttatgtc    1200
   aatccaagcg gagataacgt tgtgttacct gccttaacag gtaatttaat tcctaataca    1260
   aagagtaatg cgttaataga tgcaaaaaac actgatatta aagtttatag agtcgataat    1320
   gctaatgatt tatctgaaag ttattatgtg aatcctagcg attttgaaga tgtaactaat    1380
   caagttagaa tttcatttcc aaatgctaat caatacaaag tagaatttcc tacggacgat    1440
   gaccaaatta caacaccgta tattgtagtt gttaatggcc atattgatcc tgctagtaca    1500
   ggtgatttag cactacgttc gacattttat ggttatgatt ctaattttat atggagatct    1560
   atgtcatggg acaacgaagt agcatttaat aacggatcag gttctggtga cggtatcgat    1620
   aaaccagttg ttcctgaaca acctgatgag cctggtgaaa ttgaaccaat tccagag       1677
<210> 72
   <211> 559
   <212> PRT
   <213> Staphylococcus aureus
<400> 72
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
   180 185 190
   Asn Thr Asp Ala Ser Asn Lys Asp Val Val Ser Gln Ala Val Asn Pro
   195 200 205
   Ser Thr Pro Arg Met Arg Ala Phe Ser Leu Ala Ala Val Ala Ala Asp
   210 215 220
   Ala Pro Ala Ala Gly Thr Asp Ile Thr Asn Gln Leu Thr Asp Val Lys
   225 230 235 240
   Val Thr Ile Asp Ser Gly Thr Thr Val Tyr Pro His Gln Ala Gly Tyr
   245 250 255
   Val Lys Leu Asn Tyr Gly Phe Ser Val Pro Asn Ser Pro Val Lys Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asp Asn Lys Glu Asn Val Thr Ala Asn Ile Thr Met Pro
   325 330 335
   Ala Tyr Ile Asp Pro Glu Asn Val Thr Lys Thr Gly Asn Val Thr Leu
   340 345 350
   Thr Thr Gly Ile Gly Thr Asn Thr Ala Ser Lys Thr Val Leu Ile Asp
   355 360 365
   Tyr Glu Lys Tyr Gly Gln Phe His Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Val Leu Pro Ala Leu Thr Gly Asn Leu
   405 410 415
   Ile Pro Asn Thr Lys Ser Asn Ala Leu Ile Asp Ala Lys Asn Thr Asp
   420 425 430
   Ile Lys Val Tyr Arg Val Asp Asn Ala Asn Asp Leu Ser Glu Ser Tyr
           435                 440                 445
   Tyr Val Asn Pro Ser Asp Phe Glu Asp Val Thr Asn Gln Val Arg Ile
       450                 455                 460
   Ser Phe Pro Asn Ala Asn Gln Tyr Lys Val Glu Phe Pro Thr Asp Asp
   465                 470                 475                 480
   Asp Gln Ile Thr Thr Pro Tyr Ile Val Val Val Asn Gly His Ile Asp
                   485                 490                 495
   Pro Ala Ser Thr Gly Asp Leu Ala Leu Arg Ser Thr Phe Tyr Gly Tyr
               500                 505                 510
   Asp Ser Asn Phe Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 73
   <211> 1677
   <212> DNA
   <213> Staphylococcus aureus
<400> 73
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc tgatagcgca agtaacgaaa gcaaaagtaa tgattcaagt 180
   agcattaatg ctgcacctaa aacagacaac acaaacgtga gtgatactaa aacaacgtca 240
   aacactaata atggcgaaac gagtgtggcg caaaatccag cacaacagga aacgacacaa 300
   tcagcatcaa caaatgcaac tacggaagaa acgccggtaa ctggtgaaac tactactacg 360
   gcaacgaatc aagctaatac accggcaaca actcaatcaa gcaatacaaa tgcggaggaa 420
   ttagtgaatc aaacaagtaa tgaaacgact tctaatgata ctaatacagt atcatctgta 480
   aattcacctc aaaattctac aaatgcggaa aatgtttcaa caacgcaaga tacttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggca 660
   gtagctgcag atgcaccggc agctggcaca gatattacga atcagttgac agatgtgaaa 720
   gttactattg actctggtac gactgtgtat ccgcaccaag caggttatgt caaactgaat 780
   tatggttttt cagtgcctaa ttctgctgtt aaaggtgaca cattcaaaat aactgtacct 840
   aaagaattaa acttaaatgg tgtaacttca actgctaaag tgccaccaat tatggctgga 900
   gatcaagtat tggcaaatgg tgtaatcgat agtgatggta atgttattta tacatttaca 960
   gactatgttg ataataaaga aaatgtaaca gctaatatta ctatgccagc ttatattgac 1020
   cctgaaaatg ttacaaagac aggtaatgtg acattgacaa ctggcatagg aaccaatact 1080
   gctagtaaga cagtattaat cgactatgag aaatatggac aattccataa tttatcaatt 1140
   aaaggtacga ttgatcaaat cgataaaaca aataatacgt atcgccaaac aatttatgtc 1200
   aatccaagcg gagataacgt tgtgttacct gccttaacag gtaatttaat tcctaataca 1260
   aagagtaatg cgttaataga tgcaaaaaac actgatatta aagtttatag agtcgataat 1320
   gctaatgatt tatctgaaag ttattatgtg aatcctagcg attttgaaga tgtaactaat 1380
   caagttagaa tttcatttcc aaatgctaat caatacaaag tagaatttcc tacggacgat 1440
   gaccaaatta caacaccgta tattgtagtt gttaatggcc atattgatcc tgctagtaca 1500
   ggtgatttag cactacgttc gacattttat ggttatgatt ctaattttat atggagatct 1560
   atgtcatggg acaacgaagt agcatttaat aacggatcag gttctggtga cggtatcgat 1620
   aaaccagttg ttcctgaaca acctgatgag cctggtgaaa ttgaaccaat tccagag 1677
<210> 74
   <211> 559
   <212> PRT
   <213> Staphylococcus aureus
<400> 74
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Ile Asn Ala
       50                  55                  60
   Ala Pro Lys Thr Asp Asn Thr Asn Val Ser Asp Thr Lys Thr Thr Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ala Ser Thr Asn Ala Thr Thr Glu Glu Thr Pro
               100                 105                 110
   Val Thr Gly Glu Thr Thr Thr Thr Ala Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Ser Thr Asp Ala Ser Asn Lys Asp Val Val Asn Gln Ala Val Asn Thr
           195                 200                 205
   Ser Ala Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asp Val Lys
   225                 230                 235                 240
   Val Thr Ile Asp Ser Gly Thr Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
           275                 280                 285
   Thr Ser Thr Ala Lys Val Pro Pro Ile Met Ala Gly Asp Gln Val Leu
       290                 295                 300
   Ala Asn Gly Val Ile Asp Ser Asp Gly Asn Val Ile Tyr Thr Phe Thr
   305                 310                 315                 320
   Asp Tyr Val Asp Asn Lys Glu Asn Val Thr Ala Asn Ile Thr Met Pro
                   325                 330                 335
   Ala Tyr Ile Asp Pro Glu Asn Val Thr Lys Thr Gly Asn Val Thr Leu
               340                 345                 350
   Thr Thr Gly Ile Gly Thr Asn Thr Ala Ser Lys Thr Val Leu Ile Asp
           355                 360                 365
   Tyr Glu Lys Tyr Gly Gln Phe His Asn Leu Ser Ile Lys Gly Thr Ile
       370                 375                 380
   Asp Gln Ile Asp Lys Thr Asn Asn Thr Tyr Arg Gln Thr Ile Tyr Val
   385                 390                 395                 400
   Asn Pro Ser Gly Asp Asn Val Val Leu Pro Ala Leu Thr Gly Asn Leu
                   405                 410                 415
   Ile Pro Asn Thr Lys Ser Asn Ala Leu Ile Asp Ala Lys Asn Thr Asp
               420                 425                 430
   Ile Lys Val Tyr Arg Val Asp Asn Ala Asn Asp Leu Ser Glu Ser Tyr
           435                 440                 445
   Tyr Val Asn Pro Ser Asp Phe Glu Asp Val Thr Asn Gln Val Arg Ile
       450                 455                 460
   Ser Phe Pro Asn Ala Asn Gln Tyr Lys Val Glu Phe Pro Thr Asp Asp
   465                 470                 475                 480
   Asp Gln Ile Thr Thr Pro Tyr Ile Val Val Val Asn Gly His Ile Asp
                   485                 490                 495
   Pro Ala Ser Thr Gly Asp Leu Ala Leu Arg Ser Thr Phe Tyr Gly Tyr
               500                 505                 510
   Asp Ser Asn Phe Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 75
   <211> 1677
   <212> DNA
   <213> Staphylococcus aureus
<400> 75
   atgaatatga agaaaaaaga aaaacacgca attcggaaaa aatcgattgg cgtggcttca      60
   gtgcttgtag gtacgttaat cggtttcgga ttacttagca gtaaagaagc agatgcaagt     120
   gaaaatagtg ttacgcaatc tgatagcgca agtaacgaaa gcaaaagtaa tgattcaagt     180
   agcgttaatg ctgcacctaa aacagacaac acaaacgtga gtgatactaa aacaacgtca     240
   aacactaata atggcgaaac gagtgtggcg caaaatccag cacaacagga aacgatacaa     300
   tcagcatcaa caaatgcaac tacggaagaa acaccggtaa ctggtgaagc tactactacg     360
   gcaacgaagc aagctaatac accggcaaca actcaatcaa gcaatacaaa tgcggaggaa     420
   ttagtgaatc aaacaagtaa tgaaacgact tctaatgata ctaatacagt atcatctgta     480
   aattcacctc aaaattctac aaatgcggaa aatgtttcaa caacgcaaga tacttcaact     540
   gaagcaacac cttcaaacaa tgaatcagct ccacagagta cagatgcaag taataaagat     600
   gtagttaatc aagcggttaa tacaagtgcg cctagaatga gagcatttag tttagcggct     660
   gtagctgcag atgcaccggc tgctggcaca gatattacga atcagttgac agatgtgaaa     720
   gttactattg actctggtac gactgtgtat ccgcaccaag caggttatgt caaactgaat     780
   tatggttttt cagtgcctaa ttctgctgtt aaaggtgaca cattcaaaat aactgtacct     840
   aaagaattaa acttaaatgg tgtaacttca actgctaaag tgccaccaat tatggccgga     900
   gatcaagtat tggcaaatgg tgtaatcgat agtgatggta atgttattta tacatttaca     960
   gactatgtaa atactaaaga tgatgttaaa gcaactttga ccatgcccgc ttatattgac    1020
   cctgaaaatg ttacaaagac aggtaatgtg acattggcta ctggcatagg tagtacaaca    1080
   gcaaacaaaa cagtattagt agattatgaa aaatatggta agttttataa cttatctatt    1140
   aaaggtacaa ttgaccaaat cgataaaaca aataatacgt atcgtcagac aatttatgtc    1200
   aatccaagtg gagataacgt tattgcgccg gttttaacag gtaatttaaa accaaatacg    1260
   gatagtaatg cattaataga tcagcaaaat acaagtatta aagtatataa agtagataat    1320
   gcagctgatt tatctgaaag ttactttgtg aatccagaaa actttgagga tgtcactaat    1380
   agtgtgaata ttacattccc aaatccaaat caatataaag tagagtttaa tacgcctgat    1440
   gatcaaatta caacaccgta tattgtagtt gttaatggtc atattgatcc gaatagcaaa    1500
   ggtgatttag ctttacgttc aactttatat gggtatgact caaggtttgt atggagatct    1560
   atgtcatggg acaacgaagt agcatttaat aacggatcag gttctggtga cggtatcgat    1620
   aaaccagttg ttcctgaaca acctgatgag cctggtgaaa ttgaaccaat tccagag       1677
<210> 76
   <211> 559
   <212> PRT
   <213> Staphylococcus aureus
<400> 76
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Asn Ala
       50                  55                  60
   Ala Pro Lys Thr Asp Asn Thr Asn Val Ser Asp Thr Lys Thr Thr Ser
   65                  70                  75                  80
   Asn Thr Asn Asn Gly Glu Thr Ser Val Ala Gln Asn Pro Ala Gln Gln
                   85                  90                  95
   Glu Thr Ile Gln Ser Ala Ser Thr Asn Ala Thr Thr Glu Glu Thr Pro
               100                 105                 110
   Val Thr Gly Glu Ala Thr Thr Thr Ala Thr Lys Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Ser Thr Asp Ala Ser Asn Lys Asp Val Val Asn Gln Ala Val Asn Thr
           195                 200                 205
   Ser Ala Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asp Val Lys
   225                 230                 235                 240
   Val Thr Ile Asp Ser Gly Thr Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asp Pro Glu Asn Val Thr Lys Thr Gly Asn Val Thr Leu
   340 345 350
   Ala Thr Gly Ile Gly Ser Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Asp Ser Asn Ala Leu Ile Asp Gln Gln Asn Thr Ser
   420 425 430
   Ile Lys Val Tyr Lys Val Asp Asn Ala Ala Asp Leu Ser Glu Ser Tyr
   435 440 445
   Phe Val Asn Pro Glu Asn Phe Glu Asp Val Thr Asn Ser Val Asn Ile
   450 455 460
   Thr Phe Pro Asn Pro Asn Gln Tyr Lys Val Glu Phe Asn Thr Pro Asp
   465 470 475 480
   Asp Gln Ile Thr Thr Pro Tyr Ile Val Val Val Asn Gly His Ile Asp
   485 490 495
   Pro Asn Ser Lys Gly Asp Leu Ala Leu Arg Ser Thr Leu Tyr Gly Tyr
   500 505 510
   Asp Ser Arg Phe Val Trp Arg Ser Met Ser Trp Asp Asn Glu Val Ala
   515 520 525
   Phe Asn Asn Gly Ser Gly Ser Gly Asp Gly Ile Asp Lys Pro Val Val
   530 535 540
   Pro Glu Gln Pro Asp Glu Pro Gly Glu Ile Glu Pro Ile Pro Glu
   545 550 555
<210> 77
   <211> 1677
   <212> DNA
   <213> Staphylococcus aureus
<400> 77
   atgaatatga agaaacaaga aaaacacgca attcgtaaaa aatcgattgg cgtggcttca      60
   gtgcttgtag gtacgttaat cggttttgga ctactcagca gtaaagaagc agatgcaagt     120
   gaaaatagtg ttacgcaaac ggaaaatacg agtaatgaga gcaaaagtaa tgattcgagt     180
   agcgttaatg ctgcacctaa aacagacaac acaaacgtga gtgattctaa tacaacgaca     240
   aacactaata gtgacgaaac gaatgtagcg caaaatccag cacaacagga aacgacacaa     300
   tcagcatcaa caaatgcaac tacagaagaa acaccggtaa ctggtgaagt tactactacg     360
   gcaacgaatc aagctaatac accggcaaca actcaatcaa gcaatacaaa tgcggaaaaa     420
   tcagtgaatc aaacaagtaa tgaaacgact tctaatgata ctaatacagt atcatctgta     480
   aattcacctc aaaattctac aaatgcggaa aatgtttcaa caacgcaaga tacttcaact     540
   gaagcaaaac cttcaaacaa tgaatcagct ccacagagta cagatgcaag taataaagat     600
   gtagttaatc aagcggttaa tacaagtgcg cctagaatga gagcatttag tttagcggct     660
   gtagctgcag atgcaccggc tgctggcaca gatattacga atcagttgac gaatgtgaca     720
   gttggtattg actctggaga tacagtttat ccgcaccaag caggctatgt caaactgaat     780
   tatgggttct cagtacccaa ttctgcggtt caaggtgaca catttaaaat aactgtacct     840
   aaagaattaa acttaaatgg tgtaacctca actgctaaag tgccaccaat tatggccgga     900
   gatcaagtat tggcaaatgg tgtaatcgat agtgatggta atgttattta tacatttaca     960
   gactatgtaa atactaaaga tgatgttaaa gcaacattaa ctgtgccggc ttatattgac    1020
   cctgaatatg ttacacatac tggtaatgtg acattggcta ctggcatagg aaatacaaca    1080
   gcaaacaaaa cagtattagt agattatgaa aaatatggta agttttataa cttatctatt    1140
   aaaggtacaa ttgaccaaat cgataaaaca aataatacgt atcgtcagac aatttatgtc    1200
   aatccaagtg gagataacgt tattgcaccg gttttaacag gtaatttaaa accaaatacg    1260
   gaaagtaatg cattaataga tcagcaaaat acaagtatta aagtatataa agtagataat    1320
   gcagctgatt tatctgaaag ttactttgtg aatccagaaa actttgagga tgtcactaat    1380
   agtgtaaata ttacattccc aaatccaaat caatataaag tagagtttaa tacgcctgat    1440
   gatcaaataa caacaccata tattgtagtt gttaatgggc atattgatcc taatagtaaa    1500
   ggtgatttag ctttacgttc aactttatat ggatataatt cgaatataat ttggagatct    1560
   atgtcatggg acaacgaagt agcatttaat aacggatcag gttctggtga cggtatcgat    1620
   aaacctgttg ttcctgaaca acctgatgag cctggtgaaa ttgaaccaat tccagag       1677
<210> 78
   <211> 559
   <212> PRT
   <213> Staphylococcus aureus
<400> 78
   Met Asn Met Lys Lys Gln Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Thr Glu
           35                  40                  45
   Asn Thr Ser Asn Glu Ser Lys Ser Asn Asp Ser Ser Ser Val Asn Ala
       50                  55                  60
   Ala Pro Lys Thr Asp Asn Thr Asn Val Ser Asp Ser Asn Thr Thr Thr
   65                  70                  75                  80
   Asn Thr Asn Ser Asp Glu Thr Asn Val Ala Gln Asn Pro Ala Gln Gln
                   85                  90                  95
   Glu Thr Thr Gln Ser Ala Ser Thr Asn Ala Thr Thr Glu Glu Thr Pro
               100                 105                 110
   Val Thr Gly Glu Val Thr Thr Thr Ala Thr Asn Gln Ala Asn Thr Pro
           115                 120                 125
   Ala Thr Thr Gln Ser Ser Asn Thr Asn Ala Glu Lys Ser Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Lys Pro Ser Asn Asn Glu Ser Ala Pro Gln
   180 185 190
   Ser Thr Asp Ala Ser Asn Lys Asp Val Val Asn Gln Ala Val Asn Thr
   195 200 205
   Ser Ala Pro Arg Met Arg Ala Phe Ser Leu Ala Ala Val Ala Ala Asp
   210 215 220
   Ala Pro Ala Ala Gly Thr Asp Ile Thr Asn Gln Leu Thr Asn Val Thr
   225 230 235 240
   Val Gly Ile Asp Ser Gly Asp Thr Val Tyr Pro His Gln Ala Gly Tyr
   245 250 255
   Val Lys Leu Asn Tyr Gly Phe Ser Val Pro Asn Ser Ala Val Gln Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Val Pro
   325 330 335
   Ala Tyr Ile Asp Pro Glu Tyr Val Thr His Thr Gly Asn Val Thr Leu
   340 345 350
   Ala Thr Gly Ile Gly Asn Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Glu Ser Asn Ala Leu Ile Asp Gln Gln Asn Thr Ser
   420 425 430
   Ile Lys Val Tyr Lys Val Asp Asn Ala Ala Asp Leu Ser Glu Ser Tyr
           435                 440                 445
   Phe Val Asn Pro Glu Asn Phe Glu Asp Val Thr Asn Ser Val Asn Ile
       450                 455                 460
   Thr Phe Pro Asn Pro Asn Gln Tyr Lys Val Glu Phe Asn Thr Pro Asp
   465                 470                 475                 480
   Asp Gln Ile Thr Thr Pro Tyr Ile Val Val Val Asn Gly His Ile Asp
                   485                 490                 495
   Pro Asn Ser Lys Gly Asp Leu Ala Leu Arg Ser Thr Leu Tyr Gly Tyr
               500                 505                 510
   Asn Ser Asn Ile Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 79
   <211> 1677
   <212> DNA
   <213> Staphylococcus aureus
<400> 79
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacagga aacgacacaa     300
   tcatcatcaa caaatgcaac tacggaagaa acgccggtaa ctggtgaagc tactactacg     360
   acaacgaatc aagctaatac accggcaaca actcaatcaa gcaatacaaa tgcggaggaa     420
   ttagtgaatc aaacaagtaa tgaaacgact tctaatgata ctaatacagt atcatctgta     480
   aattcacctc aaaattctac aaatgcggaa aatgtttcaa caacgcaaga tacttcaact     540
   gaagcaacac cttcaaacaa tgaatcagct ccacagagta cagatgcaag taataaagat     600
   gtagttaatc aagcggttaa tacaagtgcg cctagaatga gagcatttag tttagcggca     660
   gtagctgcag atgcaccggt agctggcaca gatattacga atcagttgac gaatgtgaca     720
   gttggtattg actctggtac gactgtgtat ccgcaccaag caggttatgt caaactgaat     780
   tatggttttt cagtgcctaa ttctgctgtt aaaggtgaca cattcaaaat aactgtacct     840
   aaagaattaa acttaaatgg tgtaacttca actgctaaag tgccaccaat tatggctgga     900
   gatcaagtat tggcaaatgg tgtaatcgat agtgatggta atgttattta tacatttaca     960
   gactatgtaa atactaaaga tgatgtaaaa gcaactttga ccatgcccgc ttatattgac    1020
   cctgaaaatg ttaaaaagac aggtaatgtg acattggcta ctggcatagg tagtacaaca    1080
   gcaaacaaaa cagtattagt agattatgaa aaatatggta agttttataa cttatctatt    1140
   aaaggtacaa ttgaccaaat cgataaaaca aataatacgt atcgtcagac aatttatgtc    1200
   aatccaagtg gagataacgt tattgcgccg gttttaacag gtaatttaaa accaaatacg    1260
   gatagtaatg cattaataga tcagcaaaat acaagtatta aagtatataa agtagataat    1320
   gcagctgatt tatctgaaag ttactttgtg aatccagaaa actttgagga tgtcactaat    1380
   agtgtgaata ttacattccc aaatccaaat caatataaag tagagtttaa tacgcctgat    1440
   gatcaaatta caacaccgta tatagtagtt gttaatggtc atattgatcc gaatagcaaa    1500
   ggtgatttag ctttacgttc aactttatat gggtataact cgaatataat ttggcgctct    1560
   atgtcatggg acaacgaagt agcatttaat aacggatcag gttctggtga cggtatcgat    1620
   aaaccagttg ttcctgaaca acctgatgag cctggtgaaa ttgaaccaat tccagag       1677
<210> 80
   <211> 559
   <212> PRT
   <213> Staphylococcus aureus
<400> 80
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
   100 105 110
   Val Thr Gly Glu Ala Thr Thr Thr Thr Thr Asn Gln Ala Asn Thr Pro
   115 120 125
   Ala Thr Thr Gln Ser Ser Asn Thr Asn Ala Glu Glu Leu Val Asn Gln
   130 135 140
   Thr Ser Asn Glu Thr Thr Ser Asn Asp Thr Asn Thr Val Ser Ser Val
   145 150 155 160
   Asn Ser Pro Gln Asn Ser Thr Asn Ala Glu Asn Val Ser Thr Thr Gln
   165 170 175
   Asp Thr Ser Thr Glu Ala Thr Pro Ser Asn Asn Glu Ser Ala Pro Gln
   180 185 190
   Ser Thr Asp Ala Ser Asn Lys Asp Val Val Asn Gln Ala Val Asn Thr
   195 200 205
   Ser Ala Pro Arg Met Arg Ala Phe Ser Leu Ala Ala Val Ala Ala Asp
   210 215 220
   Ala Pro Val Ala Gly Thr Asp Ile Thr Asn Gln Leu Thr Asn Val Thr
   225 230 235 240
   Val Gly Ile Asp Ser Gly Thr Thr Val Tyr Pro His Gln Ala Gly Tyr
   245 250 255
   Val Lys Leu Asn Tyr Gly Phe Ser Val Pro Asn Ser Ala Val Lys Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asp Pro Glu Asn Val Lys Lys Thr Gly Asn Val Thr Leu
   340 345 350
   Ala Thr Gly Ile Gly Ser Thr Thr Ala Asn Lys Thr Val Leu Val Asp
           355                 360                 365
   Tyr Glu Lys Tyr Gly Lys Phe Tyr Asn Leu Ser Ile Lys Gly Thr Ile
       370                 375                 380
   Asp Gln Ile Asp Lys Thr Asn Asn Thr Tyr Arg Gln Thr Ile Tyr Val
   385                 390                 395                 400
   Asn Pro Ser Gly Asp Asn Val Ile Ala Pro Val Leu Thr Gly Asn Leu
                   405                 410                 415
   Lys Pro Asn Thr Asp Ser Asn Ala Leu Ile Asp Gln Gln Asn Thr Ser
               420                 425                 430
   Ile Lys Val Tyr Lys Val Asp Asn Ala Ala Asp Leu Ser Glu Ser Tyr
           435                 440                 445
   Phe Val Asn Pro Glu Asn Phe Glu Asp Val Thr Asn Ser Val Asn Ile
       450                 455                 460
   Thr Phe Pro Asn Pro Asn Gln Tyr Lys Val Glu Phe Asn Thr Pro Asp
   465                 470                 475                 480
   Asp Gln Ile Thr Thr Pro Tyr Ile Val Val Val Asn Gly His Ile Asp
                   485                 490                 495
   Pro Asn Ser Lys Gly Asp Leu Ala Leu Arg Ser Thr Leu Tyr Gly Tyr
               500                 505                 510
   Asn Ser Asn Ile Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 81
   <211> 1677
   <212> DNA
   <213> Staphylococcus aureus
<400> 81
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacagga aacgacacaa     300
   tcatcatcaa caaatgcaac tacggaagaa acgccggtaa ctggtgaagc tactactacg     360
   acaacgaatc aagctaatac accggcaaca actcaatcaa gcaatacaaa tgcggaggaa     420
   ttagtgaatc aaacaagtaa tgaaacgact tttaatgata ctaatacagt atcatctgta     480
   aattcacctc aaaattctac aaatgcggaa aatgtttcaa caacgcaaga tacttcaact     540
   gaagcaacac cttcaaacaa tgaatcagct ccacagagta cagatgcaag taataaagat     600
   gtagttaatc aagcggttaa tacaagtgcg cctagaatga gagcatttag tttagcggca     660
   gtagctgcag atgcaccggc agctggcaca gatattacga atcagttgac gaatgtgaca     720
   gttggtattg actctggtac gactgtgtat ccgcaccaag caggttatgt caaactgaat     780
   tatggttttt cagtgcctaa ttctgctgtt aaaggtgaca cattcaaaat aactgtacct     840
   aaagaattaa acttaaatgg tgtaacttca actgctaaag tgccaccaat tatggctgga     900
   gatcaagtat tggcaaatgg tgtaatcgat agtgatggta atgttattta tacatttaca     960
   gactatgtaa atactaaaga tgatgtaaaa gcaactttga ccatgcccgc ttatattgac    1020
   cctgaaaatg ttaaaaagac aggtaatgtg acattggcta ctggcatagg tagtacaaca    1080
   gcaaacaaaa cagtattagt agattatgaa aaatatggta agttttataa cttatctatt    1140
   aaaggtacaa ttgaccaaat cgataaaaca aataatacgt atcgtcagac aatttatgtc    1200
   aatccaagtg gagataacgt tattgcgccg gttttaacag gtaatttaaa accaaatacg    1260
   gatagtaatg cattaataga tcagcaaaat acaagtatta aagtatataa agtagataat    1320
   gcagctgatt tatctgaaag ttactttgtg aatccagaaa actttgagga tgtcactaat    1380
   agtgtgaata ttacattccc aaatccaaat caatataaag tagagtttaa tacgcctgat    1440
   gatcaaatta caacaccgta tatagtagtt gttaatggtc atattgatcc gaatagcaaa    1500
   ggtgatttag ctttacgttc aactttatat gggtataact cgaatataat ttggcgctct    1560
   atgtcatggg acaacgaagt agcatttaat aacggatcag gttctggtga cggtatcgat    1620
   aaaccagttg ttcctgaaca acctgatgag cctggtgaaa ttgaaccaat tccagag        1677
<210> 82
   <211> 559
   <212> PRT
   <213> Staphylococcus aureus
<400> 82
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Phe Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Ser Thr Asp Ala Ser Asn Lys Asp Val Val Asn Gln Ala Val Asn Thr
           195                 200                 205
   Ser Ala Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asn Val Thr
   225                 230                 235                 240
   Val Gly Ile Asp Ser Gly Thr Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asp Pro Glu Asn Val Lys Lys Thr Gly Asn Val Thr Leu
   340 345 350
   Ala Thr Gly Ile Gly Ser Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Asp Ser Asn Ala Leu Ile Asp Gln Gln Asn Thr Ser
   420 425 430
   Ile Lys Val Tyr Lys Val Asp Asn Ala Ala Asp Leu Ser Glu Ser Tyr
   435 440 445
   Phe Val Asn Pro Glu Asn Phe Glu Asp Val Thr Asn Ser Val Asn Ile
   450 455 460
   Thr Phe Pro Asn Pro Asn Gln Tyr Lys Val Glu Phe Asn Thr Pro Asp
   465 470 475 480
   Asp Gln Ile Thr Thr Pro Tyr Ile Val Val Val Asn Gly His Ile Asp
   485 490 495
   Pro Asn Ser Lys Gly Asp Leu Ala Leu Arg Ser Thr Leu Tyr Gly Tyr
   500 505 510
   Asn Ser Asn Ile Ile Trp Arg Ser Met Ser Trp Asp Asn Glu Val Ala
   515 520 525
   Phe Asn Asn Gly Ser Gly Ser Gly Asp Gly Ile Asp Lys Pro Val Val
   530 535 540
   Pro Glu Gln Pro Asp Glu Pro Gly Glu Ile Glu Pro Ile Pro Glu
   545 550 555
<210> 83
   <211> 1674
   <212> DNA
   <213> Staphylococcus aureus
<400> 83
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacagga aacgacacaa     300
   tcagcattaa caaatgcaac tacggaagaa actccggtaa ctggtgaagc tactacggca     360
   acgaatcaag ctaatacacc ggcaacaact caatcaagca atacaaatgc ggaggaatta     420
   gtgaatcaaa caagtaatga aacgacttct aatgatacta atacagtatc atctgtaaat     480
   tcacctcaaa attctacaaa tgcggaaaat gtttcaacaa cgcaagatac ttcaactgaa     540
   gcaacacctt caaacaatga atcagctcca cagagtacag atgcaagtaa taaagatgta     600
   gttaatcaag cggttaatac aagtgcgcct agaatgagag catttagttt atcggcagta     660
   gctgcagatg caccggcagc tggcaaagat attacgaatc agttgacgaa tgtgacagtt     720
   ggtattgact ctggagatac agtttatccg caccaagcag gctatgtcaa actgaattat     780
   ggtttttcag tgcctaattc tgctgttaaa ggtgacacat tcaaaataac tgtacctaaa     840
   gaattaaact taaatggtgt aacttcaact gctaaagtgc ctccaattat ggccggagat     900
   caagtattgg caaatggtgt aatcgatagt gatggtaatg ttatttatac atttacagac     960
   tatgttgata ctaaagaaaa tgtaacagct aatattacta tgccagctta tattgaccct    1020
   gaaaatgtta caaagacagg taatgtaaca ttgacaactg gcataggtag tacaacagca    1080
   aacaaaacag tattagtaga ttatgaaaaa tatggtaagt tttataactt atctattaaa    1140
   ggtacaattg accaaatcga taaaacaaat aatacgtatc gtcagacaat ttatgtcaat    1200
   ccaagtggag ataatgttat tgcgccggtt ttaacaggta atttaaaacc aaatacggat    1260
   agtaatgcat taatagatca gcaaaataca agtattaaag tatataaagt agataatgca    1320
   gctgatttat ctgaaagtta ctttgtgaat ccagaaaact ttgaggatgt cactaatagt    1380
   gtgaatatta cattcccaaa tccaaatcaa tataaagtag agtttaatac gcctgatgat    1440
   caaattacaa caccgtatat tgtagttgtt aatggtcata ttgatccgaa tagcaaaggt    1500
   gatttagctt tacgttcaac tttatatgga tatgactcaa ggtttgtatg gagatctatg    1560
   tcatgggaca acgaagtagc atttaataac ggatcaggtt ctggtgacgg tatcgataaa    1620
   ccagttgttc ctgaacaacc tgatgagcct ggtgaaattg aaccaattcc agag          1674
<210> 84
   <211> 558
   <212> PRT
   <213> Staphylococcus aureus
<400> 84
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ala Leu Thr Asn Ala Thr Thr Glu Glu Thr Pro
               100                 105                 110
   Val Thr Gly Glu Ala Thr Thr Ala Thr Asn Gln Ala Asn Thr Pro Ala
           115                 120                 125
   Thr Thr Gln Ser Ser Asn Thr Asn Ala Glu Glu Leu Val Asn Gln Thr
       130                 135                 140
   Ser Asn Glu Thr Thr Ser Asn Asp Thr Asn Thr Val Ser Ser Val Asn
   145                 150                 155                 160
   Ser Pro Gln Asn Ser Thr Asn Ala Glu Asn Val Ser Thr Thr Gln Asp
                   165                 170                 175
   Thr Ser Thr Glu Ala Thr Pro Ser Asn Asn Glu Ser Ala Pro Gln Ser
               180                 185                 190
   Thr Asp Ala Ser Asn Lys Asp Val Val Asn Gln Ala Val Asn Thr Ser
           195                 200                 205
   Ala Pro Arg Met Arg Ala Phe Ser Leu Ser Ala Val Ala Ala Asp Ala
       210                 215                 220
   Pro Ala Ala Gly Lys Asp Ile Thr Asn Gln Leu Thr Asn Val Thr Val
   225                 230                 235                 240
   Gly Ile Asp Ser Gly Asp Thr Val Tyr Pro His Gln Ala Gly Tyr Val
                   245                 250                 255
   Lys Leu Asn Tyr Gly Phe Ser Val Pro Asn Ser Ala Val Lys Gly Asp
               260                 265                 270
   Thr Phe Lys Ile Thr Val Pro Lys Glu Leu Asn Leu Asn Gly Val Thr
           275                 280                 285
   Ser Thr Ala Lys Val Pro Pro Ile Met Ala Gly Asp Gln Val Leu Ala
       290                 295                 300
   Asn Gly Val Ile Asp Ser Asp Gly Asn Val Ile Tyr Thr Phe Thr Asp
   305                 310                 315                 320
   Tyr Val Asp Thr Lys Glu Asn Val Thr Ala Asn Ile Thr Met Pro Ala
                   325                 330                 335
   Tyr Ile Asp Pro Glu Asn Val Thr Lys Thr Gly Asn Val Thr Leu Thr
               340                 345                 350
   Thr Gly Ile Gly Ser Thr Thr Ala Asn Lys Thr Val Leu Val Asp Tyr
           355                 360                 365
   Glu Lys Tyr Gly Lys Phe Tyr Asn Leu Ser Ile Lys Gly Thr Ile Asp
       370                 375                 380
   Gln Ile Asp Lys Thr Asn Asn Thr Tyr Arg Gln Thr Ile Tyr Val Asn
   385                 390                 395                 400
   Pro Ser Gly Asp Asn Val Ile Ala Pro Val Leu Thr Gly Asn Leu Lys
                   405                 410                 415
   Pro Asn Thr Asp Ser Asn Ala Leu Ile Asp Gln Gln Asn Thr Ser Ile
               420                 425                 430
   Lys Val Tyr Lys Val Asp Asn Ala Ala Asp Leu Ser Glu Ser Tyr Phe
           435                 440                 445
   Val Asn Pro Glu Asn Phe Glu Asp Val Thr Asn Ser Val Asn Ile Thr
       450                 455                 460
   Phe Pro Asn Pro Asn Gln Tyr Lys Val Glu Phe Asn Thr Pro Asp Asp
   465                 470                 475                 480
   Gln Ile Thr Thr Pro Tyr Ile Val Val Val Asn Gly His Ile Asp Pro
                   485                 490                 495
   Asn Ser Lys Gly Asp Leu Ala Leu Arg Ser Thr Leu Tyr Gly Tyr Asp
               500                 505                 510
   Ser Arg Phe Val Trp Arg Ser Met Ser Trp Asp Asn Glu Val Ala Phe
           515                 520                 525
   Asn Asn Gly Ser Gly Ser Gly Asp Gly Ile Asp Lys Pro Val Val Pro
       530                 535                 540
   Glu Gln Pro Asp Glu Pro Gly Glu Ile Glu Pro Ile Pro Glu
   545                 550                 555
<210> 85
   <211> 1674
   <212> DNA
   <213> Staphylococcus aureus
<400> 85
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacagga aacgacacaa     300
   tcagcattaa caaatgcaac tacggaagaa actccgttaa ctggtgaagc tactacggca     360
   acgaatcaag ctaatacacc ggcaacaact caatcaagca atacaaatgc ggaggaatta     420
   gtgaatcaaa caagtaatga aacgacttct aatgatacta atacagtatc atctgtaaat     480
   tcacctcaaa attctacaaa tgcggaaaat gtttcaacaa cgcaagatac ttcaactgaa     540
   gcaacacctt caaacaatga atcagctcca cagagtacag atgcaagtaa taaagatgta     600
   gttaatcaag cggttaatac aagtgcgcct agaatgagag catttagttt agcggcagta     660
   gctgcagatg caccggcagc tggcacagat attacgaatc agttgacaga tgtgaaagtt     720
   actattgact ctggtacgac tgtgtatccg caccaagcag gttatgtcaa actgaattat     780
   ggtttttcag tgcctaattc tgctgttaaa ggtgacacat tcaaaataac tgtacctaaa     840
   gaattaaact taaatggtgt aacttcaact gctaaagtgc ctccaattat ggccggagat     900
   caagtattgg caaatggtgt aatcgatagt gatggtaatg ttatttatac atttacagac     960
   tatgttgata ctaaaaatga tgttaaagca acactaactg tgcctgcata cattgatcca    1020
   gaaaatgtta caaagacggg taatgtaaca ttgaaaactg gcataggaac caatactgat    1080
   agtaagacag ttttaatcga ctatgagaaa tatggacaat tccataattt atcaattaaa    1140
   ggtacgattg atcaaatcga taaaacaaat aatacgtatc gtcaaacaat ttatgtcaat    1200
   ccaagcggag ataacgttgt gttacctgcc ttaacaggta atttaattcc taatacaaag    1260
   agtaatgcgt taatagatgc aaaaaacact gatattaaag tttatagagt agataatgct    1320
   aatgatttat ctgaaagtta ttatgtgaat cctagcgatt ttgaagatgt aactaatcaa    1380
   gttagaattt catttccaaa tgctaatcaa tacaaagtag aatttcctac ggacgatgat    1440
   caaattacaa caccgtatat tgtagttgtt aatggccata ttgatcctgc tagcacaggt    1500
   gatttagcac tacgttcgac attttatggt tatgattcta attttatatg gagatctatg    1560
   tcatgggaca acgaagtagc atttaataac ggatcaggtt caggtgacgg tatcgataaa    1620
   cctgttgttc ctgaacaacc tgatgagcct ggtgaaattg aaccaattcc agag          1674
<210> 86
   <211> 558
   <212> PRT
   <213> Staphylococcus aureus
<400> 86
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ala Leu Thr Asn Ala Thr Thr Glu Glu Thr Pro
               100                 105                 110
   Leu Thr Gly Glu Ala Thr Thr Ala Thr Asn Gln Ala Asn Thr Pro Ala
           115                 120                 125
   Thr Thr Gln Ser Ser Asn Thr Asn Ala Glu Glu Leu Val Asn Gln Thr
       130                 135                 140
   Ser Asn Glu Thr Thr Ser Asn Asp Thr Asn Thr Val Ser Ser Val Asn
   145                 150                 155                 160
   Ser Pro Gln Asn Ser Thr Asn Ala Glu Asn Val Ser Thr Thr Gln Asp
                   165                 170                 175
   Thr Ser Thr Glu Ala Thr Pro Ser Asn Asn Glu Ser Ala Pro Gln Ser
               180                 185                 190
   Thr Asp Ala Ser Asn Lys Asp Val Val Asn Gln Ala Val Asn Thr Ser
           195                 200                 205
   Ala Pro Arg Met Arg Ala Phe Ser Leu Ala Ala Val Ala Ala Asp Ala
       210                 215                 220
   Pro Ala Ala Gly Thr Asp Ile Thr Asn Gln Leu Thr Asp Val Lys Val
   225                 230                 235                 240
   Thr Ile Asp Ser Gly Thr Thr Val Tyr Pro His Gln Ala Gly Tyr Val
                   245                 250                 255
   Lys Leu Asn Tyr Gly Phe Ser Val Pro Asn Ser Ala Val Lys Gly Asp
               260                 265                 270
   Thr Phe Lys Ile Thr Val Pro Lys Glu Leu Asn Leu Asn Gly Val Thr
           275                 280                 285
   Ser Thr Ala Lys Val Pro Pro Ile Met Ala Gly Asp Gln Val Leu Ala
       290                 295                 300
   Asn Gly Val Ile Asp Ser Asp Gly Asn Val Ile Tyr Thr Phe Thr Asp
   305                 310                 315                 320
   Tyr Val Asp Thr Lys Asn Asp Val Lys Ala Thr Leu Thr Val Pro Ala
                   325                 330                 335
   Tyr Ile Asp Pro Glu Asn Val Thr Lys Thr Gly Asn Val Thr Leu Lys
               340                 345                 350
   Thr Gly Ile Gly Thr Asn Thr Asp Ser Lys Thr Val Leu Ile Asp Tyr
           355                 360                 365
   Glu Lys Tyr Gly Gln Phe His Asn Leu Ser Ile Lys Gly Thr Ile Asp
       370                 375                 380
   Gln Ile Asp Lys Thr Asn Asn Thr Tyr Arg Gln Thr Ile Tyr Val Asn
   385                 390                 395                 400
   Pro Ser Gly Asp Asn Val Val Leu Pro Ala Leu Thr Gly Asn Leu Ile
                   405                 410                 415
   Pro Asn Thr Lys Ser Asn Ala Leu Ile Asp Ala Lys Asn Thr Asp Ile
               420                 425                 430
   Lys Val Tyr Arg Val Asp Asn Ala Asn Asp Leu Ser Glu Ser Tyr Tyr
           435                 440                 445
   Val Asn Pro Ser Asp Phe Glu Asp Val Thr Asn Gln Val Arg Ile Ser
       450                 455                 460
   Phe Pro Asn Ala Asn Gln Tyr Lys Val Glu Phe Pro Thr Asp Asp Asp
   465                 470                 475                 480
   Gln Ile Thr Thr Pro Tyr Ile Val Val Val Asn Gly His Ile Asp Pro
                   485                 490                 495
   Ala Ser Thr Gly Asp Leu Ala Leu Arg Ser Thr Phe Tyr Gly Tyr Asp
               500                 505                 510
   Ser Asn Phe Ile Trp Arg Ser Met Ser Trp Asp Asn Glu Val Ala Phe
           515                 520                 525
   Asn Asn Gly Ser Gly Ser Gly Asp Gly Ile Asp Lys Pro Val Val Pro
       530                 535                 540
   Glu Gln Pro Asp Glu Pro Gly Glu Ile Glu Pro Ile Pro Glu
   545                 550                 555
<210> 87
   <211> 1674
   <212> DNA
   <213> Staphylococcus aureus
<400> 87
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttaatg ctgcacctaa aacagacaac acaaacgtga gtgatactaa aacaacgtca     240
   aacactaata atggcgaaac gagtgtggcg caatatctag cacaacagga aacgacacaa     300
   tcagtatcaa caaatgcaac tacggaagaa acaccggtaa ctggtgaagc tactacggca     360
   acgaatcaag ctaatacacc ggcaacaact caatcaagca atacaaatgc ggaggaatta     420
   gtgaatcaaa caagtaatga aacgacttct aatgatacta atacagtatc atctgtaaat     480
   tcacctcaaa attctacaaa tgcggaaaat gtttcaacaa cgcaagatac ttcaactgaa     540
   gcaacacctt caaacaatga atcagctcca cagagtacag atgcaagtaa taaagatgta     600
   gttaatcaag cggttaatac aagtgcgcct agaatgagag catttagttt agcggctgta     660
   gctgcagatg caccggcagc tggcacagat attacgaatc agttgacgaa tgtgacagtt     720
   ggtattgact ctggtacgac tgtgtatccg caccaagcag gttatgtcaa actgaattat     780
   ggtttttcag tgcctaattc tgctgttaaa ggtgacacat tcaaaataac tgtacctaaa     840
   gaattaaact taaatggtgt aacttcaact gctaaagtgc ctccaattat ggctggagat     900
   caagtattgg caaatggtgt aatcgataat gatggtaatg ttatttatac atttacagac     960
   tatgtaaata ctaaagatga tgttaaagca actttgacta tgcccgctta tattgaccct    1020
   gaaaatgtta caaagacagg taatgtgaca ttggctactg gcataggtag tacaacagca    1080
   aacaaaacag tattagtaga ttatgaaaaa tatggtaagt tttataactt atctattaaa    1140
   ggtacaattg accaaatcga taaaacaaat aatacgtatc gtcagacaat ttatgtcaat    1200
   ccaagtggag ataacgttat tgcgccggtt ttaacaggta atttaaaacc aaatacggat    1260
   agtaatgcat taatagatca gcaaaataca agtattaaag tatataaagt agataatgca    1320
   gctgatttat ctgaaagtta ctttgtgaat ccagaaaact ttgaggatgt cactaatagt    1380
   gtgaatatta cattcccaaa tccaaatcaa tataaagtag agtttccaac agacgatgat    1440
   caaattacaa caccgtatat tgtagttgtt aatggtcata ttgatccgaa tagcaaaggt    1500
   gatttagctt tacgttcaac tttatatggg tataactcga atataatttg gcgctctatg    1560
   tcatgggaca acgaagtagc atttaataac ggatcaggtt ctggtgacgg tatcgataaa    1620
   cctgttgttc ctgaacaacc tgatgagcct ggtgaaattg aaccaattcc agag          1674
<210> 88
   <211> 558
   <212> PRT
   <213> Staphylococcus aureus
<400> 88
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Asn Ala
       50                  55                  60
   Ala Pro Lys Thr Asp Asn Thr Asn Val Ser Asp Thr Lys Thr Thr Ser
   65                  70                  75                  80
   Asn Thr Asn Asn Gly Glu Thr Ser Val Ala Gln Tyr Leu Ala Gln Gln
                   85                  90                  95
   Glu Thr Thr Gln Ser Val Ser Thr Asn Ala Thr Thr Glu Glu Thr Pro
               100                 105                 110
   Val Thr Gly Glu Ala Thr Thr Ala Thr Asn Gln Ala Asn Thr Pro Ala
           115                 120                 125
   Thr Thr Gln Ser Ser Asn Thr Asn Ala Glu Glu Leu Val Asn Gln Thr
       130                 135                 140
   Ser Asn Glu Thr Thr Ser Asn Asp Thr Asn Thr Val Ser Ser Val Asn
   145                 150                 155                 160
   Ser Pro Gln Asn Ser Thr Asn Ala Glu Asn Val Ser Thr Thr Gln Asp
                   165                 170                 175
   Thr Ser Thr Glu Ala Thr Pro Ser Asn Asn Glu Ser Ala Pro Gln Ser
               180                 185                 190
   Thr Asp Ala Ser Asn Lys Asp Val Val Asn Gln Ala Val Asn Thr Ser
           195                 200                 205
   Ala Pro Arg Met Arg Ala Phe Ser Leu Ala Ala Val Ala Ala Asp Ala
       210                 215                 220
   Pro Ala Ala Gly Thr Asp Ile Thr Asn Gln Leu Thr Asn Val Thr Val
   225                 230                 235                 240
   Gly Ile Asp Ser Gly Thr Thr Val Tyr Pro His Gln Ala Gly Tyr Val
                   245                 250                 255
   Lys Leu Asn Tyr Gly Phe Ser Val Pro Asn Ser Ala Val Lys Gly Asp
               260                 265                 270
   Thr Phe Lys Ile Thr Val Pro Lys Glu Leu Asn Leu Asn Gly Val Thr
   275 280 285
   Ser Thr Ala Lys Val Pro Pro Ile Met Ala Gly Asp Gln Val Leu Ala
   290 295 300
   Asn Gly Val Ile Asp Asn Asp Gly Asn Val Ile Tyr Thr Phe Thr Asp
   305 310 315 320
   Tyr Val Asn Thr Lys Asp Asp Val Lys Ala Thr Leu Thr Met Pro Ala
   325 330 335
   Tyr Ile Asp Pro Glu Asn Val Thr Lys Thr Gly Asn Val Thr Leu Ala
   340 345 350
   Thr Gly Ile Gly Ser Thr Thr Ala Asn Lys Thr Val Leu Val Asp Tyr
   355 360 365
   Glu Lys Tyr Gly Lys Phe Tyr Asn Leu Ser Ile Lys Gly Thr Ile Asp
   370 375 380
   Gln Ile Asp Lys Thr Asn Asn Thr Tyr Arg Gln Thr Ile Tyr Val Asn
   385 390 395 400
   Pro Ser Gly Asp Asn Val Ile Ala Pro Val Leu Thr Gly Asn Leu Lys
   405 410 415
   Pro Asn Thr Asp Ser Asn Ala Leu Ile Asp Gln Gln Asn Thr Ser Ile
   420 425 430
   Lys Val Tyr Lys Val Asp Asn Ala Ala Asp Leu Ser Glu Ser Tyr Phe
   435 440 445
   Val Asn Pro Glu Asn Phe Glu Asp Val Thr Asn Ser Val Asn Ile Thr
   450 455 460
   Phe Pro Asn Pro Asn Gln Tyr Lys Val Glu Phe Pro Thr Asp Asp Asp
   465 470 475 480
   Gln Ile Thr Thr Pro Tyr Ile Val Val Val Asn Gly His Ile Asp Pro
   485 490 495
   Asn Ser Lys Gly Asp Leu Ala Leu Arg Ser Thr Leu Tyr Gly Tyr Asn
   500 505 510
   Ser Asn Ile Ile Trp Arg Ser Met Ser Trp Asp Asn Glu Val Ala Phe
   515 520 525
   Asn Asn Gly Ser Gly Ser Gly Asp Gly Ile Asp Lys Pro Val Val Pro
   530 535 540
   Glu Gln Pro Asp Glu Pro Gly Glu Ile Glu Pro Ile Pro Glu
   545 550 555
<210> 89
   <211> 1674
   <212> DNA
   <213> Staphylococcus aureus
<400> 89
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacagga aacgacacaa     300
   tcagcattaa caaatgcaac tacggaagaa actccggtaa ctggtgaagc tactacggca     360
   acgaatcaag ctaatacacc ggcaacaact caatcaagca atacaaatgc ggaggaatta     420
   gtgaatcaaa caagtaatga aacgacttct aatgatacta atacagtatc atctgtaaat     480
   tcacctcaaa attctacaaa tgcggaaaat gtttcaacaa cgcaagatac ttcaactgaa     540
   gcaacacctt caaacaatga atcagctcca cagaatacag atgcaagtaa taaagatgta     600
   gttaatcaag cggttaatac aagtgcgcct agaaagagag catttagttt agcggctgta     660
   gctgcagatg caccggcagc tggcacagat attacgaatc agttgacaga tgtgaaagtt     720
   actattgact ctggtacgac tgtgtatccg caccaagcag gttatgtcaa actgaattat     780
   ggtttttcag tgcctaattc tgctgttaaa ggtgacacat tcaaaataac tgtacctaaa     840
   gaattaaact taaatggtgt aacttcaact gctaaagtgc caccaattat ggctggagat     900
   caagtattgg caaatggtgt aatcgatagt gatggtaatg ttatttatac atttacagac     960
   tatgttgata ataaagaaaa tgtaacagct aatattacta tgccagctta tattgaccct    1020
   gaaaatgtta caaagacagg taatgtgaca ttgacaactg gcataggaac caatactgct    1080
   agtaagacag tattaatcga ctatgagaaa tatggacaat tccataattt atcaattaaa    1140
   ggtacgattg atcaaatcga taaaacaaat aatacgtatc gccaaacaat ttatgtcaat    1200
   ccaagcggag ataacgttgt gttacctgcc ttaacaggta atttaattcc taatacaaag    1260
   agtaatgcgt taatagatgc aaaaaacact gatattaaag tttatagagt cgataatgct    1320
   aatgatttat ctgaaagtta ttatgtgaat cctagcgatt ttgaagatgt aactaatcaa    1380
   gttagaattt catttccaaa tgctaatcaa tacaaagtag aatttcctac ggacgatgac    1440
   caaattacaa caccgtatat tgtagttgtt aatggccata ttgatcctgc tagtacaggt    1500
   gatttagcac tacgttcgac attttatggt tatgattcta attttatatg gagatctatg    1560
   tcatgggaca acgaagtagc atttaataac ggatcaggtt ctggtgacgg tatcgataaa    1620
   ccagttgttc ctgaacaacc tgatgagcct ggtgaaattg aaccaattcc agag          1674
<210> 90
   <211> 558
   <212> PRT
   <213> Staphylococcus aureus
<400> 90
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ala Leu Thr Asn Ala Thr Thr Glu Glu Thr Pro
               100                 105                 110
   Val Thr Gly Glu Ala Thr Thr Ala Thr Asn Gln Ala Asn Thr Pro Ala
           115                 120                 125
   Thr Thr Gln Ser Ser Asn Thr Asn Ala Glu Glu Leu Val Asn Gln Thr
       130                 135                 140
   Ser Asn Glu Thr Thr Ser Asn Asp Thr Asn Thr Val Ser Ser Val Asn
   145                 150                 155                 160
   Ser Pro Gln Asn Ser Thr Asn Ala Glu Asn Val Ser Thr Thr Gln Asp
                   165                 170                 175
   Thr Ser Thr Glu Ala Thr Pro Ser Asn Asn Glu Ser Ala Pro Gln Asn
   180 185 190
   Thr Asp Ala Ser Asn Lys Asp Val Val Asn Gln Ala Val Asn Thr Ser
   195 200 205
   Ala Pro Arg Lys Arg Ala Phe Ser Leu Ala Ala Val Ala Ala Asp Ala
   210 215 220
   Pro Ala Ala Gly Thr Asp Ile Thr Asn Gln Leu Thr Asp Val Lys Val
   225 230 235 240
   Thr Ile Asp Ser Gly Thr Thr Val Tyr Pro His Gln Ala Gly Tyr Val
   245 250 255
   Lys Leu Asn Tyr Gly Phe Ser Val Pro Asn Ser Ala Val Lys Gly Asp
   260 265 270
   Thr Phe Lys Ile Thr Val Pro Lys Glu Leu Asn Leu Asn Gly Val Thr
   275 280 285
   Ser Thr Ala Lys Val Pro Pro Ile Met Ala Gly Asp Gln Val Leu Ala
   290 295 300
   Asn Gly Val Ile Asp Ser Asp Gly Asn Val Ile Tyr Thr Phe Thr Asp
   305 310 315 320
   Tyr Val Asp Asn Lys Glu Asn Val Thr Ala Asn Ile Thr Met Pro Ala
   325 330 335
   Tyr Ile Asp Pro Glu Asn Val Thr Lys Thr Gly Asn Val Thr Leu Thr
   340 345 350
   Thr Gly Ile Gly Thr Asn Thr Ala Ser Lys Thr Val Leu Ile Asp Tyr
   355 360 365
   Glu Lys Tyr Gly Gln Phe His Asn Leu Ser Ile Lys Gly Thr Ile Asp
   370 375 380
   Gln Ile Asp Lys Thr Asn Asn Thr Tyr Arg Gln Thr Ile Tyr Val Asn
   385 390 395 400
   Pro Ser Gly Asp Asn Val Val Leu Pro Ala Leu Thr Gly Asn Leu Ile
   405 410 415
   Pro Asn Thr Lys Ser Asn Ala Leu Ile Asp Ala Lys Asn Thr Asp Ile
   420 425 430
   Lys Val Tyr Arg Val Asp Asn Ala Asn Asp Leu Ser Glu Ser Tyr Tyr
           435                 440                 445
   Val Asn Pro Ser Asp Phe Glu Asp Val Thr Asn Gln Val Arg Ile Ser
       450                 455                 460
   Phe Pro Asn Ala Asn Gln Tyr Lys Val Glu Phe Pro Thr Asp Asp Asp
   465                 470                 475                 480
   Gln Ile Thr Thr Pro Tyr Ile Val Val Val Asn Gly His Ile Asp Pro
                   485                 490                 495
   Ala Ser Thr Gly Asp Leu Ala Leu Arg Ser Thr Phe Tyr Gly Tyr Asp
               500                 505                 510
   Ser Asn Phe Ile Trp Arg Ser Met Ser Trp Asp Asn Glu Val Ala Phe
           515                 520                 525
   Asn Asn Gly Ser Gly Ser Gly Asp Gly Ile Asp Lys Pro Val Val Pro
       530                 535                 540
   Glu Gln Pro Asp Glu Pro Gly Glu Ile Glu Pro Ile Pro Glu
   545                 550                 555
<210> 91
   <211> 1674
   <212> DNA
   <213> Staphylococcus aureus
<400> 91
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacagga aacgacacaa     300
   tcatcattaa caaatgcaac tacggaagaa actccggtaa ctggtgaagc tactacggca     360
   acgaatcaag ctaatacacc ggcaacaact caatcaagca atacaaatgc ggaggaatta     420
   gtgaatcaaa caagtaatga aacgacttct aatgatacta atacagtatc atctgtaaat     480
   tcacctcaaa attctacaaa tgcggaaaat gtttcaacaa cgcaagatac ttcaactgaa     540
   gcaacacctt caaacaatga atcagctcca cagagtacag atgcaagtaa taaagattta     600
   gttaatcaag cggttaatac aagtgcgcct agaatgagag catttagttt agcggcagta     660
   gctgcagatg cacctgcagc tggcacagat attacgaatc agttgacaga tgttaaagtt     720
   actattgact ctggtacgac tgtgtatccg caccaagcag gttatgtcaa actgaattat     780
   ggtttttcag tgcctaattc tgctgttaaa ggtgacacat tcaaaataac tgtacctaaa     840
   gaattaaact taaatggtgt aacttcaact gctaaagtgc caccaattat ggctggagat     900
   caagtattgg caaatggtgt aatcgatagt gatggtaatg ttatttatac atttacagac     960
   tatgttgata ataaaaacga tgttaaagca actttgacca tgcccgctta tattgatcca    1020
   gaaaatgtaa cgaaaacagg taatgtaaca ttgacaactg gcataggtag tacaacagca    1080
   aacaaaacag tattagtaga ttatgaaaaa tatggtaagt tttataactt atctattaaa    1140
   ggtacaattg accaaatcga taaaacaaat aatacgtatc gtcagacaat ttatgtcaat    1200
   ccaagtggag ataacgttat tgcgccggtt ttaacaggta atttaaaacc aaatacggat    1260
   agtaatgcat taatagatca gcaaaataca agtattaaag tatataaagt agataatgca    1320
   gctgatttat ctgaaagtta ctttgtaaat ccagaaaact ttgaggatgt cactaatagt    1380
   gtgaatatta cattcccaaa tccaaatcaa tataaagtag agtttaatac gcctgatgat    1440
   caaattacaa caccgtatat tgtagttgtt aatggtcata ttgatccgaa tagcaaaggt    1500
   gatttagctt tacgttcaac tttatatggg tataactcga atataatttg gcgctctatg    1560
   tcatgggaca acgaagtagc atttaataac ggatcaggtt ctggtgacgg tatcgataaa    1620
   cctgttgtcc ctgaacaacc tgatgagcct ggtgaaattg aaccaattcc agag          1674
<210> 92
   <211> 558
   <212> PRT
   <213> Staphylococcus aureus
<400> 92
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Leu Thr Asn Ala Thr Thr Glu Glu Thr Pro
               100                 105                 110
   Val Thr Gly Glu Ala Thr Thr Ala Thr Asn Gln Ala Asn Thr Pro Ala
           115                 120                 125
   Thr Thr Gln Ser Ser Asn Thr Asn Ala Glu Glu Leu Val Asn Gln Thr
       130                 135                 140
   Ser Asn Glu Thr Thr Ser Asn Asp Thr Asn Thr Val Ser Ser Val Asn
   145                 150                 155                 160
   Ser Pro Gln Asn Ser Thr Asn Ala Glu Asn Val Ser Thr Thr Gln Asp
                   165                 170                 175
   Thr Ser Thr Glu Ala Thr Pro Ser Asn Asn Glu Ser Ala Pro Gln Ser
               180                 185                 190
   Thr Asp Ala Ser Asn Lys Asp Leu Val Asn Gln Ala Val Asn Thr Ser
           195                 200                 205
   Ala Pro Arg Met Arg Ala Phe Ser Leu Ala Ala Val Ala Ala Asp Ala
       210                 215                 220
   Pro Ala Ala Gly Thr Asp Ile Thr Asn Gln Leu Thr Asp Val Lys Val
   225                 230                 235                 240
   Thr Ile Asp Ser Gly Thr Thr Val Tyr Pro His Gln Ala Gly Tyr Val
                   245                 250                 255
   Lys Leu Asn Tyr Gly Phe Ser Val Pro Asn Ser Ala Val Lys Gly Asp
               260                 265                 270
   Thr Phe Lys Ile Thr Val Pro Lys Glu Leu Asn Leu Asn Gly Val Thr
           275                 280                 285
   Ser Thr Ala Lys Val Pro Pro Ile Met Ala Gly Asp Gln Val Leu Ala
       290                 295                 300
   Asn Gly Val Ile Asp Ser Asp Gly Asn Val Ile Tyr Thr Phe Thr Asp
   305                 310                 315                 320
   Tyr Val Asp Asn Lys Asn Asp Val Lys Ala Thr Leu Thr Met Pro Ala
                   325                 330                 335
   Tyr Ile Asp Pro Glu Asn Val Thr Lys Thr Gly Asn Val Thr Leu Thr
               340                 345                 350
   Thr Gly Ile Gly Ser Thr Thr Ala Asn Lys Thr Val Leu Val Asp Tyr
   355 360 365
   Glu Lys Tyr Gly Lys Phe Tyr Asn Leu Ser Ile Lys Gly Thr Ile Asp
   370 375 380
   Gln Ile Asp Lys Thr Asn Asn Thr Tyr Arg Gln Thr Ile Tyr Val Asn
   385 390 395 400
   Pro Ser Gly Asp Asn Val Ile Ala Pro Val Leu Thr Gly Asn Leu Lys
   405 410 415
   Pro Asn Thr Asp Ser Asn Ala Leu Ile Asp Gln Gln Asn Thr Ser Ile
   420 425 430
   Lys Val Tyr Lys Val Asp Asn Ala Ala Asp Leu Ser Glu Ser Tyr Phe
   435 440 445
   Val Asn Pro Glu Asn Phe Glu Asp Val Thr Asn Ser Val Asn Ile Thr
   450 455 460
   Phe Pro Asn Pro Asn Gln Tyr Lys Val Glu Phe Asn Thr Pro Asp Asp
   465 470 475 480
   Gln Ile Thr Thr Pro Tyr Ile Val Val Val Asn Gly His Ile Asp Pro
   485 490 495
   Asn Ser Lys Gly Asp Leu Ala Leu Arg Ser Thr Leu Tyr Gly Tyr Asn
   500 505 510
   Ser Asn Ile Ile Trp Arg Ser Met Ser Trp Asp Asn Glu Val Ala Phe
   515 520 525
   Asn Asn Gly Ser Gly Ser Gly Asp Gly Ile Asp Lys Pro Val Val Pro
   530 535 540
   Glu Gln Pro Asp Glu Pro Gly Glu Ile Glu Pro Ile Pro Glu
   545 550 555
<210> 93
   <211> 1674
   <212> DNA
   <213> Staphylococcus aureus
<400> 93
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc tgatagcgca agtaacgaaa gcaaaagtaa tgatccaagt 180
   agcgttaatg ctgcacctaa aacagacaac acaaacgtga gtgatactaa aacatcgtca 240
   aacactaata atggcgaaac gagtgtggcg caaaatccag cacaacagga aacgacacaa 300
   tcatcattaa caaatgcaac tacggaagaa actccggtaa ctggtgaagc tactacggca 360
   acgaatcaag ctaatacacc ggcaacaact caatcaagca atacaaatgc ggaggaatta 420
   gtgaatcaaa caagtaatga aacgacttct aatgatacta atacagtatc atctgtaaat 480
   tcacctcaaa attctacaaa tgcggaaaat gtttcaacaa cgcaagatac ttcaactgaa 540
   gcaacacctt caaacaatga atcagctcca cagagtacag atgcaagtaa taaagattta 600
   gttaatcaag cggttaatac aagtgcgcct agaatgagag catttagttt agcggcagta 660
   gctgcagatg cacctgcagc tggcacagat attacgaatc agttgacaga tgttaaagtt 720
   actattgact ctggtacgac tgtgtatccg caccaagcag gttatgtcaa actgaattat 780
   ggtttttcag tgcctaattc tgctgttaaa ggtgacacat tcaaaataac tgtacctaaa 840
   gaattaaact taaatggtgt aacttcaact gctaaagtgc ctccaattat ggccggagat 900
   caagtattgg caaatggtgt aatcgatagt gatggtaatg ttatttatac atttacagac 960
   tatgttgata ctaaagaaaa tgtaacagct aatattacta tgccagctta tattgaccct 1020
   gaaaatgtta caaagacagg taatgtaaca ttgacaactg gcataggtag tacaacagca 1080
   aacaaaacag tattagtaga ttatgaaaaa tatggtaagt tttataactt atctattaaa 1140
   ggtacaattg accaaatcga taaaacaaat aatacgtatc gtcagacaat ttatgtcaat 1200
   ccaagtggag ataacgttat tgcgccggtt ttaacaggta atttaaaacc caatacggat 1260
   agtaatgcat taatagatca gcaaaataca agtattaaag tatataaagt agataatgca 1320
   gctgatttat ctgaaagtta ctttgtaaat ccagaaaact ttgaggatgt cactaatagt 1380
   gtgaatatta cattcccaaa tccaaatcaa tataaagtag agtttaatac gcctgatgat 1440
   caaattacaa caccgtatat tgtagttgtt aatggtcata ttgatccgaa tagcaaaggt 1500
   gatttagctt tacgttcaac tttatatggg tataactcga atataatttg gcgctctatg 1560
   tcatgggaca acgaagtagc atttaataac ggatcaggtt ctggtgacgg tatcgataaa 1620
   cctgttgtcc ctgaacaacc tgatgagcct ggtgaaattg aaccaattcc agag 1674
<210> 94
   <211> 558
   <212> PRT
   <213> Staphylococcus aureus
<400> 94
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Pro Ser Ser Val Asn Ala
       50                  55                  60
   Ala Pro Lys Thr Asp Asn Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Leu Thr Asn Ala Thr Thr Glu Glu Thr Pro
               100                 105                 110
   Val Thr Gly Glu Ala Thr Thr Ala Thr Asn Gln Ala Asn Thr Pro Ala
           115                 120                 125
   Thr Thr Gln Ser Ser Asn Thr Asn Ala Glu Glu Leu Val Asn Gln Thr
       130                 135                 140
   Ser Asn Glu Thr Thr Ser Asn Asp Thr Asn Thr Val Ser Ser Val Asn
   145                 150                 155                 160
   Ser Pro Gln Asn Ser Thr Asn Ala Glu Asn Val Ser Thr Thr Gln Asp
                   165                 170                 175
   Thr Ser Thr Glu Ala Thr Pro Ser Asn Asn Glu Ser Ala Pro Gln Ser
               180                 185                 190
   Thr Asp Ala Ser Asn Lys Asp Leu Val Asn Gln Ala Val Asn Thr Ser
           195                 200                 205
   Ala Pro Arg Met Arg Ala Phe Ser Leu Ala Ala Val Ala Ala Asp Ala
       210                 215                 220
   Pro Ala Ala Gly Thr Asp Ile Thr Asn Gln Leu Thr Asp Val Lys Val
   225                 230                 235                 240
   Thr Ile Asp Ser Gly Thr Thr Val Tyr Pro His Gln Ala Gly Tyr Val
                   245                 250                 255
   Lys Leu Asn Tyr Gly Phe Ser Val Pro Asn Ser Ala Val Lys Gly Asp
               260                 265                 270
   Thr Phe Lys Ile Thr Val Pro Lys Glu Leu Asn Leu Asn Gly Val Thr
   275 280 285
   Ser Thr Ala Lys Val Pro Pro Ile Met Ala Gly Asp Gln Val Leu Ala
   290 295 300
   Asn Gly Val Ile Asp Ser Asp Gly Asn Val Ile Tyr Thr Phe Thr Asp
   305 310 315 320
   Tyr Val Asp Thr Lys Glu Asn Val Thr Ala Asn Ile Thr Met Pro Ala
   325 330 335
   Tyr Ile Asp Pro Glu Asn Val Thr Lys Thr Gly Asn Val Thr Leu Thr
   340 345 350
   Thr Gly Ile Gly Ser Thr Thr Ala Asn Lys Thr Val Leu Val Asp Tyr
   355 360 365
   Glu Lys Tyr Gly Lys Phe Tyr Asn Leu Ser Ile Lys Gly Thr Ile Asp
   370 375 380
   Gln Ile Asp Lys Thr Asn Asn Thr Tyr Arg Gln Thr Ile Tyr Val Asn
   385 390 395 400
   Pro Ser Gly Asp Asn Val Ile Ala Pro Val Leu Thr Gly Asn Leu Lys
   405 410 415
   Pro Asn Thr Asp Ser Asn Ala Leu Ile Asp Gln Gln Asn Thr Ser Ile
   420 425 430
   Lys Val Tyr Lys Val Asp Asn Ala Ala Asp Leu Ser Glu Ser Tyr Phe
   435 440 445
   Val Asn Pro Glu Asn Phe Glu Asp Val Thr Asn Ser Val Asn Ile Thr
   450 455 460
   Phe Pro Asn Pro Asn Gln Tyr Lys Val Glu Phe Asn Thr Pro Asp Asp
   465 470 475 480
   Gln Ile Thr Thr Pro Tyr Ile Val Val Val Asn Gly His Ile Asp Pro
   485 490 495
   Asn Ser Lys Gly Asp Leu Ala Leu Arg Ser Thr Leu Tyr Gly Tyr Asn
   500 505 510
   Ser Asn Ile Ile Trp Arg Ser Met Ser Trp Asp Asn Glu Val Ala Phe
   515 520 525
   Asn Asn Gly Ser Gly Ser Gly Asp Gly Ile Asp Lys Pro Val Val Pro
   530 535 540
   Glu Gln Pro Asp Glu Pro Gly Glu Ile Glu Pro Ile Pro Glu
   545 550 555
<210> 95
   <211> 1677
   <212> DNA
   <213> Staphylococcus aureus
<400> 95
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc tgatagcgca agtaacgaaa gcaaaagtaa tgattcaagt 180
   agcgttagtg ctgcacctaa aacagacgac acaaacgtga gtgatactaa aacatcgtca 240
   aacactaata atggcgaaac gagtgtggcg caaaatctag cacaacagga aacgacacaa 300
   tcatcatcaa caaatgcaac tacggaagaa acgccggtaa ctggtgaagc tactactacg 360
   acaacgaatc aagctaatac accggcaaca actcaatcaa gcaatacaaa tgcggaggaa 420
   ttagtgaatc aaacaagtaa tgaaacgact tctaatgata ctaatacagt atcatctgta 480
   aattcacctc aaaattctac aaatgcggaa aatgtttcaa caacgcaaga tacttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggca 660
   gtagctgcag atgcaccggc agctggcaca gatattacga atcagttgac gaatgtgaca 720
   gttggtattg actctggtac gactgtgtat ccgcaccaag caggttatgt caaactgaat 780
   tatggttttt cagtgcctaa ttctgctgtt aaaggtgaca cattcaaaat aactgtacct 840
   aaagaattaa acttaaatgg tgtaacttca actgctaaag tgccaccaat tatggctgga 900
   gatcaagtat tggcaaatgg tgtaatcgat agtgatggta atgttattta tacatttaca 960
   gactatgtaa atactaaaga tgatgttaaa gcaactttga ccatgcccgc ttatattgac 1020
   cctgaaaatg ttacaaagac aggtaatgtg acattggcta ctggcatagg aaatacaaca 1080
   gcaaacaaaa cagtattagt agattatgaa aaatatggta agttttataa cttatctatt 1140
   aaaggtacaa ttgaccaaat cgataaaaca aataatacgt atcgtcagac aatttatgtc 1200
   aatccaagtg gagataacgt tattgcgccg gttttaacag gtaatttaaa accaaatacg 1260
   gatagtaatg cattaataga tcagcaaaat acaagtatta aagtatataa agtagataat 1320
   gcagctgatt tatctgaaag ttactttgtg aatccagaaa actttgagga tgtcactaat 1380
   agtgtgaata ttacattccc aaatccaaat caatataaag tagagtttaa tacgcctgat 1440
   gatcaaatta caacaccgta tatagtagtt gttaatggtc atattgatcc gaatagcaaa 1500
   ggtgatttag ctttacgttc aactttatat gggtataact cgaatataat ttggcgctct 1560
   atgtcatggg acaacgaagt agcatttaat aacggatcag gttctggtga cggtatcgat 1620
   aaacctgttg ttcctgaaca acctgatgag cctggtgaaa ttgaaccaat tccagag 1677
<210> 96
   <211> 559
   <212> PRT
   <213> Staphylococcus aureus
<400> 96
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Leu Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
   180 185 190
   Ser Thr Asp Ala Ser Asn Lys Asp Val Val Asn Gln Ala Val Asn Thr
   195 200 205
   Ser Ala Pro Arg Met Arg Ala Phe Ser Leu Ala Ala Val Ala Ala Asp
   210 215 220
   Ala Pro Ala Ala Gly Thr Asp Ile Thr Asn Gln Leu Thr Asn Val Thr
   225 230 235 240
   Val Gly Ile Asp Ser Gly Thr Thr Val Tyr Pro His Gln Ala Gly Tyr
   245 250 255
   Val Lys Leu Asn Tyr Gly Phe Ser Val Pro Asn Ser Ala Val Lys Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asp Pro Glu Asn Val Thr Lys Thr Gly Asn Val Thr Leu
   340 345 350
   Ala Thr Gly Ile Gly Asn Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Asp Ser Asn Ala Leu Ile Asp Gln Gln Asn Thr Ser
   420 425 430
   Ile Lys Val Tyr Lys Val Asp Asn Ala Ala Asp Leu Ser Glu Ser Tyr
           435                 440                 445
   Phe Val Asn Pro Glu Asn Phe Glu Asp Val Thr Asn Ser Val Asn Ile
       450                 455                 460
   Thr Phe Pro Asn Pro Asn Gln Tyr Lys Val Glu Phe Asn Thr Pro Asp
   465                 470                 475                 480
   Asp Gln Ile Thr Thr Pro Tyr Ile Val Val Val Asn Gly His Ile Asp
                   485                 490                 495
   Pro Asn Ser Lys Gly Asp Leu Ala Leu Arg Ser Thr Leu Tyr Gly Tyr
               500                 505                 510
   Asn Ser Asn Ile Ile Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 97
   <211> 1674
   <212> DNA
   <213> Staphylococcus aureus
<400> 97
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc tgatagcgca agtaacgaaa gcaaaagtaa tgattcaagt 180
   agcgttagtg ctgcacctaa aacagacgac acaaacgtga gtgatactaa aacatcgtca 240
   aacactaata atggcgaaac gagtgtggcg caaaatccag cacaacagga aacgacacaa 300
   tcagcattaa caaatgcaac tacggaagaa actccgttaa ctggtgaagc tactacggca 360
   acgaatcaag ctaatacacc ggcaacaact caatcaagca atacaaatgc ggaggaatta 420
   gtgaatcaaa caagtaatga aacgacttct aatgatacta atacagtatc atctgtaaat 480
   tcacctcaaa attctacaaa tgcggaaaat gtttcaacaa cgcaagatac ttcaactgaa 540
   gcaacacctt caaacaatga atcagctcca cagagtacag atgcaagtaa taaagatgta 600
   gttaatcaag cggttaatac aagtgcgcct agaatgagag catttagttt agcggcagta 660
   gctgcagatg caccggcagc tggcacagat attacgaatc agttgacaga tgtgaaagtt 720
   actattgact ctggtacgac tgtgtatccg caccaagcag gttatgtcaa actgaattat 780
   ggtttttcag tgcctaattc tgctgttaaa ggtgacacat tcaaaataac tgtacctaaa 840
   gaattaaact taaatggtgt aacttcaact gctaaagtgc ctccaattat ggccggagat 900
   caagtattgg caaatggtgt aatcgatagt gatggtaatg ttatttatac atttacagac 960
   tatgttgata ataaaaacga tgttaaagca acactaactg ttcctgcata cattgatcca 1020
   gaaaatgtaa cgaaaacagg taatgtaaca ttgacaactg gcataggaac caatactgct 1080
   agtaagacag ttttaatcga ctatgagaaa tatggacaat tccataattt atcaattaaa 1140
   ggtacaattg atcaaatcga caaaacaaac aatacgtatc gtcaaacgat ttatgtcaat 1200
   ccaagtggag acaatgttgt attaccagtg ttaactggta atctaattcc taagagtaat 1260
   agtaatgctt taatagatgc caacaatact aatattaaag tttataaagt ggataatgct 1320
   aatgatttat ctgaaagtta ttatgtgaat cctagcgatt ttgaagatgt aactaatcaa 1380
   gttagaattt catttccaaa tgctaatcaa tacaaagtag aatttcctac ggacgatgac 1440
   caaattacaa caccgtatat tgtagttgtt aatggccata ttgatcctgc tagtacaggt 1500
   gatttagcac tacgttcgac attttatggt tatgattcta attttatatg gagatctatg 1560
   tcatgggaca acgaagtagc atttaataac ggatcaggtt ctggtgacgg tatcgataaa 1620
   ccagttgttc ctgaacaacc tgatgagcct ggtgaaattg aaccaattcc agag 1674
<210> 98
   <211> 558
   <212> PRT
   <213> Staphylococcus aureus
<400> 98
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ala Leu Thr Asn Ala Thr Thr Glu Glu Thr Pro
               100                 105                 110
   Leu Thr Gly Glu Ala Thr Thr Ala Thr Asn Gln Ala Asn Thr Pro Ala
           115                 120                 125
   Thr Thr Gln Ser Ser Asn Thr Asn Ala Glu Glu Leu Val Asn Gln Thr
       130                 135                 140
   Ser Asn Glu Thr Thr Ser Asn Asp Thr Asn Thr Val Ser Ser Val Asn
   145                 150                 155                 160
   Ser Pro Gln Asn Ser Thr Asn Ala Glu Asn Val Ser Thr Thr Gln Asp
                   165                 170                 175
   Thr Ser Thr Glu Ala Thr Pro Ser Asn Asn Glu Ser Ala Pro Gln Ser
               180                 185                 190
   Thr Asp Ala Ser Asn Lys Asp Val Val Asn Gln Ala Val Asn Thr Ser
           195                 200                 205
   Ala Pro Arg Met Arg Ala Phe Ser Leu Ala Ala Val Ala Ala Asp Ala
       210                 215                 220
   Pro Ala Ala Gly Thr Asp Ile Thr Asn Gln Leu Thr Asp Val Lys Val
   225                 230                 235                 240
   Thr Ile Asp Ser Gly Thr Thr Val Tyr Pro His Gln Ala Gly Tyr Val
                   245                 250                 255
   Lys Leu Asn Tyr Gly Phe Ser Val Pro Asn Ser Ala Val Lys Gly Asp
               260                 265                 270
   Thr Phe Lys Ile Thr Val Pro Lys Glu Leu Asn Leu Asn Gly Val Thr
           275                 280                 285
   Ser Thr Ala Lys Val Pro Pro Ile Met Ala Gly Asp Gln Val Leu Ala
       290                 295                 300
   Asn Gly Val Ile Asp Ser Asp Gly Asn Val Ile Tyr Thr Phe Thr Asp
   305                 310                 315                 320
   Tyr Val Asp Asn Lys Asn Asp Val Lys Ala Thr Leu Thr Val Pro Ala
                   325                 330                 335
   Tyr Ile Asp Pro Glu Asn Val Thr Lys Thr Gly Asn Val Thr Leu Thr
               340                 345                 350
   Thr Gly Ile Gly Thr Asn Thr Ala Ser Lys Thr Val Leu Ile Asp Tyr
           355                 360                 365
   Glu Lys Tyr Gly Gln Phe His Asn Leu Ser Ile Lys Gly Thr Ile Asp
       370                 375                 380
   Gln Ile Asp Lys Thr Asn Asn Thr Tyr Arg Gln Thr Ile Tyr Val Asn
   385                 390                 395                 400
   Pro Ser Gly Asp Asn Val Val Leu Pro Val Leu Thr Gly Asn Leu Ile
                   405                 410                 415
   Pro Lys Ser Asn Ser Asn Ala Leu Ile Asp Ala Asn Asn Thr Asn Ile
               420                 425                 430
   Lys Val Tyr Lys Val Asp Asn Ala Asn Asp Leu Ser Glu Ser Tyr Tyr
           435                 440                 445
   Val Asn Pro Ser Asp Phe Glu Asp Val Thr Asn Gln Val Arg Ile Ser
       450                 455                 460
   Phe Pro Asn Ala Asn Gln Tyr Lys Val Glu Phe Pro Thr Asp Asp Asp
   465                 470                 475                 480
   Gln Ile Thr Thr Pro Tyr Ile Val Val Val Asn Gly His Ile Asp Pro
                   485                 490                 495
   Ala Ser Thr Gly Asp Leu Ala Leu Arg Ser Thr Phe Tyr Gly Tyr Asp
               500                 505                 510
   Ser Asn Phe Ile Trp Arg Ser Met Ser Trp Asp Asn Glu Val Ala Phe
           515                 520                 525
   Asn Asn Gly Ser Gly Ser Gly Asp Gly Ile Asp Lys Pro Val Val Pro
       530                 535                 540
   Glu Gln Pro Asp Glu Pro Gly Glu Ile Glu Pro Ile Pro Glu
   545                 550                 555
<210> 99
   <211> 1674
   <212> DNA
   <213> Staphylococcus aureus
<400> 99
   atgaatatga agaaaaaaga aaaacacgca attcggaaaa aatcgattgg cgtggcttca      60
   gtgcttgtag gtacgttaat cggttttgga ctactcagca gtaaagaagc agatgcaagt     120
   gaaaatagtg ttacgcaatc tgatagcgca agtaacgaaa gcaaaagtaa tgattcaagt     180
   agcgttagtg ctgcacctaa aacagacgac acaaacgtga gtgatactaa aacatcgtca     240
   aacactaata atggcgaaac gagtgtggcg caaaatccag cacaacaaga aacgacacaa     300
   tcagcattaa caaatgcaac tacggaagaa actccggtaa ctggtgaagc tactacggca     360
   acgaatcaag ctaatacacc ggcaacaact caatcaagca atacaaatgc ggaggaatta     420
   gtgaatcaaa caagtaatga aacgacttct aatgatacta atacagtatc atctgtaaat     480
   tcacctcaaa attctacaaa tgcggaaaat gtttcaacaa cgcaagatac ttcaactgaa     540
   gcaacacctt caaacaatga atcagctcca cagaatacag atgcaagtaa taaagatgta     600
   gttaatcaag cggttaatac aagtgcgcct agaaagagag catttagttt agcggctgta     660
   gctgcagatg caccggcagc tggcacagat attacgaatc agttgacaga tgtgaaagtt     720
   actattgact ctggtacgac tgtgtatccg caccaagcag gttatgtcaa actgaattat     780
   ggtttttcag tgcctaattc tgctgttaaa ggtgacacat tcaaaataac tgtacctaaa     840
   gaattaaact taaatggtgt aacttcaact gctaaagcgc caccaattat ggctggagat     900
   caagtattgg caaatggtgt aatcgatagt gatggtaatg ttatttatac atttacagac     960
   tatgttgata ataaagaaaa tgtaacagct aatattacta tgccagctta tattgaccct    1020
   gaaaatgtta caaagacagg taatgtgaca ttgacaactg gcataggaac caatactgct    1080
   agtaagacag tattaatcga ctatgagaaa tatggacaat tccataattt atcaattaaa    1140
   ggtacgattg atcaaatcga taaaacaaat aatacgtatc gccaaacaat ttatgtcaat    1200
   ccaagcggag ataacgttgt gttacctgcc ttaacaggta atttaattcc taatacaaag    1260
   agtaatgcgt taatagatgc aaaaaacact gatattaaag tttatagagt cgataatgct    1320
   aatgatttat ctgaaagtta ttatgtgaat cctagcgatt ttgaagatgt aactaatcaa    1380
   gttagaattt catttccaaa tgctaatcaa tacaaagtag aatttcctac ggacgatgac    1440
   caaattacaa caccgtatat tgtagttgtt aatggccata ttgatcctgc tagtacaggt    1500
   gatttagcac tacgttcgac attttatggt tatgattcta attttatatg gagatctatg    1560
   tcatgggaca acgaagtagc atttaataac ggatcaggtt ctggtgacgg tatcgataaa    1620
   ccagttgttc ctgaacaacc tgatgagcct ggtgaaattg aaccaattcc agag          1674
<210> 100
   <211> 558
   <212> PRT
   <213> Staphylococcus aureus
<400> 100
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ala Leu Thr Asn Ala Thr Thr Glu Glu Thr Pro
               100                 105                 110
   Val Thr Gly Glu Ala Thr Thr Ala Thr Asn Gln Ala Asn Thr Pro Ala
           115                 120                 125
   Thr Thr Gln Ser Ser Asn Thr Asn Ala Glu Glu Leu Val Asn Gln Thr
       130                 135                 140
   Ser Asn Glu Thr Thr Ser Asn Asp Thr Asn Thr Val Ser Ser Val Asn
   145                 150                 155                 160
   Ser Pro Gln Asn Ser Thr Asn Ala Glu Asn Val Ser Thr Thr Gln Asp
                   165                 170                 175
   Thr Ser Thr Glu Ala Thr Pro Ser Asn Asn Glu Ser Ala Pro Gln Asn
               180                 185                 190
   Thr Asp Ala Ser Asn Lys Asp Val Val Asn Gln Ala Val Asn Thr Ser
           195                 200                 205
   Ala Pro Arg Lys Arg Ala Phe Ser Leu Ala Ala Val Ala Ala Asp Ala
       210                 215                 220
   Pro Ala Ala Gly Thr Asp Ile Thr Asn Gln Leu Thr Asp Val Lys Val
   225                 230                 235                 240
   Thr Ile Asp Ser Gly Thr Thr Val Tyr Pro His Gln Ala Gly Tyr Val
                   245                 250                 255
   Lys Leu Asn Tyr Gly Phe Ser Val Pro Asn Ser Ala Val Lys Gly Asp
               260                 265                 270
   Thr Phe Lys Ile Thr Val Pro Lys Glu Leu Asn Leu Asn Gly Val Thr
   275 280 285
   Ser Thr Ala Lys Ala Pro Pro Ile Met Ala Gly Asp Gln Val Leu Ala
   290 295 300
   Asn Gly Val Ile Asp Ser Asp Gly Asn Val Ile Tyr Thr Phe Thr Asp
   305 310 315 320
   Tyr Val Asp Asn Lys Glu Asn Val Thr Ala Asn Ile Thr Met Pro Ala
   325 330 335
   Tyr Ile Asp Pro Glu Asn Val Thr Lys Thr Gly Asn Val Thr Leu Thr
   340 345 350
   Thr Gly Ile Gly Thr Asn Thr Ala Ser Lys Thr Val Leu Ile Asp Tyr
   355 360 365
   Glu Lys Tyr Gly Gln Phe His Asn Leu Ser Ile Lys Gly Thr Ile Asp
   370 375 380
   Gln Ile Asp Lys Thr Asn Asn Thr Tyr Arg Gln Thr Ile Tyr Val Asn
   385 390 395 400
   Pro Ser Gly Asp Asn Val Val Leu Pro Ala Leu Thr Gly Asn Leu Ile
   405 410 415
   Pro Asn Thr Lys Ser Asn Ala Leu Ile Asp Ala Lys Asn Thr Asp Ile
   420 425 430
   Lys Val Tyr Arg Val Asp Asn Ala Asn Asp Leu Ser Glu Ser Tyr Tyr
   435 440 445
   Val Asn Pro Ser Asp Phe Glu Asp Val Thr Asn Gln Val Arg Ile Ser
   450 455 460
   Phe Pro Asn Ala Asn Gln Tyr Lys Val Glu Phe Pro Thr Asp Asp Asp
   465 470 475 480
   Gln Ile Thr Thr Pro Tyr Ile Val Val Val Asn Gly His Ile Asp Pro
   485 490 495
   Ala Ser Thr Gly Asp Leu Ala Leu Arg Ser Thr Phe Tyr Gly Tyr Asp
   500 505 510
   Ser Asn Phe Ile Trp Arg Ser Met Ser Trp Asp Asn Glu Val Ala Phe
   515 520 525
   Asn Asn Gly Ser Gly Ser Gly Asp Gly Ile Asp Lys Pro Val Val Pro
   530 535 540
   Glu Gln Pro Asp Glu Pro Gly Glu Ile Glu Pro Ile Pro Glu
   545 550 555
<210> 101
   <211> 1677
   <212> DNA
   <213> Staphylococcus aureus
<400> 101
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc tgatagcgca agtaacgaaa gcaaaagtaa tgattcaagt 180
   agcgttagtg ctgcacctaa aacagacgac acaaacgtga gtgatactaa aacatcgtca 240
   aacactaata atggcgaaac gagtgtggcg caaaatccag cacaacagga aacgacacaa 300
   tcatcatcaa caaatgcaac tacggaagaa acgccggtaa ctggtgaagc tactactacg 360
   acaacgaatc aagctaatac accggcaaca actcaatcaa gcaatacaaa tgcggaggaa 420
   ttagtgaatc aaacaagtaa tgaaacgact tctaatgata ctaatacagt atcatctgta 480
   aattcacctc aaaattctac aaatgcggaa aatgtttcaa caacgcaaga tacttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggca 660
   gtagctgcag atgcaccggc agctggcaca gatattacga atcagttgac gaatgtgaca 720
   gttggtattg actctggaga tacagtttat ccgcaccaag caggctatgt caaactgaat 780
   tatggttttt cagtgcctaa ttctgctgtt aaaggtgaca cattcaaaat aactgtacct 840
   aaagaattaa acttaaatgg tgtaacttca actgctaaag tgccaccaat tatggctgga 900
   gatcaagtat tggcaaatgg tgtaatcgat agtgatggta atgttattta tacatttaca 960
   gactatgtaa atactaaaga tgatgttaaa gcaactttga ccatgcccgc ttatattgac 1020
   cctgaaaatg ttacaaagac aggtaatgtg acattggcta ctggcatagg aaatacaaca 1080
   gcaaacaaaa cagtattagt agattatgaa aaatatggta agttttataa cttatctatt 1140
   aaaggtacaa ttgaccaaat cgataaaaca aataatacgt atcgtcagac aatttatgtc 1200
   aatccaagtg gagataacgt tattgcgccg gttttaacag gtaatttaaa accaaatacg 1260
   gatagtaatg cattaataga tcagcaaaat acaagtatta aagtatataa agtagataat 1320
   gcagctgatt tatctgaaag ttactttgtg aatccagaaa actttgagga tgtcactaat 1380
   agtgtgaata ttacattccc aaatccaaat caatataaag tagagtttaa tacgcctgat 1440
   gatcaaatta caacaccgta tatagtagtt gttaatggtc atattgatcc gaatagcaaa 1500
   ggtgatttag ctttacgttc aactttatat gggtatgact caaggtttgt atggagatct 1560
   atgtcatggg acaacgaagt agcatttaat aacggatcag gttctggtga cggtatcgat 1620
   aaaccagttg ttcctgaaca acctgatgag cctggtgaaa ttgaaccaat tccagag 1677
<210> 102
   <211> 559
   <212> PRT
   <213> Staphylococcus aureus
<400> 102
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
   180 185 190
   Ser Thr Asp Ala Ser Asn Lys Asp Val Val Asn Gln Ala Val Asn Thr
   195 200 205
   Ser Ala Pro Arg Met Arg Ala Phe Ser Leu Ala Ala Val Ala Ala Asp
   210 215 220
   Ala Pro Ala Ala Gly Thr Asp Ile Thr Asn Gln Leu Thr Asn Val Thr
   225 230 235 240
   Val Gly Ile Asp Ser Gly Asp Thr Val Tyr Pro His Gln Ala Gly Tyr
   245 250 255
   Val Lys Leu Asn Tyr Gly Phe Ser Val Pro Asn Ser Ala Val Lys Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asp Pro Glu Asn Val Thr Lys Thr Gly Asn Val Thr Leu
   340 345 350
   Ala Thr Gly Ile Gly Asn Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Asp Ser Asn Ala Leu Ile Asp Gln Gln Asn Thr Ser
   420 425 430
   Ile Lys Val Tyr Lys Val Asp Asn Ala Ala Asp Leu Ser Glu Ser Tyr
               435                 440                 445
   Phe Val Asn Pro Glu Asn Phe Glu Asp Val Thr Asn Ser Val Asn Ile
       450                 455                 460
   Thr Phe Pro Asn Pro Asn Gln Tyr Lys Val Glu Phe Asn Thr Pro Asp
   465                 470                 475                 480
   Asp Gln Ile Thr Thr Pro Tyr Ile Val Val Val Asn Gly His Ile Asp
                   485                 490                 495
   Pro Asn Ser Lys Gly Asp Leu Ala Leu Arg Ser Thr Leu Tyr Gly Tyr
               500                 505                 510
   Asp Ser Arg Phe Val Trp Arg Ser Met Ser Trp Asp Asn Glu Val Ala
           515                 520                 525
   Phe Asn Asn Gly Ser Gly Ser Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 103
   <211> 1677
   <212> DNA
   <213> Staphylococcus aureus
<400> 103
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc cgatagcgca agtaacgaaa gcaaaagtaa tgattcaagt 180
   agcgttagtg ctgcacctaa aacagtcgac acaaacgtga gtgatactaa aacaacgtca 240
   aacactaata gtggcgaaac aagtgtggcg caaaatccgg cacaacagga aacgacacaa 300
   tcagcatcaa caaatgcaac tacggaagaa actccggcaa ctggtgaagc tactactacg 360
   gcaacgaatc aagctaatac accggcaaca actcaatcaa gcaatacaaa tgcggaggaa 420
   ttagtgaatc aaacaaataa tgaaacgact tctaatgata ctaatacagt atcatctgta 480
   aattcacctc aaaattctac aaatgcggaa aatgtttcaa caacgcaaga tacttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggca 660
   gtagctgcag atgcaccggc tgctggcaca gatattacga atcagttgac agatgtgaaa 720
   gttactattg actctggtac gactgtgtat ccgcaccaag caggttatgt caaactgaat 780
   tatggttttt cagtccctaa ttctgctgtt aaaggtgaca cattcaaaat aactgtacct 840
   aaagaattaa acttaaatgg tgtaacttca actgctaagg tgccaccaat tatggccgga 900
   gatcaagtat tggcaaatgg tgtaatcgat agtgatggta atgttattta tacatttaca 960
   gactatgttg ataataaaga aaatgtaaca gctaatatta ctatgccagc ttatattgac 1020
   cctgaaaatg ttacaaagac aggtaatgtg acattgacaa ctggcatagg aaccaatact 1080
   gctagtaaga cagtattaat cgactatgag aaatatggac aattccataa tttatcaatt 1140
   aaaggtacga ttgatcaaat cgataaaaca aataatacgt atcgccaaac aatttatgtc 1200
   aatccaagcg gagataacgt tgtgttacct gccttaacag gtaatttaat tcctaataca 1260
   aagagtaatg cgttaataga tgcaaaaaac actgatatta aagtttatag agtcgataat 1320
   gctaatgatt tatctgaaag ttattatgtg aatcctagcg attttgaaga tgtaactaat 1380
   caagttagaa tttcatttcc aaatgctaat caatacaaag tagaatttcc tacggacgat 1440
   gaccaaatta caacaccgta tattgtagtt gttaatggcc atattgatcc tgctagtaca 1500
   ggtgatttag cactacgttc gacattttat ggttatgatt ctaattttat atggagatct 1560
   atgtcatggg acaacgaagt agcatttaat aacggatcag gctctggtga cggtatcgat 1620
   aaaccagttg ttcctgaaca acctgatgag cctggtgaaa ttgaaccaat tccagag 1677
<210> 104
   <211> 559
   <212> PRT
   <213> Staphylococcus aureus
<400> 104
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Val Asp Thr Asn Val Ser Asp Thr Lys Thr Thr Ser
   65                  70                  75                  80
   Asn Thr Asn Ser Gly Glu Thr Ser Val Ala Gln Asn Pro Ala Gln Gln
                   85                  90                  95
   Glu Thr Thr Gln Ser Ala Ser Thr Asn Ala Thr Thr Glu Glu Thr Pro
               100                 105                 110
   Ala Thr Gly Glu Ala Thr Thr Thr Ala Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Asn Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Ser Thr Asp Ala Ser Asn Lys Asp Val Val Asn Gln Ala Val Asn Thr
           195                 200                 205
   Ser Ala Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asp Val Lys
   225                 230                 235                 240
   Val Thr Ile Asp Ser Gly Thr Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
           275                 280                 285
   Thr Ser Thr Ala Lys Val Pro Pro Ile Met Ala Gly Asp Gln Val Leu
       290                 295                 300
   Ala Asn Gly Val Ile Asp Ser Asp Gly Asn Val Ile Tyr Thr Phe Thr
   305                 310                 315                 320
   Asp Tyr Val Asp Asn Lys Glu Asn Val Thr Ala Asn Ile Thr Met Pro
                   325                 330                 335
   Ala Tyr Ile Asp Pro Glu Asn Val Thr Lys Thr Gly Asn Val Thr Leu
               340                 345                 350
   Thr Thr Gly Ile Gly Thr Asn Thr Ala Ser Lys Thr Val Leu Ile Asp
   355 360 365
   Tyr Glu Lys Tyr Gly Gln Phe His Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Val Leu Pro Ala Leu Thr Gly Asn Leu
   405 410 415
   Ile Pro Asn Thr Lys Ser Asn Ala Leu Ile Asp Ala Lys Asn Thr Asp
   420 425 430
   Ile Lys Val Tyr Arg Val Asp Asn Ala Asn Asp Leu Ser Glu Ser Tyr
   435 440 445
   Tyr Val Asn Pro Ser Asp Phe Glu Asp Val Thr Asn Gln Val Arg Ile
   450 455 460
   Ser Phe Pro Asn Ala Asn Gln Tyr Lys Val Glu Phe Pro Thr Asp Asp
   465 470 475 480
   Asp Gln Ile Thr Thr Pro Tyr Ile Val Val Val Asn Gly His Ile Asp
   485 490 495
   Pro Ala Ser Thr Gly Asp Leu Ala Leu Arg Ser Thr Phe Tyr Gly Tyr
   500 505 510
   Asp Ser Asn Phe Ile Trp Arg Ser Met Ser Trp Asp Asn Glu Val Ala
   515 520 525
   Phe Asn Asn Gly Ser Gly Ser Gly Asp Gly Ile Asp Lys Pro Val Val
   530 535 540
   Pro Glu Gln Pro Asp Glu Pro Gly Glu Ile Glu Pro Ile Pro Glu
   545 550 555
<210> 105
   <211> 1677
   <212> DNA
   <213> Staphylococcus aureus
<400> 105
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc cgatagcgca agtaacgaaa gcaaaagtaa taatccaagt 180
   agcgttaacg ctgcacctaa aacagacaac acaaacgtga gtgatactaa aacaacgtca 240
   aacactaata atagcgaaac gagtgtggca caaaatccag cacaacagga aacgacacaa 300
   tcagcatcaa caaatgcaac tacggaagaa acaccggtaa ctggtgaagg tactactacg 360
   gcaacgaatc aagctaatac accggcagca actcaatcaa acaatacaaa tgcggaggaa 420
   ttagtgaatc aaacaagtaa tgaaacgact tctaatgata ctaatacagt atcatctgta 480
   aattcacctc aaaattctac aaatgcggaa aatgtttcaa caacgcaaga cacttcaact 540
   gaagcaacac cttcaaacaa tgaatcagct ccacagagta cagatgcaag taataaagat 600
   gtagttagtc aagccgttaa tccaagtgcg cctagaatga gagcatttag tttagcggct 660
   gtagctgcag atgcaccggc tgctggcaca gatattacga atcagttgac agatgttact 720
   gttggtattg aatctggaga tacagtttat ccgcaccaag caggttatgt caaactgaat 780
   tatggttttt ccgtgcctaa ttctgctgtt aaaggtgaca cattcaaaat aactgtacct 840
   aaagaattaa acttaaatgg tgtaacttca actgctaaag ttcctccaat tatggctgga 900
   gatcaagtat tagctaatgg tgtaatcgat agtgatggta atgttattta tacatttaca 960
   gactatgtaa atactaaaga tgatgttaaa gcaacactaa ctgtgcctgc atacattgac 1020
   cctgaatatg ttacacatac tggtaatgtg acattggcta ctggcatagg aaatacaaca 1080
   gcaaacaaaa cagtattagt agattatgaa aaatatggta agttttataa cttatctatt 1140
   aaaggtacaa ttgaccaagt cgataaaaca aataatacgt atcgtcagac aatttatgtc 1200
   aatccaagtg gagataacgt tattgcaccg gttttaacag gtaatttaaa accaaatacg 1260
   gatagtaatg cattaataga tcagcaaaat acaagtatta aagtatataa agtagataat 1320
   gcagctgatt tatctgaaag ttactttgtg aatccagaaa actttgagga tgtcactaat 1380
   agtgtaaata ttacattccc aaatccaaat caatataaag tagagtttaa tacgcctgat 1440
   gatcaaatta caacaccgta tattgtagtt gttaatggtc atattgatcc gaatagcaaa 1500
   ggtgatttag ctttacgttc aactttatat ggatataact caaatataat ttggcgctct 1560
   atgtcatggg acaacgaagt agcatttaat aacggatcag gttctggtga cggtatcgat 1620
   aaacctgttg ttcctgaaca acctgatgag ccgggtgaaa ttgaaccaat tccagag 1677
<210> 106
   <211> 559
   <212> PRT
   <213> Staphylococcus aureus
<400> 106
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asn Pro Ser Ser Val Asn Ala
       50                  55                  60
   Ala Pro Lys Thr Asp Asn Thr Asn Val Ser Asp Thr Lys Thr Thr Ser
   65                  70                  75                  80
   Asn Thr Asn Asn Ser Glu Thr Ser Val Ala Gln Asn Pro Ala Gln Gln
                   85                  90                  95
   Glu Thr Thr Gln Ser Ala Ser Thr Asn Ala Thr Thr Glu Glu Thr Pro
               100                 105                 110
   Val Thr Gly Glu Gly Thr Thr Thr Ala Thr Asn Gln Ala Asn Thr Pro
           115                 120                 125
   Ala Ala Thr Gln Ser Asn Asn Thr Asn Ala Glu Glu Leu Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Ser Thr Asp Ala Ser Asn Lys Asp Val Val Ser Gln Ala Val Asn Pro
           195                 200                 205
   Ser Ala Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asp Val Thr
   225                 230                 235                 240
   Val Gly Ile Glu Ser Gly Asp Thr Val Tyr Pro His Gln Ala Gly Tyr
                   245                 250                 255
   Val Lys Leu Asn Tyr Gly Phe Ser Val Pro Asn Ser Ala Val Lys Gly
               260                 265                 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Val Pro
   325 330 335
   Ala Tyr Ile Asp Pro Glu Tyr Val Thr His Thr Gly Asn Val Thr Leu
   340 345 350
   Ala Thr Gly Ile Gly Asn Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Val Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Asp Ser Asn Ala Leu Ile Asp Gln Gln Asn Thr Ser
   420 425 430
   Ile Lys Val Tyr Lys Val Asp Asn Ala Ala Asp Leu Ser Glu Ser Tyr
   435 440 445
   Phe Val Asn Pro Glu Asn Phe Glu Asp Val Thr Asn Ser Val Asn Ile
   450 455 460
   Thr Phe Pro Asn Pro Asn Gln Tyr Lys Val Glu Phe Asn Thr Pro Asp
   465 470 475 480
   Asp Gln Ile Thr Thr Pro Tyr Ile Val Val Val Asn Gly His Ile Asp
   485 490 495
   Pro Asn Ser Lys Gly Asp Leu Ala Leu Arg Ser Thr Leu Tyr Gly Tyr
   500 505 510
   Asn Ser Asn Ile Ile Trp Arg Ser Met Ser Trp Asp Asn Glu Val Ala
   515 520 525
   Phe Asn Asn Gly Ser Gly Ser Gly Asp Gly Ile Asp Lys Pro Val Val
   530 535 540
   Pro Glu Gln Pro Asp Glu Pro Gly Glu Ile Glu Pro Ile Pro Glu
   545 550 555
<210> 107
   <211> 1677
   <212> DNA
   <213> Staphylococcus aureus
<400> 107
   atgaatatga agaaacaaga aaaacacgca attcgtaaaa aatcgattgg cgtggcttca 60
   gtgcttgtag gtacgttaat cggttttgga ctactcagca gtaaagaagc agatgcaagt 120
   gaaaatagta tgacacaaac ggaaaatacg agtaatgaga gcaaaagtaa tgatccaagt 180
   agcgttaatg ctgcacctaa aacagacaac acaaacgtga gtgattctaa tacaacgaca 240
   aacactaata gtgacgaaac gaatgtagcg caaaatccag cacaacagga aacgacacaa 300
   tcagcatcaa caaatgcaac tacagaagaa acaccggtaa ctggtgaagt tactactacg 360
   gcaacgaatc aagctaatac accggcaaca actcaatcaa gcaatacaaa tgcggaagaa 420
   tcagtgaatc aaacaagtaa tgaaacgact tctaatgata ctaatacagt atcatctgta 480
   aattcacctc aaaattctac aaatgcggaa aatgtttcaa caacgcaaga catttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggct 660
   gtagctgcag atgcaccggc tgctggcaaa gatattacga atcagttgac gaatgtgaca 720
   gttggtattg actctggaga tacagtttat ccgcaccaag caggctatgt caaactgaat 780
   tatgggttct cagtaccaaa tgaggctgtt caaggtgaca cattcaaaat aactgtgccc 840
   aaagaattaa acttaaatgg tgtaacttca actgctaaag tgccaccaat tatggccgga 900
   gatcaagtat tggcaaatgg tgtaatcgat agtgatggta atgttattta tacatttaca 960
   gactatgtaa atactaaaga tgatgttaaa gcaactttga ccatgcccgc ttatattgac 1020
   cctgaaaatg ttacaaagac aggtaatgtg acattggcta ctggcatagg tagtacaaca 1080
   gcaaacaaaa cagtattagt agattatgaa aaatatggta agttttataa cttatctatt 1140
   aaaggtacaa ttgaccaaat cgataaaaca aataatacgt atcgtcagac aatttatgtc 1200
   aatccaagtg gagataacgt tattgcgccg gttttaacag gtaatttaaa accaaatacg 1260
   gatagtaatg cattaataga tgcacaaaat actagtatta aagtatataa agttgataat 1320
   gcatcagact tgtctgaaag ttattatgtg aatccagata actttgaaga tgtcactgat 1380
   agtgtgaata ttacattccc aaatccaaat caatataaag tagagttcaa tacgcctgat 1440
   gatcaaataa caacaccata tattgtagtt gttaatgggc atattgatcc taatagtaaa 1500
   ggtgatttag ctttacgttc aactttatat ggatataatt cgaatataat ttggcgatca 1560
   atgtcatggg ataatgaagt agcatttaat aacggatcag gttttggtga cggtatcgat 1620
   aaacctgttg ttcctgaaca acctgatgag ccgggtgaaa ttgaaccaat tccagag 1677
<210> 108
   <211> 559
   <212> PRT
   <213> Staphylococcus aureus
<400> 108
   Met Asn Met Lys Lys Gln Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Met Thr Gln Thr Glu
           35                  40                  45
   Asn Thr Ser Asn Glu Ser Lys Ser Asn Asp Pro Ser Ser Val Asn Ala
       50                  55                  60
   Ala Pro Lys Thr Asp Asn Thr Asn Val Ser Asp Ser Asn Thr Thr Thr
   65                  70                  75                  80
   Asn Thr Asn Ser Asp Glu Thr Asn Val Ala Gln Asn Pro Ala Gln Gln
                   85                  90                  95
   Glu Thr Thr Gln Ser Ala Ser Thr Asn Ala Thr Thr Glu Glu Thr Pro
               100                 105                 110
   Val Thr Gly Glu Val Thr Thr Thr Ala Thr Asn Gln Ala Asn Thr Pro
           115                 120                 125
   Ala Thr Thr Gln Ser Ser Asn Thr Asn Ala Glu Glu Ser Val Asn Gln
       130                 135                 140
   Thr Ser Asn Glu Thr Thr Ser Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Ile Ser Thr Glu Ala Thr Pro Ser Asn Asn Glu Ser Ala Pro Gln
   180 185 190
   Ser Thr Asp Ala Ser Asn Lys Asp Val Val Asn Gln Ala Val Asn Thr
   195 200 205
   Ser Ala Pro Arg Met Arg Ala Phe Ser Leu Ala Ala Val Ala Ala Asp
   210 215 220
   Ala Pro Ala Ala Gly Lys Asp Ile Thr Asn Gln Leu Thr Asn Val Thr
   225 230 235 240
   Val Gly Ile Asp Ser Gly Asp Thr Val Tyr Pro His Gln Ala Gly Tyr
   245 250 255
   Val Lys Leu Asn Tyr Gly Phe Ser Val Pro Asn Glu Ala Val Gln Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asp Pro Glu Asn Val Thr Lys Thr Gly Asn Val Thr Leu
   340 345 350
   Ala Thr Gly Ile Gly Ser Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Asp Ser Asn Ala Leu Ile Asp Ala Gln Asn Thr Ser
   420 425 430
   Ile Lys Val Tyr Lys Val Asp Asn Ala Ser Asp Leu Ser Glu Ser Tyr
           435                 440                 445
   Tyr Val Asn Pro Asp Asn Phe Glu Asp Val Thr Asp Ser Val Asn Ile
       450                 455                 460
   Thr Phe Pro Asn Pro Asn Gln Tyr Lys Val Glu Phe Asn Thr Pro Asp
   465                 470                 475                 480
   Asp Gln Ile Thr Thr Pro Tyr Ile Val Val Val Asn Gly His Ile Asp
                   485                 490                 495
   Pro Asn Ser Lys Gly Asp Leu Ala Leu Arg Ser Thr Leu Tyr Gly Tyr
               500                 505                 510
   Asn Ser Asn Ile Ile Trp Arg Ser Met Ser Trp Asp Asn Glu Val Ala
           515                 520                 525
   Phe Asn Asn Gly Ser Gly Phe Gly Asp Gly Ile Asp Lys Pro Val Val
       530                 535                 540
   Pro Glu Gln Pro Asp Glu Pro Gly Glu Ile Glu Pro Ile Pro Glu
   545                 550                 555
<210> 109
   <211> 29
   <212> DNA
   <213> Artificial Sequence
<220>
   <223> Description of Artificial Sequence: Synthetic primer
<400> 109
   cacaaaattt acgaatagaa agaaacgag 29
<210> 110
   <211> 31
   <212> DNA
   <213> Artificial Sequence
<220>
   <223> Description of Artificial Sequence: Synthetic primer
<400> 110
   aaaatattgg agataccaat attttaggtt g 31
<210> 111
   <211> 37
   <212> DNA
   <213> Artificial Sequence
<220>
   <223> Description of Artificial Sequence: Synthetic primer
<400> 111
   tttcttggat ccggtactgg tggtaaacaa agcagtg 37
<210> 112
   <211> 39
   <212> DNA
   <213> Artificial Sequence
<220>
   <223> Description of Artificial Sequence: Synthetic primer
<400> 112
   tttcttgcat gcttatttca tgcttccgtg tacagtttc 39
<210> 113
   <211> 34
   <212> DNA
   <213> Artificial Sequence
<220>
   <223> Description of Artificial Sequence: Synthetic primer
<400> 113
   tttcttccat gggtactggt ggtaaacaaa gcag 34
<210> 114
   <211> 37
   <212> DNA
   <213> Artificial Sequence
<220>
   <223> Description of Artificial Sequence: Synthetic primer
<400> 114
   tttcttgctc agcattattt catgcttccg tgtacag 37
<210> 115
   <211> 309
   <212> PRT
   <213> Staphylococcus epidermidis
<400> 115
   Val Lys Lys Ile Leu Ala Leu Ala Ile Ala Phe Leu Ile Ile Leu Ala
   1               5                   10                  15
   Ala Cys Gly Asn His Ser Asn His Glu His His Ser His Glu Gly Lys
               20                  25                  30
   Leu Lys Val Val Thr Thr Asn Ser Ile Leu Tyr Asp Met Val Lys Arg
           35                  40                  45
   Val Gly Gly Asn Lys Val Asp Val His Ser Ile Val Pro Val Gly Gln
       50                  55                  60
   Asp Pro His Glu Tyr Glu Val Lys Pro Lys Asp Ile Lys Ala Leu Thr
   65 70 75 80
   Asp Ala Asp Val Val Phe Tyr Asn Gly Leu Asn Leu Glu Thr Gly Asn
   85 90 95
   Gly Trp Phe Glu Lys Ala Leu Asp Gln Ala Gly Lys Ser Thr Lys Asp
   100 105 110
   Lys Asn Val Ile Ala Ala Ser Asn Asn Val Lys Pro Ile Tyr Leu Asn
   115 120 125
   Gly Glu Glu Gly Asn Lys Asn Lys Gln Asp Pro His Ala Trp Leu Ser
   130 135 140
   Leu Glu Asn Gly Ile Lys Tyr Val Lys Thr Ile Gln Lys Ser Leu Glu
   145 150 155 160
   His His Asp Lys Lys Asp Lys Ser Thr Tyr Glu Lys Gln Gly Asn Ala
   165 170 175
   Tyr Ile Ser Lys Leu Glu Glu Leu Asn Lys Asp Ser Lys Asn Lys Phe
   180 185 190
   Asp Asp Ile Pro Lys Asn Gln Arg Ala Met Met Thr Ser Glu Gly Ala
   195 200 205
   Phe Lys Tyr Phe Ala Gln Gln Phe Asp Val Lys Pro Gly Tyr Ile Trp
   210 215 220
   Glu Ile Asn Thr Glu Lys Gln Gly Thr Pro Gly Gln Met Lys Gln Ala
   225 230 235 240
   Ile Lys Phe Val Lys Asp Asn His Leu Lys His Leu Leu Val Glu Thr
   245 250 255
   Ser Val Asp Lys Lys Ala Met Gln Ser Leu Ser Glu Glu Thr Lys Lys
   260 265 270
   Asp Ile Tyr Gly Glu Val Phe Thr Asp Ser Ile Gly Lys Ala Gly Thr
   275 280 285
   Lys Gly Asp Ser Tyr Tyr Lys Met Met Lys Ser Asn Ile Asp Thr Ile
   290 295 300
   His Gly Ser Met Lys
   305
<210> 116
   <211> 930
   <212> DNA
   <213> Staphylococcus epidermidis
<400> 116
   gtgaaaaaaa ttctcgcttt agcaatagca tttttaatta tccttgccgc atgtgggaat        60
   cacagtaacc atgaacatca ctcacatgaa ggaaaattaa aagttgtaac tacaaactct       120
   attctctatg acatggttaa acgtgtcggt ggaaataagg tcgatgttca tagcatcgtt       180
   ccagtaggac aagatccaca tgaatatgag gttaaaccta aagatattaa agcattaaca       240
   gatgctgacg ttgtatttta taatggttta aacctagaaa ctggaaatgg ttggtttgaa       300
   aaagcacttg accaagcagg aaaatcaaca aaagataaaa atgtgatagc agcatcaaat       360
   aatgttaaac caatatactt aaatggtgag gaaggtaaca aaaacaaaca agatccacat       420
   gcatggttaa gtttagagaa tggaattaaa tacgtaaaaa caatacaaaa atcactagaa       480
   catcatgata aaaaagataa gtctacatat gaaaaacaag ggaatgcata tatatcaaaa       540
   ttagaagaac ttaataaaga tagtaaaaat aaatttgatg acatacccaa aaatcaacgt       600
   gccatgatga caagtgaagg tgcatttaaa tattttgctc aacaattcga tgttaaacca       660
   ggttatattt gggagataaa cacagaaaaa caaggtacac ctggtcaaat gaaacaagcc       720
   attaaatttg ttaaagataa tcatttaaaa catttattag tcgaaacaag cgtagataaa       780
   aaagctatgc aaagtttatc agaagaaact aagaaagata tttatggtga agtatttacc       840
   gactctatag gtaaggcagg tactaaaggt gactcatact ataaaatgat gaaatctaat       900
   attgatacaa tacatggtag tatgaaataa                                        930
<210> 117
   <211> 510
   <212> PRT
   <213> Staphylococcus aureus
<400> 117
   Met Ala Ser Met Thr Gly Gly Gln Gln Met Gly Ala Ser Glu Gln Ser
   1               5                   10                  15
   Asn Asp Thr Thr Gln Ser Ser Lys Asn Asn Ala Ser Ala Asp Ser Glu
               20                  25                  30
   Lys Asn Asn Met Ile Glu Thr Pro Gln Leu Asn Thr Thr Ala Asn Asp
           35                  40                  45
   Thr Ser Asp Ile Ser Ala Asn Thr Asn Ser Ala Asn Val Asp Ser Thr
       50                  55                  60
   Thr Lys Pro Met Ser Thr Gln Thr Ser Asn Thr Thr Thr Thr Glu Pro
   65                  70                  75                  80
   Ala Ser Thr Asn Glu Thr Pro Gln Pro Thr Ala Ile Lys Asn Gln Ala
                   85                  90                  95
   Thr Ala Ala Lys Met Gln Asp Gln Thr Val Pro Gln Glu Ala Asn Ser
               100                 105                 110
   Gln Val Asp Asn Lys Thr Thr Asn Asp Ala Asn Ser Ile Ala Thr Asn
           115                 120                 125
   Ser Glu Leu Lys Asn Ser Gln Thr Leu Asp Leu Pro Gln Ser Ser Pro
       130                 135                 140
   Gln Thr Ile Ser Asn Ala Gln Gly Thr Ser Lys Pro Ser Val Arg Thr
   145                 150                 155                 160
   Arg Ala Val Arg Ser Leu Ala Val Ala Glu Pro Val Val Asn Ala Ala
                   165                 170                 175
   Asp Ala Lys Gly Thr Asn Val Asn Asp Lys Val Thr Ala Ser Asn Phe
               180                 185                 190
   Lys Leu Glu Lys Thr Thr Phe Asp Pro Asn Gln Ser Gly Asn Thr Phe
           195                 200                 205
   Met Ala Ala Asn Phe Thr Val Thr Asp Lys Val Lys Ser Gly Asp Tyr
       210                 215                 220
   Phe Thr Ala Lys Leu Pro Asp Ser Leu Thr Gly Asn Gly Asp Val Asp
   225                 230                 235                 240
   Tyr Ser Asn Ser Asn Asn Thr Met Pro Ile Ala Asp Ile Lys Ser Thr
                   245                 250                 255
   Asn Gly Asp Val Val Ala Lys Ala Thr Tyr Asp Ile Leu Thr Lys Thr
               260                 265                 270
   Tyr Thr Phe Val Phe Thr Asp Tyr Val Asn Asn Lys Glu Asn Ile Asn
           275                 280                 285
   Gly Gln Phe Ser Leu Pro Leu Phe Thr Asp Arg Ala Lys Ala Pro Lys
       290                 295                 300
   Ser Gly Thr Tyr Asp Ala Asn Ile Asn Ile Ala Asp Glu Met Phe Asn
   305                 310                 315                 320
   Asn Lys Ile Thr Tyr Asn Tyr Ser Ser Pro Ile Ala Gly Ile Asp Lys
                   325                 330                 335
   Pro Asn Gly Ala Asn Ile Ser Ser Gln Ile Ile Gly Val Asp Thr Ala
               340                 345                 350
   Ser Gly Gln Asn Thr Tyr Lys Gln Thr Val Phe Val Asn Pro Lys Gln
           355                 360                 365
   Arg Val Leu Gly Asn Thr Trp Val Tyr Ile Lys Gly Tyr Gln Asp Lys
       370                 375                 380
   Ile Glu Glu Ser Ser Gly Lys Val Ser Ala Thr Asp Thr Lys Leu Arg
   385                 390                 395                 400
   Ile Phe Glu Val Asn Asp Thr Ser Lys Leu Ser Asp Ser Tyr Tyr Ala
                   405                 410                 415
   Asp Pro Asn Asp Ser Asn Leu Lys Glu Val Thr Asp Gln Phe Lys Asn
               420                 425                 430
   Arg Ile Tyr Tyr Glu His Pro Asn Val Ala Ser Ile Lys Phe Gly Asp
           435                 440                 445
   Ile Thr Lys Thr Tyr Val Val Leu Val Glu Gly His Tyr Asp Asn Thr
       450                 455                 460
   Gly Lys Asn Leu Lys Thr Gln Val Ile Gln Glu Asn Val Asp Pro Val
   465                 470                 475                 480
   Thr Asn Arg Asp Tyr Ser Ile Phe Gly Trp Asn Asn Glu Asn Val Val
                   485                 490                 495
   Arg Tyr Gly Gly Gly Ser Ala Asp Gly Asp Ser Ala Val Asn
               500                 505                 510
<210> 118
   <211> 1533
   <212> DNA
   <213> Staphylococcus aureus
<400> 118
   atggctagca tgactggtgg acagcaaatg ggtgcttcag aacaatcgaa cgatacaacg      60
   caatcttcga aaaataatgc aagtgcagat tccgaaaaaa acaatatgat agaaacacct     120
   caattaaata caacggctaa tgatacatct gatattagtg caaacacaaa cagtgcgaat     180
   gtagatagca caacaaaacc aatgtctaca caaacgagca ataccactac aacagagcca     240
   gcttcaacaa atgaaacacc tcaaccgacg gcaattaaaa atcaagcaac tgctgcaaaa      300
   atgcaagatc aaactgttcc tcaagaagca aattctcaag tagataataa aacaacgaat      360
   gatgctaata gcatagcaac aaacagtgag cttaaaaatt ctcaaacatt agatttacca      420
   caatcatcac cacaaacgat ttccaatgcg caaggaacta gtaaaccaag tgttagaacg      480
   agagctgtac gtagtttagc tgttgctgaa ccggtagtaa atgctgctga tgctaaaggt      540
   acaaatgtaa atgataaagt tacggcaagt aatttcaagt tagaaaagac tacatttgac      600
   cctaatcaaa gtggtaacac atttatggcg gcaaatttta cagtgacaga taaagtgaaa      660
   tcaggggatt attttacagc gaagttacca gatagtttaa ctggtaatgg agacgtggat      720
   tattctaatt caaataatac gatgccaatt gcagacatta aaagtacgaa tggcgatgtt      780
   gtagctaaag caacatatga tatcttgact aagacgtata catttgtctt tacagattat      840
   gtaaataata aagaaaatat taacggacaa ttttcattac ctttatttac agaccgagca      900
   aaggcaccta aatcaggaac atatgatgcg aatattaata ttgcggatga aatgtttaat      960
   aataaaatta cttataacta tagttcgcca attgcaggaa ttgataaacc aaatggcgcg     1020
   aacatttctt ctcaaattat tggtgtagat acagcttcag gtcaaaacac atacaagcaa     1080
   acagtatttg ttaaccctaa gcaacgagtt ttaggtaata cgtgggtgta tattaaaggc     1140
   taccaagata aaatcgaaga aagtagcggt aaagtaagtg ctacagatac aaaactgaga     1200
   atttttgaag tgaatgatac atctaaatta tcagatagct actatgcaga tccaaatgac     1260
   tctaacctta aagaagtaac agaccaattt aaaaatagaa tctattatga gcatccaaat     1320
   gtagctagta ttaaatttgg tgatattact aaaacatatg tagtattagt agaagggcat     1380
   tacgacaata caggtaagaa cttaaaaact caggttattc aagaaaatgt tgatcctgta     1440
   acaaatagag actacagtat tttcggttgg aataatgaga atgttgtacg ttatggtggt     1500
   ggaagtgctg atggtgattc agcagtaaat taa                                  1533
<210> 119
   <211> 292
   <212> PRT
   <213> Staphylococcus aureus
<400> 119
   Met Gly Thr Gly Gly Lys Gln Ser Ser Asp Lys Ser Asn Gly Lys Leu
   1               5                   10                  15
   Lys Val Val Thr Thr Asn Ser Ile Leu Tyr Asp Met Ala Lys Asn Val
               20                  25                  30
   Gly Gly Asp Asn Val Asp Ile His Ser Ile Val Pro Val Gly Gln Asp
           35                  40                  45
   Pro His Glu Tyr Glu Val Lys Pro Lys Asp Ile Lys Lys Leu Thr Asp
       50                  55                  60
   Ala Asp Val Ile Leu Tyr Asn Gly Leu Asn Leu Glu Thr Gly Asn Gly
   65                  70                  75                  80
   Trp Phe Glu Lys Ala Leu Glu Gln Ala Gly Lys Ser Leu Lys Asp Lys
                   85                  90                  95
   Lys Val Ile Ala Val Ser Lys Asp Val Lys Pro Ile Tyr Leu Asn Gly
               100                 105                 110
   Glu Glu Gly Asn Lys Asp Lys Gln Asp Pro His Ala Trp Leu Ser Leu
           115                 120                 125
   Asp Asn Gly Ile Lys Tyr Val Lys Thr Ile Gln Gln Thr Phe Ile Asp
       130                 135                 140
   Asn Asp Lys Lys His Lys Ala Asp Tyr Glu Lys Gln Gly Asn Lys Tyr
   145                 150                 155                 160
   Ile Ala Gln Leu Glu Lys Leu Asn Asn Asp Ser Lys Asp Lys Phe Asn
                   165                 170                 175
   Asp Ile Pro Lys Glu Gln Arg Ala Met Ile Thr Ser Glu Gly Ala Phe
               180                 185                 190
   Lys Tyr Phe Ser Lys Gln Tyr Gly Ile Thr Pro Gly Tyr Ile Trp Glu
           195                 200                 205
   Ile Asn Thr Glu Lys Gln Gly Thr Pro Glu Gln Met Arg Gln Ala Ile
       210                 215                 220
   Glu Phe Val Lys Lys His Lys Leu Lys His Leu Leu Val Glu Thr Ser
   225                 230                 235                 240
   Val Asp Lys Lys Ala Met Glu Ser Leu Ser Glu Glu Thr Lys Lys Asp
                   245                 250                 255
   Ile Phe Gly Glu Val Tyr Thr Asp Ser Ile Gly Lys Glu Gly Thr Lys
               260                 265                 270
   Gly Asp Ser Tyr Tyr Lys Met Met Lys Ser Asn Ile Glu Thr Val His
           275                 280                 285
   Gly Ser Met Lys
<210> 120
   <211> 879
   <212> DNA
   <213> Staphylococcus aureus
<400> 120
   atgggtactg gtggtaaaca aagcagtgat aagtcaaatg gcaaattaaa agtagtaacg      60
   acgaattcaa ttttatatga tatggctaaa aatgttggtg gagacaacgt cgatattcat     120
   agtattgtac ctgttggtca agatcctcat gaatatgaag ttaaacctaa agatattaaa     180
   aagttaactg acgctgacgt tattttatac aacggattaa atttagagac tggtaacggt     240
   tggtttgaaa aagccttaga acaggctggt aaatcattaa aagataaaaa agttatcgca     300
   gtatcaaaag atgttaaacc tatctattta aacggtgaag aaggcaacaa agataaacaa     360
   gatccacacg catggttaag tttagataat ggtattaaat acgtaaaaac aattcaacaa     420
   acatttatcg ataacgacaa aaaacataaa gcagattatg aaaagcaagg taacaaatac     480
   attgctcaat tggaaaaatt aaataatgac agtaaagaca aatttaatga cattccaaaa     540
   gaacaacgtg ccatgattac aagtgaaggt gccttcaagt acttctcaaa acaatacggt     600
   attacaccag gttatatttg ggaaattaac actgaaaaac aaggtacacc tgaacaaatg     660
   agacaagcta ttgagtttgt taaaaagcac aaattaaaac acttattagt agaaacaagt     720
   gttgataaga aagcaatgga aagtttatct gaagaaacga agaaagatat ctttggtgaa     780
   gtgtacacag attcaatcgg taaagaaggc actaaaggtg actcttacta caaaatgatg     840
   aaatcaaata ttgaaactgt acacggaagc atgaaataa                            879
<210> 121
   <211> 292
   <212> PRT
   <213> Staphylococcus epidermidis
<400> 121
   Met Gly Asn His Ser Asn His Glu His His Ser His Glu Gly Lys Leu
   1               5                   10                  15
   Lys Val Val Thr Thr Asn Ser Ile Leu Tyr Asp Met Val Lys Arg Val
               20                  25                  30
   Gly Gly Asn Lys Val Asp Val His Ser Ile Val Pro Val Gly Gln Asp
           35                  40                  45
   Pro His Glu Tyr Glu Val Lys Pro Lys Asp Ile Lys Ala Leu Thr Asp
       50                  55                  60
   Ala Asp Val Val Phe Tyr Asn Gly Leu Asn Leu Glu Thr Gly Asn Gly
   65 70 75 80
   Trp Phe Glu Lys Ala Leu Asp Gln Ala Gly Lys Ser Thr Lys Asp Lys
   85 90 95
   Asn Val Ile Ala Ala Ser Asn Asn Val Lys Pro Ile Tyr Leu Asn Gly
   100 105 110
   Glu Glu Gly Asn Lys Asn Lys Gln Asp Pro His Ala Trp Leu Ser Leu
   115 120 125
   Glu Asn Gly Ile Lys Tyr Val Lys Thr Ile Gln Lys Ser Leu Glu His
   130 135 140
   His Asp Lys Lys Asp Lys Ser Thr Tyr Glu Lys Gln Gly Asn Ala Tyr
   145 150 155 160
   Ile Ser Lys Leu Glu Glu Leu Asn Lys Asp Ser Lys Asn Lys Phe Asp
   165 170 175
   Asp Ile Pro Lys Asn Gln Arg Ala Met Met Thr Ser Glu Gly Ala Phe
   180 185 190
   Lys Tyr Phe Ala Gln Gln Phe Asp Val Lys Pro Gly Tyr Ile Trp Glu
   195 200 205
   Ile Asn Thr Glu Lys Gln Gly Thr Pro Gly Gln Met Lys Gln Ala Ile
   210 215 220
   Lys Phe Val Lys Asp Asn His Leu Lys His Leu Leu Val Glu Thr Ser
   225 230 235 240
   Val Asp Lys Lys Ala Met Gln Ser Leu Ser Glu Glu Thr Lys Lys Asp
   245 250 255
   Ile Tyr Gly Glu Val Phe Thr Asp Ser Ile Gly Lys Glu Gly Thr Lys
   260 265 270
   Gly Asp Ser Tyr Tyr Lys Met Met Lys Ser Asn Ile Asp Thr Ile His
   275 280 285
   Gly Ser Met Lys
   290
<210> 122
   <211> 879
   <212> DNA
   <213> Staphylococcus epidermidis
<400> 122
   atggggaatc acagtaacca tgaacatcac tcacatgaag gaaaattaaa agttgtaact      60
   acaaactcta ttctctatga catggttaaa cgtgtcggtg gaaataaggt cgatgttcat     120
   agcatcgttc cagtaggaca agatccacat gaatatgagg ttaaacctaa agatattaaa     180
   gcattaacag atgctgacgt tgtattttat aatggtttaa acctagaaac tggaaatggt     240
   tggtttgaaa aagcacttga ccaagcagga aaatcaacaa aagataaaaa tgtgatagca     300
   gcatcaaata atgttaaacc aatatactta aatggtgagg aaggtaacaa aaacaaacaa     360
   gatccacatg catggttaag tttagagaat ggaattaaat acgtaaaaac aatacaaaaa     420
   tcactagaac atcatgataa aaaagataag tctacatatg aaaaacaagg gaatgcatat     480
   atatcaaaat tagaagaact taataaagat agtaaaaata aatttgatga catacccaaa     540
   aatcaacgtg ccatgatgac aagtgaaggt gcatttaaat attttgctca acaattcgat     600
   gttaaaccag gttatatttg ggagataaac acagaaaaac aaggtacacc tggtcaaatg     660
   aaacaagcca ttaaatttgt taaagataat catttaaaac atttattagt cgaaacaagc     720
   gtagataaaa aagctatgca aagtttatca gaagaaacta agaaagatat ttatggtgaa     780
   gtatttaccg actctatagg taaggaaggt actaaaggtg actcatacta taaaatgatg     840
   aaatctaata ttgatacaat acatggtagt atgaaataa                            879
<210> 123
   <211> 531
   <212> PRT
   <213> Staphylococcus aureus
<400> 123
   Met Ala Ser Met Thr Gly Gly Gln Gln Met Gly Ser Glu Asn Ser Val
   1               5                   10                  15
   Thr Gln Ser Asp Ser Ala Ser Asn Glu Ser Lys Ser Asn Asp Ser Ser
               20                  25                  30
   Ser Val Ser Ala Ala Pro Lys Thr Asp Asp Thr Asn Val Ser Asp Thr
           35                  40                  45
   Lys Thr Ser Ser Asn Thr Asn Asn Gly Glu Thr Ser Val Ala Gln Asn
       50                  55                  60
   Pro Ala Gln Gln Glu Thr Thr Gln Ser Ser Ser Thr Asn Ala Thr Thr
   65                  70                  75                  80
   Glu Glu Thr Pro Val Thr Gly Glu Ala Thr Thr Thr Thr Thr Asn Gln
                   85                  90                  95
   Ala Asn Thr Pro Ala Thr Thr Gln Ser Ser Asn Thr Asn Ala Glu Glu
   100 105 110
   Leu Val Asn Gln Thr Ser Asn Glu Thr Thr Phe Asn Asp Thr Asn Thr
   115 120 125
   Val Ser Ser Val Asn Ser Pro Gln Asn Ser Thr Asn Ala Glu Asn Val
   130 135 140
   Ser Thr Thr Gln Asp Thr Ser Thr Glu Ala Thr Pro Ser Asn Asn Glu
   145 150 155 160
   Ser Ala Pro Gln Ser Thr Asp Ala Ser Asn Lys Asp Val Val Asn Gln
   165 170 175
   Ala Val Asn Thr Ser Ala Pro Arg Met Arg Ala Phe Ser Leu Ala Ala
   180 185 190
   Val Ala Ala Asp Ala Pro Ala Ala Gly Thr Asp Ile Thr Asn Gln Leu
   195 200 205
   Thr Asn Val Thr Val Gly Ile Asp Ser Gly Thr Thr Val Tyr Pro His
   210 215 220
   Gln Ala Gly Tyr Val Lys Leu Asn Tyr Gly Phe Ser Val Pro Asn Ser
   225 230 235 240
   Ala Val Lys Gly Asp Thr Phe Lys Ile Thr Val Pro Lys Glu Leu Asn
   245 250 255
   Leu Asn Gly Val Thr Ser Thr Ala Lys Val Pro Pro Ile Met Ala Gly
   260 265 270
   Asp Gln Val Leu Ala Asn Gly Val Ile Asp Ser Asp Gly Asn Val Ile
   275 280 285
   Tyr Thr Phe Thr Asp Tyr Val Asn Thr Lys Asp Asp Val Lys Ala Thr
   290 295 300
   Leu Thr Met Pro Ala Ala Ile Asp Pro Glu Asn Val Lys Lys Thr Gly
   305 310 315 320
   Asn Val Thr Leu Ala Thr Gly Ile Gly Ser Thr Thr Ala Asn Lys Thr
   325 330 335
   Val Leu Val Asp Tyr Glu Lys Tyr Gly Lys Phe Tyr Asn Leu Ser Ile
   340 345 350
   Lys Gly Thr Ile Asp Gln Ile Asp Lys Thr Asn Asn Thr Tyr Arg Gln
   355 360 365
   Thr Ile Tyr Val Asn Pro Ser Gly Asp Asn Val Ile Ala Pro Val Leu
   370 375 380
   Thr Gly Asn Leu Lys Pro Asn Thr Asp Ser Asn Ala Leu Ile Asp Gln
   385 390 395 400
   Gln Asn Thr Ser Ile Lys Val Tyr Lys Val Asp Asn Ala Ala Asp Leu
   405 410 415
   Ser Glu Ser Tyr Phe Val Asn Pro Glu Asn Phe Glu Asp Val Thr Asn
   420 425 430
   Ser Val Asn Ile Thr Phe Pro Asn Pro Asn Gln Tyr Lys Val Glu Phe
   435 440 445
   Asn Thr Pro Asp Asp Gln Ile Thr Thr Pro Tyr Ile Val Val Val Asn
   450 455 460
   Gly His Ile Asp Pro Asn Ser Lys Gly Asp Leu Ala Leu Arg Ser Thr
   465 470 475 480
   Leu Tyr Gly Tyr Asn Ser Asn Ile Ile Trp Arg Ser Met Ser Trp Asp
   485 490 495
   Asn Glu Val Ala Phe Asn Asn Gly Ser Gly Ser Gly Asp Gly Ile Asp
   500 505 510
   Lys Pro Val Val Pro Glu Gln Pro Asp Glu Pro Gly Glu Ile Glu Pro
   515 520 525
   Ile Pro Glu
   530
<210> 124
   <211> 1596
   <212> DNA
   <213> Staphylococcus aureus
<400> 124
   atggctagca tgactggtgg acagcaaatg ggtagtgaaa atagtgttac gcaatctgat      60
   agcgcaagta acgaaagcaa aagtaatgat tcaagtagcg ttagtgctgc acctaaaaca     120
   gacgacacaa acgtgagtga tactaaaaca tcgtcaaaca ctaataatgg cgaaacgagt     180
   gtggcgcaaa atccagcaca acaggaaacg acacaatcat catcaacaaa tgcaactacg     240
   gaagaaacgc cggtaactgg tgaagctact actacgacaa cgaatcaagc taatacaccg     300
   gcaacaactc aatcaagcaa tacaaatgct gaagaattag tgaatcaaac aagtaatgaa     360
   acgactttta atgatactaa tacagtatca tctgtaaatt cacctcaaaa ttctacaaat     420
   gcggaaaatg tttcaacaac gcaagatact tcaactgaag caacaccttc aaacaatgaa     480
   tcagctccac agagtacaga tgcaagtaat aaagatgtag ttaatcaagc ggttaataca     540
   agtgcgccta gaatgagagc atttagttta gcggcagtag ctgcagatgc accggcagct     600
   ggcacagata ttacgaatca gttgacgaat gtgacagttg gtattgactc tggtacgact     660
   gtgtatccac accaagcagg ttatgtcaaa ctgaattatg gtttttcagt gcctaattct     720
   gctgttaaag gtgacacatt caaaataact gtacctaaag aattaaactt aaatggtgta     780
   acttcaactg ctaaagtgcc accaattatg gctggagatc aagtattggc aaatggtgta     840
   atcgatagtg atggtaatgt tatttataca tttacagact atgtaaatac taaagatgat     900
   gtaaaagcaa ctttgaccat gcccgctgct attgaccctg aaaatgttaa aaagacaggt     960
   aatgtgacat tggctactgg cataggtagt acaacagcaa acaaaacagt attagtagat    1020
   tatgaaaaat atggtaagtt ttataactta tctattaaag gtacaattga ccaaatcgat    1080
   aaaacaaata atacgtatcg tcagacaatt tatgtcaatc caagtggaga taacgttatt    1140
   gcgccggttt taacaggtaa tttaaaacca aatacggata gtaatgcatt aatagatcag    1200
   caaaatacaa gtattaaagt atataaagta gataatgcag ctgatttatc tgaaagttac    1260
   tttgtgaatc cagaaaactt tgaggatgtc actaatagtg tgaatattac attcccaaat    1320
   ccaaatcaat ataaagtaga gtttaatacg cctgatgatc aaattacaac accgtatata    1380
   gtagttgtta atggtcatat tgatccgaat agcaaaggtg atttagcttt acgttcaact    1440
   ttatatgggt ataactcgaa tataatttgg cgctctatgt catgggacaa cgaagtagca    1500
   tttaataacg gatcaggttc tggtgacggt atcgataaac cagttgttcc tgaacaacct    1560
   gatgagcctg gtgaaattga accaattcca gagtag                              1596
<210> 125
   <211> 5
   <212> PRT
   <213> Artificial Sequence
<220>
   <223> Description of Artificial Sequence: Synthetic peptide
<220>
   <221> MOD_RES
   <222> (3)..(3)
   <223> Any amino acid
<400> 125
   Leu Pro Xaa Thr Gly
   1               5
<210> 126
   <211> 9
   <212> PRT
   <213> Artificial Sequence
<220>
   <223> Description of Artificial Sequence: Synthetic peptide
<400> 126
   Thr Tyr Thr Phe Thr Asp Tyr Val Asp
   1               5
<210> 127
   <211> 12
   <212> PRT
   <213> Artificial Sequence
<220>
   <223> Description of Artificial Sequence: Synthetic peptide
<400> 127
   Met Arg Gly Ser His His His His His His Gly Ser
   1               5                   10
<210> 128
   <211> 14
   <212> PRT
   <213> Artificial Sequence
<220>
   <223> Description of Artificial Sequence: Synthetic peptide
<400> 128
   Met Ala Ser Met Thr Gly Gly Gln Gln Met Gly Arg Gly Ser
   1               5                   10
<210> 129
   <211> 11
   <212> PRT
   <213> Artificial Sequence
<220>
   <223> Description of Artificial Sequence: Synthetic peptide
<400> 129
   Met Ala Ser Met Thr Gly Gly Gln Gln Met Gly
   1 5 10
<210> 130
   <211> 933
   <212> PRT
   <213> Staphylococcus aureus
<400> 130
   Met Asn Met Lys Lys Lys Glu Lys His Ala Ile Arg Lys Lys Ser Ile
   1               5                   10                  15
   Gly Val Ala Ser Val Leu Val Gly Thr Leu Ile Gly Phe Gly Leu Leu
               20                  25                  30
   Ser Ser Lys Glu Ala Asp Ala Ser Glu Asn Ser Val Thr Gln Ser Asp
           35                  40                  45
   Ser Ala Ser Asn Glu Ser Lys Ser Asn Asp Ser Ser Ser Val Ser Ala
       50                  55                  60
   Ala Pro Lys Thr Asp Asp Thr Asn Val Ser Asp Thr Lys Thr Ser Ser
   65                  70                  75                  80
   Asn Thr Asn Asn Gly Glu Thr Ser Val Ala Gln Asn Pro Ala Gln Gln
                   85                  90                  95
   Glu Thr Thr Gln Ser Ser Ser Thr Asn Ala Thr Thr Glu Glu Thr Pro
               100                 105                 110
   Val Thr Gly Glu Ala Thr Thr Thr Thr Thr Asn Gln Ala Asn Thr Pro
           115                 120                 125
   Ala Thr Thr Gln Ser Ser Asn Thr Asn Ala Glu Glu Leu Val Asn Gln
       130                 135                 140
   Thr Ser Asn Glu Thr Thr Phe Asn Asp Thr Asn Thr Val Ser Ser Val
   145                 150                 155                 160
   Asn Ser Pro Gln Asn Ser Thr Asn Ala Glu Asn Val Ser Thr Thr Gln
                   165                 170                 175
   Asp Thr Ser Thr Glu Ala Thr Pro Ser Asn Asn Glu Ser Ala Pro Gln
               180                 185                 190
   Ser Thr Asp Ala Ser Asn Lys Asp Val Val Asn Gln Ala Val Asn Thr
           195                 200                 205
   Ser Ala Pro Arg Met Arg Ala Phe Ser Leu Ala Ala Val Ala Ala Asp
       210                 215                 220
   Ala Pro Ala Ala Gly Thr Asp Ile Thr Asn Gln Leu Thr Asn Val Thr
   225 230 235 240
   Val Gly Ile Asp Ser Gly Thr Thr Val Tyr Pro His Gln Ala Gly Tyr
   245 250 255
   Val Lys Leu Asn Tyr Gly Phe Ser Val Pro Asn Ser Ala Val Lys Gly
   260 265 270
   Asp Thr Phe Lys Ile Thr Val Pro Lys Glu Leu Asn Leu Asn Gly Val
   275 280 285
   Thr Ser Thr Ala Lys Val Pro Pro Ile Met Ala Gly Asp Gln Val Leu
   290 295 300
   Ala Asn Gly Val Ile Asp Ser Asp Gly Asn Val Ile Tyr Thr Phe Thr
   305 310 315 320
   Asp Tyr Val Asn Thr Lys Asp Asp Val Lys Ala Thr Leu Thr Met Pro
   325 330 335
   Ala Tyr Ile Asp Pro Glu Asn Val Lys Lys Thr Gly Asn Val Thr Leu
   340 345 350
   Ala Thr Gly Ile Gly Ser Thr Thr Ala Asn Lys Thr Val Leu Val Asp
   355 360 365
   Tyr Glu Lys Tyr Gly Lys Phe Tyr Asn Leu Ser Ile Lys Gly Thr Ile
   370 375 380
   Asp Gln Ile Asp Lys Thr Asn Asn Thr Tyr Arg Gln Thr Ile Tyr Val
   385 390 395 400
   Asn Pro Ser Gly Asp Asn Val Ile Ala Pro Val Leu Thr Gly Asn Leu
   405 410 415
   Lys Pro Asn Thr Asp Ser Asn Ala Leu Ile Asp Gln Gln Asn Thr Ser
   420 425 430
   Ile Lys Val Tyr Lys Val Asp Asn Ala Ala Asp Leu Ser Glu Ser Tyr
   435 440 445
   Phe Val Asn Pro Glu Asn Phe Glu Asp Val Thr Asn Ser Val Asn Ile
   450 455 460
   Thr Phe Pro Asn Pro Asn Gln Tyr Lys Val Glu Phe Asn Thr Pro Asp
   465 470 475 480
   Asp Gln Ile Thr Thr Pro Tyr Ile Val Val Val Asn Gly His Ile Asp
   485 490 495
   Pro Asn Ser Lys Gly Asp Leu Ala Leu Arg Ser Thr Leu Tyr Gly Tyr
   500 505 510
   Asn Ser Asn Ile Ile Trp Arg Ser Met Ser Trp Asp Asn Glu Val Ala
   515 520 525
   Phe Asn Asn Gly Ser Gly Ser Gly Asp Gly Ile Asp Lys Pro Val Val
   530 535 540
   Pro Glu Gln Pro Asp Glu Pro Gly Glu Ile Glu Pro Ile Pro Glu Asp
   545 550 555 560
   Ser Asp Ser Asp Pro Gly Ser Asp Ser Gly Ser Asp Ser Asn Ser Asp
   565 570 575
   Ser Gly Ser Asp Ser Gly Ser Asp Ser Thr Ser Asp Ser Gly Ser Asp
   580 585 590
   Ser Ala Ser Asp Ser Asp Ser Ala Ser Asp Ser Asp Ser Ala Ser Asp
   595 600 605
   Ser Asp Ser Ala Ser Asp Ser Asp Ser Ala Ser Asp Ser Asp Ser Asp
   610 615 620
   Asn Asp Ser Asp Ser Asp Ser Asp Ser Asp Ser Asp Ser Asp Ser Asp
   625 630 635 640
   Ser Asp Ser Asp Ser Asp Ser Asp Ser Asp Ser Asp Ser Asp Ser Asp
   645 650 655
   Ser Asp Ser Asp Ser Asp Ser Asp Ser Asp Ser Asp Ser Asp Ser Asp
   660 665 670
   Ser Asp Ser Asp Ser Asp Ser Asp Ser Asp Ser Asp Ser Asp Ser Asp
   675 680 685
   Ser Asp Ser Asp Ser Asp Ser Asp Ser Asp Ser Asp Ser Asp Ser Asp
   690 695 700
   Ser Asp Ser Asp Ser Asp Ser Asp Ser Asp Ser Asp Ser Asp Ser Asp
   705 710 715 720
   Ser Asp Ser Asp Ser Asp Ser Asp Ser Asp Ser Asp Ser Asp Ser Asp
   725 730 735
   Ser Asp Ser Asp Ser Asp Ser Asp Ser Asp Ser Asp Ser Asp Ser Asp
   740 745 750
   Ser Asp Ser Asp Ser Asp Ser Asp Ser Asp Ser Asp Ser Asp Ser Ala
   755 760 765
   Ser Asp Ser Asp Ser Asp Ser Asp Ser Asp Ser Asp Ser Asp Ser Asp
   770 775 780
   Ser Asp Ser Asp Ser Asp Ser Asp Ser Asp Ser Asp Ser Asp Ser Asp
   785 790 795 800
   Ser Asp Ser Asp Ser Asp Ser Glu Ser Asp Ser Asp Ser Glu Ser Asp
   805 810 815
   Ser Asp Ser Asp Ser Asp Ser Asp Ser Asp Ser Asp Ser Asp Ser Asp
   820 825 830
   Ser Asp Ser Asp Ser Ala Ser Asp Ser Asp Ser Gly Ser Asp Ser Asp
   835 840 845
   Ser Ser Ser Asp Ser Asp Ser Glu Ser Asp Ser Asn Ser Asp Ser Glu
   850 855 860
   Ser Gly Ser Asn Asn Asn Val Val Pro Pro Asn Ser Pro Lys Asn Gly
   865 870 875 880
   Thr Asn Ala Ser Asn Lys Asn Glu Ala Lys Asp Ser Lys Glu Pro Leu
   885 890 895
   Pro Asp Thr Gly Ser Glu Asp Glu Ala Asn Thr Ser Leu Ile Trp Gly
   900 905 910
   Leu Leu Ala Ser Ile Gly Ser Leu Leu Leu Phe Arg Arg Lys Lys Glu
   915 920 925
   Asn Lys Asp Lys Lys
   930
<210> 131
   <211> 2802
   <212> DNA
   <213> Staphylococcus aureus
<400> 131
   atgaatatga agaaaaaaga aaaacacgca attcggaaaa aatcgattgg cgtggcttca 60
   gtgcttgtag gtacgttaat cggttttgga ctactcagca gtaaagaagc agatgcaagt 120
   gaaaatagtg ttacgcaatc tgatagcgca agtaacgaaa gcaaaagtaa tgattcaagt 180
   agcgttagtg ctgcacctaa aacagacgac acaaacgtga gtgatactaa aacatcgtca 240
   aacactaata atggcgaaac gagtgtggcg caaaatccag cacaacagga aacgacacaa 300
   tcatcatcaa caaatgcaac tacggaagaa acgccggtaa ctggtgaagc tactactacg 360
   acaacgaatc aagctaatac accggcaaca actcaatcaa gcaatacaaa tgcggaggaa 420
   ttagtgaatc aaacaagtaa tgaaacgact tttaatgata ctaatacagt atcatctgta 480
   aattcacctc aaaattctac aaatgcggaa aatgtttcaa caacgcaaga tacttcaact 540
   gaagcaacac cttcaaacaa tgaatcagct ccacagagta cagatgcaag taataaagat 600
   gtagttaatc aagcggttaa tacaagtgcg cctagaatga gagcatttag tttagcggca 660
   gtagctgcag atgcaccggc agctggcaca gatattacga atcagttgac gaatgtgaca 720
   gttggtattg actctggtac gactgtgtat ccgcaccaag caggttatgt caaactgaat 780
   tatggttttt cagtgcctaa ttctgctgtt aaaggtgaca cattcaaaat aactgtacct 840
   aaagaattaa acttaaatgg tgtaacttca actgctaaag tgccaccaat tatggctgga 900
   gatcaagtat tggcaaatgg tgtaatcgat agtgatggta atgttattta tacatttaca 960
   gactatgtaa atactaaaga tgatgtaaaa gcaactttga ccatgcccgc ttatattgac 1020
   cctgaaaatg ttaaaaagac aggtaatgtg acattggcta ctggcatagg tagtacaaca 1080
   gcaaacaaaa cagtattagt agattatgaa aaatatggta agttttataa cttatctatt 1140
   aaaggtacaa ttgaccaaat cgataaaaca aataatacgt atcgtcagac aatttatgtc 1200
   aatccaagtg gagataacgt tattgcgccg gttttaacag gtaatttaaa accaaatacg 1260
   gatagtaatg cattaataga tcagcaaaat acaagtatta aagtatataa agtagataat 1320
   gcagctgatt tatctgaaag ttactttgtg aatccagaaa actttgagga tgtcactaat 1380
   agtgtgaata ttacattccc aaatccaaat caatataaag tagagtttaa tacgcctgat 1440
   gatcaaatta caacaccgta tatagtagtt gttaatggtc atattgatcc gaatagcaaa 1500
   ggtgatttag ctttacgttc aactttatat gggtataact cgaatataat ttggcgctct 1560
   atgtcatggg acaacgaagt agcatttaat aacggatcag gttctggtga cggtatcgat 1620
   aaaccagttg ttcctgaaca acctgatgag cctggtgaaa ttgaaccaat tccagaggat 1680
   tcagattctg acccaggttc agattctggc agcgattcta attcagatag cggttcagat 1740
   tcgggtagtg attctacatc agatagtggt tcagattcag cgagtgattc agattcagca 1800
   agtgattcag actcagcgag tgattcagat tcagcaagcg attccgactc agcgagcgat 1860
   tccgactcag acaatgactc ggattcagat agcgattctg actcagacag tgactcagat 1920
   tccgacagtg actcagattc agatagcgat tctgactcag acagtgactc agattcagat 1980
   agcgattcag attcagatag cgattcagat tccgacagtg attccgactc agacagcgat 2040
   tctgactccg acagtgattc cgactcagac agcgattcag attccgacag tgattccgac 2100
   tcagatagcg attccgactc agatagcgac tcagattcag acagcgattc agattcagac 2160
   agcgattcag attcagatag cgattcagat tccgacagtg actcagattc cgacagtgac 2220
   tcggattcag atagcgattc agattccgac agtgactcag attccgacag tgactcagac 2280
   tcagacagtg attcggattc agcgagtgat tcggattcag atagtgattc cgactccgac 2340
   agtgactcgg attcagatag cgactcagac tcggatagcg actcggattc agatagcgat 2400
   tcggactcag atagcgattc agaatcagac agcgattcag aatcagacag cgattcagat 2460
   tcagacagcg actcagacag tgactcagat tcagatagtg actcggattc agcgagtgat 2520
   tcagactcag gtagtgactc cgattcatca agtgattccg actcagaaag tgattcaaat 2580
   agcgattccg agtcaggttc taacaataat gtagttccgc ctaattcacc taaaaatggt 2640
   actaatgctt ctaataaaaa tgaggctaaa gatagtaaag aaccattacc agatacaggt 2700
   tctgaagatg aagcaaatac gtcactaatt tggggattat tagcatcaat aggttcatta 2760
   ctacttttca gaagaaaaaa agaaaataaa gataagaaat aa 2802
<210> 132
   <211> 309
   <212> PRT
   <213> Staphylococcus epidermidis
<400> 132
   Met Lys Lys Ile Leu Ala Leu Ala Ile Ala Phe Leu Ile Ile Leu Ala
   1 5 10 15
   Ala Cys Gly Asn His Ser Asn His Glu His His Ser His Glu Gly Lys
   20 25 30
   Leu Lys Val Val Thr Thr Asn Ser Ile Leu Tyr Asp Met Val Lys Arg
   35 40 45
   Val Gly Gly Asn Lys Val Asp Val His Ser Ile Val Pro Val Gly Gln
   50 55 60
   Asp Pro His Glu Tyr Glu Val Lys Pro Lys Asp Ile Lys Ala Leu Thr
   65 70 75 80
   Asp Ala Asp Val Val Phe Tyr Asn Gly Leu Asn Leu Glu Thr Gly Asn
   85 90 95
   Gly Trp Phe Glu Lys Ala Leu Asp Gln Ala Gly Lys Ser Thr Lys Asp
   100 105 110
   Lys Asn Val Ile Ala Ala Ser Asn Asn Val Lys Pro Ile Tyr Leu Asn
   115 120 125
   Gly Glu Glu Gly Asn Lys Asn Lys Gln Asp Pro His Ala Trp Leu Ser
   130 135 140
   Leu Glu Asn Gly Ile Lys Tyr Val Lys Thr Ile Gln Lys Ser Leu Glu
   145 150 155 160
   His His Asp Lys Lys Asp Lys Ser Thr Tyr Glu Lys Gln Gly Asn Ala
   165 170 175
   Tyr Ile Ser Lys Leu Glu Glu Leu Asn Lys Asp Ser Lys Asn Lys Phe
   180 185 190
   Asp Asp Ile Pro Lys Asn Gln Arg Ala Met Met Thr Ser Glu Gly Ala
   195 200 205
   Phe Lys Tyr Phe Ala Gln Gln Phe Asp Val Lys Pro Gly Tyr Ile Trp
   210 215 220
   Glu Ile Asn Thr Glu Lys Gln Gly Thr Pro Gly Gln Met Lys Gln Ala
   225 230 235 240
   Ile Lys Phe Val Lys Asp Asn His Leu Lys His Leu Leu Val Glu Thr
   245 250 255
   Ser Val Asp Lys Lys Ala Met Gln Ser Leu Ser Glu Glu Thr Lys Lys
   260 265 270
   Asp Ile Tyr Gly Glu Val Phe Thr Asp Ser Ile Gly Lys Glu Gly Thr
   275 280 285
   Lys Gly Asp Ser Tyr Tyr Lys Met Met Lys Ser Asn Ile Asp Thr Ile
   290 295 300
   His Gly Ser Met Lys
   305
<210> 133
   <211> 930
   <212> DNA
   <213> Staphylococcus epidermidis
<400> 133
   gtgaaaaaaa ttctcgcttt agcaatagca tttttaatta tccttgccgc atgtgggaat 60
   cacagtaacc atgaacatca ctcacatgaa ggaaaattaa aagttgtaac tacaaactct 120
   attctctatg acatggttaa acgtgtcggt ggaaataagg tcgatgttca tagcatcgtt 180
   ccagtaggac aagatccaca tgaatatgag gttaaaccta aagatattaa agcattaaca 240
   gatgctgacg ttgtatttta taatggttta aacctagaaa ctggaaatgg ttggtttgaa 300
   aaagcacttg accaagcagg aaaatcaaca aaagataaaa atgtgatagc agcatcaaat 360
   aatgttaaac caatatactt aaatggtgag gaaggtaaca aaaacaaaca agatccacat 420
   gcatggttaa gtttagagaa tggaattaaa tacgtaaaaa caatacaaaa atcactagaa 480
   catcatgata aaaaagataa gtctacatat gaaaaacaag ggaatgcata tatatcaaaa 540
   ttagaagaac ttaataaaga tagtaaaaat aaatttgatg acatacccaa aaatcaacgt 600
   gccatgatga caagtgaagg tgcatttaaa tattttgctc aacaattcga tgttaaacca 660
   ggttatattt gggagataaa cacagaaaaa caaggtacac ctggtcaaat gaaacaagcc 720
   attaaatttg ttaaagataa tcatttaaaa catttattag tcgaaacaag cgtagataaa 780
   aaagctatgc aaagtttatc agaagaaact aagaaagata tttatggtga agtatttacc 840
   gactctatag gtaaggaagg tactaaaggt gactcatact ataaaatgat gaaatctaat 900
   attgatacaa tacatggtag tatgaaataa 930
<210> 134
   <211> 309
   <212> PRT
   <213> Staphylococcus aureus
<400> 134
   Met Lys Lys Leu Val Pro Leu Leu Leu Ala Leu Leu Leu Leu Val Ala
   1               5                   10                  15
   Ala Cys Gly Thr Gly Gly Lys Gln Ser Ser Asp Lys Ser Asn Gly Lys
               20                  25                  30
   Leu Lys Val Val Thr Thr Asn Ser Ile Leu Tyr Asp Met Ala Lys Asn
           35                  40                  45
   Val Gly Gly Asp Asn Val Asp Ile His Ser Ile Val Pro Val Gly Gln
       50                  55                  60
   Asp Pro His Glu Tyr Glu Val Lys Pro Lys Asp Ile Lys Lys Leu Thr
   65                  70                  75                  80
   Asp Ala Asp Val Ile Leu Tyr Asn Gly Leu Asn Leu Glu Thr Gly Asn
                   85                  90                  95
   Gly Trp Phe Glu Lys Ala Leu Glu Gln Ala Gly Lys Ser Leu Lys Asp
               100                 105                 110
   Lys Lys Val Ile Ala Val Ser Lys Asp Val Lys Pro Ile Tyr Leu Asn
   115 120 125
   Gly Glu Glu Gly Asn Lys Asp Lys Gln Asp Pro His Ala Trp Leu Ser
   130 135 140
   Leu Asp Asn Gly Ile Lys Tyr Val Lys Thr Ile Gln Gln Thr Phe Ile
   145 150 155 160
   Asp Asn Asp Lys Lys His Lys Ala Asp Tyr Glu Lys Gln Gly Asn Lys
   165 170 175
   Tyr Ile Ala Gln Leu Glu Lys Leu Asn Asn Asp Ser Lys Asp Lys Phe
   180 185 190
   Asn Asp Ile Pro Lys Glu Gln Arg Ala Met Ile Thr Ser Glu Gly Ala
   195 200 205
   Phe Lys Tyr Phe Ser Lys Gln Tyr Gly Ile Thr Pro Gly Tyr Ile Trp
   210 215 220
   Glu Ile Asn Thr Glu Lys Gln Gly Thr Pro Glu Gln Met Arg Gln Ala
   225 230 235 240
   Ile Glu Phe Val Lys Lys His Lys Leu Lys His Leu Leu Val Glu Thr
   245 250 255
   Ser Val Asp Lys Lys Ala Met Glu Ser Leu Ser Glu Glu Thr Lys Lys
   260 265 270
   Asp Ile Phe Gly Glu Val Tyr Thr Asp Ser Ile Gly Lys Glu Gly Thr
   275 280 285
   Lys Gly Asp Ser Tyr Tyr Lys Met Met Lys Ser Asn Ile Glu Thr Val
   290 295 300
   His Gly Ser Met Lys
   305
<210> 135
   <211> 930
   <212> DNA
   <213> Staphylococcus aureus
<400> 135
   atgaaaaaat tagtaccttt attattagcc ttattacttc tagttgctgc atgtggtact      60
   ggtggtaaac aaagcagtga taagtcaaat ggcaaattaa aagtagtaac gacgaattca     120
   attttatatg atatggctaa aaatgttggt ggagacaacg tcgatattca tagtattgta     180
   cctgttggtc aagatcctca tgaatatgaa gttaaaccta aagatattaa aaagttaact     240
   gacgctgacg ttattttata caacggatta aatttagaga ctggtaacgg ttggtttgaa     300
   aaagccttag aacaggctgg taaatcatta aaagataaaa aagttatcgc agtatcaaaa     360
   gatgttaaac ctatctattt aaacggtgaa gaaggcaaca aagataaaca agatccacac     420
   gcatggttaa gtttagataa tggtattaaa tacgtaaaaa caattcaaca aacatttatc     480
   gataacgaca aaaaacataa agcagattat gaaaagcaag gtaacaaata cattgctcaa     540
   ttggaaaaat taaataatga cagtaaagac aaatttaatg acattccaaa agaacaacgt     600
   gccatgatta caagtgaagg tgccttcaag tacttctcaa aacaatacgg tattacacca     660
   ggttatattt gggaaattaa cactgaaaaa caaggtacac ctgaacaaat gagacaagct     720
   attgagtttg ttaaaaagca caaattaaaa cacttattag tagaaacaag tgttgataag     780
   aaagcaatgg aaagtttatc tgaagaaacg aagaaagata tctttggtga agtgtacaca     840
   gattcaatcg gtaaagaagg cactaaaggt gactcttact acaaaatgat gaaatcaaat     900
   attgaaactg tacacggaag catgaaataa                                      930
<210> 136
   <211> 876
   <212> DNA
   <213> Staphylococcus aureus
<400> 136
   atgggtactg gtggtaaaca aagcagtgat aagtcaaatg gcaaattaaa agtagtaacg      60
   acgaattcaa ttttatatga tatggctaaa aatgttggtg gagacaacgt cgatattcat     120
   agtattgtac ctgttggtca agatcctcat gaatatgaag ttaaacctaa agatattaaa     180
   aagttaactg acgctgacgt tattttatac aacggattaa atttagagac tggtaacggt     240
   tggtttgaaa aagccttaga acaggctggt aaatcattaa aagataaaaa agttatcgca     300
   gtatcaaaag atgttaaacc tatctattta aacggtgaag aaggcaacaa agataaacaa     360
   gatccacacg catggttaag tttagataat ggtattaaat acgtaaaaac aattcaacaa     420
   acatttatcg ataacgacaa aaaacataaa gcagattatg aaaagcaagg taacaaatac     480
   attgctcaat tggaaaaatt aaataatgac agtaaagaca aatttaatga cattccaaaa     540
   gaacaacgtg ccatgattac aagtgaaggt gccttcaagt acttctcaaa acaatacggt     600
   attacaccag gttatatttg ggaaattaac actgaaaaac aaggtacacc tgaacaaatg     660
   agacaagcta ttgagtttgt taaaaagcac aaattaaaac acttattagt agaaacaagt     720
   gttgataaga aagcaatgga aagtttatct gaagaaacga agaaagatat ctttggtgaa     780
   gtgtacacag attcaatcgg taaagaaggc actaaaggtg actcttacta caaaatgatg     840
   aaatcaaata ttgaaactgt acacggaagc atgaaa                               876
<210> 137
   <211> 876
   <212> DNA
   <213> Staphylococcus epidermidis
<400> 137
   atggggaatc acagtaacca tgaacatcac tcacatgaag gaaaattaaa agttgtaact    60
   acaaactcta ttctctatga catggttaaa cgtgtcggtg gaaataaggt cgatgttcat   120
   agcatcgttc cagtaggaca agatccacat gaatatgagg ttaaacctaa agatattaaa   180
   gcattaacag atgctgacgt tgtattttat aatggtttaa acctagaaac tggaaatggt   240
   tggtttgaaa aagcacttga ccaagcagga aaatcaacaa aagataaaaa tgtgatagca   300
   gcatcaaata atgttaaacc aatatactta aatggtgagg aaggtaacaa aaacaaacaa   360
   gatccacatg catggttaag tttagagaat ggaattaaat acgtaaaaac aatacaaaaa   420
   tcactagaac atcatgataa aaaagataag tctacatatg aaaaacaagg gaatgcatat   480
   atatcaaaat tagaagaact taataaagat agtaaaaata aatttgatga catacccaaa   540
   aatcaacgtg ccatgatgac aagtgaaggt gcatttaaat attttgctca acaattcgat   600
   gttaaaccag gttatatttg ggagataaac acagaaaaac aaggtacacc tggtcaaatg   660
   aaacaagcca ttaaatttgt taaagataat catttaaaac atttattagt cgaaacaagc   720
   gtagataaaa aagctatgca aagtttatca gaagaaacta agaaagatat ttatggtgaa   780
   gtatttaccg actctatagg taaggaaggt actaaaggtg actcatacta taaaatgatg   840
   aaatctaata ttgatacaat acatggtagt atgaaa                             876